# EUROPEAN PATENT APPLICATION

(11) **EP 4 692 090 A1**
(43) Date of publication of application: **11.02.2026**
(21) Application number: 24784261.0
(22) Date of filing: 02.04.2024
(51) Int. Cl.: C07D 487/06, C07D 498/04, C07D 471/16, A61K 31/55, A61P 35/00

(54) **TRICYCLIC COMPOUND AS ATR KINASE INHIBITOR**

(30) Priority: 03.04.2023 CN 202310348275
(71) Applicant: Shanghai Fosun Pharmaceutical (Group) Co., Ltd., Shanghai 200233 (CN); Shanghai Fosun Pharmaceutical Industrial Development Co., Ltd., Shanghai 201315 (CN)
(72) Inventor: LIU, Peng, Shanghai 200233 (CN); HU, Bin, Shanghai 200233 (CN); ZHENG, Jiamin, Shanghai 201203 (CN); ZHANG, Zhisen, Shanghai 201203 (CN); YU, Huaxing, Shanghai 201203 (CN); DING, Xiao, Shanghai 201203 (CN); YAN, Shangjun, Shanghai 200233 (CN)
(74) Representative: E. Blum & Co. AG
(86) International application number: PCT/CN2024/085457
(87) International publication number: WO 2024/208177

(57) **Abstract**

The present application relates to a compound represented by Formula I, its isomer, isotope-labeled compound, or pharmaceutically acceptable salt thereof, and the use thereof as an ATR kinase inhibitor and for treating a cancer, wherein each group is as defined herein.

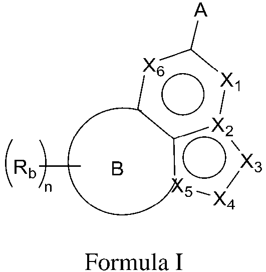

## Description

### Technical Field

The present invention belongs to the field of pharmaceutical technology and relates to tricyclic compounds represented by Formula I, methods for their preparation, pharmaceutical compositions containing the compounds, and their use as therapeutic agents, particularly their use as ATR kinase inhibitors and in the manufacture of medicaments for treating and/or preventing hyperproliferative diseases.

### Background Art

Human cells continuously sustain thousands of DNA damage events daily. To maintain genomic stability and cell survival, cellular repair of DNA damage is crucial. In normal cells, multiple DNA damage repair mechanisms coexist. These mechanisms compensate for each other during the DNA repair process, highly coordinately repairing damage and acting as cellular checkpoints to prevent replication of cells with damaged DNA. Compared to normal cells, tumor cells carry more endogenous cellular damage and have defects in one or more DNA damage repair pathways, resulting in a greater reliance on intact repair pathways.

Ataxia telangiectasia mutated and Rad3-related kinase (ATR kinase) belongs to the PIKK family and is a key protein kinase in the homologous recombination repair pathway. When activated, ATR regulates cellular biological processes through a variety of signals, including arresting cell cycle progression, inhibiting replication origins, and initiating replication forks, and so on, thereby promoting DNA damage repair. ATR kinase works with ataxia telangiectasia mutated kinase (ATM kinase) and numerous other proteins to regulate cellular responses to DNA damage. Due to oncogenic mutations, dysfunctional G1/S checkpoint regulation, and defects in other DNA repair pathways, cancer cells are more dependent than normal cells on the remaining intact repair pathways, including ATR, to regulate cellular DNA damage repair and maintain cell survival, making ATR an important target for cancer therapy.

ATR kinase inhibitors can be used alone or in combination with DNA-damaging agents or other targeted agents related to DNA damage repair pathways for cancer treatment. Currently, five small molecule entities, AZD-6738, RP-3500, VX-970, ART-0380, and ATRN-119, have entered Phase II or Phase III clinical trials, but no corresponding drugs have yet been marketed.

Therefore, there remains an urgent need for ATR kinase inhibitors with higher efficacy and improved pharmacokinetic parameters for the treatment of cancers.

### Contents of the present invention

The represent application provides a tricyclic compound represented by Formula I, which have excellent ATR kinase inhibitory activity and can be used as an ATR kinase inhibitor for the treatment and/or prevention of a proliferative or hyperproliferative disease, such as cancer.

In one aspect, the present application provides a compound represented by Formula I, its isomer, isotope-labeled compound, or pharmaceutically acceptable salt thereof: 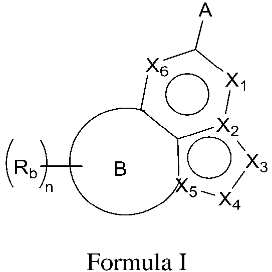 wherein:
○ represents that the ring to which it belongs is an aromatic ring system;
X₁ is selected from the group consisting of N or C(R_{X1});
X₂ is selected from the group consisting of C or N;
X₃ is selected from the group consisting of N, C(R_{X2}), or N(R_{X3});
X₄ is selected from the group consisting of N or C(R_{X4});
X₅ is selected from the group consisting of C or N;
X₆ is selected from the group consisting of N, N(R_{X5}), or C(R_{X6});
R_{X1}, R_{X2}, R_{X3}, R_{X4}, R_{X5}, and R_{X6} are each independently selected from the group consisting of H, halogen, C₆₋₁₀ aryl, C₃₋₁₀ cycloalkyl, C₃₋₁₀ cycloalkenyl, 3- to 10-membered heterocyclyl, or 5- to 10-membered heteroaryl, wherein the C₆₋₁₀ aryl, C₃₋₁₀ cycloalkyl, C₃₋₁₀ cycloalkenyl, 3- to 10-membered heterocyclyl, and 5- to 10-membered heteroaryl are each optionally substituted with 1, 2, or 3 R_{X} groups;
each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
A is selected from the group consisting of 4- to 10-membered heterocyclyl, wherein the 4-to 10-membered heterocyclyl is optionally substituted with 1, 2, or 3 Rₐ groups;
each Rₐ is independently selected from the group consisting of C₁₋₁₀ alkyl, C₁₋₁₀ heteroalkyl, amino, or -S(=O)₂-C₁₋₁₀ alkyl;
Ring B is selected from the group consisting of 5- to 7-membered heterocyclic ring, 5- to 7-membered carbocyclic ring, benzene ring, or 5- to 7-membered heteroaromatic ring;
each R_{b} is independently selected from the group consisting of halogen, hydroxyl, cyano, amino, oxo, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, or 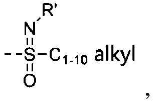 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl and 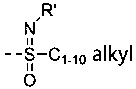 are each optionally substituted with 1, 2, 3 or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONRᵢRⱼ;
R' is selected from the group consisting of H, C₁₋₁₀ alkyl, C₃₋₁₀ cycloalkyl, or CN;
R_{c}, R_{d}, Rₑ, R_{f}, R_{g}, Rₕ, Rᵢ, and Rⱼ are each independently selected from the group consisting of H or C₁₋₁₀ alkyl;
alternatively, two R_{b} groups are connected together to form a C₃₋₁₀ cycloalkyl or a 3- to 10-membered heterocyclyl, wherein the C₃₋₁₀ cycloalkyl and 3- to 10-membered heterocyclyl are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, C₁₋₁₀ heteroalkyl, C₃₋₁₀ cycloalkyl, or 3- to 10-membered heterocyclyl;
n is selected from the group consisting of 0, 1, 2, 3, or 4.

In some embodiments, the compound has structural Formula II: 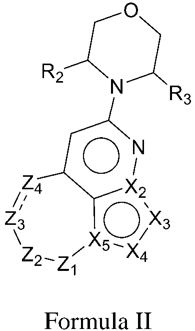 wherein:
○ represents that the ring to which it belongs is an aromatic ring system;
-̅-̅-̅ represents either a single bond or a double bond;
R₁ and R₂ are each independently selected from the group consisting of hydrogen or C₁₋₆ alkyl; or
R₁ and R₂, together with the carbon atom to which they are attached and the adjacent nitrogen atom, form a 4- to 8-membered azacyclic ring;
X₂ is selected from the group consisting of C or N;
X₃ is selected from the group consisting of C(R₃) or N(R₃);
X₄ is selected from the group consisting of N or CH;
X₅ is selected from the group consisting of C or N, and when X₅ is N, X₃ is C(R₃);
R₃ is selected from the group consisting of C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups,
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, and the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently selected from the group consisting of hydrogen or C₁₋₁₀ alkyl;
Z₁ is CR₆R₇;
Z₂ is selected from the group consisting of CR₈R₉, N, NR₁₀, O, S, -C(O)-, or -SO₂-;
Z₃ is selected from the group consisting of CR₁₁, CR₁₂R₁₃, N, NR₁₄, O, -C(O)-, -SO₂-, or -S(O)(NR₁₅)-;
Z₄ is selected from the group consisting of CR₁₆, CR₁₇R₁₈, N, NR₁₉, O, S, -C(O)-, -SO₂-, or -S(O)(NR₂₀)-;
one or two of Z₁, Z₂, Z₃, and Z₄ may be absent; and the ring containing X₅, Z₁, Z₂, Z₃, and Z₄ is a stable ring structure;
R₆, R₇, R₈, R₉, R₁₀, R₁₁, R₁₂, R₁₃, R₁₄, R₁₅, R₁₆, R₁₇, R₁₈, R₁₉, R₂₀ are each independently selected from the group consisting of hydrogen, halogen, hydroxyl, cyano, amino, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl or 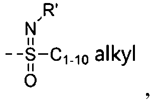 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl and 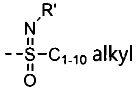 are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, -CONR₂₅R₂₆;
R' is selected from the group consisting of H, C₁₋₁₀ alkyl, C₃₋₁₀ cycloalkyl, or CN;
R₂₁, R₂₂, R₂₃, R₂₄, R₂₅, and R₂₆ are each independently selected from the group consisting of H or C₁₋₁₀ alkyl;
alternatively, R₆ and R₇, R₈ and R₉, R₁₂ and R₁₃, or R₁₇ and R₁₈ are connected together to form a C₃₋₁₀ cycloalkyl or a 3- to 10-membered heterocyclyl, wherein the C₃₋₁₀ cycloalkyl or 3-to 10-membered heterocyclyl is optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, C₁₋₁₀ heteroalkyl, C₃₋₁₀ cycloalkyl, 3- to 10-membered heterocyclyl.

In some embodiments, the compound has the structural Formula IIA, IIB, or IIC: 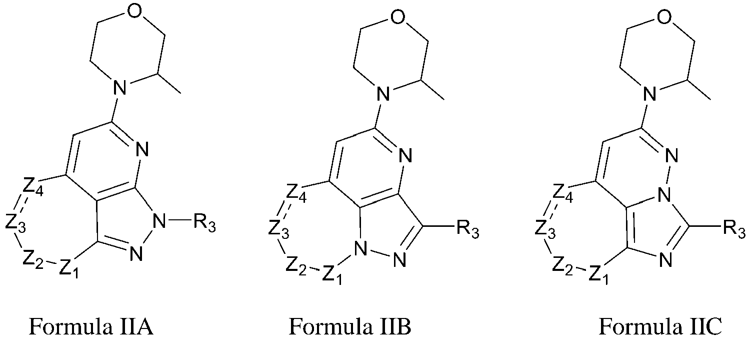 wherein:
R₃ is C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups;
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently hydrogen or C₁₋₁₀ alkyl;
Z₁, Z₂, Z₃, and Z₄ are as defined in Formula II above.

In another aspect, the present application provides a pharmaceutical composition, which comprises the compound, its isomer, isotope-labeled compound, or pharmaceutically acceptable salt thereof of the present application, and a pharmaceutically acceptable excipient.

In another aspect, the present application provides the compound, its isomer, isotope-labeled compound, or pharmaceutically acceptable salt thereof of the present application, which is used as an ATR kinase inhibitor.

In another aspect, the present application provides a use of the compound, its isomer, isotope-labeled compound, or pharmaceutically acceptable salt thereof of the present application, in the manufacture of an ATR kinase inhibitor.

In another aspect, the present application provides a use of the compound, its isomer, isotope-labeled compound, or pharmaceutically acceptable salt thereof of the present application, in the manufacture of a medicament for treating a cancer.

In another aspect, the present application provides the compound, its isomer, isotope-labeled compound, or pharmaceutically acceptable salt thereof of the present application, which is used for treating a cancer.

In another aspect, the present application provides a method for treating a cancer in a patient in need thereof, which comprises administering to the patient a therapeutically effective amount of the compound, its isomer, isotope-labeled compound, or pharmaceutically acceptable salt thereof of the present application.

In some embodiments, the cancer is a colon cancer.

### Specific Modes for Carrying Out the present invention

### Definitions

Unless otherwise defined, technical and scientific terms used herein have the same meanings as commonly understood by one skilled in the art to which the claimed subject matter belongs.

It should be understood that the foregoing brief description and the following detailed description are exemplary and illustrative only and are not intended to limit the subject matter of the present invention.

All references or portions of references cited in the represent application, including but not limited to patents, patent applications, articles, books, manuals, and treatises, are incorporated herein by reference in their entirety.

It should be noted that, unless otherwise indicated, the terms "or" and "alternative" as used herein mean "and/or". Furthermore, the term "comprise" and other forms such as "contain," "include," and "have" are intended to be non-limiting.

Certain chemical groups defined herein are preceded by a shorthand notation to indicate the total number of carbon atoms present in the groups. For example, C₁₋₁₀ alkyl refers to an alkyl, as defined below, having a total of 1 to 10 carbon atoms; C₃₋₁₀ cycloalkyl refers to a cycloalkyl, as defined below, having a total of 3 to 10 carbon atoms; and C₆₋₁₀ aryl refers to an aryl group, as defined below, having a total of 6 to 10 carbon atoms. The total number of carbon atoms in the shorthand notation does not include carbons that may be present in substituents of the group.

In addition to the foregoing, when used in the description and claims of the represent application, unless otherwise specifically indicated, the following terms have the meanings indicated below:
The term "amino" refers to -NH₂ groups.

The term "cyano" refers to a -CN group.

The term "hydroxyl" refers to a -OH group.

The term "oxo" refers to a =O substituent.

As used herein, the term "halogen" refers to fluorine, chlorine, bromine, or iodine, preferably fluorine.

As used herein, as a stand-alone group or as part of another group, the term "alkyl" refers to a straight-chain or branched group consisting solely of carbon and hydrogen atoms, free of unsaturated bonds, and attached to the rest of the molecule by a single bond. Alkyls can have, for example, 1 to 10, 1 to 8, 1 to 6, or 1 to 4 carbon atoms. Examples of alkyls include, but are not limited to, methyl, ethyl, propyl, isopropyl, n-butyl, isobutyl, tert-butyl, n-pentyl, 2-pentyl, hexyl, heptyl, 2-methylhexyl, 3-methylhexyl, octyl, nonyl, decyl, and the like. Hydrogen atoms on an alkyl may be optionally replaced by any suitable group, such as halogen, hydroxyl, alkyl, haloalkyl, alkoxy, cycloalkyl, heterocyclyl, and the like.

As used herein, the term "heteroalkyl" refers to an alkyl as defined above that contains one, two, or three heteroatoms selected from the group consisting of nitrogen, oxygen, and sulfur in the alkyl chain. As used herein, heteroalkyl primarily includes an alkyl containing oxygen in the alkyl chain, including but not limited to alkoxy, alkoxyalkyl, alkoxyalkoxyalkyl, and the like. Examples of alkoxy include but are not limited to methoxy, ethoxy, propoxy, isopropoxy, n-butoxy, isobutoxy, tert-butoxy, and the like. Examples of alkoxyalkyl include but are not limited to methoxymethyl, ethoxymethyl, propoxymethyl, methoxyethyl, ethoxyethyl, propoxyethyl, and the like. Hydrogen atoms on heteroalkyl may be optionally replaced by any suitable group, such as halogen, hydroxyl, alkyl, haloalkyl, cycloalkyl, heterocyclyl, and the like.

As used herein, the term "hydroxy-alkyl" refers to an alkyl as defined above that has a hydroxyl substituent at a suitable position on the alkyl chain. Examples of hydroxy-alkyl include, but are not limited to, hydroxymethyl, hydroxyethyl, hydroxypropyl, hydroxybutyl, 2-hydroxyethyl, 2-hydroxypropyl, 2-hydroxybutyl, and the like.

As used herein, the term "haloalkyl" refers to an alkyl as defined above that is substituted with one or more halogen atoms. Examples include, but are not limited to, fluoromethyl, difluoromethyl, trifluoromethyl, fluoroethyl, 1,1-difluoroethyl, chloromethyl, chloroethyl, dichloromethyl, 1,2-dichloroethyl, and the like.

As used herein, the term "alkenyl" refers to a straight or branched hydrocarbon chain group consisting solely of carbon and hydrogen atoms, containing at least one double bond, having, for example, 2 to 10, 2 to 8, 2 to 6, 2 to 5, or 2 to 4 carbon atoms, and attached to the rest of the molecule by a single bond. Examples include, but are not limited to, ethenyl, propenyl, allyl, but-1-enyl, but-2-enyl, pent-1-enyl, pent-2-enyl, pent-1,4-dienyl, and the like. The hydrogen atoms on alkenyl may be optionally substituted with any suitable group, such as halogen, hydroxyl, amino, monosubstituted amino, disubstituted amino, alkyl, haloalkyl, alkoxy, cycloalkyl, heterocyclyl, and the like.

As used herein, the term "alkynyl" refers to a straight or branched hydrocarbon chain group consisting solely of carbon and hydrogen atoms, containing at least one triple bond and optionally one or more double bonds, having, for example, 2 to 10, 2 to 8, 2 to 6, 2 to 5, or 2 to 4 carbon atoms, and attached to the rest of the molecule by a single bond. Examples of alkynyl include, but are not limited to, ethynyl, prop-1-ynyl, pent-1-en-4-ynyl, and the like. The hydrogen atoms on alkynyl may be optionally substituted with any suitable group, such as halogen, hydroxyl, amino, alkyl, haloalkyl, alkoxy, cycloalkyl, heterocyclyl, and the like.

In the represent application, as an independent group or a part of another group, the term "cycloalkyl" refers to a stable, saturated, non-aromatic monocyclic or polycyclic hydrocarbon group consisting only of carbon atoms and hydrogen atoms, which may include spiro, fused or bridged ring systems, having, for example, 3 to 12, 3 to 10, 3 to 8 or 3 to 6 carbon atoms, and connected to the rest of the molecule by a single bond via any suitable carbon atom on the ring. Cycloalkyl includes, but is not limited to, cyclopropyl, cyclobutyl, cyclopentyl, cyclohexyl, cycloheptyl, cyclooctyl, spiro[2.3]hexyl, spiro[3.3]heptyl, bicyclo[1.1.1]pentyl, bicyclo[2.2.1]heptyl, bicyclo[2.2.2]octyl, bicyclo[3.1.1]heptyl, bicyclo[3.2.1]octyl and adamantyl, etc. The hydrogen atom on cycloalkyl may be optionally substituted with any suitable group, such as halogen, hydroxyl, cyano, alkyl, haloalkyl, hydroxyalkyl, heteroalkyl, cycloalkyl, heterocyclyl, and the like.

As used herein, the term "cycloalkenyl" refers to a stable, non-aromatic monocyclic or polycyclic hydrocarbon group consisting solely of carbon and hydrogen atoms and containing double bonds, which may include spiro, fused, or bridged ring systems, having, for example, 3 to 12, 3 to 10, 3 to 8, or 3 to 6 carbon atoms, and attached to the rest of the molecule by a single bond via any suitable carbon atom in the ring. Examples of cycloalkenyl include, but are not limited to, cyclobutenyl, cyclopentenyl, cyclohexenyl, 1,3-cyclohexadienyl, 1,4-cyclohexadienyl, 1*H*-indenyl, 2,3-dihydroindenyl, 1,2,3,4-tetrahydro-naphthyl, 5,6,7,8-tetrahydro-naphthyl, 8,9-dihydro-7*H*-benzocyclohepten-6-yl, 6,7,8,9-tetrahydro-5-hydro-benzocycloheptenyl, 5,6,7,8,9,10-hexahydro-benzocyclooctenyl, fluorenyl, bicyclo[2.2.1]heptenyl, bicyclo[2.2.2]octyl, bicyclo[2.2.2]octenyl, bicyclo[3.2.1]octenyl, octahydro-4,7-methylene-1-hydro-indenyl, and octahydro-2,5-methylene-pentalenyl, and the like. The hydrogen atoms on cycloalkenyl may be optionally substituted with any suitable group, such as halogen, hydroxyl, amino, monosubstituted amino, disubstituted amino, alkyl, alkoxy, heterocyclyl, and the like.

As used herein, the term "heterocyclic ring" or "heterocyclyl" as an independent group or as a part of another group refers to a stable 3- to 12-membered non-aromatic cyclic group containing 1 to 4 heteroatoms selected from the group consisting of nitrogen, oxygen, and sulfur. The heterocyclic ring or heterocyclyl may be a monocyclic, bicyclic, tricyclic, or polycyclic ring system, which may include spiro, fused, or bridged ring systems. For example, the heterocyclic ring or heterocyclyl can be a stable 3- to 10-membered non-aromatic monocyclic, bicyclic, spiro, fused, or bridged ring group containing 1, 2, or 3 heteroatoms selected from the group consisting of nitrogen, oxygen, and sulfur, for example, a stable 3- to 8-membered non-aromatic monocyclic, bicyclic, spiro, fused, or bridged ring group containing 1, 2, or 3 heteroatoms selected from the group consisting of nitrogen, oxygen, and sulfur, for example, a stable 4- to 8-membered non-aromatic monocyclic, bicyclic, spiro, fused, or bridged ring group containing 1 or 2 heteroatoms selected from the group consisting of nitrogen, oxygen, and sulfur. In the heterocyclic ring or heterocyclyl, the nitrogen, carbon, or sulfur atom can be optionally oxidized; the nitrogen atom can be optionally quaternized; and the heterocyclic ring or heterocyclyl can be partially or fully saturated. The heterocyclic ring or heterocyclyl can be attached to the rest of the molecule by a single bond via a carbon atom or a heteroatom. In a heterocyclic ring or heterocyclyl containing fused rings, one or more rings can be aryl or heteroaryl, provided that the point of attachment to the rest of the molecule is a non-aromatic ring atom. Examples of heterocyclic ring or heterocyclyl include, but are not limited to, oxetanyl, azetidinyl, pyranyl, tetrahydropyranyl, thiopyranyl, tetrahydrofuranyl, morpholinyl, thiomorpholinyl, piperazinyl, piperidinyl, oxazinyl, dioxolanyl, tetrahydroisoquinolinyl, decahydroisoquinolinyl, imidazolinyl, imidazolidinyl, quinolizinyl, thiazolidinyl, isothiazolidinyl, isoxazolidinyl, indolinyl, octahydroindolyl, octahydroisoindolyl, pyrrolidinyl, pyrazolidinyl, 8-oxabicyclo[3.2.1]octyl, 8-oxa-3-azabicyclo[3.2.1]octyl, 3-oxa-8-azabicyclo[3.2.1]octyl, 3,6-dihydro-2H-pyranyl, 3-oxabicyclo[4.1.0]heptyl, 2-oxa-5-azabicyclo[2.2.1]heptyl, etc. The heterocyclic ring or heterocyclyl may be optionally substituted with any suitable substituent, including but not limited to halogen, hydroxyl, amino, alkyl, alkoxy, alkylcarbonyl, etc.

In the represent application, "azacyclic ring" or "azacyclyl" refers to a heterocyclic or heterocyclyl as defined above that contains at least one nitrogen atom in the ring.

As used herein, the term "aryl," as an independent group or as a part of another group, refers to a ring system having 6 to 18, preferably 6 to 12, more preferably 6 to 10, carbon atoms, and at least one aromatic ring. An aryl can be a monocyclic, bicyclic, tricyclic, or polycyclic system, which can include fused or bridged ring systems. An aryl is attached to the rest of the molecule by a single bond via an aromatic ring atom. Examples of aryl include, but are not limited to, phenyl, naphthyl, anthracenyl, phenanthrenyl, fluorenyl, and the like.

As used herein, the term "heteroaromatic ring" or "heteroaryl", as an independent group or as a part of another group, refers to a 5- to 16-membered ring system group having 1 to 4 heteroatoms selected from the group consisting of nitrogen, oxygen, and sulfur within the ring, and at least one aromatic ring. A heteroaryl can be a monocyclic, bicyclic, tricyclic, or polycyclic ring system, which can include fused or bridged ring systems, provided that the point of attachment is through an aromatic ring atom. In the heteroaromatic ring or heteroaryl, the nitrogen, carbon or sulfur atom may be optionally oxidized; the nitrogen atom may be optionally quaternized. For example, the heteroaromatic ring or heteroaryl may be a stable 5- to 10-membered aromatic monocyclic or bicyclic group containing 1 to 3 heteroatoms selected from the group consisting of nitrogen, oxygen and sulfur, for example, a stable 5- to 8-membered aromatic monocyclic or bicyclic group containing 1 to 3 heteroatoms selected from the group consisting of nitrogen, oxygen and sulfur, for example, a stable 5- to 6-membered aromatic monocyclic group containing 1 to 2 heteroatoms selected from the group consisting of nitrogen, oxygen and sulfur. Examples of heteroaromatic ring or heteroaryl include, but are not limited to, thienyl, furanyl, pyrrolyl, imidazolyl, benzimidazolyl, pyrazolyl, benzopyrazolyl, triazolyl, tetrazolyl, pyridinyl, pyrazinyl, triazinyl, pyrimidinyl, pyridazinyl, indolizinyl, indolyl, isoindolyl, indazolyl, isoindazolyl, purinyl, quinolyl, isoquinolyl, phenopiazinyl, naphthyridinyl, quinoxalinyl, pteridinyl, carbazolyl, carbolinyl, phenanthridinyl, phenanthrolinyl, acridinyl, phenazinyl, thiazolyl, isothiazolyl, benzothiazolyl, benzothienyl, oxazolyl, isoxazolyl, oxadiazolyl, oxatriazolyl, cinnolinyl, quinazolinyl, phenylthio, indolizinyl, orthophenanthrolenyl, phenoxazinyl, phenothiazinyl, 4,5,6,7-tetrahydrobenzo[b]thienyl, naphthopyridinyl, imidazo[1,2-*a*]pyridinyl, and the like. The heteroaromatic ring or heteroaryl may be optionally substituted with any suitable substituent, including, but not limited to, halogen, hydroxyl, amino, alkyl, haloalkyl, alkoxy, alkylcarbonyl, and the like.

The term "isomer" refers to an isomer resulting from a difference in the spatial arrangement of atoms in a molecule. Unless otherwise indicated, the compound described herein comprises all isomers thereof, such as stereoisomers, geometric isomers, tautomers, and stable conformers, and may exist as a mixture of isomers or as an isolated isomer. For example, when the compound has an asymmetric carbon atom, enantiomers and diastereomers may be produced; when the compound has a carbon-carbon double bond, a carbon-nitrogen double bond, or a ring structure, cis-trans isomers may be produced. Methods for preparing optically active products from optically inactive starting materials are known in the art, such as by resolving racemic mixtures or by stereoselective synthesis.

The compounds described herein may have one or more asymmetric carbon atoms (optical centers), and some compounds may also have double bonds or ring structures. All resulting isomers, such as racemates, enantiomers, diastereomers, geometric isomers, regioisomers, and individual isomers (e.g., individual enantiomers), and mixtures thereof, are intended to fall within the scope of the present invention. When a stereochemical description is made, it refers to a compound in which one isomer is present and substantially free of the other isomer. "Substantially free" of the other isomer means that the ratio of the two isomers is at least 80/20, for example, 90/10, or 95/5 or higher. In some embodiments, one isomer will be present in an amount of at least 99%.

The compound of the present invention may contain unnatural proportions of atomic isotopes at one or more atoms constituting the compound. For example, the compound may be labeled with a radioactive isotope, such as deuterium (²H), tritium (³H), iodine-125 (¹²⁵I), or C-14 (¹⁴C). All isotopic variations of the compound of the present invention, whether radioactive or not, are encompassed by the present invention. In some embodiments of the present invention, reference to H encompasses deuterium or tritium. In some embodiments, isotope-labeled compounds of the present invention refer to deuterated compounds.

As used herein, the term "optionally" indicates that the subsequently described event or circumstance may or may not occur, and that the description includes instances where the event or circumstance occurs and instances where it does not. For example, "alkyl optionally substituted with one or more halogens" means that the alkyl is unsubstituted or substituted with one or more halogens, and the description includes both substituted and unsubstituted alkyls.

As used herein, the term "pharmaceutically acceptable salt" refers to a salt that converts a basic group in the parent compound into a salt form that retains the biological efficacy of the free base compound and has no biological or other adverse effects.

As used herein, the term "pharmaceutical composition" refers to a formulation of the compound of the present invention and a medium generally accepted in the art for delivering biologically active compounds to a mammal (e.g., a human). The medium includes pharmaceutically acceptable excipients. The pharmaceutical composition of the present invention may be a single preparation or a combination of multiple preparations.

As used herein, the term "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authorities as acceptable for human or animal use.

As used herein, the term "subject" or "patient" refers to an individual suffering from a disease, disorder, or condition, including mammals and non-mammals. In one embodiment, the mammal is a human.

As used herein, the term "treatment" includes alleviating, relieving, or ameliorating the symptoms of a disease or condition, inhibiting the disease or condition, such as arresting its progression, relieving the disease or condition, causing amelioration of the disease or condition, alleviating symptoms caused by the disease or condition, or halting symptoms of the disease or condition, preventing other symptoms, and ameliorating or preventing the underlying metabolic cause of the symptoms. Furthermore, the term encompasses prophylactic purposes. The term also encompasses achieving a therapeutic effect and/or a prophylactic effect. The therapeutic effect refers to curing or ameliorating the underlying disease being treated. Furthermore, curing or ameliorating one or more physiological symptoms associated with the underlying disease is also a therapeutic effect, e.g., observing improvement in the patient's condition, even though the patient may still be affected by the underlying disease. For prophylactic effects, the composition can be administered to a patient at risk for a particular disease or to a patient suffering from one or more physiological symptoms of the disease, even if the disease has not yet been diagnosed.

As used herein, the term "administering" refers to a method in which a compound or composition is delivered to a desired site for biological action. Such method includes, but is not limited to, oral route administration, intraduodenal route administration, parenteral injection (including intravenous, subcutaneous, intraperitoneal, intramuscular, intraarterial injection or infusion), topical application, and rectal administration. A person skilled in the art well know the techniques suitable for administering the compounds and methods described herein, such as those discussed by Goodman and Gilman, The Pharmacological Basis of Therapeutics, current ed.; Pergamon; and Remington's, Pharmaceutical Sciences (current edition), Mack Publishing Co., Easton, Pa.

As used herein, the term "effective amount" or "therapeutically effective amount" refers to an amount of at least one active substance (e.g., the compound of the present invention) sufficient, upon administration, to alleviate to some extent one or more symptoms of the disease or condition being treated. This can result in a reduction and/or alleviation of the signs, symptoms, or causes of the disease, or any other desired change in a biological system. For example, effective amount for therapy is the amount of a composition comprising the compound disclosed herein required to provide a clinically significant alleviation of symptoms. Techniques such as dose escalation studies can be used to determine the effective amount appropriate for any individual case.

### Compound and pharmaceutical composition of the present application

In one aspect, the present invention provides a compound represented by Formula I, its isomer, isotope-labeled compound, or pharmaceutically acceptable salt thereof: 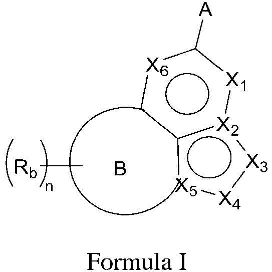 wherein:
○ indicates that the ring to which it belongs is an aromatic ring system;
X₁ is selected from the group consisting of N or C(R_{X1});
X₂ is selected from the group consisting of C or N;
X₃ is selected from the group consisting of N, C(R_{X2}), or N(R_{X3});
X₄ is selected from the group consisting of N or C(R_{X4});
X₅ is selected from the group consisting of C or N;
X₆ is selected from the group consisting of N, N(R_{X5}), or C(R_{X6});
R_{X1}, R_{X2}, R_{X3}, R_{X4}, R_{X5}, and R_{X6} are each independently selected from the group consisting of H, halogen, C₆₋₁₀ aryl, C₃₋₁₀ cycloalkyl, C₃₋₁₀ cycloalkenyl, 3- to 10-membered heterocyclyl, or 5- to 10-membered heteroaryl, wherein the C₆₋₁₀ aryl, C₃₋₁₀ cycloalkyl, C₃₋₁₀ cycloalkenyl, 3- to 10-membered heterocyclyl, and 5- to 10-membered heteroaryl are each optionally substituted with 1, 2, or 3 R_{X} groups;
each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
A is selected from the group consisting of 4- to 10-membered heterocyclyl, wherein the 4-to 10-membered heterocyclyl is optionally substituted with 1, 2, or 3 Rₐ groups;
each Rₐ is independently selected from the group consisting of C₁₋₁₀ alkyl, C₁₋₁₀ heteroalkyl, amino, or -S(=O)₂-C₁₋₁₀ alkyl;
Ring B is selected from the group consisting of 5- to 7-membered heterocyclic ring, 5- to 7-membered carbocyclic ring, benzene ring, or 5- to 7-membered heteroaromatic ring;
each R_{b} is independently selected from the group consisting of halogen, hydroxyl, cyano, amino, oxo, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, or 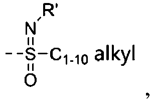 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl and 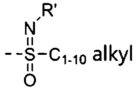 are each optionally substituted with 1, 2, 3 or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONRᵢRⱼ;
R' is selected from the group consisting of H, C₁₋₁₀ alkyl, C₃₋₁₀ cycloalkyl, or CN;
R_{c}, R_{d}, R_{c}, R_{f}, R_{g}, Rₕ, Rᵢ, and Rⱼ are each independently selected from the group consisting of H or C₁₋₁₀ alkyl;
alternatively, two R_{b} groups are connected together to form a C₃₋₁₀ cycloalkyl or a 3- to 10-membered heterocyclyl, wherein the C₃₋₁₀ cycloalkyl and 3- to 10-membered heterocyclyl are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, C₁₋₁₀ heteroalkyl, C₃₋₁₀ cycloalkyl, or 3- to 10-membered heterocyclyl;
n is selected from the group consisting of 0, 1, 2, 3, or 4.

In one embodiment of the compound represented by Formula I, X₁ is N. In one embodiment of the compound represented by Formula I, X₁ is CH. In one embodiment of the compound represented by Formula I, X₁ is C(R_{X1}), wherein R_{X1} is selected from the group consisting of halogen, C₆₋₁₀ aryl, C₃₋₁₀ cycloalkyl, C₃₋₁₀ cycloalkenyl, 3- to 10-membered heterocyclyl or 5-10-membered heteroaryl, wherein the C₆₋₁₀ aryl, C₃₋₁₀ cycloalkyl, C₃₋₁₀ cycloalkenyl, 3- to 10-membered heterocyclyl or 5- 10-membered heteroaryl are each optionally substituted with 1, 2 or 3 R_{X} groups, and each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, 3- to 10-membered heterocyclyl, and -NR_{c}R_{d} are each optionally substituted with 1, 2, or 3 substituents independently selected from the group consisting of halogen, cyano, hydroxyl, or C₁₋₆ alkyl, wherein R_{c} and Rₐ are each independently hydrogen or C₁₋₆ alkyl.

In one embodiment of the compound represented by Formula I, X₂ is C. In one embodiment of the compound represented by Formula I, X₂ is N.

In one embodiment of the compound represented by Formula I, X₃ is N. In one embodiment of the compound represented by Formula I, X₃ is C(R_{X2}) or N(R_{X3}), wherein R_{X2} and R_{X3} are independently selected from the group consisting of H, halogen, C₆₋₁₀ aryl, C₃₋₁₀ cycloalkyl, C₃₋₁₀ cycloalkenyl, 3- to 10-membered heterocyclyl or 5- 10-membered heteroaryl, and the halogen, C₆₋₁₀ aryl, C₃₋₁₀ cycloalkyl, C₃₋₁₀ cycloalkenyl, 3- to 10-membered heterocyclyl or 5-10-membered heteroaryl are optionally substituted with 1, 2 or 3 R_{X} groups; each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl, wherein R_{c} and R_{d} are each independently hydrogen or C₁₋₆ alkyl.

In one embodiment of the compound represented by Formula I, X₃ is selected from the group consisting of C(R_{X2}) or N(R_{X3}), wherein R_{X2} and R_{X3} are independently selected from the group consisting of 5- to 10-membered heteroaryl, wherein the 5- to 10-membered heteroaryl is optionally substituted with 1, 2, or 3 R_{X} groups, and each R_{X} is as defined above. In one embodiment of the compound represented by Formula I, X₃ is selected from the group consisting of C(R_{X2}) or N(R_{X3}), wherein R_{X2} and R_{X3} are independently selected from the group consisting of 5- to 6-membered heteroaryl, and the 5- to 6-membered heteroaryl is optionally substituted with 1 or 2 R_{X} groups; each R_{X} is as defined above. In one embodiment of the compound represented by Formula I, X₃ is selected from the group consisting of C(R_{X2}) or N(R_{X3}), wherein R_{X2} and R_{X3} are independently selected from the group consisting of 5-membered heteroaryl, and the 5-membered heteroaryl is optionally substituted with one or two R_{X} groups; each R_{X} is independently selected from the group consisting of C₁₋₆ alkyl. In one embodiment of the compound represented by Formula I, X₃ is selected from the group consisting of C(R_{X2}) or N(R_{X3}), wherein R_{X2} and R_{X3} are independently pyrazolyl, and the pyrazolyl is optionally substituted with one substituent selected from the group consisting of C₁₋₆ alkyl, such as C₁₋₄ alkyl, such as methyl. In one embodiment of the compound represented by Formula I, X₃ is selected from the group consisting of C(R_{X2}) or N(R_{X3}), wherein R_{X2} and R_{X3} are independently pyrazolyl.

In one embodiment of the compound represented by Formula I, X₄ is N. In one embodiment of the compound represented by Formula I, X₄ is CH. In one embodiment of the compound represented by Formula I, X₄ is C(R_{X4}), wherein R_{X4} is selected from the group consisting of halogen, C₆₋₁₀ aryl, C₃₋₁₀ cycloalkyl, C₃₋₁₀ cycloalkenyl, 3- to 10-membered heterocyclyl or 5-10-membered heteroaryl, wherein the C₆₋₁₀ aryl, C₃₋₁₀ cycloalkyl, C₃₋₁₀ cycloalkenyl, 3- to 10-membered heterocyclyl and 5- 10-membered heteroaryl are each optionally substituted with 1, 2 or 3 R_{X} groups, and each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl, and wherein R_{c} and R_{d} are each independently hydrogen or C₁₋₆ alkyl.

In one embodiment of the compound represented by Formula I, X₅ is C. In one embodiment of the compound represented by Formula I, X₅ is N.

In one embodiment of the compound represented by Formula I, X₆ is CH. In one embodiment of the compound represented by Formula I, X₆ is N. In one embodiment of the compound represented by Formula I, X₆ is selected from the group consisting of N(R_{X5}) or C(R_{X6}), wherein R_{X5} and R_{X6} are each independently selected from the group consisting of halogen, C₆₋₁₀ aryl, C₃₋₁₀ cycloalkyl, C₃₋₁₀ cycloalkenyl, 3- to 10-membered heterocyclyl or 5-10-membered heteroaryl, wherein the C₆₋₁₀ aryl, C₃₋₁₀ cycloalkyl, C₃₋₁₀ cycloalkenyl, 3- to 10-membered heterocyclyl and 5- 10-membered heteroaryl are each optionally substituted with 1, 2 or 3 R_{X} groups; each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl, wherein R_{c} and R_{d} are each independently hydrogen or C₁₋₆ alkyl.

In one embodiment of the compound represented by Formula I, A is a 4- to 10-membered heterocyclyl, and the 4- to 10-membered heterocyclyl is optionally substituted with 1, 2, or 3 Rₐ groups, each Rₐ independently selected from the group consisting of C₁₋₁₀ alkyl, C₁₋₁₀ heteroalkyl, amino, or -S(=O)₂-C₁₋₁₀ alkyl. In one embodiment of the compound represented by Formula I, A is a 5- to 8-membered heterocyclyl, the 5- to 8-membered heterocyclyl is optionally substituted with 1, 2 or 3 Rₐ groups, each Rₐ is independently selected from the group consisting of C₁₋₁₀ alkyl, C₁₋₁₀ heteroalkyl, amino or -S(=O)₂-C₁₋₁₀ alkyl. In one embodiment of the compound represented by Formula I, A is a 5- to 8-membered heterocyclyl containing 1 or 2 heteroatoms selected from the group consisting of nitrogen and oxygen, the 5- to 8-membered heterocyclyl is optionally substituted with 1 or 2 Rₐ groups, each Rₐ is independently selected from the group consisting of C₁₋₆ alkyl, C₁₋₆ heteroalkyl, amino or -S(=O)₂-C₁₋₆ alkyl. In one embodiment of the compound represented by Formula I, A is a 6-membered heterocyclyl, the 6-membered heterocyclyl is optionally substituted with 1 or 2 Rₐ, each Rₐ is independently selected from the group consisting of C₁₋₁₀ alkyl, C₁₋₁₀ heteroalkyl, amino or -S(=O)₂-C₁₋₁₀ alkyl. In one embodiment of the compound represented by Formula I, A is a 6-membered azacyclic ring group, and the azacyclic ring is optionally substituted with one or two Rₐ groups, each Rₐ is independently selected from the group consisting of C₁₋₁₀ alkyl, such as C₁₋₆ alkyl, such as methyl.

In one embodiment of the compound represented by Formula I, A is selected from the group consisting of:

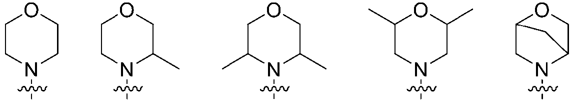

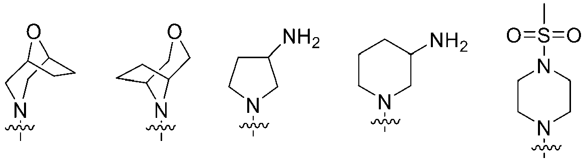

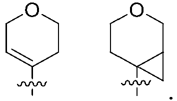

In one embodiment of the compound represented by Formula I, A is 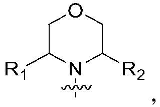 wherein R₁ and R₂ are each independently hydrogen or C₁₋₆ alkyl, such as C₁₋₄ alkyl, such as methyl. In one embodiment of the compound represented by Formula I, A is 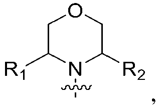 wherein R₁ and R₂, together with the carbon atom to which they are attached and the adjacent nitrogen atom, form a 4- to 8-membered azacyclic ring, such as

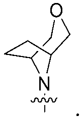

In one embodiment of the compound represented by Formula I, A is 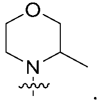 In one embodiment of the compound represented by Formula I, A is

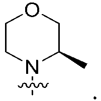

In one embodiment of the compound represented by Formula I, Ring B is selected from the group consisting of 5- to 7-membered heterocyclic ring, 5- to 7-membered carbocyclic ring, benzene ring, or 5- to 7-membered heteroaromatic ring. In one embodiment of the compound represented by Formula I, Ring B is a 5-membered ring. In another embodiment, Ring B is a 6-membered ring. In another embodiment, Ring B is a 7-membered ring. In one embodiment of the compound represented by Formula I, Ring B is selected from the group consisting of 7-membered heterocyclic ring, 7-membered carbocyclic ring, benzene ring, or 7-membered heteroaromatic ring. In one embodiment of the compound represented by Formula I, Ring B is selected from the group consisting of 7-membered heterocyclic ring, 7-membered carbocyclic ring, or benzene ring. In one embodiment of the compound represented by Formula I, Ring B is selected from the group consisting of 7-membered heterocyclic ring or 7-membered carbocyclic ring.

In one embodiment of the compound represented by Formula I, Ring B has the following structure: 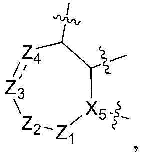 wherein:
-̅-̅-̅ represents a single bond or a double bond;
X₅ is selected from the group consisting of C or N;
Z₁ is CR₆R₇;
Z₂ is selected from the group consisting of CR₈R₉, N, NR₁₀, O, S, -C(O)-, or -SO₂-;
Z₃ is selected from the group consisting of CR₁₁, CR₁₂R₁₃, N, NR₁₄, O, -C(O)-, -SO₂-, or -S(O)(NR₁₅)-;
Z₄ is selected from the group consisting of CR₁₆, CR₁₇R₁₈, N, NR₁₉, O, S, -C(O)-, -SO₂-, or -S(O)(NR₂₀)-;
one or both of Z₁, Z₂, Z₃ and Z₄ may be absent; and the ring comprising X₅, Z₁, Z₂, Z₃, and Z₄ is a stable ring structure;
R₆, R₇, R₈, R₉, R₁₀, R₁₁, R₁₂, R₁₃, R₁₄, R₁₅, R₁₆, R₁₇, R₁₈, R₁₉, and R₂₀ are each independently selected from the group consisting of hydrogen, halogen, hydroxyl, cyano, amino, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl or 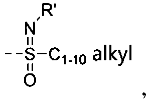 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, and 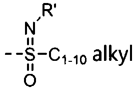 are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONR₂₅R₂₆;
R' is selected from the group consisting of H, C₁₋₁₀ alkyl, C₃₋₁₀ cycloalkyl, or CN;
R₂₁, R₂₂, R₂₃, R₂₄, R₂₅, and R₂₆ are each independently selected from the group consisting of H or C₁₋₁₀ alkyl,
alternatively, R₆ and R₇, R₈ and R₉, R₁₂ and R₁₃, or R₁₇ and R₁₈ are connected together to form a C₃₋₁₀ cycloalkyl or a 3- to 10-membered heterocyclyl, wherein the C₃₋₁₀ cycloalkyl or 3-to 10-membered heterocyclyl is optionally substituted with 1, 2, 3, or 4 substituents independently selected from the group consisting of halogen, hydroxyl, cyano, amino, C₁₋₁₀ alkyl, C₁₋₁₀ heteroalkyl, C₃₋₁₀ cycloalkyl, or 3- to 10-membered heterocyclyl. In one embodiment of the aforementioned Ring B, R₆, R₇, R₈, R₉, R₁₁, R₁₂, R₁₃, R₁₆, R₁₇ and R₁₈ are each independently hydrogen, halogen, hydroxyl, cyano, C₁₋₁₀ alkyl, C₃₋₁₀ cycloalkyl, 3- to 10-membered heterocyclyl, C₁₋₁₀ alkoxy, C₂₋₅ alkenyl, C₂₋₅ alkynyl, C₆₋₁₀ aryl, 5- 10-membered heteroaryl, -COC₁₋₁₀ alkyl, -CONR₂₁R₂₂, -C(O)OC₁₋₁₀ alkyl, -SO₂NR₂₃R₂₄ or -SO₂C₁₋₁₀ alkyl, wherein the C₁₋₁₀ alkyl, C₃₋₁₀ cycloalkyl, 3- to 10-membered heterocyclyl, C₁₋₁₀ alkoxy, C₂₋₅ alkenyl, C₂₋₅ alkynyl, C₆₋₁₀ aryl, 5- to 10-membered heteroaryl, -CO-C₁₋₁₀ alkyl, -CONR₂₁R₂₂, -C(O)OC₁₋₁₀ alkyl, -SO₂NR₂₃R₂₄ and -SO₂C₁₋₁₀ alkyl are each optionally substituted with 1, 2, 3 or 4 substituents independently selected from the group consisting of halogen, hydroxyl, cyano, amino, C₁₋₆ alkyl, halogenated C₁₋₆ alkyl, hydroxy-C₁₋₆ alkyl, C₁₋₆ heteroalkyl, C₃₋₆ cycloalkyl, 3-to 6-membered heterocyclyl, -CONR₂₅R₂₆; or, R₆ and R₇, R₈ and R₉, R₁₂ and R₁₃, or R₁₇ and R₁₈ are connected together to form a C₃₋₁₀ cycloalkyl, wherein the C₃₋₁₀ cycloalkyl is optionally substituted with 1 or 2 substituents independently selected from the group consisting of halogen, hydroxyl, cyano, amino, C₁₋₆ alkyl, C₁₋₆ heteroalkyl, C₃₋₆ cycloalkyl, 3- to 6-membered heterocyclyl; R₁₀, R₁₄, R₁₅, R₁₉ and R₂₀ are each independently hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, C₁₋₁₀ alkyloxy, -CO-C₁₋₁₀ alkyl, -CO-C₃₋₁₀ cycloalkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl or 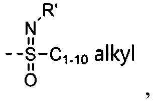 wherein the C₁₋₁₀ alkyl, C₃₋₁₀ cycloalkyl, 3- to 10-membered heterocyclyl, C₂₋₅ alkenyl, C₂₋₅ alkynyl, C₆₋₁₀ aryl, 5- to 10-membered heteroaryl, C₁₋₁₀ alkoxy, -CO-C₁₋₁₀ alkyl, -CO-C₃₋₁₀ cycloalkyl, -CONR₂₁R₂₂, -C(O)OC₁₋₁₀ alkyl, -SO₂NR₂₃R₂₄, -SO₂C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl- 5- to 10-membered heteroaryl and 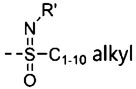 are each optionally substituted with 1, 2, 3, or 4 substituents independently selected from the group consisting of deuterium, halogen, hydroxyl, cyano, amino, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, or -CONR₂₅R₂₆; R' is selected from the group consisting of H, C₁₋₁₀ alkyl, C₃₋₁₀ cycloalkyl, or CN; R₂₁, R₂₂, R₂₃, R₂₄, R₂₅, and R₂₆ are each independently selected from the group consisting of H or C₁₋₆ alkyl.

In one embodiment of the aforementioned Ring B, one of Z₁, Z₂, Z₃, and Z₄ may be absent, for example, Z₃ may be absent, and for another example, Z₄ may be absent, in which case Ring B is a 6-membered ring. In one embodiment of the aforementioned Ring B, two of Z₁, Z₂, Z₃, and Z₄ may be absent, for example, both Z₃ and Z₄ are absent, in which case Ring B is a 5-membered ring.

In one embodiment of the aforementioned Ring B, Z₁ is CR₆R₇; Z₂ is selected from the group consisting of CR₈R₉ or -C(O)-; Z₃ is selected from the group consisting of absence, CR₁₁, CR₁₂R₁₃, N, NR₁₄, O or -C(O)-; Z₄ is selected from the group consisting of absence, CR₁₆, CR₁₇R₁₈, N, NR₁₉ or -C(O)-, wherein R₆, R₇, R₈, R₉, R₁₁, R₁₂, R₁₃, R₁₄, R₁₆, R₁₇, R₁₈ and R₁₉ are as defined above. In one specific embodiment, R₆, R₇, R₈, R₉, R₁₁, R₁₂, R₁₃, R₁₆, R₁₇ and R₁₈ are each independently selected from the group consisting of hydrogen, hydroxyl, C₁₋₁₀ alkyl, C₃₋₁₀ cycloalkyl, 3- to 10-membered heterocyclyl, C₆₋₁₀ aryl or 5- to 10-membered heteroaryl, wherein the C₁₋₁₀ alkyl, C₃₋₁₀ cycloalkyl, 3- to 10-membered heterocyclyl, C₆₋₁₀ aryl and 5- to 10-membered heteroaryl are each optionally substituted with 1 or 2 substituents independently selected from the group consisting of halogen, C₁₋₆ alkyl or halogenated C₁₋₆ alkyl; or R₆ and R₇, R₈ and R₉, R₁₂ and R₁₃, or R₁₇ and R₁₈ are connected together to form a C₃₋₈ cycloalkyl or 3- to 8-membered heterocyclyl, and the C₃₋₈ cycloalkyl or 3- to 8-membered heterocyclyl is optionally substituted with 1 or 2 substituents independently selected from the group consisting of halogen, hydroxyl, cyano, amino, C₁₋₄ alkyl, halogenated C₁₋₄ alkyl, C₁₋₄ heteroalkyl, C₃₋₆ cycloalkyl, 3- to 6-membered heterocyclyl; R₁₄ and R₁₉ are each independently selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂NR₂₃R₂₄, -SO₂-C₁₋₁₀ alkyl, -SO₂-C₂₋₆ alkenyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl or 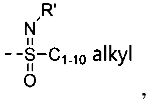 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₂₋₆ alkenyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl, and 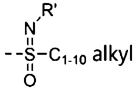 are each optionally substituted with 1, 2 or 3 substituents independently selected from the group consisting of deuterium, halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, -CONR₂₅R₂₆; R' is selected from the group consisting of H, C₁₋₁₀ alkyl, C₃₋₁₀ cycloalkyl or CN; R₂₃, R₂₄, R₂₅ and R₂₆ are each independently selected from the group consisting of hydrogen or C₁₋₆ alkyl.

In one embodiment of the aforementioned Ring B, Z₁ is CR₆R₇; Z₂ is selected from the group consisting of CR₈R₉ or -C(O)-; Z₃ is selected from the group consisting of CR₁₁, CR₁₂R₁₃, N, NR₁₄, O or -C(O)-; Z₄ is selected from the group consisting of absence, CR₁₆, CR₁₇R₁₈, N, NR₁₉ or -C(O)-; wherein R₆, R₇, R₈, R₉, R₁₁, R₁₂, R₁₃, R₁₄, R₁₆, R₁₇, R₁₈ and R₁₉ are as defined above. In a specific embodiment, R₆, R₇, R₈, R₉, R₁₁, R₁₂, R₁₃, R₁₆, R₁₇ and R₁₈ are each independently selected from the group consisting of hydrogen, hydroxyl, C₁₋₆ alkyl, C₃₋₈ cycloalkyl, 3- to 8-membered heterocyclyl, phenyl or 5- to 6-membered heteroaryl, wherein the C₁₋₆ alkyl, C₃₋₈ cycloalkyl, 3- to 8-membered heterocyclyl, phenyl and 5- to 6-membered heteroaryl are each optionally substituted with 1 or 2 substituents independently selected from the group consisting of halogen, C₁₋₆ alkyl or halogenated C₁₋₆ alkyl; R₁₄ and R₁₉ are each independently selected from the group consisting of hydrogen, C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂NR₂₃R₂₄, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl, -C₁₋₄ alkyl-phenyl, -C₁₋₄ alkyl-5- to 6-membered heteroaryl or 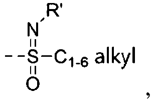 wherein the C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-memebered heterocyclyl, -C₁₋₄ alkyl-phenyl, -C₁₋₄ alkyl-5- to 6-membered heteroaryl, and 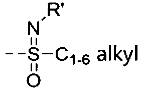 are each optionally substituted with 1, 2, or 3 substituents independently selected from the group consisting of deuterium, halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, -CONR₂₅R₂₆; R' is selected from the group consisting of H, C₁₋₆ alkyl, C₃₋₆ cycloalkyl, or CN; R₂₃, R₂₄, R₂₅, and R₂₆ are each independently selected from the group consisting of hydrogen or C₁₋₆ alkyl.

In one embodiment of the aforementioned Ring B, Z₁ is CR₆R₇; wherein R₆ and R₇ are as defined above. In one embodiment, R₆ and R₇ are each independently selected from the group consisting of hydrogen or C₁₋₁₀ alkyl, such as C₁₋₆ alkyl, such as C₁₋₄ alkyl, such as methyl. In one embodiment of the aforementioned Ring B, Z₁ is CH₂.

In one embodiment of the aforementioned Ring B, Z₂ is selected from the group consisting of -C(O)- or CR₈R₉; wherein R₈ and R₉ are as defined above. In one embodiment, R₈ and R₉ are each independently selected from the group consisting of hydrogen or C₁₋₁₀ alkyl, such as C₁₋₆ alkyl, such as C₁₋₄ alkyl, such as methyl. In one embodiment of the aforementioned Ring B, Z₂ is selected from the group consisting of CR₈R₉; R₈ and R₉ are each independently selected from the group consisting of hydrogen or C₁₋₆ alkyl, such as C₁₋₄ alkyl, such as methyl. In one embodiment of the aforementioned Ring B, Z₂ is CH₂.

In one embodiment of the aforementioned Ring B, Z₃ is selected from the group consisting of CR₁₁, CR₁₂R₁₃, N, NR₁₄, O, or -C(O)-; wherein R₁₁, R₁₂, R₁₃, and R₁₄ are as defined above. In one embodiment, R₁₁, R₁₂ and R₁₃ are each independently selected from the group consisting of hydrogen, hydroxyl, C₁₋₁₀ alkyl, C₃₋₁₀ cycloalkyl, 3- to 10-membered heterocyclyl or 5- to 10-membered heteroaryl, wherein the C₁₋₁₀ alkyl, C₃₋₁₀ cycloalkyl, 3- to 10-membered heterocyclyl and 5- to 10-membered heteroaryl are each optionally substituted with 1 or 2 substituents independently selected from the group consisting of C₁₋₆ alkyl or halogenated C₁₋₆ alkyl; R₁₄ is selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂NR₂₃R₂₄, -SO₂-C₁₋₁₀ alkyl, -SO₂-C₂₋₆ alkenyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl or 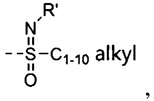 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₃₋₁₀ cycloalkyl, -SO₂-C₂₋₆ alkenyl, -SO₂-3- to 10-membered heterocyclyl, -C₁₋₄ alkyl-C₃₋₁₀ cycloalkyl, -C₁₋₄ alkyl-3- to 10-membered heterocyclyl, and 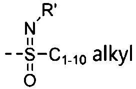 are each optionally substituted with 1, 2, or 3 substituents independently selected from the group consisting of deuterium, halogen, hydroxyl, cyano, C₁₋₆ alkyl, or halogenated C₁₋₆ alkyl; R' is selected from the group consisting of H, C₁₋₁₀ alkyl, C₃₋₁₀ cycloalkyl, or CN; R₂₃ and R₂₄ are each independently selected from the group consisting of hydrogen or C₁₋₆ alkyl.

In one embodiment of the aforementioned Ring B, Z₃ is selected from the group consisting of CR₁₂R₁₃, N, NR₁₄, O, or -C(O)-; wherein R₁₂, R₁₃, and R₁₄ are as defined above. In one embodiment, R₁₂ and R₁₃ are each independently selected from the group consisting of hydrogen, hydroxyl, C₁₋₆ alkyl, C₃₋₇ cycloalkyl, 3- to 7-membered heterocyclyl or 5- to 6-membered heteroaryl, wherein the C₁₋₆ alkyl, C₃₋₇ cycloalkyl, 3- to 7-membered heterocyclyl or 5- to 6-membered heteroaryl are each optionally substituted with 1 or 2 substituents independently selected from the group consisting of halogen, C₁₋₆ alkyl or halogenated C₁₋₆ alkyl; R₁₄ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂NR₂₃R₂₄, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl or 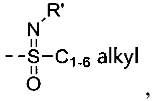 wherein the C₁₋₆ alkyl, C₃₋₈ cycloalkyl, 3-to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂-C₁₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl, and 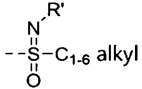 are each optionally substituted with 1, 2, or 3 substituents independently selected from the group consisting of deuterium, halogen, hydroxyl, cyano, C₁₋₆ alkyl, or halogenated C₁₋₆ alkyl; R' is selected from the group consisting of H, C₁₋₆ alkyl, or C₃₋₆ cycloalkyl; R₂₃ and R₂₄ are each independently selected from the group consisting of hydrogen or C₁₋₆ alkyl.

In one embodiment of the aforementioned Ring B, Z₃ is selected from the group consisting of CR₁₂R₁₃, N, NR₁₄, O, or -C(O)-; wherein R₁₂, R₁₃, and R₁₄ are as defined above. In one embodiment, R₁₂ and R₁₃ are each independently selected from the group consisting of hydrogen or C₁₋₆ alkyl, wherein each C₁₋₆ alkyl is optionally substituted with 1 or 2 substituents independently selected from the group consisting of halogen, C₁₋₄ alkyl or halogenated C₁₋₄ alkyl; R₁₄ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂NR₂₃R₂₄, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl or 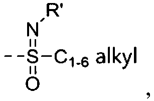 wherein the C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl and 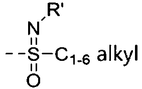 are each optionally substituted with 1, 2 or 3 substituents independently selected from the group consisting of deuterium, halogen, hydroxyl, cyano, C₁₋₆ alkyl or halogenated C₁₋₆ alkyl; R' is selected from the group consisting of H, C₁₋₆ alkyl or C₃₋₆ cycloalkyl; R₂₃ and R₂₄ are each independently selected from the group consisting of hydrogen or C₁₋₆ alkyl.

In one embodiment of the aforementioned ring B, Z₃ is selected from the group consisting of CR₁₂R₁₃, N, NR₁₄, O or -C(O)-; wherein R₁₂ and R₁₃ are each independently selected from the group consisting of hydrogen or C₁₋₄ alkyl, wherein C₁₋₄ alkyl is each optionally substituted with 1 or 2 substituents independently selected from the group consisting of halogen, C₁₋₄ alkyl or halogenated C₁₋₄ alkyl; R₁₄ is selected from the group consisting of C₁₋₆ alkyl, C₃₋₆ cycloalkyl, 3-to 8-membered heterocyclyl, C₂₋₆ alkenyl, C₂₋₆ alkynyl, -CO-C₁₋₆ alkyl, -CO-C₃₋₆ cycloalkyl, -SO₂-C₁₋₆ alkyl, -SO₂-C₂₋₆ alkenyl, -SO₂-C₃₋₆ cycloalkyl, -C₁₋₄ alkyl-C₃₋₆ cycloalkyl, or 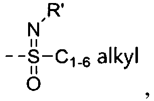 wherein the C₁₋₆ alkyl, C₃₋₆ cycloalkyl, 3- to 8-membered heterocyclyl, C₂₋₆ alkenyl, C₂₋₆ alkynyl, -CO-C₁₋₆ alkyl, -CO-C₃₋₆ cycloalkyl, -SO₂-C₁₋₆ alkyl, -SO₂-C₂₋₆ alkenyl, -SO₂-C₃₋₆ cycloalkyl, -C₁₋₄ alkyl-C₃₋₆ cycloalkyl, or 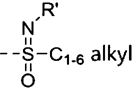 are each optionally substituted with 1, 2, or 3 substituents independently selected from the group consisting of deuterium, halogen, hydroxyl, cyano, C₁₋₄ alkyl, and halogenated C₁₋₄ alkyl; R' is selected from the group consisting of H, C₁₋₆ alkyl, or C₃₋₆ cycloalkyl.

In one embodiment of the aforementioned Ring B, Z₃ is selected from the group consisting of CR₁₂R₁₃, NR₁₄, O, or -C(O)-; wherein R₁₂, R₁₃, and R₁₄ are as defined above. In one embodiment, R₁₂ and R₁₃ are each H; R₁₄ is selected from the group consisting of C₁₋₄ alkyl, C₃₋₆ cycloalkyl, 3- to 6-membered heterocyclyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, or 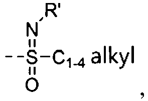 wherein C₁₋₄ alkyl, C₃₋₆ cycloalkyl, 3-to 6-membered heterocyclyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, or 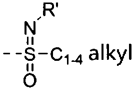 is each optionally substituted with 1, 2, or 3 substituents independently selected from the group consisting of deuterium, halogen, hydroxyl, cyano, C₁₋₄ alkyl, and halogenated C₁₋₄ alkyl; R' is selected from the group consisting of H or C₁₋₄ alkyl.

In one embodiment of the aforementioned B ring, Z₄ is selected from the group consisting of absence, CR₁₆, CR₁₇R₁₈, N, NR₁₉ or -C(O)-; wherein R₁₆, R₁₇, R₁₈ and R₁₉ are as defined above. In one embodiment, R₁₆, R₁₇ and R₁₈ are each independently selected from the group consisting of hydrogen, C₁₋₁₀ alkyl or C₃₋₁₀ cycloalkyl, wherein the C₁₋₁₀ alkyl and C₃₋₁₀ cycloalkyl are each optionally substituted with 1, 2 or 3 substituents independently selected from the group consisting of halogen, C₁₋₆ alkyl or halogenated C₁₋₆ alkyl; or, R₁₇ and R₁₈ are connected together to form a C₃₋₈ cycloalkyl or a 3- to 8-membered heterocyclyl, wherein the C₃₋₈ cycloalkyl or 3- to 8-membered heterocyclyl is optionally substituted with 1 or 2 substituents independently selected from the group consisting of halogen, hydroxyl, cyano, amino, C₁₋₄ alkyl, halogenated C₁₋₄ alkyl, C₁₋₄ heteroalkyl, C₃₋₆ cycloalkyl, 3- to 6-membered heterocyclyl; R₁₉ is selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -SO₂₋C₁₋₁₀ alkyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl or -C₁₋₄ alkyl-5- to 10-membered heteroaryl, wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5-to 10-membered heteroaryl, -SO₂C₁₋₁₀ alkyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, and -C₁₋₄ alkyl-5- to 10-membered heteroaryl are each optionally substituted with 1, 2, or 3 substituents independently selected from the group consisting of halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, or -CONR₂₅R₂₆; R₂₅ and R₂₆ are each independently selected from the group consisting of hydrogen or C₁₋₆ alkyl.

In one embodiment of the aforementioned Ring B, Z₄ is selected from the group consisting of CR₁₆, CR₁₇R₁₈, NR₁₉, or -C(O)-; wherein R₁₆, R₁₇, R₁₈, and R₁₉ are as defined above. In one embodiment, R₁₆, R₁₇ and R₁₈ are each independently selected from the group consisting of hydrogen, C₁₋₆ alkyl or C₃₋₆ cycloalkyl, wherein the C₁₋₆ alkyl and C₃₋₆ cycloalkyl are each optionally substituted with 1, 2 or 3 substituents independently selected from the group consisting of halogen, C₁₋₆ alkyl or halogenated C₁₋₆ alkyl; or, R₁₇ and R₁₈ are connected together to form a C₃₋₆ cycloalkyl or a 3- to 6-membered heterocyclyl, wherein the C₃₋₆ cycloalkyl or 3- to 6-membered heterocyclyl is optionally substituted with 1 or 2 substituents independently selected from the group consisting of halogen, C₁₋₄ alkyl, halogenated C₁₋₄ alkyl, C₁₋₄ heteroalkyl or C₃₋₆ cycloalkyl; R₁₉ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -SO₂-C₁₋₆ alkyl, -C₁₋₄ alkyl-C₃₋₈ cycloalkyl or -C₁₋₄ alkyl-5- to 6-membered heteroaryl, wherein the C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -SO₂-C₁₋₆ alkyl, -C₁₋₄ alkyl-C₃₋₈ cycloalkyl or -C₁₋₄ alkyl-5-to 6-membered heteroaryl are each optionally substituted with 1, 2 or 3 substituents independently selected from the group consisting of halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, -CONR₂₅R₂₆; R₂₅ and R₂₆ are each independently selected from the group consisting of hydrogen or C₁₋₆ alkyl.

In one embodiment of the aforementioned B ring, Z₄ is selected from the group consisting of CR₁₆, CR₁₇R₁₈, NR₁₉ or -C(O)-; R₁₆, R₁₇ and R₁₈ are each independently selected from the group consisting of hydrogen, C₁₋₆ alkyl or C₃₋₆ cycloalkyl, wherein the C₁₋₆ alkyl or C₃₋₆ cycloalkyl are each optionally substituted with 1, 2 or 3 substituents independently selected from the group consisting of halogen, C₁₋₆ alkyl or halogenated C₁₋₆ alkyl; or, R₁₇ and R₁₈ are connected together to form a C₃₋₆ cycloalkyl, wherein the C₃₋₆ cycloalkyl is optionally substituted with 1 or 2 substituents independently selected from the group consisting of halogen, C₁₋₄ alkyl or halogenated C₁₋₄ alkyl; R₁₉ is selected from the group consisting of hydrogen, C₁₋₆ alkyl, C₃₋₈ cycloalkyl alkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl, or -C₁₋₄ alkyl-5- to 6-membered heteroaryl, wherein the C₁₋₆ alkyl, C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl, or -C₁₋₄ alkyl-5- to 6-membered heteroaryl are each optionally substituted with 1, 2, or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl, and C₃₋₆ cycloalkyl.

In one embodiment of the aforementioned Ring B, Z₄ is selected from the group consisting of CR₁₇R₁₅, NR₁₉, or -C(O)-; wherein R₁₇, R₁₈, and R₁₉ are as defined above. In one embodiment, R₁₇ and R₁₈ are each independently selected from the group consisting of hydrogen, C₁₋₄ alkyl, or C₃₋₆ cycloalkyl, wherein the C₁₋₄ alkyl or C₃₋₆ cycloalkyl are each optionally substituted with 1 or 2 substituents independently selected from the group consisting of halogen and C₁₋₄ alkyl; or, R₁₇ and R₁₈ are connected together to form a C₃₋₆ cycloalkyl; R₁₉ is selected from the group consisting of C₁₋₄ alkyl, C₃₋₈ cycloalkyl, C₂₋₆ alkenyl, -SO₂-C₁₋₄ alkyl, or -C₁₋₄ alkyl-C₃₋₆ cycloalkyl, wherein C₁₋₄ alkyl, C₃₋₈ cycloalkyl, C₂₋₆ alkenyl, -SO₂-C₁₋₄ alkyl, or -C₁₋₄ alkyl-C₃₋₆ cycloalkyl are each optionally substituted with 1, 2, or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl, and C₃₋₆ cycloalkyl.

In one embodiment of the aforementioned Ring B:
Z₁ is CR₆R₇;
Z₂ is selected from the group consisting of CR₈R₉ or -C(O)-;
Z₃ is selected from the group consisting of absence, CR₁₁, CR₁₂R₁₃, N, NR₁₄, O, or -C(O)-;
Z₄ is selected from the group consisting of absence, CR₁₆, CR₁₇R₁₈, N, NR₁₉, or -C(O)-;
R₆, R₇, R₈, R₉, R₁₁, R₁₂, R₁₃, R₁₆, R₁₇, and R₁₈ are each independently selected from the group consisting of hydrogen, hydroxyl, C₁₋₁₀ alkyl, C₃₋₁₀ cycloalkyl, 3- to 10-membered heterocyclyl, C₆₋₁₀ aryl, or 5- to 10-membered heteroaryl, wherein C₁₋₁₀ alkyl, C₃₋₁₀ cycloalkyl, 3-to 10-membered heterocyclyl, C₆₋₁₀ aryl, and 5- to 10-membered heteroaryl are each optionally substituted with 1 or 2 substituents independently selected from the group consisting of halogen, C₁₋₆ alkyl, or halogenated C₁₋₆ alkyl;
alternatively, R₆ and R₇, R₈ and R₉, R₁₂ and R₁₃, or R₁₇ and R₁₈ are connected together to form a C₃₋₈ cycloalkyl or 3- to 8-membered heterocyclyl, wherein the C₃₋₈ cycloalkyl or 3- to 8-membered heterocyclyl is optionally substituted with 1 or 2 substituents independently selected from the group consisting of halogen, hydroxyl, cyano, amino, C₁₋₄ alkyl, halogenated C₁₋₄ alkyl, C₁₋₄ heteroalkyl, C₃₋₆ cycloalkyl, 3- to 6-membered heterocyclyl;
R₁₄ and R₁₉ are each independently selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5-to 10-membered heteroaryl, -CO-C₁₋₁₀ alkyl, -CO-C₃₋₁₀ cycloalkyl, -C(O)OC₁₋₁₀ alkyl, -SO₂NR₂₃R₂₄, -SO₂-C₁₋₁₀ alkyl, deuterium-SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl or 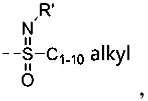 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5-to 10-membered heteroaryl, -CO-C₁₋₁₀ alkyl, -CO-C₃₋₁₀ cycloalkyl, -C(O)OC₁₋₁₀ alkyl, -SO₂₋C₁₋₁₀ alkyl, -SO₂-C₂₋₆ alkenyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl, and 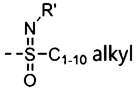 are each optionally substituted with 1, 2, or 3 substituents independently selected from the group consisting of deuterium, halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, or -CONR₂₅R₂₆;
R' is selected from the group consisting of H, C₁₋₁₀ alkyl, C₃₋₁₀ cycloalkyl, or CN;
R₂₃, R₂₄, R₂₅, and R₂₆ are each independently selected from the group consisting of hydrogen or C₁₋₆ alkyl.

In one embodiment of the aforementioned Ring B, Z₁ is CR₆R₇; Z₂ is CR₈R₉ or -C(O)-; Z₃ is CR₁₁, CR₁₂R₁₃, N, NR₁₄, O or -C(O)-; and Z₄ is absent, CR₁₆, CR₁₇R₁₃, N, NR₁₉ or -C(O)-; wherein R₆, R₇, R₈, R₉, R₁₁, R₁₂, R₁₃, R₁₄, R₁₆, R₁₇, R₁₈ and R₁₉ are as defined above.

In one embodiment of the aforementioned Ring B, Z₁ is CR₆R₇; Z₂ is CR₈R₉; Z₃ is CR₁₂R₁₃, N, NR₁₄, O, or -C(O)-; and Z₄ is CR₁₆, CR₁₇R₁₈, NR₁₉, or -C(O)-; wherein R₆, R₇, R₈, R₉, R₁₂, R₁₃, R₁₄, R₁₆, R₁₇, R₁₈, and R₁₉ are as defined above.

In one embodiment of the aforementioned Ring B:
Z₁ is CR₆R₇;
Z₂ is CR₈R₉;
Z₃ is CR₁₂R₁₃, N, NR₁₄, O, or -C(O)-;
Z₄ is CR₁₆, CR₁₇R₁₈, NR₁₉, or -C(O)-;
R₆, R₇, R₈, R₉, R₁₂, and R₁₃ are each independently selected from the group consisting of hydrogen and C₁₋₄ alkyl, wherein each C₁₋₄ alkyl is optionally substituted with 1 or 2 substituents independently selected from the group consisting of halogen, C₁₋₄ alkyl, or halogenated C₁₋₄ alkyl;
R₁₄ is selected from the group consisting of C₁₋₆ alkyl, C₃₋₆ cycloalkyl, 3- to 8-membered heterocyclyl, C₂₋₆ alkenyl, C₂₋₆ alkynyl, -CO-C₁₋₆ alkyl, -CO-C₃₋₆ cycloalkyl, -SO₂-C₁₋₆ alkyl, -SO₂-C₂₋₆ alkenyl, -SO₂-C₃₋₆ cycloalkyl, -C₁₋₄ alkyl-C₃₋₆ cycloalkyl, or 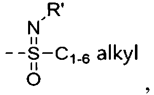 wherein the C₁₋₆ alkyl, C₃₋₆ cycloalkyl, 3- to 8-membered heterocyclyl, C₂₋₆ alkenyl, C₂₋₆ alkynyl, -CO-C₁₋₆ alkyl, -CO-C₃₋₆ cycloalkyl, -SO₂-C₁₋₆ alkyl, -SO₂-C₂₋₆ alkenyl, -SO₂-C₃₋₆ cycloalkyl, -C₁₋₄ alkyl-C₃₋₆ cycloalkyl, or 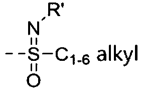 is each optionally substituted with 1, 2 or 3 substituents independently selected from the group consisting of deuterium, halogen, hydroxyl, cyano, C₁₋₄ alkyl and halogenated C₁₋₄ alkyl;
R' is selected from the group consisting of H, C₁₋₆ alkyl, or C₃₋₆ cycloalkyl;
R₁₆, R₁₇, and R₁₈ are each independently selected from the group consisting of hydrogen, C₁₋₆ alkyl, or C₃₋₆ cycloalkyl, wherein the C₁₋₆ alkyl or C₃₋₆ cycloalkyl is each optionally substituted with 1, 2, or 3 substituents independently selected from the group consisting of halogen, C₁₋₆ alkyl, or halogenated C₁₋₆ alkyl;
alternatively, R₁₇ and R₁₈ are connected together to form a C₃₋₆ cycloalkyl, wherein the C₃₋₆ cycloalkyl is optionally substituted with 1 or 2 substituents independently selected from the group consisting of halogen, C₁₋₄ alkyl, or halogenated C₁₋₄ alkyl;
R₁₉ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl, or -C₁₋₄ alkyl-5- to 6-membered heteroaryl, wherein C₁₋₆ alkyl, C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl, or -C₁₋₄ alkyl-5- to 6-membered heteroaryl are each optionally substituted with 1, 2, or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl, and C₃₋₆ cycloalkyl.

In one embodiment of the aforementioned ring B, Z₁ is CR₆R₇; Z₂ is CR₈R₉; Z₃ is CR₁₂R₁₃, NR₁₄, O, or -C(O)-; Z₄ is CR₁₇R₁₈, NR₁₉, or -C(O)-; wherein R₆, R₇, R₈, R₉, R₁₂, R₁₃, R₁₄, R₁₇, R₁₈, and R₁₉ are as defined above.

In one embodiment of the aforementioned Ring B:
Z₁ is CR₆R₇;
Z₂ is CR₈R₉;
Z₃ is CR₁₂R₁₃, NR₁₄, O, or -C(O)-;
Z₄ is CR₁₇R₁₈, NR₁₉, or -C(O)-;
R₆, R₇, R₈, R₉, R₁₂, and R₁₃ are each H;
R₁₄ is selected from the group consisting of the group consisting of C₁₋₄ alkyl, C₃₋₆ cycloalkyl, 3- to 6-membered heterocyclyl, -CO-C₁₋₄ alkyl, -CO-C₃₋₆ cycloalkyl, -SO₂- C₁₋₄ alkyl, -SO₂-C₂₋₄ alkenyl, -SO₂-C₃₋₆ cycloalkyl, or 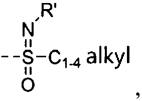 wherein C₁₋₄ alkyl, C₃₋₆ cycloalkyl, 3-to 6-membered heterocyclyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, or 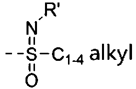 is each optionally substituted with 1, 2 or 3 substituents independently selected from the group consisting of deuterium, halogen, hydroxyl, cyano, C₁₋₄ alkyl and halogenated C₁₋₄ alkyl;
R' is selected from the group consisting of H or C₁₋₄ alkyl;
R₁₇ and R₁₈ are each independently selected from the group consisting of hydrogen, C₁₋₄ alkyl, or C₃₋₆ cycloalkyl, wherein the C₁₋₄ alkyl or C₃₋₆ cycloalkyl is each optionally substituted with 1 or 2 substituents independently selected from the group consisting of halogen and C₁₋₄ alkyl;
alternatively, R₁₇ and R₁₈ are connected together to form a C₃₋₆ cycloalkyl;
R₁₉ is selected from the group consisting of C₁₋₄ alkyl, C₃₋₈ cycloalkyl, C₂₋₆ alkenyl, -SO₂-C₁₋₄ alkyl or -C₁₋₄ alkyl-C₃₋₆ cycloalkyl, wherein the C₁₋₄ alkyl, C₃₋₈ cycloalkyl, C₂₋₆ alkenyl, -SO₂-C₁₋₄ alkyl, or -C₁₋₄ alkyl-C₃₋₆ cycloalkyl is each optionally substituted with 1, 2, or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl, and C₃₋₆ cycloalkyl.

In one embodiment of the compound represented by Formula I, each R_{b} is independently selected from the group consisting of halogen, hydroxyl, cyano, amino, oxo, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, or 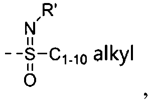 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂₋C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, and 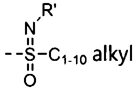 are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONRᵢRⱼ; R' is selected from the group consisting of H, C₁₋₁₀ alkyl, C₃₋₁₀ cycloalkyl, or CN; Rₑ, R_{f}, R_{g}, Rₕ, Rᵢ, and Rⱼ are each independently selected from the group consisting of H or C₁₋₁₀ alkyl.

In one embodiment of the compound represented by Formula I, each R_{b} is independently selected from the group consisting of hydroxyl, oxo, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂NR_{g}Rₕ, -SO₂-C₁₋₁₀ alkyl, -SO₂-C₃₋₁₀ cycloalkyl, -SO₂-C₂₋₆ alkenyl, -SO₂-C₂₋₆ alkynyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-5- to 10-membered heteroaryl or 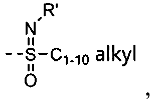 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-5- to 10-membered heteroaryl and 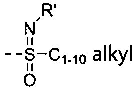 are each optionally substituted with 1, 2 or 3 substituents independently selected from the group consisting of deuterium, halogen, hydroxyl, cyano, C₁₋₁₀ alkyl, halo C₁₋₁₀ alkyl, hydroxy C₁₋₁₀ alkyl, C₁₋₁₀ heteroalkyl, C₃₋₁₀ cycloalkyl, 3- to 10-membered heterocyclyl or -CONRᵢRⱼ; R' is selected from the group consisting of H, C₁₋₁₀ alkyl, C₃₋₁₀ cycloalkyl or CN; R_{g}, Rₕ, Rᵢ and Rⱼ are each independently selected from the group consisting of H or C₁₋₁₀ alkyl.

In one embodiment of the compound represented by Formula I, each R_{b} is independently selected from the group consisting of hydroxyl, oxo, C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂NR_{g}Rₕ, -SO₂-C₁₋₆ alkyl, -SO₂-C₂₋₆ alkenyl, -SO₂-C₂₋₆ alkynyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl, -C₁₋₄ alkyl-5- to 6-membered heteroaryl or 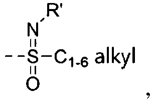 wherein the C₁₋₆ alkyl, C₃₋₈ cycloalkyl, 3- to 8-membered heterocyclyl, C₂₋₆ alkenyl, C₂₋₆ alkynyl, phenyl, 5- to 6-membered heteroaryl, -CO-C₁₋₆ alkyl, -CO-C₃₋₈ cycloalkyl, -C(O)OC₁₋₆ alkyl, -SO₂-C₁₋₆ alkyl, -SO₂-C₂₋₆ alkenyl, -SO₂-C₂₋₆ alkynyl, -SO₂-C₃₋₈ cycloalkyl, -SO₂-3- to 8-membered heterocyclyl, -C₁₋₄ alkyl-C₃₋₈ cycloalkyl, -C₁₋₄ alkyl-3- to 8-membered heterocyclyl, -C₁₋₄ alkyl-5- to 6-membered Heteroaryl and 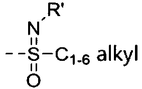 are each optionally substituted with 1, 2 or 3 substituents independently selected from the group consisting of deuterium, halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl or -CONRᵢRⱼ; R' is selected from the group consisting of H, C₁₋₆ alkyl or C₃₋₆ cycloalkyl; R_{g}, Rₕ, Rᵢ and Rⱼ are each independently selected from the group consisting of H or C₁₋₆ alkyl.

In one embodiment of the compound represented by Formula I, each R_{b} is independently selected from the group consisting of oxo, C₁₋₆ alkyl, C₃₋₈ cycloalkyl, 3- to 8-membered heterocyclyl, C₂₋₆ alkenyl, C₂₋₆ alkynyl, -CO-C₁₋₆ alkyl, -CO-C₃₋₈ cycloalkyl, -SO₂-C₁₋₆ alkyl, -SO₂-C₂₋₆ alkenyl, -SO₂-C₂₋₆ alkynyl, -SO₂-C₃₋₈ cycloalkyl, -C₁₋₄ alkyl-C₃₋₈ cycloalkyl, -C₁₋₄ alkyl-3- to 8-membered heterocyclyl, -C₁₋₄ alkyl-5- to 6-membered heteroaryl, or 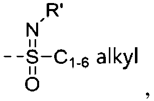 wherein the C₁₋₆ alkyl, C₃₋₈ cycloalkyl, 3- to 8-membered heterocyclyl, C₂₋₆ alkenyl, C₂₋₆ alkynyl, -CO-C₁₋₆ alkyl, -CO-C₃₋₈ cycloalkyl, -SO₂-C₁₋₆ alkyl, -SO₂-C₂₋₆ alkenyl, -SO₂-C₂₋₆ alkynyl, -SO₂-C₃₋₈ cycloalkyl, -C₁₋₄ alkyl-C₃₋₈ cycloalkyl, -C₁₋₄ alkyl-3- to 8-membered heterocyclyl, -C₁₋₄ alkyl-5- to 6-membered heteroaryl, and 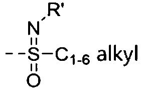 are each optionally substituted with 1, 2 or 3 substituents independently selected from the group consisting of deuterium, halogen, hydroxyl, cyano, C₁₋₆ alkyl, halogenated C₁₋₆ alkyl, hydroxy-C₁₋₆ alkyl, C₁₋₆ heteroalkyl and C₃₋₆ cycloalkyl; R' is selected from the group consisting of H, C₁₋₆ alkyl or C₃₋₆ cycloalkyl.

In one embodiment of the compound represented by Formula I, each R_{b} is independently selected from the group consisting of oxo, C₁₋₄ alkyl, C₃₋₆ cycloalkyl, 3- to 6-membered heterocyclyl, C₂₋₆ alkenyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, -C₁₋₄ alkyl-C₃₋₆ cycloalkyl or 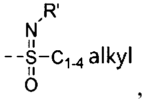 wherein the C₁₋₄ alkyl, C₃₋₆ cycloalkyl, 3- to 6-membered heterocyclyl, C₂₋₆ alkenyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, -C₁₋₄ alkyl-C₃₋₆ cycloalkyl or 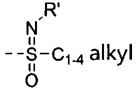 is each optionally substituted with 1, 2, or 3 substituents independently selected from the group consisting of deuterium, halogen, hydroxyl, cyano, C₁₋₄ alkyl, halogenated C₁₋₄ alkyl, hydroxy-C₁₋₄ alkyl, C₁₋₄ heteroalkyl, and C₃₋₆ cycloalkyl; R' is selected from the group consisting of H or C₁₋₄ alkyl.

In one embodiment of the compound represented by Formula I, n is selected from the group consisting of 0, 1, 2, or 3.

In one embodiment, the present invention provides a compound represented by Formula II, its isomer, isotope-labeled compound thereof, or pharmaceutically acceptable salt thereof: 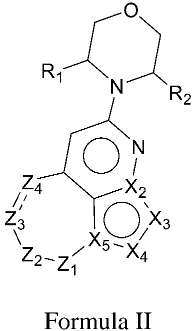 wherein:
○ indicates that the ring to which it belongs is an aromatic ring system;
-̅ -̅ -̅ represents either a single bond or a double bond;
R₁ and R₂ are each independently selected from the group consisting of hydrogen or C₁₋₆ alkyl; or
R₁ and R₂, together with the carbon atom to which they are attached and the adjacent nitrogen atom, form a 4- to 8-membered azacyclic ring;
X₂ is selected from the group consisting of C or N;
X₃ is selected from the group consisting of C(R₃) or N(R₃);
X₄ is selected from the group consisting of N or CH;
X₅ is selected from the group consisting of C or N, and when X₅ is N, X₃ is C(R₃);
R₃ is selected from the group consisting of C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups,
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, and the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently selected from the group consisting of hydrogen or C₁₋₁₀ alkyl;
Z₁ is CR₆R₇;
Z₂ is selected from the group consisting of CR₈R₉, N, NR₁₀, O, S, -C(O)-, or -SO₂-;
Z₃ is selected from the group consisting of CR₁₁, CR₁₂R₁₃, N, NR₁₄, O, -C(O)-, -SO₂-, or -S(O)(NR₁₅)-;
Z₄ is selected from the group consisting of CR₁₆, CR₁₇R₁₈, N, NR₁₉, O, S, -C(O)-, -SO₂-, or -S(O)(NR₂₀)-;
one or two of Z₁, Z₂, Z₃, and Z₄ may be absent; and the ring containing X₅, Z₁, Z₂, Z₃, and Z₄ is a stable ring structure;
R₆, R₇, R₈, R₉, R₁₀, R₁₁, R₁₂, R₁₃, R₁₄, R₁₅, R₁₆, R₁₇, R₁₈, R₁₉, R₂₀ are each independently selected from the group consisting of hydrogen, halogen, hydroxyl, cyano, amino, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂-C₁₋₁₀ alkyl, -SO₂-C₃₋₁₀ cycloalkyl, -SO₂-3- to 10-membered heterocyclyl or 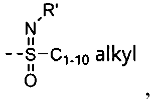 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3-to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl and 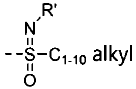 are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, -CONR₂₅R₂₆;
R' is selected from the group consisting of H, C₁₋₁₀ alkyl, C₃₋₁₀ cycloalkyl, or CN;
R₂₁, R₂₂, R₂₃, R₂₄, R₂₅, and R₂₆ are each independently selected from the group consisting of H or C₁₋₁₀ alkyl;
alternatively, R₆ and R₇, R₈ and R₉, R₁₂ and R₁₃, or R₁₇ and R₁₈ are connected together to form a C₃₋₁₀ cycloalkyl or a 3- to 10-membered heterocyclyl, wherein the C₃₋₁₀ cycloalkyl or 3-to 10-membered heterocyclyl is optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, C₁₋₁₀ heteroalkyl, C₃₋₁₀ cycloalkyl, 3- to 10-membered heterocyclyl.

In one embodiment of the compound represented by Formula II, R₁ is hydrogen or C₁₋₆ alkyl. In one embodiment of the compound represented by Formula II, R₁ is hydrogen. In one embodiment of the compound represented by Formula II, R₁ is C₁₋₆ alkyl, such as C₁₋₄ alkyl, such as methyl.

In one embodiment of the compound represented by Formula II, R₂ is hydrogen or C₁₋₆ alkyl. In one embodiment of the compound represented by Formula II, R₂ is hydrogen. In one embodiment of the compound represented by Formula II, R₂ is C₁₋₆ alkyl, such as C₁₋₄ alkyl, such as methyl.

In one embodiment of the compound represented by Formula II, R₁ and R₂, together with the carbon atom to which they are attached and the adjacent nitrogen atom, form a 4- to 8-membered azacyclic ring.

In one embodiment of the compound represented by Formula II, 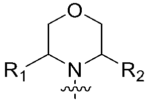 is selected from the group consisting of: 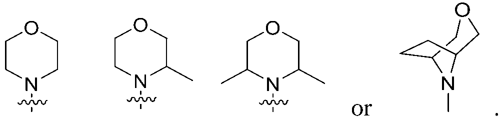 In one embodiment of the compound represented by Formula II, 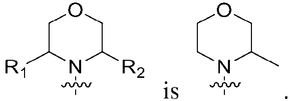 In one embodiment of the compound represented by Formula II, 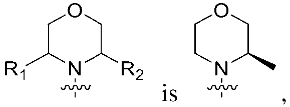 in which case the compound represented by Formula II has the following structural formula:

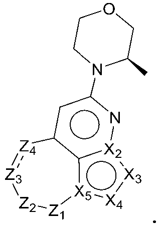

In one embodiment of the compound represented by Formula II, X₂ is C. In one embodiment of the compound represented by Formula II, X₂ is N.

In one embodiment of the compound represented by Formula II, X₃ is selected from the group consisting of C(R₃) or N(R₃), wherein R₃ is selected from the group consisting of C₆₋₁₀ aryl, C₃₋₁₀ cycloalkyl, or 5- 10-membered heteroaryl, wherein the C₆₋₁₀ aryl, C₃₋₁₀ cycloalkyl, and 5-10-membered heteroaryl are each optionally substituted with 1, 2, or 3 R₃ₐ groups, each R₃ₐ is a substituent independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently selected from the group consisting of hydrogen or C₁₋₁₀ alkyl.

In one embodiment of the compound represented by Formula II, X₃ is selected from the group consisting of C(R₃) or N(R₃), wherein R₃ is a 5- 10-membered heteroaryl, the 5-10-membered heteroaryl is optionally substituted with 1, 2, or 3 R₃ₐ groups, each R₃ₐ is independently selected from the group consisting of halogen, cyano, hydroxyl, C₁₋₆ alkyl, halogenated C₁₋₆ alkyl, C₁₋₆ alkoxy, halogenated C₁₋₆ alkoxy, C₃₋₁₀ cycloalkyl, 3- to 10-membered heterocyclyl, or -NR₄R₅, wherein R₄ and R₅ are each independently hydrogen or C₁₋₆ alkyl.

In one embodiment of the compound represented by Formula II, X₃ is selected from the group consisting of C(R₃) or N(R₃), wherein R₃ is a 5- to 6-membered heteroaryl, the 5- to 6-membered heteroaryl is optionally substituted with one or two R₃ₐ groups, each R₃ₐ is independently selected from the group consisting of halogen, cyano, hydroxyl, C₁₋₆ alkyl, halogenated C₁₋₆ alkyl, C₁₋₆ alkoxy, halogenated C₁₋₆ alkoxy, C₃₋₈ cycloalkyl, 3- to 8-membered heterocyclyl, or -NR₄R₅, wherein R₄ and R₅ are each independently hydrogen or C₁₋₆ alkyl. In one embodiment of the compound represented by Formula II, X₃ is selected from the group consisting of C(R₃) or N(R₃), wherein R₃ is a 5- to 6-membered heteroaryl, and the 5- to 6-membered heteroaryl is optionally substituted with 1 or 2 substituents each independently selected from the group consisting of C₁₋₆ alkyl.

In one embodiment of the compound represented by Formula II, X₃ is selected from the group consisting of C(R₃) or N(R₃), wherein R₃ is a 5-membered heteroaryl, the 5-membered heteroaryl is optionally substituted with 1 or 2 substituents independently selected from the group consisting of C₁₋₆ alkyl, such as C₁₋₄ alkyl, such as methyl. In one embodiment of the compound represented by Formula II, X₃ is selected from the group consisting of C(R₃) or N(R₃), wherein R₃ is pyrazolyl, and the pyrazolyl is optionally substituted with one substituent selected from the group consisting of C₁₋₆ alkyl, such as C₁₋₄ alkyl, such as methyl. In one embodiment of the compound represented by Formula II, X₃ is selected from the group consisting of C(R₃) or N(R₃), wherein R₃ is pyrazolyl.

In one embodiment of the compound represented by Formula II, X₄ is N. In one embodiment of the compound represented by Formula II, X₄ is CH.

In one embodiment of the compound represented by Formula II, X₅ is C. In one embodiment of the compound represented by Formula II, X₅ is N.

In one embodiment of the compound represented by Formula II, when X₅ is N, X₃ is C(R₃), wherein R₃ is as defined above.

In one embodiment of the compound represented by Formula II, Z₁, Z₂, Z₃, and Z₄, and combinations thereof, are as defined above for Z₁, Z₂, Z₃, and Z₄ in Ring B of Formula I, and combinations thereof.

In one embodiment, the present invention provides a compound represented by Formula IIA, IIB, or IIC, its isomer, isotope-labeled compound thereof, or pharmaceutically acceptable salt thereof: 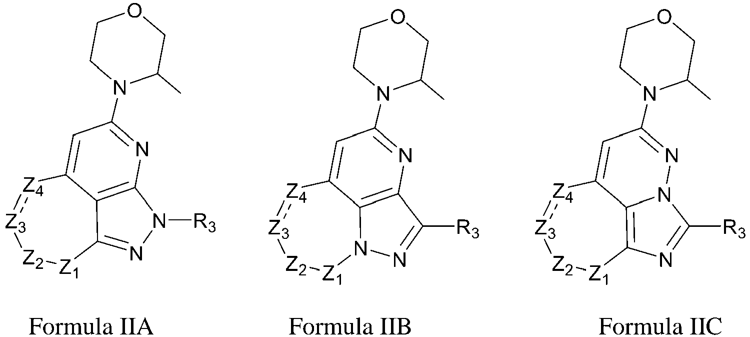 wherein:
R₃ is C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups;
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently hydrogen or C₁₋₁₀ alkyl;
Z₁ is CR₆R₇;
Z₂ is selected from the group consisting of CR₈R₉, N, NR₁₀, O, S, -C(O)-, or -SO₂-;
Z₃ is selected from the group consisting of CR₁₁, CR₁₂R₁₃, N, NR₁₄, O, -C(O)-, -SO₂-, or -S(O)(NR₁₅)-;
Z₄ is selected from the group consisting of CR₁₆, CR₁₇R₁₈, N, NR₁₉, O, S, -C(O)-, -SO₂-, or -S(O)(NR₂₀)-;
one or two of Z₁, Z₂, Z₃, and Z₄ may be absent; and the ring containing X₅, Z₁, Z₂, Z₃, and Z₄ is a stable ring structure;
R₆, R₇, R₈, R₉, R₁₀, R₁₁, R₁₂, R₁₃, R₁₄, R₁₅, R₁₆, R₁₇, R₁₈, R₁₉, R₂₀ are each independently selected from the group consisting of hydrogen, halogen, hydroxyl, cyano, amino, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl or 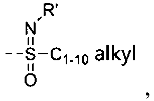 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, and 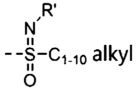 are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONR₂₅R₂₆;
R' is selected from the group consisting of H, C₁₋₁₀ alkyl, C₃₋₁₀ cycloalkyl, or CN;
R₂₁, R₂₂, R₂₃, R₂₄, R₂₅, and R₂₆ is each independently selected from the group consisting of H or C₁₋₁₀ alkyl.
alternatively, R₆ and R₇, R₈ and R₉, R₁₂ and R₁₃, or R₁₇ and R₁₈ are connected together to form a C₃₋₁₀ cycloalkyl or a 3- to 10-membered heterocyclyl, wherein the C₃₋₁₀ cycloalkyl or 3-to 10-membered heterocyclyl is optionally substituted with 1, 2, 3, or 4 substituents independently selected from the group consisting of halogen, hydroxyl, cyano, amino, C₁₋₁₀ alkyl, halogenated C₁₋₁₀ alkyl, C₁₋₁₀ heteroalkyl, C₃₋₁₀ cycloalkyl, or a 3- to 10-membered heterocyclyl.

In one embodiment, the compound represented by Formula IIA, IIB, or IIC has the structure represented by the following formula:

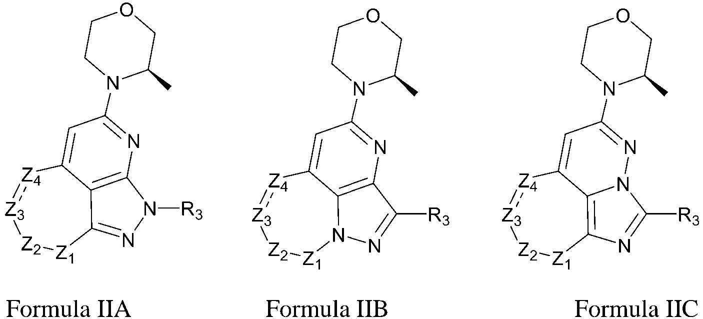

In one embodiment of the compound represented by Formula IIA, IIB, or IIC, R₃ is a 5-10-membered heteroaryl, the 5- 10-membered heteroaryl is optionally substituted with one, two, or three R₃ₐ groups, each R₃ₐ is independently selected from the group consisting of halogen, cyano, hydroxyl, C₁₋₆ alkyl, C₁₋₆ heteroalkyl, C₃₋₈ cycloalkyl, 3- to 8-membered heterocyclyl, or -NR₄R₅, wherein the C₁₋₆ alkyl, C₁₋₆ heteroalkyl, C₃₋₈ cycloalkyl, 3- to 8-membered heterocyclyl, or -NR₄R₅ is each optionally substituted with one, two, or three substituents independently selected from the group consisting of halogen, cyano, hydroxyl, or C₁₋₆ alkyl; and R₄ and R₅ are each independently hydrogen or C₁₋₆ alkyl. In one embodiment of the compound represented by Formula IIA, IIB or IIC, R₃ is a 5- to 6-membered heteroaryl, the 5- to 6-membered heteroaryl is optionally substituted with one or two R₃ₐ, each R₃ₐ is independently selected from the group consisting of halogen, cyano, hydroxyl, C₁₋₆ alkyl, halogenated C₁₋₆ alkyl, C₁₋₆ alkoxy, C₃₋₈ cycloalkyl, 3- to 8-membered heterocyclyl, or -NR₄R₅, wherein R₄ and R₅ are each independently hydrogen or C₁₋₆ alkyl. In one embodiment of the compound represented by Formula IIA, IIB or IIC, R₃ is a 5- to 6-membered heteroaryl, the 5- 10-membered heteroaryl is optionally substituted with one or two R₃ₐ, each R₃ₐ is independently selected from the group consisting of C₁₋₆ alkyl, such as C₁₋₄ alkyl, such as methyl. In one embodiment of the compound represented by formula IIA, IIB or IIC, R₃ is a 5-membered heteroaryl, the 5-membered heteroaryl is optionally substituted with 1 or 2 R₃ₐ groups, each R₃ₐ is independently selected from the group consisting of C₁₋₆ alkyl, such as C₁₋₄ alkyl, such as methyl. In one embodiment of the compound represented by formula IIA, IIB or IIC, R₃ is pyrazolyl, the pyrazolyl is optionally substituted with 1 substituent selected from the group consisting of C₁₋₆ alkyl, such as C₁₋₄ alkyl, such as methyl. In one embodiment of the compound represented by Formula IA, IIB, or IIC, R₃ is pyrazolyl.

In one embodiment of the compound represented by Formula IIA, IIB, or IIC, Z₁, Z₂, Z₃, and Z₄, and combinations thereof, are as defined above for Ring B of Formula I, or Z₁, Z₂, Z₃, and Z₄ and combinations thereof in Formula II.

In one embodiment of the compound represented by Formula IIA, IIB, or IIC:
R₃ is a 5- to 6-membered heteroaryl, the 5- to 6-membered heteroaryl is optionally substituted with one or two R₃ₐ groups, each R₃ₐ is independently selected from the group consisting of C₁₋₆ alkyl;
Z₁ is CR₆R₇;
Z₂ is CR₈R₉;
Z₃ is selected from the group consisting of absence, CR₁₂R₁₃, N, NR₁₄, O, or -C(O)-;
Z₄ is selected from the group consisting of absence, CR₁₆, CR₁₇R₁₈, NR₁₉, or -C(O)-;
wherein R₆, R₇, R₈, R₉, R₁₂, R₁₃, R₁₄, R₁₆, R₁₇, R₁₈, and R₁₉ are each as defined above. In a specific embodiment, R₆, R₇, R₈, R₉, R₁₂ and R₁₃ are independently selected from the group consisting of hydrogen and C₁₋₄ alkyl, wherein each C₁₋₄ alkyl is optionally substituted with 1 or 2 substituents independently selected from the group consisting of halogen, C₁₋₄ alkyl or halogenated C₁₋₄ alkyl; R₁₄ is selected from the group consisting of C₁₋₆ alkyl, C₃₋₆ cycloalkyl, 3-to 8-membered heterocyclyl, C₂₋₆ alkenyl, C₂₋₆ alkynyl, -CO-C₁₋₆ alkyl, -CO-C₃₋₆ cycloalkyl, -SO₂-C₁₋₆ alkyl, -SO₂-C₂₋₆ alkenyl, -SO₂-C₃₋₆ cycloalkyl, -C₁₋₄ alkyl-C₃₋₆ cycloalkyl, or 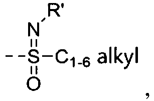 wherein the C₁₋₆ alkyl, C₃₋₆ cycloalkyl, 3- to 8-membered heterocyclyl, C₂₋₆ alkenyl, C₂₋₆ alkynyl, -CO-C₁₋₆ alkyl, -CO-C₃₋₆ cycloalkyl, -SO₂-C₁₋₆ alkyl, -SO₂-C₂₋₆ alkenyl, -SO₂-C₃₋₆ cycloalkyl, -C₁₋₄ alkyl-C₃₋₆ cycloalkyl, or 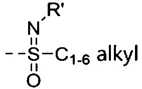 is each optionally substituted with 1, 2 or 3 substituents independently selected from the group consisting of deuterium, halogen, hydroxyl, cyano, C₁₋₄ alkyl and halogenated C₁₋₄ alkyl; R' is selected from the group consisting of H, C₁₋₆ alkyl or C₃₋₆ cycloalkyl; R₁₆, R₁₇ and R₁₈ are each independently selected from the group consisting of hydrogen, C₁₋₆ alkyl or C₃₋₆ cycloalkyl, wherein the C₁₋₆ alkyl or C₃₋₆ cycloalkyl is each optionally substituted with 1, 2 or 3 substituents independently selected from the group consisting of halogen, C₁₋₆ alkyl or halogenated C₁₋₆ alkyl; or, R₁₇ and R₁₈ are connected together to form a C₃₋₆ cycloalkyl, wherein the C₃₋₆ cycloalkyl is optionally substituted with 1 or 2 substituents independently selected from the group consisting of halogen, C₁₋₄ alkyl or halogenated C₁₋₄ alkyl; R₁₉ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl or -C₁₋₄ alkyl-5- to 6-membered heteroaryl, wherein the C₁₋₆ alkyl, C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl or -C₁₋₄ alkyl-5- to 6-membered heteroaryl is each optionally substituted with 1, 2 or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl and C₃₋₆ cycloalkyl.

In one embodiment of the compound represented by Formula IIA, IIB, or IIC:
R₃ is a 5-membered heteroaryl, the 5-membered heteroaryl is optionally substituted with one R₃ₐ, wherein R₃ₐ is selected from the group consisting of C₁₋₄ alkyl;
Z₁ is CR₆R₇;
Z₂ is CR₈R₉;
Z₃ is selected from the group consisting of CR₁₂R₁₃, NR₁₄, O, or -C(O)-;
Z₄ is selected from the group consisting of CR₁₇R₁₈, NR₁₉, or -C(O)-;
R₆, R₇, R₈, R₉, R₁₂, and R₁₃ are each H;
R₁₄ is selected from the group consisting of C₁₋₄ alkyl, C₃₋₆ cycloalkyl, 3- to 6-membered heterocyclyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, or 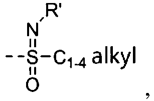 wherein the C₁₋₄ alkyl, C₃₋₆ cycloalkyl, 3- to 6-membered heterocyclyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl or 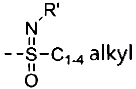 is each optionally substituted with 1, 2 or 3 substituents independently selected from the group consisting of deuterium, halogen, hydroxyl, cyano, C₁₋₄ alkyl and halogenated C₁₋₄ alkyl;
R' is selected from the group consisting of H or C₁₋₄ alkyl;
R₁₇ and R₁₈ are each independently selected from the group consisting of hydrogen, C₁₋₄ alkyl, or C₃₋₆ cycloalkyl, wherein the C₁₋₄ alkyl or C₃₋₆ cycloalkyl is each optionally substituted with 1 or 2 substituents independently selected from the group consisting of halogen and C₁₋₄ alkyl;
alternatively, R₁₇ and R₁₈ are connected together to form a C₃₋₆ cycloalkyl;
R₁₉ is selected from the group consisting of C₁₋₄ alkyl, C₃₋₈ cycloalkyl, C₂₋₆ alkenyl, -SO₂-C₁₋₄ alkyl or -C₁₋₄ alkyl-C₃₋₆ cycloalkyl, wherein the C₁₋₄ alkyl, C₃₋₈ cycloalkyl, C₂₋₆ alkenyl, -SO₂-C₁₋₄ alkyl or -C₁₋₄ alkyl-C₃₋₆ cycloalkyl is each optionally substituted with 1, 2 or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl and C₃₋₆ cycloalkyl.

In one embodiment of the compound represented by Formula IIA:
R₃ is a 5- to 6-membered heteroaryl, the 5- to 6-membered heteroaryl is optionally substituted with one or two R₃ₐ groups, each R₃ₐ is independently selected from the group consisting of C₁₋₆ alkyl;
Z₁ is CR₆R₇;
Z₂ is CR₈R₉;
Z₃ is NR₁₄, O, or -C(O)-;
Z₄ is CR₁₇R₁₈, NR₁₉, or -C(O)-;
R₆, R₇, R₈, and R₉ are each independently selected from the group consisting of hydrogen and C₁₋₄ alkyl, wherein each C₁₋₄ alkyl is optionally substituted with 1 or 2 substituents independently selected from the group consisting of halogen, C₁₋₄ alkyl or halogenated C₁₋₄ alkyl;
R₁₄ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, C₆₋₁₀ aryl, 5- to 10-membered heteroaryl, -CO-C₁₋₆ alkyl, -CO-C₃₋₈ cycloalkyl, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₆ alkyl-3- to 8-membered heterocyclyl or 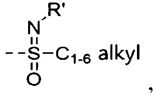 wherein the C₁₋₆ alkyl, C₃₋₈ cycloalkyl, 3- to 8-membered heterocyclyl, C₂₋₆ alkenyl, C₂₋₆ alkynyl, C₆₋₁₀ aryl, 5- to 10-membered heteroaryl, -CO-C₁₋₆ alkyl, -CO-C₃₋₈ cycloalkyl, -SO₂-C₁₋₆ alkyl, -SO₂-C₂₋₆ alkenyl, -SO₂-C₃₋₈ cycloalkyl, -SO₂-3- to 8-membered heterocyclyl, -C₁₋₄ alkyl-C₃₋₈ cycloalkyl, -C₁₋₆ alkyl-3- to 8-membered heterocyclyl, or 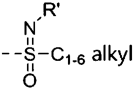 is each optionally substituted with 1, 2 or 3 substituents independently selected from the group consisting of deuterium, halogen, hydroxyl, cyano, C₁₋₄ alkyl and halogenated C₁₋₄ alkyl;
R' is selected from the group consisting of H, C₁₋₆ alkyl, or C₃₋₆ cycloalkyl;
R₁₇ and R₁₈ are each independently selected from the group consisting of hydrogen, C₁₋₆ alkyl, or C₃₋₆ cycloalkyl, wherein the C₁₋₆ alkyl or C₃₋₆ cycloalkyl is each optionally substituted with 1, 2, or 3 substituents independently selected from the group consisting of halogen, C₁₋₄ alkyl, or halogenated C₁₋₄ alkyl;
alternatively, R₁₇ and R₁₈ are connected together to form a C₃₋₆ cycloalkyl, wherein the C₃₋₆ cycloalkyl is optionally substituted with 1 or 2 substituents independently selected from the group consisting of halogen, C₁₋₄ alkyl, or halogenated C₁₋₄ alkyl;
R₁₉ is selected from the group consisting of hydrogen, C₁₋₆ alkyl, C₃₋₈ cycloalkyl, or -C₁₋₄ alkyl-C₃₋₈ cycloalkyl, wherein the C₁₋₆ alkyl, C₃₋₈ cycloalkyl, or -C₁₋₄ alkyl-C₃₋₈ cycloalkyl is each optionally substituted with 1, 2, or 3 substituents independently selected from the group consisting of halogen, C₁₋₄ alkyl, and halogenated C₁₋₄ alkyl.

In one embodiment of the compound represented by Formula IIA:
R₃ is a 5-membered heteroaryl, the 5-membered heteroaryl is optionally substituted with one C₁₋₄ alkyl;
Z₁ is CR₆R₇;
Z₂ is CR₈R₉;
Z₃ is NR₁₄, O, or -C(O)-;
Z₄ is CR₁₇R₁₈, NR₁₉, or -C(O)-;
R₆, R₇, R₈, and R₉ are each independently hydrogen;
R₁₄ is selected from the group consisting of the group consisting of C₁₋₄ alkyl, C₃₋₆ cycloalkyl, 3- to 6-membered heterocyclyl, C₂₋₆ alkynyl, -CO-C₁₋₄ alkyl, -CO-C₃₋₆ cycloalkyl, -SO₂-C₁₋₄ alkyl, -SO₂-C₂₋₆ alkenyl, -SO₂-C₃₋₆ cycloalkyl, -SO₂-3- to 6-membered heterocyclyl, -C₁₋₄ alkyl-3- to 6-membered heterocyclyl, or 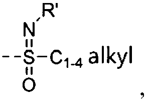 wherein the C₁₋₄ alkyl, C₃₋₆ cycloalkyl, 3- to 6-membered heterocyclyl, C₂₋₆ alkynyl, -CO-C₁₋₄ alkyl, -CO-C₃₋₆ cycloalkyl, -SO₂-C₁₋₄ alkyl, -SO₂-C₂₋₆ alkenyl, -SO₂-C₃₋₆ cycloalkyl, -SO₂-3- to 6-membered heterocyclyl, -C₁₋₄ alkyl-3- to 6-membered heterocyclyl or 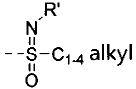 is each optionally substituted with 1, 2 or 3 substituents independently selected from the group consisting of deuterium, halogen, hydroxyl, cyano, C₁₋₄ alkyl and halogenated C₁₋₄ alkyl;
R' is selected from the group consisting of H, C₁₋₄ alkyl, or C₃₋₆ cycloalkyl;
R₁₇ and R₁₈ are each independently selected from the group consisting of hydrogen, C₁₋₄ alkyl, or C₃₋₆ cycloalkyl, wherein the C₁₋₄ alkyl or C₃₋₆ cycloalkyl is each optionally substituted with 1, 2, or 3 substituents independently selected from the group consisting of halogen, C₁₋₄ alkyl, or halogenated C₁₋₄ alkyl;
alternatively, R₁₇ and R₁₈ are connected together to form a C₃₋₆ cycloalkyl, wherein the C₃₋₆ cycloalkyl is optionally substituted with 1 or 2 substituents independently selected from the group consisting of halogen, C₁₋₄ alkyl, or halogenated C₁₋₄ alkyl;
R₁₉ is selected from the group consisting of hydrogen, C₁₋₄ alkyl, C₃₋₆ cycloalkyl, or -C₁₋₄ alkyl-C₃₋₆ cycloalkyl, wherein the C₁₋₄ alkyl, C₃₋₆ cycloalkyl, or -C₁₋₄ alkyl-C₃₋₆ cycloalkyl is each optionally substituted with 1, 2, or 3 substituents independently selected from the group consisting of halogen, C₁₋₄ alkyl, and halogenated C₁₋₄ alkyl.

In one embodiment of the compound represented by Formula IIA:
R₃ is a 5-membered heteroaryl;
Z₁ is CR₆R₇;
Z₂ is CR₈R₉;
Z₃ is NR₁₄, O, or -C(O)-;
Z₄ is CR₁₇R₁₈, NR₁₉, or -C(O)-;
R₆, R₇, R₈, and R₉ are each independently hydrogen;
R₁₄ is selected from the group consisting of the group consisting of C₁₋₄ alkyl, C₃₋₆ cycloalkyl, 3- to 6-membered heterocyclyl, -CO-C₁₋₄ alkyl, -CO-C₃₋₆ cycloalkyl, -SO₂ -C₁₋₄ alkyl, -SO₂-C₂₋₄ alkenyl, -SO₂-C₃₋₆ cycloalkyl, or 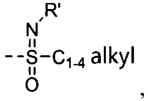 wherein the C₁₋₄ alkyl, C₃₋₆ cycloalkyl, 3- to 6-membered heterocyclyl, -CO-C₁₋₄ alkyl, -CO-C₃₋₆ cycloalkyl, -SO₂-C₁₋₄ alkyl, -SO₂-C₂₋₄ alkenyl, -SO₂-C₃₋₆ cycloalkyl, or 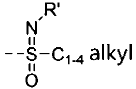 is each optionally substituted with 1, 2 or 3 substituents independently selected from the group consisting of deuterium, halogen, hydroxyl, cyano, C₁₋₄ alkyl and halogenated C₁₋₄ alkyl;
R' is selected from the group consisting of H or C₁₋₄ alkyl;
R₁₇ and R₁₈ are each independently selected from the group consisting of hydrogen, C₁₋₄ alkyl, or C₃₋₆ cycloalkyl, wherein the C₁₋₄ alkyl or C₃₋₆ cycloalkyl is each optionally substituted with 1 or 2 substituents independently selected from the group consisting of halogen or C₁₋₄ alkyl;
alternatively, R₁₇ and R₁₈ are connected together to form a C₃₋₆ cycloalkyl;
R₁₉ is selected from the group consisting of C₁₋₄ alkyl, C₃₋₆ cycloalkyl, or -C₁₋₄ alkyl-C₃₋₆ cycloalkyl, wherein the C₁₋₄ alkyl, C₃₋₆ cycloalkyl, or -C₁₋₄ alkyl-C₃₋₆ cycloalkyl is each optionally substituted with 1, 2, or 3 substituents independently selected from the group consisting of halogen, C₁₋₄ alkyl, and halogenated C₁₋₄ alkyl.

In one embodiment of the compound represented by Formula IIB:
R₃ is a 5- to 6-membered heteroaryl, which is optionally substituted with one or two R₃ₐ groups, each R₃ₐ is independently selected from the group consisting of C₁₋₆ alkyl;
Z₁ is CR₆R₇;
Z₂ is CR₈R₉ or -C(O)-;
Z₃ is CR₁₂R₁₃, N, NR₁₄ or -C(O)-;
Z₄ is CR₁₆, CR₁₇R₁₈ or NR₁₉;
R₆, R₇, R₈ and R₉ are each independently selected from the group consisting of hydrogen and C₁₋₄ alkyl, wherein each C₁₋₄ alkyl is optionally substituted with 1 or 2 substituents independently selected from the group consisting of halogen, C₁₋₄ alkyl or halogenated C₁₋₄ alkyl;
R₁₂ and R₁₃ are each independently selected from the group consisting of hydrogen, hydroxyl, C₁₋₆ alkyl, C₃₋₆ cycloalkyl, 3- to 6-membered heterocyclyl, C₆₋₁₀ aryl or 5- or 6-membered heteroaryl, wherein the C₁₋₆ alkyl, C₃₋₆ cycloalkyl, 3- to 6-membered heterocyclyl, C₆₋₁₀ aryl, or 5- or 6-membered heteroaryl is each optionally substituted with 1 or 2 substituents independently selected from the group consisting of halogen, C₁₋₄ alkyl, or halogenated C₁₋₄ alkyl;
R₁₄ is selected from the group consisting of hydrogen, C₁₋₆ alkyl, C₃₋₆ cycloalkyl, 3- to 6-membered heterocyclyl, C₆₋₁₀ aryl, or 5- or 6-membered heteroaryl, -CO-C₁₋₆ alkyl, -CO-C₃₋₆ cycloalkyl, -C(O)OC₁₋₆ alkyl or -SO₂-C₁₋₆ alkyl, wherein the C₁₋₆ alkyl, C₃₋₆ cycloalkyl, 3- to 6-membered heterocyclyl, C₆₋₁₀ aryl, or 5- or 6-membered heteroaryl, -CO-C₁₋₆ alkyl, -CO-C₃₋₆ cycloalkyl, -C(O)OC₁₋₆ alkyl or -SO₂-C₁₋₆ alkyl is each optionally substituted with 1, 2 or 3 substituents independently selected from the group consisting of halogen, C₁₋₄ alkyl and halogenated C₁₋₄ alkyl;
R₁₆ is selected from the group consisting of C₁₋₆ alkyl;
R₁₇ and R₁₈ are each independently selected from the group consisting of hydrogen or C₁₋₆ alkyl, wherein each C₁₋₆ alkyl is optionally substituted with 1, 2 or 3 substituents independently selected from the group consisting of halogen, C₁₋₄ alkyl or halogenated C₁₋₄ alkyl;
R₁₉ is selected from the group consisting of hydrogen, C₁₋₆ alkyl, C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl, or -C₁₋₄ alkyl-5- to 6-membered heteroaryl, wherein the C₁₋₆ alkyl, -C₃₋₈ cycloalkyl, 3- to 8-membered heterocyclyl, C₂₋₆ alkenyl, C₂₋₆ alkynyl, -SO₂-C₁₋₆ alkyl, -C₁₋₄ alkyl-C₃₋₈ cycloalkyl, or -C₁₋₄ alkyl-5- to 6-membered heteroaryl is each optionally substituted with 1, 2, or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl, C₃₋₆ cycloalkyl, and -CONR₂₅R₂₆;
R₂₅ and R₂₆ are each independently selected from the group consisting of hydrogen or C₁₋₄ alkyl.

In one embodiment of the compound represented by Formula IIB:
R₃ is a 5-membered heteroaryl, the 5-membered heteroaryl is optionally substituted with one C₁₋₄ alkyl;
Z₁ is CR₆R₇;
Z₂ is CR₈R₉;
Z₃ is CR₁₂R₁₃, N, NR₁₄, or -C(O)-;
Z₄ is CR₁₆, CR₁₇R₁₈, or NR₁₉;
R₆ and R₇ are each independently selected from the group consisting of hydrogen;
R₈ and R₉ are each independently selected from the group consisting of hydrogen or C₁₋₄ alkyl, wherein each C₁₋₄ alkyl is optionally substituted with 1 or 2 substituents independently selected from the group consisting of halogen, C₁₋₄ alkyl, or halogenated C₁₋₄ alkyl;
R₁₂ and R₁₃ are each independently selected from the group consisting of hydrogen or C₁₋₄ alkyl, wherein each C₁₋₄ alkyl is optionally substituted with 1 or 2 substituents independently selected from the group consisting of halogen,, C₁₋₄ alkyl, or halogenated C₁₋₄ alkyl;
R₁₄ is selected from the group consisting of C₁₋₄ alkyl, C₃₋₆ cycloalkyl, 3- to 6-membered heterocyclyl, -CO-C₁₋₄ alkyl, -CO-C₃₋₆ cycloalkyl, or -SO₂-C₁₋₄ alkyl, wherein the C₁₋₄ alkyl, C₃₋₆ cycloalkyl, 3- to 6-membered heterocyclyl, -CO-C₁₋₄ alkyl, -CO-C₃₋₆ cycloalkyl, and -SO₂-C₁₋₄ alkyl are each optionally substituted with 1, 2, or 3 substituents independently selected from the group consisting of halogen, C₁₋₄ alkyl, and a halogenated C₁₋₄ alkyl;
R₁₆ is selected from the group consisting of C₁₋₄ alkyl;
R₁₇ and R₁₈ are each independently selected from the group consisting of hydrogen or C₁₋₄ alkyl, wherein each C₁₋₄ alkyl is optionally substituted with 1, 2 or 3 substituents independently selected from the group consisting of halogen, C₁₋₄ alkyl or halogenated C₁₋₄ alkyl;
R₁₉ is selected from the group consisting of hydrogen, C₁₋₄ alkyl, C₃₋₆ cycloalkyl, 3- to 6-membered heterocyclyl, C₂₋₆ alkenyl, C₂₋₆ alkynyl, -SO₂-C₁₋₄ alkyl, -C₁₋₄ alkyl-C₃₋₆ cycloalkyl, or -C₁₋₄ alkyl-5- to 6-membered heteroaryl, wherein the C₁₋₄ alkyl, C₃₋₆ cycloalkyl, 3- to 6-membered heterocyclyl, C₂₋₆ alkenyl, C₂₋₆ alkynyl, -SO₂-C₁₋₄ alkyl, -C₁₋₄ alkyl-C₃₋₆ cycloalkyl or -C₁₋₄ alkyl-5- to 6-membered heteroaryl is each optionally substituted with 1, 2 or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl and C₃₋₆ cycloalkyl.

In one embodiment of the compound represented by Formula IIB:
R₃ is a 5-membered heteroaryl, the 5-membered heteroaryl is optionally substituted with one C₁₋₄ alkyl;
Z₁ is CR₆R₇;
Z₂ is CR₈R₉;
Z₃ is CR₁₂R₁₃ or -C(O)-;
Z₄ is CR₁₇R₁₈ or NR₁₉;
R₆, R₇, R₈, R₉, R₁₂, R₁₃, R₁₇, and R₁₈ are each independently hydrogen;
R₁₉ is selected from the group consisting of C₁₋₄ alkyl, C₃₋₆ cycloalkyl, C₂₋₆ alkenyl, -SO₂-C₁₋₄ alkyl, or -C₁₋₄ alkyl-C₃₋₆ cycloalkyl, wherein the C₁₋₄ alkyl, C₃₋₆ cycloalkyl, C₂₋₆ alkenyl, -SO₂-C₁₋₄ alkyl, or -C₁₋₄ alkyl-C₃₋₆ cycloalkyl is each optionally substituted with 1, 2, or 3 substituents independently selected from the group consisting of halogen, hydroxyl, cyano, C₁₋₄ alkyl, halogenated C₁₋₄ alkyl, hydroxy-C₁₋₄ alkyl, C₁₋₄ heteroalkyl, and C₃₋₆ cycloalkyl.

### Other Embodiments

Embodiment 1: A compound represented by Formula I, its isomer, isotope-labeled compound, or pharmaceutically acceptable salt thereof: 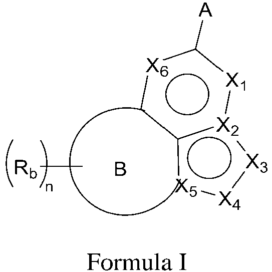 wherein:
○ indicates that the ring to which it belongs is an aromatic ring system;
X₁ is selected from the group consisting of N or C(R_{X1});
X₂ is selected from the group consisting of C or N;
X₃ is selected from the group consisting of N, C(R_{X2}), or N(R_{X3});
X₄ is selected from the group consisting of N or C(R_{X4});
X₅ is selected from the group consisting of C or N;
X₆ is selected from the group consisting of N, N(R_{X5}), or C(R_{X6});
R_{X1}, R_{X2}, R_{X3}, R_{X4}, R_{X5}, and R_{X6} are each independently selected from the group consisting of H, halogen, C₆₋₁₀ aryl, C₃₋₁₀ cycloalkyl, C₃₋₁₀ cycloalkenyl, 3- to 10-membered heterocyclyl, or 5- to 10-membered heteroaryl, wherein the C₆₋₁₀ aryl, C₃₋₁₀ cycloalkyl, C₃₋₁₀ cycloalkenyl, 3- to 10-membered heterocyclyl, and 5- to 10-membered heteroaryl are each optionally substituted with 1, 2, or 3 R_{X} groups;
each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
A is selected from the group consisting of 4- to 10-membered heterocyclyl, wherein the 4-to 10-membered heterocyclyl is optionally substituted with 1, 2, or 3 Rₐ groups;
each Rₐ is independently selected from the group consisting of C₁₋₁₀ alkyl, C₁₋₁₀ heteroalkyl, amino, or -S(=O)₂-C₁₋₁₀ alkyl;
Ring B is selected from the group consisting of 5- to 7-membered heterocyclic ring, 5- to 7-membered carbocyclic ring, benzene ring, or 5- to 7-membered heteroaromatic ring;
each R_{b} is independently selected from the group consisting of halogen, hydroxyl, cyano, amino, oxo, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, or 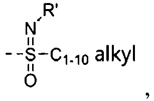 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl and 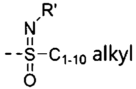 are each optionally substituted with 1, 2, 3 or 4 substituents independently selected from the group consisting of halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONRᵢRⱼ;
R' is selected from the group consisting of H, C₁₋₁₀ alkyl, or CN;
R_{c}, R_{d}, Rₑ, R_{f}, R_{g}, Rₕ, Rᵢ, and Rⱼ are each independently selected from the group consisting of H or C₁₋₁₀ alkyl;
alternatively, two R_{b} groups are connected together to form a C₃₋₁₀ cycloalkyl or a 3- to 10-membered heterocyclyl, wherein the C₃₋₁₀ cycloalkyl and 3- to 10-membered heterocyclyl are each optionally substituted with 1, 2, 3, or 4 substituents independently selected from the group consisting of halogen, hydroxyl, cyano, amino, C₁₋₁₀ alkyl, C₁₋₁₀ heteroalkyl, C₃₋₁₀ cycloalkyl, or 3- to 10-membered heterocyclyl;
n is selected from the group consisting of 0, 1, 2, 3, or 4.

Embodiment 2: The compound, its isomer, isotope-labeled compound, or pharmaceutically acceptable salt thereof according to Embodiment 1, wherein the compound has the structural Formula II: 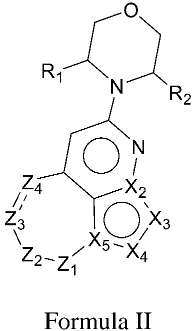 wherein:
○ indicates that the ring to which it belongs is an aromatic ring system;
-̅ -̅ -̅ represents either a single bond or a double bond;
R₁ and R₂ are each independently selected from the group consisting of hydrogen or C₁₋₆ alkyl; or
R₁ and R₂, together with the carbon atom to which they are attached and the adjacent nitrogen atom, form a 4- to 8-membered azacyclic ring;
X₂ is selected from the group consisting of C or N;
X₃ is selected from the group consisting of C(R₃) or N(R₃);
X₄ is selected from the group consisting of N or CH;
X₅ is selected from the group consisting of C or N, and when X₅ is N, X₃ is C(R₃);
R₃ is selected from the group consisting of C₆₋₁₀ aryl, C₃₋₁₀ cycloalkyl or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups;
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently selected from the group consisting of hydrogen or C₁₋₁₀ alkyl;
Z₁ is CR₆R₇;
Z₂ is selected from the group consisting of CR₈R₉, N, NR₁₀, O, S, -C(O)-, or -SO₂-;
Z₃ is selected from the group consisting of CR₁₁, CR₁₂R₁₃, N, NR₁₄, O, -C(O)-, -SO₂-, or -S(O)(NR₁₅)-;
Z₄ is selected from the group consisting of CR₁₆, CR₁₇R₁₈, N, NR₁₉, O, S, -C(O)-, -SO₂-, or -S(O)(NR₂₀)-;
provided that one or two of Z₁, Z₂, Z₃, and Z₄ may not be present; and the ring containing X₃, Z₁, Z₂, Z₃, and Z₄ is a stable ring structure;
R₆, R₇, R₈, R₉, R₁₀, R₁₁, R₁₂, R₁₃, R₁₄, R₁₅, R₁₆, R₁₇, R₁₈, R₁₉, R₂₀ are each independently selected from the group consisting of halogen, hydroxyl, cyano, amino, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂-C₁₋₁₀ alkyl, -SO₂-C₃₋₁₀ cycloalkyl, -SO₂-3- to 10-membered heterocyclyl or 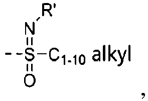 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂C₁₋₁₀ alkyl, -SO₂-C₃₋₁₀ cycloalkyl, -SO₂-3- to 10-membered heterocyclyl, and 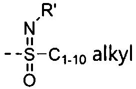 are each optionally substituted with 1, 2, 3, or 4 substituents independently selected from the group consisting of halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONR₂₅R₂₆;
R' is selected from the group consisting of H, C₁₋₁₀ alkyl, or CN;
R₂₁, R₂₂, R₂₃, R₂₄, R₂₅, and R₂₆ is each independently selected from the group consisting of H or C₁₋₁₀ alkyl.
alternatively, R₆ and R₇, R₈ and R₉, R₁₂ and R₁₃, or R₁₇ and R₁₈ are connected together to form a C₃₋₁₀ cycloalkyl or a 3- to 10-membered heterocyclyl, wherein the C₃₋₁₀ cycloalkyl and 3-to 10-membered heterocyclyl are each optionally substituted with 1, 2, 3, or 4 substituents independently selected from the group consisting of halogen, hydroxyl, cyano, amino, C₁₋₁₀ alkyl, C₁₋₁₀ heteroalkyl, C₃₋₁₀ cycloalkyl, or 3- to 10-membered heterocyclyl.

Embodiment 3: The compound, its isomer, isotope-labeled compound, or pharmaceutically acceptable salt thereof according to Embodiment 2, wherein:
R₆, R₇, R₈, R₉, R₁₁, R₁₂, R₁₃, R₁₆, R₁₇, and R₁₈ are each independently hydrogen, halogen, hydroxyl, cyano, C₁₋₁₀ alkyl, C₃₋₁₀ cycloalkyl, 3- to 10-membered heterocyclyl, C₁₋₁₀ alkoxy, C₂₋₅ alkenyl, C₂₋₅ alkynyl, C₆₋₁₀ aryl, 5- 10-membered heteroaryl, -COC₁₋₁₀ alkyl, -CONR₂₁R₂₂, -C(O)OC₁₋₁₀ alkyl, -SO₂NR₂₃R₂₄, or -SO₂C₁₋₁₀ alkyl, wherein the C₁₋₁₀ alkyl, C₃₋₁₀ cycloalkyl, 3-to 10-membered heterocyclyl, C₁₋₁₀ alkoxy, C₂₋₅ alkenyl, C₂₋₅ alkynyl, C₆₋₁₀ aryl, 5-10-membered heteroaryl, -CO-C₁₋₁₀ alkyl, -CONR₂₁R₂₂, -C(O)OC₁₋₁₀ alkyl, -SO₂NR₂₃R₂₄, and -SO₂C₁₋₁₀ alkyl are each optionally substituted with 1, 2, 3, or 4 substituents independently selected from the group consisting of halogen, hydroxyl, cyano, amino, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, and -CONR₂₅R₂₆;
R₁₀, R₁₄, R₁₅, R₁₉, and R₂₀ are each independently hydrogen, C₁₋₁₀ alkyl, C₃₋₁₀ cycloalkyl, 3-to 10-membered heterocyclyl, C₂₋₆ alkenyl, C₂₋₆ alkynyl, C₆₋₁₀ aryl, 5- to 10-membered heteroaryl, C₁₋₁₀ alkoxy, -CO-C₁₋₁₀ alkyl, -CO-C₃₋₁₀ cycloalkyl, -CONR₂₁R₂₂, -C(O)OC₁₋₁₀ alkyl, -SO₂NR₂₃R₂₄, -SO₂-C₁₋₁₀ alkyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl or 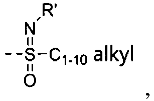 wherein the C₁₋₁₀ alkyl, C₃₋₁₀ cycloalkyl, 3- to 10-membered heterocyclyl, C₂₋₅ alkenyl, C₂₋₅ alkynyl, C₆₋₁₀ aryl, 5- to 10-membered heteroaryl, C₁₋₁₀ alkoxy, -CO-C₁₋₁₀ alkyl, -CO-C₃₋₁₀ cycloalkyl, -CONR₂₁R₂₂, -C(O)OC₁₋₁₀ alkyl, -SO₂NR₂₃R₂₄, -SO₂C₁₋₁₀ alkyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, and 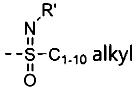 are each optionally substituted with 1, 2, 3 or 4 substituents independently selected from the group consisting of halogen, hydroxyl, cyano, amino, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, -CONR₂₅R₂₆;
R' is selected from the group consisting of H, C₁₋₁₀ alkyl, or CN;
R₂₁, R₂₂, R₂₃, R₂₄, R₂₅, and R₂₆ are each independently selected from the group consisting of H or C₁₋₆ alkyl.

Embodiment 4: The compound, its isomer, isotope-labeled compound, or pharmaceutically acceptable salt thereof according to Embodiment 2 or 3, wherein the compound has the structural formula IIA, IIB, or IIC: 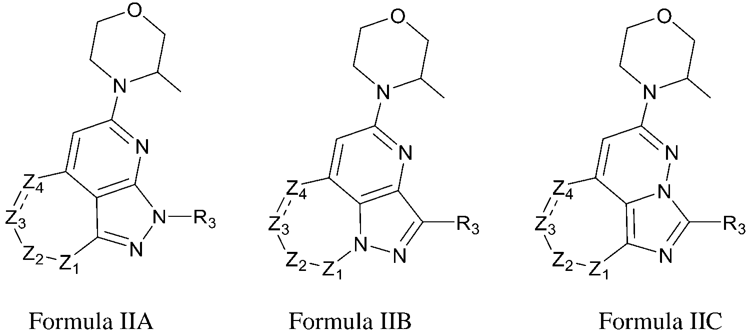 wherein:
R₃ is selected from the group consisting of C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups;
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, and 3- to 10-membered heterocyclyl and -NR₄R₅ are each optionally substituted with 1, 2, or 3 substituents independently selected from the group consisting of halogen, cyano, hydroxyl, or C₁₋₆ alkyl;
R₄ and R₅ are each independently hydrogen or C₁₋₁₀ alkyl.

Embodiment 5: The compound, its isomer, or pharmaceutically acceptable salt thereof according to Embodiment 4, wherein R₃ is a 5- to 6-membered heteroaryl, the 5- to 6-membered heteroaryl is optionally substituted with 1 or 2 substituents independently selected from the group consisting of C₁₋₆ alkyl.

Embodiment 6: The compound, its isomer, isotope-labeled compound, or pharmaceutically acceptable salt thereof according to any one of Embodiments 2 to 5, wherein:
Z₁ is CR₆R₇;
Z₂ is selected from the group consisting of CR₈R₉ or -C(O)-;
Z₃ is selected from the group consisting of CR₁₁, CR₁₂R₁₃, N, NR₁₄, or -C(O)-;
Z₄ is selected from the group consisting of CR₁₆, CR₁₇R ₁₈, N, NR₁₉, or -C(O)-;
R₆, R₇, R₈, R₉, R₁₁, R₁₂, R₁₃, R₁₆, R₁₇, and R₁₈ are each independently selected from the group consisting of hydrogen, hydroxyl, C₁₋₁₀ alkyl, C₃₋₁₀ cycloalkyl, 3- to 10-membered heterocyclyl, C₆₋₁₀ aryl, or 5- to 10-membered heteroaryl, wherein the C₁₋₁₀ alkyl, C₃₋₁₀ cycloalkyl, 3- to 10-membered heterocyclyl, C₆₋₁₀ aryl, and 5- to 10-membered heteroaryl are each optionally substituted with 1 or 2 substituents independently selected from the group consisting of C₁₋₆ alkyl or halogenated C₁₋₆ alkyls;
R₁₄ and R₁₉ are each independently selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5-to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl, or 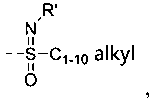 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl, and 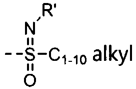 are each optionally substituted with 1, 2, or 3 substituents independently selected from the group consisting of halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, or -CONR₂₅R₂₆;
R' is selected from the group consisting of H, C₁₋₁₀ alkyl, or CN;
R₂₅ and R₂₆ are each independently selected from the group consisting of hydrogen or C₁₋₆ alkyl.

Embodiment 7: The compound, its isomer, isotope-labeled compound, or pharmaceutically acceptable salt thereof according to any one of Embodiments 2 to 6, wherein:
Z₁ is CR₆R₇;
R₆ and R₇ are each independently selected from the group consisting of hydrogen or C₁₋₁₀ alkyl.

Embodiment 8: The compound, its isomer, isotope-labeled compound, or pharmaceutically acceptable salt thereof according to any one of Embodiments 2 to 7, wherein:
Z₂ is selected from the group consisting of CR₈R₉ or -C(O)-;
R₈ and R₉ are each independently selected from the group consisting of hydrogen or C₁₋₁₀ alkyl.

Embodiment 9: The compound, its isomer, isotope-labeled compound, or pharmaceutically acceptable salt thereof according to any one of Embodiments 2 to 8, wherein:
Z₃ is selected from the group consisting of CR₁₁, CR₁₂R₁₃, N, NR₁₄, or -C(O)-; R₁₁, R₁₂, and R₁₃ are each independently selected from the group consisting of hydrogen, hydroxyl, C₁₋₁₀ alkyl, C₃₋₁₀ cycloalkyl, 3- to 10-membered heterocyclyl, or 5- to 10-membered heteroaryl, wherein the C₁₋₁₀ alkyl, C₃₋₁₀ cycloalkyl, 3- to 10-membered heterocyclyl, and 5- to 10-membered heteroaryl is each optionally substituted with 1 or 2 substituents independently selected from the group consisting of C₁₋₆ alkyl or halogenated C₁₋₆ alkyl;
R₁₄ is selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl or 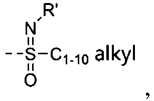 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl alkyl, -C(O)OC₁₋₁₀ alkyl, -SO₂-C₁₋₁₀ alkyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, and 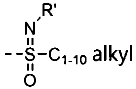 are each optionally substituted with 1, 2, or 3 substituents independently selected from the group consisting of halogen, hydroxyl, cyano, C₁₋₆ alkyl, or halogenated C₁₋₆ alkyl;
R' is selected from the group consisting of H, C₁₋₁₀ alkyl, or CN.

Embodiment 10: The compound, its isomer, isotope-labeled compound, or pharmaceutically acceptable salt thereof according to any one of Embodiments 2 to 9, wherein:
Z₄ is selected from the group consisting of CR₁₆, CR₁₇R₁₈, N, NR₁₉, or -C(O)-;
R₁₆, R₁₇, and R₁₈ are each independently selected from the group consisting of hydrogen, C₁₋₁₀ alkyl, or C₃₋₁₀ cycloalkyl, wherein the C₁₋₁₀ alkyl and C₃₋₁₀ cycloalkyl are each optionally substituted with 1 or 2 substituents independently selected from the group consisting of C₁₋₆ alkyl or halogenated C₁₋₆ alkyl;
R₁₉ is selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, -SO₂-C₁₋₁₀ alkyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, or -C₁₋₄ alkyl-5- to 10-membered heteroaryl, wherein the C₁₋₁₀ alkyl, C₃₋₁₀ cycloalkyl, 3- to 10-membered heterocyclyl, C₂₋₅ alkenyl, C₂₋₅ alkynyl, -SO₂C₁₋₁₀ alkyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, and -C₁₋₄ alkyl-5- to 10-membered heteroaryl are each optionally substituted with 1, 2, or 3 substituents independently selected from the group consisting of halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, or -CONR₂₅R₂₆; and
R₂₅ and R₂₆ are each independently selected from the group consisting of hydrogen or C₁₋₆ alkyl.

Those skilled in the art will understand that, in the above description of the represent application, combinations of substituents are permitted only if such combinations result in stable compounds. Therefore, when describing compounds of the general formulas, combinations of substituents that do not result in stable compounds are not included in the scope of the represent application. Those skilled in the art will readily be able to exclude such impermissible substituent combinations.

In one embodiment, the compound of the represent application is selected from the group consisting of: 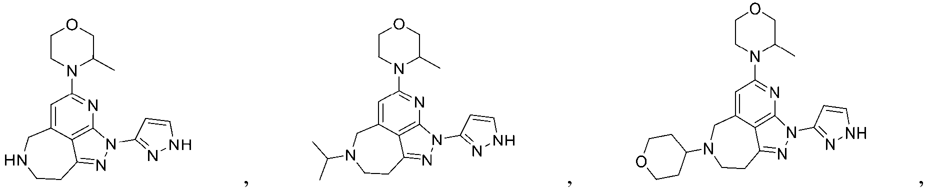 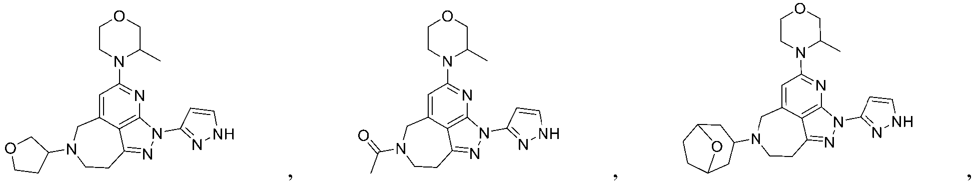 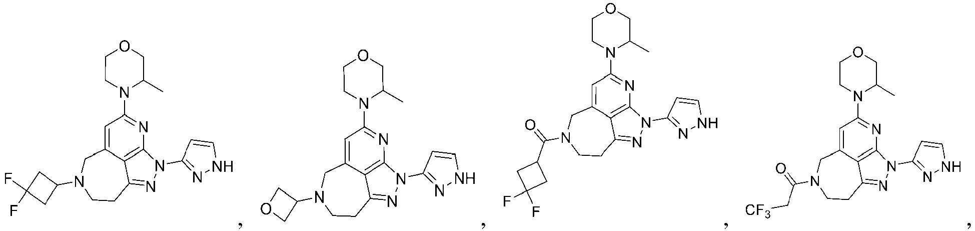 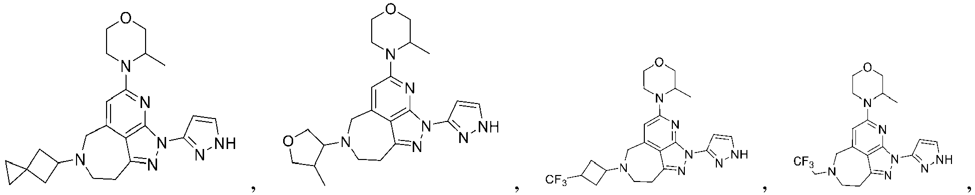 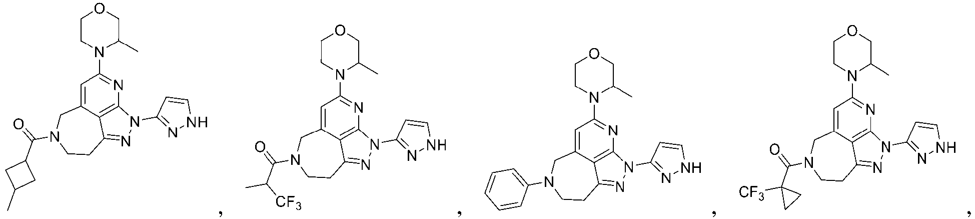 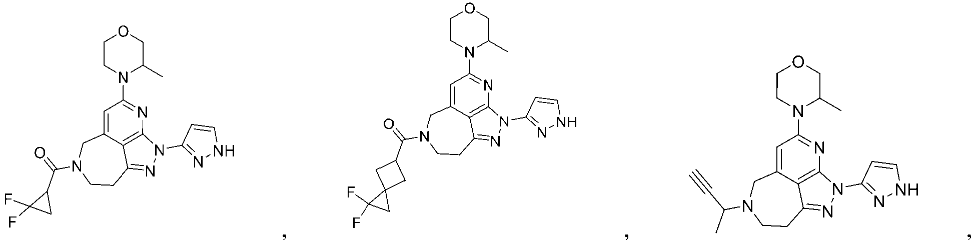 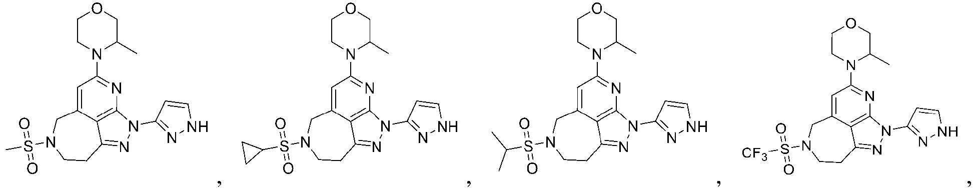 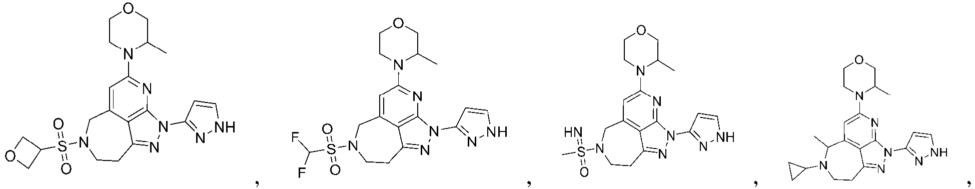 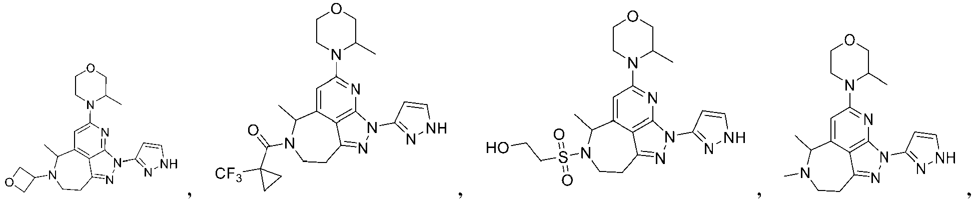 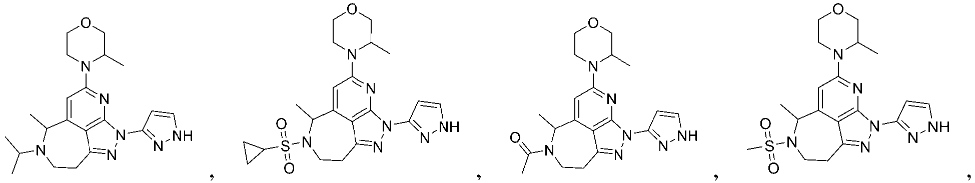 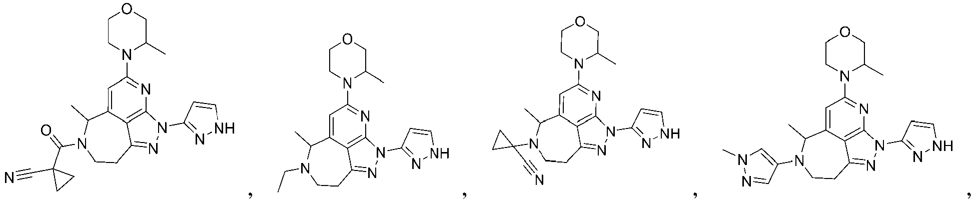 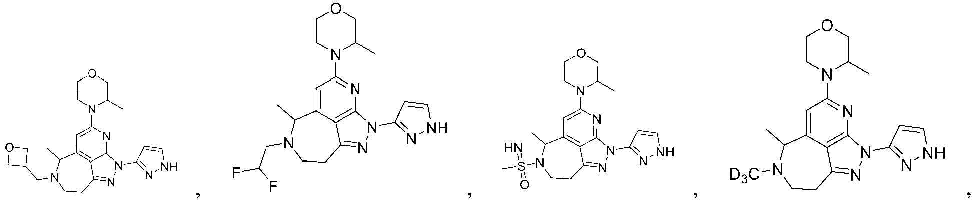 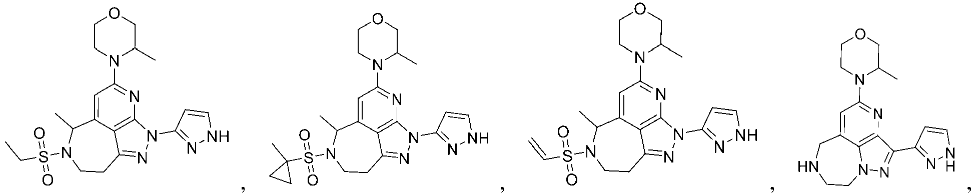 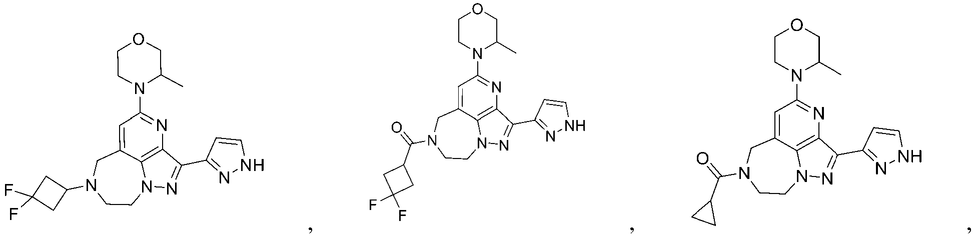 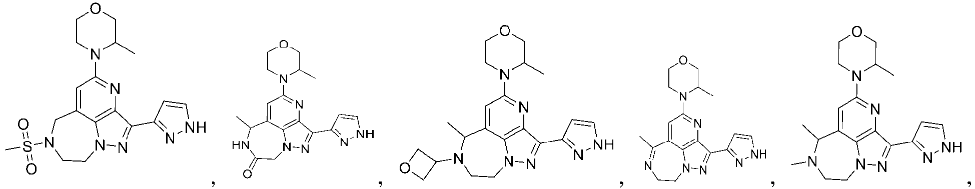 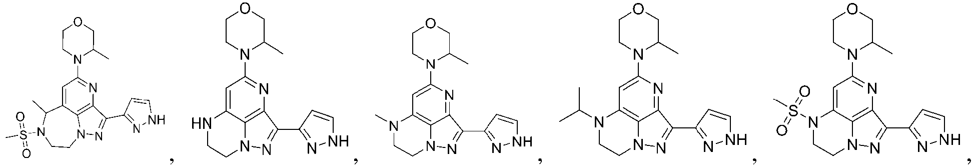 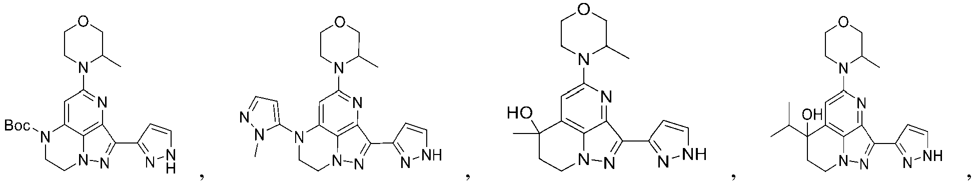 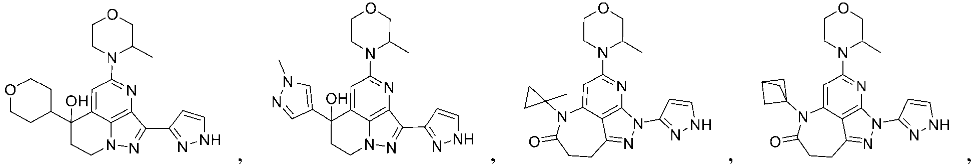 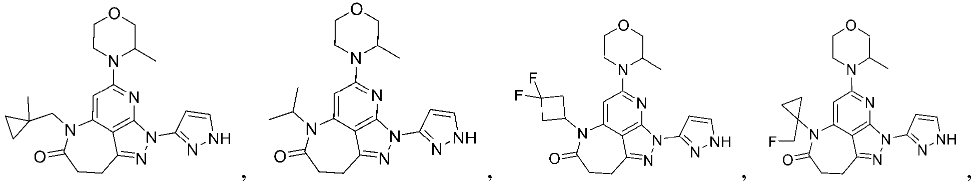 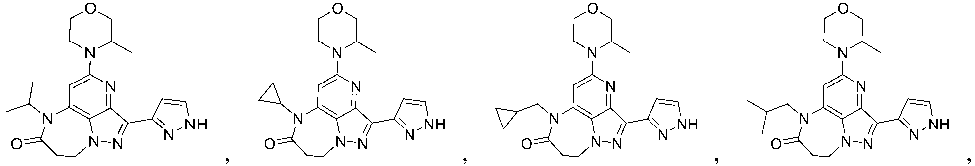 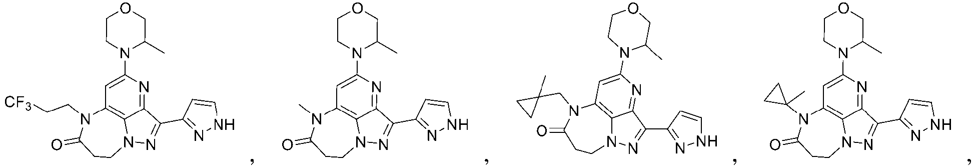 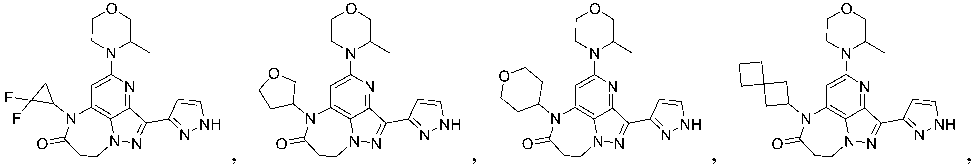 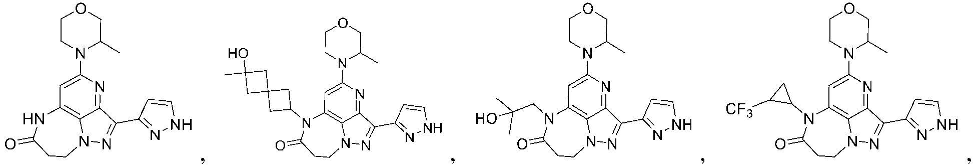 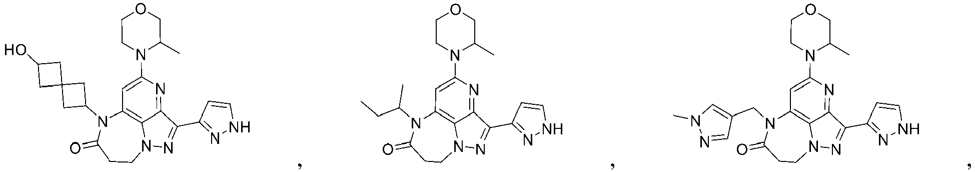 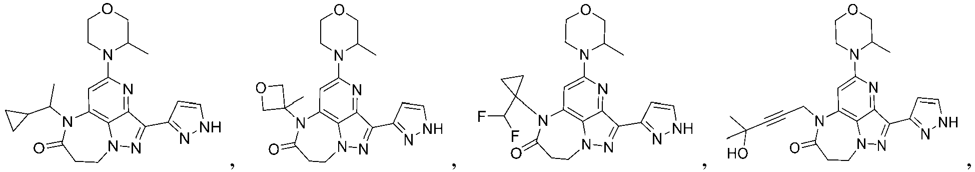 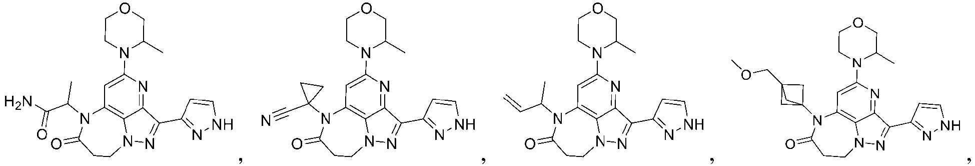 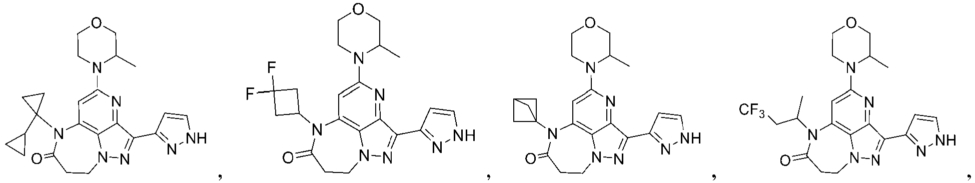 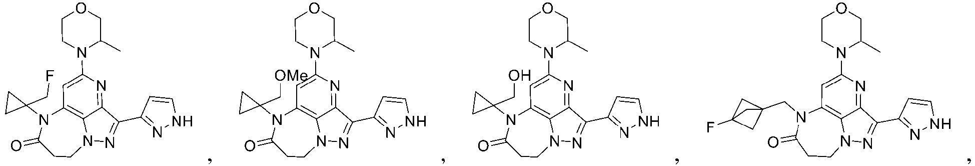 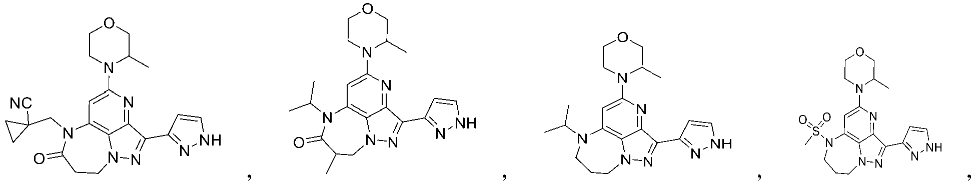 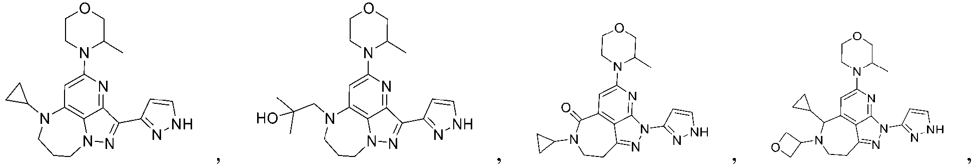 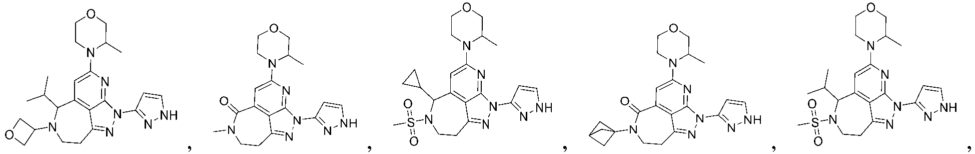 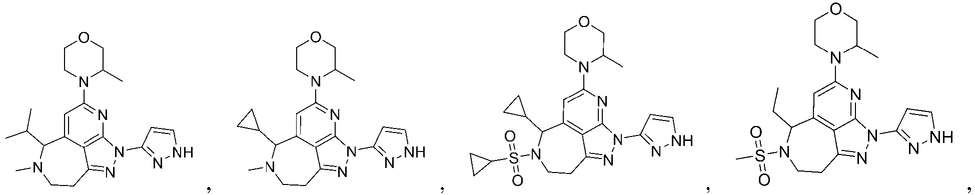 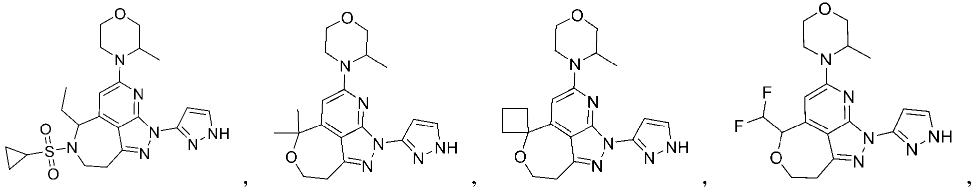 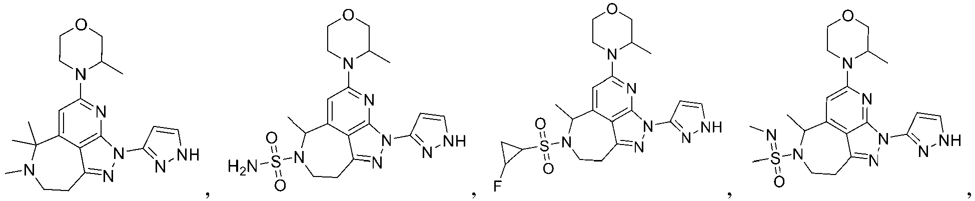 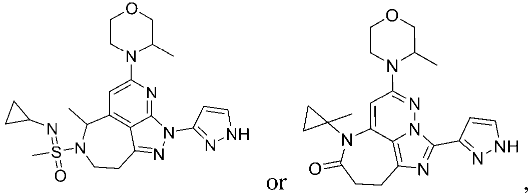 or, an isomer, isotope-labeled compound of the above compound, or a pharmaceutically acceptable salt thereof.

In one embodiment, the compound of the present application is selected from the group consisting of: 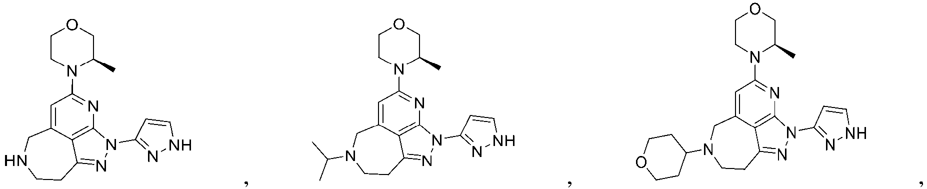 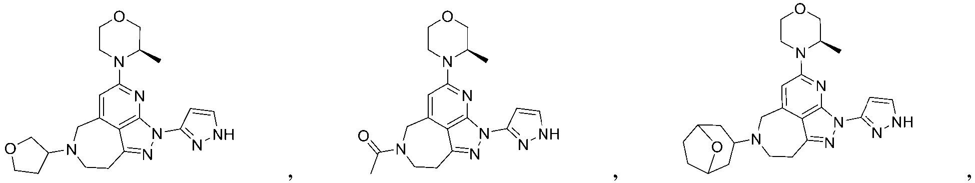 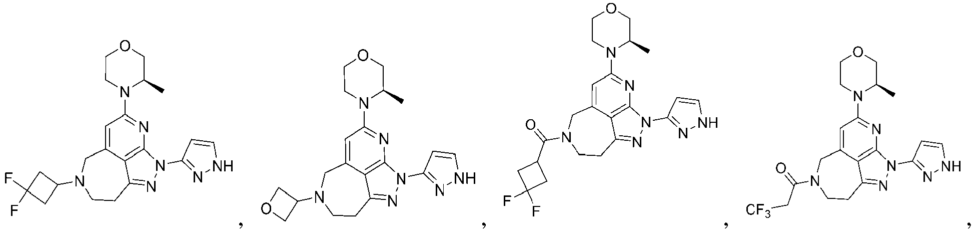 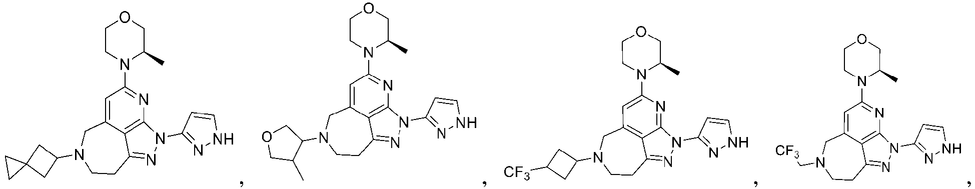 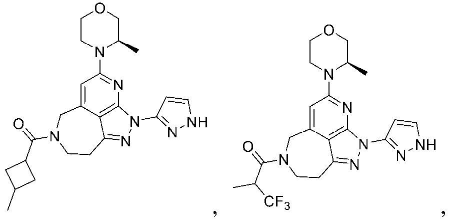 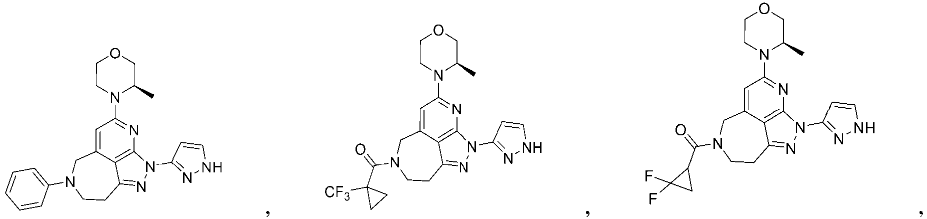 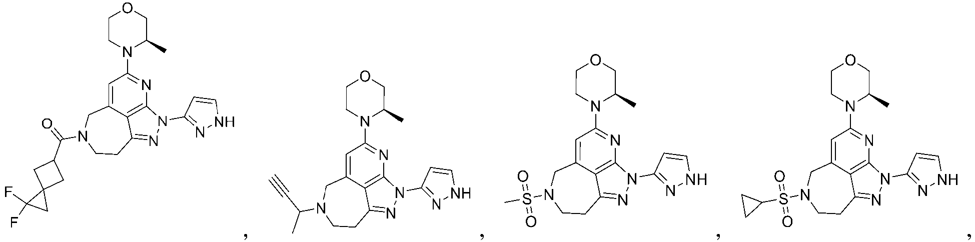 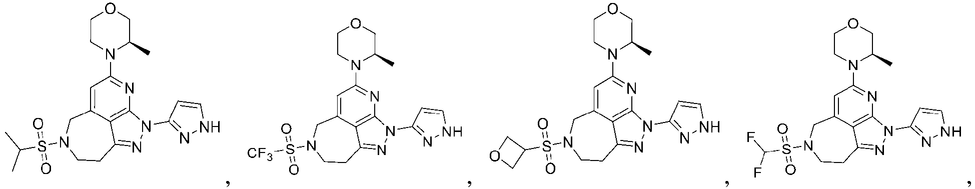 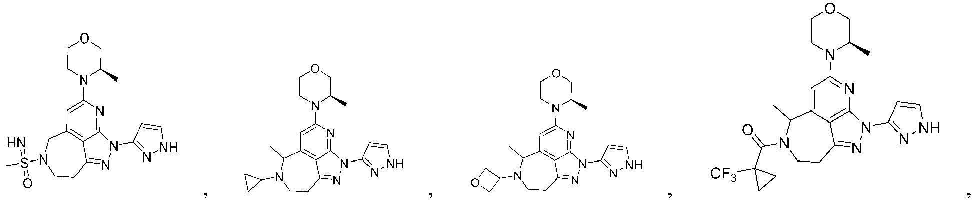 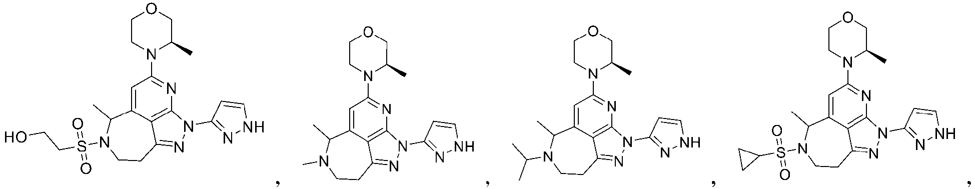 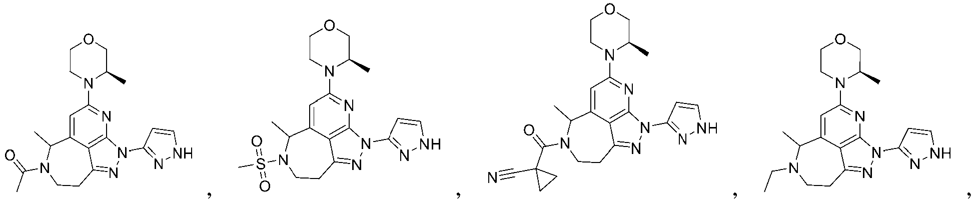 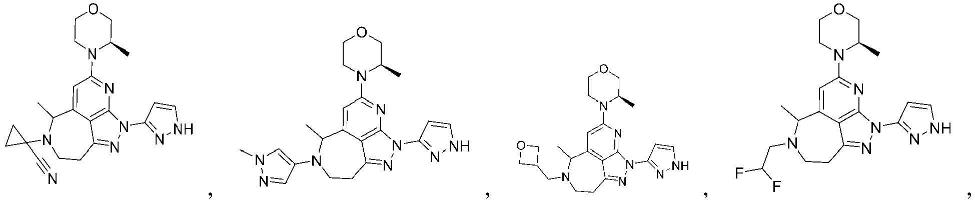 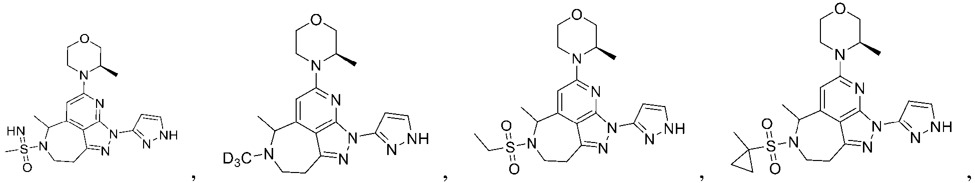 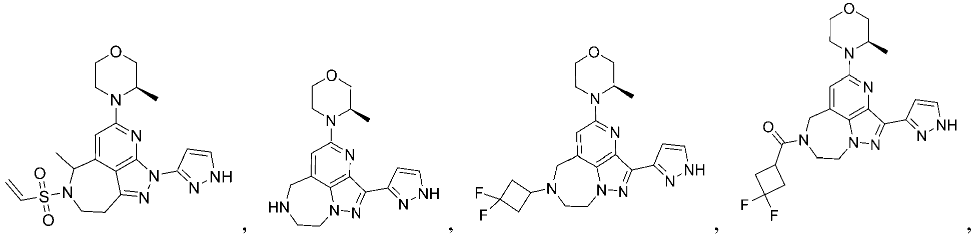 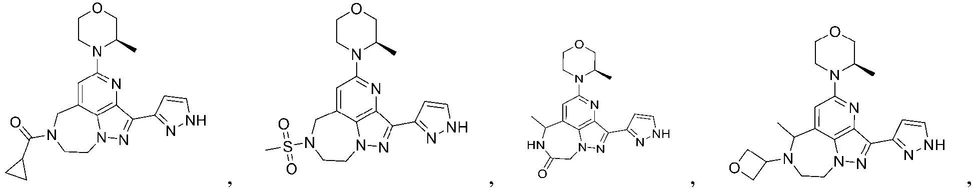 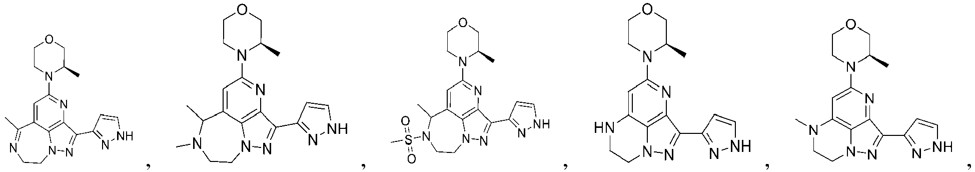 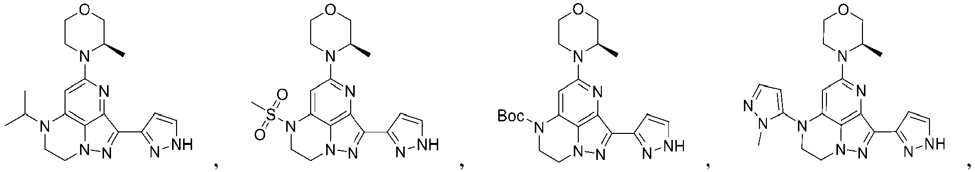 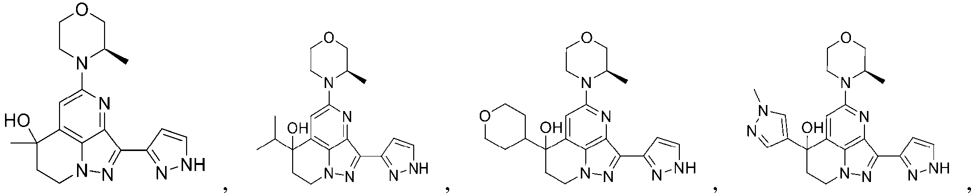 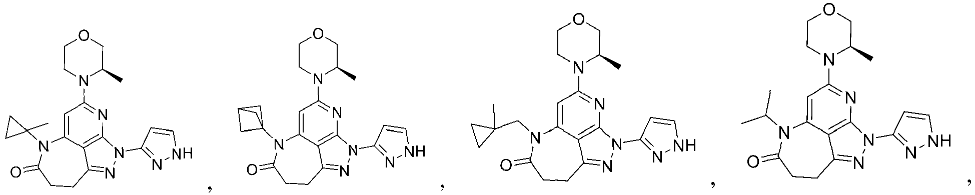 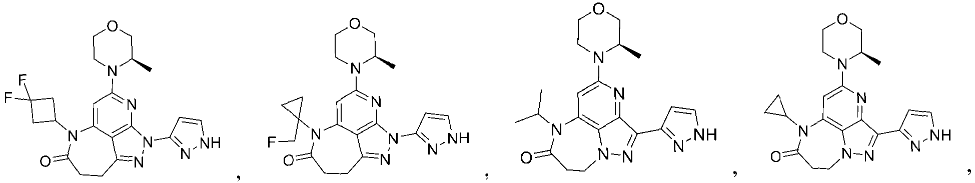 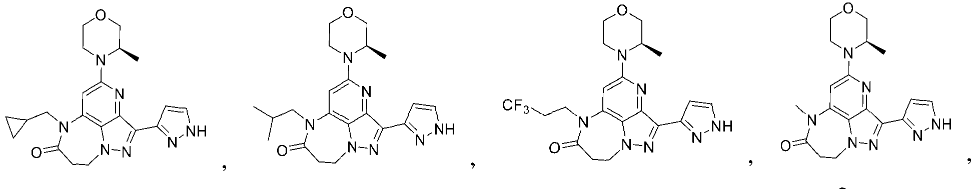 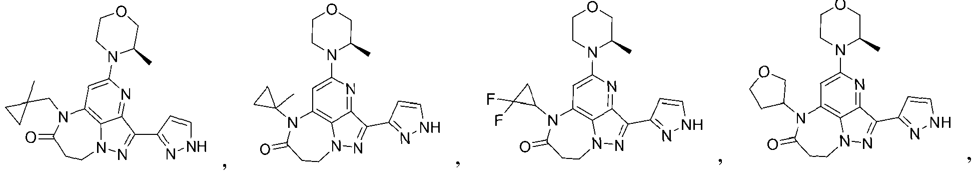 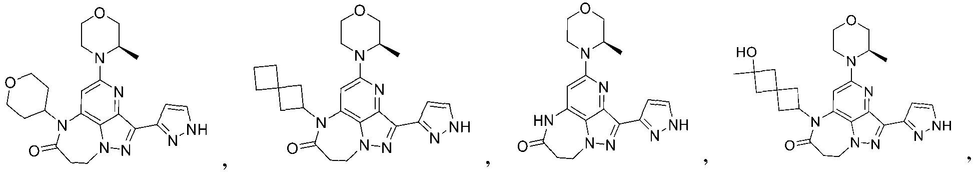 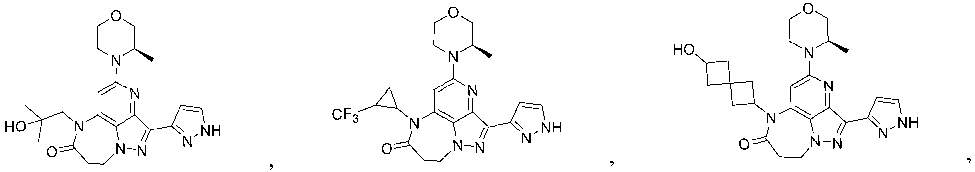 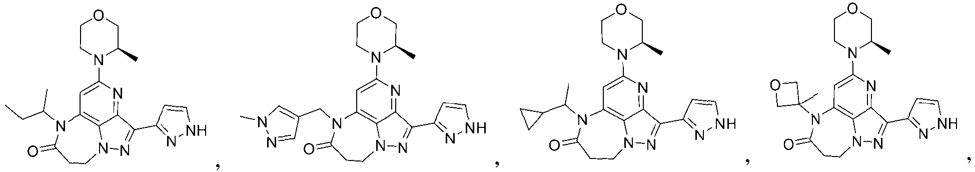 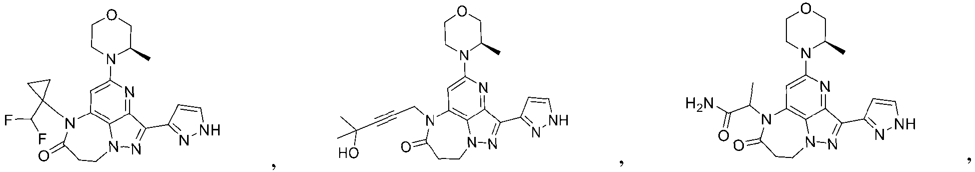 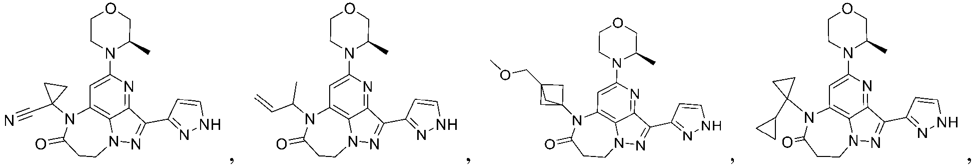 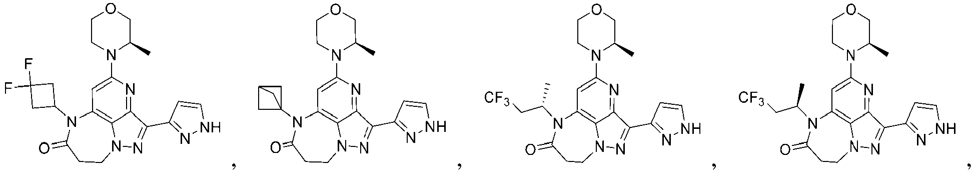 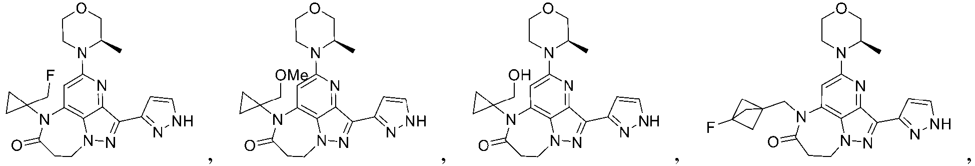 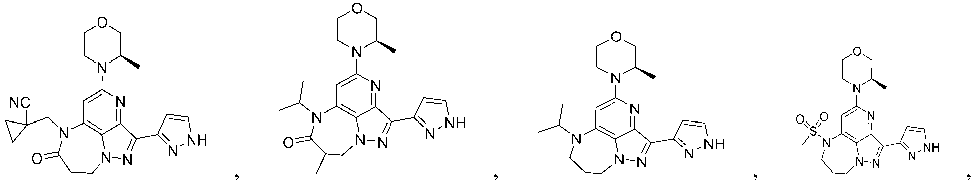 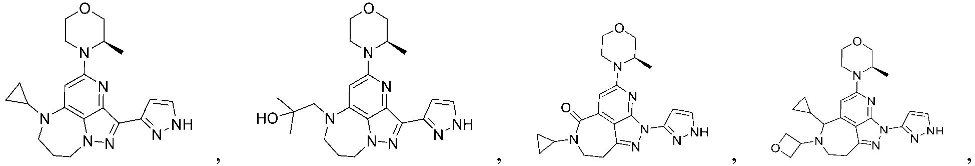 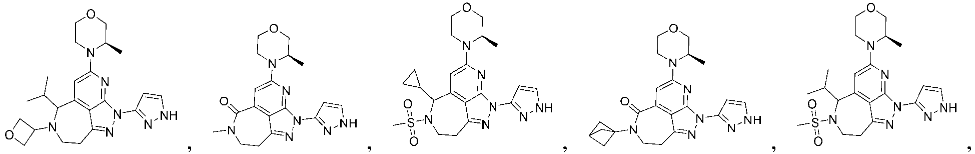 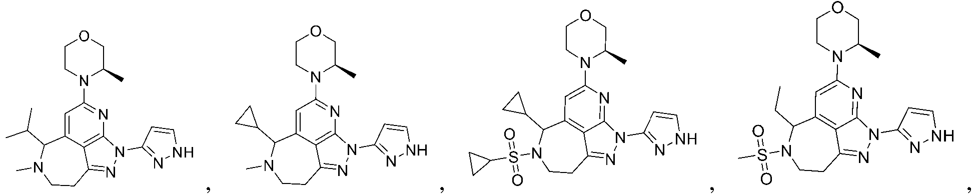 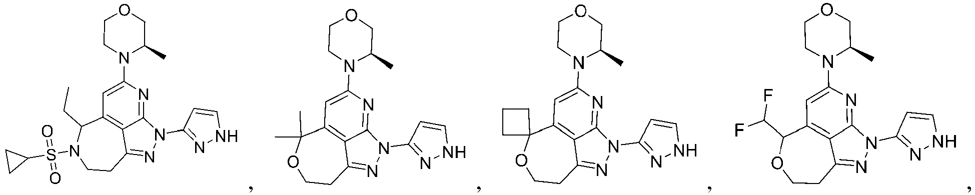 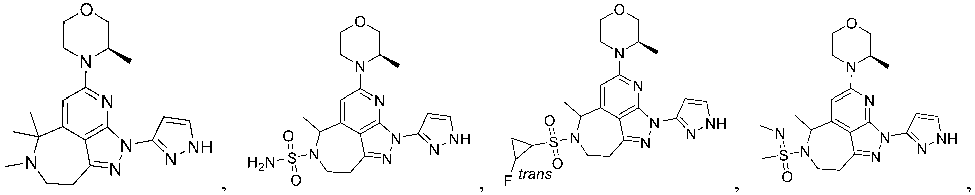 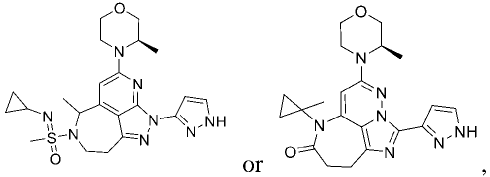 or an isomer, isotope-labeled compound of the above compound, or a pharmaceutically acceptable salt thereof.

In another aspect, the present invention provides a pharmaceutical composition, which comprises the compound, its isomer, isotope-labeled compound, or pharmaceutically acceptable salt thereof of the present application, and a pharmaceutically acceptable excipient.

### Usage method or use of the compound of the present application

In another aspect, the present application provides the compound, its isomer, isotope-labeled compound, or pharmaceutically acceptable salt thereof of the present application, which is used as an ATR kinase inhibitor.

In another aspect, the present application provides a use of the compound, its isomer, isotope-labeled compound, or pharmaceutically acceptable salt thereof of the present application, in the manufacture of an ATR kinase inhibitor.

In another aspect, the present application provides a use of the compound, its isomer, isotope-labeled compound, or pharmaceutically acceptable salt thereof of the present application, in the manufacture of medicament for treating a cancer.

In another aspect, the present application provides the compound, its isomer, isotope-labeled compound, or pharmaceutically acceptable salt thereof of the present application, which is used for treating a cancer.

In another aspect, the present application provides a method for treating a cancer in a patient in need thereof, comprising administering to the patient a therapeutically effective amount of the compound, its isomer, isotope-labeled compound, or pharmaceutically acceptable salt thereof of the present application.

In some embodiments, the cancer is a colon cancer.

In another aspect, the present application provides a use of the compound, its isomer, isotope-labeled compound, or pharmaceutically acceptable salt thereof of the present application, in the manufacture of a medicament for treating a disease mediated by ATR kinase.

In another aspect, the present application provides the compound, its isomer, isotope-labeled compound, or pharmaceutically acceptable salt thereof of the present application, which is used for treating a disease mediated by ATR kinase.

In another aspect, the present application provides a method for treating an ATR kinase-mediated disease in a patient in need thereof, comprising administering to the patient a therapeutically effective amount of the compound, its isomer, isotope-labeled compound, or pharmaceutically acceptable salt thereof of the present application.

In some embodiments, the ATR kinase-mediated disease is a proliferative or hyperproliferative disease, such as a proliferative or hyperproliferative disease caused by excessive or abnormal cell proliferation, particularly a proliferative or hyperproliferative disease caused by excessive or abnormal cell proliferation mediated by ATR kinase. In some embodiments, examples of ATR kinase-mediated disease include, but are not limited to, cancers and myeloproliferative diseases.

### Preparation of the compound of the present application

The following reaction schemes illustrate the methods for preparing the compounds of the present invention.

### Preparation of Intermediate INT-1

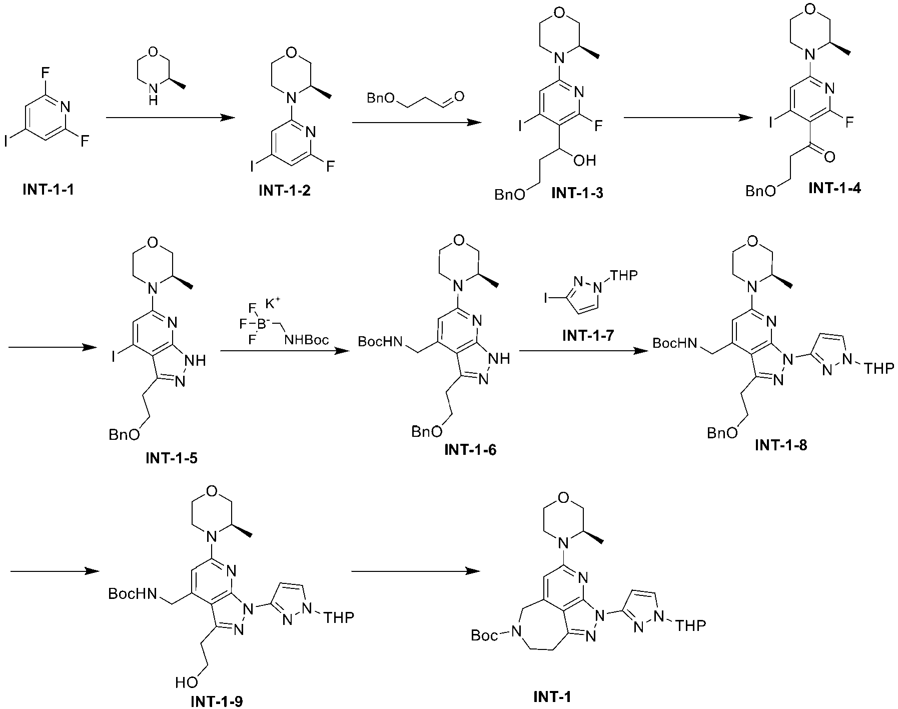

Step 1: 2,6-Difluoro-4-iodopyridine (50 g, 207 mmol) was dissolved in dimethyl sulfoxide (500 mL). (R)-3-methylmorpholine (19.9 g, 197 mmol) and N,N-diisopropylethylamine (68.6 mL, 415 mmol) were added in sequence. The reaction mixture was stirred at 100°C overnight. After the reaction was completed, the reaction mixture was cooled to room temperature, diluted with ethyl acetate, washed sequentially with water and saturated brine, dried over anhydrous sodium sulfate, and filtered. The filtrate was concentrated under reduced pressure to obtain a crude product, which was then separated and purified by silica gel chromatography to yield Intermediate **INT-1-2** (47 g, 70.3%). LCMS (ESI) [M+H]⁺: 323.0.

Step 2: Intermediate **INT-1-2** (48 g, 149 mmol) was dissolved in anhydrous tetrahydrofuran (500 mL) and slowly added dropwise with lithium diisopropylamide (149 mL, 2 M) at -70°C. The reaction mixture was stirred at -70°C for 1 hour. Then, 3-benzyloxypropanal (29.3 g, 179 mmol) was slowly added dropwise to the reaction mixture at -70°C, and the reaction mixture was stirred at -70°C for 1 hour. After the reaction was completed, the reaction mixture was slowly added dropwise with formic acid (12.5 mL) at -60°C, and then diluted with ethyl acetate. When the reaction mixture reached room temperature, it was washed sequentially with water and saturated brine, dried over anhydrous sodium sulfate, and filtered. The filtrate was concentrated under reduced pressure to obtain a crude product, which was then separated and purified by silica gel chromatography to yield Intermediate **INT-1-3** (22.7 g, 31.3%). LCMS (ESI) [M+H]⁺: 487.1.

Step 3: Intermediate **INT-1-3** (23 g, 47.3 mmol) was dissolved in dichloromethane (200 mL). Dess-Martin periodinane (30.1 g, 70.9 mmol) was slowly added in batches at 0°C. The reaction mixture was stirred at room temperature under a nitrogen atmosphere for 1 hour. After the reaction was completed, sodium bicarbonate was added to the reaction mixture to adjust the pH to 8, and then the reaction mixture was filtered. The filtrate was washed with water, dried over anhydrous sodium sulfate, and filtered. The filtrate was concentrated under reduced pressure to obtain a crude product, which was separated and purified by silica gel chromatography to yield Intermediate **INT-1-4** (20 g, 87.3%). LCMS (ESI) [M+H]⁺: 485.1.

Step 4: Intermediate **INT-1-4** (20 g, 41.3 mmol) was dissolved in ethanol (200 mL), and hydrazine hydrate (20.4 mL, 413 mmol, 98.0% wt) was added at room temperature. The reaction mixture was stirred at 80°C under a nitrogen atmosphere for 2 hours. After the reaction was completed, the reaction mixture was concentrated under reduced pressure to remove most of the ethanol, then diluted with ethyl acetate. The organic phase was washed with water, dried over anhydrous sodium sulfate, and filtered. The filtrate was concentrated under reduced pressure to obtain a crude product, which was separated and purified by silica gel chromatography to yield Intermediate **INT-1-5** (7 g, 35.4%). LCMS (ESI) [M+H]⁺: 479.1.

Step 5: Intermediate **INT-1-5** (4.9 g, 10.2 mmol) and potassium N-Boc-aminomethyltrifluoroborate (3.64 g, 15.3 mmol) were dissolved in a mixed solvent of dioxane (90 mL) and water (10 mL), and n-butyl-di(1-adamantyl)phosphine (0.73 g, 2.05 mmol), palladium acetate (0.46 g, 2.05 mmol), and cesium carbonate (10 g, 30.7 mmol) were added in sequence. The reaction mixture was stirred at 100°C under a nitrogen atmosphere for 6 hours. After the reaction was completed, the mixture was cooled to room temperature and diluted with ethyl acetate. The organic phase was washed with water, dried over anhydrous sodium sulfate, and filtered. The filtrate was concentrated under reduced pressure to obtain a crude product, which was separated and purified by silica gel chromatography to yield Intermediate **INT-1-6** (4.8 g, 97.3%). LCMS (ESI) [M+H]⁺: 482.3.

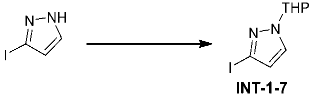

Step 6: 3-Iodo-1-hydrogen-pyrazole (14.5 g, 74.7 mmol) was dissolved in dichloromethane (150 mL), and p-toluenesulfonic acid monohydrate (1.29 g, 7.47 mmol) and 3,4-dihydro-2H-pyran (12.6 g, 149 mmol) were added in sequence. The reaction mixture was stirred at room temperature overnight. After the reaction was completed, the dichloromethane was removed under reduced pressure to obtain a residue, which was separated and purified by silica gel chromatography to yield Intermediate **INT-1-7** (14 g, 67.4%). LCMS (ESI) [M+H]⁺: 279.0.

Step 7: Intermediate **INT-1-6** (4.60 g, 9.55 mmol) and Intermediate **INT-1-7** (3.19 g, 11.46 mmol) were dissolved in toluene (50 mL), and cuprous iodide (1.82 g, 9.55 mmol), anhydrous potassium phosphate (8.11 g, 38.2 mmol), and N,N-dimethylethylenediamine (1.68 g, 19.1 mmol) were added in sequence. The reaction mixture was then heated to 110°C and stirred overnight at 110°C under a nitrogen atmosphere. After the reaction was completed, the reaction mixture was cooled to room temperature and filtered. The filtrate was concentrated under reduced pressure to obtain a crude product, which was then separated and purified by silica gel chromatography to yield Intermediate **INT-1-8** (3 g, 49.7%). LCMS (ESI) [M+H]⁺: 632.3.

Step 8: Intermediate **INT-1-8** (3 g, 4.75 mmol) was dissolved in methanol (30 mL), and palladium hydroxide-on-carbon (6.67 g, 4.75 mmol, 10.0% wt) was added. The reaction mixture was stirred at room temperature overnight under a hydrogen atmosphere (50 psi). After the reaction was completed, the reaction mixture was filtered and the filtrate was concentrated to obtain crude Intermediate **INT-1-9** (1.50 g, 2.77 mmol). LCMS (ESI) [M+H]⁺: 542.3.

Step 9: Intermediate **INT-1-9** (400 mg, 0.74 mmol) was dissolved in dioxane (20 mL), and cyanomethylenetributylphosphorane (891 mg, 3.69 mmol) was added. The reaction mixture was stirred at 150°C for 1 hour under microwave irradiation. After cooling, the reaction mixture was concentrated under reduced pressure to obtain a crude product, which was then separated and purified by silica gel chromatography to yield Intermediate **INT-1** (320 mg, 82.7%). LCMS (ESI) [M+H]⁺: 524.3.

### Preparation of Intermediate INT-2

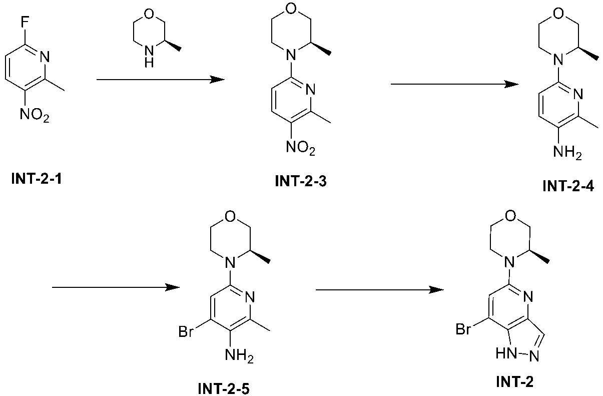

Step 1: **INT-2-1** (20 g, 128.1 mmol) was dissolved in N-methylpyrrolidone (80 mL), and (R)-3-methylmorpholine (19.44 g, 192.2 mmol) and diisopropylethylamine (33.12 g, 256.2 mmol) were added. The reaction mixture was heated to 110°C under nitrogen and stirred for 1 hour. The reaction mixture was cooled to 25°C and quenched with ice water (100 mL). The aqueous phase was extracted with ethyl acetate (50 mL ×3). The organic phase was washed with water and then saturated brine. The organic phase was dried over anhydrous sodium sulfate, filtered, and concentrated to yield crude **INT-2-3** (25 g, 82.3%). LCMS (ESI) [M+H]⁺: 238.2.

Step 2: **INT-2-3** (25 g, 105.4 mmol) was dissolved in anhydrous ethanol (250 mL). 10% palladium-on-carbon (2.24 g, 21.1 mmol) was added, and then the reaction was stirred at 25°C under a hydrogen atmosphere for 24 hours. The reaction mixture was filtered through diatomite, and the filter cake was washed with anhydrous methanol. The filtrate was concentrated to yield **INT-2-4** (21.50 g, 98.4%). LCMS (ESI) [M+H]⁺: 208.4.

Step 3: **INT-2-4** (7.5 g, 36.18 mmol) was dissolved in trifluoroacetic acid (75 mL). At 25°C, N-bromosuccinimide (10.3 g, 57.9 mmol) was added in batches to the reaction system. The reaction system was stirred for half an hour, then dichloromethane (70 mL) was added to dilute the reaction solution. The reaction was quenched with saturated sodium carbonate solution at 0°C, and the pH was adjusted to 8. The aqueous phase was extracted with dichloromethane (60 mL × 3), and the combined organic phases were washed with saturated brine (50 mL × 3). The organic phases were dried over anhydrous sodium sulfate, filtered, and concentrated to obtain a crude product. The crude product was purified by silica gel column chromatography to yield **INT-2-5** (4.2 g, 40.6%). LCMS (ESI) [M+H]⁺: 286.2, 288.2.

Step 4: **INT-2-5** (4.2 g, 14.7 mmol) was dissolved in toluene (15 mL). Potassium acetate (2.88 g, 29.4 mmol) and acetic acid (2 mL) were added at 0°C under a nitrogen atmosphere. The reaction system was stirred well, then isoamyl nitrite (2.58 g, 22 mmol) was slowly added. The reaction was heated to 30°C and reacted for 4 hours. The reaction mixture was diluted with ethyl acetate (20 mL) and adjusted with saturated sodium bicarbonate solution to pH=8. The aqueous phase was extracted with ethyl acetate (30 mL × 2). The combined organic phases were washed with saturated brine (50 mL × 3), dried over anhydrous sodium sulfate, filtered, and concentrated to obtain a crude product. The crude product was purified by silica gel column chromatography to yield **INT-2** (2.5 g, 57.79%). LCMS (ESI) [M+H]⁺: 296.8 and 299.0.

### Preparation of Intermediate INT-3

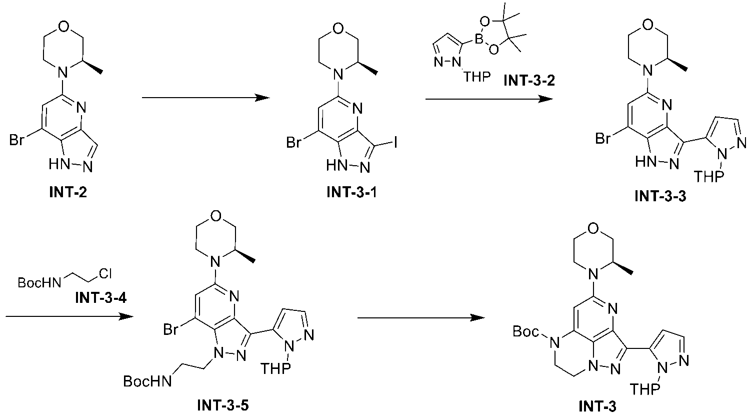

Step 1: **INT-2** was dissolved in N,N-dimethylformamide (25 mL), and add with N-iodosuccinimide (2.5 g, 10.94 mmol) in batches. The reaction mixture was stirred at 25°C for 1 hour. Then, the reaction mixture was quenched with ice water (50 mL) at 25°C. The aqueous phase was extracted with ethyl acetate (20 mL × 3). The organic phase was washed sequentially with water and saturated brine, dried over anhydrous sodium sulfate, filtered, and spin-dried to obtain a crude product. The crude product was purified by silica gel column chromatography to yield **INT-3-1** (1.9 g, 53.4%). LCMS (ESI) [M+H]⁺: 423.0, 425.0.

Step 2: **INT-3-1** (0.63 g, 1.49 mmol) was dissolved in dioxane/water (10/1) (9 mL), and **INT-3-2** (1.65 g, 5.96 mmol), tetrakis(triphenylphosphine palladium) (0.34 g, 0.3 mmol), and potassium carbonate (0.62 g, 4.47 mmol) were added in sequence. The mixture was stirred at 80°C under a nitrogen atmosphere for 6 hours. The reaction was quenched with water at 25°C. The aqueous phase was extracted with ethyl acetate (20 mL × 3). The organic phase was washed with water (20 mL) and saturated brine (20 mL), dried over anhydrous sodium sulfate, filtered, and spin-dried to obtain a crude product. The crude product was purified by silica gel column chromatography to yield **INT-3-3** (440 mg, 66%). LCMS (ESI) [M+H]⁺: 447.0, 449.0.

Step 3: **INT-3-3** (0.44 g, 0.98 mmol) was dissolved in N,N-dimethylformamide (5 mL), and **INT-3-4** (0.27 g, 1.48 mmol), potassium carbonate (0.41 g, 2.95 mmol), and sodium iodide (44.2 mg, 0.3 mmol) were added in sequence. The reaction mixture was stirred at 120°C for 1 hour. The reaction was quenched with ice water at 25°C. The aqueous phase was extracted with ethyl acetate (20 mL × 3). The organic phase was washed with water (20 mL) and saturated brine (20 mL), dried over anhydrous sodium sulfate, filtered, and spin-dried to obtain a crude product. The crude product was purified by silica gel column chromatography to obtain **INT-3-5** (0.52 g, 89.5%). LCMS (ESI) [M+H]⁺: 590.5, 592.5.

Step 4: **INT-3-5** (0.57 g, 0.97 mmol) was dissolved in dioxane (8 mL), and BrettPhos Pd G3 (87.5 mg, 0.097 mmol) and cesium carbonate (0.95 g, 2.9 mmol) were added in sequence. The reaction mixture was stirred at 100°C under nitrogen for 2 hours. Then the reaction mixture was cooled to room temperature, filtered, and concentrated to obtain a crude product. The crude product was purified by silica gel column chromatography to yield INT-3 (0.3 g, 61%). LCMS (ESI) [M+H]⁺: 510.2.

### Preparation of Intermediate INT-4

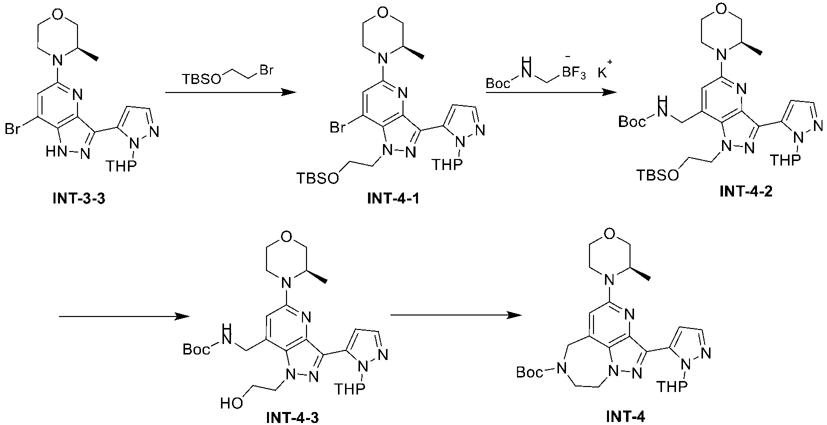

Step 1: **INT-3-3** (1.50 g, 3.35 mmol) was dissolved in N,N-dimethylformamide (15 mL), and (2-bromoethoxy)-tert-butyldimethylsilane (2.00 g, 8.38 mmol), potassium carbonate (1.40 g, 10.1 mmol), and sodium iodide (0.10 g, 0.671 mmol) were added in sequence. The reaction mixture was stirred at 120°C for 1 hour. The reaction mixture was cooled to room temperature and quenched with ice-cold water, then extracted with ethyl acetate (10 mL × 3). The combined organic phases were washed sequentially with water and saturated brine, dried over anhydrous sodium sulfate, filtered, and concentrated to obtain a crude product, which was purifed by silica gel column chromatography to yield Intermediate **INT-4-1** (1.72 g, 84.7%). LCMS (ESI) [M+H]⁺: 606.4.

Step 2: Intermediate **INT-4-1** (1.5 g, 2.48 mmol) was dissolved in dioxane/water (10 mL, 4/1), and potassium [(tert-butoxycarbonylamino)methyl]trifluoroborate (1.17 g, 4.95 mmol), [1,1'-bis(diphenylphosphino)ferrocene]palladium dichloride (362 mg, 0.495 mmol), and cesium carbonate (2.42 g, 7.43 mmol) were added in sequence. The reaction mixture was stirred at 100°C under a nitrogen atmosphere for 2 hours. The reaction mixture was cooled to room temperature, quenched with ice water, and extracted with ethyl acetate (10 mL × 3). The combined organic phases were washed sequentially with water and saturated brine. The organic phase was dried over anhydrous sodium sulfate, filtered, and concentrated to obtain a crude product, which was purified by silica gel column chromatography to yield Intermediate **INT-4-2** (1 g, 55.2%). LCMS (ESI) [M+H]⁺: 656.2.

Step 3: Intermediate **INT-4-2** (110 mg, 0.168 mmol) was dissolved in tetrahydrofuran, and tetrabutylammonium fluoride-tetrahydrofuran solution (0.25 mL, 1 M) was added in sequence. The reaction mixture was stirred at room temperature for half an hour. The solvent was spin-dried to obtain a crude product, which was purified by silica gel column chromatography to yield Intermediate **INT-4-3** (70 mg, 77.1%). LCMS (ESI) [M+H]⁺: 542.0.

Step 4: Intermediate **INT-4-3** (50 mg, 0.092 mmol) was dissolved in toluene (3 mL), and cyanomethylenetributylphosphorane (159 mg, 0.462 mmol) was added. The reaction mixture was stirred at 120°C under a nitrogen atmosphere for 2 hours. The solvent was spin-dried to obtain a crude product, which was purified by silica gel column chromatography to yield Intermediate **INT-4** (48 mg, 99.3%). LCMS (ESI) [M+H]⁺: 524.2.

### Preparation of Intermediate INT-5

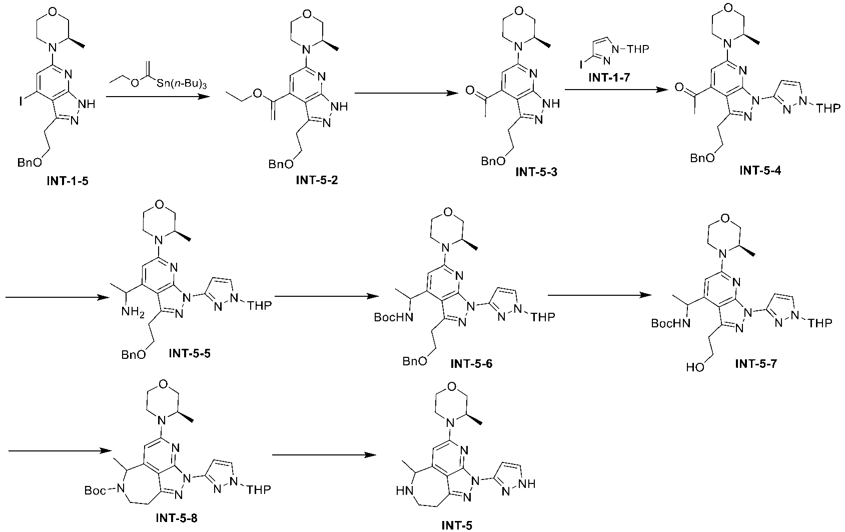

Step 1: Intermediate INT-1-5 (30 g, 62.7 mmol) and tetrakis(triphenylphosphine)palladium (7.2 g, 6.27 mmol) was dissolved in anhydrous dioxane (400 mL), and added tributyl(1-ethoxyethylene)tin (49.8 g, 138 mmol) under a nitrogen atmosphere. The reaction mixture was stirred at 120°C overnight. After the reaction was completed, the reaction mixture was cooled to room temperature and slowly poured into a 10% potassium fluoride solution (500 mL). After stirring and filtering, the filtrate was extracted with ethyl acetate (300 mL × 3). The combined organic phases were washed with saturated brine (500 mL), dried over anhydrous sodium sulfate, and filtered. The filtrate was concentrated under reduced pressure to yield Intermediate **INT-5-2** (28 g, crude product). LCMS (ESI) [M+H]⁺: 423.4.

Step 2: Intermediate **INT-5-2** (28 g, crude) was dissolved in tetrahydrofuran (300 mL), and aqueous hydrochloric acid (49.7 mL, 2 M) was added. The reaction mixture was cooled at room temperature for 1 hour. After the reaction was completed, the reaction solution was neutralized with aqueous sodium bicarbonate (200 mL). The reaction mixture was extracted with ethyl acetate (200 mL × 3). The organic phase was washed sequentially with water and saturated brine, dried over anhydrous sodium sulfate, and filtered. The filtrate was concentrated under reduced pressure to obtain a crude product, which was then separated and purified by silica gel column chromatography to yield Intermediate **INT-5-3** (17 g, 65.0%). LCMS (ESI) [M+H]⁺: 395.4.

Step 3: Intermediate **INT-5-3** (17 g, 43.1 mmol) and Intermediate **INT-1-7** (14.4 g, 51.7 mmol) were dissolved in toluene (300 mL), and cuprous iodide (8.2 g, 43.1 mmol), anhydrous potassium phosphate (36.6 g, 172.4 mmol) and N,N-dimethylethylenediamine (7.6 g, 86.2 mmol) were added in sequence. The reaction solution was stirred at 110°C overnight under a nitrogen atmosphere. After the reaction was completed, the reaction solution was cooled to room temperature, poured into ice water, and then extracted with ethyl acetate (300 mL × 3). The organic phase was washed with water and saturated brine in sequence, dried over anhydrous sodium sulfate, and filtered. The filtrate was concentrated under reduced pressure to obtain a product, which was separated and purified by silica gel column chromatography to yield Intermediate **INT-5-4** (21 g, 89.5%). LCMS (ESI) [M+H]⁺: 545.4.

Step 4: Intermediate **INT-5-4** (10 g, 18.4 mmol) and ammonium acetate (14.2 g, 183.6 mmol) were dissolved in methanol (200 mL) at room temperature. The reaction mixture was stirred at room temperature for 1 hour, then sodium cyanoborohydride (5.8 g, 91.8 mmol) was added in batches. The reaction mixture was stirred at 70°C overnight. After the reaction was completed, the reaction mixture was cooled to room temperature, poured into ice water, and extracted with dichloromethane (200 mL × 3). The organic phase was washed with water, dried over anhydrous sodium sulfate, and filtered. The filtrate was concentrated under reduced pressure to yield the crude product, which was separated and purified by silica gel column chromatography to yield Intermediate **INT-5-5** (4.3 g, 42.7%). LCMS (ESI) [M+H]⁺: 546.4.

Step 5: Intermediate **INT-5-5** (9.0 g, 16.5 mmol) was dissolved in dichloromethane (100 mL), and N,N-diisopropylethylamine (5.46 mL, 33.1 mmol) and di-tert-butyl dicarbonate (4.0 g, 18.1 mmol) were added in sequence. The reaction mixture was stirred at room temperature overnight. After the reaction was completed, the reaction mixture was poured into ice water and extracted with dichloromethane (200 mL × 3). The organic phase was washed with water, dried over anhydrous sodium sulfate, and filtered. The filtrate was concentrated under reduced pressure to yield Intermediate **INT-5-6** (12 g, crude product). LCMS (ESI) [M+H]⁺: 646.5.

Step 6: Intermediate **INT-5-6** (12 g, crude) was dissolved in methanol (100 mL), and wet palladium-on-carbon (4.0 g, 3.72 mmol) and palladium hydroxide-on-carbon (5.2 g, 10.0% wt) were added. The reaction mixture was stirred at 50°C under a hydrogen atmosphere (15 psi) for 72 hours. After the reaction was completed, the reaction mixture was filtered and the filtrate was concentrated to yield Intermediate **INT-5-7** (10 g, crude). LCMS (ESI) [M+H]⁺: 556.5.

Step 7: Intermediate **INT-5-7** (10 g, 18.0 mmol) was dissolved in dioxane (200 mL) at room temperature, and cyanomethylenetributylphosphorane (21.7 g, 90.0 mmol) was added. The reaction mixture was stirred at 120°C for 2 hours. After cooling, the reaction mixture was concentrated under reduced pressure to remove dioxane to obtain a crude product. The crude product was then separated and purified by silica gel column chromatography to yield Intermediate **INT-5-8** (9 g, 93.0%). LCMS (ESI) [M+H]⁺: 538.4.

Step 8: Intermediate **INT-5-8** (9.0 g, 16.7 mmol) was dissolved in methanol (100 mL), and a solution of hydrochloride in dioxane (41.8 mL, 4 M) was added. The reaction mixture was stirred at room temperature for 2 hours, and then concentrated under reduced pressure to yield Intermediate **INT-5** (8 g, crude product). LCMS (ESI) [M+H]⁺: 354.2.

### Preparation of Intermediate INT-6

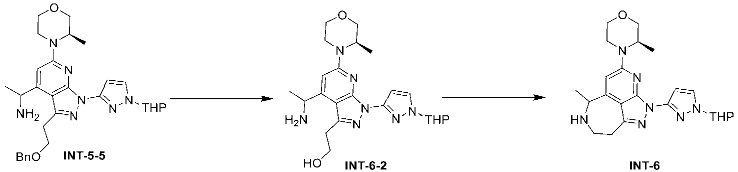

Step 1: Intermediate **INT-5-5** (7.5 g, 13.7 mmol) was dissolved in methanol (100 mL), and wet palladium-on-carbon (2.9 g, 2.75 mmol) and palladium hydroxide-on-carbon (3.9 g, 2.75 mmol) were added. The reaction mixture was stirred at 50°C under a hydrogen atmosphere (15 psi) for 48 hours. After the reaction was completed, the reaction mixture was filtered and the filtrate was concentrated to obtain a crude product, which was purified by silica gel column chromatography to yield Intermediate **INT-6-2** (6.0 g, 95.8%). LCMS (ESI) [M+H]⁺: 456.3.

Step 2: Intermediate **INT-6-2** (4.0 g, 8.8 mmol) was dissolved in dioxane (100 mL) at room temperature, and cyanomethylenetributylphosphorane (10.6 g, 43.9 mmol) was added. The reaction mixture was stirred at 120°C for 2 hours. After cooling, the reaction mixture was concentrated under reduced pressure to obtain the crude product, which was then separated and purified by silica gel column chromatography to yield Intermediate INT-6 (2.5 g, 65.1%). LCMS (ESI) [M+H]⁺: 438.2.

### Preparation of Intermediate INT-7

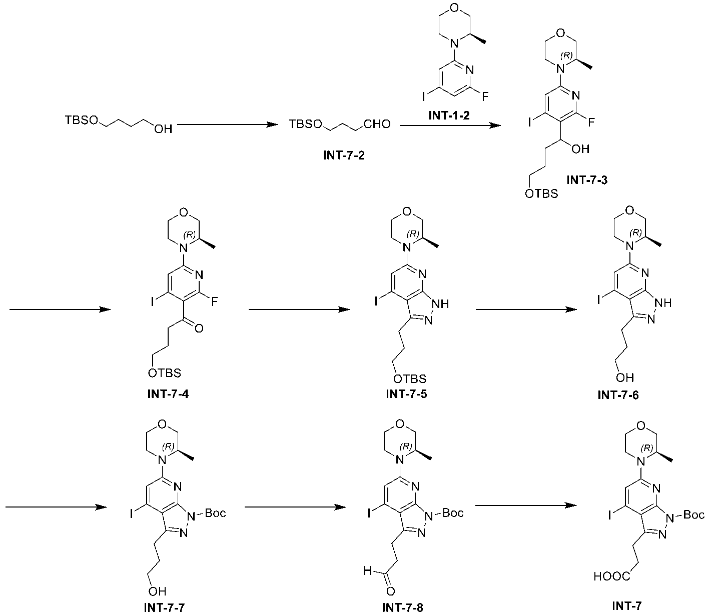

Step 1: 4-(Tert-butyldimethylsilyl)oxo-1-butanol (25 g, 122.30 mmol) was dissolved in dichloromethane (200 mL). Dess-Martin periodinane (57.1 g, 134.54 mmol) was added under a nitrogen atmosphere. The reaction mixture was stirred at room temperature for 2 hours. The reaction was quenched with sodium bicarbonate and sodium thiosulfate (500 mL, 1:1). The organic phase was separated, and the aqueous phase was extracted with dichloromethane (100 mL × 3). The organic phase was washed sequentially with water and saturated brine, dried over anhydrous sodium sulfate, filtered, and concentrated to obtain a crude product. The crude product was purified by silica gel column chromatography to yield Intermediate **INT-7-2** (23.0 g, 92.9%). ¹H NMR (400 MHz, CDCl₃) δ 9.79 (s, 1H), 3.85 - 3.48 (m, 2H), 2.50 (m, 2H), 2.03 - 1.74 (m, 2H), 0.89 (s, 9H), 0.04 (s, 6H).

Step 2: Under a nitrogen atmosphere, Intermediate **INT-1-2** (20 g, 62.09 mmol) was dissolved in tetrahydrofuran (200 mL), cooled to -70°C, added dropwise with lithium diisopropylamide (62.09 mL, 2M in THF), and stirred at -70°C for 1 hour. A solution of Intermediate **INT-7-2** (15.1 g, 74.51 mmol) in tetrahydrofuran (50 mL) was added dropwise to the reaction mixture. After the addition was completed, the reaction mixture was stirred continuously for 1 hour. The reaction was quenched with ammonium chloride solution (500 mL), and the aqueous phase was extracted with ethyl acetate (100 mL × 3). The organic phase was washed sequentially with water and saturated brine, dried over anhydrous sodium sulfate, filtered, and concentrated to obtain a crude product. The crude product was purified by silica gel column chromatography to yield Intermediate **INT-7-3** (22.0 g, 67.6%). LCMS (ESI) [M+Na]⁺: 546.9.

Step 3: Under a nitrogen atmosphere, Intermediate **INT-7-3** (20 g, 38.12 mmol) was dissolved in dichloromethane (200 mL), and Dess-Martin periodinane (26.0 g, 57.18 mmol) was added. The reaction mixture was stirred at room temperature for 2 hours. The reaction was quenched by adding water. The reaction was quenched with sodium bicarbonate and sodium thiosulfate (500 mL, 1:1). The resulting organic phase was separated and the aqueous phase was extracted with dichloromethane (100 mL × 3). The organic phase was washed sequentially with water and saturated brine, dried over anhydrous sodium sulfate, filtered, and concentrated to obtain a crude product. The crude product was purified by silica gel column chromatography to yield Intermediate **INT-7-4** (14.0 g, 70.3%). LCMS (ESI) [M+H]⁺: 523.2.

Step 4: Under a nitrogen atmosphere, Intermediate **INT-7-4** (6.0 g, 11.48 mmol) was dissolved in ethanol (60 mL), and hydrazine hydrate (5.8 g, 114.8 mmol) was added. The reaction mixture was stirred at 80°C for 2 hours. The reaction was quenched by adding water. The resulting organic phase was separated and the aqueous phase was extracted with ethyl acetate (50 mL × 3). The organic phase was washed sequentially with water and saturated brine, dried over anhydrous sodium sulfate, filtered, and concentrated to obtain a crude product. The crude product was purified by silica gel column chromatography to yield Intermediate **INT-7-5** (900 mg, 15.6%). LCMS (ESI) [M+H]⁺: 517.4.

Step 5: Under a nitrogen atmosphere, Intermediate **INT-7-5** (670 mg, 1.30 mmol) was dissolved in tetrahydrofuran (10 mL), and a methanolic hydrochloric acid solution (6 M, 5 mL) was added, and the reaction mixture was stirred at 50°C for 1 hour. The reaction was quenched by adding water at room temperature. The resulting organic phase was separated, and the aqueous phase was extracted with dichloromethane (20 mL × 3). The organic phase was washed sequentially with water and saturated brine, dried over anhydrous sodium sulfate, filtered, and concentrated to obtain a crude product. The crude product was separated and purified by silica gel column chromatography to yield Intermediate **INT-7-6** (400 mg, 73.7%). LCMS (ESI) [M+H]⁺: 403.2.

Step 6: Under a nitrogen atmosphere, Intermediate **INT-7-6** (500 mg, 1.24 mmol) was dissolved in dichloromethane (10 mL), and di-tert-butyl dicarbonate (0.43 mL, 1.87 mmol) and triethylamine (0.52 mL, 3.73 mmol) were added. The reaction mixture was stirred at room temperature for 2 hours. The reaction was quenched by adding water. The resulting organic phase was separated and the aqueous phase was extracted with dichloromethane (10 mL × 3). The organic phase was washed sequentially with water and saturated brine, dried over anhydrous sodium sulfate, filtered, and concentrated to obtain a crude product. The crude product was separated and purified by silica gel column chromatography to yield Intermediate **INT-7-7** (500 mg, 80.1%). LCMS (ESI) [M+H]⁺: 503.2.

Step 7: Under a nitrogen atmosphere, Intermediate **INT-7-7** (500 mg, 1.00 mmol) was dissolved in dichloromethane (10 mL), and Dess-Martin periodinane (506.4 mg, 1.19 mmol) was added. The reaction mixture was stirred at room temperature for half an hour. The reaction was quenched by adding water. The resulting organic phase was separated, and the aqueous phase was extracted with dichloromethane (10 mL × 3). The organic phase was washed sequentially with water and saturated brine, dried over anhydrous sodium sulfate, filtered, and concentrated to obtain a crude product. The crude product was separated and purified by silica gel column chromatography to yield Intermediate **INT-7-8** (390 mg, 78.3%). LCMS (ESI) [M+Na]⁺: 523.1.

Step 8: Under a nitrogen atmosphere, Intermediate **INT-7-8** (390 mg, 0.78 mmol) was dissolved in acetonitrile (10 mL), and 2,2,6,6-tetramethylpiperidin-1-oxyl (243.2 mg, 1.56 mmol) and iodophenyldiacetic acid (505.3 mg, 1.56 mmol) were added. The reaction mixture was stirred at room temperature for 2 hours. The reaction was quenched by adding water. The resulting organic phase was separated and the aqueous phase is extracted with dichloromethane (10 mL × 3). The organic phase was washed sequentially with water and saturated brine, dried over anhydrous sodium sulfate, filtered, and concentrated to obtain a crude product. The crude product was purified by silica gel column chromatography to yield Intermediate **INT-7** (360 mg, 89.4%). LCMS (ESI) [M+H]⁺: 517.2.

### Example 1: Preparation of (R)-4-(2-(1H-pyrazol-3-yl)-6,7,8,9-tetrahydro-2H-1,2,3,7-tetraazabenzo[cd]azulen-4-yl)-3-methylmorpholine (Compound 1)

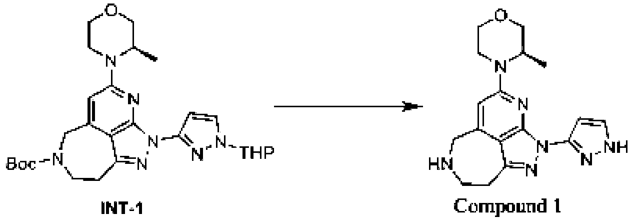

Intermediate INT-1 (300 mg, 0.57 mmol) was dissolved in methanol (2 mL), and a solution of hydrochloride in dioxane (2 mL, 4 M) was added. The reaction mixture was stirred at room temperature for 1 hour, and then concentrated under reduced pressure to obtain a crude product. The crude product was separated and purified by preparative reverse-phase high-performance liquid chromatography to yield (*R*)-4-(2-(1*H*-pyrazol-3-yl)-6,7,8,9-tetrahydro-2*H*-1,2,3,7-tetraazabenzo[*cd*]azulen-4-yl)-3-methylmorpholine (Compound 1) (80.0 mg, 41.2%). LCMS (ESI) [M+H]⁺ : 340.1. ¹H NMR (400 MHz, CD₃OD) δ 7.76 (d, *J* = 2.0 Hz, 1H), 6.95 (d, *J* = 2.3 Hz, 1H), 6.69 (s, 1H), 4.52 (s, 3H), 4.12 (dd, *J =* 2.1, 13.2 Hz, 1H), 4.05 (dd, *J =* 3.8, 11.4 Hz, 1H), 3.89 - 3.82 (m, 1H), 3.82 - 3.75 (m, 1H), 3.65 - 3.56 (m, 3H), 3.36 (d, *J* = 3.9 Hz, 1H), 3.30 (s, 2H), 1.32 (d, *J* = 6.6 Hz, 3H).

### Example 2: Preparation of (R)-4-(7-isopropyl-2-(1H-pyrazol-3-yl)-6,7,8,9-tetrahydro-2H-1,2,3,7-tetraazabenzo[cd]azulen-4-yl)-3-methylmorpholine carboxylate (Compound 2)

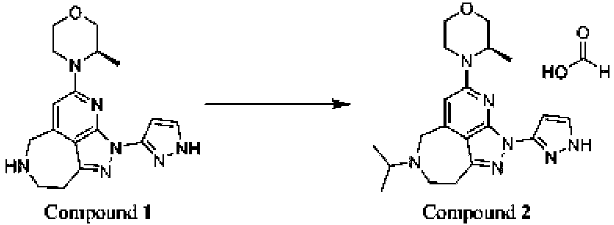

Compound 1 (200 mg, 0.589 mmol) was dissolved in methanol (3 mL). N,N-diisopropylethylamine (0.5 mL) was added, and then the reaction was stirred at room temperature for 20 minutes. Acetone (0.217 mL, 2.95 mmol) and sodium triacetoxyborohydride (624 mg, 2.95 mmol) were then added sequentially, and the reaction mixture was stirred at room temperature for 2 hours. The crude product was concentrated under reduced pressure and purified by preparative reverse-phase high performance liquid chromatography to yield (*R*)-4-(7-isopropyl-2-(1*H*-pyrazol-3-yl)-6,7,8,9-tetrahydro-2*H*-1,2,3,7-tetraazabenzo[*cd*]azulen-4-yl)-3-methylmorpholine carboxylate (Compound 2) (55.0 mg, 24.4%). LCMS (ESI) [M+H]⁺: 382.1. ¹H NMR (400 MHz, CD₃OD) δ 8.31 (s, 1H), 7.73 (d, *J =* 2.0 Hz, 1H), 6.93 (d, *J =* 2.0 Hz, 1H), 6.66 (s, 1H), 4.54 - 4.42 (m, 3H), 4.14 - 3.98 (m, 2H), 3.86 - 3.73 (m, 2H), 3.62 (dt, *J* = 2.9, 11.8 Hz, 1H), 3.57 - 3.44 (m, 3H), 3.26 - 3.18 (m, 2H), 1.33 (d, *J =* 6.6 Hz, 6H), 1.30 (d, *J =* 6.6 Hz, 4H).

### Example 3: Preparation of (R)-4-(2-(1H-pyrazol-3-yl)-7-(tetrahydro-2H-pyran-4-yl)-6,7,8,9-tetrahydro-2H-1,2,3,7-tetraazabenzo[cd]azulen-4-yl)-3-methylmorpholine (Compound 3)

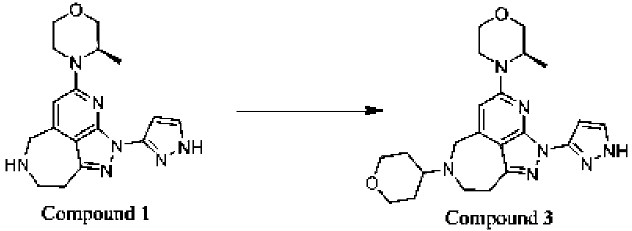

Similar to the preparation method of Example 2, the title Compound 3 was prepared using tetrahydropyran-4-one instead of acetone. (*R*)-4-(2-(1*H*-Pyrazol-3-yl)-7-(tetrahydro-2*H*-pyran-4-yl)-6,7,8,9-tetrahydro-2*H-*1,2,3,7-tetraazabenzo[*cd*]azulen-4-yl)-3-methylmorpholine (Compound 3) (6.20 mg, 24.8%) was obtained. LCMS (ESI) [M+H]⁺ : 424.1. ¹H NMR (400 MHz, CD₃OD) δ 7.74 (d, *J =* 1.5 Hz, 1H), 6.93 (s, 1H), 6.60 (s, 1H), 4.52 (d, *J =* 4.8 Hz, 1H), 4.28 (s, 2H), 4.15 - 3.94 (m, 4H), 3.87 - 3.74 (m, 2H), 3.63 (dt, *J =* 2.6, 11.8 Hz, 1H), 3.45 - 3.35 (m, 4H), 3.29 - 3.20 (m, 1H), 3.13 (s, 2H), 3.00 (t, *J =* 11.0 Hz, 1H), 1.87 (d, *J =* 12.8 Hz, 2H), 1.77 - 1.60 (m, 2H), 1.30 (d, *J =* 6.6 Hz, 3H).

### Example 4: Preparation of diastereomeric mixture of (3R)-4-(2-(1H-pyrazol-3-yl)-7-(tetrahydrofuran-3-yl)-6,7,8,9-tetrahydro-2H-1,2,3,7-tetraazabenz o[cd]azulen-4-yl)-3-methylmorpholine (Compound 4)

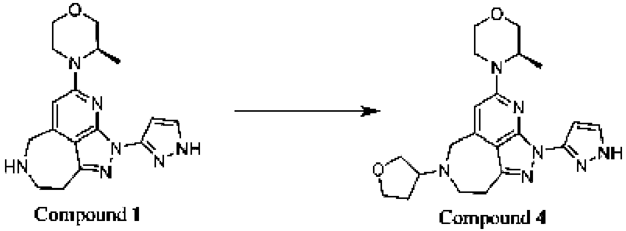

Similar to the preparation method of Example 2, the title compound 4 was prepared using 3-tetrahydrofuranone instead of acetone. A diastereomeric mixture of (3R)-4-(2-(1*H*-pyrazol-3-yl)-7-(tetrahydrofuran-3-yl)-6,7,8,9-tetrahydro-2*H*-1,2,3,7-tetraazabenz o[*cd*]azulen-4-yl)-3-methylmorpholine (Compound **4**) (23.2 mg, 63.2%) was obtained. LCMS (ESI) [M+H]⁺ : 410.2. ¹H NMR (400 MHz, CD₃OD) δ 7.73 (br s, 1H), 6.93 (s, 1H), 6.59 (s, 1H), 4.52 (br d, *J* = 5.4 Hz, 1H), 4.18 (s, 2H), 4.10 (br d, *J =* 12.5 Hz, 1H), 4.06 - 3.98 (m, 2H), 3.95 - 3.89 (m, 1H), 3.87 - 3.81 (m, 1H), 3.81 - 3.73 (m, 3H), 3.70 - 3.59 (m, 2H), 3.32 - 3.30 (m, 2H), 3.28 (br d, *J =* 5.4 Hz, 1H), 3.16 (br d, *J* = 5.5 Hz, 2H), 2.20 (dtd, *J =* 3.9, 7.8, 11.9 Hz, 1H), 2.06 - 1.93 (m, 1H), 1.31 (d, *J =* 6.6 Hz, 3H).

### Example 5: Preparation of (R)-1-(4-(3-methylmorpholino)-2-(1H-pyrazol-3-yl)-2,6,8,9-tetrahydro-7H-1,2,3,7-tetraazabenzo[cd]azulen-4-yl)ethan-1-one (Compound 5)

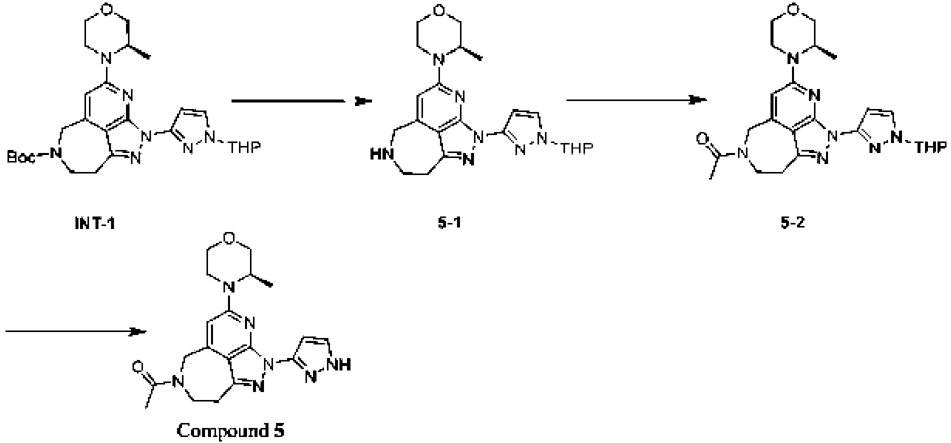

Step 1: Intermediate **INT-1** (300 mg, 0.573 mmol) was dissolved in trifluoroethanol (2 mL), and added dropwise with trimethylsilyl chloride (0.073 mL, 0.573 mmol). The reaction mixture was stirred at room temperature for 1 hour, and concentrated under reduced pressure to obtain a crude product, which was separated and purified by preparative reverse-phase high performance liquid chromatography to yield Intermediate 5-1 (100 mg, 41.2%). LCMS (ESI) [M+H]⁺: 424.2.

Step 2: Intermediate 5-1 (50 mg, 0.118 mmol) and triethylamine (49 µL, 0.354 mmol) were dissolved in dichloromethane (1 mL), and added dropwise with acetyl chloride (17 µL, 0.236 mmol) in an ice bath under nitrogen atmosphere. The reaction mixture was stirred in an ice bath for 1 hour, and concentrated under reduced pressure to obtain a crude product of Intermediate 5-2, which was used directly in the next reaction. LCMS (ESI) [M+H]⁺: 466.2.

Step 3: Intermediate 5-2 (50 mg, 0.107 mmol) was dissolved in methanol (2 mL), and a dioxane solution of hydrochloride (2 mL, 1 M) was added. The reaction solution was stirred at room temperature for 4 hours, and then concentrated under reduced pressure to obtain a crude product, which was separated and purified by preparative reverse-phase high performance liquid chromatography to give (*R*)-1-(4-(3-methylmorpholino)-2-(1*H*-pyrazol-3-yl)-2,6,8,9-tetrahydro-7*H*-1,2,3,7-tetraazabenzo[*cd*]azulen-4-yl)ethan-1-one (Compound 5) (14.7 mg, 33.2%). LCMS (ESI) [M+H]⁺ : 382.2. ¹H NMR (400 MHz, CD₃OD) δ 7.72 (s, 1H), 6.91 (s, 1H), 6.73 - 6.63 (m, 1H), 4.99 (d, *J* = 4.8 Hz, 1H), 4.93 (d, *J =* 4.5 Hz, 2H), 4.61 - 4.45 (m, 1H), 4.16 - 3.93 (m, 4H), 3.87 - 3.72 (m, 2H), 3.62 (t, *J =* 11.6 Hz, 1H), 3.26 (d, *J =* 5.5 Hz, 3H), 2.26 (d, *J =* 1.9 Hz, 1H), 2.12 (d, *J =* 1.3 Hz, 2H), 1.34 - 1.26 (m, 3H).

### Example 6: Preparation of a diastereomeric mixture of (3R)-4-(7-(8-oxabicyclo[3.2.1]octan-3-yl)-2-(1H-pyrazol-3-yl)-6,7,8,9-tetrahydro-2H-1,2,3,7-tetr aazabenzo[cd]azulen-4-yl)-3-methylmorpholine (Compound 6)

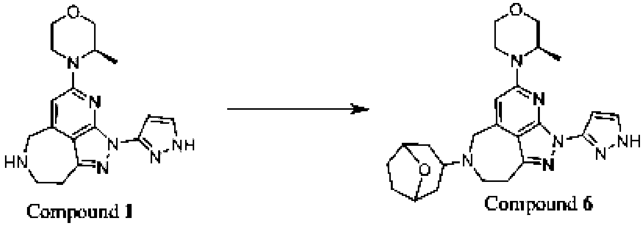

Similar to the preparation method of Example 2, the title Compound 6 was prepared using 8-oxabicyclo[3.2.1]octan-3-one instead of acetone. (3*R*)-4-(7-(8-Oxabicyclo[3.2.1]octan-3-yl)-2-(1*H*-pyrazol-3-yl)-6,7,8,9-tetrahydro-2*H*-1,2,3,7-tetraazabenzo[*cd*]azulen-4-yl)-3-methylmorp holine (Compound 6) (4.47 mg, 10.9%) was obtained. LCMS (ESI) [M+H]⁺: 450.2. ¹H NMR (400 MHz, CD₃OD) δ 7.73 (d, *J =* 1.5 Hz, 1H), 6.93 (d, *J =* 1.6 Hz, 1H), 6.59 (s, 1H), 4.56 - 4.49 (m, 1H), 4.47 (s, 2H), 4.22 (s, 2H), 4.14 - 4.00 (m, 2H), 3.88 - 3.75 (m, 2H), 3.63 (dt, *J =* 3.0, 11.8 Hz, 1H), 3.32 - 3.30 (m, 1H), 3.30 - 3.22 (m, 3H), 3.13 - 3.06 (m, 2H), 1.98 - 1.88 (m, 2H), 1.84 - 1.71 (m, 6H), 1.30 (d, *J =* 6.6 Hz, 3H).

### Example 7: Preparation of (R)-4-(7-(3,3-difluorocyclobutyl)-2-(1H-pyrazol-3-yl)-6,7,8,9-tetrahydro-2H-1,2,3,7-tetraazabenzo[cd]azulen-4-yl)-3-methylmorpholine (Compound 7)

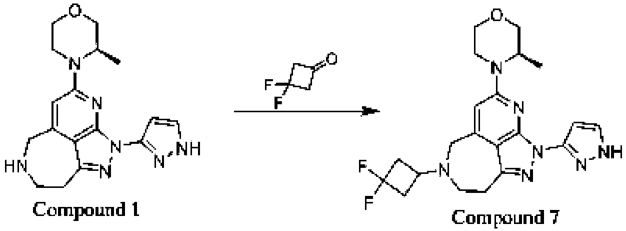

Similar to the preparation method of Example 2, the title Compound 7 was prepared using 3,3-difluorocyclobutyl-1-one instead of acetone. (*R*)-4-(7-(3,3-Difluorocyclobutyl)-2-(1*H*-pyrazol-3-yl)-6,7,8,9-tetrahydro-2*H*-1,2,3,7-tetraazabenzo[*cd*]azulen-4-yl)-3-methylmorpho line (Compound 7) (6.26 mg, 22.9%) was obtained. LCMS (ESI) [M+H]⁺: 430.1. ¹H NMR (400 MHz, CD₃OD) δ 7.61 (s, 1H), 6.81 (s, 1H), 6.46 (s, 1H), 4.51 - 4.35 (m, 2H), 4.01 - 3.88 (m, 4H), 3.77 - 3.62 (m, 2H), 3.51 (dt, *J* = 3.0, 11.8 Hz, 1H), 3.19 - 3.15 (m, 1H), 3.14 - 3.07 (m, 2H), 3.05 - 2.98 (m, 2H), 2.67 (ddt, *J =* 3.1, 7.3, 13.8 Hz, 2H), 2.49 - 2.32 (m, 2H), 1.19 (d, *J =* 6.8 Hz, 3H).

### Example 8: Preparation of (R)-3-methyl-4-(7-(oxetan-3-yl)-2-(1H-pyrazol-3-yl)-6,7,8,9-tetrahydro-2H-1,2,3,7-tetraazabenzo[cd]azulen-4-yl)morpholine (Compound 8)

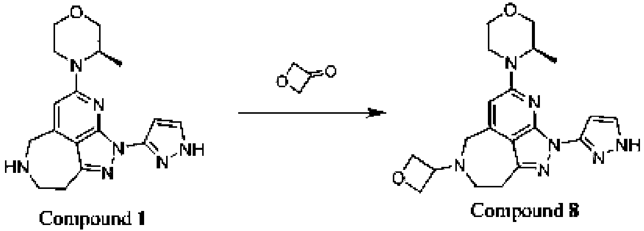

Similar to the preparation method of Example 2, the title Compound 8 was prepared using 3-oxetanone instead of acetone. (*R*)-3-Methyl-4-(7-(oxetan-3-yl)-2-(1*H*-pyrazol-3-yl)-6,7,8,9-tetrahydro-2*H*-1,2,3,7-tetraazabenzo[*cd*]azulen-4-yl)morpholine (Compound 8) (46.5 mg, 39.9%) was obtained. LCMS (ESI) [M+H]⁺: 396.2. ¹H NMR (400 MHz, CDCl₃) δ 7.62 (d, *J =* 2.1 Hz, 1H), 6.85 (s, 1H), 6.30 (s, 1H), 4.69 (dt, *J =* 16.3, 6.3 Hz, 4H), 4.38 (d, *J =* 6.8 Hz, 1H), 4.14 (d, *J* = 6.6 Hz, 1H), 4.09 - 3.95 (m, 4H), 3.80 (dt, *J =* 11.4, 7.1 Hz, 2H), 3.64 (td, *J =* 11.9, 3.0 Hz, 1H), 3.34 (td, *J =* 12.7, 3.9 Hz, 1H), 3.17 (dd, *J =* 10.0, 3.5 Hz, 4H), 1.32 (d, *J =* 6.8 Hz, 3H).

### Example 9: Preparation of (R)-(3,3-difluorocyclobutyl)(4-(3-methylmorpholino)-2-(1H-pyrazol-3-yl)-2,6,8,9-tetrahydro-7H-1,2,3,7-tetraazabenzo[cd]azulene-7-yl)methanone (Compound 9)

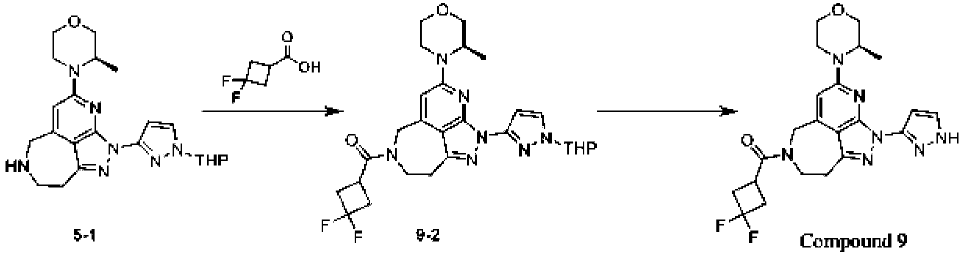

Step 1: 3,3-Difluorocyclobutanecarboxylic acid (6 mg, 0.047 mmol) was dissolved in *N,N*-dimethylformamide (2 mL), and *N,N*-diisopropylethylamine (18 mg, 0.141 mmol), *O*-(7-azabenzotriazol-1-yl)-*N,N,N',N'*-tetramethyluronium hexafluorophosphate (27 mg, 0.071 mmol), and Intermediate 5-1 (20 mg, 0.047 mmol) were added in sequence. The reaction mixture was stirred at room temperature for 16 hours, diluted by adding ice water (10 mL), and extracted with ethyl acetate (10 mL × 3). The combined organic phases were washed with saturated brine (20 mL), dried over anhydrous sodium sulfate, and filtered. The filtrate was concentrated under reduced pressure to remove the organic solvent to yield a crude product. The crude product was purified by silica gel chromatography to yield Intermediate 9-2 (10 mg, 39.1%). LCMS (ESI) [M+H]⁺: 542.3.

Step 2: Intermediate 9-2 (10 mg) was dissolved in methanol (2 mL), and a solution of 4 M hydrochloric acid in 1,4-dioxane (0.5 mL) was added. The reaction mixture was stirred at room temperature for 18 hours. The reaction solution was concentrated under reduced pressure, and the resulting crude product was separated and purified by preparative reverse-phase high performance liquid chromatography to give (*R*)-(3,3-difluorocyclobutyl)(4-(3-methylmorpholino)-2-(1*H*-pyrazol-3-yl)-2,6,8,9-tetrahydro-7*H*-1,2,3,7-tetraazabenzo[*cd*]azulene-7-yl)methanone (Compound **9**) (4 mg, 47.3%). LCMS (ESI) [M+H]⁺ : 458.3. ¹H NMR (400 MHz, DMSO-d6) δ 12.7 (s, 1H), 7.82 (s, 1H), 6.81 - 6.76 (m, 2H), 4.95 - 4.87 (m, 2H), 4.53 - 4.49 (m, 1H), 4.01 - 3.98 (m, 4H), 3.92 - 3.77 (m, 2H), 3.56 - 3.45 (m, 1H), 3.18 - 3.12 (m, 4H), 2.78 - 2.50 (m, 4H), 1.24 - 1.17 (m, 3H).

### Example 10: Preparation of (R)-3,3,3-trifluoro-1-(4-(3-methylmorpholino)-2-(1H-pyrazol-3-yl)-2,6,8,9-tetrahydro-7H-1,2,3,7-tetraazabenzo[cd]azulen-7-yl)propan-1-one (Compound 10)

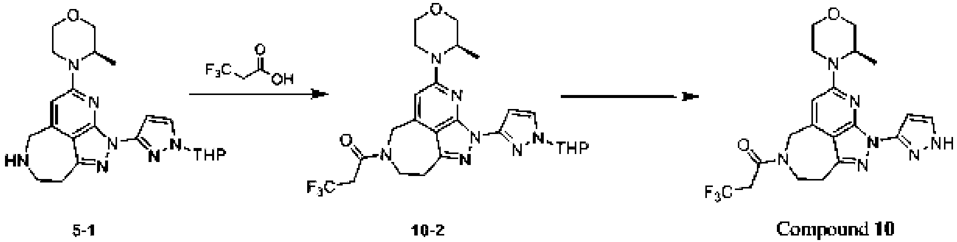

Similar to the preparation method of Example 9, the title Compound 10 was prepared using 3,3,3-trifluoropropionic acid instead of 3,3-difluorocyclobutanecarboxylic acid. (*R*)-3,3,3-Trifluoro-1-(4-(3-methylmorpholino)-2-(1*H*-pyrazol-3-yl)-2,6,8,9-tetrahydro-7*H*-1,2,3, 7-tetraazabenzo[*cd*]azulen-7-yl)propan-1-one (Compound 10) (6.9 mg, 32.8%) was obtained. LCMS (ESI) [M+H]⁺: 450.4. ¹H NMR (400 MHz, CD₃OD) δ 7.74 (br s, 1H), 6.93 (br d, *J =* 4.3 Hz, 1H), 6.71 (d, *J =* 4.1 Hz, 1H), 5.08 - 4.95 (m, 2H), 4.61 - 4.47 (m, 1H), 4.23 - 3.99 (m, 4H), 3.87 - 3.49 (m, 5H), 3.32 - 3.25 (m, 3H), 1.38 - 1.23 (m, 3H).

### Example 11: Preparation of (R)-4-(2-(1H-pyrazol-3-yl)-7-(spiro[2.3]hexan-5-yl)-6,7,8,9-tetrahydro-2H-benzo[cd]azulen-4-yl)-3-methylmorpholine (Compound 11)

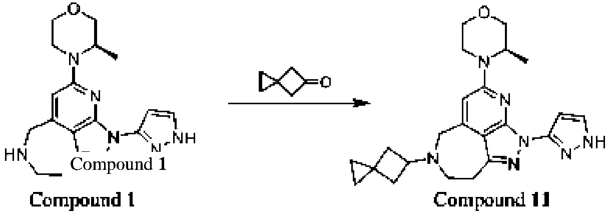

Similar to the preparation method of Example 2, the title Compound 11 was prepared using spiro[2.3]hexan-5-one instead of acetone. (*R*)-4-(2-(1*H*-Pyrazol-3-yl)-7-(spiro[2.3]hexan-5-yl)-6,7,8,9-tetrahydro-2*H*-benzo[*cd*]azulen-4-yl)-3-methylmorpholine (Compound 11) (26.5 mg, 21.4%) was obtained. LCMS (ESI) [M+H]⁺: 420.3. ¹H NMR (400 MHz, CDCl₃) δ 7.61 (d, *J =* 2.1 Hz, 1H), 6.83 (s, 1H), 6.31 (s, 1H), 4.38 (d, *J =* 6.3 Hz, 1H), 4.13 - 3.95 (m, 4H), 3.81 (dt, *J* = 11.5, 7.2 Hz, 2H), 3.64 (td, *J* = 11.9, 3.0 Hz, 2H), 3.40 - 3.14 (m, 5H), 2.29 (d, *J* = 54.0 Hz, 2H), 2.05 (dd, *J* = 11.6, 7.6 Hz, 3H), 1.31 (d, *J* = 6.7 Hz, 4H), 0.54 - 0.36 (m, 4H).

### Example 12: Preparation of diastereomeric mixture of (3R)-3-methyl-4-(7-(4-methyltetrahydrofuran-3-yl)-2-(1H-pyrazol-3-yl)-6,7,8,9-tetrahydro-2H-1 ,2,3,7-tetraazabenzo[cd]azulen-4-yl)morpholine (Compound 12)

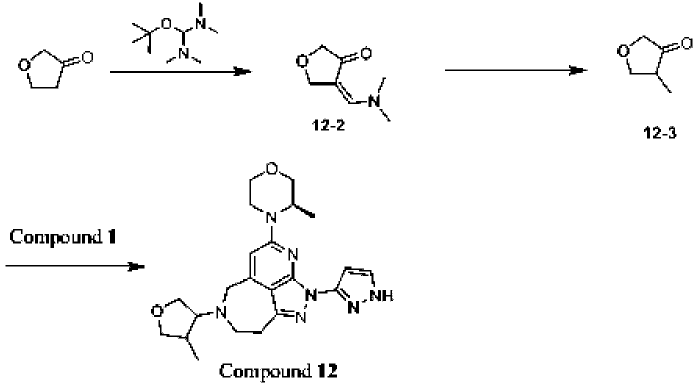

Step 1: 3-Tetrahydrofuranone (11.4 g, 132 mmol) and tert-butoxybis(dimethylamino)methane (23.1 g, 132 mmol) were dissolved in toluene (130 mL). The reaction mixture was stirred at room temperature for 12 hours. The reaction mixture was concentrated to yield Intermediate 12-2 (17.0 g, 90.9%). LCMS (ESI) [M+H]⁺: 141.9.

Step 2: Intermediate 12-2 (17.0 g, 120 mmol) was dissolved in acetone (170 mL), and wet palladium-on-carbon (1.70 g, 15.9 mmol) was added. The reaction mixture was stirred under a hydrogen atmosphere at room temperature for 12 hours. The reaction mixture was filtered, and the filtrate was concentrated to obtain a crude product, which was then distilled to obtain Intermediate 12-3 (200 mg, 1.70%). ¹H NMR (400 MHz, CDCl₃) δ 4.45 (t, *J* = 8.88 Hz, 1 H), 4.04 (d, *J =* 17.13 Hz, 1 H), 3.80 (d, *J =* 17.13 Hz, 1 H), 3.68 (t, *J =* 9.26 Hz, 1 H), 2.57 - 2.45 (m, 1 H), 1.12 (d, *J* = 7.13 Hz, 3 H).

Step 3: Similar to the preparation method of Example 2, the Intermediate 12-3 was used instead of acetone to prepare the title Compound 12. (3*R*)-3-Methyl-4-(7-(4-methyltetrahydrofuran-3-yl)-2-(1*H*-pyrazol-3-yl)-6,7,8,9-tetrahydro-2*H*-1 ,2,3,7-tetraazabenzo[*cd*]azulen-4-yl)morpholine (Compound 12) (3.6 mg, 5.6%) was obtained. LCMS (ESI) [M+H]⁺: 424.3. ¹H NMR (400 MHz, CD₃OD) δ 7.72 (s, 1 H), 6.91 (s, 1 H), 6.55 (s, 1 H), 4.51 (d, *J* = 6.38 Hz, 1 H), 4.18 - 3.89 (m, 6 H), 3.86 - 3.73 (m, 3 H), 3.67 - 3.48 (m, 3 H), 3.36 - 3.33 (m, 1 H), 3.30 - 3.22 (m, 2 H), 3.21 - 3.12 (m, 2 H), 2.50 - 2.36 (m, 1 H), 1.29 (d, *J* = 6.63 Hz, 3 H), 1.07 (d, *J =* 6.88 Hz, 3 H).

### Example 13A and Example 13B: Preparation of (R)-4-(2-(1H-pyrazol-3-yl)-7-(3-(trifluoromethyl)cyclobutyl)-6,7,8,9-tetrahydro-2H-1,2,3,7-tetra azabenzo[cd]azulen-4-yl)-3-methylmorpholine (Compound 13A, Isomer 1) and (R)-4-(2-(1H-pyrazol-3-yl)-7-(3-(trifluoromethyl)cyclobutyl)-6,7,8,9-tetrahydro-2H-1,2,3,7-tetra azabenzo[cd]azulen-4-yl)-3-methylmorpholine (Compound 13B, Isomer 2)

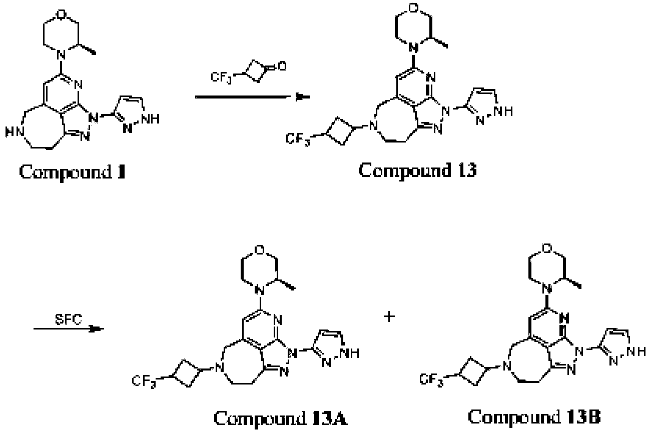

Step 1: Similar to the preparation method of Example 2, the title Compound 13 was prepared using 3-(trifluoromethyl)cyclobutan-1-one instead of acetone. A diastereomeric mixture of (*R*)-4-(2-(1*H*-pyrazol-3-yl)-7-(3-(trifluoromethyl)cyclobutyl)-6,7,8,9-tetrahydro-2H-1,2,3,7-tetraazabenzo[*cd*]azulen-4-yl)-3-methylmorpholine (Compound 13) (20.0 mg, 14.7%) was obtained. LCMS (ESI) [M+H]⁺: 462.4.

Step 2: The diastereomeric mixture of (*R*)-4-(2-(1*H*-pyrazol-3-yl)-7-(3-(trifluoromethyl) cyclobutyl)-6,7,8,9-tetrahydro-2*H*-1,2,3,7-tetraazabenzo[*cd*]azulen-4-yl)-3-methylmorpholine (Compound 13) (20.0 mg) was further purified by chiral SFC. SFC analysis: Daicel Chiral PAK IB column (100 × 3.0 mm, 3 µm), mobile phase: 20% methanol (containing 0.05% ammonia) in CO₂, temperature: 35°C, flow rate: 2.0 mL/min, detection wavelength: 210 nm.

The first peak was (*R*)-4-(2-(1*H*-pyrazol-3-yl)-7-(3-(trifluoromethyl)cyclobutyl)-6,7,8,9-tetrahydro-2*H*-1,2,3,7-tetraazabenzo[*cd*]azulen-4-yl)-3-methylmorpholine (Compound 13A, Isomer 1) (1.6 mg). LCMS (ESI) [M+H]⁺: 462.4. HPLC-SFC: Rt = 2.812 min. ¹H NMR (400 MHz, CDCl₃) δ 7.62 (s, 1H), 6.95 (d, *J* = 40.0 Hz, 1H), 6.28 (s, 1H), 4.38 (d, *J* = 5.4 Hz, 1H), 4.02 (dd, *J =* 25.8, 11.8 Hz, 4H), 3.81 (dd, *J =* 22.8, 11.0 Hz, 2H), 3.63 (t, *J =* 11.2 Hz, 1H), 3.39 - 3.22 (m, 2H), 3.04 (d, *J* = 55.5 Hz, 4H), 2.52 (dt, *J* = 17.2, 8.6 Hz, 1H), 2.34 - 2.05 (m, 4H), 1.44 - 1.26 (m, 4H).

The second peak was (*R*)-4-(2-(1*H*-pyrazol-3-yl)-7-(3-(trifluoromethyl)cyclobutyl)-6,7,8,9-tetrahydro-2*H*-1,2,3,7-tetraazabenzo[*cd*]azulen-4-yl)-3-methylmorpholine (Compound 13B, Isomer 2) (2.6 mg). LCMS (ESI) [M+H]⁺: 462.4. HPLC-SFC: Rt = 3.253 min. ¹H NMR (400 MHz, CDCl₃) δ 7.63 (d, *J =* 1.8 Hz, 1H), 6.87 (s, 1H), 6.29 (s, 1H), 4.38 (d, *J =* 6.0 Hz, 1H), 4.12 - 3.94 (m, 4H), 3.84 - 3.75 (m, 2H), 3.64 (m, 1H), 3.31 (dd, *J =* 15.2, 6.4 Hz, 2H), 3.17 - 3.02 (m, 3H), 2.55 (td, *J* = 17.6, 8.8 Hz, 1H), 2.32 - 2.21 (m, 2H), 2.14 - 1.96 (m, 2H), 1.45 - 1.25 (m, 4H).

### Example 14: Preparation of (R)-4-(2-(1H-pyrazol-3-yl)-7-(2,2,2-trifluoroethyl)-6,7,8,9-tetrahydro-2H-1,2,3,7-tetraazabenzo[cd]azulen-4-yl)-3-methylmorpholine (Compound 14)

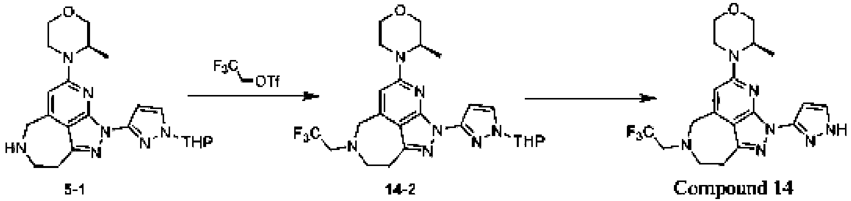

Step 1: Intermediate 5-1 (50.0 mg, 0.118 mmol) was dissolved in toluene (2.0 mL), and N,N-diisopropylethylamine (31.0 mg, 0.236 mmol) and trifluoroethyl trifluoromethanesulfonate (55.0 mg, 0.236 mmol) were added in sequence. The reaction mixture was stirred at 100°C for 18 hours. The reaction mixture was cooled to room temperature, quenched by adding ice-cold water, and extracted with ethyl acetate (20 mL × 3). The combined organic phases were washed sequentially with water and saturated brine, dried over anhydrous sodium sulfate, filtered, and concentrated to obtain a crude product, which was purified by silica gel column chromatography to yield Intermediate 14-2 (30.0 mg, 50.3%). LCMS (ESI) [M+H]⁺: 506.4.

Step 2: 14-2 (30.0 mg, 0.059 mmol) was dissolved in methanol (3.0 mL), and a solution of hydrochloric acid in 1,4-dioxane (1.0 mL, 4 M) was added. The reaction mixture was stirred at room temperature for 3 hours. The reaction solution was concentrated under reduced pressure to obtain a crude product, which was then separated and purified by preparative reverse-phase high performance liquid chromatography to give (*R*)-4-(2-(1*H*-pyrazol-3-yl)-7-(2,2,2-trifluoroethyl)-6,7,8,9-tetrahydro-2*H*-1,2,3,7-tetraazabenzo[*cd*]azulen-4-yl)-3-methylmorpholine (Compound 14) (8.9 mg, 35.8%). LCMS (ESI) [M+H]⁺ : 422.4. ¹H NMR (400 MHz, CD₃OD) δ 7.73 (d, *J* = 1.9 Hz, 1H), 6.92 (d, *J =* 1.9 Hz, 1H), 6.53 (, 1H), 4.58 - 4.43 (m, 1H), 4.34 (s, 2H), 4.13 - 3.96 (m, 2H), 3.86 - 3.70 (m, 2H), 3.61 (dt, *J* = 3.0, 11.8 Hz, 1H), 3.49 - 3.37 (m, 4H), 3.30 - 3.24 (m, 2H), 3.18 (t, *J* = 5.6 Hz, 2H), 1.28 (d, *J =* 6.8 Hz, 3H).

### Example 15: Preparation of (R)-(3-methylcyclobutyl)(4-(3-methylmorpholino)-2-(1H-pyrazol-3-yl)-2,6,8,9-tetrahydro-7H-1,2,3,7-tetraazabenzo[cd]azulene-7-yl)methanone (Compound 15)

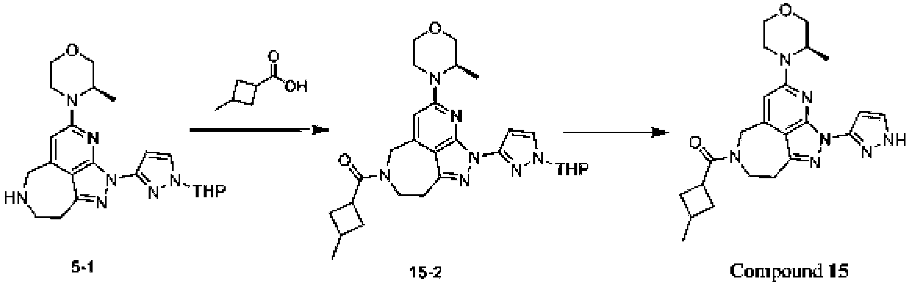

Similar to the preparation method of Example 9, the title Compound 15 was prepared using 3-methylcyclobutanecarboxylic acid instead of 3,3-difluorocyclobutanecarboxylic acid. (*R*)-(3-Methylcyclobutyl)(4-(3-methylmorpholino)-2-(1*H*-pyrazol-3-yl)-2,6,8,9-tetrahydro-7*H*-1, 2,3,7-tetraazabenzo[*cd*]azulene-7-yl)methanone (Compound 15) (4.2 mg, 23.5%) was obtained. LCMS (ESI) [M+H]⁺: 436.5. ¹H NMR (400 MHz, CD₃OD) δ 7.71 (s, 1H), 6.90 (s, 1H), 6.67 - 6.63 (m, 1H), 4.97 - 4.90 (m, 2H), 4.51 (d, *J =* 6.3 Hz, 1H), 4.11 (d, *J =* 13.5 Hz, 1H), 4.07 - 3.93 (m, 3H), 3.87 - 3.71 (m, 2H), 3.71 - 3.56 (m, 1H), 3.50 - 3.33 (m, 2H), 3.29 - 3.16 (m, 3H), 2.43 - 2.16 (m, 3H), 1.97 - 1.58 (m, 1H), 1.37 - 1.24 (m, 3H), 1.05 (d, *J =* 6.1 Hz, 1H), 0.95 (d, *J =* 6.0 Hz, 2H).

### Example 16: Preparation of 3,3,3-trifluoro-2-methyl-1-(4-((R)-3-methylmorpholinyl)-2-(1H-pyrazol-3-yl)-2,6,8,9-tetrahydro-7H-1,2,3,7-tetraazabenzo[cd]azulen-7-yl)propan-1-one (Compound 16)

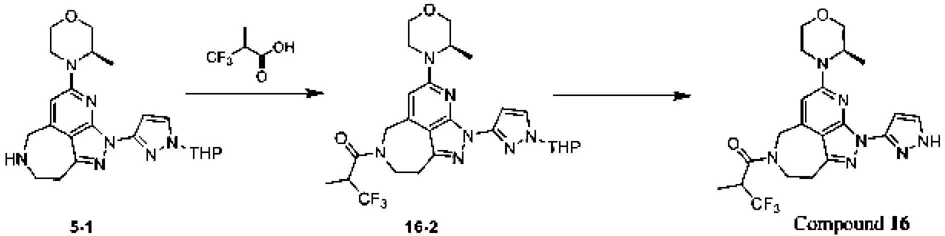

Similar to the preparation method of Example 9, the title Compound 16 was prepared using 3,3,3-trifluoro-2-methylpropionic acid instead of 3,3-difluorocyclobutanecarboxylic acid. 3,3,3-Trifluoro-2-methyl-1-(4-((*R*)-3-methylmorpholinyl)-2-(1*H*-pyrazol-3-yl)-2,6,8,9-tetrahydro -7*H*-1,2,3,7-tetraazabenzo[*cd*]azulen-7-yl)propan-1-one (Compound 16) (25 mg, 36.9%) was obtained. LCMS (ESI) [M+H]⁺: 464.2. ¹H NMR (400 MHz, CDCl₃) δ 7.66 (d, *J =* 1.5 Hz, 1H), 6.97 - 6.87 (m, 1H), 6.49 - 6.34 (m, 1H), 5.34 - 4.87 (m, 1H), 4.83 - 4.66 (m, 1H), 4.48 - 4.37 (m, 1H), 4.35 - 4.17 (m, 1H), 4.13 - 3.97 (m, 2H), 3.95 - 3.53 (m, 5H), 3.50 - 3.18 (m, 4H), 1.52 - 1.27 (m, 6H).

### Example 17: Preparation of (R)-3-methyl-4-(7-phenyl-2-(1H-pyrazol-3-yl)-6,7,8,9-tetrahydro-2H-1,2,3,7-tetraazabenzo[cd]azulen-4-yl)morpholine (Compound 17)

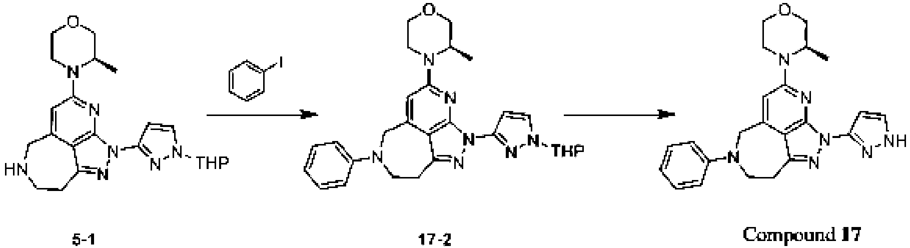

Step 1: Intermediate 5-1 (20.0 mg, 0.047 mmol) and iodobenzene (28.9 mg, 0.142 mmol) were dissolved in toluene (1 mL. 2-dicyclohexylphosphino-2,6-diisopropoxy-1,1-biphenyl (2.20 mg, 0.005 mmol), tris(dibenzylideneacetone)dipalladium (4.30 mg, 0.005 mmol), and cesium carbonate (30.8 mg, 0.094 mmol) were added. The reaction mixture was stirred at 90°C under a nitrogen atmosphere for 2 hours. After the reaction was completed, the reaction mixture was cooled to room temperature and diluted with ethyl acetate. The organic phase was washed with water, dried over anhydrous sodium sulfate, and filtered. The filtrate was concentrated under reduced pressure to yield a crude product, which was purified by preparative thin-layer chromatography to give Intermediate 17-2 (20.0 mg, 84.8%). LCMS (ESI) [M+H]⁺: 500.7.

Step 2: Intermediate 17-2 (20.0 mg, 0.040 mmol) was dissolved in methanol (2 mL), and a solution of hydrochloride acid in dioxane (2 mL, 4 M) was added. The reaction solution was stirred at room temperature for 1 hour, and then concentrated under reduced pressure to remove dioxane and methanol to obtain a crude product, which was then separated and purified by preparative reverse-phase high performance liquid chromatography to give (*R*)-3-methyl-4-(7-phenyl-2-(1*H*-pyrazol-3-yl)-6,7,8,9-tetrahydro-2*H*-1,2,3,7-tetraazabenzo[*cd*]azulen-4-yl)morpholine (Compound 17) (4.31 mg, 25.9%). LCMS (ESI) [M+H]⁺ : 416.3. ¹H NMR (400 MHz, CD₃OD) δ 7.71 (s, 1H), 7.15 (t, *J =* 7.6 Hz, 2H), 6.94 - 6.76 (m, 4H), 6.67 - 6.60 (m, 1H), 4.86 (s, 2H), 4.63 - 4.53 (m, 1H), 4.25 - 4.12 (m, 3H), 4.06 (dd, *J =* 2.8, 11.1 Hz, 1H), 3.90 - 3.78 (m, 2H), 3.71 - 3.61 (m, 1H), 3.43 - 3.36 (m, 2H), 3.30 - 3.26 (m, 1H), 1.35 (d, *J =* 6.6 Hz, 3H).

### Example 18: Preparation of (R)-(4-(3-methylmorpholinyl)-2-(1H-pyrazol-3-yl)-2,6,8,9-tetrahydro-7H-1,2,3,7-tetraazabenzo[cd]azulen-7-yl)(1-(trifluoromethyl)cyclopropyl)methanone (Compound 18)

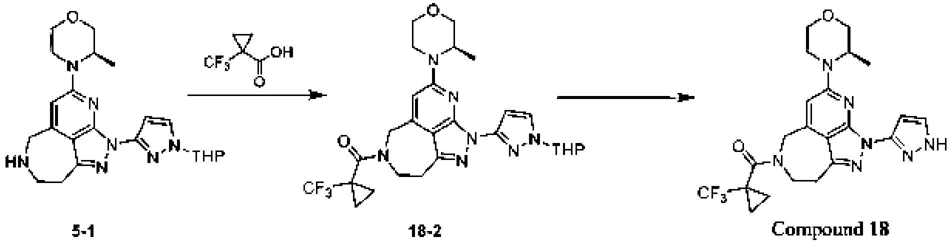

Similar to the preparation method of Example 9, the title Compound 18 was prepared using 1-trifluoromethylcyclopropane-1-carboxylic acid instead of 3,3-difluorocyclobutanecarboxylic acid. (*R*)-(4-(3-Methylmorpholinyl)-2-(1*H*-pyrazol-3-yl)-2,6,8,9-tetrahydro-7*H*-1,2,3,7-tetraazabenzo[*cd*]azulen-7-yl)(1-(trifluoromethyl)cyclopropyl)methanone (Compound 18) (22.4 mg, 37.7%) was obtained. LCMS (ESI) [M+H]⁺: 476.2. ¹H NMR (400 MHz, CDCl₃) δ 7.68 (d, *J* = 2.0 Hz, 1H), 6.91 (d, *J =* 1.6 Hz, 1H), 6.39 (s, 1H), 5.09 (s, 2H), 4.40 (d, *J =* 6.7 Hz, 1H), 4.15 - 3.96 (m, 4H), 3.89 - 3.77 (m, 2H), 3.71 - 3.61 (m, 1H), 3.46 - 3.27 (m, 3H), 1.34 (d, *J =* 6.8 Hz, 3H), 1.29 (t, *J =* 5.6 Hz, 2H), 1.03 (s, 2H).

### Example 19: Preparation of (2,2-difluorocyclopropyl)(4-((R)-3-methylmorpholino)-2-(1H-pyrazol-3-yl)-2,6,8,9-tetrahydro-7H-1,2,3,7-tetraazabenzo[cd]azulene-7-yl)methanone (Compound 19)

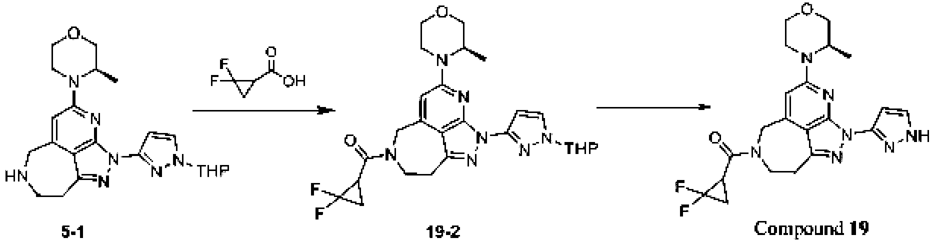

Similar to the preparation method of Example 9, the title Compound 19 was prepared using 2,2-difluorocyclopropylcarboxylic acid instead of 3,3-difluorocyclobutanecarboxylic acid. (2,2-Difluorocyclopropyl)(4-((*R*)-3-methylmorpholino)-2-(1*H*-pyrazol-3-yl)-2,6,8,9-tetrahydro-7 *H*-1,2,3,7-tetraazabenzo[*cd*]azulene-7-yl)methanone (Compound 19) (43.1 mg, 42.7%) was obtained. LCMS (ESI) [M+H]⁺: 444.2. ¹H NMR (400 MHz, DMSO-d6) δ 12.76 (s, 1H), 7.82 (s, 1H), 6.77 (s, 2H), 5.25 - 4.75 (m, 2H), 4.54 - 3.87 (m, 5H), 3.81 - 3.72 (m, 1H), 3.69 - 3.60 (m, 1H), 3.55 - 3.44 (m, 1H), 3.32 - 3.25 (m, 1H), 3.23 - 3.03 (m, 3H), 2.05 - 1.78 (m, 2H), 1.24 - 1.15 (m, 3H).

### Example 20A and Example 20B: Preparation of (R)-(1,1-difluorospiro[2.3]hexan-5-yl) (4-(3-methylmorpholino)-2-(1H-pyrazol-3-yl)-2,6,8,9-tetrahydro-7H-1,2,3,7-tetraazabenzo[cd]az ulen-7-yl)methanone (Compound 20A, Isomer 1) and preparation of (R)-(1,1-difluorospiro[2.3] hexan-5-yl)(4-(3-methylmorpholino)-2-(1H-pyrazol-3-yl)-2,6,8,9-tetrahydro-7H-1,2,3,7-tetraaza benzo[cd]azulen-7-yl)methanone (Compound 20B, Isomer 2)

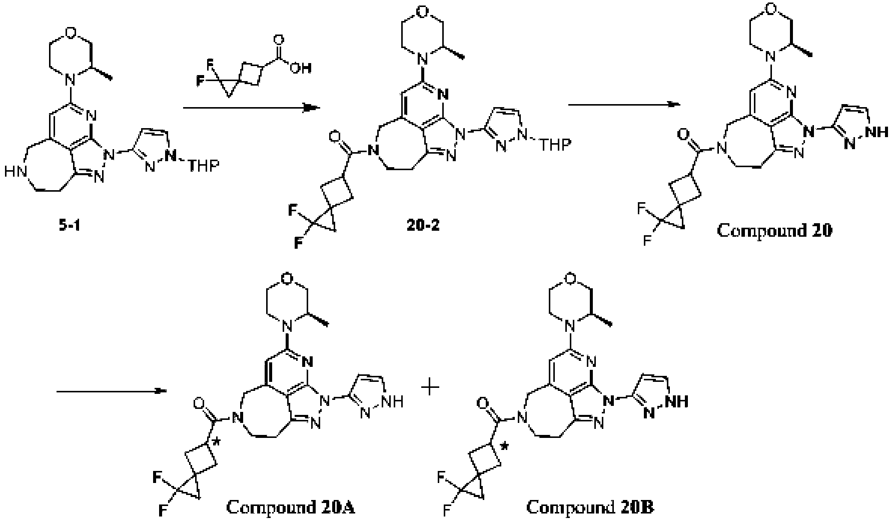

Similar to the preparation method of Example 9, the title Compound 20A and Compound 20B were prepared using 1,1-difluorospiro[2.3]hexane-5-carboxylic acid instead of 3,3-difluorocyclobutanecarboxylic acid. Reverse-phase high-performance liquid chromatography was used for preparative separation and purification.

The first peak was (*R*)-(1,1-difluorospiro[2.3]hexan-5-yl)(4-(3-methylmorpholino)-2-(1*H*-pyrazol-3-yl)-2,6,8,9-tetrahydro-7*H*-1,2,3,7-tetraazabenzo[*cd*]azulen-7-yl)methanone (Compound 20A, Isomer 1) (4.9 mg). LCMS (ESI) [M+H]⁺: 484.2. ¹H NMR (400 MHz, CD₃OD) δ 7.72 (s, 1H), 6.94 - 6.87 (m, 1H), 6.73 - 6.66 (m, 1H), 5.07 - 4.90 (m, 3H), 4.63 - 4.45 (m, 1H), 4.11 - 3.90 (m, 4H), 3.88 - 3.76 (m, 3H), 3.73 - 3.53 (m, 2H), 3.29 - 3.20 (m, 2H), 2.68 - 2.20 (m, 4H), 1.36 - 1.21 (m, 5H).

The second peak was (*R*)-(1,1-difluorospiro[2.3]hexan-5-yl)(4-(3-methylmorpholino)-2-(1*H*-pyrazol-3-yl)-2,6,8,9-tetrahydro-7*H*-1,2,3,7-tetraazabenzo[*cd*]azulen-7-yl)methanone (Compound 20B, Isomer 2) (3.3 mg). LCMS (ESI) [M+H]⁺: 484.2. ¹H NMR (400 MHz, CD₃OD) δ 7.72 (s, 1H), 6.90 - 6.89 (m, 1H), 6.78 - 6.60 (m, 1H), 5.07 - 4.90 (m, 3H), 4.63 - 4.44 (m, 1H), 4.18 - 3.94 (m, 4H), 3.88 - 3.71 (m, 3H), 3.68 - 3.53 (m, 2H), 3.28 - 3.11 (m, 2H), 2.58 - 2.20 (m, 4H), 1.40 - 1.22 (m, 3H), 1.14 (t, *J =* 8.6 Hz, 2H).

### Example 21: Preparation of (3R)-4-(7-(but-3-yn-2-yl)-2-(1H-pyrazol-3-yl)-6,7,8,9-tetrahydro-2H-1,2,3,7-tetraazabenzo[cd]azulen-4-yl)-3-methylmorpholine (Compound 21)

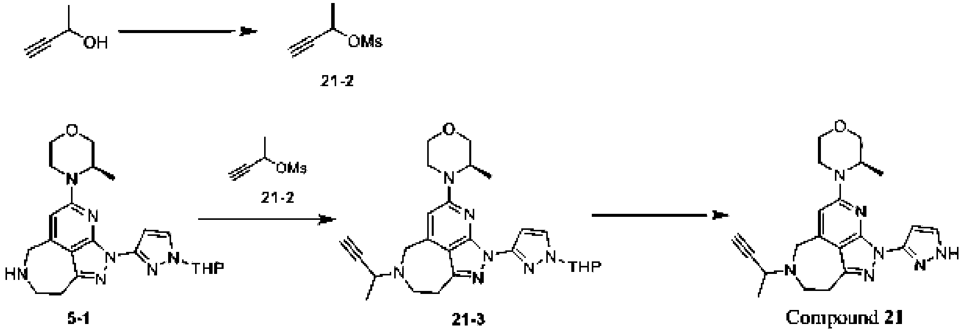

Step 1: 3-Butyn-2-ol (500 mg, 7.1 mmol) was dissolved in dichloromethane (10 mL) at room temperature. Triethylamine (1.48 mL, 10.7 mmol) was added, and methanesulfonyl chloride (980 mg, 8.6 mmol) was slowly added dropwise. The reaction mixture was stirred at 25°C overnight. The reaction was quenched with ice water (10 mL). The aqueous phase was extracted with dichloromethane (10 mL × 3). The organic phase was washed with saturated brine (20 mL), dried over anhydrous sodium sulfate, filtered, and concentrated to obtain crude Intermediate 21-2 (900 mg, 85.1%).

Step 2: Similar to the preparation method of Example 14, Intermediate 21-2 was used instead of trifluoroethyl trifluoromethanesulfonate to prepare the title Compound 21. (3*R*)-4-(7-(But-3-yn-2-yl)-2-(1*H*-pyrazol-3-yl)-6,7,8,9-tetrahydro-2*H*-1,2,3,7-tetraazabenzo[*cd*]a zulen-4-yl)-3-methylmorpholine (Compound 21) (43.1 mg, 42.7%) was obtained. LCMS (ESI) [M+H]⁺: 392.4. ¹H NMR (400 MHz, CDCl₃) δ 7.62 (d, *J =* 2.2 Hz, 1H), 6.84 (d, *J =* 2.1 Hz, 1H), 6.33 (s, 1H), 4.44 - 4.30 (m, 1H), 4.27 - 4.15 (m, 1H), 4.11 - 3.95 (m, 3H), 3.88 - 3.75 (m, 3H), 3.69 - 3.60 (m, 1H), 3.39 - 3.28 (m, 2H), 3.25 - 2.93 (m, 3H), 2.33 (d, *J* = 2.1 Hz, 1H), 1.47 (d, *J* = 7.0 Hz, 3H), 1.30 (dd, *J =* 6.7, 2.2 Hz, 3H).

### Example 22: Preparation of (R)-3-methyl-4-(7-(methylsulfonyl)-2-(1H-pyrazol-3-yl)-6,7,8,9-tetrahydro-2H-1,2,3,7-tetraazabenzo[cd]azulen-4-yl)morpholine (Compound 22)

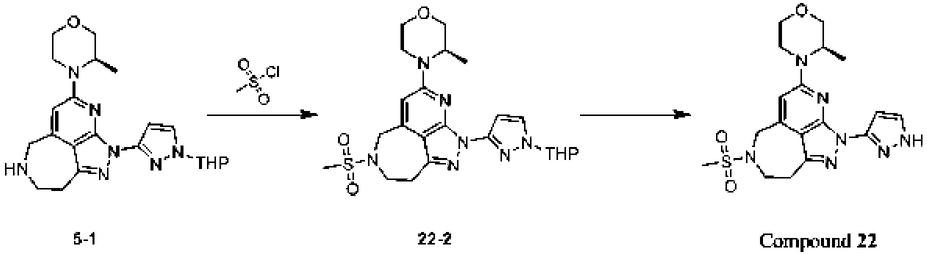

Step 1: Intermediate 5-1 (100 mg, 0.236 mmol) was dissolved in dichloromethane (5 mL) at room temperature. Triethylamine (0.07 mL, 0.472 mmol) was added, and methanesulfonyl chloride (40.6 mg, 0.354 mmol) was added under stirring. The reaction mixture was stirred at room temperature for 1 hour, then quenched with ice water (5 mL). The aqueous phase was extracted with dichloromethane (5 mL × 3). The organic phase was washed with saturated brine (20 mL), dried over anhydrous sodium sulfate, filtrated, and concentrated to obtain Intermediate 22-2 (100 mg, 84.4%). LCMS (ESI) [M+H]⁺: 502.2.

Step 2: Intermediate 22-2 (100 mg, 0.199 mmol) was dissolved in methanol (5 mL) at room temperature, and then aqueous hydrochloric acid (1 mL, 2 M) was added. The reaction mixture was stirred at 50°C for 4 hours. After the reaction was completed, the methanol was removed by concentration, the reaction mixture was diluted with dichloromethane (10 mL), and the pH was adjusted to 8 with saturated sodium bicarbonate solution. The aqueous phase was extracted with dichloromethane (5 mL × 3). The combined organic phases were washed with saturated brine (10 mL), dried over anhydrous sodium sulfate, filtered, and concentrated to obtain a crude product, which was then separated and purified by preparative reverse-phase high-performance liquid chromatography to obtain (R)-3-methyl-4-(7-(methylsulfonyl)-2-(1H-pyrazol-3-yl)-6,7,8,9-tetrahydro-2H-1,2,3,7-tetraazabenzo[*cd*]azulen-4-yl)morpholine (Compound 22) (36.5 mg, 43.9%). LCMS (ESI) [M+H]⁺ : 418.2. ¹H NMR (400 MHz, DMSO-d6) δ 12.77 (s, 1H), 7.83 (s, 1H), 6.75 (d, *J* = 28.1 Hz, 2H), 4.76 (s, 2H), 4.45 (d, *J* = 4.9 Hz, 1H), 4.08 - 3.93 (m, 2H), 3.86 - 3.74 (m, 3H), 3.69 - 3.61 (m, 1H), 3.56 - 3.44 (m, 1H), 3.16 (d, *J =* 5.5 Hz, 3H), 2.98 (s, 3H), 1.19 (d, *J* = 6.6 Hz, 3H).

### Example 23: Preparation of (R)-4-(7-(cyclopropylsulfonyl)-2-(1H-pyrazol-3-yl)-6,7,8,9-tetrahydro-2H-1,2,3,7-tetraazabenzo[cd]azulen-4-yl)-3-methylmorpholine (Compound 23)

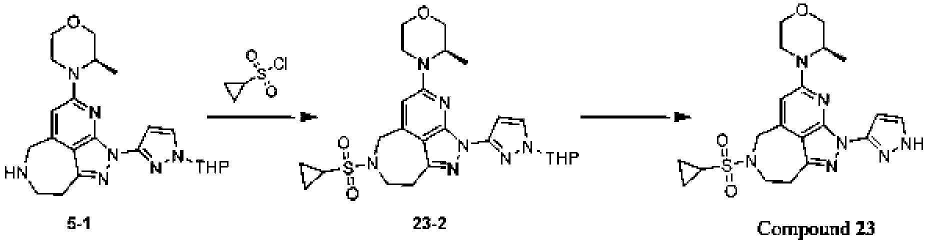

Similar to the preparation method of Example 22, the title Compound 23 was prepared using cyclopropanesulfonyl chloride instead of methanesulfonyl chloride. (*R*)-4-(7-(Cyclopropylsulfonyl)-2-(1*H*-pyrazol-3-yl)-6,7,8,9-tetrahydro-2*H*-1,2,3,7-tetraazabenzo [cd]azulen-4-yl)-3-methylmorpholine (Compound 23) (7.8 mg, 37.4%) was obtained. LCMS (ESI) [M+H]⁺: 444.4. ¹H NMR (400 MHz, CD₃OD) δ 7.72 (s, 1H), 6.92 (s, 1H), 6.64 (s, 1H), 4.83 (s, 2H), 4.50 (d, *J* = 5.1 Hz, 1H), 4.11 - 3.94 (m, 3H), 3.85 - 3.73 (m, 2H), 3.67 - 3.57 (m, 1H), 3.35 - 3.32 (m, 2H), 3.29 - 3.25 (m, 2H), 2.53 - 2.34 (m, 1H), 1.29 (d, *J =* 6.8 Hz, 3H), 1.21 (t, *J = 7.1* Hz, 1H), 1.09 - 1.04 (m, 2H), 0.89 - 0.85 (m, 1H).

### Example 24: Preparation of (R)-4-(7-(isopropylsulfonyl)-2-(1H-pyrazol-3-yl)-6,7,8,9-tetrahydro-2H-1,2,3,7-tetraazabenzo[cd]azulen-4-yl)-3-methylmorpholine (Compound 24)

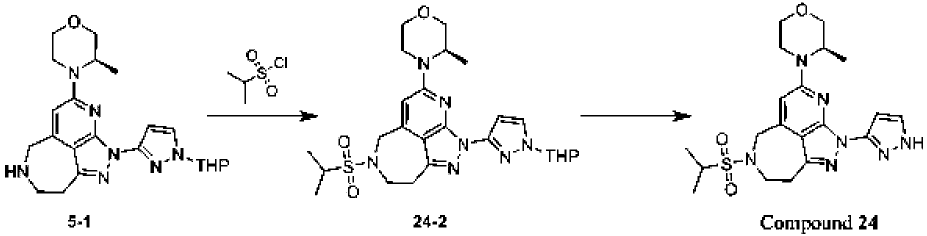

Similar to the preparation method of Example 22, the title Compound 24 was prepared using isopropanesulfonyl chloride instead of methanesulfonyl chloride. (*R*)-4-(7-(Isopropylsulfonyl)-2-(1*H*-pyrazol-3-yl)-6,7,8,9-tetrahydro-2*H*-1,2,3,7-tetraazabenzo[*c* d]azulen-4-yl)-3-methylmorpholine (Compound 24) (8.4 mg, 50.2%) was obtained. LCMS (ESI) [M+H]⁺: 446.4. ¹H NMR (400 MHz, CD₃OD) δ 7.72 (s, 1H), 6.92 (s, 1H), 6.61 (s, 1H), 4.82 (s, 2H), 4.57 - 4.44 (m, 1H), 4.05 - 4.13 (m, 1H), 4.01 (dd, *J =* 3.5, 11.5 Hz, 1H), 3.91 (t, *J =* 5.4 Hz, 2H), 3.84 - 3.73 (m, 2H), 3.66 - 3.56 (m, 1H), 3.47 (td, *J =* 6.8, 13.5 Hz, 1H), 3.34 - 3.33 (m, 1H), 3.30 - 3.25 (m, 2H), 1.35 (d, *J =* 6.8 Hz, 6H), 1.29 (d, *J =* 6.8 Hz, 3H).

### Example 25: Preparation of (R)-4-(2-(1H-pyrazol-3-yl)-7-((trifluoromethyl)sulfonyl)-6,7, 8,9-tetrahydro-2H-1,2,3,7-tetraazabenzo[cd]azulen-4-yl)-3-methylmorpholine (Compound 25)

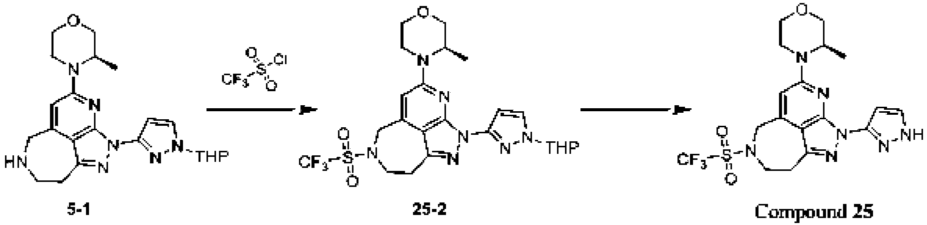

Similar to the preparation method of Example 22, the title Compound 25 was prepared using trifluoromethanesulfonyl chloride instead of methanesulfonyl chloride. (*R*)-4-(2-(1*H*-Pyrazol-3-yl)-7-((trifluoromethyl)sulfonyl)-6,7,8,9-tetrahydro-2*H*-1,2,3,7-tetraazab enzo[*cd*]azulen-4-yl)-3-methylmorpholine (Compound **25**) (1.1 mg, 16.3%) was obtained. LCMS (ESI) [M+H]⁺: 472.2. ¹H NMR (400 MHz, CD₃OD) δ 7.75 (s, 1H), 7.03 - 6.89 (m, 1H), 6.70 (s, 1H), 5.06 - 4.96 (m, 3H), 4.60 - 4.49 (m, 1H), 4.31 - 4.00 (m, 4H), 3.89 - 3.74 (m, 2H), 3.69 - 3.58 (m, 1H), 3.55 - 3.41 (m, 2H), 1.32 (d, *J =* 6.9 Hz, 3H).

### Example 26: Preparation of (R)-3-methyl-4-(7-(oxetan-3-ylsulfonyl)-2-(1H-pyrazol-3-yl)-6,7,8,9-tetrahydro-2H-1,2,3,7-tetraazabenzo[cd]azulen-4-yl)morpholine (Compound 26)

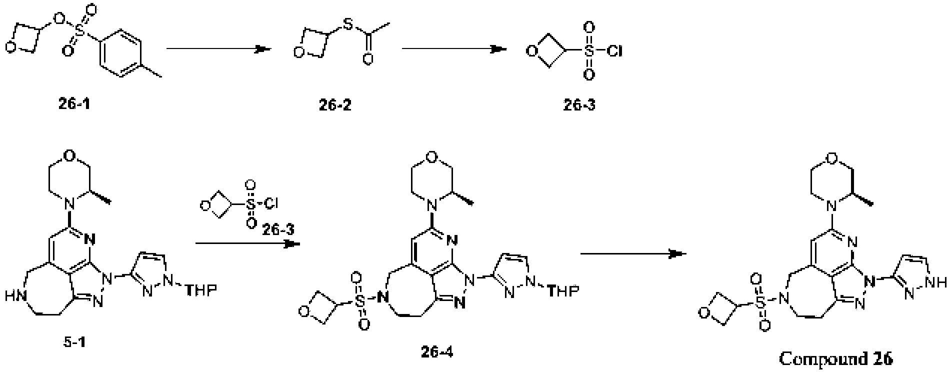

Step 1: Oxetan-3-yl toluene-4-sulfonate (10.0 g, 43.8 mmol) and potassium thioacetate (15.1 g, 131 mmol) were dissolved in *N,N*-dimethylformamide (50.0 mL). The reaction mixture was stirred at 100°C for 18 hours. The reaction mixture was cooled to room temperature and quenched with aqueous lithium chloride (200 mL, 5% wt). The mixture was extracted with dichloromethane (100 mL × 3). The combined organic phases were washed sequentially with water and saturated brine, dried over anhydrous sodium sulfate, filtered, and concentrated to obtain a crude product, which was purified by silica gel column chromatography to yield Intermediate **26-2** (2.0 g, 34.5%). ¹H NMR (400 MHz, CDCl₃) δ 5.06 (t, *J =* 7.2 Hz, 2H), 4.71 - 4.62 (m, 1H), 4.60 - 4.55 (m, 2H), 2.33 (s, 3H).

Step 2: N-chlorosuccinimide (808 mg, 6.05 mmol) was dissolved in acetonitrile (10 mL). Aqueous hydrochloric acid (1.13 mL, 2 M) was added at 0°C, then a solution of Intermediate **26-2** (200 mg, 1.51 mmol) in acetonitrile (10 mL) was slowly added dropwise. The reaction mixture was stirred at 0°C for half an hour. After the reaction was completed, the reaction mixture was concentrated under reduced pressure. Saturated sodium bicarbonate solution (10 mL) was added, and the reaction mixture was extracted with ethyl acetate (10 mL × 3). The organic phase was washed sequentially with saturated sodium thiosulfate solution and saturated brine, dried over anhydrous sodium sulfate, filtered, and concentrated to yield Intermediate **26-3** (100 mg, 42.2%) (36.5 mg, 43.9%). ¹H NMR (400 MHz, CDCl₃) δ 5.13 - 4.93 (m, 5H).

Step 3: Similar to the preparation method of Example 22, Intermediate **26-3** was used instead of methanesulfonyl chloride to prepare the title Compound **26.** (*R*)-3-Methyl-4-(7-(oxetan-3-ylsulfonyl)-2-(1*H*-pyrazol-3-yl)-6,7,8,9-tetrahydro-2*H*-1,2,3,7-tetra azabenzo[*cd*]azulen-4-yl)morpholine (Compound **26**) (1.5 mg, 11.6%) was obtained. LCMS (ESI) [M+H]⁺: 460.4. ¹H NMR (400 MHz, CD₃OD) δ 7.73 (d, *J =* 1.5 Hz, 1H), 6.93 (s, 1H), 6.64 (s, 1H), 4.84 - 4.83 (m, 3H), 4.71 (dt, *J =* 3.8, 7.4 Hz, 3H), 4.67 - 4.60 (m, 1H), 4.56 - 4.47 (m, 1H), 4.13 - 4.07 (m, 1H), 4.06 - 3.99 (m, 1H), 3.93 (t, *J =* 5.8 Hz, 2H), 3.86 - 3.74 (m, 3H), 3.62 (dt, *J =* 2.9, 11.7 Hz, 2H), 3.29 - 3.26 (m, 2H), 1.30 (d, *J =* 6.8 Hz, 3H).

### Example 27: Preparation of (R)-4-(7-((difluoromethyl)sulfonyl)-2-(1H-pyrazol-3-yl)-6,7, 8,9-tetrahydro-2H-1,2,3,7-tetraazabenzo[cd]azulen-4-yl)-3-methylmorpholine (Compound 27)

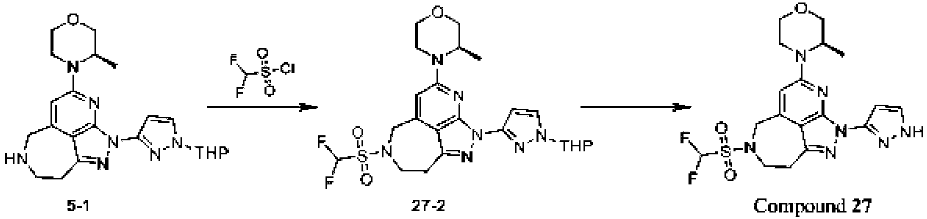

Step 1: Similarly to the preparation method in Example 22, the title Compound **27** was prepared using difluoromethanesulfonyl chloride instead of methanesulfonyl chloride. (*R*)-4-(7-((Difluoromethyl)sulfonyl)-2-(1*H*-pyrazol-3-yl)-6,7,8,9-tetrahydro-2*H*-1,2,3,7-tetraazab enzo[*cd*]azulen-4-yl)-3-methylmorpholine (Compound **27**) (3.0 mg, 7.1%) was obtained. LCMS (ESI) [M+H]⁺: 454.2. ¹H NMR (400 MHz, CD₃OD) δ 12.85 (s, 1H), 7.89 (s, 1H), 7.27 (t, *J =* 52.4 Hz, 1H), 6.84 (s, 2H), 4.99 (s, 2H), 4.53 - 4.51 (m, 1H), 4.06 - 4.04 (m, 4H), 3.84 - 3.82 (m, 1H), 3.73 - 3.69 (m, 1H), 3.57 - 3.55 (m, 1H), 3.29 - 3.22 (m, 3H), 1.26 (d, *J =* 6.8 Hz, 3H).

### Example 28: Preparation of (3R)-3-methyl-4-(7-(S-methylsulfonylimino)-2-(1H-pyrazol-3-yl)-6,7,8,9-tetrahydro-2H-1,2,3,7-tetraazabenzo[cd]azulen-4-yl)morpholine (Compound 28)

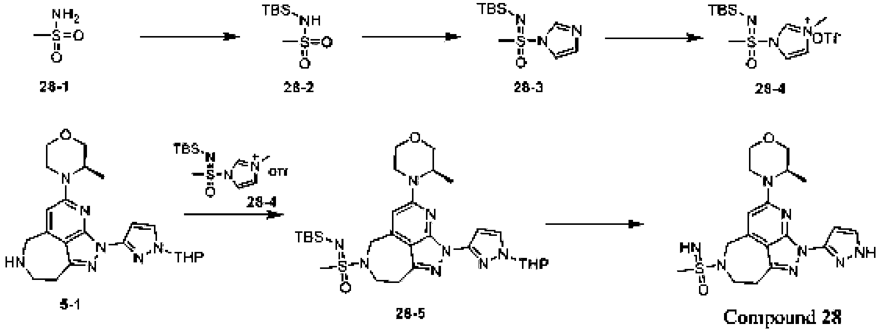

Step 1: Methylsulfonamide (5.0 g, 52.5 mmol) was dissolved in chloroform (60 mL) at 0°C, and triethylamine (10.6 g, 105.1 mmol) and tert-butyldimethylsilyl chloride (11.9 g, 78.8 mmol) were added. The reaction mixture was slowly warmed to room temperature, and stirred overnight. The reaction was quenched by adding ice water, and the aqueous phase was extracted with ethyl acetate (200 mL × 3). The combined organic phases were washed sequentially with water and saturated brine, dried over anhydrous sodium sulfate, filtered, and concentrated to obtain a crude product, which was purified by silica gel column chromatography to yield Intermediate **28-2** (10.0 g, 90.8%). ¹H NMR (400 MHz, DMSO-d6) δ 7.07 (s, 1H), 2.93 (s, 3H), 0.91 (s, 9H), 0.18 (s, 6H).

Step 2: Hexachloroethane (6.8 g, 28.7 mmol) and triphenylphosphine (7.5 g, 28.7 mmol) were dissolved in chloroform (100 mL) at room temperature. The reaction mixture was stirred at 70°C under a nitrogen atmosphere for 18 hours. The reaction mixture was cooled to room temperature. triethylamine (4.8 g, 47.8 mmol) and Intermediate **28-2** (5 g, 23.9 mmol) were added, and the reaction was continuously stirred at room temperature for 1 hour. A solution of imidazole (2.0 g, 28.7 mmol) and triethylamine (4.8 g, 47.8 mmol) in tetrahydrofuran was added dropwise to the reaction mixture. The mixture was stirred at room temperature under a nitrogen atmosphere for 18 hours. After the reaction was completed, the reaction solution was concentrated and extracted with n-hexane (150 mL × 3). The combined organic phases were washed sequentially with water and saturated brine, dried over anhydrous sodium sulfate, filtered, and concentrated to obtain a crude product, which was purified by silica gel column chromatography to yield Intermediate **28-3** (3.0 g, 48.4%). ¹H NMR (400 MHz, CDCl₃) δ 7.92 (s, 1H), 7.28 (dd, *J =* 2.5, 1.2 Hz, 1H), 7.15 - 7.09 (m, 1H), 3.22 (s, 3H), 0.92 (s, 9H), 0.10 (s, 3H), 0.07 (s, 3H).

Step 3: Intermediate **28-3** (1.2 g, 4.63 mmol) was dissolved in diethyl ether (30 mL) at 0°C, and methyl trifluoromethanesulfonate (0.8 g, 4.62 mmol) was added. The reaction mixture was stirred at room temperature for 2 hours, and filtered, and the filter cake was washed with diethyl ether to obtain a crude product as Intermediate **28-4** (1.6 g). ¹H NMR (400 MHz, CDCl₃) δ 9.07 (d, *J* = 0.5 Hz, 1H), 7.54 (t, *J =* 1.9 Hz, 1H), 7.50 (t, *J =* 1.8 Hz, 1H), 4.08 (s, 3H), 3.57 (s, 3H), 0.91 (s, 9H), 0.13 (s, 3H), 0.10 (s, 3H).

Step 4: Under a nitrogen atmosphere, Intermediate **5-1** (130 mg, 0.31 mmol) was dissolved in anhydrous acetonitrile (2.0 mL) at room temperature, and triethylamine (62 mg, 0.61 mmol) was added. Intermediate **28-4** (195 mg, 0.46 mmol) was dissolved in anhydrous acetonitrile (2.0 mL), and added dropwise to the reaction system. The reaction mixture was stirred at 50°C for 16 hours. The reaction was quenched by adding ice water, and the aqueous phase was extracted with dichloromethane (50 mL × 3). The combined organic phases were washed sequentially with water and saturated brine, dried over anhydrous sodium sulfate, filtered, and concentrated to obtain a crude product, which was purified by silica gel column chromatography to yield Intermediate **28-5** (60 mg, 31.8%). LCMS (ESI) [M+H]⁺: 615.6.

Step 5: Intermediate **28-5** (40 mg, 0.055 mmol) was dissolved in tetrahydrofuran (3.0 mL) at room temperature, and aqueous hydrochloric acid (3.0 mL, 6 M) was added. The reaction mixture was stirred at 40°C for 1 hour. The reaction was quenched by adding ice water, and the pH was adjusted to 8 with saturated sodium bicarbonate solution. The aqueous phase was extracted with dichloromethane (30 mL × 3). The combined organic phases were washed sequentially with water and saturated brine, dried over anhydrous sodium sulfate, filtered, and concentrated to obtain a crude product, which was then separated and purified by preparative reverse-phase high-performance liquid chromatography to yield (3*R*)-3-methyl-4-(7-(*S*-methylsulfonylimino)-2-(1*H-*pyrazol-3-yl)-6,7,8,9-tetrahydro-2*H*-1,2,3,7-tetraazabenzo[*cd*]azulen-4-yl)morpholine (Compound **28**) (4.0 mg, 14.8%). LCMS (ESI) [M+H]⁺: 417.2. ¹H NMR (400 MHz, CD₃OD) δ 7.75 (s, 1H), 6.94 (s, 1H), 6.65 (s, 1H), 4.58 - 4.47 (m, 1H), 4.11 (d, *J =* 12.0 Hz, 1H), 4.06 - 4.02 (m, 1H), 3.99 - 3.86 (m, 2H), 3.84 (d, *J =* 11.4 Hz, 1H), 3.80 - 3.75 (m, 1H), 3.66 - 3.59 (m, 1H), 3.37 - 3.32 (m, 2H), 3.30 - 3.27 (m, 3H), 2.94 (s, 3H), 1.31 (d, *J =* 6.8 Hz, 3H).

### Example 31A and Example 31B: Preparation of (3R)-4-(7-cyclopropyl-6-methyl-2-(1H-pyrazol-3-yl)-6,7,8,9-tetrahydro-2H-1,2,3,7-tetraazabenzo[cd]azulen-4-yl)-3-methylmorpholine (Compound 31A, Isomer 1) and preparation of (3R)-4-(7-cyclopropyl-6-methyl-2-(1H-pyrazol-3-yl)-6,7,8,9-tetrahydro-2H-1,2,3,7-tetraazabenzo[cd]azulen-4-yl)-3-methylmorp holine (Compound 31B, Isomer 2)

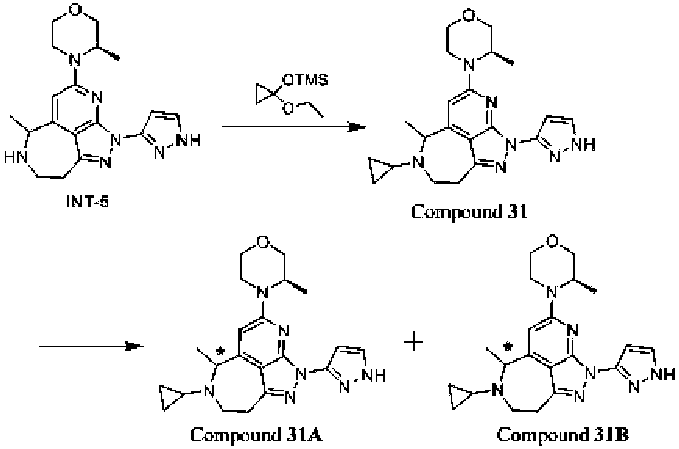

Step 1: Intermediate **INT-5** (100 mg, 0.23 mmol) and 1-ethoxy-1-trimethylsilyloxycyclopropane (172.6 mg, 0.99 mmol) were dissolved in methanol (5 mL), and acetic acid (0.16 mL, 2.83 mmol) and sodium cyanoborohydride (80.0 mg, 1.27 mmol) were added sequentially under an ice-water bath. The reaction mixture was allowed to naturally warm to room temperature and stirred for 2 hours. Saturated aqueous ammonium chloride (10 mL) was added to quench the reaction. The aqueous phase was extracted with ethyl acetate (20 mL × 3). The organic phase was washed sequentially with water and saturated brine, dried over anhydrous sodium sulfate, filtered, and spin-dried to obtain a crude product. The crude product was purified by silica gel column chromatography to obtain a diastereomeric mixture of (3*R*)-4-(7-cyclopropyl-6-methyl-2-(1*H*-pyrazol-3-yl)-6,7,8,9-tetrahydro-2*H*-1,2,3,7-tetraazabenz o[*cd*]azulen-4-yl)-3-methylmorpholine (Compound **31**) (70.0 mg, 63.6%). LCMS (ESI) [M+H]⁺: 394.4.

Step 2: The diastereomeric mixture (3*R*)-4-(7-cyclopropyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **31**) (70.0 mg) was further purified by chiral SFC. SFC analysis: Daicel Chiral PAK^{®} OD column (100 × 3.0 mm, 3 µm), mobile phase: 20% methanol (containing 0.1% diethylamine) in CO₂, temperature: 35°C, flow rate: 1.5 mL/min, detection wavelength: 214 nm.

The first peak was (3*R*)-4-(7-cyclopropyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **31A,** Isomer 1) (10 mg). LCMS (ESI) [M+H]⁺: 394.4. HPLC-SFC: Rt = 3.106 min. ¹H NMR (400 MHz, CD₃OD) δ 7.70 (s, 1H), 6.90 (s, 1H), 6.54 (s, 1H), 4.51 (d, *J =* 6.8 Hz, 1H), 4.46 (q, *J =* 7.2 Hz, 1H), 4.07 (d, *J* = 10.8 Hz, 1H), 4.01 (dd, *J* = 11.4, 3.6 Hz, 1H), 3.81 (d, *J* = 11.4 Hz, 1H), 3.76 (dd, *J* = 11.4, 2.9 Hz, 1H), 3.66 - 3.55 (m, 2H), 3.46 - 3.35 (m, 1H), 3.33 (d, *J =* 3.8 Hz, 1H), 3.26 (d, *J =* 3.8 Hz, 1H), 3.20 (d, *J* = 14.6 Hz, 1H), 3.07 (d, *J* = 17.4 Hz, 1H), 2.29 - 2.20 (m, 1H), 1.49 (d, *J* = 7.2 Hz, 3H), 1.29 (d, *J =* 6.8 Hz, 3H), 0.61 - 0.48 (m, 4H).

The second peak was (3*R*)-4-(7-cyclopropyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **31B,** Isomer 2) (10 mg). LCMS (ESI) [M+H]⁺: 394.4. HPLC-SFC: Rt = 3.755 min. ¹H NMR (400 MHz, CD₃OD) δ 7.70 (s, 1H), 6.90 (s, 1H), 6.54 (s, 1H), 4.51 (d, *J* = 6.4 Hz, 1H), 4.46 (q, *J =* 7.2 Hz, 1H), 4.07 (d, *J =* 10.8 Hz, 1H), 4.01 (dd, *J =* 11.4, 3.7 Hz, 1H), 3.82 (d, *J =* 11.4 Hz, 1H), 3.76 (dd, *J* = 11.4, 2.8 Hz, 1H), 3.66 - 3.54 (m, 2H), 3.46 - 3.36 (m, 1H), 3.35 - 3.31 (m, 1H), 3.29 - 3.24 (m, 1H), 3.20 (dd, *J* = 10.8, 3.6 Hz, 1H), 3.07 (d, *J* = 17.2 Hz, 1H), 2.29 - 2.19 (m, 1H), 1.49 (d, *J =* 7.2 Hz, 3H), 1.28 (d, *J =* 6.7 Hz, 3H), 0.62 - 0.46 (m, 4H).

### Example 32A and Example 32B: Preparation of (3R)-3-methyl-4-(6-methyl-7-(oxetan-3-yl)-2-(1H-pyrazol-3-yl)-6,7,8,9-tetrahydro-2H-1,2,3,7-tetraazabenzo[cd]azulen-4-yl)m orpholine (Compound 32A, Isomer 1) and preparation of (3R)-3-methyl-4-(6-methyl-7-(oxetan-3-yl)-2-(1H-pyrazol-3-yl)-6,7,8,9-tetrahydro-2H-1,2,3,7-tetraazabenzo[cd]azulen-4-yl)m orpholine (Compound 32B, Isomer 2)

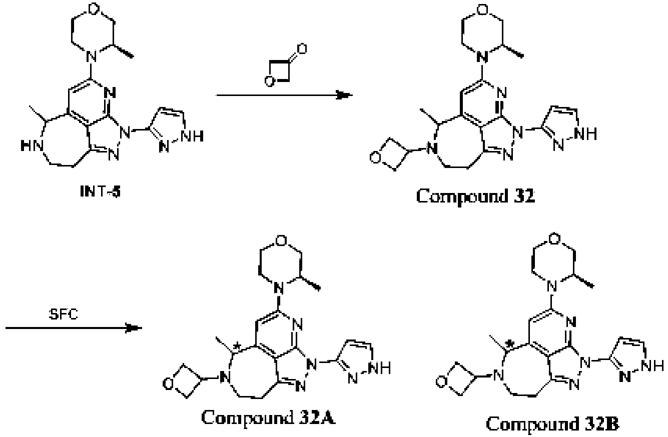

Step 1: Intermediate **INT-5** (4.0 g, 11.3 mmol) was dissolved in methanol (100 mL). Diisopropylethylamine (5.62 mL, 34.0 mmol) was added. The reaction was stirred at room temperature for 20 minutes, and then 3-oxetanone (4.1 g, 56.6 mmol) was added. After stirring at room temperature for half an hour, sodium triacetoxyborohydride (11.9 g, 56.6 mmol) was added in batches. The reaction mixture was stirred at room temperature overnight. After the reaction was completed, the reaction mixture was poured into ice water, extracted with dichloromethane (100 mL × 3), dried over anhydrous sodium sulfate, and filtered. The filtrate was concentrated under reduced pressure to obtain a crude product, which was then separated and purified by silica gel column chromatography to obtain a diastereomeric mixture of (3*R*)-3-methyl-4-(6-methyl-7-(oxetan-3-yl)-2-(1*H*-pyrazol-3-yl)-6,7,8,9-tetrahydro-2*H*-1,2,3,7-tetraazabenzo[*cd*]azulen-4-yl) morpholine (Compound **32**) (2.5 g, 53.9%). LCMS (ESI) [M+H]⁺: 410.2.

Step 2: The diastereomeric mixture of (3*R*)-3-methyl-4-(6-methyl-7-(oxetan-3-yl)-2-(1*H-*pyrazol-3-yl)-6,7,8,9-tetrahydro-2*H*-1,2,3,7-tetraazabenzo[*cd*]azulen-4-yl)morpholine (Compound **32**) (2.5 g, 6.1 mmol) was further purified by chiral SFC. SFC analysis: Daicel ChiralPAK IG column (100 × 3.0 mm, 3 µm), mobile phase: 20% methanol (containing 0.05% diethylamine) in CO₂, temperature: 35°C, flow rate: 2.0 mL/min, detection wavelength: 210 nm.

The first peak was (3*R*)-3-methyl-4-(6-methyl-7-(oxetan-3-yl)-2-(1*H*-pyrazol-3-yl)-6,7,8,9-tetrahydro-2*H*-1,2,3,7-tetraazabenzo[*cd*]azulen-4-yl)morpholine (Compound **32A,** Isomer 1) (865.8 mg). LCMS (ESI) [M+H]⁺: 410.2. HPLC-SFC: Rt = 1.839 min. ¹H NMR (400 MHz, CD₃OD) δ 7.71 (s, 1H), 6.91 (s, 1H), 6.53 (s, 1H), 4.74 (d, *J =* 7.2 Hz, 2H), 4.65 (d, *J =* 6.8 Hz, 2H), 4.49 (d, *J =* 5.2 Hz, 1H), 4.36 - 4.23 (m, 2H), 4.11 - 3.97 (m, 2H), 3.89 - 3.72 (m, 2H), 3.67 - 3.49 (m, 2H), 3.30 - 3.13 (m, 2H), 3.08 - 2.97 (m, 2H), 1.48 (d, *J* = 7.2 Hz, 3H), 1.28 (d, *J =* 6.8 Hz, 3H).

The second peak was (3*R*)-3-methyl-4-(6-methyl-7-(oxetan-3-yl)-2-(1*H*-pyrazol-3-yl)-6,7,8,9-tetrahydro-2*H*-1,2,3,7-tetraazabenzo[*cd*]azulen-4-yl)morpholine (Compound **32B,** Isomer 2) (6.2 mg). LCMS (ESI) [M+H]⁺: 410.2. HPLC-SFC: Rt = 2.368 min. ¹H NMR (400 MHz, CD₃OD) δ 7.72 (s, 1H), 6.91 (s, 1H), 6.53 (s, 1H), 4.74 (d, *J =* 7.2 Hz, 2H), 4.66 (d, *J =* 6.8 Hz, 2H), 4.51 (d, *J =* 5.2 Hz, 1H), 4.38 - 4.26 (m, 2H), 4.11 - 3.98 (m, 2H), 3.85 - 3.73 (m, 2H), 3.66 - 3.51 (m, 2H), 3.30 - 3.13 (m, 2H), 3.08 - 3.01 (m, 2H), 1.48 (d, *J =* 7.2 Hz, 3H), 1.29 (d, *J =* 6.8 Hz, 3H).

### Example 33A and Example 33B: Preparation of (R)-(4-(3-methylmorpholinyl)-2-(1H-pyrazol-3-yl)-2,6,8,9-tetrahydro-7H-1,2,3,7-tetraazabenzo[cd]azulen-7-yl)(1-(trifluoromet hyl)cyclopropyl)methanone (Compound 33A, Isomer 1) and preparation of (R)-(4-(3-methylmorpholinyl)-2-(1H-pyrazol-3-yl)-2,6,8,9-tetrahydro-7H-1,2,3,7-tetraazabenzo[ cd]azulen-7-yl)(1-(trifluoromethyl)cyclopropyl)methanone (Compound 33B, Isomer 2)

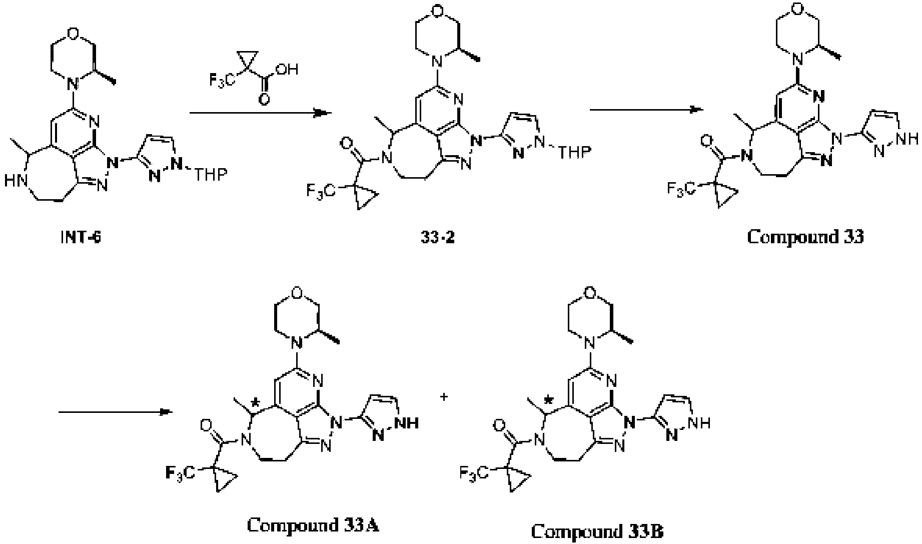

Step 1: 1-Trifluoromethylcyclopropane-1-carboxylic acid (54.9 mg, 0.36 mmol), *N,N*-diisopropylethylamine (0.12 mL, 0.71 mmol), and *O*-(7-azabenzotriazol-1-yl)-*N,N,N',N'*-tetramethyluronium hexafluorophosphate (135 mg, 0.357 mmol) were dissolved in *N,N*-dimethylformamide (3 mL). The reaction solution was stirred at room temperature for 10 minutes. Intermediate **INT-6** (78.0 mg, 0.178 mmol) was added to the reaction system, and stirring was continued for 18 hours. The reaction was quenched with ice water (10 mL), and the mixture was extracted with ethyl acetate (20 mL × 3). The organic phase was washed sequentially with water and saturated brine, dried over anhydrous sodium sulfate, filtered, and spin-dried to obtain a crude product. The crude product was purified by silica gel column chromatography to yield Intermediate **33-2** (11.0 mg, 10.8%). LCMS (ESI) [M+H]⁺: 574.6.

Step 2: Intermediate **33-2** (11.0 mg, 0.02 mmol) was dissolved in a solution of hydrochloride solution in dioxane (5 mL), and the reaction mixture was stirred at room temperature for 2 hours. The reaction mixture was concentrated under reduced pressure to obtain a crude product, which was separated and purified by preparative reverse-phase high-performance liquid chromatography to obtain a diastereomeric mixture of (*R*)-(4-(3-methylmorpholinyl)-2-(1*H*-pyrazol-3-yl)-2,6,8,9-tetrahydro-7*H*-1,2,3,7-tetraazabenzo[*cd*]azulene-7-yl)(1-(trifluoromethyl)cyclopropyl)methanone (Compound **33**) (11 mg, crude). LCMS (ESI) [M+H]⁺: 490.3.

Step 3: The diastereomeric mixture of (*R*)-(4-(3-methylmorpholinyl)-2-(1*H*-pyrazol-3-yl)-2,6,8,9-tetrahydro-7*H*-1,2,3,7-tetraazabenzo[*cd*]azulene-7-yl)(1-(trifluoromethyl)cyclopropyl)me thanone (Compound **33**) (11 mg) was further purified by chiral SFC. SFC analysis: Daicel ChiralPAK IC-3 column (50 × 3.0 mm, 3 µm), mobile phase: 40% methanol (containing 0.05% diethylamine) in CO₂, temperature: 35°C, flow rate: 3.0 mL/min, detection wavelength: 220 nm.

The first peak was (*R*)-(4-(3-methylmorpholinyl)-2-(1*H*-pyrazol-3-yl)-2,6,8,9-tetrahydro-7*H*-1,2,3,7-tetraazabenzo[*cd*]azulen-7-yl)(1-(trifluoromethyl)cyclopropyl)methanone (Compound **33A,** Isomer 1) (4.0 mg). LCMS (ESI) [M+H]⁺: 490.3. HPLC-SFC: Rt = 1.276 min. ¹H NMR (400 MHz, CD₃OD) δ 7.72 (br d, *J =* 1.6 Hz, 1H), 6.90 (br d, *J* = 4.3 Hz, 1H), 6.75 (s, 1H), 6.08 - 5.95 (m, 1H), 4.61 - 4.46 (m, 2H), 4.40 - 4.28 (m, 1H), 4.11 (br dd, *J* = 1.4, 14.0 Hz, 1H), 4.03 (dd, *J* = 3.4, 11.0 Hz, 1H), 3.87 - 3.74 (m, 2H), 3.74 - 3.58 (m, 2H), 3.42 - 3.34 (m, 1H), 3.16 - 3.02 (m, 1H), 1.73 (d, *J =* 6.9 Hz, 3H), 1.45 - 1.33 (m, 3H), 1.32 - 1.20 (m, 5H).

The second peak was (*R*)-(4-(3-methylmorpholinyl)-2-(1*H*-pyrazol-3-yl)-2,6,8,9-tetrahydro-7*H*-1,2,3,7-tetraazabenzo[*cd*]azulen-7-yl)(1-(trifluoromethyl)cyclopropyl)methanone (Compound **33B,** Isomer 2) (4.0 mg). LCMS (ESI) [M+H]⁺: 490.3. HPLC-SFC: Rt = 2.760 min. ¹H NMR (400 MHz, CD₃OD) δ 7.72 (br s, 1H), 6.91 (br s, 1H), 6.79 - 6.68 (m, 1H), 6.08 - 5.94 (m, 1H), 4.62 - 4.46 (m, 2H), 4.40 - 4.27 (m, 1H), 4.17 - 4.08 (m, 1H), 4.03 (br dd, *J* = 2.9, 10.8 Hz, 1H), 3.88 - 3.58 (m, 4H), 3.41 - 3.34 (m, 1H), 3.16 - 3.02 (m, 1H), 1.73 (br d, *J =* 6.9 Hz, 3H), 1.43 - 1.34 (m, 3H), 1.33 - 1.23 (m, 7H).

### Example 34: Preparation of (R)-2-((4-(3-methylmorpholinyl)-2-(1H-pyrazol-3-yl)-2,6,8,9-tetrahydro-7H-1,2,3,7-tetraazabenzo[cd]azulene-7-yl)sulfonyl)ethan-1-ol (Compound 34)

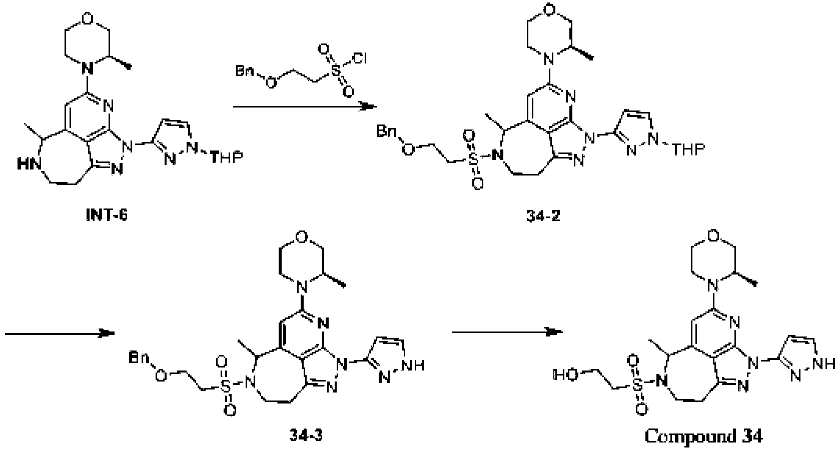

Step 1: Intermediate **INT-6** (170 mg, 0.389 mmol) was dissolved in pyridine (5 mL), and 2-(benzyloxy)ethane-1-sulfonyl chloride (274 mg, 1.17 mmol) was added. The reaction mixture was stirred at 25°C for 30 minutes. The reaction was quenched with ice water (20 mL) and the resulting mixture was extracted with ethyl acetate (20 mL × 3). The organic phase was washed sequentially with water and saturated brine. The organic phase was dried over anhydrous sodium sulfate, filtered, and concentrated to obtain a crude product. The crude product was purified by C-18 column chromatography using an acetonitrile/water system to yield Intermediate **34-2** (120 mg, 48.6%). LCMS (ESI) [M+H]⁺: 636.6.

Step 2: Intermediate **34-2** (120 mg, 0.19 mmol) was dissolved in dichloromethane (2 mL), and trifluoroacetic acid (2 mL) was added. The reaction mixture was stirred at 25°C for 18 hours. The reaction was quenched with ice water (20 mL), the pH of the resulting mixture was adjusted to 8 with saturated sodium bicarbonate solution, and the aqueous phase was extracted with dichloromethane (30 mL × 3). The organic phase was washed sequentially with water and saturated brine. The organic phase was dried over anhydrous sodium sulfate, filtered, and concentrated to obtain a crude product. The crude product was separated and purified by silica gel chromatography to yield Intermediate **34-3** (52 mg, 50.5%). LCMS (ESI) [M+H]⁺: 552.4.

Step 3: Intermediate **34-3** (40 mg, 0.073 mmol) was dissolved in tetrahydrofuran (10 mL). Palladium-on-carbon (15 mg, 0.145 mmol) and palladium hydroxide (15 mg, 0.145 mmol) were added in sequence, and the reaction mixture was stirred at 60°C under a hydrogen atmosphere for 18 hours. The reaction mixture was cooled to room temperature, filtered, and concentrated to obtain a crude product. The crude product was separated and purified by preparative reverse-phase high-performance liquid chromatography to yield (*R*)-2-((4-(3-methylmorpholinyl)-2-(1*H*-pyrazol-3-yl)-2,6,8,9-tetrahydro-7*H*-1,2,3,7-tetraazabenzo[*cd*]azulen-7-yl)sulfonyl)ethan-1-ol (Compound **34**) (2.9 mg, 8.7%). LCMS (ESI) [M+H]⁺ : 462.4. ¹H NMR (400 MHz, CD₃OD) δ 7.71 (s, 1H), 6.90 (s, 1H), 6.61 (s, 1H), 5.46 (q, *J =* 8.0 Hz, 1H), 4.51 (d, *J =* 6.2 Hz, 1H), 4.08 (d, *J =* 13.6 Hz, 2H), 4.04 - 3.99 (m, 1H), 3.91 (t, *J =* 6.2 Hz, 2H), 3.84 - 3.73 (m, 3H), 3.65 - 3.57 (m, 1H), 3.46 - 3.32 (m, 3H), 3.27 (d, *J* = 5.6 Hz, 1H), 3.17 (d, *J =* 17.2 Hz, 1H), 1.65 (d, *J =* 7.2 Hz, 3H), 1.29 (t, *J =* 6.8 Hz, 3H).

### Example 35A and Example 35B: Preparation of (3R)-4-(6,7-dimethyl-2-(1H-pyrazol-3-yl)-6,7,8,9-tetrahydro-2H-1,2,3,7-tetraazabenzo[cd]azulen-4-yl)-3-methylmorpholine (Compound 35A, Isomer 1) and preparation of (3R)-4-(6,7-dimethyl-2-(1H-pyrazol-3-yl)-6,7,8,9-tetrahydro-2H-1,2,3,7-tetraazabenzo[cd]azulen-4-yl)-3-methylmorpholine (Compound 35B, Isomer 2)

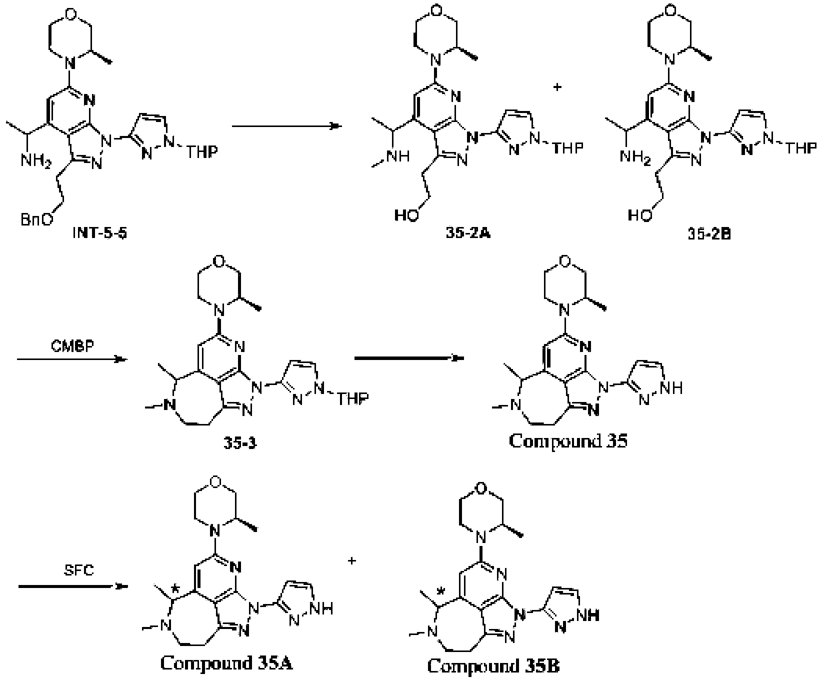

Step 1: Intermediate **INT-5-5** (10 g, 18.4 mmol) and ammonium acetate (14.2 g, 183.6 mmol) were dissolved in methanol (200 mL). The reaction mixture was stirred at room temperature for 1 hour. Sodium cyanoborohydride (5.8 g, 91.8 mmol) was added in batches. The reaction mixture was stirred at 70°C overnight. The reaction mixture was cooled to room temperature, poured into ice water, and extracted with dichloromethane (200 mL × 3). The organic phase was washed sequentially with water and saturated brine. The organic phase was dried over anhydrous sodium sulfate, filtered, and concentrated to obtain a crude product. The crude product was separated and purified by silica gel chromatography to yield Intermediate mixture of **35-2A** and **35-2B** (9 g).

Step 2: Intermediate mixture of **36-2A** and **36-2B** (9 g) was dissolved in dioxane (40 mL) at room temperature, and cyanomethylenetributylphosphorane (5.3 g, 21.9 mmol) was added. The reaction mixture was stirred at 120°C for 2 hours. After cooling, the reaction mixture was concentrated under reduced pressure to remove the dioxane to obtain a crude product, which was purified by silica gel chromatography to yield Intermediate **35-3** (0.5 g, 25.2%).

Step 3: Intermediate **35-3** (0.5 g, 1.1 mmol) was dissolved in methanol (10 mL) at room temperature, then a solution of hydrochloride acid in dioxane (2.8 mL, 4 M) was added. The reaction mixture was stirred at 60°C for 1 hour. After the reaction was completed, the reaction mixture was concentrated, diluted with dichloromethane (20 mL), and the pH was adjusted to approximately 7 with saturated sodium bicarbonate solution. The reaction mixture was extracted with dichloromethane (10 mL x 3), and the organic phase was washed sequentially with water and saturated brine. The organic phase was dried over anhydrous sodium sulfate, filtered, and concentrated to obtain a crude product. The crude product was separated and purified by preparative reverse-phase high performance liquid chromatography to yield an enantiomeric mixture of (3*R*)-4-(6,7-dimethyl-2-(1*H*-pyrazol-3-yl)-6,7,8,9-tetrahydro-2H-1,2,3,7-tetraazabenzo[*cd*]azulen -4-yl)-3-methylmorpholine (Compound **35**) (200 mg, 49.0%). LCMS (ESI) [M+H]⁺: 368.2.

Step 4: The enantiomeric mixture of (3*R*)-4-(6,7-dimethyl-2-(1*H*-pyrazol-3-yl)-6,7,8,9-tetrahydro-2*H*-1,2,3,7-tetraazabenzo[*cd*]azulen-4-yl)-3-methylmorpholine (Compound **35**) (200 mg) was further purified by chiral SFC. SFC analysis: Daicel CHIRALPAK^{®} OD column (100 × 3.0 mm, 3 µm), mobile phase: 20% methanol (containing 0.1% diethylamine) in CO₂, temperature: 35°C, flow rate: 1.5 mL/min, detection wavelength: 214 nm.

The first peak was (3*R*)-4-(6,7-dimethyl-2-(1*H*-pyrazol-3-yl)-6,7,8,9-tetrahydro-2H-1,2,3,7-tetraazabenzo[*cd*]azulen-4-yl)-3-methylmorpholine (Compound **35A,** Isomer 1) (70.0 mg). LCMS (ESI) [M+H]⁺: 368.2. HPLC-SFC: Rt = 2.445 min. ¹H NMR (400 MHz, CD₃OD) δ 7.72 (s, 1H), 6.92 (s, 1H), 6.55 (s, 1H), 4.51 (d, *J =* 5.2 Hz, 1H), 4.35 - 4.24 (m, 1H), 4.14 - 3.95 (m, 2H), 3.87 - 3.73 (m, 2H), 3.70 - 3.55 (m, 2H), 3.31 - 3.26 (m, 2H), 3.16 - 3.06 (m, 2H), 2.56 (s, 3H), 1.53 (d, *J* = 7.2 Hz, 3H), 1.29 (d, *J =* 6.8 Hz, 3H).

The second peak was (3*R*)-4-(6,7-dimethyl-2-(1*H*-pyrazol-3-yl)-6,7,8,9-tetrahydro-2*H-*1,2,3,7-tetraazabenzo[*cd*]azulen-4-yl)-3-methylmorpholine (Compound **35B,** Isomer 2) (59.8 mg). LCMS (ESI) [M+H]⁺: 368.2. HPLC-SFC: Rt = 2.968 min. ¹H NMR (400 MHz, CD₃OD) δ 7.72 (s, 1H), 6.91 (s, 1H), 6.54 (s, 1H), 4.51 (d, *J* = 4.8 Hz, 1H), 4.31 - 4.22 (m, 1H), 4.14 - 3.98 (m, 2H), 3.89 - 3.74 (m, 2H), 3.72 - 3.57 (m, 2H), 3.31 - 3.24 (m, 2H), 3.16 - 3.05 (m, 2H), 2.56 (s, 3H), 1.54 (d, *J =* 7.2 Hz, 3H), 1.29 (d, *J =* 6.8 Hz, 3H).

### Example 36A and Example 36B: Preparation of (3R)-4-(7-isopropyl-6-methyl-2-(1H-pyrazol-3-yl)-6,7,8,9-tetrahydro-2H-1,2,3,7-tetraazabenzo[cd]azulen-4-yl)-3-methylmorp holine (Compound 36A, Isomer 1) and preparation of (3R)-4-(7-isopropyl-6-methyl-2-(1H-pyrazol-3-yl)-6,7,8,9-tetrahydro-2H-1,2,3,7-tetraazabenzo[cd]azulen-4-yl)-3-methylmorp holine (Compound 36B, Isomer 2)

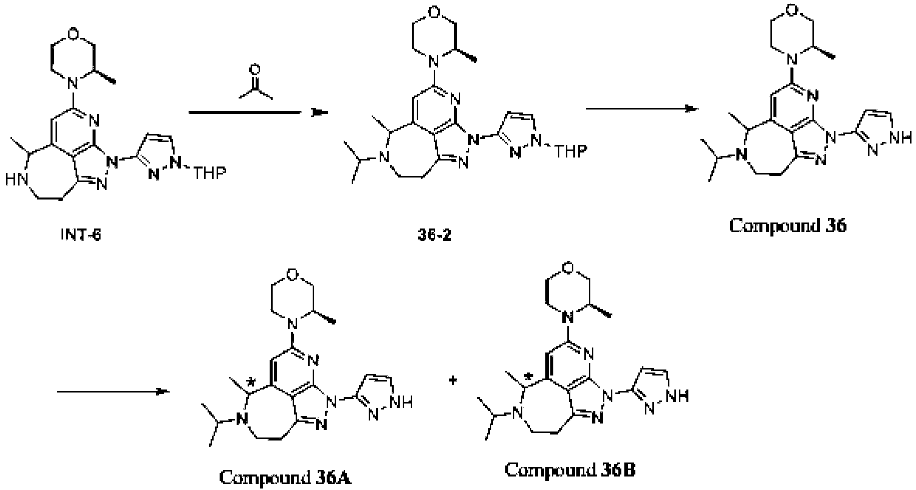

Step 1: Intermediate **INT-6** (190 mg, 0.434 mmol) was dissolved in methanol (5 mL), and acetone (252 mg, 4.34 mmol), zinc cyanide (204 mg, 1.74 mmol), and sodium cyanoborohydride (111 mg, 1.74 mmol) were added in sequence. The reaction mixture was stirred at 60°C for 18 hours. The reaction was quenched by adding ice water (20 mL), and the aqueous phase was extracted with ethyl acetate (20 mL × 3). The organic phase was washed sequentially with water and saturated brine. The organic phase was dried over anhydrous sodium sulfate, filtered, and concentrated to obtain a crude product. The crude product was separated and purified by silica gel chromatography to yield Intermediate **36-2** (110 mg, 52.8%). LCMS (ESI) [M+H]⁺: 480.1.

Step 2: Intermediate **36-2** (110 mg, 0.229 mmol) was dissolved in dichloromethane (2 mL), and added dropwise with trifluoroacetic acid (2 mL). The reaction mixture was stirred at room temperature for half an hour. The solvent was spin-dried to obtain a crude product, which was then diluted with dichloromethane (20 mL). The mixture was adjusted to a pH of approximately 8 with saturated sodium bicarbonate solution. The aqueous phase was extracted with dichloromethane (20 mL × 3). The organic phase was washed sequentially with water and saturated brine. The organic phase was dried over anhydrous sodium sulfate, filtered, and concentrated to obtain a crude product. The crude product was separated and purified by preparative reverse-phase high-performance liquid chromatography to obtain a diastereomeric mixture of (3*R*)-4-(7-isopropyl-6-methyl-2-(1*H*-pyrazol-3-yl)-6,7,8,9-tetrahydro-2*H*-1,2,3,7-tetraazabenzo[*c d*]azulen-4-yl)-3-methylmorpholine (Compound **36**) (25.0 mg, 27.6%). LCMS (ESI) [M+H]⁺: 396.4.

Step 3: The enantiomeric mixture (3*R*)-4-(7-isopropyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **36**) (25.0 mg) was further purified by chiral SFC. SFC analysis: Daicel CHIRALPAK^{®} IC column (100 × 3.0 mm, 3 µm), mobile phase: 20% methanol (containing 0.1% diethylamine) in CO₂, temperature: 35°C, flow rate: 1.5 mL/min, detection wavelength: 214 nm.

The first peak was (3*R*)-4-(7-isopropyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **36A,** Isomer 1) (4.9 mg). LCMS (ESI) [M+H]⁺: 396.4. HPLC-SFC: Rt = 2.474 min. ¹H NMR (400 MHz, CD₃OD) δ 7.75 (s, 1H), 6.94 (s, 1H), 6.57 (s, 1H), 4.59 (d, *J* = 6.8 Hz, 1H), 4.55 (s, 1H), 4.11 (d, *J* = 13.2 Hz, 1H), 4.05 (d, *J =* 8.0 Hz, 1H), 3.85 (d, *J =* 11.6 Hz, 1H), 3.80 (d, *J =* 11.2 Hz, 1H), 3.64 (d, *J* = 11.6 Hz, 2H), 3.33 - 3.23 (m, 3H), 3.19 (d, *J =* 6.8 Hz, 1H), 3.05 (d, *J =* 17.6 Hz, 1H), 1.54 (d, *J =* 6.8 Hz, 3H), 1.32 (d, *J =* 6.8 Hz, 3H), 1.23 (d, *J =* 6.4 Hz, 3H), 1.16 (d, *J =* 6.4 Hz, 3H).

The second peak was (3*R*)-4-(7-isopropyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **36B,** Isomer 2) (3.5 mg). LCMS (ESI) [M+H]⁺: 396.4. HPLC-SFC: Rt = 2.859 min. ¹H NMR (400 MHz, CD₃OD) δ 7.74 (s, 1H), 6.94 (s, 1H), 6.58 (s, 1H), 4.62 - 4.57 (m, 1H), 4.53 (d, *J =* 6.0 Hz, 1H), 4.12 (d, *J =* 13.6 Hz, 1H), 4.05 (d, *J* = 11.2 Hz, 1H), 3.85 (d, *J* = 11.2 Hz, 1H), 3.80 (d, *J* = 11.2 Hz, 1H), 3.67 (d, *J =* 9.2 Hz, 1H), 3.61 (d, *J =* 11.2 Hz, 1H), 3.33 - 3.25 (m, 3H), 3.22 - 3.17 (m, 1H), 3.06 (d, *J =* 17.6 Hz, 1H), 1.54 (d, *J =* 6.8 Hz, 3H), 1.32 (d, *J =* 6.8 Hz, 3H), 1.23 (d, *J =* 6.4 Hz, 3H), 1.16 (d, *J =* 6.4 Hz, 3H).

### Example 37A and Example 37B: Preparation of (3R)-4-(7-(cyclopropylsulfonyl)-6-methyl-2-(1H-pyrazol-3-yl)-6,7,8,9-tetrahydro-2H-1,2,3,7-tetraazabenzo[cd]azulen-4-yl)-3-methylmorp holine (Compound 37A, Isomer 1) and preparation of (3R)-4-(7-(cyclopropylsulfonyl)-6-methyl-2-(1H-pyrazol-3-yl)-6,7,8,9-tetrahydro-2H-1,2,3,7-tetraazabenzo[cd]azulen-4-yl)-3-methylmorp holine (Compound 37B, Isomer 2)

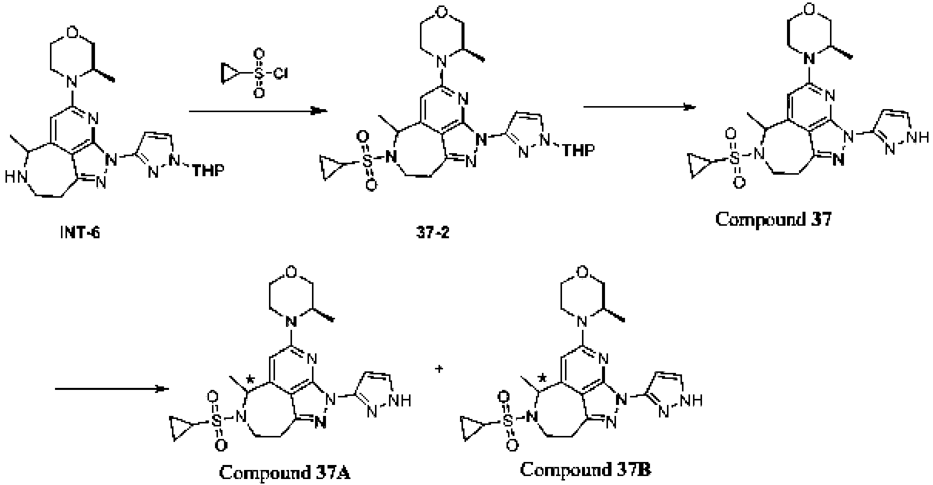

Step 1: Intermediate **INT-6** (100 mg, 0.23 mmol) was dissolved in tetrahydrofuran (10 mL), and added dropwise with lithium diisopropylamide (0.23 mL, 2 M) at -78°C. After the addition was completed, the mixture was stirred at -78°C for half hour, and then cyclopropanesulfonyl chloride (96.4 mg, 0.69 mmol) was added. The mixture was stirred at room temperature overnight. The reaction was quenched by adding ice water (20 mL), and the aqueous phase was extracted with ethyl acetate (20 mL × 3). The organic phase was washed sequentially with water and saturated brine. The organic phase was dried over anhydrous sodium sulfate, filtered, and concentrated to obtain a crude product. The crude product was separated and purified by silica gel chromatography to obtain Intermediate **37-2** (40 mg, 32.3%). LCMS (ESI) [M+H]⁺: 541.9.

Step 2: Intermediate **37-2** (30 mg, 0.055 mmol) was dissolved in dichloromethane (6 mL). trifluoroacetic acid (1.5 mL) was added, and the reaction was stirred at room temperature for 1 hour. After the reaction was completed, ice water (20 mL) was added to quench the reaction, and the pH of the resulting mixture was adjusted to approximately 8 with saturated sodium bicarbonate solution. The aqueous phase was extracted with dichloromethane (10 mL × 3). The organic phase was washed sequentially with water and saturated brine. The organic phase was dried over anhydrous sodium sulfate, filtered, and concentrated to obtain a crude product. The crude product was separated and purified by silica gel chromatography to obtain a diastereomeric mixture of (3*R*)-4-(7-(cyclopropylsulfonyl)-6-methyl-2-(1*H*-pyrazol-3-yl)-6,7,8,9-tetrahydro-2*H*-1,2,3,7-tetr aazabenzo[*cd*]azulen-4-yl)-3-methylmorpholine (Compound **37**) (18 mg, 71.5%). LCMS (ESI) [M+H]⁺: 458.4.

Step 3: The diastereomeric mixture of (3*R*)-4-(7-(cyclopropylsulfonyl)-6-methyl-*2*-(1*H*-pyrazol-3-yl)-6,7,8,9-tetrahydro-2*H*-1,2,3,7-tetraazabenzo[*cd*]azulen-4-yl)-3-methylmorp holine (Compound **37**) (18 mg) was further purified by chiral SFC. SFC analysis: Daicel CHIRALPAK-ADHS column (150 × 4.6 mm, 5 µm), mobile phase: 50% ethanol in n-hexane, temperature: 35°C, flow rate: 1.0 mL/min, detection wavelength: 254 nm.

The first peak was (3*R*)-4-(7-(cyclopropylsulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **37A,** Isomer 1) (2.5 mg). LCMS (ESI) [M+H]⁺: 458.4. HPLC-SFC: Rt = 4.710 min. ¹H NMR (400 MHz, CD₃OD) δ 7.71 (s, 1H), 6.91 (s, 1H), 6.63 (s, 1H), 5.49 - 5.38 (m, 1H), 4.54 - 4.44 (m, 1H), 4.14 - 4.05 (m, 2H), 4.04 - 3.98 (m, 1H), 3.90 - 3.71 (m, 3H), 3.66 - 3.57 (m, 1H), 3.46 - 3.35 (m, 1H), 3.23 - 3.14 (m, 1H), 2.48 - 2.39 (m, 1H), 1.67 (d, *J =* 7.2 Hz, 3H), 1.29 (d, *J =* 6.4 Hz, 4H), 1.13 - 1.03 (m, 2H), 0.94 - 0.84 (m, 2H).

The second peak was (3*R*)-4-(7-(cyclopropylsulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **37B,** Isomer 2) (2.3 mg). LCMS (ESI) [M+H]⁺: 458.4. HPLC-SFC: Rt = 7.152 min. ¹H NMR (400 MHz, CD₃OD) δ 7.71 (s, 1H), 6.91 (s, 1H), 6.62 (s, 1H), 5.52 - 5.38 (m, 1H), 4.49 (d, *J =* 6.8 Hz, 1H), 4.20 - 3.94 (m, 3H), 3.91 - 3.70 (m, 3H), 3.67 - 3.56 (m, 1H), 3.46 - 3.34 (m, 1H), 3.22 - 3.14 (m, 1H), 2.50 - 2.41 (m, 1H), 1.68 (d, *J =* 7.2 Hz, 3H), 1.27 (d,*J* = 7.8 Hz, 4H), 1.13 - 1.04 (m, 2H), 0.93 - 0.87 (m, 2H).

### Example 38: Preparation of 1-(6-methyl-4-((R)-3-methylmorpholinyl)-2-(1H-pyrazol-3-yl)-2,6,8,9-tetrahydro-7H-1,2,3,7-tetraazabenzo[cd]azulen-7-yl)ethan-1-one (Compound 38)

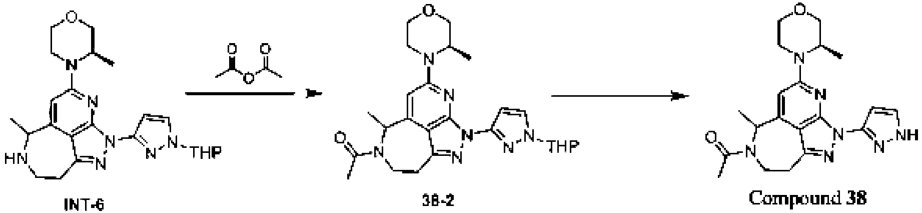

Step 1: Intermediate **INT-6** (100 mg, 0.23 mmol) was dissolved in tetrahydrofuran (5 mL). Acetic anhydride (0.11 mL, 1.14 mmol) and triethylamine (0.16 mL, 1.14 mmol) were added at room temperature, and the reaction was continuously stirred for 1 hour. The reaction mixture was concentrated to yield crude Intermediate **38-2** (80 mg, 73.0%). LCMS (ESI) [M+H]⁺: 480.0.

Step 2: Intermediate **38-2** (80 mg, 0.167 mmol) was dissolved in dichloromethane (2 mL). Trifluoroacetic acid (2 mL) was added, and the reaction was stirred at room temperature for 1 hour. After the reaction was completed, the reaction was quenched by adding ice water (10 mL), and the pH of the resulting mixture was adjusted to approximately 8 with saturated sodium bicarbonate solution. The aqueous phase was extracted with dichloromethane (10 mL × 3). The organic phase was washed sequentially with water and saturated brine. The organic phase was dried over anhydrous sodium sulfate, filtered, and concentrated to yield a crude product. The crude product was purified by preparative high performance liquid chromatography to give 1-(6-methyl-4-((*R*)-3-methylmorpholinyl)-2-(1*H*-pyrazol-3-yl)-2,6,8,9-tetrahydro-7*H*-1,2,3,7-tetraazabenzo[*cd*]azule n-7-yl)ethan-1-one (Compound **38**) (50 mg, 75.8%). LCMS (ESI) [M+H]⁺: 396.4. ¹H NMR (400 MHz, CD₃OD) δ 7.72 (s, 1H), 6.91 (d, *J =* 5.5 Hz, 1H), 6.66 (d, *J =* 10.2 Hz, 1H), 6.37 - 5.47 (m, 1H), 4.57 - 4.31 (m, 2H), 4.16 - 3.90 (m, 3H), 3.86 - 3.72 (m, 2H), 3.67 - 3.57 (m, 2H), 3.44 - 3.32 (m, 1H), 3.30 - 3.18 (m, 1H), 3.30 - 3.02 (m, 2H), 3.10 - 3.01 (m, 1H), 2.33 - 2.14 (m, 3H), 1.74 - 1.55 (m, 3H), 1.33 - 1.26 (m, 3H).

### Example 39A and Example 39B: Preparation of (3R)-3-methyl-4-(6-methyl-7-(methylsulfonyl)-2-(1H-pyrazol-3-yl)-6,7,8,9-tetrahydro-2H-1,2,3,7-tetraazabenzo[cd]azulen-4 -yl)morpholine (Compound 39A, Isomer 1) and preparation of (3R)-3-methyl-4-(6-methyl-7-(methylsulfonyl)-2-(1H-pyrazol-3-yl)-6,7,8,9-tetrahydro-2H-1,2,3,7-tetraazabenzo[cd]azulen-4 -yl)morpholine (Compound 39B, Isomer 2)

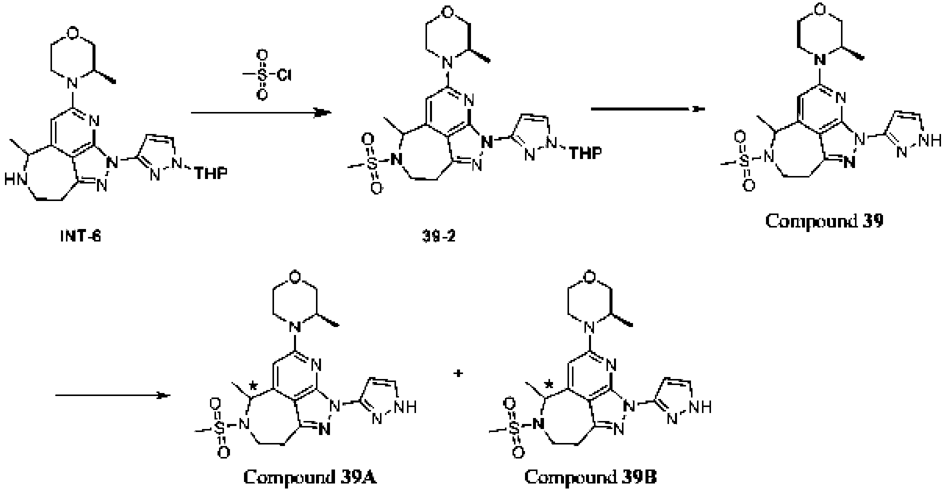

Step 1: Similar to the preparation method in Example 37, the title Compound **39** was prepared using methanesulfonyl chloride instead of cyclopropanesulfonyl chloride. A diastereomeric mixture of (3*R*)-3-methyl-4-(6-methyl-7-(methylsulfonyl)-2-(1*H*-pyrazol-3-yl)-6,7,8,9-tetrahydro-2*H*-1,2,3,7-tetraazabenzo[*cd*]azulen-4-yl)morpholine (Compound **39**) (960 mg, 88.3%) was obtained. LCMS (ESI) [M+H]⁺: 432.4.

Step 2: The diastereomeric mixture of (3*R*)-3-methyl-4-(6-methyl-7-(methylsulfonyl)-2-(1*H-*pyrazol-3-yl)-6,7,8,9-tetrahydro-2*H*-1,2,3,7-tetraazabenzo[*cd*]azulen-4-yl)morpholine (Compound **39**) (960 mg) was further purified by chiral SFC. SFC analysis: Daicel ChiralPAK OD column (150 × 4.6 mm, 5 µm), mobile phase: 50% methanol (containing 0.05% ammonia) in CO₂, temperature: 35°C, flow rate: 1.0 mL/min, detection wavelength: 210 nm.

The first peak was (3*R*)-3-methyl-4-(6-methyl-7-(methylsulfonyl)-2-(1*H*-pyrazol-3-yl)-6,7,8,9-tetrahydro-2*H*-1,2,3,7-tetraazabenzo[*cd*]azulen-4-yl)morpholine (Compound **39A,** Isomer 1) (364.5 mg). LCMS (ESI) [M+H]⁺: 432.4. HPLC-SFC: Rt = 4.710 min. ¹H NMR (400 MHz, DMSO-d6) δ 12.77 (s, 1H), 7.82 (s, 1H), 6.74 (d, *J =* 17.8 Hz, 1H), 5.50 - 5.21 (m, 1H), 4.46 (d, *J* = 5.2 Hz, 1H), 4.07 - 3.90 (m, 1H), 3.83 - 3.61 (m, 1H), 3.56 - 3.45 (m, 1H), 3.26 - 3.08 (m, 1H), 2.99 (s, 1H), 1.59 (d, *J =* 10.4 Hz, 1H), 1.18 (d, *J =* 6.6 Hz, 1H).

The second peak was (3*R*)-3-methyl-4-(6-methyl-7-(methylsulfonyl)-2-(1*H*-pyrazol-3-yl)-6,7,8,9-tetrahydro-2*H*-1,2,3,7-tetraazabenzo[*cd*]azulen-4-yl)morpholine (Compound **39B,** Isomer 2) (304.1 mg). LCMS (ESI) [M+H]⁺: 432.4. HPLC-SFC: Rt = 7.152 min. ¹H NMR (400 MHz, CD₃OD) δ 12.75 (s, 1H), 7.81 (s, 1H), 6.74 (d, *J =* 16.2 Hz, 1H), 5.40 (m, 1H), 4.11 - 3.88 (m, 1H), 3.81 - 3.61 (m, 1H), 3.49 (m, 1H), 3.23 - 3.08 (m, 1H), 2.98 (s, 1H), 1.57 (d, *J =* 7.2 Hz, 1H), 1.20 (d, *J =* 6.6 Hz, 1H).

### Example 40A and Example 40B: Preparation of 1-(6-methyl-4-((R)-3-methylmorpholinyl)-2-(1H-pyrazol-3-yl)-6,7,8,9-tetrahydro-2H-1,2,3,7-tetraazabenzo[cd]azulene-7-carbonyl)cyclopr opane-1-carbonitrile (Compound 40A, Isomer 1) and preparation of 1-(6-methyl-4-((R)-3-methylmorpholinyl)-2-(1H-pyrazol-3-yl)-6,7,8,9-tetrahydro-2H-1,2,3,7-tetraazabenzo[cd]azule ne-7-carbonyl)cyclopropane-1-carbonitrile (Compound 40B, Isomer 2)

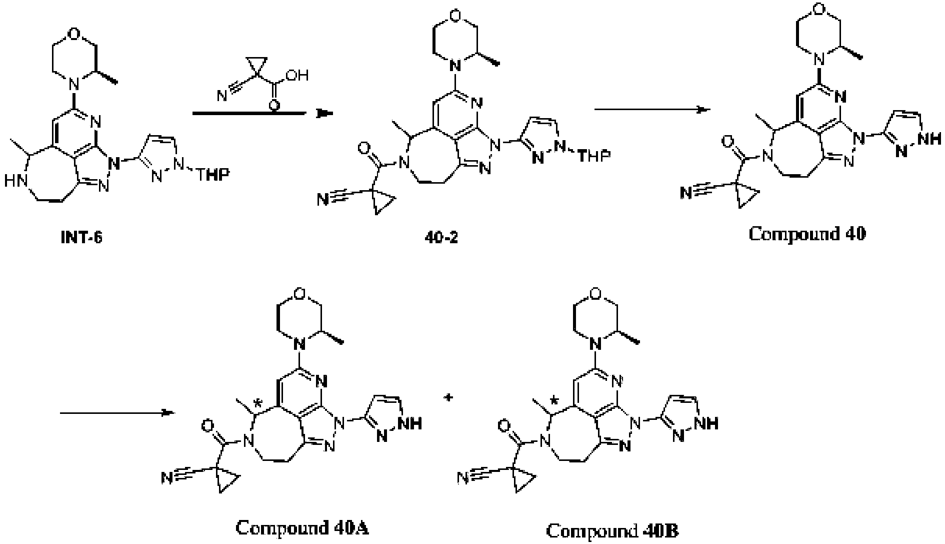

Step 1: Similarly to the preparation method in Example 33, Compound **40** was prepared using 1-cyano-1-cyclopropanecarboxylic acid instead of 1-trifluoromethylcyclopropane-1-carboxylic acid. A diastereomeric mixture of 1-(6-methyl-4-((*R*)-3-methylmorpholinyl)-2-(1*H*-pyrazol-3-yl)-6,7,8,9-tetrahydro-2*H*-1,2,3,7-tetr aazabenzo[*cd*]azulene-7-carbonyl)cyclopropane-1-carbonitrile (Compound **40**) (40 mg, 39.6%) was obtained. LCMS (ESI) [M+H]⁺: 447.2.

Step 2: The diastereomeric mixture of 1-(6-methyl-4-((*R*)-3-methylmorpholinyl)-2-(1*H*-pyrazol-3-yl)-6,7,8,9-tetrahydro-2*H*-1,2,3,7-tetraazabenzo[*cd*]azulene-7-carbonyl)cyclopr opane-1-carbonitrile (Compound **40**) (40 mg) was further purified by chiral SFC. SFC analysis: Daicel ChiralPAK AD column (150 × 4.6 mm, 5 µm), mobile phase: 20% methanol in n-hexane, temperature: 35°C, flow rate: 1.0 mL/min, detection wavelength: 254 nm.

The first peak was 1-(6-methyl-4-((*R*)-3-methylmorpholinyl)-2-(1*H*-pyrazol-3-yl)-6,7,8,9-tetrahydro-2*H*-1,2,3,7-tetraazabenzo[*cd*]azulene-7-carbonyl)cyclopropane-1-carbonitrile (Compound **40A,** Isomer 1) (7.0 mg). LCMS (ESI) [M+H]⁺: 447.2. HPLC-SFC: Rt = 2.578 min. ¹H NMR (400 MHz, CDCl₃) δ 7.74 (d, *J =* 2.0 Hz, 1H), 6.87 (d, *J =* 2.0 Hz, 1H), 6.51 - 6.36 (m, 1H), 5.99 - 5.90 (m, 1H), 4.52 - 4.29 (m, 2H), 4.25 - 4.03 (m, 2H), 3.93 - 3.80 (m, 2H), 3.71 - 3.30 (m, 4H), 3.18 - 3.10 (m, 1H), 1.81 (d, *J =* 6.8 Hz, 3H), 1.62 - 1.47 (m, 3H), 1.33 (d, *J =* 8.8 Hz, 3H), 1.30 - 1.19 (m, 2H).

The second peak was 1-(6-methyl-4-((*R*)-3-methylmorpholinyl)-2-(1*H*-pyrazol-3-yl)-6,7,8,9-tetrahydro-2*H*-1,2,3,7-tetraazabenzo[*cd*]azulene-7-carbonyl)cyclopropane-1-carbonitrile (Compound **40B,** Isomer 2) (8.7 mg). LCMS (ESI) [M+H]⁺: 447.2. HPLC-SFC: Rt = 3.484 min. ¹H NMR (400 MHz, CDCl₃) δ 7.64 (d, *J =* 2.0 Hz, 1H), 6.90 (d, *J =* 2.0 Hz, 1H), 6.49 - 6.3 (m, 1H), 5.97 - 5.90 (m, 1H), 4.46 - 4.29 (m, 2H), 4.17 - 3.98 (m, 3H), 3.84 - 3.78 (m, 2H), 3.75 - 3.55 (m, 2H), 3.37 - 3.30 (m, 2H), 3.22 - 3.05 (m, 1H), 1.80 (d, *J =* 6.0 Hz, 3H), 1.67 - 1.51 (m, 3H), 1.35 (d, *J =* 6.4 Hz, 3H), 0.94 - 0.79 (m, 1H).

### Example 41A and Example 41B: Preparation of (3R)-4-(7-ethyl-6-methyl-2-(1H-pyrazol-3-yl)-6,7,8,9-tetrahydro-2H-1,2,3,7-tetraazabenzo[cd]azulen-4-yl)-3-methylmorp holine (Compound 41A, Isomer 1) and preparation of (3R)-4-(7-ethyl-6-methyl-2-(1H-pyrazol-3-yl)-6,7,8,9-tetrahydro-2H-1,2,3,7-tetraazabenzo[cd]azulen-4-yl)-3-methylmorp holine (Compound 41B, Isomer 2)

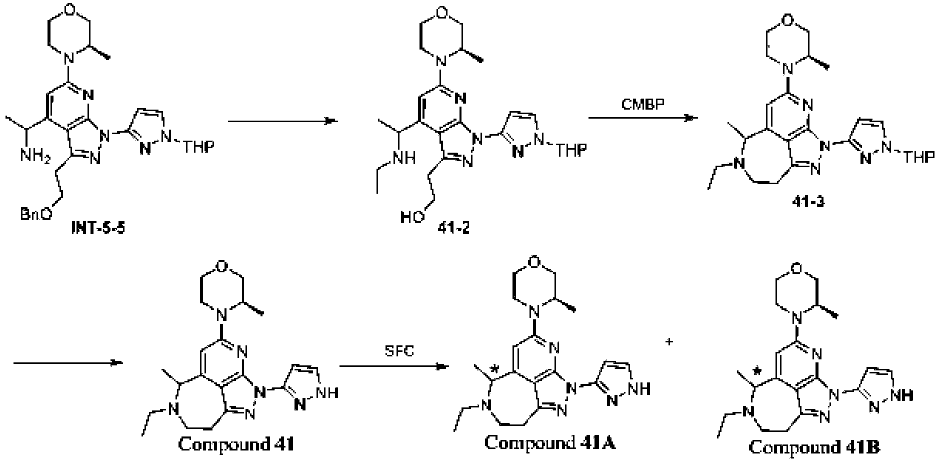

Step 1: Intermediate **INT-5-5** (5.0 g, 9.16 mmol) was dissolved in ethanol (50 mL), and wet palladium-on-carbon (1.9 g, 1.83 mmol) and palladium hydroxide-on-carbon (2.6 g, 1.83 mmol) were added. The mixture was stirred at room temperature under a hydrogen atmosphere (15 psi) for 48 hours, filtered and concentrated to obtain a crude product. The crude product was separated and purified by silica gel chromatography to give Intermediate **41-2** (0.4 g, 9.0%). LCMS (ESI) [M+H]⁺: 484.2.

Step 2: Similar to the preparation method in Example 35, the title Compound **41** was prepared using Intermediate **41-2** instead of Intermediate **35-2A.** A diastereomeric mixture of (3*R*)-4-(7-ethyl-6-methyl-2-(1*H*-pyrazol-3-yl)-6,7,8,9-tetrahydro-2*H*-1,2,3,7-tetraazabenzo[*cd*]az ulen-4-yl)-3-methylmorpholine (Compound **41**) (95 mg, 77.3%) was obtained. LCMS (ESI) [M+H]⁺: 382.2.

Step 3: The diastereomeric mixture of (3*R*)-4-(7-ethyl-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **41**) (95 mg) was further purified by chiral SFC. SFC analysis: Daicel ChiralPAK^{®} OD column (100 × 3.0 mm, 3 µm), mobile phase: 20% methanol in CO₂, temperature: 35°C, flow rate: 1.5 mL/min, detection wavelength: 210 nm.

The first peak was (3*R*)-4-(7-ethyl-6-methyl-2-(1*H*-pyrazol-3-yl)-6,7,8,9-tetrahydro-2*H-*1,2,3,7-tetraazabenzo[*cd*]azulen-4-yl)-3-methylmorpholine (Compound **41A,** Isomer 1) (15mg). LCMS (ESI) [M+H]⁺: 382.2. HPLC-SFC: Rt = 2.713 min. ¹H NMR (400 MHz, DMSO-d6) δ 12.71 (s, 1H), 7.80 (s, 1H), 6.76 (s, 1H), 6.56 (s, 1H), 4.49 - 4.37 (m, 1H), 4.37 - 4.21 (m, 1H), 4.02 (d, *J* = 13.3 Hz, 1H), 3.96 (dd, *J =* 11.5, 3.2 Hz, 2H), 3.75 (d, *J =* 11.3 Hz, 1H), 3.65 (dd, *J* = 11.3, 3.0 Hz, 1H), 3.57 - 3.41 (m, 2H), 3.17 (dd, *J* = 12.6, 3.2 Hz, 1H), 3.13 - 3.03 (m, 2H), 3.01 - 2.87 (m, 1H), 2.86 - 2.72 (m, 1H), 2.68 - 2.58 (m, 1H), 1.43 (d, *J =* 6.9 Hz, 3H), 1.19 (d, *J* = 6.7 Hz, 3H), 1.09 (t, *J =* 6.9 Hz, 3H).

The second peak was (3*R*)-4-(7-ethyl-6-methyl-2-(1*H*-pyrazol-3-yl)-6,7,8,9-tetrahydro-2*H-*1,2,3,7-tetraazabenzo[*cd*]azulen-4-yl)-3-methylmorpholine (Compound **41B,** Isomer 2) (15 mg). LCMS (ESI) [M+H]⁺: 382.2. HPLC-SFC: Rt = 3.488 min. ¹H NMR (400 MHz, DMSO-d6) δ 12.74 (s, 1H), 7.79 (s, 1H), 6.76 (s, 1H), 6.56 (s, 1H), 4.50 - 4.39 (m, 1H), 4.37 - 4.25 (m, 1H), 4.09 - 3.91 (m, 2H), 3.76 (d, *J =* 10.5 Hz, 1H), 3.64 (d, *J =* 9.5 Hz, 1H), 3.57 - 3.44 (m, 2H), 3.23 - 3.03 (m, 3H), 2.95 (d, *J =* 17.9 Hz, 1H), 2.84 - 2.72 (m, 1H), 2.70 - 2.59 (m, 1H), 1.44 (brs, 3H), 1.18 (d, *J* = 5.9 Hz, 3H), 1.09 (brs, 3H).

### Example 42A and Example 42B: Preparation of 1-(6-methyl-4-((R)-3-methylmorpholinyl)-2-(1H-pyrazol-3-yl)-2,6,8,9-tetrahydro-7H-1,2,3,7-tetraazabenzo[[cd]azulen-7-yl)cyclopropane-1-carbonitrile (Compound 42A, Isomer 1) and preparation of 1-(6-methyl-4-((R)-3-methylmorpholinyl)-2-(1H-pyrazol-3-yl)-2,6,8,9-tetrahydro-7H-1,2,3,7-tetr aazabenzo[[cd]azulen-7-yl)cyclopropane-1-carbonitrile (Compound 42B, Isomer 2)

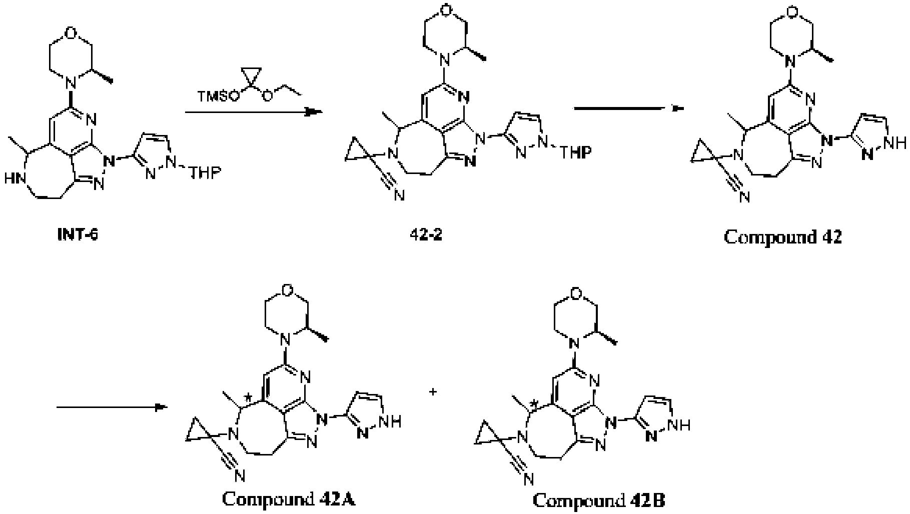

Step 1: **INT-6** (200 mg, 0.46 mmol) was dissolved in isopropanol (2.0 mL), and 1-ethoxy-1-trimethylsilyloxycyclopropane (79.7 mg, 0.46 mmol), trimethylsilyl cyanide (90.7 mg, 0.92 mmol), and acetic acid (0.2 mL) were added in sequence. The reaction mixture was stirred at 60°C for 18 hours. The reaction was quenched by adding ice water (20 mL), and the aqueous phase was extracted with ethyl acetate (20 mL × 3). The organic phase was washed sequentially with water and saturated brine, dried over anhydrous sodium sulfate, and filtered. The filtrate was concentrated under reduced pressure to obtain a crude product, which was purified by silica gel chromatography to yield Intermediate **42-2** (100 mg, 43.5%). LCMS (ESI) [M+H]⁺: 503.2.

Step 2: Intermediate **42-2** (100 mg, 0.20 mmol) was dissolved in dichloromethane (1 mL), added dropwise with trifluoroacetic acid (2 mL), and stirred at room temperature for half an hour. The reaction mixture was concentrated. Ice water (10 mL) was added to the residue, and then the residue was extracted with ethyl acetate (20 mL × 3). The combined organic phases were washed sequentially with water and saturated brine, dried over anhydrous sodium sulfate, and filtered. The filtrate was concentrated under reduced pressure to obtain a crude product. The crude product was separated and purified by silica gel column chromatography to obtain a diastereomeric mixture of 1-(6-methyl-4-((*R*)-3-methylmorpholinyl)-2-(1*H*-pyrazol-3-yl)-2,6,8,9-tetrahydro-7*H*-1,2,3,7-tetraazabenzo[[*cd*]azulen-7-yl)cyclopropane-1-carbonitrile (Compound **42**) (30 mg, 36.0%). LCMS (ESI) [M+H]⁺: 419.2.

Step 3: The diastereomeric mixture of 1-(6-methyl-4-((*R*)-3-methylmorpholinyl)-*2*-(1*H*-pyrazol-3-yl)-2,6,8,9-tetrahydro-7*H*-1,2,3,7-tetraazabenzo[[*cd*]azulen-7-yl)cyclopropane-1-carbonitrile (Compound **42**) (30 mg) was further purified by chiral SFC. SFC analysis: WHELK-O^{®}1 column (150 × 4.6 mm, 5 µm), mobile phase: 40% methanol in CO₂, temperature: 35°C, flow rate: 2.0 mL/min, detection wavelength: 210 nm.

The first peak was 1-(6-methyl-4-((*R*)-3-methylmorpholinyl)-2-(1*H*-pyrazol-3-yl)-2,6,8,9-tetrahydro-7*H*-1,2,3,7-tetraazabenzo[[*cd*]azulen-7-yl)cyclopropane-1-carbonitrile (Compound **42A,** Isomer 1) (8.0 mg). LCMS (ESI) [M+H]⁺: 419.2. HPLC-SFC: Rt = 5.205 min. ¹H NMR (400 MHz, CDCl₃) δ 7.68 (s, 1H), 6.86 (s, 1H), 6.27 (s, 1H), 4.58 - 4.49 (m, 1H), 4.44 - 4.35 (m, 1H), 4.11 - 3.96 (m, 2H), 3.88 - 3.77 (m, 2H), 3.69 - 3.58 (m, 2H), 3.38 - 3.13 (m, 4H), 1.61 (d, *J* = 6.8 Hz, 3H), 1.32 (d, *J =* 6.6 Hz, 3H), 1.29 - 1.23 (m, 3H), 1.19 - 1.13 (m, 1H).

The second peak was 1-(6-methyl-4-((*R*)-3-methylmorpholinyl)-2-(1*H*-pyrazol-3-yl)-2,6,8,9-tetrahydro-7*H*-1,2,3,7-tetraazabenzo[[*cd*]azulen-7-yl)cyclopropane-1-carbonitrile (Compound **42B,** Isomer 2) (4.7 mg). LCMS (ESI) [M+H]⁺: 419.2. HPLC-SFC: Rt = 5.951 min. ¹H NMR (400 MHz, CDCl₃) δ 7.76 (s, 1H), 6.91 (s, 1H), 6.28 (s, 1H), 4.58 - 4.50 (m, 1H), 4.42 - 4.34 (m, 1H), 4.12 - 3.98 (m, 2H), 3.88 - 3.76 (m, 2H), 3.70 - 3.59 (m, 2H), 3.38 - 3.14 (m, 4H), 1.62 (d, *J =* 6.8 Hz, 3H), 1.34 (d, *J =* 6.6 Hz, 3H), 1.33 - 1.25 (m, 3H), 1.20 - 1.15 (m, 1H).

### Example 43: Preparation of (3R)-3-methyl-4-(6-methyl-7-(1-methyl-1H-pyrazol-4-yl)-2-(1H-pyrazol-3-yl)-6,7,8,9-tetrahydro-2H-1,2,3,7-tetraazabenzo[cd]azulen-4-yl)morpholine (Compound 43)

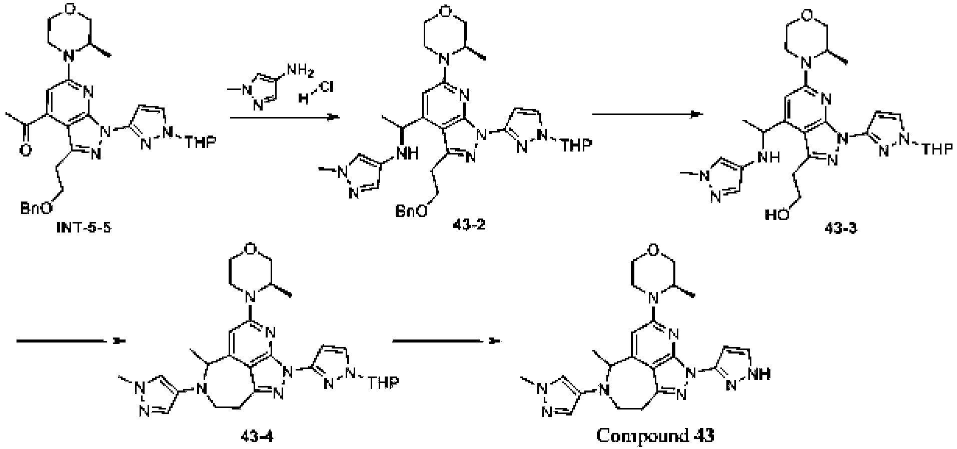

Step 1: Intermediate **INT-5-5** (500 mg, 0.92 mmol) and 1-methyl-1*H*-pyrazol-4-amine hydrochloride (367.9 mg, 2.75 mmol) were dissolved in methanol (10 mL) at room temperature, and tetraethyl titanate (521.8 mg, 1.84 mmol) was added. The reaction mixture was stirred at room temperature for 1 hour. Sodium triacetoxyborohydride (387.2 mg, 1.84 mmol) was then added to the reaction mixture in batches, and the reaction mixture was stirred continuously overnight. The reaction was quenched by adding ice water (10 mL), and extracted with dichloromethane (10 mL × 3). The combined organic phases were washed sequentially with water and saturated brine, dried over anhydrous sodium sulfate, and filtered. The filtrate was concentrated under reduced pressure to obtain a crude product. The crude product was purified by silica gel column chromatography to yield Intermediate **43-2** (200 mg, 34.8%). LCMS (ESI) [M+H]⁺: 626.4.

Step 2: Intermediate **43-2** (200 mg, 0.32 mmol) was dissolved in ethanol (10 mL) at room temperature, and wet palladium-on-carbon (68.0 mg, 0.064 mmol) and palladium hydroxide-on-carbon (89.7 mg, 0.064 mmol) were added. The reaction mixture was stirred at 50°C overnight under a hydrogen atmosphere (15 psi). The reaction mixture was filtered, and the filtrate was concentrated under reduced pressure to obtain a crude product. The crude product was purified by silica gel column chromatography to obtain Intermediate **43-3** (60 mg, 35.0%). LCMS (ESI) [M+H]⁺: 536.4.

Step 3: Intermediate **43-3** (30 mg, 0.056 mmol) was dissolved in dichloromethane (2 mL) at room temperature, and triethylamine (0.04 mL, 0.28 mmol) and methanesulfonyl chloride (9.6 mg, 0.084 mmol) were added. The mixture was stirred at room temperature overnight. The reaction was quenched by adding ice water (5 mL), and extracted with dichloromethane (5 mL × 3). The combined organic phases were washed sequentially with water and saturated brine, dried over anhydrous sodium sulfate, and filtered. The filtrate was concentrated under reduced pressure to yield a crude product. The crude product was separated and purified by silica gel column chromatography to obtain Intermediate **43-4** (10 mg, 34.5%). LCMS (ESI) [M+H]⁺: 518.3.

Step 4: Intermediate **43-4** (10 mg, 0.019 mmol) was dissolved in methanol (1 mL) at room temperature, and hydrochloric acid/ethyl acetate (0.05 mL, 4 M) was added. The reaction mixture was stirred at room temperature for 2 hours. After the reaction was completed, the crude product was concentrated and the pH was adjusted to 7 by adding saturated sodium bicarbonate solution. The aqueous phase was extracted with dichloromethane (5 mL × 3). The combined organic phases were washed sequentially with water and saturated brine, dried over anhydrous sodium sulfate, and filtered. The filtrate was concentrated under reduced pressure to obtain a crude product. The crude product was separated and purified by silica gel column chromatography to give (3*R*)-3-methyl-4-(6-methyl-7-(1-methyl-1*H*-pyrazol-4-yl)-*2*-(1*H*-pyrazol-3-yl)-6,7,8,9-tetrahydro-2*H*-1,2,3,7-tetraazabenzo[*cd*]azulen-4-yl)morpholine (Compound **43**) (4.1 mg, 48.9%). LCMS (ESI) [M+H]⁺: 434.4. ¹H NMR (400 MHz, DMSO-d6) δ 12.70 (s, 1H), 7.78 (s, 1H), 7.06 (d, *J =* 1.4 Hz, 2H), 6.73 (s, 2H), 4.77 (d, *J =* 6.8 Hz, 1H), 4.43 (s, 1H), 4.15 - 3.86 (m, 3H), 3.82 - 3.72 (m, 1H), 3.72 - 3.58 (m, 5H), 3.57 - 3.46 (m, 1H), 3.45 - 3.35 (m, 1H), 3.17 (t, *J =* 12.8 Hz, 1H), 2.90 (d, *J =* 16.8 Hz, 1H), 1.56 (d, *J =* 7.2 Hz, 3H), 1.20 (d, *J =* 7.8 Hz, 3H).

### Example 44A and Example 44B: Preparation of (3R)-3-methyl-4-(6-methyl-7-(oxetan-3-ylmethyl)-2-(1H-pyrazol-3-yl)-6,7,8,9-tetrahydro-2H-1,2,3,7-tetraazabenzo[cd]azule n-4-yl)morpholine (Compound 44A, Isomer 1) and preparation of (3R)-3-methyl-4-(6-methyl-7-(oxetan-3-ylmethyl)-2-(1H-pyrazol-3-yl)-6,7,8,9-tetrahydro-2H-1,2,3,7-tetraazabenzo[cd]azule n-4-yl)morpholine (Compound 44B, Isomer 2)

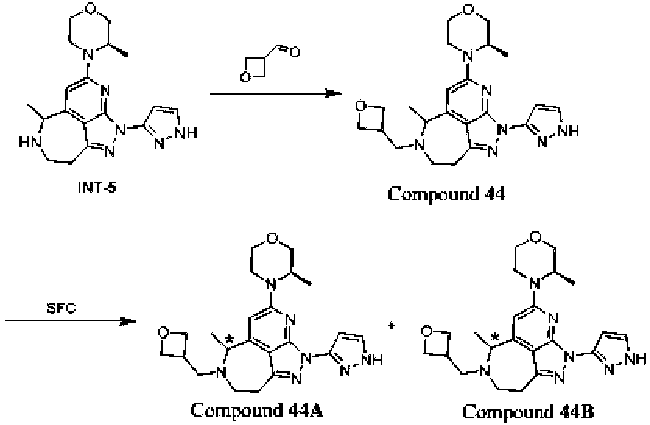

Step 1: Similar to the preparation method in Example 32A, the title Compound **44** was prepared using oxetane-3-carboxaldehyde instead of 3-oxetanone. A diastereomeric mixture of (3*R*)-3-methyl-4-(6-methyl-7-(oxetan-3-ylmethyl)-2-(1*H*-pyrazol-3-yl)-6,7,8,9-tetrahydro-2*H*-1, 2,3,7-tetraazabenzo[*cd*]azulen-4-yl)morpholine (Compound **44**) (100 mg, 41.7%) was obtained. LCMS (ESI) [M+H]⁺: 424.2.

Step 2: The diastereomeric mixture of (3*R*)-3-methyl-4-(6-methyl-7-(oxetan-3-ylmethyl)-2-(1*H-*pyrazol-3-yl)-6,7,8,9-tetrahydro-2*H*-1,2,3,7-tetraazabenzo[*cd*]azulen-4-yl)morpholine (Compound **44**) (100 mg) was further purified by chiral SFC. SFC analysis: Daicel ChiralPAK^{®}AS column (100 × 3.0 mm, 3 µm), mobile phase: 10% methanol (containing 0.1% ammonia) in CO₂, temperature: 35°C, flow rate: 1.5 mL/min, detection wavelength: 210 nm.

The first peak was (3*R*)-3-methyl-4-(6-methyl-7-(oxetan-3-ylmethyl)-2-(1*H*-pyrazol-3-yl)-6,7,8,9-tetrahydro-2*H*-1,2,3,7-tetraazabenzo[*cd*]azulen-4-yl)morpholine (Compound **44A,** Isomer 1) (865.8 mg). LCMS (ESI) [M+H]⁺: 424.2. HPLC-SFC: Rt = 3.156 min. ¹H NMR (400 MHz, DMSO-d6) δ 12.71 (s, 1H), 7.80 (s, 1H), 6.75 (s, 1H), 6.54 (s, 1H), 4.71 - 4.55 (m, 2H), 4.42 (d, *J =* 4.8 Hz, 1H), 4.21 - 4.18 (m, 3H), 4.09 - 3.89 (m, 2H), 3.75 (d, *J =* 11.2 Hz, 1H), 3.68 - 3.61 (m, 1H), 3.49 (t, *J =* 10.8 Hz, 2H), 3.24 - 3.06 (m, 3H), 3.04 - 2.81 (m, 4H), 1.44 (d, *J* = 6.8 Hz, 3H), 1.18 (d, *J =* 6.8 Hz, 3H).

The second peak was (3*R*)-3-methyl-4-(6-methyl-7-(oxetan-3-ylmethyl)-2-(1*H*-pyrazol-3-yl)-6,7,8,9-tetrahydro-2*H*-1,2,3,7-tetraazabenzo[*cd*]azulen-4-yl)morpholine (Compound **44B,** Isomer 2) (6.2 mg). LCMS (ESI) [M+H]⁺: 424.2. HPLC-SFC: Rt = 4.043 min. ¹H NMR (400 MHz, DMSO-d6) δ 12.71 (s, 1H), 7.79 (s, 1H), 6.75 (s, 1H), 6.53 (s, 1H), 4.66 - 4.56 (m, 2H), 4.42 (d, *J =* 5.6 Hz, 1H), 4.25 - 4.13 (m, 3H), 3.98 - 3.88 (m, 2H), 3.75 (d, *J =* 11.2 Hz, 1H), 3.69 - 3.59 (m, 1H), 3.49 (t, *J =* 11.6 Hz, 2H), 3.27 - 3.07 (m, 3H), 3.03 - 2.79 (m, 4H), 1.43 (d, *J* = 7.2 Hz, 3H), 1.17 (d, *J =* 6.8 Hz, 3H).

### Examples 45A and 45B: Preparation of (3R)-4-(7-(2,2-difluoroethyl)-6-methyl-2-(1H-pyrazol-3-yl)-6,7,8,9-tetrahydro-2H-1,2,3,7-tetraazabenzo[cd]azulen-4-yl)-3-methylmorp holine (Compound 45A, Isomer 1) and preparation of (3R)-4-(7-(2,2-difluoroethyl)-6-methyl-2-(1H-pyrazol-3-yl)-6,7,8,9-tetrahydro-2H-1,2,3,7-tetraazabenzo[cd]azulen-4-yl)-3-methylmorp holine (Compound 45B, Isomer 2)

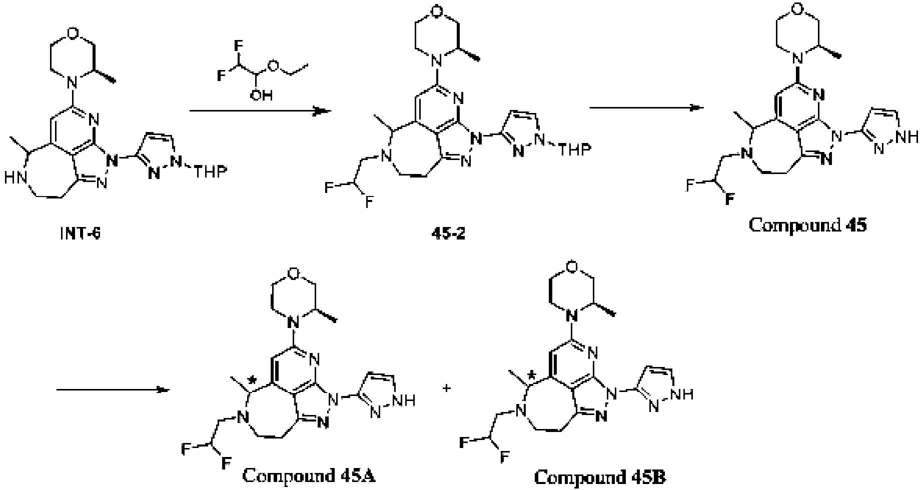

Step 1: Similarly to the preparation method in Example 36A, the title Compound **45** was prepared using difluoroacetaldehyde ethyl hemiacetal instead of acetone. A diastereomeric mixture of (3*R*)-4-(7-(2,2-difluoroethyl)-6-methyl-2-(1*H*-pyrazol-3-yl)-6,7,8,9-tetrahydro-2*H-*1,2,3,7-tetraazabenzo[*cd*]azulen-4-yl)-3-methylmorpholine (Compound **45**) (95 mg, 63.8%) was obtained. LCMS (ESI) [M+H]⁺: 418.2.

Step 2: The diastereomeric mixture of (3*R*)-4-(7-(2,2-difluoroethyl)-6-methyl-2-(1*H*-pyrazol-3-yl)-6,7,8,9-tetrahydro-2*H*-1,2,3,7-tetraazabenzo[*cd*]azulen-4-yl)-3-methylmorpho line (Compound **45**) (100 mg) was further purified by chiral SFC. SFC analysis: Daicel ChiralPAK^{®} OD column (150 × 4.6 mm, 5 µm), mobile phase: 20% methanol in n-hexane, temperature: 35°C, flow rate: 1.0 mL/min, detection wavelength: 210 nm.

The first peak was (3*R*)-4-(7-(2,2-difluoroethyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **45A,** Isomer 1) (40 mg). LCMS (ESI) [M+H]⁺: 418.2. HPLC-SFC: Rt = 2.484 min. ¹H NMR (400 MHz, DMSO-d6) δ 12.69 (s, 1H), 7.76 (s, 1H), 6.73 (s, 1H), 6.48 (s, 1H), 6.05 (tt, *J =* 56.4, 4.2 Hz, 1H), 4.42 - 4.35 (m, 1H), 4.35 - 4.26 (m, 1H), 3.98 (d, *J =* 12.7 Hz, 1H), 3.95 - 3.89 (m, 1H), 3.71 (d, *J* = 11.3 Hz, 1H), 3.64 - 3.37 (m, 3H), 3.21 - 3.04 (m, 4H), 3.00 - 2.82 (m, 2H), 1.43 (d, *J =* 7.0 Hz, 3H), 1.15 (d, *J =* 6.6 Hz, 3H).

The second peak was (3*R*)-4-(7-(2,2-difluoroethyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **45B,** Isomer 2) (40 mg). LCMS (ESI) [M+H]⁺: 418.2. HPLC-SFC: Rt = 2.900 min. ¹H NMR (400 MHz, DMSO-d6) δ 12.69 (s, 1H), 7.76 (s, 1H), 6.73 (s, 1H), 6.48 (s, 1H), 6.05 (tt, *J =* 56.4, 4.2 Hz, 1H), 4.44 - 4.36 (m, 1H), 4.34 - 4.25 (m, 1H), 4.02 - 3.88 (m, 2H), 3.72 (d, *J =* 11.2 Hz, 1H), 3.65 - 3.40 (m, 3H), 3.22 - 3.02 (m, 4H), 3.00 - 2.81 (m, 2H), 1.43 (d, *J =* 7.0 Hz, 3H), 1.13 (d, *J =* 6.6 Hz, 3H).

### Example 46: Preparation of (3R)-3-methyl-4-(6-methyl-7-(S-methylsulfonylimino)-2-(1H-pyrazol-3-yl)-6,7,8,9-tetrahydro-2H-1,2,3,7-tetraazabenzo[cd]azulen-4-yl)morpholine (Compound 46)

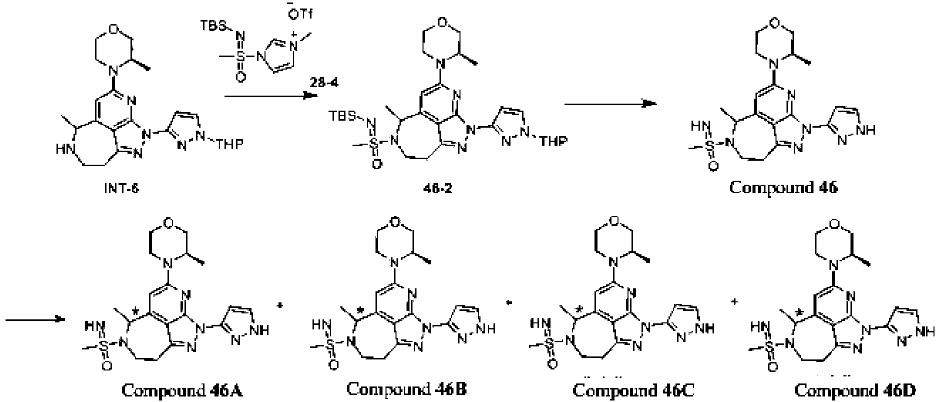

Step 1: Similar to the preparation method of Example 28, the title Compound **46** was prepared using Intermediate **INT-6** instead of Intermediate **INT-5-1.** An isomeric mixture of (3*R*)-3-methyl-4-(6-methyl-7-(*S*-methylsulfonylimino)-2-(1*H*-pyrazol-3-yl)-6,7,8,9-tetrahydro-2 *H*-1,2,3,7-tetraazabenzo[*cd*]azulen-4-yl)morpholine (Compound **46**) (150 mg, crude) was obtained. LCMS (ESI) [M+H]⁺: 431.2.

Step 2: The isomeric mixture of (3*R*)-3-methyl-4-(6-methyl-7-(*S*-methylsulfonylimino)-2-(1*H*-pyrazol-3-yl)-6,7,8,9-tetrahydro-2*H*-1,2,3,7-tetraazabenzo[*cd*]azulen-4-yl)morpholine (Compound **46**) (150 mg) was further purified by chiral SFC.

SFC analysis: Daicel ChiralPAK^{®} OJ column (100 × 3.0 mm, 3 µm), mobile phase: 20% methanol (containing 0.1% diethylamine) in CO₂, temperature: 35°C, flow rate: 1.5 mL/min, detection wavelength: 214 nm.

The first peak was (3*R*)-3-methyl-4-(6-methyl-7-(*S*-methylsulfonylimino)-2-(1*H*-pyrazol-3-yl)-6,7,8,9-tetrahydro-2*H*-1,2,3,7-tetraazabenzo[*cd*]azulen-4-yl)morpholine (Compound **46A,** Isomer 1) (12.0 mg). LCMS (ESI) [M+H]⁺: 431.2. HPLC-SFC: Rt = 2.781 min. ¹H NMR (400 MHz, DMSO-d6) δ 7.78 (s, 1H), 6.75 (d, *J =* 1.8 Hz, 1H), 6.69 (s, 1H), 5.55 (d, *J* = 7.1 Hz, 1H), 4.47 (d, *J =* 6.2 Hz, 1H), 4.05 - 3.92 (m, 3H), 3.80 (s, 1H), 3.76 (d, *J =* 11.4 Hz, 1H), 3.64 (d, *J =* 11.8 Hz, 2H), 3.50 (s,1H), 3.19 (d, *J =* 3.8 Hz, 1H), 3.16 (s, 1H), 3.04 (d, *J =* 16.9 Hz, 1H), 2.83 (s, 3H), 1.54 (d, *J =* 7.1 Hz, 3H), 1.17 (d, *J =* 6.6 Hz, 3H).

The second peak was (3*R*)-3-methyl-4-(6-methyl-7-(*S*-methylsulfonylimino)-2-(1*H-*pyrazol-3-yl)-6,7,8,9-tetrahydro-2*H-*1,2,3,7-tetraazabenzo[*cd*]azulen-4-yl)morpholine (Compound **46B,** Isomer 2) (12.0 mg). LCMS (ESI) [M+H]⁺: 431.2. HPLC-SFC: Rt = 3.237 min. ¹H NMR (400 MHz, DMSO-d6) δ 12.75 (s, 1H), 7.79 (s, 1H), 6.76 (d, *J =* 1.6 Hz, 1H), 6.69 (s, 1H), 5.50 (q, *J =* 6.9 Hz, 1H), 4.45 (d, *J =* 5.4 Hz, 1H), 4.07 - 3.90 (m, 3H), 3.85 (s, 1H), 3.76 (d, *J = 11.3* Hz, 1H), 3.69 - 3.56 (m, 2H), 3.49 (dd, *J =* 11.6, 9.4 Hz, 1H), 4.32 - 3.22 (m, 1H), 3.14 (td, *J =* 12.7, 3.3 Hz, 1H), 3.03 (d, *J =* 17.0 Hz, 1H), 2.80 (3, 3H), 1.54 (d, *J =* 7.1 Hz, 3H), 1.17 (d, *J =* 6.6 Hz, 3H).

SFC analysis: Daicel ChiralPAK^{®} OD column (100 × 3.0 mm, 3 µm), mobile phase: 20% methanol (containing 0.1% diethylamine) in CO₂, temperature: 35°C, flow rate: 1.5 mL/min, detection wavelength: 214 nm.

The third peak was (3*R*)-3-methyl-4-(6-methyl-7-(*S*-methylsulfonylimino)-2-(1*H*-pyrazol-3-yl)-6,7,8,9-tetrahydro-2*H*-1,2,3,7-tetraazabenzo[*cd*]azulen-4-yl)morpholine (Compound **46C,** Isomer 3) (10.0 mg). LCMS (ESI) [M+H]⁺: 431.4. HPLC-SFC: Rt = 4.059 min. ¹H NMR (400 MHz, CDCl₃) δ 7.63 (s, 1H), 6.86 (s, 1H), 6.28 (s, 1H), 5.65 (t, *J =* 6.8 Hz, 1H), 4.38 (t, *J =* 5.8 Hz, 1H), 4.19 - 3.94 (m, 3H), 23.83 (dd, *J =* 24.4, 11.3 Hz, 2H), 3.65 (t, *J =* 12.5 Hz, 2H), 3.50 (d, *J =* 3.9 Hz, 1H), 3.37 (dd, *J =* 12.8,3.5 Hz, 1H), 3.25 (d, *J =* 17.1 Hz, 1H), 2.91 (s, 3H), 1.61 (d, *J* = 7.1 Hz, 3H), 1.34 (d, *J =* 6.7 Hz, 3H).

The fourth peak was (3*R*)-3-methyl-4-(6-methyl-7-(*S*-methylsulfonylimino)-2-(1*H*-pyrazol-3-yl)-6,7,8,9-tetrahydro-2*H*-1,2,3,7-tetraazabenzo[*cd*]azulen-4-yl)morpholine (Compound **46D,** Isomer 4) (8.0 mg). LCMS (ESI) [M+H]⁺: 431.4. HPLC-SFC: Rt = 4.970 min. ¹H NMR (400 MHz, CDCl₃) δ 7.81 (s, 1H), 6.76 (s, 1H), 6.69 (s, 1H), 5.51 (d, *J* = 7.1 Hz, 1H), 4.44 (d, *J* = 4.8 Hz, 1H), 4.14 - 3.89 (m, 3H), 3.84 (s, 1H), 3.75 (d, *J =* 11.3 Hz, 1H), 3.64 -3.56 (m, 2H), 3.49 (t, *J = 10.6* Hz, 1H), 3.28 (d, *J =* 13.3 Hz, 1H), 3.20 - 3.10 (m,1H), 3.02 (d, *J =* 16.9 Hz, 1H), 2.79(3, 3H), 1.54 (d, *J* = 7.1 Hz, 3H), 1.19 (d, *J =* 6.6 Hz, 3H).

### Example 47A and Example 47B: Preparation of (3R)-3-methyl-4-(6-methyl-7-(methyl-d3)-2-(1H-pyrazol-3-yl)-6,7,8,9-tetrahydro-2H-1,2,3,7-tetraazabenzo[cd]azulen-4-yl)morpholine (Compound 47A, Isomer 1) and preparation of (3R)-3-methyl-4-(6-methyl-7-(methyl-d3)-2-(1H-pyrazol-3-yl)-6,7,8,9-tetrahydro-2H-1,2,3,7-tetraazabenzo[cd]azulen-4-yl)morpholine (Compound 47B, Isomer 2)

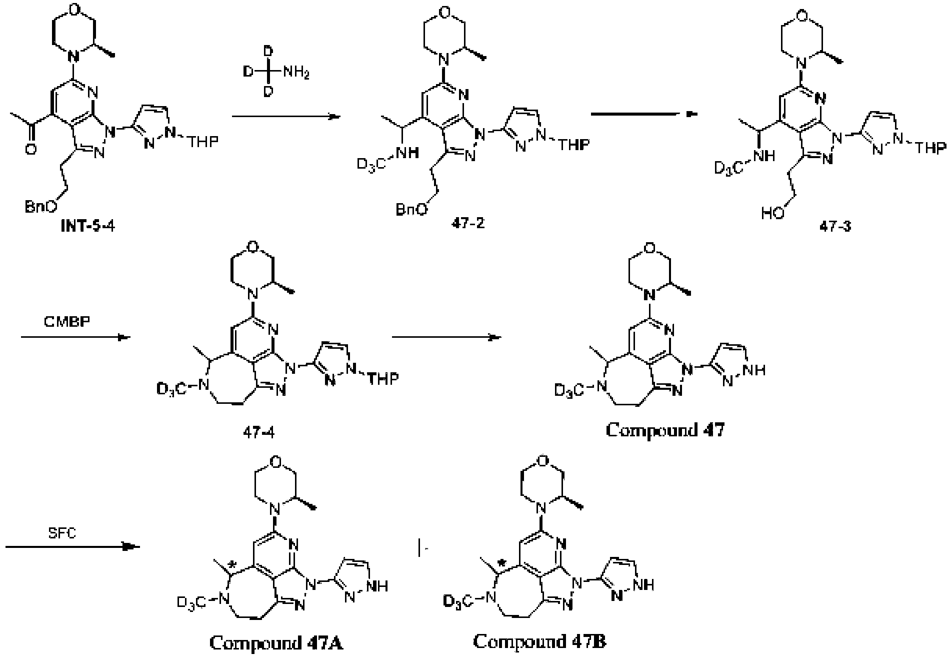

Step 1: Similar to the preparation method in Example 43, the title compound **47** was prepared by using deuterated methylamine hydrochloride instead of 1-methyl-1*H*-pyrazol-4-amine hydrochloride. A diastereomeric mixture of (3*R*)-3-methyl-4-(6-methyl-7-(methyl-d3)-2-(1*H*-pyrazol-3-yl)-6,7,8,9-tetrahydro-2*H*-1,2,3,7-tetr aazabenzo[*cd*]azulen-4-yl)morpholine (Compound **47**) (200 mg, 76.8%) was obtained. LCMS (ESI) [M+H]⁺: 370.8.

Step 2: The diastereomeric mixture of (3*R*)-3-methyl-4-(6-methyl-7-(methyl-d3)-2-(1*H*-pyrazol-3-yl)-6,7,8,9-tetrahydro-2*H*-1,2,3,7-tetraazabenzo[*cd*]azulen-4-yl)morpholine (Compound **47**) (100 mg) was further purified by chiral SFC. SFC analysis: Daicel ChiralCel OD column (150 × 4.6 mm, 5 µm), mobile phase: 20% methanol (containing 0.1% ammonia) in CO₂, flow rate: 1.0 mL/min, detection wavelength: 210 nm.

The first peak was (3*R*)-3-methyl-4-(6-methyl-7-(methyl-d3)-2-(1*H*-pyrazol-3-yl)-6,7,8,9-tetrahydro-2*H*-1,2,3,7-tetraazabenzo[*cd*]azulen-4-yl)morpholine (Compound **47A,** Isomer 1) (42 mg). LCMS (ESI) [M+H]⁺: 370.8. HPLC-SFC: Rt = 2.571 min. ¹H NMR (400 MHz, DMSO-d6) δ 12.73 (s, 1H), 7.81 (s, 1H), 6.77 (s, 1H), 6.55 (s, 1H), 4.43 (d, *J* = 5.2 Hz, 1H), 4.20 (s, 1H), 4.08 - 3.91 (m, 2H), 3.75 (d, *J =* 11.3 Hz, 1H), 3.68 - 3.61 (m, 1H), 3.59 - 3.44 (m, 2H), 3.24 - 2.93 (m, 4H), 1.46 (d, *J =* 5.9 Hz, 3H), 1.18 (d, *J =* 6.6 Hz, 3H).

The second peak was (3*R*)-3-methyl-4-(6-methyl-7-(methyl-d3)-2-(1*H*-pyrazol-3-yl)-6,7,8,9-tetrahydro-2*H*-1,2,3,7-tetraazabenzo[*cd*]azulen-4-yl)morpholine (Compound **47B,** Isomer 2) (40 mg). LCMS (ESI) [M+H]⁺: 370.8. HPLC-SFC: Rt = 3.217 min. ¹H NMR (400 MHz, DMSO-d6) δ 12.73 (s, 1H), 7.81 (s, 1H), 6.77 (s, 1H), 6.55 (s, 1H), 4.44 (d, *J* = 4.6 Hz, 1H), 4.19 (s, 1H), 4.07 - 3.92 (m, 2H), 3.76 (d, *J =* 11.3 Hz, 1H), 3.68 - 3.60 (m, 1H), 3.59 - 3.45 (m, 2H), 3.25 - 2.93 (m, 4H), 1.46 (d, *J =* 5.8 Hz, 3H), 1.18 (d, *J =* 6.6 Hz, 3H).

### Example 48A and Example 48B: Preparation of (3R)-4-(7-(Ethylsulfonyl)-6-methyl-2-(1H-pyrazol-3-yl)-6,7,8,9-tetrahydro-2H-1,2,3,7-tetraazabenzo[cd]azulen-4-yl)-3-methylmorp holine (Compound 48A, Isomer 1) and preparation of (3R)-4-(7-(Ethylsulfonyl)-6-methyl-2-(1H-pyrazol-3-yl)-6,7,8,9-tetrahydro-2H-1,2,3,7-tetraazabenzo[cd]azulen-4-yl)-3-methylmorp holine (Compound 48B, Isomer 2)

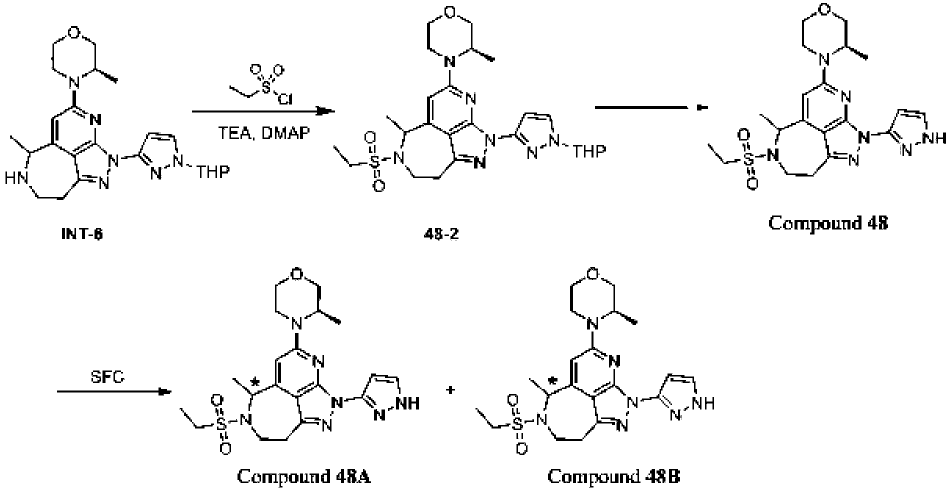

Step 1: Similar to the preparation method in Example 37, the title Compound **48** was prepared using ethanesulfonyl chloride instead of cyclopropanesulfonyl chloride. A diastereomeric mixture of (3*R*)-4-(7-(ethylsulfonyl)-6-methyl-2-(1*H*-pyrazol-3-yl)-6,7,8,9-tetrahydro-2*H-*1,2,3,7-tetraazabenzo[*cd*]azulen-4-yl)-3-methylmorpholine (Compound **48**) (80 mg, 54.4%) was obtained. LCMS (ESI) [M+H]⁺: 446.2.

Step 2: The diastereomeric mixture of (3*R*)-4-(7-(ethylsulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **48**) (80 mg) was further purified by chiral SFC. SFC analysis: Daicel ChiralPAK IH column (150 × 4.6 mm, 5 µm), mobile phase: 15% methanol in n-hexane, temperature: 35°C, flow rate: 2.0 mL/min, detection wavelength: 254 nm.

The first peak was (3*R*)-4-(7-(ethylsulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **48A,** Isomer 1) (10.3 mg). LCMS (ESI) [M+H]⁺: 446.2. HPLC-SFC: Rt = 1.783 min. ¹H NMR (400 MHz, DMSO-d6) δ 12.76 (s, 1H), 7.82 (s, 1H), 6.75 - 6.73 (m, 2H), 5.38 - 5.36 (m, 1H), 4.44 - 4.42 (m, 1H), 4.05 - 4.03 (m, 1H), 3.97 - 3.95 (m, 2H), 3.76 - 3.74 (m, 2H), 3.66 - 3.64 (m, 1H), 3.50 - 3.48 (m, 1H), 3.15 - 3.13 (m, 5H), 1.58 (d, *J =* 7.2 Hz, 3H), 1.19 - 1.17 (m, 6H).

The second peak was (3R)-4-(7-(ethylsulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **48B,** Isomer 2) (12.8 mg). LCMS (ESI) [M+H]⁺: 446.3. HPLC-SFC: Rt = 2.790 min. ¹H NMR (400 MHz, DMSO-d6) δ 12.76 (s, 1H), 7.81 (s, 1H), 6.74 - 6.72 (m, 2H), 5.35 - 5.33 (m, 1H), 4.46 - 4.44 (m, 1H), 3.98 - 3.96 (m, 3H), 3.78 - 3.76 (m, 2H), 3.66 - 3.64 (m, 1H), 3.51 - 3.49 (m, 1H), 3.22 - 3.07 (m, 5H), 1.58 (d, *J =* 7.2 Hz, 3H), 1.22 - 1.15 (m, 6H).

### Example 49A and Example 49B: Preparation of (3R)-3-methyl-4-(6-methyl-7-((1-methylcyclopropyl)sulfonyl)-2-(1H-pyrazol-3-yl)-6,7,8,9-tetrahydro-2H-1,2,3,7-tetraazabenz o[cd]azulen-4-yl)morpholine (Compound 49A, Isomer 1) and preparation of (3R)-3-methyl-4-(6-methyl-7-((1-methylcyclopropyl)sulfonyl)-2-(1H-pyrazol-3-yl)-6,7,8,9-tetrahydro-2H-1,2,3,7-tetraazabenzo[cd]azulen-4-yl)morpholine (Compound 49B, Isomer 2)

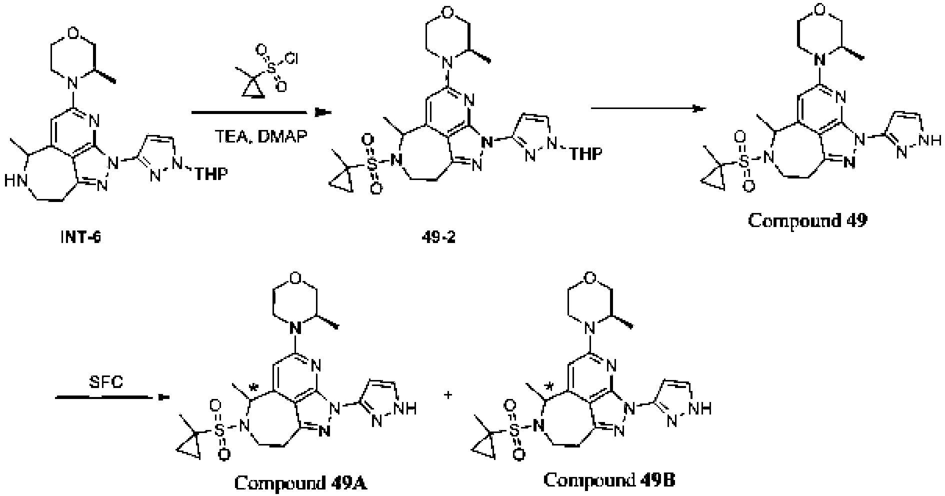

Step 1: Similarly to the preparation method in Example 37, the title Compound 49 was prepared using 1-methylcyclopropanesulfonyl chloride instead of cyclopropanesulfonyl chloride. A diastereomeric mixture of (3*R*)-3-methyl-4-(6-methyl-7-((1-methylcyclopropyl)sulfonyl)-2-(1*H*-pyrazol-3-yl)-6,7,8,9-tetrahydro-2*H*-1,2,3,7-tetraazabenzo[*cd*]azulen-4-yl)morpholine (Compound **49**) (50 mg, 65.5%) was obtained. LCMS (ESI) [M+H]⁺: 472.4.

Step 2: The diastereomeric mixture (3*R*)-3-methyl-4-(6-methyl-7-((1-methylcyclopropyl) sulfonyl)-2-(1*H*-pyrazol-3-yl)-6,7,8,9-tetrahydro-2*H*-1,2,3,7-tetraazabenzo[*cd*]azulen-4-yl)morp holine (Compound **49**) (50 mg) was further purified by chiral SFC. SFC analysis method: Daicel ChiralPAK OJ column (150 × 4.6 mm, 5 µm), mobile phase: 10% methanol in n-hexane, flow rate: 1.0 mL/min, detection wavelength: 210 nm.

The first peak was (3*R*)-3-methyl-4-(6-methyl-7-((1-methylcyclopropyl)sulfonyl)-2-(1*H*-pyrazol-3-yl)-6,7,8,9-tetrahydro-2*H*-1,2,3,7-tetraazabenzo[*cd*]azulen-4-yl)morpholine (Compound **49A,** Isomer 1) (15 mg). LCMS (ESI) [M+H]⁺: 472.4. HPLC-SFC: Rt = 2.428 min. ¹H NMR (400 MHz, DMSO-d6) δ 12.76 (s, 1H), 7.82 (s, 1H), 6.78 (s,1H), 6.77 (s,1H), 5.35 (t, *J* = 7.0 Hz, 1H), 4.51 - 4.36 (m, 1H), 4.09 - 3.94 (m, 3H), 3.89 - 3.74 (m, 2H), 3.71 - 3.61 (m, 1H), 3.59 - 3.46 (m, 1H), 3.22 - 3.07 (m, 3H), 1.63 (d, *J =* 7.0 Hz, 3H), 1.37 (s, 3H), 1.35 - 1.31 (m, 1H), 1.26 - 1.23 (m, 1H), 1.18 (d, *J =* 6.6 Hz, 3H), 0.99 - 0.92 (m, 1H), 0.90 - 0.82 (m, 1H).

The second peak was (3*R*)-3-methyl-4-(6-methyl-7-((1-methylcyclopropyl)sulfonyl)-2-(1*H-*pyrazol-3-yl)-6,7,8,9-tetrahydro-2*H*-1,2,3,7-tetraazabenzo[*cd*]azulen-4-yl)morpholine (Compound **49B,** Isomer 2) (15 mg). LCMS (ESI) [M+H]⁺: 472.4. HPLC-SFC: Rt = 3.462 min. ¹H NMR (400 MHz, DMSO-d6) δ 12.76 (s, 1H), 7.82 (s, 1H), 6.78 (s, 1H), 6.77 (s, 1H), 5.45 - 5.28 (m, 1H), 4.55 - 4.35 (m, 1H), 4.15 - 3.89 (m, 3H), 3.89 - 3.71 (m, 2H), 3.70 - 3.61 (m, 1H), 3.54 - 3.46 (m, 1H), 3.22 - 3.07 (m, 3H), 1.62 (d, *J =* 6.7 Hz, 3H), 1.40 - 1.35 (m, 1H), 1.34 (s, 3H), 1.27 - 1.23 (m, 1H), 1.20 (d, *J =* 6.3 Hz, 3H), 0.98 - 0.90 (m, 1H), 0.88 - 0.81 (m, 1H).

### Example 50: Preparation of diastereomeric mixture of (3R)-3-methyl-4-(6-methyl-2-(1H-pyrazol-3-yl)-7-(vinylsulfonyl)-6,7,8,9-tetrahydro-2H-1,2,3,7-tetraazabenzo[cd]azulen-4-yl) morpholine (Compound 50)

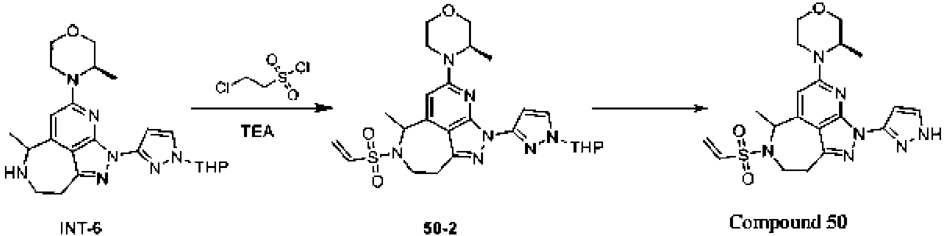

Step 1: Intermediate **INT-6** (1.0 g, 2.29 mmol) was dissolved in acetonitrile (50 mL), and triethylamine (0.32 mL, 2.29 mmol) and 2-chloroethanesulfonyl chloride (373 mg, 2.29 mmol) were added in sequence. The mixture reacted at room temperature for 2 hours, then saturated ammonium chloride (50 mL) was added to quench the reaction. The aqueous phase was extracted with ethyl acetate (200 mL × 3). The combined organic phases were washed sequentially with water and saturated brine, dried over anhydrous sodium sulfate, filtered, and concentrated to obtain a crude product, which was purified by silica gel column chromatography to yield Intermediate **50-2** (500 mg, 41.5%). LCMS (ESI) [M+H]⁺: 528.2.

Step 2: Intermediate **50-2** (50 mg, 0.09 mmol) was dissolved in methanol (1 mL), and hydrochloric acid (1 mL, 1 M) was added. The reaction mixture was stirred at room temperature for 2 hours. The reaction mixture was concentrated to dryness to give a crude product, which was then separated and purified by silica gel column chromatography to yield a diastereomeric mixture of (3*R*)-3-methyl-4-(6-methyl-2-(1*H*-pyrazol-3-yl)-7-(vinylsulfonyl)-6,7,8,9-tetrahydro-2*H*-1,2,3,7-tetraazabenzo[*cd*]azulen-4-yl)morpholine (Compound **50**) (15 mg, 35.7%). LCMS (ESI) [M+H]⁺: 444.4. ¹H NMR (400 MHz, DMSO-d6) δ 12.75 (s, 1H), 7.80 (s, 1H), 6.85 (ddd, *J =* 16.3, 9.9, 5.6 Hz, 1H), 6.76 (d, *J =* 2.2 Hz, 1H), 6.74 (s, 1H), 6.14 (dd, *J =* 16.4, 1.9 Hz, 1H), 6.01 (dd, *J* = 9.9, 5.8 Hz, 1H), 5.38 - 5.28 (m, 1H), 4.50 - 4.39 (m, 1H), 4.08 - 3.94 (m, 2H), 3.90 - 3.82 (m, 1H), 3.80 - 3.68 (m, 2H), 3.65 (dd, *J* = 11.4, 2.8 Hz, 1H), 3.56 - 3.45 (m, 1H), 3.22 - 3.07 (m, 3H), 1.55 (d, *J =* 7.1 Hz, 3H), 1.22 - 1.17 (m, 3H).

### Example 51: Preparation of (R)-4-(2-(1H-pyrazol-3-yl)-6,7,8,9-tetrahydro-1,3,7,9a-tetraazabenzo[cd]azulen-4-yl)-3-methylmorpholine (Compound 51)

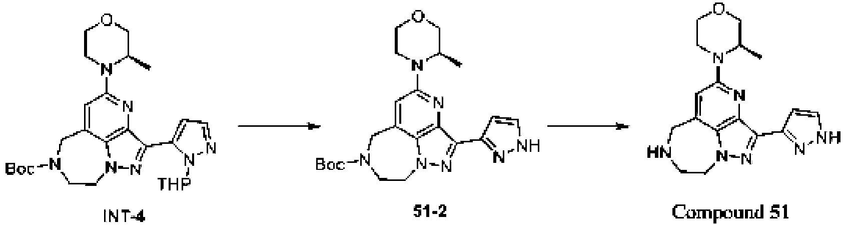

Step 1: Intermediate **INT-4** (48 mg, 0.092 mmol) was dissolved in tetrahydrofuran (1 mL), and dilute hydrochloric acid (1 mL, 1 M) was added. The reaction mixture was stirred at room temperature for half an hour, spin-dried to remove the solvent to yield Intermediate **51-2** (32.0 mg, 79.1%). LCMS (ESI) [M+H]⁺: 440.2.

Step 2: Intermediate **51-2** (32 mg, 0.073 mmol) was dissolved in dichloromethane (2 mL), and trifluoroacetic acid (83.0 mg, 0.728 mmol) was added. The reaction mixture was stirred at room temperature for half an hour, and spin-dried to remove the solvent to obtain a crude product, which was purified by reverse-phase high performance liquid chromatography to yield (*R*)-4-(2-(1*H*-pyrazol-3-yl)-6,7,8,9-tetrahydro-1,3,7,9a-tetraazabenzo[*cd*]azulen-4-yl)-3-methylm orpholine (Compound **51**) (13 mg, 52.6%). LCMS (ESI) [M+H]⁺: 340.2. ¹H NMR (400 MHz, DMSO-d6) δ 13.05 (d, *J =* 117.2 Hz, 1H), 7.60 (s, 1H), 7.04 (s, 1H), 6.89 (s, 1H), 4.44 - 4.34 (m, 3H), 4.18 (s, 2H), 4.02 - 3.94 (m, 2H), 3.76 (d, *J =* 11.2 Hz, 1H), 3.71 - 3.66 (m, 1H), 3.56 - 3.49 (m, 1H), 3.32 - 3.24 (m, 3H), 3.17 - 3.09 (m, 1H), 1.14 (d, *J =* 6.6 Hz, 3H).

### Example 52: Preparation of (R)-4-(7-(3,3-difluorocyclobutyl)-2-(1H-pyrazol-3-yl)-6,7,8,9-tetrahydro-1,3,7,9a-tetraazabenzo[cd]azulen-4-yl)-3-methylmorpholine (Compound 52)

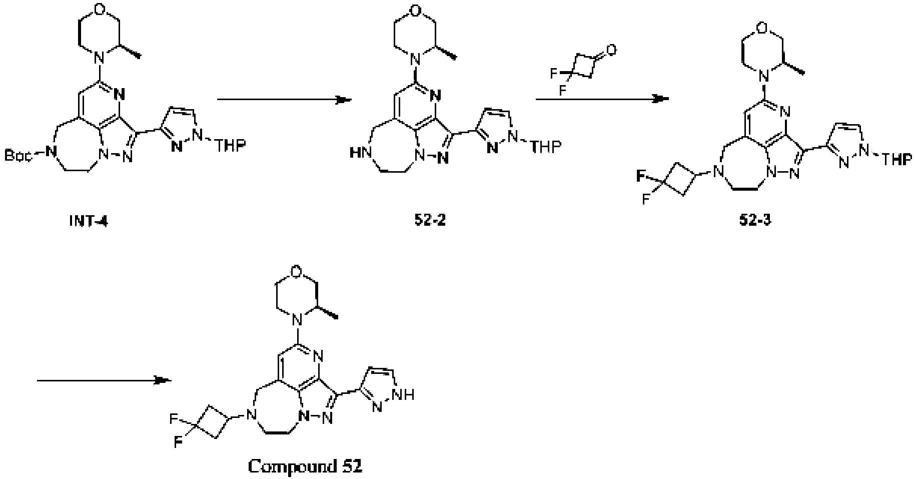

Step 1: Intermediate **INT-4** (200 mg, 0.382 mmol) was dissolved in dichloromethane. Hexamethyldisilazane (185 mg, 1.146 mmol) and trimethylsilyl trifluoromethanesulfonate (127.4 mg, 0.573 mmol) were added in sequence, and the reaction mixture was stirred at 0°C for 0.5 h. The reaction mixture was quenched with water at 25°C. The resulting mixture was separated, and the aqueous phase was extracted with ethyl acetate (10 mL × 3). The combined organic phases were washed sequentially with water (10 mL) and saturated brine (10 mL). The organic phase was dried over anhydrous sodium sulfate, filtered, and concentrated to obtain a crude product, which was separated and purified by silica gel column chromatography to yield Intermediate **52-2** (115 mg, 71.1%). LCMS (ESI) [M+H]⁺: 424.2.

Step 2: Intermediate **52-2** (100 mg, 0.236 mmol) was dissolved in isopropyl acetate (10 mL), and 3,3-difluorocyclobutyl-1-one (50.1 mg, 0.472 mmol), trifluoroacetic acid (26.9 mg, 0.236 mmol), and sodium triacetoxyborohydride (60.0 mg, 0.283 mmol) were added in sequence. The reaction mixture was stirred at room temperature for 1 hour, and spin-dried to remove the solvent to obtain a crude product. The crude product was purified by column chromatography to yield Intermediate **52-3** (50 mg, 41.2%). LCMS (ESI) [M+H]⁺: 514.0.

Step 3: Intermediate **52-3** (45 mg, 0.088 mmol) was dissolved in dichloromethane (4.5 mL). Trifluoroacetic acid (1.125 mL) was added. The reaction mixture was stirred at room temperature for half an hour, then spin-dried to remove solvent to obtain a crude product. The crude product was purified by preparative reverse-phase high-performance liquid chromatography to yield (*R*)-4-(7-(3,3-difluorocyclobutyl)-2-(1*H*-pyrazol-3-yl)-6,7,8,9-tetrahydro-1,3,7,9a-tetraazabenzo[ *cd*]azulen-4-yl)-3-methylmorpholine (Compound **52**) (14 mg, 37.2%). LCMS (ESI) [M+H]⁺ : 429.9. ¹H NMR (400 MHz, CD₃OD) δ 7.73 (d, *J =* 2.0 Hz, 1H), 7.04 (d, *J =* 2.0 Hz, 1H), 6.99 (s, 1H), 4.51 - 4.45 (m, 2H), 4.44 - 4.36 (m, 1H), 4.14 (s, 2H), 4.09 - 4.02 (m, 1H), 4.00 - 3.92 (m, 1H), 3.89 - 3.79 (m, 2H), 3.72 - 3.63 (m, 1H), 3.44 - 3.37 (m, 1H), 3.37 - 3.32 (m, 3H), 2.89 - 2.77 (m, 2H), 2.65 - 2.50 (m, 2H), 1.30 (d, *J =* 6.8 Hz, 3H).

### Example 53: Preparation of (R)-(3,3-difluorocyclobutyl)(4-(3-methylmorpholino)-2-(1H-pyrazol-3-yl)-8,9-dihydro-1,3,7,9a-tetraazabenzo[cd]azulene-7(6H)-yl)methanone (Compound 53)

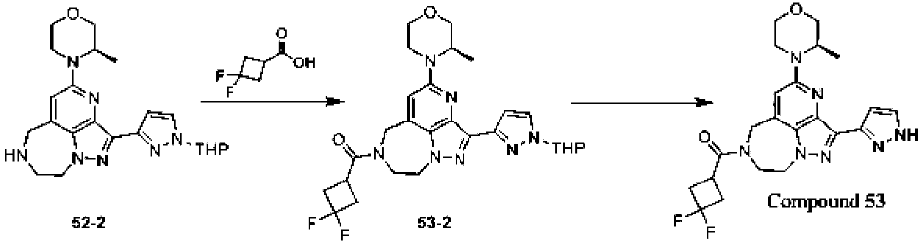

Step 1: Intermediate **52-2** (98.8 mg, 0.260 mmol) was dissolved in *N,N*-dimethylformamide (10 mL), and 3,3-difluorocyclobutanecarboxylic acid (50.0 mg, 0.118 mmol), *O*-(7-azabenzotriazol-1-yl)-*N,N,N',N'*-tetramethyluronium hexafluorophosphate (98.8 mg, 0.260 mmol), and diisopropylethylamine (45.8 mg, 0.354 mmol) were added in sequence. The mixture was stirred at room temperature for 1 hour. The reaction mixture was quenched with water at 25°C. The resulting mixture was separated, and the aqueous phase was extracted with ethyl acetate (10 mL × 3). The organic phase was washed sequentially with water (10 mL) and saturated brine (10 mL). The organic phase was dried over anhydrous sodium sulfate, filtered, and concentrated to obtain a crude product, which was purified by silica gel column chromatography to yield Intermediate **53-2** (35.0 mg, 54.7%). LCMS (ESI) [M+H]⁺: 542.2.

Step 2: Intermediate **53-2** (35.0 mg, 0.065 mmol) was dissolved in methanol (1.5 mL), and dilute hydrochloric acid (3.0 mL, 1 M) was added. The mixture was stirred at 30°C overnight, and spin-dried to remove the solvent to obtain a crude product, which was then purified by preparative reverse-phase high performance liquid chromatography to give (*R*)-(3,3-difluorocyclobutyl)(4-(3-methylmorpholino)-2-(1*H*-pyrazol-3-yl)-8,9-dihydro-1,3,7,9a-t etraazabenzo[*cd*]azulene-7(6*H*)-yl)methanone (Compound **53**) (13.8 mg, 46.7%). LCMS (ESI) [M+H]⁺ : 458.2. ¹H NMR (400 MHz, DMSO-d6) δ 13.11 (d, *J =* 119.5 Hz, 1H), 7.70 (d, *J =* 75.7 Hz, 1H), 7.27 - 6.91 (m, 2H), 5.07 - 4.89 (m, 2H), 4.52 - 4.41 (m, 3H), 4.22 - 4.10 (m, 2H), 4.08 - 3.95 (m, 2H), 3.86 - 3.66 (m, 2H), 3.61 - 3.50 (m, 1H), 3.36 - 3.27 (m, 1H), 3.24 - 3.10 (m, 1H), 2.92 - 2.62 (m, 4H), 1.23 - 1.13 (m, 3H).

### Example 54: Preparation of (R)-cyclopropyl(4-(3-methylmorpholino)-2-(1H-pyrazol-3-yl)-8,9-dihydro-1,3,7,9a-tetraazabenzo[cd]azulene-7(6H)-yl)methanone (Compound 54)

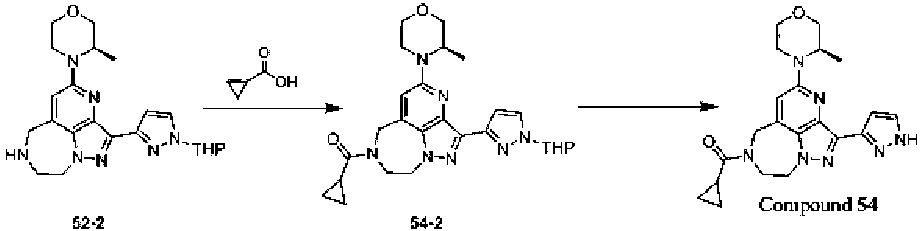

Similar to the preparation method of Example 53, the title Compound **54** was prepared using cyclopropanecarboxylic acid instead of 3,3-difluorocyclobutanecarboxylic acid. (*R*)-Cyclopropyl(4-(3-methylmorpholino)-2-(1*H*-pyrazol-3-yl)-8,9-dihydro-1,3,7,9a-tetraazabenz o[*cd*]azulene-7(6*H*)-yl)methanone (Compound **54**) (5.0 mg, 20.1%) was obtained. LCMS (ESI) [M+H]⁺: 408.0. ¹H NMR (400 MHz, CDCl₃) δ 7.63 (s, 1H), 6.84 (s, 1H), 6.65 (d, *J* = 12.3 Hz, 1H), 5.04 - 4.89 (m, 2H), 4.66 - 4.48 (m, 2H), 4.28 (s, 2H), 4.13 - 3.98 (m, 2H), 3.90 - 3.73 (m, 3H), 3.64 - 3.55 (m, 1H), 3.34 - 3.25 (m, 1H), 1.77 - 1.67 (m, 1H), 1.25 - 1.20 (m, 3H), 1.01 - 0.92 (m, 2H), 0.83 - 0.73 (m, 2H).

### Example 55: Preparation of (R)-3-methyl-4-(7-(methylsulfonyl)-2-(1H-pyrazol-3-yl)-6,7,8,9-tetrahydro-1,3,7,9a-tetraazabenzo[cd]azulen-4-yl)morpholine (Compound 55)

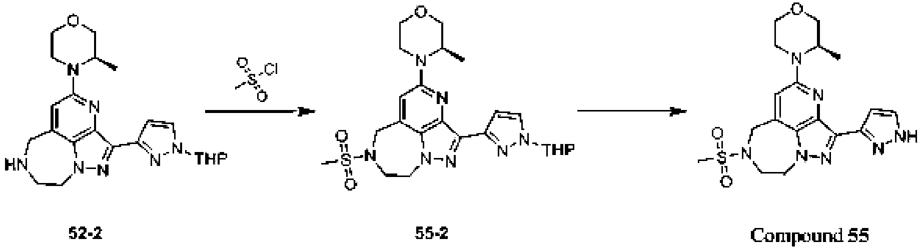

Similar to the preparation method of Example 22, the title Compound **55** was prepared using Intermediate **52-2** instead of Intermediate **5-1.** (*R*)-3-Methyl-4-(7-(methylsulfonyl)-2-(1*H*-pyrazol-3-yl)-6,7,8,9-tetrahydro-1,3,7,9a-tetraazabenzo[*cd*]azulen-4-yl)morpholine (Compound **55**) (22.5 mg, 60.1%) was obtained. LCMS (ESI) [M+H]⁺: 418.3. ¹H NMR (400 MHz, DMSO-d6) δ 13.25 - 12.95 (m, 1H), 7.79 - 7.62 (m, 1H), 7.06 (s, 2H), 4.82 (s, 2H), 4.52 (s, 2H), 4.43 (d, *J* = 5.4 Hz, 1H), 4.10 - 3.93 (m, 4H), 3.78 (d, *J =* 11.2 Hz, 1H), 3.73 - 3.65 (m, 1H), 3.58 - 3.50 (m, 1H), 3.21 - 3.11 (m, 1H), 3.05 (s, 3H), 1.17 (d, *J =* 6.6 Hz, 3H).

### Example 56: Preparation of 6-methyl-4-((R)-3-methylmorpholinyl)-2-(1H-pyrazol-3-yl)-6,7-dihydro-1,3,7,9a-tetraazabenzo[cd]azulen-8(9H)-one (Compound 56)

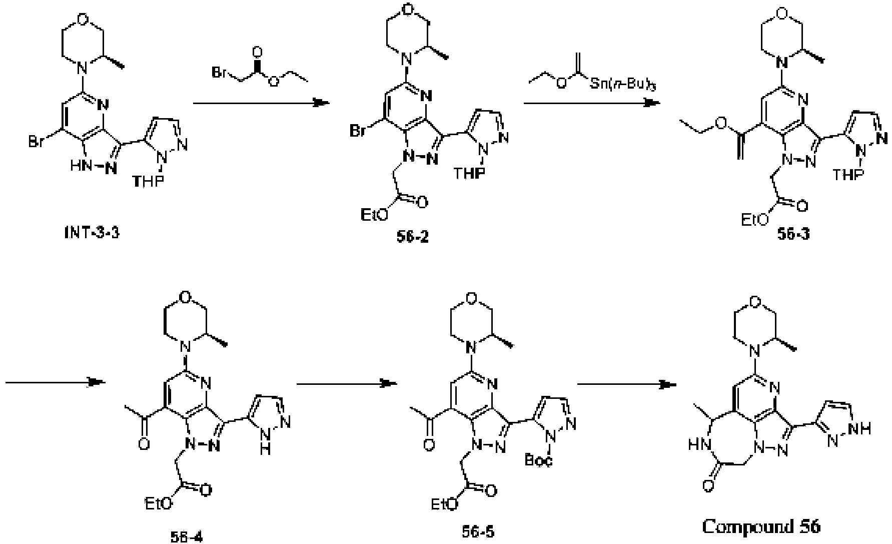

Step 1: Under a nitrogen atmosphere, **INT-3-3** (2.60 g, 5.81 mmol) was dissolved in *N,N*-dimethylformamide (20 mL), and potassium carbonate (2.41 g, 17.44 mmol) and ethyl bromoacetate (0.64 g, 2.80 mmol) were added in sequence. The reaction mixture was stirred at 50°C for 12 hours. The mixture was cooled to room temperature. The reaction mixture was concentrated under reduced pressure to remove most of the *N,N*-dimethylformamide, then diluted with dichloromethane (20 mL) and filtered. The filtrate was concentrated under reduced pressure to obtain a crude product. The crude product was purified by silica gel column chromatography to yield Intermediate **56-2** (2.45 g, 79.0%). LCMS (ESI) [M+H]⁺: 534.8.

Step 2: Under a nitrogen atmosphere, Intermediate **56-2** (2.45 g, 4.59 mmol) was dissolved in 1,4-dioxane (20 mL), and tributyl(1-ethoxyethylene)tin (3.3 g, 9.18 mmol) and tetrakis(triphenylphosphine)palladium (0.50 g, 0.459 mmol) were added in sequence. The reaction mixture was stirred at 110°C for 12 hours. After the reaction was completed, the mixture was cooled to room temperature, and 10% aqueous potassium fluoride (20 mL) was added. The mixture was stirred for 30 minutes, filtered, and the filter cake was washed with dichloromethane. The filtrate was extracted with dichloromethane (20 mL x 3). The combined organic phases were washed sequentially with water and saturated brine, dried over anhydrous sodium sulfate, and filtered. The filtrate was concentrated under reduced pressure to yield crude Intermediate **56-3** (2.4 g, 99.6%). LCMS (ESI) [M+H]⁺: 525.0.

Step 3: Intermediate **56-3** (2.40 g, 4.58 mmol) was dissolved in tetrahydrofuran (5 mL), and then dilute hydrochloric acid (1 mL, 2 M) was added. The reaction mixture was stirred at room temperature for 3 hours. After the reaction was completed, saturated sodium bicarbonate solution (20 mL) was added to quench the reaction, and the aqueous phase was extracted with dichloromethane (30 mL x 3). The combined organic phases were washed sequentially with water and saturated brine, dried over anhydrous sodium sulfate, and filtered. The filtrate was concentrated under reduced pressure to yield a crude product. The crude product was separated and purified by silica gel column chromatography to yield Intermediate **56-4** (1.05 g, 55.7%). LCMS (ESI) [M+H]⁺: 412.9.

Step 4: Intermediate **56-4** (1.30 g, 3.15 mmol) was dissolved in acetonitrile (20 mL), and triethylamine (1.30 mL, 9.45 mmol), di-tert-butyl dicarbonate (1.45 mL, 6.30 mmol), and 4-dimethylaminopyridine (269.6 mg, 2.21 mmol) were added in sequence. The reaction mixture was stirred at room temperature for 1 hour. After the reaction was completed, the reaction mixture was concentrated to obtain a crude product. The crude product was separated and purified by silica gel column chromatography to yield Intermediate **56-5** (1.00 g, 61.9%). LCMS (ESI) [M+H]⁺: 512.9.

Step 5: Under a nitrogen atmosphere, Intermediate **56-5** (0.87 mg, 1.70 mmol) was dissolved in ethanol (15 mL), and then ammonium acetate (1.31 g, 17.0 mmol) was added. The reaction mixture was stirred at 80°C for 2 hours, after which sodium cyanoborohydride (532.9 mg, 8.49 mmol) was added in batches to the reaction mixture, and the reaction mixture was continuously stirred at 80°C for 22 hours. After the reaction was completed, the reaction mixture was cooled to room temperature, saturated sodium bicarbonate solution was added to quench the reaction, and the aqueous phase was extracted with dichloromethane (30 mL x 3). The combined organic phases were washed sequentially with water and saturated brine, dried over anhydrous sodium sulfate, and filtered. The filtrate was concentrated under reduced pressure to yield a crude product. The crude product was separated and purified by silica gel column chromatography to give 6-methyl-4-((*R*)-3-methylmorpholinyl)-2-(1*H*-pyrazol-3-yl)-6,7-dihydro-1,3,7,9a-tetraazabenzo[*cd*]azulen-8(9*H*)-one (Compound **56**) (410.0 mg, 51.7%). LCMS (ESI) [M+H]⁺ : 368.4. ¹H NMR (400 MHz, DMSO-d6) δ 13.19 - 12.87 (m, 1H), 8.40 (d, *J =* 5.2 Hz, 1H), 7.66 (s, 1H), 7.00 (s, 1H), 6.82 (s, 1H), 5.65 (d, *J =* 15.2 Hz, 1H), 5.34 - 5.19 (m, 1H), 4.86 (d, *J* = 14.8 Hz, 1H), 4.88 - 4.79 (m, 1H), 4.06 - 3.91 (m, 2H), 3.76 (d, *J* = 11.4 Hz, 1H), 3.68 (dd, *J =* 11.4, 2.4 Hz, 1H), 3.53 (td, *J* = 11.4, 2.4 Hz, 1H), 3.16 (td, *J =* 12.8, 3.6 Hz, 1H), 1.63 (d, *J =* 6.8 Hz, 3H), 1.16 (d, *J =* 6.8 Hz, 3H).

### Example 57: Preparation of (3R)-3-methyl-4-(6-methyl-7-(oxetan-3-yl)-2-(1H-pyrazol-3-yl)-6,7,8,9-tetrahydro-1,3,7,9a-tetraazabenzo[cd]azulen-4-yl)morpholine (Compound 57)

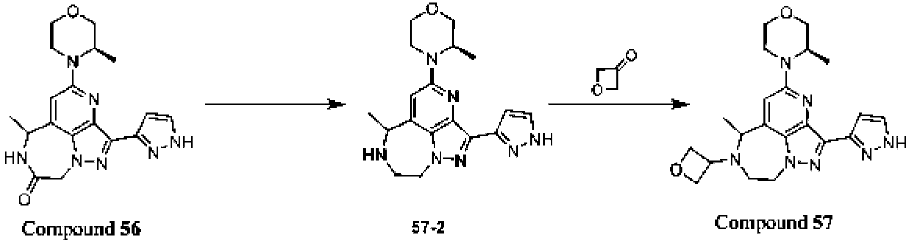

Step 1: Under a nitrogen atmosphere, Compound **56** (440.0 mg, 1.20 mmol) was dissolved in tetrahydrofuran (2 mL), and 1 M solution of borane in tetrahydrofuran (5 mL) was added. The reaction mixture was stirred at 30°C for 1 hour. Methanol (10 mL) was added to the reaction mixture, and the mixture was refluxed and stirred for 2 hours. After the reaction was completed, the reaction mixture was concentrated under reduced pressure to obtain a crude product, which was then purified by silica gel column chromatography and reverse-phase high-performance liquid chromatography to yield Intermediate **57-2** (70.0 mg, 16.5%). LCMS (ESI) [M+H]⁺: 354.0.

Step 2: Under a nitrogen atmosphere, Intermediate **57-2** (40.0 mg, 0.11 mmol) was dissolved in methanol (5 mL), and 3-oxetanone (24.5 mg, 0.34 mmol) was added. The reaction mixture was stirred at 30°C for 1 hour. Then, under a nitrogen atmosphere, sodium triacetoxyborohydride (238.7 mg, 1.13 mmol) was added in batches to the reaction mixture, and continuously stirred continued at 30°C for 2 hours. After the reaction was completed, saturated sodium chloride solution was added to quench the reaction, and the aqueous phase was extracted with dichloromethane (20 mL x 3). The combined organic phases were washed sequentially with water and saturated brine, dried over anhydrous sodium sulfate, and filtered. The filtrate was concentrated under reduced pressure to obtain a crude product. The crude product was separated and purified by preparative reverse-phase high performance liquid chromatography to give (3*R*)-3-methyl-4-(6-methyl-7-(oxetan-3-yl)-2-(1*H*-pyrazol-3-yl)-6,7,8,9-tetrahydro-1,3,7,9a-tetraazabenzo[*cd*]az ulen-4-yl)morpholine (Compound **57**) (10.0 mg, 21.6%). LCMS (ESI) [M+H]⁺: 410.4. ¹H NMR (400 MHz, DMSO-d6) δ 13.16 - 12.94 (m, 1H), 7.62 (s, 1H), 7.04 (s, 1H), 6.83 (s, 1H), 4.49 - 4.36 (m, 2H), 4.37 - 4.27 (m, 1H), 4.27 - 4.17 (m, 1H), 4.03 - 3.90 (m, 2H), 3.81 - 3.63 (m, 2H), 3.60 - 3.42 (m, 2H), 3.19 - 3.01 (m, 2H), 1.53 (d, *J =* 6.7 Hz, 3H), 1.14 (d, *J =* 6.6 Hz, 3H).

### Example 58: Preparation of (R)-3-methyl-4-(6-methyl-2-(1H-pyrazol-3-yl)-8,9-dihydro-1,3,7,9a-tetraazabenzo[cd]azulen-4-yl)morpholine (Compound 58)

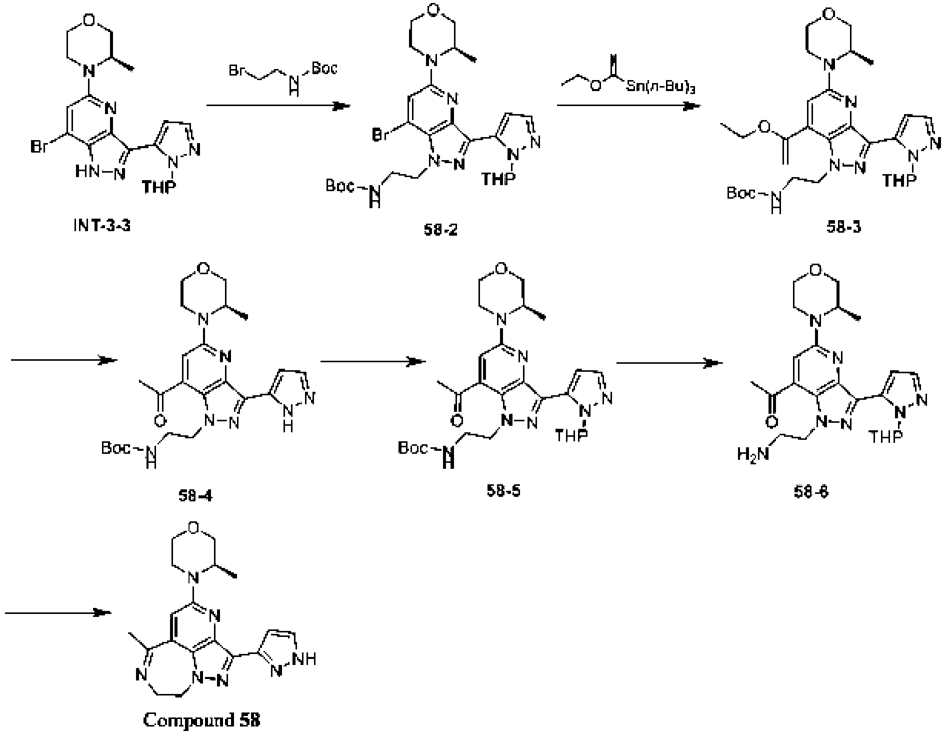

Step 1: Similarly to the preparation method of Example 56, Intermediate **58-4** was prepared using *N*-Boc-bromoethylamine instead of ethyl bromoacetate. Tert-butyl (*R*)-(2-(7-acetyl-5-(3-methylmorpholinyl)-3-(1*H*-pyrazol-5-yl)-1*H*-pyrazolo[4,3-b]pyridin-1-yl)ethyl)carbamate (Intermediate **58-4**) (180.0 mg, 89.2%) was obtained. LCMS (ESI) [M+H]⁺: 470.4.

Step 2: Under a nitrogen atmosphere, Intermediate **58-4** (180.0 mg, 0.38 mmol) was dissolved in dichloromethane (4 mL), and p-toluenesulfonic acid (6.60 mg, 0.038 mmol) and 3,4-dihydro-2*H*-pyran (20.0 mg, 0.24 mmol) were added in sequence. The reaction mixture was stirred at 40°C for 18 hours. The reaction mixture was concentrated to obtain a crude product. The crude product was separated and purified by silica gel column chromatography to yield Intermediate **58-5** (150.0 mg, 70.7%). LCMS (ESI) [M+H]⁺: 554.5.

Step 3: Under a nitrogen atmosphere, Intermediate **58-5** (150.0 mg, 0.27 mmol) was dissolved in dichloromethane (2 mL), and 2,6-dimethylpyridine (203.3 mg, 1.90 mmol) and trimethylsilyl trifluoromethanesulfonate (150.6 mg, 0.68 mmol) were added. The reaction mixture was stirred at 40°C for 3 hours. The reaction was quenched by adding saturated sodium bicarbonate solution, and the aqueous phase was extracted with dichloromethane (30 mL × 3). The combined organic phases were washed sequentially with water and saturated brine, dried over anhydrous sodium sulfate, and filtered. The filtrate was concentrated under reduced pressure to obtain a crude product. The crude product was separated and purified by silica gel column chromatography to yield Intermediate **58-6** (60.0 mg, 48.8%). LCMS (ESI) [M+H]⁺: 454.2.

Step 4: Intermediate **58-6** (60.0 mg, 0.13 mmol) was dissolved in tetrahydrofuran (3 mL), and dilute hydrochloric acid (1 mL, 2 M) was added. The reaction mixture was stirred at 40°C for 1 hour. After the reaction was completed, the reaction mixture was cooled to room temperature, and saturated sodium bicarbonate solution was added to quench the reaction. The aqueous phase was extracted with dichloromethane (30 mL x 3). The combined organic phases were washed sequentially with water and saturated brine, dried over anhydrous sodium sulfate, and filtered. The filtrate was concentrated under reduced pressure to obtain a crude product. The crude product was purified by preparative reverse-phase high performance liquid chromatography to yield (*R*)-3-methyl-4-(6-methyl-2-(1*H*-pyrazol-3-yl)-8,9-dihydro-1,3,7,9a-tetraazabenzo[*cd*] azulen-4-yl)morpholine (Compound **58**) (10.0 mg, 21.5%). LCMS (ESI) [M+H]⁺: 352.3. ¹H NMR (400 MHz, DMSO-d6) δ 13.14 (s, 1H), 7.67 (s, 1H), 7.24 (s, 1H), 7.02 (d, *J =* 1.2 Hz, 1H), 4.59 - 4.47 (m, 3H), 4.16 - 3.94 (m, 4H), 3.78 (d, *J =* 11.2 Hz, 1H), 3.71 (dd, *J =* 11.2, 2.4 Hz, 1H), 3.56 (td, *J =* 11.6, 2.4 Hz, 1H), 3.20 (td, *J =* 12.6, 2.4 Hz, 1H), 2.54 (s, 3H), 1.18 (d, *J =* 6.6 Hz, 3H).

### Examples 59A and 59B: Preparation of (3R)-4-(6,7-dimethyl-2-(1H-pyrazol-3-yl)-6,7,8,9-tetrahydro-1,3,7,9a-tetraazabenzo[cd]azulen-4-yl)-3-methylmorpholine (Compound 59A, Isomer 1) and preparation of (3R)-4-(6,7-dimethyl-2-(1H-pyrazol-3-yl)-6,7,8,9-tetrahydro-1,3,7,9a-tetraazabenzo[cd]azulen-4-yl)-3-methylmorpholine (Compound 59B, Isomer 2)

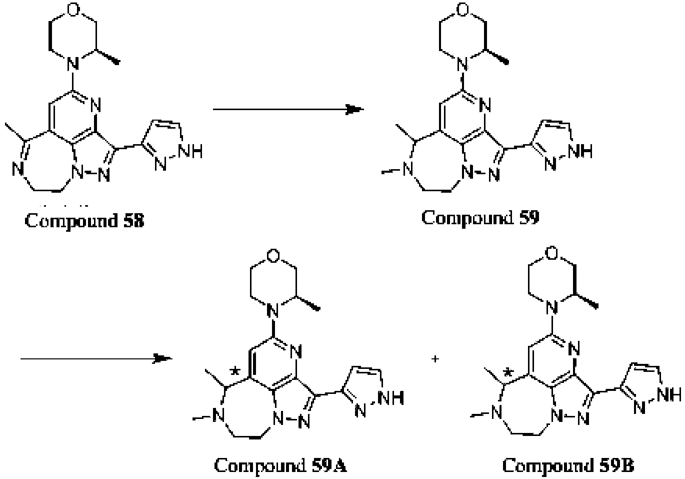

Step 1: Under nitrogen atmosphere, Compound **58** (150 mg, 0.42 mmol) was dissolved in methanol (3 mL), and paraformaldehyde (382.0 mg, 4.24 mmol) was added. The reaction mixture was stirred at 50°C for 1 hour. Sodium cyanoborohydride (131.6 mg, 2.122 mmol) was added in batches, and the reaction solution was continuously stirred at 50°C for 2 hours. The mixture was cooled to room temperature, ice water (10 mL) was added to quench the reaction, and the aqueous phase was extracted with ethyl acetate (20 mL × 3). The combined organic phases were washed sequentially with water and saturated brine, dried over anhydrous sodium sulfate, and filtered. The filtrate was concentrated under reduced pressure to obtain a crude product. The crude product was separated and purified by silica gel chromatography to yield a diastereomeric mixture of (3*R*)-4-(6,7-dimethyl-2-(1*H*-pyrazol-3-yl)-6,7,8,9-tetrahydro-1,3,7,9a-tetraazabenzo [*cd*]azulen-4-yl)-3-methylmorpholine (Compound **59**) (90.0 mg, 57.7%). LCMS (ESI) [M+H]⁺: 368.4.

Step 2: The enantiomeric mixture of (3*R*)-4-(6,7-dimethyl-2-(1*H*-pyrazol-3-yl)-6,7,8,9-tetrahydro-1,3,7,9a-tetraazabenzo[*cd*]azulen-4-yl)-3-methylmorpholine (Compound **59**) (90.0 mg) was further purified by chiral SFC. SFC analysis: Daicel CHIRALPAK AD column (150 × 4.6 mm, 5 µm), mobile phase: 35% ethanol in n-hexane, temperature: 35°C, flow rate: 1.0 mL/min, detection wavelength: 254 nm.

The first peak was (3*R*)-4-(6,7-dimethyl-2-(1*H*-pyrazol-3-yl)-6,7,8,9-tetrahydro-1,3,7,9a-tetraazabenzo[*cd*]azulen-4-yl)-3-methylmorpholine (Compound **59A,** Isomer 1) (31.9 mg). LCMS (ESI) [M+H]⁺: 368.4. HPLC-SFC: Rt = 3.538 min. ¹H NMR (400 MHz, DMSO-d6) δ 13.20 - 12.90 (m, 1H), 7.77 - 7.57 (m, 1H), 7.03 (s, 1H), 6.87 (s, 1H), 4.42 (s, 3H), 4.25 (q, *J =* 6.8 Hz, 1H), 3.98 (dd, *J =* 11.2, 2.8 Hz, 2H), 3.78 - 3.65 (m, 3H), 3.52 (td, *J =* 11.6, 2.4 Hz, 1H), 3.20 - 3.08 (m, 2H), 2.42 (s, 3H), 1.45 (d, *J =* 7.2 Hz, 3H), 1.15 (d, *J =* 6.8 Hz, 3H).

The second peak was (3*R*)-4-(6,7-dimethyl-2-(1*H*-pyrazol-3-yl)-6,7,8,9-tetrahydro-1,3,7,9a-tetraazabenzo[*cd*]azulen-4-yl)-3-methylmorpholine (Compound **59B,** Isomer 2) (27.2 mg). LCMS (ESI) [M+H]⁺: 368.4. HPLC-SFC: Rt = 4.239 min. ¹H NMR (400 MHz, DMSO-d6) δ 13.20 - 12.90 (m, 1H), 7.75 - 7.58 (m, 1H), 7.04 (s, 1H), 6.86 (s, 1H), 4.42 (s, 3H), 4.25 (q, *J =* 6.8 Hz, 1H), 4.06 - 3.89 (m, 2H), 3.81 - 3.61 (m, 3H), 3.53 (td, *J =* 11.8, 2.8 Hz, 1H), 3.16 - 3.09 (m, 2H), 2.42 (s, 3H), 1.45 (d, *J =* 7.2 Hz, 3H), 1.15 (d, *J =* 6.8 Hz, 3H).

### Example 60: Preparation of (3R)-3-methyl-4-(6-methyl-7-(methylsulfonyl)-2-(1H-pyrazol-3-yl)-6,7,8,9-tetrahydro-1,3,7,9a-tetraazabenzo[cd]azulen-4-yl)morpholine (Compound 60)

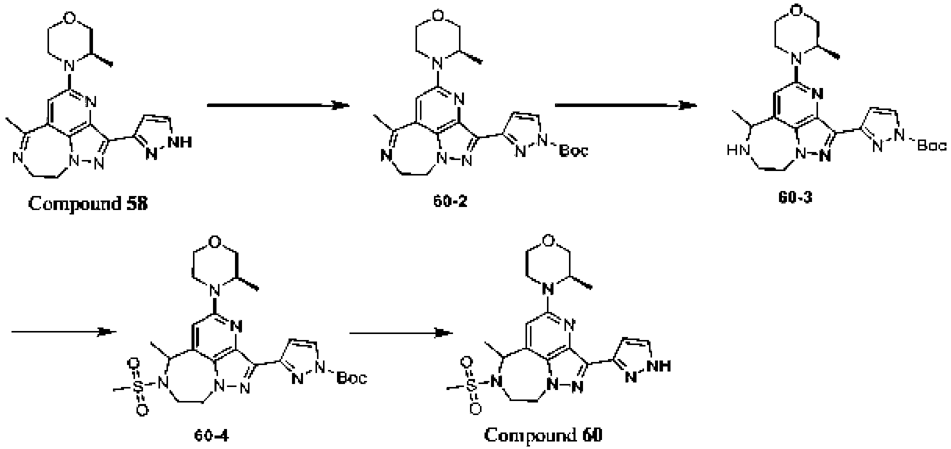

Step 1: Under a nitrogen atmosphere, Compound **58** (140.0 mg, 0.40 mmol) was dissolved in acetonitrile (5 mL), and triethylamine (0.17 mL, 1.20 mmol), 4-dimethylaminopyridine (24.3 mg, 0.20 mmol), and di-tert-butyl dicarbonate (0.18 mL, 0.80 mmol) were added in sequence. The reaction mixture was stirred at 50°C for 5 hours. The mixture was cooled to room temperature. The reaction mixture was concentrated under reduced pressure to remove acetonitrile, dissolved in dichloromethane (20 mL), and filtered. The filtrate was concentrated to obtain a crude product. The crude product was purified by silica gel chromatography to yield Intermediate **60-2** (140.0 mg, 77.8%). LCMS (ESI) [M+H]⁺: 452.4.

Step 2: Under a nitrogen atmosphere, Intermediate **60-2** (140.0 mg, 0.31 mmol) was dissolved in methanol (2 mL), and sodium cyanoborohydride (200.0 mg, 3.23 mmol) was added. The reaction mixture was stirred at 50°C for 1 hour. The mixture was cooled to room temperature, quenched by the addition of saturated sodium chloride solution, and the aqueous phase was extracted with dichloromethane (10 mL × 3). The combined organic phases were washed sequentially with water and saturated brine, dried over anhydrous sodium sulfate, and filtered. The filtrate was concentrated under reduced pressure to obtain a crude product. The crude product was separated and purified by silica gel chromatography to yield Intermediate **60-3** (90.0 mg, 64.0%). LCMS (ESI) [M+H]⁺: 454.4.

Step 3: Under a nitrogen atmosphere, Intermediate **60-3** (135.0 mg, 0.30 mmol) was dissolved in tetrahydrofuran (3 mL), and lithium diisopropylamide (0.45 mL, 2 M) was slowly added at -78°C. The reaction mixture was stirred at -78°C for 10 minutes, then methanesulfonyl chloride (51.1 mg, 0.45 mmol) was added, and the reaction mixture was continuously stirred at low temperature for 3 hours. After the reaction was completed, the reaction was quenched by the addition of saturated ammonium chloride solution, and the aqueous phase was extracted with dichloromethane (30 mL x 3). The combined organic phases were washed sequentially with water and saturated brine, dried over anhydrous sodium sulfate, and filtered. The filtrate was concentrated under reduced pressure to yield a crude product. The crude product was separated and purified by silica gel chromatography to yield Intermediate **60-4** (180.0 mg, 89.2%). LCMS (ESI) [M+H]⁺: 532.4.

Step 4: Intermediate **60-4** (40.0 mg, 0.075 mmol) was dissolved in dichloromethane (2 mL), and hydrochloric acid in ethyl acetate (1 mL, 1 M) was added. The reaction mixture was stirred at room temperature for 1 hour. The reaction was quenched by the addition of saturated sodium bicarbonate solution, and the aqueous phase was extracted with dichloromethane (30 mL × 3). The combined organic phases were washed sequentially with water and saturated brine, dried over anhydrous sodium sulfate, and filtered. The filtrate was concentrated under reduced pressure to yield a crude product. The crude product was separated and purified by preparative reverse-phase high performance liquid chromatography to give (3*R*)-3-methyl-4-(6-methyl-7-(methylsulfonyl)-2-(1*H*-pyrazol-3-yl)-6,7,8,9-tetrahydro-1,3,7,9a-tetraazabenzo[*cd*]azulen-4-yl)morpholine (Compound **60**) (10.0 mg, 30.8%). LCMS (ESI) [M+H]⁺: 432.2. ¹H NMR (400 MHz, DMSO-d6) δ 8.36 (d, *J* = 2.4 Hz, 1H), 7.43 (d, *J =* 2.4 Hz, 1H), 6.86 (s, 1H), 4.49 - 4.40 (m, 2H), 4.39 - 4.31 (m, 1H), 4.28 - 4.18 (m, 1H), 4.03 - 3.91 (m, 2H), 3.76 (dd, *J =* 11.2, 2.8 Hz, 1H), 3.73 - 3.66 (m, 1H), 3.60 (s, 3H), 3.58 - 3.45 (m, 2H), 3.21 - 3.01 (m, 3H), 1.53 (d, *J =* 6.4 Hz, 3H), 1.15 (d, *J* = 6.4 Hz, 3H).

### Example 61: Preparation of (R)-4-(1-(1H-pyrazol-3-yl)-4,5-dihydro-3H-2,2a,5,8-tetraazaacenaphthen-7-yl)-3-methylmorpholine (Compound 61)

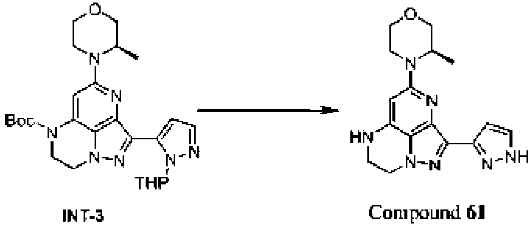

Intermediate **INT-3** (70 mg, 0.137 mmol) was dissolved in tetrahydrofuran (1 mL). Aqueous hydrochloric acid (1 mL, 1 M) was added, and the reaction mixture was stirred at 25°C for 2 hours. The mixture was concentrated to remove the solvent to obtain a crude product. The crude product was purified by silica gel column to give (*R*)-4-(1-(1*H*-pyrazol-3-yl)-4,5-dihydro-3*H*-2,2*a*,5,8-tetraazaacenaphthen-7-yl)-3-methylmorpholine (Compound **61**) (35 mg, 78.5%). LCMS (ESI) [M+H]⁺: 325.9. ¹H NMR (400 MHz, CD₃OD) δ 7.62 (s, 1H), 6.94 (s, 1H), 6.03 (s, 1H), 4.36 - 4.35 (m, 3H), 4.01 - 3.89 (m, 2H), 3.83 - 3.76 (m, 4H), 3.67 - 3.60 (m, 1H), 3.26 - 3.22 (m, 1H), 1.23 (d, *J* = 6.8 Hz, 3H).

### Example 62: Preparation of (R)-3-methyl-4-(5-methyl-1-(1H-pyrazol-3-yl)-4,5-dihydro-3H-2,2a,5,8-tetraazaacenaphthen-7-yl)morpholine (Compound 62)

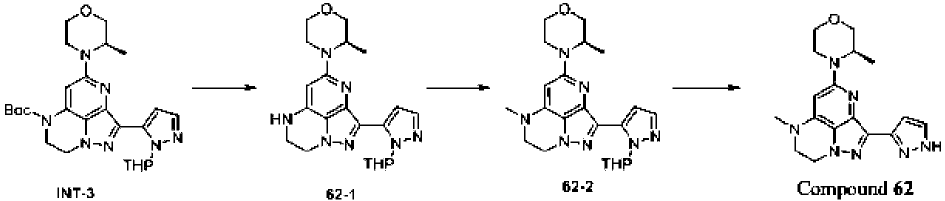

Step 1: Intermediate **INT-3** (80 mg, 0.16 mmol) was dissolved in tetrahydrofuran (2 mL), and lithium aluminum hydride (59.7 mg, 1.57 mmol) in an ice-water bath was added. The mixture was heated to reflux, and reacted for 15 minutes. The mixture was then cooled in an ice-water bath, and the reaction was quenched by adding ice water (1 mL). After spin-drying, the resulting product was separated on a preparative plate (dichloromethane/methanol = 10/1) to yield Intermediate **62-1** (40 mg, 62.2%). LCMS (ESI) [M+H]⁺: 410.0.

Step 2: Intermediate **62-1** (40.0 mg, 0.098 mmol) was dissolved in N,N-dimethylformamide (2 mL), and sodium hydride (15.7 mg, 0.39 mmol) in an ice-water bath was added. The system was stirred thoroughly, and then iodomethane (27.8 mg, 0.2 mmol) was added. The reaction mixture was heated to 50°C, stirred for 2 hours, and then ice water (1 mL) was added to quench the reaction. After spin-drying, the resulting product was separated by a preparative plate (dichloromethane/methanol = 10/1) to yield Intermediate **62-2** (20 mg, 48.2%). LCMS (ESI) [M+H]⁺: 424.2.

Step 3: Intermediate **62-2** (20.0 mg, 0.047 mmol) was dissolved in tetrahydrofuran (1 mL). Aqueous hydrochloric acid (0.24 mL, 4 M) was added. The reaction mixture was stirred at room temperature for half an hour, and then concentrated to yield a crude product. The crude product was purified by silica gel column chromatography (dichloromethane/methanol = 10/3) to give (*R*)-3-methyl-4-(5-methyl-1-(1*H*-pyrazol-3-yl)-4,5-dihydro-3*H*-2,2*a*,5,8-tetraazaacenaphthen-7-yl)morpholine (Compound **62**) (15 mg, 93.6%). LCMS (ESI) [M+H]⁺: 340.2. ¹H NMR (400 MHz, CD₃OD) δ 7.72 (s, 1H), 6.97 (s, 1H), 6.04 (s, 1H), 4.56 - 4.39 (m, 3H), 4.06 (d, *J =* 10.8 Hz, 1H), 3.98 - 3.81 (m, 5H), 3.70 (t, *J =* 11.7 Hz, 1H), 3.41 (d, *J =* 12.2 Hz, 1H), 3.17 (s, 3H), 1.31 (d, *J =* 6.5 Hz, 3H).

### Example 63: Preparation of (R)-4-(5-isopropyl-1-(1H-pyrazol-3-yl)-4,5-dihydro-3H-2,2a,5,8-tetraazaacenaphthen-7-yl)-3-methylmorpholine (Compound 63)

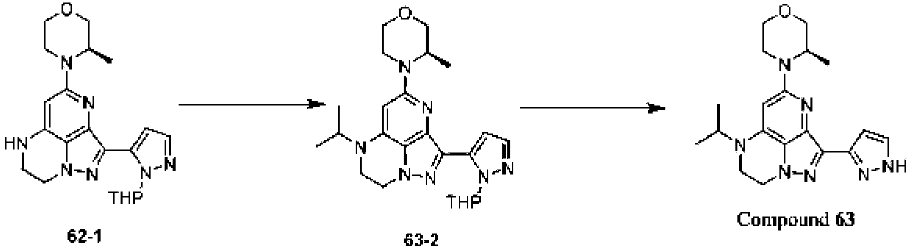

Similar to the preparation method of Example 62, the title Compound **63** was prepared using 2-bromopropane instead of iodomethane. (*R*)-4-(5-isopropyl-1-(1*H*-pyrazol-3-yl)-4,5-dihydro-3*H*-2,2*a*,5,8-tetraazaacenaphthen-7-yl)-3-methylmorpholine (Compound **63**) was obtained (15 mg, 73.7%). LCMS (ESI) [M+H]⁺: 368.2. ¹H NMR (400 MHz, CD₃OD) δ 7.70 (s, 1H), 6.96 (s, 1H), 6.09 (s, 1H), 4.43 (s, 3H), 4.39 - 4.24 (m, 2H), 4.10 - 3.99 (m, 1H), 3.94 - 3.78 (m, 5H), 3.70 (t, *J =* 10.7 Hz, 1H), 3.47 - 3.38 (m, 1H), 1.42 - 1.36 (m, 6H), 1.35 - 1.26 (m, 3H).

### Example 64: Preparation of (R)-3-methyl-4-(5-(methylsulfonyl)-1-((1H-pyrazol-3-yl)-4,5-dihydro-3H-2,2a,5,8-tetraazaacenaphthen-7-yl)morpholine (Compound 64)

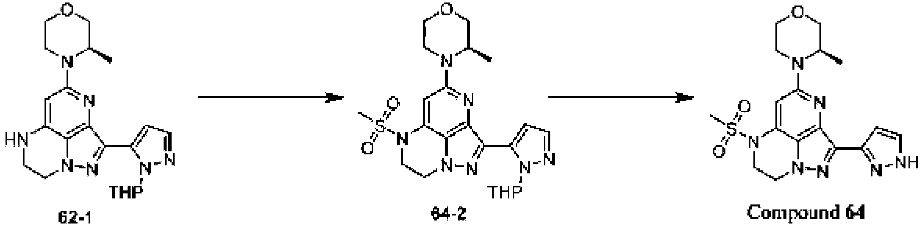

Step 1: Intermediate **62-1** (50 mg, 0.11 mmol) was dissolved in tetrahydrofuran (2 mL), and methanesulfonyl chloride (41.9 mg, 0.34 mmol) and triethylamine (61.7 mg, 0.61 mmol) were added in sequence. The reaction mixture was stirred at room temperature for 2 hours, and spin-dried to remove the solvent to obtain a crude product. The crude product was purified by silica gel column chromatography to yield Intermediate **64-2** (18 mg, 32%). LCMS (ESI) [M+H]⁺: 488.0.

Step 2: Intermediate **64-2** (18 mg, 0.037 mmol) was dissolved in tetrahydrofuran (1 mL), and added aqueous hydrochloric acid (1 mL, 4 M). The reaction mixture was stirred at room temperature for 2 hours, and spin-dried to remove the solvent to obtain a crude product. The crude product was purified by silica gel column chromatography to yield (*R*)-3-methyl-4-(5-(methylsulfonyl)-1-(1*H*-pyrazol-3-yl)-4,5-dihydro-3*H*-2,2*a*,5,8-tetraazaacena phthen-7-yl)morpholine (Compound **64**) (4.3 mg, 29.7%). LCMS (ESI) [M+H]⁺: 404.2. ¹H NMR (400 MHz, CDCl₃) δ 7.70 (d, *J* = 1.6 Hz, 1H), 6.95 (s, 1H), 6.89 (s, 1H), 4.54 - 4.50 (m, 2H), 4.38 - 4.33(m, 3H), 4.10 - 4.06 (m, 1H), 3.96 - 3.92 (m, 1H), 3.84 - 3.84 (m, 2H), 3.72 - 3.65 (m, 1H), 3.41 - 3.34 (m, 1H), 3.18 (s, 3H), 1.30 (d, *J =* 6.8 Hz, 3H).

### Example 65: Preparation of tert-butyl (R)-7-(3-methylmorpholino)-1-(1H-pyrazol-3-yl)-3,4-dihydro-5H-2,2a,5,8-tetraazaacenaphthene-5-carboxylate (Compound 65)

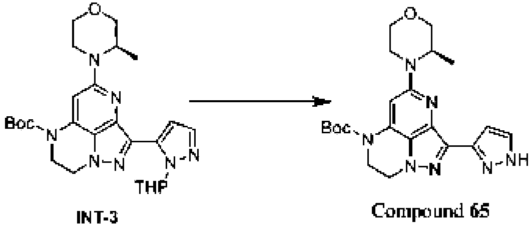

Intermediate **INT-3** (50.0 mg, 0.098 mmol) was dissolved in methanol (2 mL), and camphorsulfonic acid (22.8 mg, 0.098 mmol) was added. The reaction system was stirred at room temperature for 1 hour, and concentrated to obtain a crude product. The crude product was purified by silica gel column chromatography to obtain tert-butyl (*R*)-7-(3-methylmorpholino)-1-(1*H*-pyrazol-3-yl)-3,4-dihydro-5*H*-2,2*a*,5,8-tetraazaacenaphthene-5-carboxylate (Compound **65**) (10.6 mg, 25.4%). LCMS (ESI) [M+H]⁺: 426.1. ¹H NMR (400 MHz, DMSO-d6) δ 13.41 - 12.77 (m, 1H), 7.77 - 7.51 (m, 1H), 7.20 (s, 1H), 6.99 (s, 1H), 4.46 (s, 2H), 4.41 - 4.20 (m, 3H), 4.06 - 3.91 (m, 2H), 3.83 - 3.67 (m, 2H), 3.59 - 3.49 (m, 1H), 3.22 - 3.12 (m, 1H), 1.60 (s, 9H), 1.18 (d, *J =* 6.6 Hz, 3H).

### Example 66: Preparation of (R)-3-methyl-4-(5-(1-methyl-1H-pyrazol-5-yl)-1-(1H-pyrazol-3-yl)-4,5-dihydro-3H-2,2a,5,8-tetraazaacenaphthen-7-yl)morpholine (Compound 66)

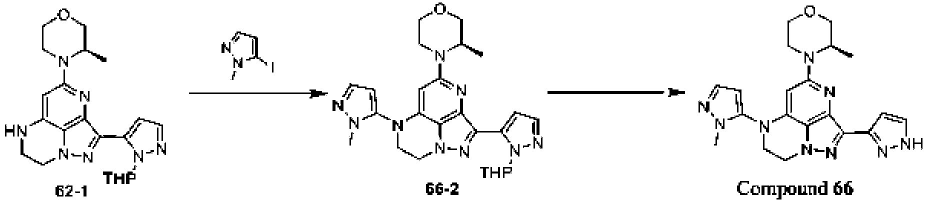

Step 1: Intermediate **62-1** (100 mg, 0.24 mmol) was dissolved in dioxane (5 mL), and 5-iodo-1-methylpyrazole (152 mg, 0.73 mmol), cuprous iodide (139 mg, 0.73 mmol), (1*R*,2*R*)-cyclohexane-1,2-diamine (84 mg, 0.733 mmol), and potassium carbonate (102 mg, 0.73 mmol) were added in sequence. The reaction mixture was stirred at 130°C for 16 hours. The mixture was cooled to 25°C and ice water (20 mL) is used to quench the reaction mixture at 25°C. The aqueous phase was extracted with dichloromethane (50 mL × 3), and the organic phase was washed sequentially with water and saturated brine. The organic phase was dried over anhydrous sodium sulfate, filtered, and concentrated to obtain a crude product, which was purified by high performance liquid chromatography to yield Intermediate **66-2** (20 mg, 16.7%). LCMS (ESI) [M+H]⁺: 490.2.

Step 2: Intermediate **66-2** (20 mg, 0.041 mmol) was dissolved in tetrahydrofuran (2 mL), added dropwise with hydrochloric acid (1 mL, 1 M), stirred at room temperature for half an hour, and then water (10 mL) was added to quench the reaction. The pH of the reaction mixture was adjusted to 8 with saturated sodium bicarbonate solution. The aqueous phase was extracted with dichloromethane (30 mL × 3). The organic phase was washed sequentially with water and saturated brine. The organic phase was dried over anhydrous sodium sulfate, filtered, concentrated, and freeze-dried to give (*R*)-3-methyl-4-(5-(1-methyl-1*H*-pyrazol-5-yl)-1-(1*H*-pyrazol-3-yl)-4,5-dihydro-3*H-*2,2a,5,8-tetraazaacenaphthen-7-yl)morpholine (Compound **66**) (8.5 mg, 51.3%). LCMS (ESI) [M+H]⁺: 406.0. ¹H NMR (400 MHz, CD₃OD) δ 7.99 (s, 1H), 7.71 (s, 1H), 7.00 (s, 1H), 6.60 (s, 1H), 5.77 (s, 1H), 4.75 (s, 2H), 4.34 (s, 2H), 4.22 (d, *J =* 6.4 Hz, 1H), 4.03 (d, *J =* 11.2 Hz, 1H), 3.86 (s, 3H), 3.81 (s, 2H), 3.72 - 3.65 (m, 2H), 3.61 (d, *J =* 5.6 Hz, 1H), 1.35 (d, *J =* 6.8 Hz, 3H).

### Example 71: Preparation of diastereomeric mixture of 6-methyl-4-((R)-3-methylmorpholino)-2-(1H-pyrazol-3-yl)-7,8-dihydro-6H-pyrazolo[4,5,1-ij][1, 7]naphthyridin-6-ol (Compound 71)

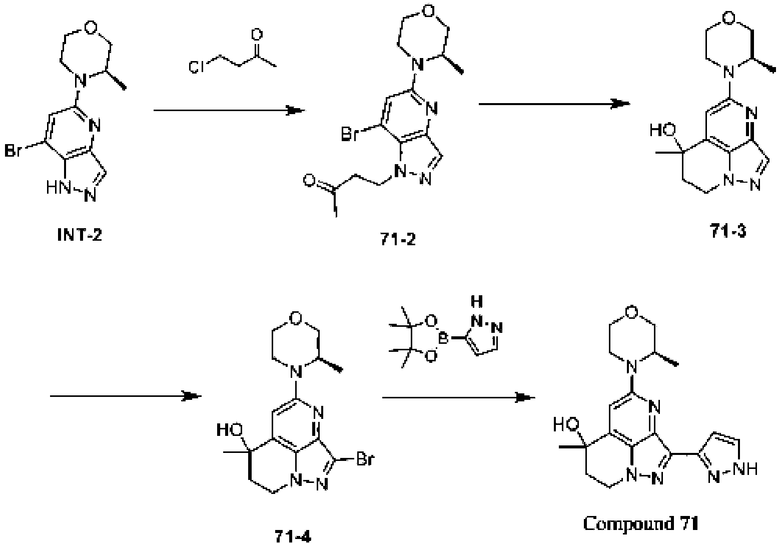

Step 1: **INT-2** (1.5 g, 5.09 mmol) was dissolved in *N,N*-dimethylformamide (10 mL), and 4-chlorobutan-2-one (0.59 g, 5.55 mmol), potassium carbonate (0.77 g, 5.55 mmol), and sodium iodide (76.5 mg, 0.51 mmol) were added in sequence. The reaction mixture was stirred at 85°C for 12 hours. The mixture was cooled to 25°C and ice water (20 mL) was added to quench the reaction. The aqueous phase was extracted with ethyl acetate (50 mL × 3). The organic phase was washed with water (20 mL) and saturated brine (20 mL). The organic phase was dried over anhydrous sodium sulfate, filtered, and concentrated to obtain a crude product. The crude product was purified by silica gel column chromatography to yield Intermediate **71-2** (0.34 g, 18.3%). LCMS (ESI) [M+H]⁺: 367.4.

Step 2: Intermediate **71-2** (0.18 g, 0.49 mmol) was dissolved in tetrahydrofuran (2 mL). Under a nitrogen atmosphere, n-butyllithium (0.24 mL, 2.5 M) was slowly added at -78°C, and stirred at low temperature for 2 hours. The mixture was warmed to 25°C and ice water (10 mL) was added to quench the reaction. The aqueous phase was extracted with ethyl acetate (30 mL × 3). The organic phase was washed with water (20 mL) and saturated brine (20 mL). The organic phase was dried over anhydrous sodium sulfate, filtered, and concentrated to obtain a crude product. The crude product was purified by silica gel column chromatography to yield Intermediate **71-3** (80 mg, 56.6%). LCMS (ESI) [M+H]⁺: 289.1.

Step 3: Intermediate **71-3** (80 mg, 0.28 mmol) was dissolved in *N*,*N*-dimethylformamide (5 mL), and *N*-bromosuccinimide (0.17 g, 0.94 mmol) was added in batches. The mixture was stirred at 80°C for 1 hour. The reaction mixture was quenched with ice water at 25°C. The aqueous phase was extracted with ethyl acetate (30 mL × 3). The organic phase was washed with water (20 mL) and saturated brine (20 mL). The organic phase was dried over anhydrous sodium sulfate, filtered, and concentrated to obtain a crude product. The crude product was purified by silica gel column chromatography to yield Intermediate **71-4** (70 mg, 68.8%). LCMS (ESI) [M+H]⁺: 367.0.

Step 4: Intermediate **71-4** (70 mg, 0.19 mmol) was dissolved in dioxane/water (2 mL, 10/1 v/v), and 1*H*-pyrazole-3-boronic acid pinacol ester (84.5 mg, 0.44 mmol), [1,1'-bis(diphenylphosphino)ferrocene]palladium dichloride (14.1 mg, 0.022 mmol), and potassium carbonate (90.3 mg, 0.65 mmol) were added in sequence. Under a nitrogen atmosphere, the reaction mixture was stirred at 80°C for 2 hours. The reaction mixture was quenched with ice-cold water at 25°C. The aqueous phase was extracted with ethyl acetate (30 mL × 3). The organic phase was washed with water (20 mL) and saturated brine (20 mL). The organic phase was dried over anhydrous sodium sulfate, filtered, and concentrated to obtain a crude product. The crude product was purified by preparative plate (ethyl acetate) to yield a diastereomeric mixture of 6-methyl-4-((*R*)-3-methylmorpholino)-2-(1*H*-pyrazol-3-yl)-7*,*8-dihydro-6*H*-pyrazolo[4,5,1*-ij*][1, 7]naphthyridin-6-ol (Compound **71)** (20 mg, 29.5%). LCMS (ESI) [M+H]⁺: 355.2. ¹H NMR (400 MHz, DMSO-d6) δ 7.66 (s, 1H), 7.02 (s, 1H), 6.97 (s, 1H), 5.64 (d, *J* = 6.4 Hz, 1H), 4.47 - 4.37 (m, 3H), 4.01 - 3.99 (m, 2H), 3.80 - 3.70 (m, 2H), 3.58 - 3.52 (m, 1H), 3.33 (s, 1H), 3.21 - 3.15 (m, 1H), 2.28 - 2.24 (m, 2H), 1.60 (s, 3H), 1.19 (d, *J =* 6.4 Hz, 3H).

### Example 72: Preparation of diastereomeric mixture of 6-isopropyl-4-((R)-3-methylmorpholino)-2-(1H-pyrazol-3-yl)-7,8-dihydro-6H-pyrazolo[4,5,1-ij][ 1,7]naphthyridin-6-ol (Compound 72)

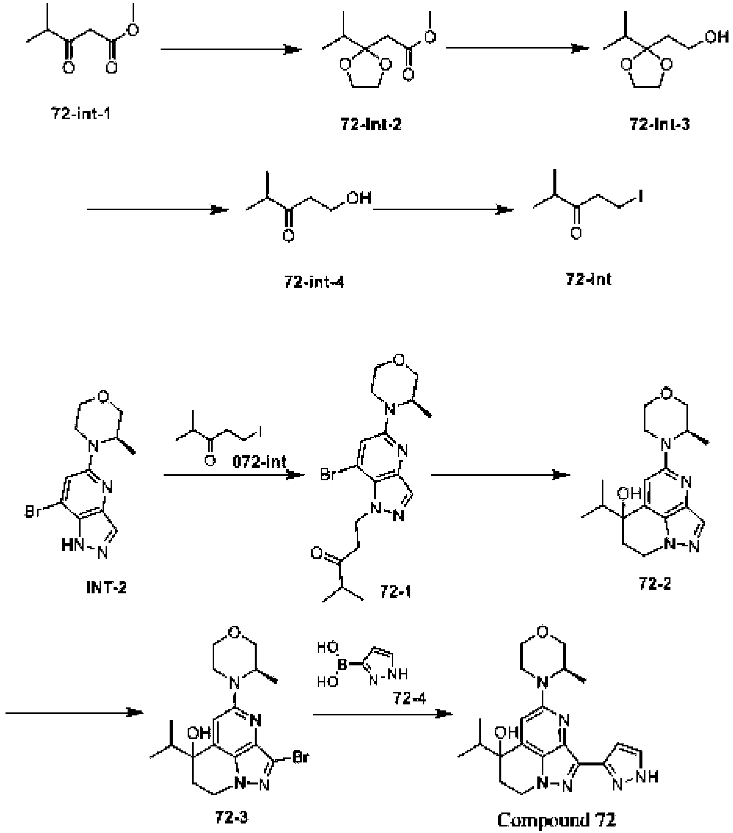

Step 1: **72-int-1** (28 g, 0.19 mol) was dissolved in toluene (150 mL), then ethylene glycol (21.7 mL, 0.39 mol) and 4-methylbenzenesulfonic acid (1.67 g, 9.71 mmol) were added. The reaction mixture was heated to reflux and stirred for 18 hours. The reaction mixture was cooled to room temperature and saturated aqueous sodium carbonate (100 mL) was added to quench the reaction. The organic phase was washed with water (50 mL × 3), dried over anhydrous magnesium sulfate, filtered, and concentrated. The resulting crude product was purified by silica gel column chromatography to yield **72-int-2** (16 g, 43.8%). H NMR (400 MHz, CDCl₃) δ 3.99 (dqd, *J =* 9.6, 8.1, 6.6 Hz, 4H), 3.69 (s, 3H), 2.69 (s, 2H), 2.11 (dt, *J =* 13.7, 6.8 Hz, 1H), 0.97 (d, *J* = 6.9 Hz, 6H).

Step 2: **72-int-2** (16 g, 85 mmol) was dissolved in tetrahydrofuran (150 mL), and then lithium aluminum hydride (4.84 g, 0.13 mol) was added in an ice bath. The reaction mixture was stirred at room temperature for 18 hours. Water (6 mL) and aqueous sodium hydroxide solution (6 mL, 10% wt) were added to the reaction mixture under an ice-water bath. After 10 minutes, 18 mL of water was added. The mixture was filtered, and the filter cake was washed with ethyl acetate (200 mL). The combined organic phases were dried over anhydrous sodium sulfate, filtered, concentrated under reduced pressure, and spin-dried. The resulting crude product was purified by silica gel column chromatography to yield **72-int-3** (8.5 g, 62.4%). ¹H NMR (400 MHz, CDCl₃) δ 4.05 - 3.95 (m, 4H), 3.75 (t, *J =* 5.5 Hz, 2H), 2.88 (br s, 1H), 2.01 - 1.88 (m, 1H), 1.92 (t, *J* = 5.5 Hz, 2H), 0.94 (d, *J =* 6.9 Hz, 6H).

Step 3: **72-int-3** (8.5 g, 53.1 mmol) was dissolved in dichloromethane (460 mL) under ice bath, and then silica gel (54 g) and oxalic acid (0.67 g, 5.31 mmol) were added to the reaction mixture and stirred at room temperature for 2 hours. After the reaction was completed, sodium bicarbonate (0.45 g, 5.31 mmol) was added to the reaction mixture and stirred for 1 hour. The mixture was filtered and concentrated to give **72-int-4** (3.5 g, 56.8%). ¹H NMR (400 MHz, CDCl₃) δ 3.86 (t, *J =* 5.4 Hz, 2H), 2.73 (t, *J =* 5.4 Hz, 2H), 2.62 (dq, *J =* 14.0, 7.0 Hz, 2H), 1.13 (d, *J* = 7.0 Hz, 6H).

Step 4: Under ice-bath, iodine (0.49 g, 1.94 mmol) was dissolved in chloroform (15 mL), and then triphenylphosphine (0.51 g, 1.94 mmol) was added. After stirring for half an hour, **72-int-4** (150 mg, 1.29 mmol) was added to the reaction mixture. The reaction mixture was stirred at room temperature for 11.5 hours. After the reaction was completed, the mixture was diluted with ice water (50 mL), and extracted with dichloromethane (50 mL × 3). The organic phases were combined, dried over anhydrous sodium sulfate, filtered, and concentrated under reduced pressure. The resulting crude product was separated and purified by silica gel column chromatography to yield Intermediate **72-int** (0.19 g, 65.1%). ¹H NMR (400 MHz, CDCl₃) δ 3.29 (t, J = 6.8 Hz, 1H), 3.11 (t, J = 6.8 Hz, 1H), 2.57 (hept, J = 7.2 Hz, 1H), 1.12 (s, 3H), 1.10 (s, 3H).

Step 5: Similar to the preparation method of Example 71, the title Compound 72 was prepared using Intermediate **72-int** instead of 4-chlorobutan-2-one. A diastereomeric mixture of 6-isopropyl-4-((*R*)-3-methylmorpholino)-2-(1*H*-pyrazol-3-yl)-7,8-dihydro-6*H*-pyrazolo[4,5,1-*ij*][ 1,7]naphthyridin-6-ol (Compound **72)** (25 mg, 57.4%) was obtained. LCMS(ESI) [M+H]⁺: 383.0. ¹H NMR (400 MHz, CD₃OD) δ 7.92 (s, 1H), 7.27 (d, *J =* 3.2 Hz, 1H), 6.94 (s, 1H), 4.59 - 4.43 (m, 3H), 4.19 - 4.09 (m, 1H), 4.05 - 3.98 (m, 1H), 3.90 (s, 2H), 3.79 - 3.68 (m, 2H), 2.67 - 2.50 (m, 1H), 2.43 - 2.26 (m, 2H), 1.47 (t, *J =* 6.8 Hz, 3H), 1.15 - 1.06 (m, 6H).

### Example 72A and Example 72B: Preparation of 6-isopropyl-4-((R)-3-methylmorpholino)-2-(1H-pyrazol-3-yl)-7,8-dihydro-6H-pyrazolo[4,5,1-ij][1,7]naphthyridin-6-ol (Compound 72A, Isomer 1) and preparation of 6-isopropyl-4-((R)-3-methylmorpholino)-2-(1H-pyrazol-3-yl)-7,8-dihydro-6H-pyrazolo[4,5,1-ij][1,7]naphthyridin-6-ol (Compound 72B, Isomer 2)

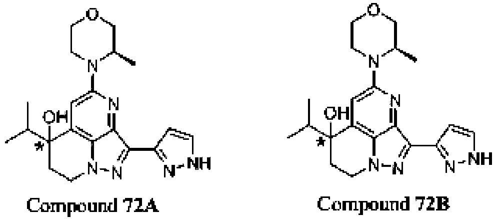

Step 1: The diastereomeric mixture of 6-isopropyl-4-((R)-3-methylmorpholino)-2-(1H-pyrazol-3-yl)-7,8-dihydro-6*H*-pyrazolo[4,5,1-ij][1,7]naphthyridin-6-ol (Compound **72)** (20 mg, 0.52 mmol) was further purified by chiral SFC. SFC analysis method: Daicel ChiralPAK IG column (100 × 3.0 mm, 3 µm), mobile phase: 20% methanol (containing 0.05% diethylamine) in CO₂, temperature: 35°C, flow rate: 2.0 mL/min, detection wavelength: 210 nm.

The first peak was 6-isopropyl-4-((R)-3-methylmorpholino)-2-(1*H*-pyrazol-3-yl)-7,8-dihydro-6*H*-pyrazolo[4,5,1-ij][1,7]naphthyridin-6-ol (Compound **72A,** Isomer 1) (6.2 mg). LCMS (ESI) [M+H]⁺: 383.3. HPLC-SFC: Rt = 2.364 min. ¹H NMR (400 MHz, CD₃OD) δ 7.66 (s, 1H), 6.98 (s, 2H), 4.42 (s, 3H), 4.03 (s, 2H), 3.83 (s, 2H), 3.66 (s, 1H), 2.54 - 2.16 (m, 3H), 2.03 (s, 1H), 1.26 (s, 3H), 1.11 (s, 3H), 1.03 (s, 3H).

The second peak was 6-isopropyl-4-((*R*)-3-methylmorpholino)-2-(1*H*-pyrazol-3-yl)-7,8-dihydro-6*H*-pyrazolo[4,5,1-*ij*][1,7]naphthyridin-6-ol (Compound **72B,** Isomer 2) (6.2 mg). LCMS (ESI) [M+H]⁺: 383.2. HPLC-SFC: Rt = 3.339 min. ¹H NMR (400 MHz, CD₃OD) δ 7.79 - 7.55 (m, 1H), 7.04 (s, 1H), 6.98 (s, 1H), 4.42 (s, 3H), 4.05 (d, *J =* 0.7 Hz, 2H), 3.83 (s, 2H), 3.73 - 3.61 (m, 1H), 2.52 - 2.13 (m, 3H), 2.02 (s, 1H), 1.26 (s, 3H), 1.12 (d, *J =* 6.0 Hz, 3H), 1.02 (d, *J* = 6.2 Hz, 3H).

### Example 73A and Example 73B: Preparation of 4-((R)-3-methylmorpholino)-2-(1H-pyrazol-3-yl)-6-(tetrahydro-2H-pyran-4-yl)-7,8-dihydro-6H-pyrazolo[4,5,1-ij][1,7]naphthyri din-6-ol (Compound 73A, Isomer 1) and preparation of 4-((R)-3-methylmorpholino)-2-(1H-pyrazol-3-yl)-6-(tetrahydro-2H-pyran-4-yl)-7,8-dihydro-6H-pyrazolo[4,5,1-ij][1,7]naphth yridin-6-ol (Compound 73B, Isomer 2)

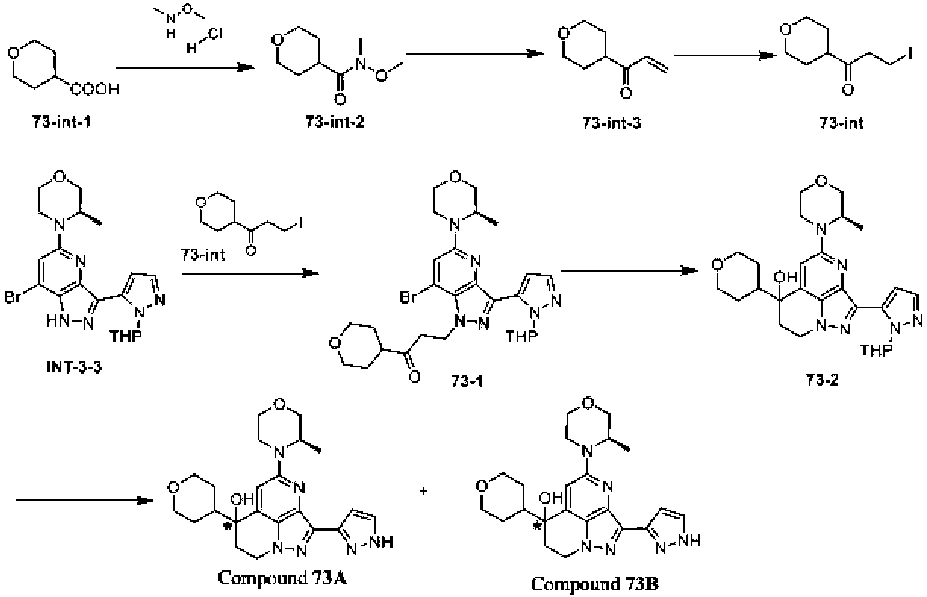

Step 1: Tetrahydropyran-4-carboxylic acid (5 g, 38.43 mmol) was dissolved in dichloromethane (100 mL), and *N*,*N*'-carbonyldiimidazole (7.5 g, 46.12 mmol) and triethylamine were slowly added. The reaction mixture was stirred at room temperature for 2 hours, *N*, *O*-dimethylhydroxylamine hydrochloride (4.1 g, 42.28 mmol) was added, and the mixture was continuously stirred for 16 hours. The reaction was quenched with saturated ammonium chloride (100 mL) at room temperature. The aqueous phase was extracted with dichloromethane (100 mL × 2). The organic phase was washed with saturated brine (100 mL), dried over anhydrous sodium sulfate, filtered, and concentrated to yield Intermediate **73-int-2** (6.2 g, 93.1%). ¹H NMR (400 MHz, CDCl₃) δ 3.98 (ddd, *J =* 11.3, 4.1, 2.0 Hz, 2H), 3.67 (s, 3H), 3.42 (td, *J =* 11.8, 2.2 Hz, 2H), 3.15 (s, 3H), 2.87 (t, *J =* 11.4 Hz, 1H), 1.90 - 1.77 (m, 2H), 1.66 - 1.58 (m, 2H).

Step 2: Intermediate **73-int-2** (5 g, 28.87 mmol) was dissolved in tetrahydrofuran (100 mL). Under a nitrogen atmosphere, vinylmagnesium bromide (57.74 mL, 1 M) was slowly added dropwise at 0°C. The mixture was stirred at 0°C for half an hour, and then at 25°C for 2 hours. Hydrochloric acid solution (200 mL, 1 M) was slowly poured into the mixture at 0°C and stirred for 20 minutes to quench the reaction. The mixture was extracted with ethyl acetate (100 mL × 3). The organic phase was washed with saturated brine (200 mL). The organic phase was dried over anhydrous sodium sulfate, filtered, and concentrated to yield Intermediate **73-int-3** (3 g, 74.1%). ¹H NMR (400 MHz, CDCl₃) δ 6.51 - 6.40 (m, 1H), 6.34 - 6.22 (m, 1H), 5.84 - 5.76 (m, 1H), 4.05 - 3.97 (m, 2H), 3.52 - 3.41 (m, 2H), 2.89 - 2.78 (m, 1H), 1.78 - 1.69 (m, 4H).

Step 3: Intermediate **73-int-3** (200 mg, 1.43 mmol) was dissolved in dichloromethane (5 mL), and trimethylsilyl iodide (342.5 mg, 1.71 mmol) was added at -40°C under a nitrogen atmosphere. The reaction mixture was stirred at -40°C for 2 hours. The reaction was quenched with saturated sodium bicarbonate solution (10 mL) at room temperature. The mixture was extracted with dichloromethane (10 mL × 3). The organic phase was washed with saturated brine (20 mL), dried over anhydrous sodium sulfate, filtered, and concentrated to yield Intermediate **73-int** (300 mg, 78.4%). ¹H NMR (400 MHz, CDCl₃) δ 4.07 - 3.96 (m, 2H), 3.44 (td, *J* = 11.4, 2.9 Hz, 2H), 3.31 (t, *J =* 6.8 Hz, 2H), 3.14 (t, *J =* 6.8 Hz, 2H), 2.62 - 2.50 (m, 1H), 1.83 - 1.68 (m, 4H).

Step 4: Similar to the preparation method of Example 71, the title Compounds **73A** and **73B** were prepared using Intermediate **73-int** instead of 4-chlorobutan-2-one. SFC analysis method: Daicel ChiralPAK-IH column, 100×3.0 mm, 3 µm, mobile phase: 40% methanol in CO₂, flow rate: 2.0 mL/min, detection wavelength: 210 nm.

The first peak was 4-((*R*)-3-methylmorpholino)-2-(1*H*-pyrazol-3-yl)-6-(tetrahydro-2*H-*pyran-4-yl)-7,8-dihydro-6*H*-pyrazolo[4,5,1-*ij*][1,7]naphthyridin-6-ol (Compound **73A,** Isomer 1) (24.3 mg). LCMS (ESI) [M+H]⁺: 425.2. HPLC-SFC: Rt = 3.182 min. ¹H NMR (400 MHz, DMSO-d6) δ 13.33 - 12.75 (m, 1H), 7.60 (s, 1H), 6.96 (s, 1H), 6.90 (s, 1H), 5.47 (s, 1H), 4.50 - 4.31 (m, 3H), 4.07 - 3.95 (m, 2H), 3.94 - 3.85 (m, 2H), 3.78 (d, *J =* 11.1 Hz, 1H), 3.74 - 3.67 (m, 1H), 3.60 - 3.50 (m, 1H), 3.32 - 3.29 (m, 1H), 3.27 - 3.10 (m, 2H), 2.44 - δ: 2.36 (m, 1H), 2.23 - 2.12 (m, 1H), 2.03 (s, 1H), 1.70 - 1.46 (m, 4H), 1.15 (d, *J =* 6.6 Hz, 3H).

The second peak was 4-((*R*)-3-methylmorpholino)-2-(1*H*-pyrazol-3-yl)-6-(tetrahydro-2*H-*pyran-4-yl)-7,8-dihydro-6*H*-pyrazolo[4,5,1-*ij*][1,7]naphthyridin-6-ol (Compound **73B,** Isomer 2) (26.6 mg). LCMS (ESI) [M+H]⁺: 425.2. HPLC-SFC: Rt = 3.673 min. ¹H NMR (400 MHz, DMSO-d6) δ 13.32 - 12.77 (m, 1H), 7.56 (s, 1H), 6.95 (s, 1H), 6.90 (s, 1H), 5.47 (s, 1H), 4.44 - 4.29 (m, 3H), 4.08 - 3.94 (m, 2H), 3.92 - 3.83 (m, 2H), 3.78 - 3.67 (m, 2H), 3.60 - 3.47 (m, 1H), 3.31 - 3.27 (m, 1H), 3.25 - 3.10 (m, 2H), 2.43 - 2.35 (m, 1H), 2.24 - 1.99 (m, 2H), 1.66 - 1.46 (m, 4H), 1.16 (d, 3H).

### Example 74A and Example 74B: Preparation of 6-(1-methyl-1H-pyrazol-4-yl)-4-((R)-3-methylmorpholino)-2-(1H-pyrazol-3-yl)-7,8-dihydro-6H-pyrazolo[4,5,1-ij][1,7]naphthyri din-6-ol (Compound 74A, Isomer 1) and preparation of 6-(1-methyl-1H-pyrazol-4-yl)-4-((R)-3-methylmorpholino)-2-(1H-pyrazol-3-yl)-7,8-dihydro-6H-pyrazolo[4,5,1-ij][1,7]naphthyri din-6-ol (Compound 74B, Isomer 2)

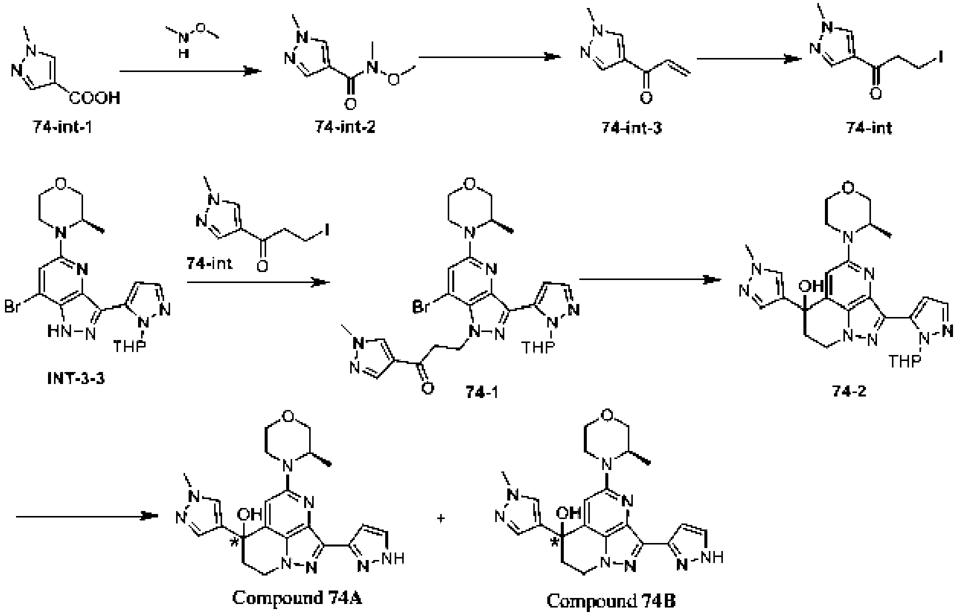

Step 1: Similar to the preparation methods of Examples 73A and 73B, the title Compounds **74A** and **74B** were prepared using 1-methyl-4-pyrazolecarboxylic acid instead of tetrahydropyran-4-carboxylic acid. SFC analysis method: Daicel Chiral PAK-OJ-H 150×4.6mm_5µm, BW009 column, mobile phase: 20% ethanol in n-hexane (containing 0.1% diethylamine), temperature: 35°C, flow rate: 1.0 mL/min, detection wavelength: 228 nm.

The first peak was 6-(1-methyl-1*H*-pyrazol-4-yl)-4-((*R*)-3-methylmorpholino)-2-(1*H*-pyrazol-3-yl)-7,8-dihydro-6*H*-pyrazolo[4,5,1-*ij*][1,7]naphthyridin-6-ol (Compound **74A,** Isomer 1) (8.3 mg). LCMS (ESI) [M+H]⁺: 421.2. HPLC-SFC: Rt = 10.374 min. ¹H NMR (400 MHz, DMSO-d6) δ 13.06 (d, *J =* 141.6 Hz, 1H), 7.68 (d, *J =* 69.7 Hz, 1H), 7.47 (s,1H), 7.40 (s, 1H), 6.96 (s, 1H), 6.86 (s, 1H), 6.15 (s, 1H), 4.50 - 4.39 (m, 1H), 4.31 (dd, *J =* 10.6, 3.6 Hz, 1H), 4.24 - 4.11 (m, 1H), 3.97 (dd, *J =* 11.2, 3.1 Hz, 2H), 3.77 (s, 3H), 3.73 (s, 1H), 3.70 (d, *J =* 2.3 Hz, 1H), 3.67 (d, *J =* 2.5 Hz, 1H), 3.51 (td, *J =* 11.6, 2.7 Hz, 1H), 3.14 (td, *J =* 12.5, 3.3 Hz, 1H), 2.60 - 2.54 (m, 1H), 1.16 (d, *J =* 6.6 Hz, 3H).

The second peak was 6-(1-methyl-1*H*-pyrazol-4-yl)-4-((*R*)-3-methylmorpholino)-2-(1*H*-pyrazol-3-yl)-7,8-dihydro-6*H*-pyrazolo[4,5,1-*ij*][1,7]naphthyridin-6-ol (Compound **74B,** Isomer 2) (7.4 mg). LCMS (ESI) [M+H]⁺: 421.2. HPLC-SFC: Rt = 15.423 min. ¹H NMR (400 MHz, DMSO-d6) δ 13.05 (d, *J =* 142.3 Hz, 1H), 7.68 (d, *J =* 71.4 Hz, 1H), 7.46 (s, 1H), 7.37 (s, 1H), 6.96 (s, 1H), 6.88 (s, 1H), 6.14 (s, 1H), 4.52 - 4.40 (m, 1H), 4.40 - 4.31 (m, 1H), 4.18 - 4.07 (m, 1H), 4.01 - 3.91 (m, 2H), 3.77 (s, 3H), 3.74 (s, 1H), 3.67 (dd, *J =* 11.4, 2.5 Hz, 1H), 3.53 (td, *J =* 11.6, 2.8 Hz, 1H), 3.15 (td, *J =* 12.6, 3.6 Hz, 1H), 2.61 - 2.53 (m, 1H), 1.15 (d, *J =* 6.6 Hz, 3H).

### Example 75: Preparation of (R)-6-(1-methylcyclopropyl)-4-(3-methylmorpholino)-2-(1H-pyrazol-3-yl)-2,6,8,9-tetrahydro-7H-1,2,3,6-tetraazabenzo[cd]azulene-7-one (Compound 75)

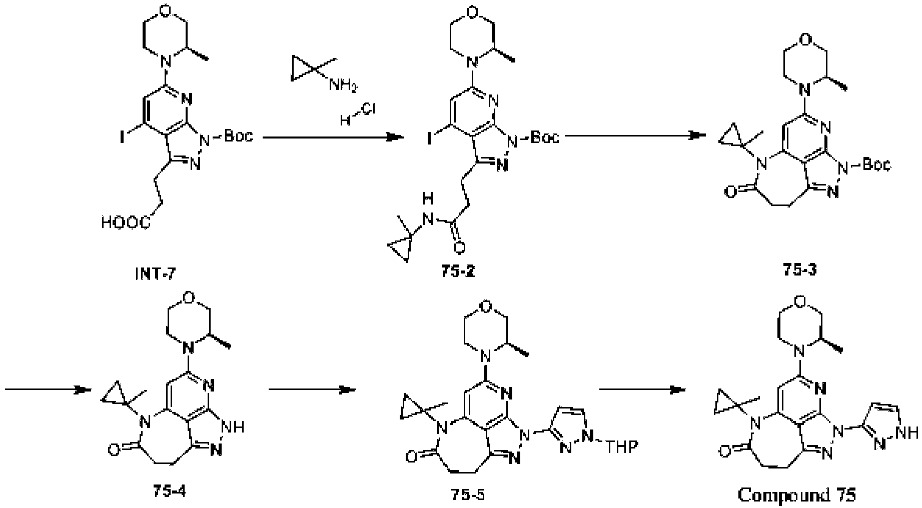

Step 1: Under a nitrogen atmosphere, Intermediate **INT-7** (360 mg, 0.70 mmol) was dissolved in *N*,*N*-dimethylformamide (5 mL), and N,N-diisopropylethylamine (0.35 mL, 2.10 mmol) and *O*-(7-azabenzotriazol-1-yl)-*N,N,N',N*'-tetramethyluronium hexafluorophosphate (397.7 mg, 1.05 mmol) were added. The reaction mixture was stirred for 15 minutes, and 1-methylcyclopropylamine hydrochloride (97.5 mg, 0.91 mmol) was added. The reaction mixture was stirred at room temperature for 2 hours. The reaction was quenched by the addition of ice water. The organic phase was separated, and the aqueous phase was extracted with dichloromethane (10 mL × 3). The combined organic phase was washed sequentially with water and saturated brine, dried over anhydrous sodium sulfate, filtered, and concentrated to obtain a crude product. The crude product was separated and purified by silica gel chromatography to yield Intermediate **75-2** (400 mg, 80.6%). LCMS (ESI) [M+H]⁺: 570.2.

Step 2: Under a nitrogen atmosphere, Intermediate **75-2** (400 mg, 0.702 mmol) was dissolved in dioxane (10 mL), and palladium acetate (78.9 mg, 0.35 mmol), cesium carbonate (686.6 mg, 2.11 mmol), and 4,5-bis(diphenylphosphino)-9,9-dimethylxanthene (203.2 mg, 0.35 mmol) were added. The reaction mixture was stirred at 100°C for 6 hours. The reaction mixture was cooled to room temperature and the reaction is quenched by adding ice-cold water. The resulting organic phase was separated, and the aqueous phase was extracted with ethyl acetate (10 mL × 3). The combined organic phases were washed sequentially with water and saturated brine, dried over anhydrous sodium sulfate, filtered, and concentrated to obtain a crude product. The crude product was separated and purified by silica gel chromatography to yield Intermediate **75-3** (340.0 mg, 68.2%). LCMS (ESI) [M+H]⁺: 442.4.

Step 3: Under a nitrogen atmosphere, Intermediate **75-3** (340.0 mg, 0.77 mmol) was dissolved in dioxane (5 mL), and a solution of hydrochloride in dioxane (1.93 mL, 4 M) was added. The reaction mixture was stirred at room temperature for half an hour, and concentrated to yield crude product Intermediate **75-4** (300.0 mg, 91.3%). LCMS (ESI) [M+H]⁺: 342.4.

Step 4: Intermediate **75-4** (300.0 mg, 0.88 mmol) was dissolved in toluene (10 mL). Cuprous iodide (167.4 mg, 0.88 mmol), potassium phosphate (559.7 mg, 2.64 mmol), N,N-dimethylethylenediamine (155.0 mg, 1.76 mmol), and Intermediate **INT-1-7** (366.6 mg, 1.32 mmol) were added. The reaction mixture was stirred at 100°C for 12 hours under a nitrogen atmosphere. The reaction mixture was cooled to room temperature, and the reaction was quenched by adding ice-cold water. The organic phase was separated, and the aqueous phase was extracted with dichloromethane (10 mL × 3). The combined organic phase was washed sequentially with water and saturated brine, dried over anhydrous sodium sulfate, filtered, and concentrated to obtain a crude product. The crude product was separated and purified by silica gel chromatography to yield Intermediate **75-5** (200.0 mg, 46.3%). LCMS (ESI) [M+H]⁺: 492.4.

Step 5: Intermediate **75-5** (200.0 mg, 0.41 mmol) was dissolved in tetrahydrofuran (10 mL), and hydrochloric acid in ethyl acetate (0.1 mL, 4 M) was added. The reaction mixture was continuously stirred at 50°C for 2 hours. The reaction mixture was cooled to room temperature, the reaction was quenched with saturated sodium bicarbonate solution, and the aqueous phase was extracted with ethyl acetate (30 mL × 3). The organic phase was washed with water and saturated brine, dried over anhydrous sodium sulfate, and filtered. The filtrate was concentrated under reduced pressure to obtain a crude product. The crude product was separated and purified by preparative reverse-phase high performance liquid chromatography to give (R)-6-(1-methylcyclopropyl)-4-(3-methylmorpholino)-2-(1*H*-pyrazol-3-yl)-2,6,8,9-tetrahydro-7*H*-1,2,3,6-tetraazabenzo[*cd*]az ulene-7-one (Compound **75)** (10.6 mg, 6.3%). LCMS (ESI) [M+H]⁺: 408.4. ¹H NMR (400 MHz, DMSO-d6) δ 12.75 (s, 1H), 7.81 (d, *J* = 2.0 Hz, 1H), 6.75 (d, *J* = 2.0 Hz, 1H), 6.51 - 6.40 (m, 1H), 4.36 (s, 1H), 4.00 - 3.80 (m, 2H), 3.74 (m, 2H), 3.53 (m, 1H), 3.23 - 2.81 (m, 5H), 1.48 (d, *J* = 12.8 Hz, 3H), 1.23 (s, 3H), 1.11 - 0.61 (m, 4H).

### Example 76: Preparation of (R)-6-(bicyclo[1.1.1]pentan-1-yl)-4-(3-methylmorpholinyl)-2-(1H-pyrazol-3-yl)-2,6,8,9-tetrahydro-7H-1,2,3,6-tetraazabenzo[cd]azulene-7-one (Compound 76)

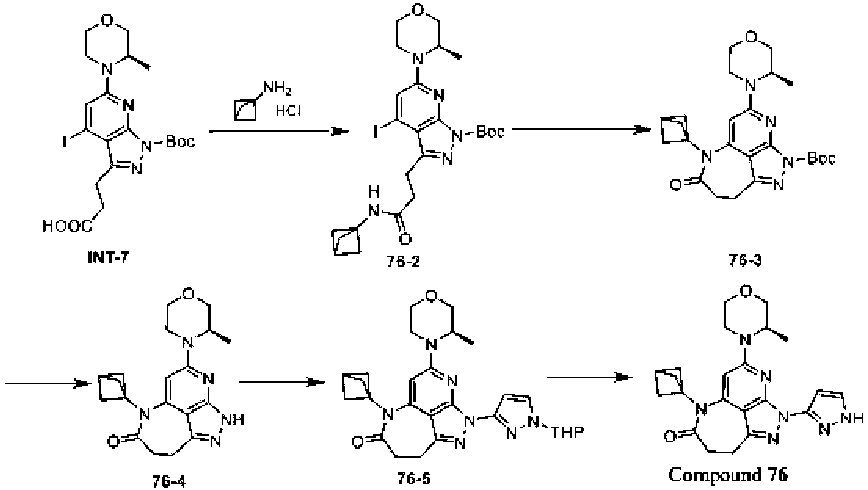

Similar to the preparation method of Example 75, the title Compound 76 was prepared using bicyclo[1,1,1]pentan-1-amine hydrochloride instead of 1-methylcyclopropylamine hydrochloride. (*R*)-6-(Bicyclo[1.1.1]pentan-1-yl)-4-(3-methylmorpholinyl)-2-(1*H*-pyrazol-3-yl)-2,6,8,9-tetrahydro-7*H*-1,2,3,6-tetraazabenzo[*cd*]azulene-7-one (Compound 76) (8.8 mg, 10.6%) was obtained. LCMS(ESI) [M+H]⁺: 419.9. ¹H NMR (400 MHz, DMSO-d6) δ 12.72 (s, 1H), 7.77 (s, 1H), 6.70 (s, 1H), 6.40 (s, 1H), 4.40 - 4.33 (m, 1H), 4.01 - 3.91 (m, 2H), 3.74 - 3.70 (m, 1H), 3.64 - 3.60 (m, 1H), 3.48 - 3.44 (m, 1H), 3.14 - 3.10 (m, 1H), 2.99 - 2.96 (m, 2H), 2.78 - 2.73 (m, 2H), 2.49 (s, 1H), 2.31 (s, 6H), 1.16 (d, *J =* 6.6 Hz, 3H).

### Example 77: Preparation of (R)-6-((1-methylcyclopropyl)methyl)-4-(3-methylmorpholino)-2-(1H-pyrazol-3-yl)-2,6,8,9-tetrahydro-7H-1,2,3,6-tetraazabenzo[cd]azulene-7-one (Compound 77)

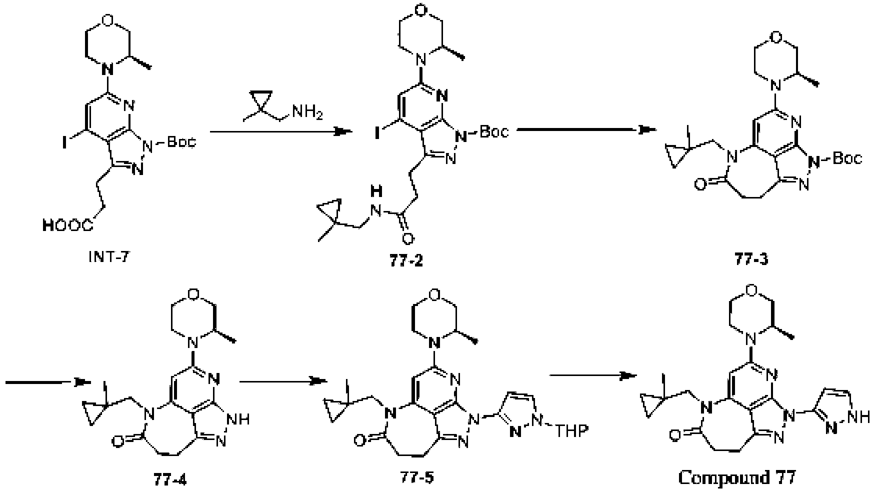

Step 1: Similar to the preparation method of Example 75, the title Compound 77 was prepared using (1-methylcyclopropyl)methanamine hydrochloride instead of 1-methylcyclopropylamine hydrochloride. (*R*)-6-((1-Methylcyclopropyl)methyl)-4-(3-methylmorpholino)-2-(1*H*-pyrazol-3-yl)-2,6,8,9-tetrahydro-7*H*-1,2,3,6-tetraazabenzo[*cd*]azulen-7-one (Compound **77)** (14.2 mg, 11.4%) was obtained. LCMS(ESI) [M+H]⁺: 421.8. ¹H NMR (400 MHz, DMSO-d6) δ 12.78 (s, 1H), 7.83 (s, 1H), 6.79 - 6.75 (m, 1H), 6.52 (s, 1H), 4.48 - 4.42 (m, 1H), 4.21 - 4.16 (m, 1H), 4.13 - 4.06 (m, 2H), 4.02 - 3.97 (m, 1H), 3.80 - 3.76 (m, 1H), 3.69 - 3.65 (m, 1H), 3.54 - 3.48 (m, 1H), 3.21 - 3.14 (m, 1H), 3.07 - δ 3.03 (m, 2H), 2.95 - 2.90 (m, 2H), 1.21 (d, *J =* 6.6 Hz, 3H), 0.94 (s, 3H), 0.49 - 0.45 (m, 2H), 0.28 - 0.25 (m, 2H).

### Example 78: Preparation of (R)-6-isopropyl-4-(3-methylmorpholino)-2-(1H-pyrazol-3-yl)-2,6,8,9-tetrahydro-7H-1,2,3,6-tetraazabenzo[cd]azulene-7-one (Compound 78)

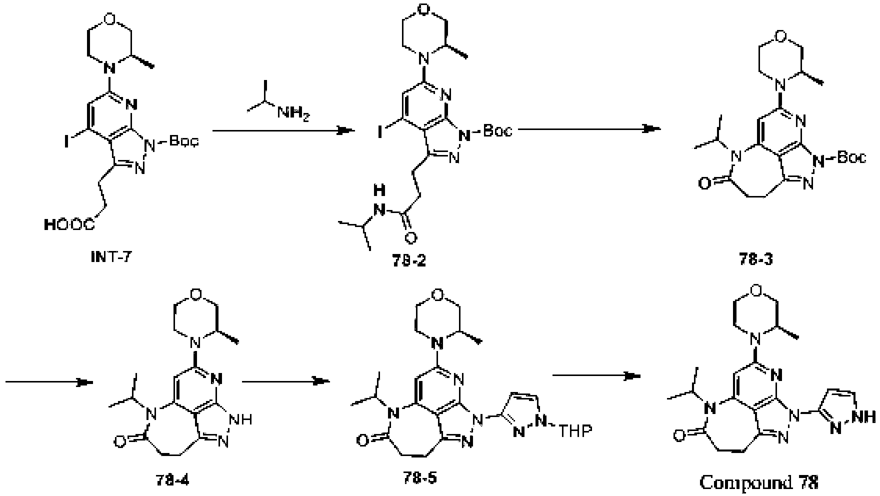

Step 1: Similar to the preparation method of Example 75, the title Compound 78 was prepared using isopropylamine instead of 1-methylcyclopropylamine hydrochloride. (*R*)-6-Isopropy1-4-(3-methylmorpholino)-2-(1*H*-pyrazol-3-yl)-2,6,8,9-tetrahydro-7*H*-1,2,3,6-tetr aazabenzo[cd]azulene-7-one (Compound **78)** (80.0 mg, 64.7%) was obtained. LCMS(ESI) [M+H]⁺: 396.2. ¹H NMR (400 MHz, DMSO-d6) δ 12.77 (s, 1H), 7.82 (s, 1H), 6.76 (s, 1H), 6.39 (s, 1H), 4.73 (dd, *J =* 13.5, 6.8 Hz, 1H), 4.48 (d, *J =* 5.5 Hz, 1H), 4.05 - 4.05 (m, 2H), 3.77 (d, *J* = 11.3 Hz, 1H), 3.66 (d, *J =* 9.2 Hz, 1H), 3.53 - 3.48 (m, 1H), 3.26 - 3.13 (m, 1H), 3.08 - 2.97 (m, 2H), 2.82 - 2.80 (m, 2H), 1.50 - 1.41 (m, 6H), 1.21 (d, *J =* 6.6 Hz, 3H).

### Example 79: Preparation of (R)-6-(3,3-difluorocyclobutyl)-4-(3-methylmorpholinyl)-2-(1H-pyrazol-3-yl)-2,6,8,9-tetrahydro-7H-1,2,3,6-tetraazabenzo[cd]azulene-7-one (Compound 79)

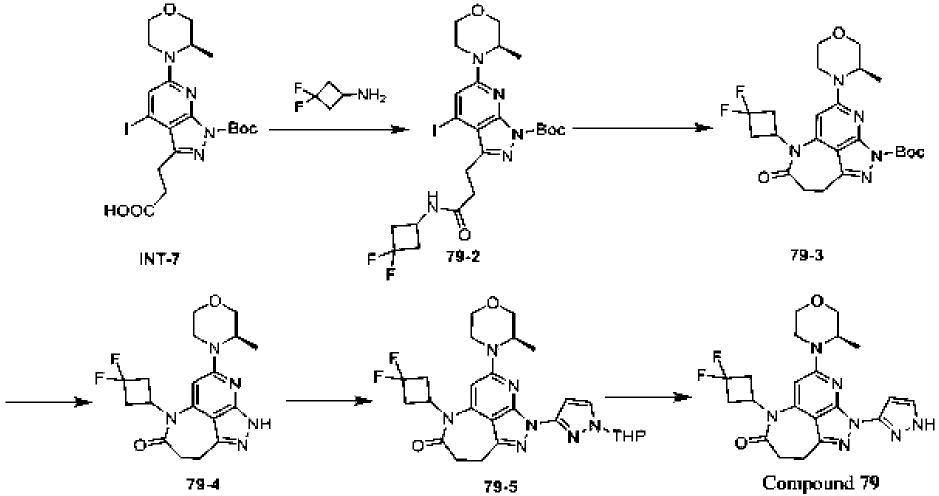

Similar to the preparation method of Example 75, the title Compound 79 was prepared using 3,3-difluorocyclobutan-1-amine instead of 1-methylcyclopropylamine hydrochloride. (*R*)-6-(3,3-Difluorocyclobutyl)-4-(3-methylmorpholinyl)-2-(1*H*-pyrazol-3-yl)-2,6,8,9-tetrahydro-7*H*-1,2,3,6-tetraazabenzo[cd]azulene-7-one (Compound **79)** (7.7 mg, 12.1%) was obtained. LCMS (ESI) [M+H]⁺: 444.2. ¹H NMR (400 MHz, DMSO-d6) δ 12.78 (s, 1H), 7.82 (s, 1H), 6.77 (s, 1H), 6.16 (s, 1H), 4.52 - 4.44 (m, 2H), 4.13 - 4.06 (m, 1H), 4.00 - 3.95 (m, 1H), 3.79 - 3.74 (m, 1H), 3.68 - 3.64 (m, 1H), 3.53 - 3.50 (m, 1H), 3.22 - 3.09 (m, 5H), 2.88 (s, 2H), 2.76 - 2.66 (m, 2H), 1.20 (d, *J =* 6.6 Hz, 3H).

### Example 80: Preparation of (R)-6-(1-(fluoromethyl)cyclopropyl)-4-(3-methylmorpholinyl)-2-(1H-pyrazol-3-yl)-2,6,8,9-tetrahydro-7H-1,2,3,6-tetraazabenzo[cd]azulene-7-one (Compound 80)

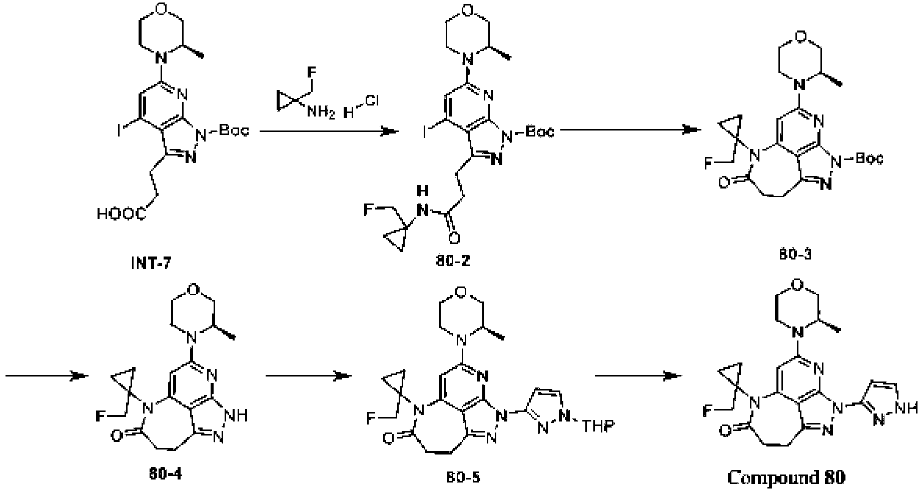

Similarly to the preparation method of Example 75, the title Compound **80** was prepared using 1-(fluoromethyl)cyclopropylamine hydrochloride instead of 1-methylcyclopropylamine hydrochloride. (*R*)-6-(1-(Fluoromethyl)cyclopropyl)-4-(3-methylmorpholinyl)-2-(1H-pyrazol-3-yl)-2,6,8,9-tetrahydro-7*H*-1,2,3,6-tetraazabenzo[*cd*]azulen-7-one (Compound **80)** (4.5 mg, 9.0%) was obtained. LCMS(ESI) [M+H]⁺: 426.2. ¹H NMR (400 MHz, DMSO-d6) δ 12.77 (s, 1H), 7.80 (s, 1H), 6.75 (s, 1H), 6.69 - 6.64 (m, 1H), 5.15 - 4.96 (m, 1H), 4.32 - 4.17 (m, 2H), 4.03 - 3.96 (m, 2H), 3.82 - 3.77 (m, 1H), 3.71 - 3.66 (m, 1H), 3.56 - 3.49 (m, 1H), 3.20 - 3.10 (m, 2H), 3.00 - 2.94 (m, 2H), 2.89 (s, 1H), 1.39 - 1.31 (m, 2H), 1.25 - 1.18 (m, 3H), 1.13 (s, 1H), 0.76 (s, 1H).

### Example 81: Preparation of (R)-6-isopropyl-4-(3-methylmorpholinyl)-2-(1H-pyrazol-3-yl)-8,9-dihydro-1,3,6,9a-tetraazabenzo[cd]azulene-7(6H)-one (Compound 81)

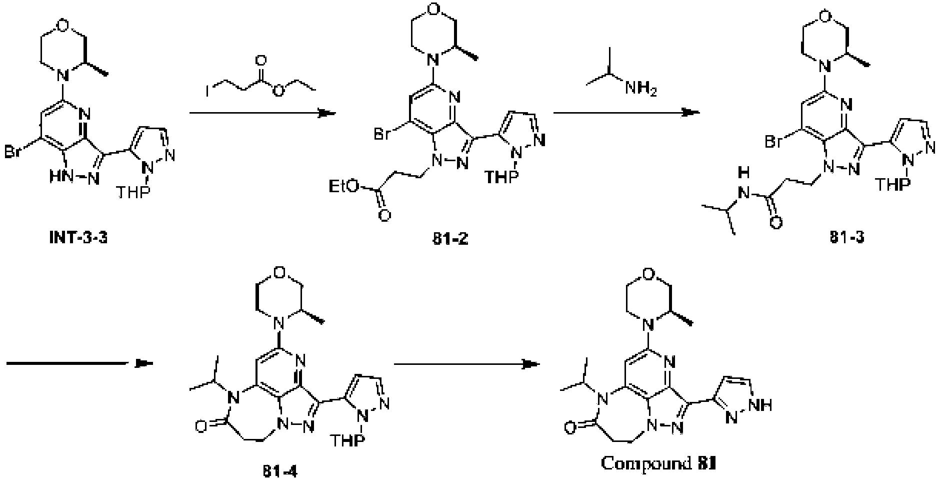

Step 1: Under a nitrogen atmosphere, **INT-3-3** (500 mg, 1.12 mmol) was dissolved in *N*,*N*-dimethylformamide (7 mL), and potassium carbonate (772.4 mg, 5.59 mmol) and ethyl 3-iodopropionate (1.60 g, 7.02 mmol) were added in sequence. The reaction mixture was stirred at 80°C for 12 hours. The mixture was cooled to room temperature. The reaction mixture was concentrated under reduced pressure, and the residue was dissolved in dichloromethane (20 mL), and filtered. The filtrate was concentrated to obtain a crude product, which was purified by silica gel column chromatography to yield Intermediate **81-2** (351.0 mg, 57.4%). LCMS (ESI) [M+H]⁺: 547.2.

Step 2: Intermediate **81-2** (250 mg, 0.46 mmol) was dissolved in tetrahydrofuran (4 mL), and dimethylaluminum chloride (2.28 mL, 1 M) and isopropylamine (269.9 mg, 4.57 mmol) were added in sequence. The reaction mixture was stirred at 45°C for 18 hours. After the reaction was completed, the mixture was cooled to room temperature, saturated sodium bicarbonate solution (15 mL) was added, and the aqueous phase was extracted with dichloromethane (10 mL × 3). The organic phase was dried over anhydrous sodium sulfate, filtered, and concentrated to obtain a crude product, which was purified by silica gel column chromatography to yield Intermediate **81-3** (160 mg, 62.5%). LCMS (ESI) [M+H]⁺: 560.2.

Step 3: Intermediate **81-3** (155.0 mg, 0.28 mmol), cesium carbonate (270.3 mg, 0.83 mmol), and 4,5-bis(diphenylphosphino)-9,9-dimethylxanthene (48.0 mg, 0.083 mmol) were dissolved in *N*,*N*-dimethylformamide (5 mL), and palladium acetate (12.4 mg, 0.055 mmol) was added under a nitrogen atmosphere. The mixture was stirred at 110°C for 3 hours. After the reaction was completed, the reaction mixture was cooled to room temperature, and saturated brine (20 mL) was added, and the aqueous phase was extracted with dichloromethane (30 mL × 3). The organic phase was dried over anhydrous sodium sulfate, filtered, and concentrated to obtain a crude product, which was purified by silica gel column chromatography to yield Intermediate **81-4** (110.0 mg, 82.9%). LCMS (ESI) [M+H]⁺: 480.6.

Step 4: Intermediate **81-4** (100.0 mg, 0.21 mmol) was dissolved in tetrahydrofuran (2 mL), and then dilute hydrochloric acid (1 mL, 2 M) was added. The reaction mixture was stirred at room temperature for half an hour. After the reaction was completed, saturated sodium bicarbonate solution (20 mL) was added, and the aqueous phase was extracted with dichloromethane (30 mL × 3). The organic phase was dried over anhydrous sodium sulfate, filtered, and concentrated to obtain a crude product, which was purified by reverse-phase high performance liquid chromatography to give (*R*)-6-isopropyl-4-(3-methylmorpholinyl)-2-(1*H*-pyrazol-3-yl)-8,9-dihydro-1,3,6,9a-tetraazabenzo[*cd*]azulene-7(6*H*)-one (Compound **81)** (20.0 mg, 24.2%). LCMS (ESI) [M+H]⁺: 396.3. ¹H NMR (400 MHz, DMSO-d6) δ 13.10 (d, *J =* 119.5 Hz, 1H), 7.63 (s, 1H), 7.04 (s, 1H), 6.68 (s, 1H), 4.67 (dt, *J =* 13.9, 7.0 Hz, 1H), 4.62 - 4.56 (m, 2H), 4.48 - 4.39 (m, 1H), 4.02 - 3.96 (m, 2H), 3.78 (d, *J =* 11.2 Hz, 1H), 3.74 - 3.67 (m, 1H), 3.54 (td, *J* = 11.4, 2.3 Hz, 1H), 3.23 - 3.12 (m, 1H), 2.97 - 2.90 (m, 2H), 1.47 (dd, *J =* 10.4, 6.9 Hz, 6H), 1.18 (d, *J =* 6.6 Hz, 3H).

### Example 82: Preparation of (R)-6-cyclopropyl-4-(3-methylmorpholino)-2-(1H-pyrazol-3-yl)-8,9-dihydro-1,3,6,9a-tetraazabenzo[cd]azulene-7(6H)-one (Compound 82)

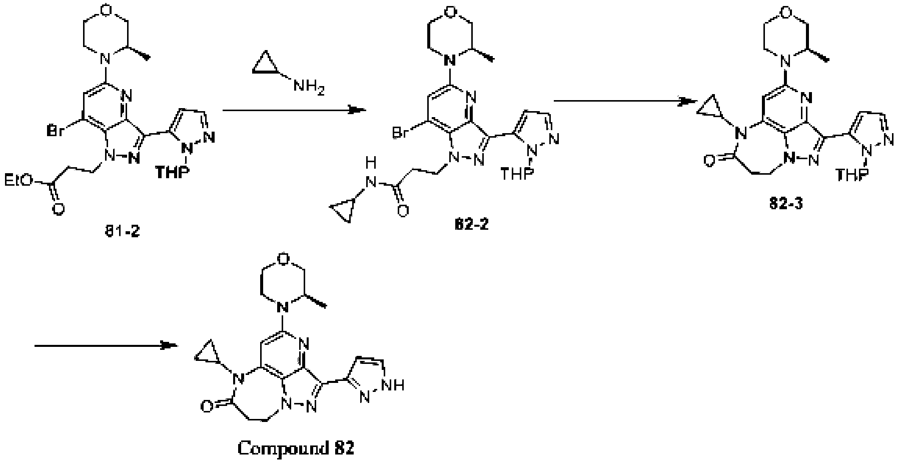

Similarly to the preparation method of Example 81, the title Compound **82** was prepared using cyclopropylamine instead of isopropylamine. (*R*)-6-Cyclopropyl-4-(3-methylmorpholino)-2-(1*H*-pyrazol-3-yl)-8,9-dihydro-1,3,6,9a-tetraazabenzo[*cd*]azulene-7(6*H*)-one (Compound **82)** (55.0 mg, 55.6%) was obtained. LCMS (ESI) [M+H]⁺: 394.3. ¹H NMR (400 MHz, DMSO-d6) δ 13.06 (d, *J =* 104.5 Hz, 1H), 7.63 (s, 1H), 7.02 (s, 1H), 6.88 (s, 1H), 4.68 - 4.46 (m, 2H), 4.39 (d, *J =* 5.9 Hz, 1H), 4.10 - 3.93 (m, 2H), 3.78 (d, *J =* 11.2 Hz, 1H), 3.71 (dd, *J =* 11.3, 2.7 Hz, 1H), 3.55 (td, *J =* 11.7, 2.9 Hz, 1H), 3.16 (td, *J =* δ (d, *J =* 12.9, 3.7 Hz, 1H), 3.09 - 2.99 (m, 3H), 1.19 (d, *J* = 6.6 Hz, 3H), 1.16 - 1.00 (m, 2H), 0.67 - 0.57 (m, 1H), 0.54 - 0.44 (m, 1H).

### Example 83: Preparation of (R)-6-(cyclopropylmethyl)-4-(3-methylmorpholino)-2-(1H-pyrazol-3-yl)-8,9-dihydro-1,3,6,9a-tetraazabenzo[cd]azulene-7(6H)-one (Compound 83)

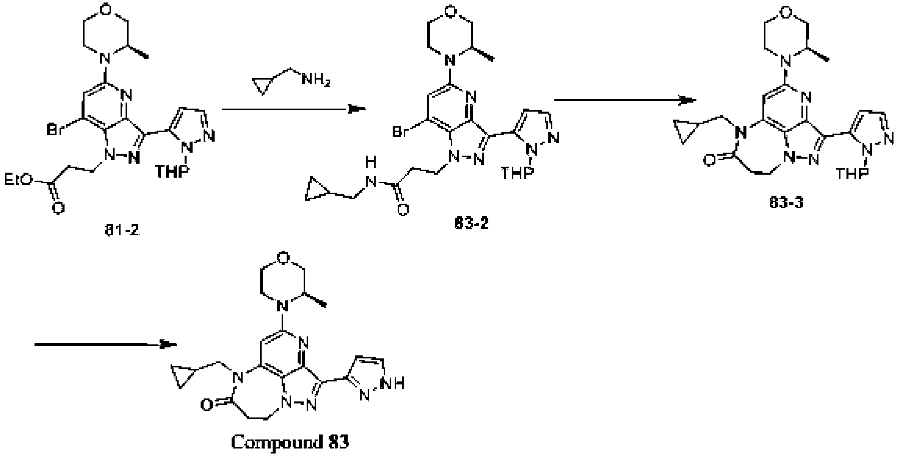

Similarly to the preparation method of Example 81, the title Compound 83 was prepared using cyclopropylmethylamine instead of isopropylamine. (*R*)-6-(Cyclopropylmethyl)-4-(3-methylmorpholino)-2-(1*H*-pyrazol-3-yl)-8,9-dihydro-1,3,6,9a-tetraazabenzo[*cd*]azulene-7(6*H* )-one (Compound 83) (14.5 mg, 29.2%) was obtained. LCMS (ESI) [M+H]⁺: 408.2. ¹H NMR (400 MHz, CD₃OD) δ 7.68 (d, *J* = 2.0 Hz, 1H), 7.09 (d, *J =* 2.0 Hz, 1H), 6.84 (s, 1H), 4.67 - 4.63 (m, 2H), 4.43 (d, *J =* 6.8 Hz, 1H), 4.09 - 4.03 (m, 3H), 4.00 (d, *J =* 11.6 Hz, 1H), 3.84 (d, *J =* 2.0 Hz, 2H), 3.72 - 3.65 (m, 1H), 3.38 - 3.32 (m, 1H), 3.13 - 3.08 (m, 2H), 1.28 (d, *J =* 6.8 Hz, 3H), 1.20 - 1.11 (m, 1H), 0.55 - 0.50 (m, 2H), 0.42 - 0.37 (m, 2H).

### Example 84: Preparation of (R)-6-isobutyl-4-(3-methylmorpholino)-2-(1H-pyrazol-3-yl)-8,9-dihydro-1,3,6,9a-tetraazabenzo[cd]azulene-7(6H)-one (Compound 84)

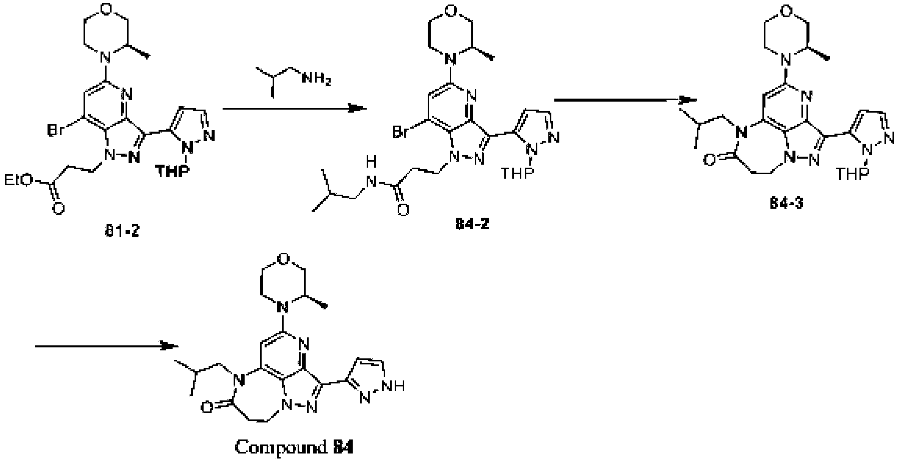

Similarly to the preparation method of Example 81, the title Compound **84** was prepared using isobutylamine instead of isopropylamine. (*R*)-6-Isobutyl-4-(3-methylmorpholino)-2-(1*H*-pyrazol-3-yl)-8,9-dihydro-1,3,6,9a-tetraazabenzo[*cd*]azulene-7(6*H*)-one (Compound **84)** (65.0 mg, 60.3%) was obtained. LCMS (ESI) [M+H]⁺: 410.4. ¹H NMR (400 MHz, DMSO-d6) δ 13.45 - 12.66 (m, 1H), 7.66 (s, 1H), 7.04 (s, 1H), 6.82 (s, 1H), 4.67 - 4.53 (m, 2H), 4.49 - 4.38 (m, 1H), 4.12 - 3.92 (m, 4H), 3.78 (d, *J =* 11.2 Hz, 1H), 3.71 (dd, *J =* 11.3, 2.7 Hz, 1H), 3.55 (td, *J =* 11.6, 2.8 Hz, 1H), 3.14 (td, *J =* 12.8, δ (d, *J =* 3.7 Hz, 1H), 3.10 - 3.03 (m, 2H), 1.97 - 1.81 (m, 1H), 1.16 (d, *J =* 6.6 Hz, 3H), 0.84 (d, *J =* 6.8 Hz, 3H), 0.81 (d, *J =* 6.8 Hz, 3H).

### Example 85: Preparation of (R)-4-(3-methylmorpholino)-2-(1H-pyrazol-3-yl)-6-(3,3,3-trifluoropropyl)-8,9-dihydro-1,3,6,9a-tetraazabenzo[cd]azulene-7(6H)-one (Compound 85)

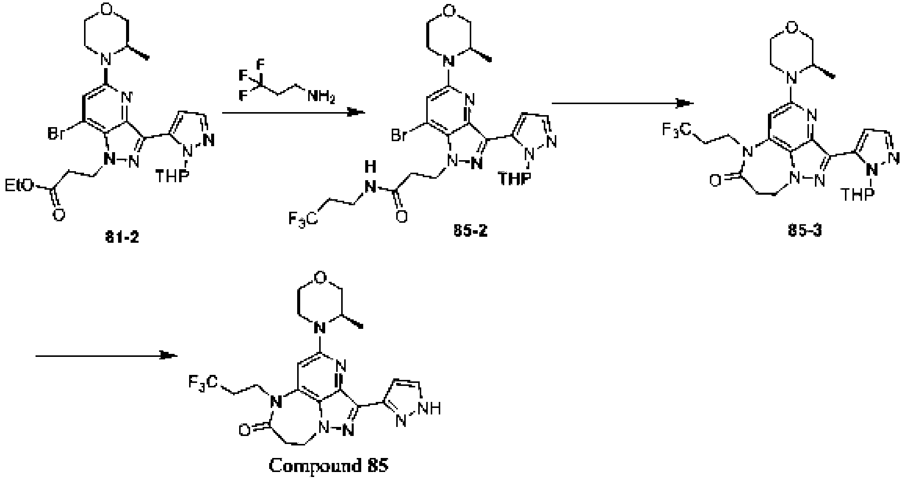

Similar to the preparation method of Example 81, the title Compound 85 was prepared using 3,3,3-trifluoropropylamine instead of isopropylamine. (R)-4-(3-Methylmorpholino)-2-(1H-pyrazol-3-yl)-6-(3,3,3-trifluoropropyl)-8,9-dihydro-1,3,6,9a-tetraazabenzo[*cd*]azulene-7(6*H*)-one (Compound 85) (26.47 mg, 29.9%) was obtained. LCMS (ESI) [M+H]⁺: 449.9. ¹H NMR (400 MHz, CD₃OD) δ 7.67 (d, *J =* 1.6 Hz, 1H), 7.11 (d, *J =* 1.6 Hz, 1H), 6.74 (s, 1H), 4.68 - 4.62 (m, 2H), 4.43 (d, *J =* 6.8 Hz, 1H), 4.41 - 4.36 (m, 2H), 4.08 - 4.00 (m, 2H), 3.84 (d, *J =* 2.0 Hz, 2H), 3.72 - 3.64 (m, 1H), 3.37 - 3.32 (m, 1H), 3.14 - 3.09 (m, 2H), 2.69 - 2.56 (m, 2H), 1.28 (d, *J =* 6.8 Hz, 3H).

### Example 86: Preparation of (R)-6-methyl-4-(3-methylmorpholino)-2-(1H-pyrazol-3-yl)-8,9-dihydro-1,3,6,9a-tetraazabenzo[cd]azulene-7(6H)-one (Compound 86)

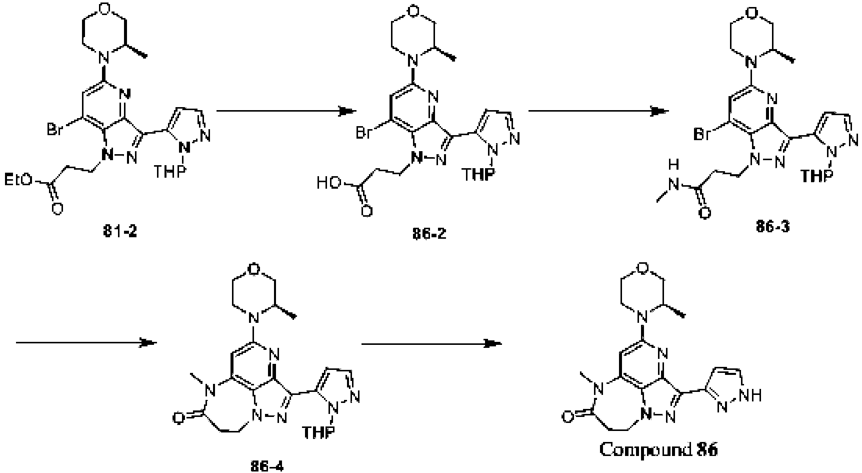

Step 1: Intermediate **81-2** (300 mg, 0.562 mmol) was dissolved in a tetrahydrofuran/water mixture (5 mL, v/v = 5/1), and lithium hydroxide (70.9 mg, 1.687 mmol) was added. The mixture was stirred at room temperature for half an hour. The reaction mixture was quenched by adding ice-cold water at room temperature. The resulting mixture was extracted with ethyl acetate (10 mL × 3). The organic phase was washed sequentially with water and saturated brine. The organic phase was dried over anhydrous sodium sulfate, filtered, and concentrated to yield Intermediate **86-2** (220 mg, 75.3%). LCMS (ESI) [M+H]⁺: 519.2.

Step 2: Intermediate **86-2** was dissolved in *N*,*N*-dimethylformamide (5 mL), and methylamine hydrochloride (104.0 mg, 1.540 mmol), *O*-(7-azabenzotriazol-1-yl)-*N,N,N',N*'-tetramethyluronium hexafluorophosphate (219.6 mg, 0.578 mmol), and *N*,*N*-diisopropylethylamine (124.4 mg, 0.963 mmol) were added in sequence. The mixture was stirred at room temperature for half an hour. The reaction mixture was quenched by adding ice-cold water at room temperature. The resulting mixture was extracted with ethyl acetate (10 mL × 3). The organic phase was washed sequentially with water and saturated brine. The organic phase was dried over anhydrous sodium sulfate, filtered, and concentrated to yield Intermediate **86-3** (40 mg, 39.0%). LCMS (ESI) [M+H]⁺: 532.2.

Step 3: Intermediate **86-3** (40.0 mg, 0.075 mmol) was dissolved in dioxane, and palladium acetate (8.4 mg, 0.038 mmol), 4,5-bis(diphenylphosphino)-9,9-dimethylxanthene (21.7 mg, 0.038 mmol), and cesium carbonate (73.4 mg, 0.225 mmol) were added in sequence. The mixture was stirred at 100°C for 2 hours under a nitrogen atmosphere. The reaction mixture was quenched with ice water at 25°C. The resulting mixture was extracted with ethyl acetate (10 mL × 3). The organic phase was washed sequentially with water and saturated brine, dried over anhydrous sodium sulfate, filtered, and concentrated to obtain a crude product. The crude product was separated and purified by silica gel chromatography to yield Intermediate **86-4** (23.0 mg, 67.8%). LCMS (ESI) [M+H]⁺: 452.0.

Step 4: Intermediate **86-4** (25 mg, 0.055 mmol) was dissolved in dichloromethane (1 mL), and trifluoroacetic acid (2 mL) was added. The mixture was stirred at room temperature for 5 minutes, and then spin-dried to remove the solvent to obtain a crude product. The crude product was separated and purified by preparative reverse-phase high performance liquid chromatography to yield (*R*)-6-methyl-4-(3-methylmorpholino)-2-(1*H*-pyrazol-3-yl)-8,9-dihydro-1,3,6,9a-tetraazabenzo[*c d*]azulene-7(6*H*)-one (Compound **86)** (9.2 mg, 45.2%). LCMS (ESI) [M+H]⁺: 368.0. ¹H NMR (400 MHz, CD₃OD) δ 7.67 (d, *J =* 2.0 Hz, 1H), 7.09 (d, *J =* 2.0 Hz, 1H), 6.71 (s, 1H), 4.73 - 4.61 (m, 2H), 4.48 (d, *J =* 6.6 Hz, 1H), 4.09 - 3.95 (m, 2H), 3.84 (d, *J =* 2.1 Hz, 2H), 3.72 - 3.63 (m, 1H), 3.53 (s, 3H), 3.39 - 3.33 (m, 1H), 3.16 - 3.09 (m, 2H), 1.28 (d, *J =* 6.7 Hz, 3H).

### Example 87: Preparation of (R)-6-((1-methylcyclopropyl)methyl)-4-(3-methylmorpholinyl)-2-(1H-pyrazol-3-yl)-8,9-dihydro-1,3,6,9a-tetraazabenzo[cd]azulene-7(6H)-one (Compound 87)

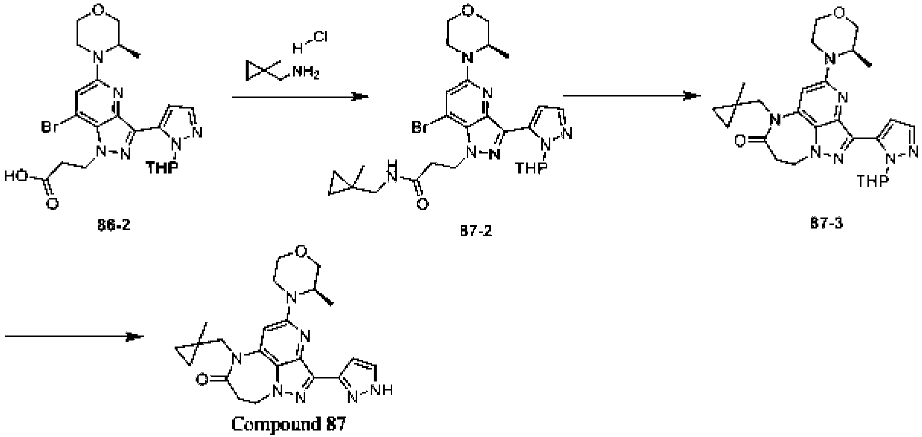

Similar to the preparation method of Example 86, the title Compound **87** was prepared using (1-methylcyclopropyl)methanamine hydrochloride instead of methylamine hydrochloride. (*R*)-6-((1-Methylcyclopropyl)methyl)-4-(3-methylmorpholinyl)-2-(1*H*-pyrazol-3-yl)-8,9-dihydro -1,3,6,9a-tetraazabenzo[cd]azulene-7(6*H*)-one (Compound **87)** (10.4 mg, 14.7%) was obtained. LCMS (ESI) [M+H]⁺: 422.4. ¹H NMR (400 MHz, CD₃OD) δ 7.71 (s, 1H), 7.15 (s, 1H), 6.97 (s, 1H), 4.75 - 4.68 (m, 2H), 4.48 (d, *J =* 6.8 Hz, 1H), 4.28 - 4.18 (m, 2H), 4.12 - 4.02 (m, 2H), 3.88 (s, 2H), 3.76 - 3.68 (m, 1H), 3.43 - 3.36 (m, 1H), 3.17 - 3.10 (m, 2H), 1.32 (d, *J =* 6.8 Hz, 3H), 1.01 (s, 3H), 0.57 (t, *J =* 4.8 Hz, 2H), 0.36 (t, *J =* 4.8 Hz, 2H).

### Example 88: Preparation of (R)-6-(1-methylcyclopropyl)-4-(3-methylmorpholinyl)-2-(1H-pyrazol-3-yl)-8,9-dihydro-1,3,6,9a-tetraazabenzo[cd]azulene-7(6H)-one (Compound 88)

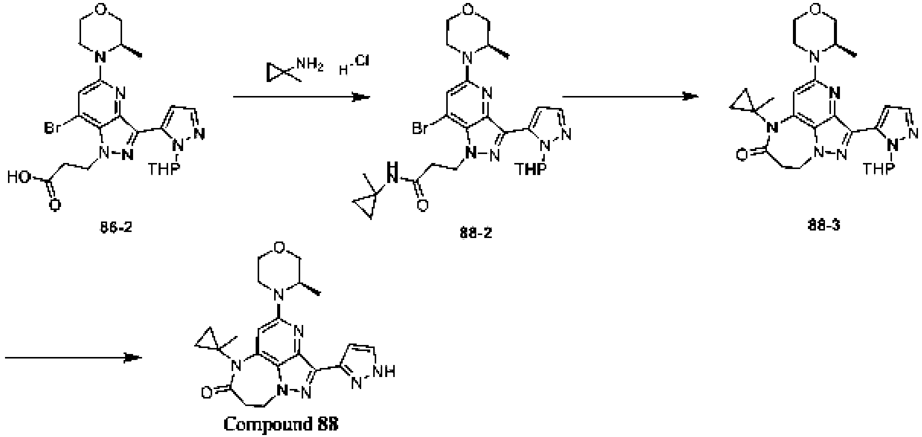

Similarly to the preparation method of Example 86, the Compound **88** was prepared using 1-methylcyclopropylamine hydrochloride instead of methylamine hydrochloride. (*R*)-6-(1-Methylcyclopropyl)-4-(3-methylmorpholinyl)-2-(1*H*-pyrazol-3-yl)-8,9-dihydro-1,3,6,9a -tetraazabenzo[cd]azulene-7(6H)-one (Compound **88)** (13.6 mg, 19.3%) was obtained. LCMS (ESI) [M+H]⁺: 408.4. ¹H NMR (400 MHz, CD₃OD) δ 7.70 (s, 1H), 7.13 (s, 1H), 6.94 (s, 1H), 4.70 - 4.61 (m, 2H), 4.40 (s, 1H), 4.07 (t, *J* = 19.2 Hz, 2H), 3.90 (s, 2H), 3.73 (t, *J* = 10.4 Hz, 1H), 3.44 - 3.37 (m, 1H), 3.20 - 3.15 (m, 1H), 3.10 - 3.01 (m, 1H), 1.69 (d, *J =* 6.0 Hz, 3H), 1.36 - 1.32 (m, 3H), 1.22 - 1.13 (m, 2H), 1.04 - 0.95 (m, 1H), 0.73 - 0.64 (m, 1H).

### Example 89: Preparation of 6-(2,2-difluorocyclopropyl)-4-((R)-3-methylmorpholinyl)-2-(1H-pyrazol-3-yl)-8,9-dihydro-1,3,6,9a-tetraazabenzo[cd]azulene-7(6H)-one (Compound 89)

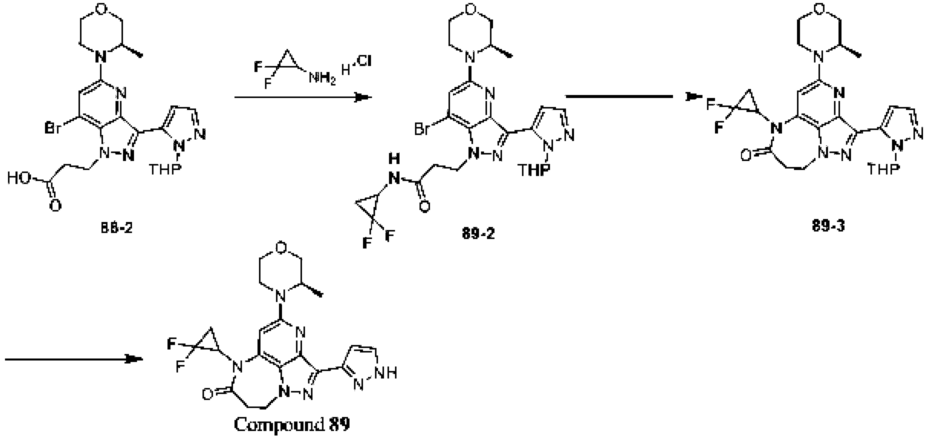

Similarly to the preparation method of Example 86, the title Compound 89 was prepared using 2,2-difluorocyclopropylamine hydrochloride instead of methylamine hydrochloride. 6-(2,2-Difluorocyclopropyl)-4-((*R*)-3-methylmorpholinyl)-2-(1*H*-pyrazol-3-yl)-8,9-dihydro-1,3, 6,9a-tetraazabenzo[*cd*]azulene-7(6*H*)-one (Compound *89*) (1.2 mg, 10.0%) was obtained. LCMS (ESI) [M+H]⁺: 430.2. ¹H NMR (400 MHz, CD₃OD) δ 7.69 (s, 1H), 7.13 (s, 1H), 6.75 (d, *J =* 11.6 Hz, 1H), 4.77 - 4.70 (m, 1H), 4.61 - 4.59 (m, 1H), 4.48 - 4.41 (m, 1H), 4.09 - 4.07 (m, 2H), 3.87 (s, 2H), 3.84 - 3.77 (m, 1H), 3.71 - 3.69 (m, 1H), 3.29 - 3.22 (m, 1H), 2.22 - 2.20 (m, 1H), 2.11 - δ 2.01 (m, 1H), 1.32 (d, *J =* 6.4 Hz, 3H), 0.93 - 0.91 (m, 2H).

### Example 90: Preparation of 4-((R)-3-methylmorpholino)-2-(1H-pyrazol-3-yl)-6-(tetrahydrofuran-3-yl)-8,9-dihydro-1,3,6,9a-tetraazabenzo[cd]azulene-7(6H)-one (Compound 90)

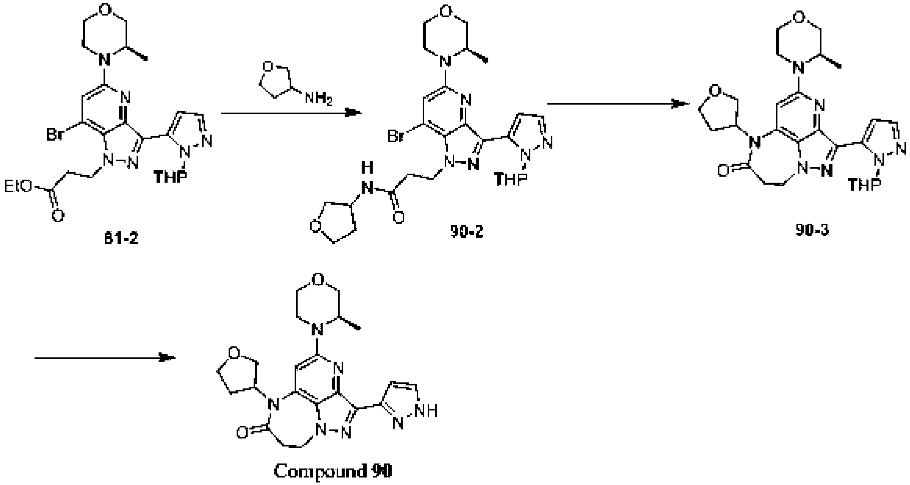

Similarly to the preparation method of Example 81, the title Compound 90 was prepared using 3-aminotetrahydrofuran in place of isopropylamine. 4-((*R*)-3-Methylmorpholino)-2-(1*H*-pyrazol-3-yl)-6-(tetrahydrofuran-3-yl)-8,9-dihydro-1,3,6,9a-tetraazabenzo[*cd*]azulene-7(6 *H*)-one (Compound **90)** (40.0 mg, 79.9%) was obtained. LCMS (ESI) [M+H]⁺: 424.0. ¹H NMR (400 MHz, CD₃OD) δ 7.68 (d, *J =* 2.0 Hz, 1H), 7.08 (d, *J =* 2.0 Hz, 1H), 6.95 (d, *J =* 7.2 Hz, 1H), 4.68 - 4.64 (m, 2H), 4.43 (d, *J =* 7.2 Hz, 1H), 4.28 (s, 1H), 4.10 - 4.05 (m, 1H), 4.04 - 3.92 (m, 3H), 3.83 (s, 2H), 3.75 (d, *J =* 7.2 Hz, 1H), 3.70 - 3.63 (m, 1H), 3.36 (d, *J =* 12.8 Hz, 1H), 3.07 (d, *J* = 7.2 Hz, 2H), 2.32 - 2.17 (m, 2H), 1.29 (d, *J =* 6.8 Hz, 3H).

### Example 91: Preparation of (R)-4-(3-methylmorpholino)-2-(1H-pyrazol-3-yl)-6-(tetrahydro-2H-pyran-4-yl)-8,9-dihydro-1,3,6,9a-tetraazabenzo[cd]azulene-7(6H)-one (Compound 91)

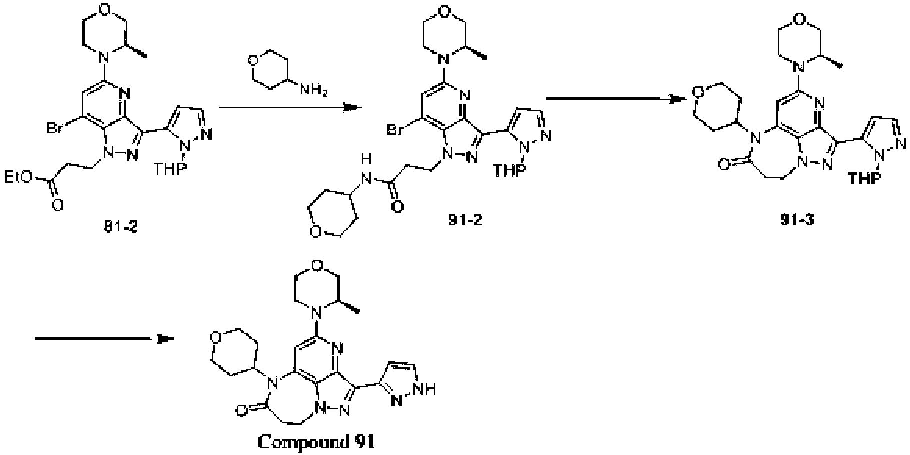

Similarly to the preparation method of Example 81, the title Compound **91** was prepared using 4-aminotetrahydropyran in place of isopropylamine. (*R*)-4-(3-Methylmorpholino)-2-(1*H-*pyrazol-3-yl)-6-(tetrahydro-2*H*-pyran-4-yl)-8,9-dihydro-1,3,6,9a-tetraazabenzo[*cd*]azulene-7(6*H* )-one (Compound **91)** (25.9 mg, 51.5%) was obtained. LCMS (ESI) [M+H]⁺: 438.2. ¹H NMR (400 MHz, CD₃OD) δ 7.67 (d, *J =* 2.0 Hz, 1H), 7.11 (d, *J =* 2.0 Hz, 1H), 6.80 (s, 1H), 4.68 - 4.63 (m, 2H), 4.58 - 4.50 (m, 1H), 4.48 - 4.43 (m, 1H), 4.08 - 3.97 (m, 4H), 3.85 (d, *J =* 2.0 Hz, 2H), 3.69 (td, *J* = 11.6, 3.2 Hz, 1H), 3.58 - 3.50 (m, 2H), 3.39 - 3.34 (m, 1H), 3.04 - 2.97 (m, 2H), 2.71 - 2.62 (m, 2H), 1.81 - 1.74 (m, 2H), 1.30 (d, *J =* 6.8 Hz, 3H).

### Example 92: Preparation of (R)-4-(3-methylmorpholino)-2-(1H-pyrazol-3-yl)-6-(spiro [3.3]hept-2-yl)-8,9-dihydro-1,3,6,9a-tetraazabenzo[cd]azulene-7(6H)-one (Compound 92)

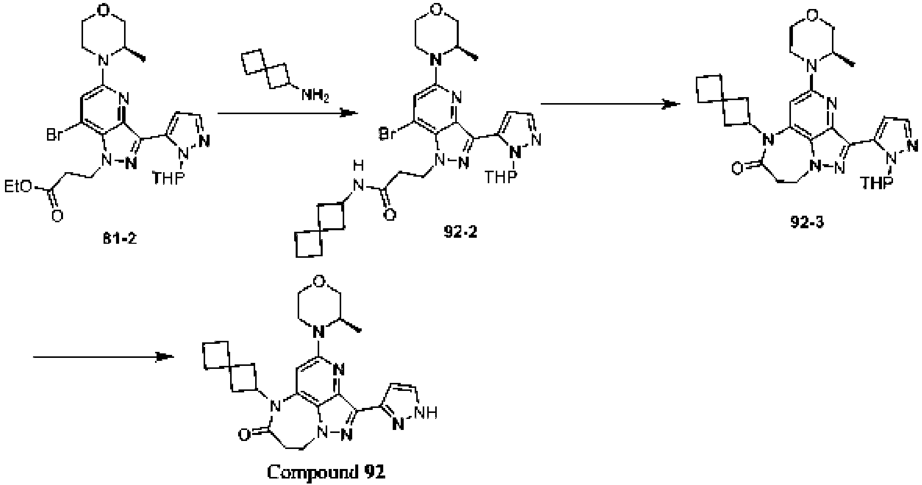

Similarly to the preparation method of Example 81, the title Compound **92** was prepared using 2-aminospiro[3.3]heptane instead of isopropylamine. (*R*)-4-(3-Methylmorpholino)-2-(1*H-*pyrazol-3-yl)-6-(spiro[3.3]hept-2-yl)-8,9-dihydro-1,3,6,9a-tetraazabenzo[*cd*]azulene-7(6*H*)-one (Compound **92)** (25.0 mg, 29.5%) was obtained. LCMS (ESI) [M+H]⁺: 448.4. ¹H NMR (400 MHz), DMSO-d6) δ 13.22 - 12.90 (m, 1H), 7.79 - 7.57 (m, 1H), 7.07 - 6.99 (m, 1H), 6.38 (s, 1H), 4.62 - 4.60 (m, 2H), 4.49 - 4.31 (m, 2H), 4.07 - 3.91 (m, 2H), 3.77 (d, *J =* 11.2 Hz, 1H), 3.69 (dd, *J =* 11.3, 2.7 Hz, 1H), 3.54 (td, *J =* 11.6, 2.8 Hz, 1H), 3.15 (td, *J =* 13.0, 3.6 Hz, 1H), 2.96 (s, 2H), 2.66 - 2.57 (m, 2H), 2.09 (t, *J =* 6.6 Hz, 2H), 1.84 - 1.70 (m, 6H), 1.17 (d, *J =* 6.6 Hz, 3H).

### Example 93: Preparation of (R)-4-(3-methylmorpholino)-2-(1H-pyrazol-3-yl)-8,9-dihydro-1,3,6,9a-tetraazabenzo[cd]azulene-7(6H)-one (Compound 93)

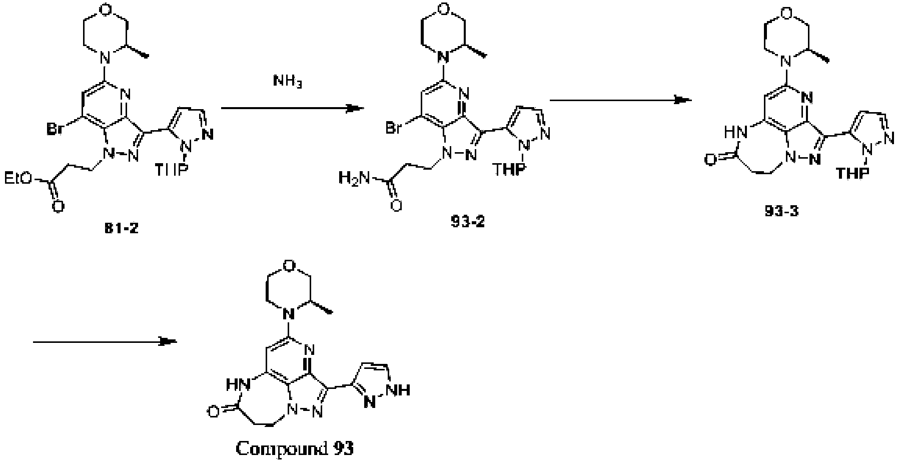

Step 1: Intermediate **81-2** (500.0 mg, 0.913 mmol) was dissolved in aminomethanol solution (10 mL). The reaction mixture was stirred at 50°C for 3 hours. The reaction mixture was cooled to room temperature and concentrated to obtain a crude product, which was purified by silica gel column chromatography to give Intermediate **93-2** (65.0 mg, 13.7%). LCMS (ESI) [M+H]⁺: 518.2.

Step 2: Similar to the preparation method of Example 81, the title Compound **93** was prepared using Intermediate **93-2** instead of Intermediate **81-3.** (*R*)-4-(3-Methylmorpholino)-2-(1*H*-pyrazol-3-yl)-8,9-dihydro-1,3,6,9a-tetraazabenzo[*cd*]azulene-7(6*H*)-one (Compound **93)** (23.0 mg, 73.2%) was obtained. LCMS (ESI) [M+H]⁺: 354.3. ¹H NMR (400 MHz, DMSO-d6) δ 13.05 (d, *J =* 117.8 Hz, 1H), 10.63 (s, 1H), 7.68 (d, *J =* 90.1 Hz, 1H), 7.03 (d, *J =* 32.9 Hz, 1H), 6.54 (s, 1H), 4.63 - 4.51 (m, 2H), 4.25 (s, 1H), 3.99 (dd, *J =* 11.2, 3.0 Hz, 1H), 3.89 (d, *J =* 13.7 Hz, 1H), 3.78 (d, *J* = 11.2 Hz, 1H), 3.68 (dd, *J* = 11.3, 2.7 Hz, 1H), 3.53 (td, *J* = 11.7, 2.9 Hz, 1H), 3.21 - 3.03 (m, 3H), 1.15 (d, *J =* 6.6 Hz, 3H).

### Example 94: Preparation of (R)-6-(6-hydroxy-6-methylspiro[3.3]hept-2-yl)-4-(3-methylmorpholinyl)-2-(1H-pyrazol-3-yl)-8,9-dihydro-1,3,6,9a-tetraazabenzo[cd]azulene-7(6H)-one (Compound 94)

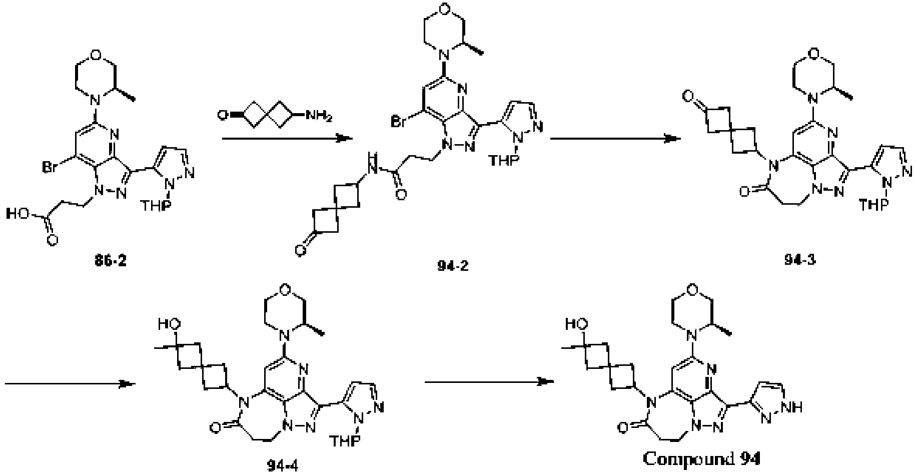

Step 1: Similar to the preparation method of Example 86, Intermediate **94-3** was prepared using 6-aminospiro[3.3]hept-2-one hydrochloride instead of methylamine hydrochloride. 4-((*R*)-3-Methylmorpholinyl)-6-(6-oxospiro[3.3]hept-2-yl)-2-(1-(tetrahydro-2H-pyran-2-yl)-1H-pyrazol-5-yl)-8,9-dihydro-1,3,6,9a-tetraazabenzo[*cd*]azulene-7(6*H*)-one (Intermediate **94-3)** (60.0 mg, 35%) was obtained. LCMS (ESI) [M+H]⁺: 546.2.

Step 2: Under a nitrogen atmosphere at -78°C, Intermediate **94-3** (80.0 mg, 0.15 mmol) was dissolved in tetrahydrofuran (3 mL), and slowly added dropwise with methylmagnesium bromide in tetrahydrofuran (0.2 mL, 3 M). The reaction mixture was stirred at 25°C for 2 hours. The reaction was quenched by the addition of saturated ammonium chloride solution (5 mL). The aqueous phase was extracted with ethyl acetate (20 mL × 3). The organic phase was washed sequentially with water and saturated brine, dried over anhydrous sodium sulfate, filtered, and spin-dried to obtain a crude product. The crude product was purified by silica gel column chromatography to yield Intermediate **94-4** (20.0 mg, 24.3%). LCMS (ESI) [M+H]⁺: 562.4.

Step 3: Intermediate **94-4** (45.0 mg, 0.08 mmol) was dissolved in tetrahydrofuran (1 mL), and aqueous hydrochloric acid (0.5 mL, 2 M) was added. The mixture was stirred at 25°C for half an hour. The reaction was quenched by the addition of ice water (10 mL). The pH of the mixture was adjusted to 8 with saturated sodium bicarbonate solution, and the aqueous phase was extracted with dichloromethane (30 mL × 3). The organic phase was washed sequentially with water and saturated brine, dried over anhydrous sodium sulfate, filtered, and spin-dried to obtain a crude product. The crude product was separated and purified by preparative reverse-phase high performance liquid chromatography to give (R)-6-(6-hydroxy-6-methylspiro[3.3]hept-2-yl)-4-(3-methylmorpholinyl)-2-(1*H*-pyrazol-3-yl)-8,9-dihydro-1,3,6,9a-tetraazabenzo[*cd*]azulene-7(6 *H*)-one (Compound **94)** (8.2 mg, 21.4%). LCMS (ESI) [M+H]⁺: 478.4. ¹H NMR (400 MHz, CD₃OD) δ 7.73 (s, 1H), 7.11 (s, 1H), 6.43 (s, 1H), 4.72 - 4.70 (m, 2H), 4.57 - 4.55 (m, 1H), 4.49 - 4.42 (m, 1H), 4.10 - 4.08 (m, 1H), 4.06 - 3.99 (m, 1H), 3.88 (s, 2H), 3.71 (t, *J =* 10.4 Hz, 1H), 3.41 (d, *J =* 14.0 Hz, 1H), 3.07 (d, *J =* 6.0 Hz, 2H), 2.88 - 2.76 (m, 1H), 2.73 - 2.63 (m, 1H), 2.34 - 2.26 (m, 2H), 2.12 - 2.02 (m, 2H), 1.98 - 1.96 (m, 2H), 1.35 - 1.31 (m, 6H).

### Example 95: Preparation of (R)-6-(2-hydroxy-2-methylpropyl)-4-(3-methylmorpholinyl)-2-(1H-pyrazol-3-yl)-8,9-dihydro-1,3,6,9a-tetraazabenzo[cd]azulene-7(6H)-one (Compound 95)

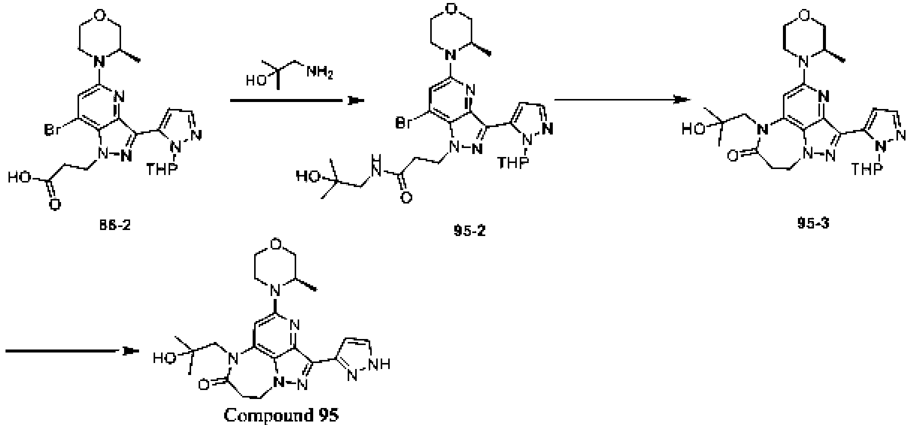

Similarly to the preparation method of Example 86, the title Compound 95 was prepared using 1-amino-2-methylpropanol instead of methylamine hydrochloride. (*R*)-6-(2-Hydroxy-2-methylpropyl)-4-(3-methylmorpholinyl)-2-(1*H*-pyrazol-3-yl)-8,9-dihydro-1, 3,6,9a-tetraazabenzo[*cd*]azulene-7(6*H*)-one (Compound 95) (2.0 mg, 24.0%) was obtained. LCMS (ESI) [M+H]⁺: 426.5. ¹H NMR (400 MHz, CD₃OD) δ 7.68 (s, 1H), 7.58 (s, 1H), 7.12 (s, 1H), 4.72 - 4.67 (m, 2H), 4.59 (s, 1H), 4.45 (d, *J =* 6.8 Hz, 1H), 4.17 (s, 2H), 4.07 (s, 1H), 4.04 (d, *J* = 2.8 Hz, 1H), 3.84 (d, *J =* 2.0 Hz, 2H), 3.70 - 3.68 (m, 1H), 3.37 (s, 1H), 3.14 - 3.12 (m, 2H), 1.30 (s, 6H), 1.27 (s, 3H).

### Example 96: Preparation of 4-((R)-3-methylmorpholinyl)-2-(1H-pyrazol-3-yl)-6-(2-(trifluoromethyl)cyclopropyl)-8,9-dihydro-1,3,6,9a-tetraazabenzo[cd]azulene-7(6H)-one (Compound 96)

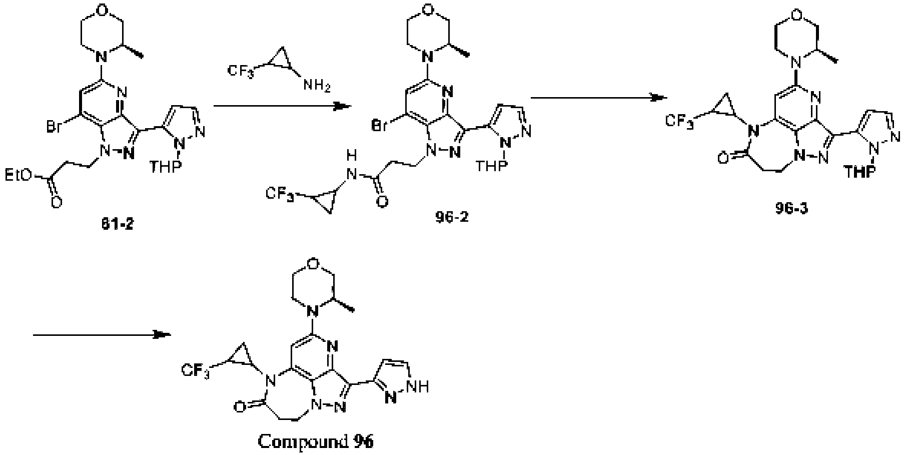

Similar to the preparation method of Example 81, the title Compound 96 was prepared using 2-(trifluoromethyl)cyclopropylamine instead of isopropylamine. 4-((*R*)-3-Methylmorpholinyl)-2-(1*H*-pyrazol-3-yl)-6-(2-(trifluoromethyl)cyclopropyl)-8,9-dihydr o-1,3,6,9a-tetraazabenzo[*cd*]azulene-7(6*H*)-one (Compound **96)** (3.5 mg, 3.0%) was obtained. LCMS (ESI) [M+H]⁺: 462.2. ¹H NMR (400 MHz, CD₃OD) δ 7.66 (d, *J* = 2.0 Hz, 1H), 7.09 (s, 1H), 6.85 (d, *J =* 3.2 Hz, 1H), 4.67 - 4.62 (m, 2H), 4.53 - 4.42 (m, 1H), 4.07 - 3.94 (m, 2H), 3.83 (s, 2H), 3.71 - 3.63 (m, 1H), 3.55 - 3.50 (m, 1H), 3.41 - 3.35 (m, 1H), 3.15 - 3.09 (m, 2H), 2.16 - 2.06 (m, δ (m, 1H), 1.66 - 1.58 (m, 1H), 1.32 - 1.28 (m, 3H), 1.19 - 1.12 (m, 1H).

### Example 97: Preparation of (R)-6-(6-hydroxyspiro[3.3]hept-2-yl)-4-(3-methylmorpholinyl)-2-(1H-pyrazol-3-yl)-8,9-dihydro-1,3,6,9a-tetraazabenzo[cd]azulene-7(6H)-one (Compound 97)

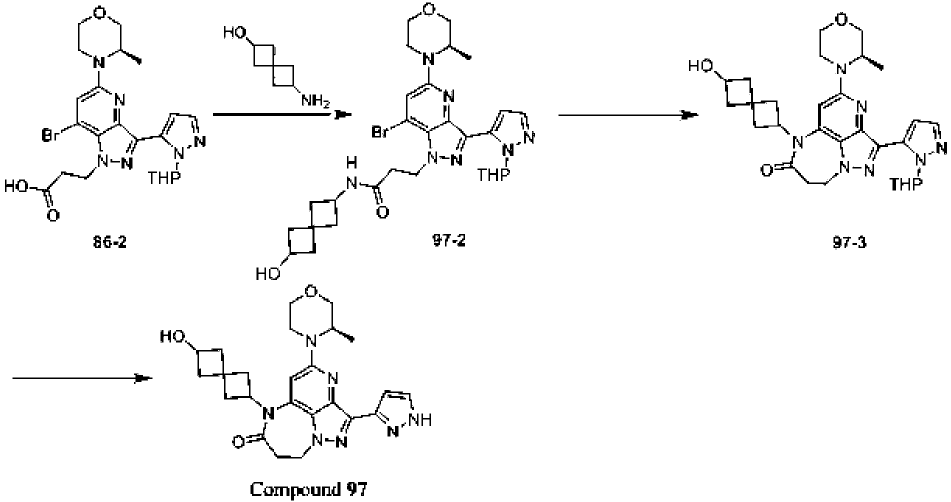

Similarly to the preparation method of Example 86, the title Compound **97** was prepared using 6-aminospiro[3.3]hept-2-ol instead of methylamine hydrochloride. (*R*)-6-(6-Hydroxyspiro[3.3]hept-2-yl)-4-(3-methylmorpholinyl)-2-(1*H*-pyrazol-3-yl)-8,9-dihydro -1,3,6,9a-tetraazabenzo[*cd*]azulene-7(6*H*)-one (Compound **97)** was obtained (4.8 mg, 11.3%). LCMS (ESI) [M+H]⁺: 464.2. ¹H NMR (400 MHz, CD₃OD) δ 7.71 (s, 1H), 7.14 (s, 1H), 6.42 (s, 1H), 4.73 - 4.68 (m, 2H), 4.63 (s, 1H), 4.59 - 4.53 (m, 1H), 4.47 (s, 1H), 4.16 (d, *J =* 4.8 Hz, 1H), 4.07 - 4.05 (m, 2H), 3.88 (s, 2H), 3.71 (t, *J =* 11.6 Hz, 1H), 3.05 (d, *J =* 4.4 Hz, 2H), 2.75 - 2.73 (m, 1H), δ: 2.67 - 2.54 (m, 2H), 2.20 (d, *J =* 4.0 Hz, 1H), 2.07 - 1.95 (m, 3H), 1.90 - 1.98 (m, 1H), 1.32 (d, *J =* 6.8 Hz, 3H).

### Example 98: Preparation of 6-(sec-butyl)-4-((R)-3-methylmorpholinyl)-2-(1H-pyrazol-3-yl)-8,9-dihydro-1,3,6,9a-tetraazabenzo[cd]azulene-7(6H)-one (Compound 98)

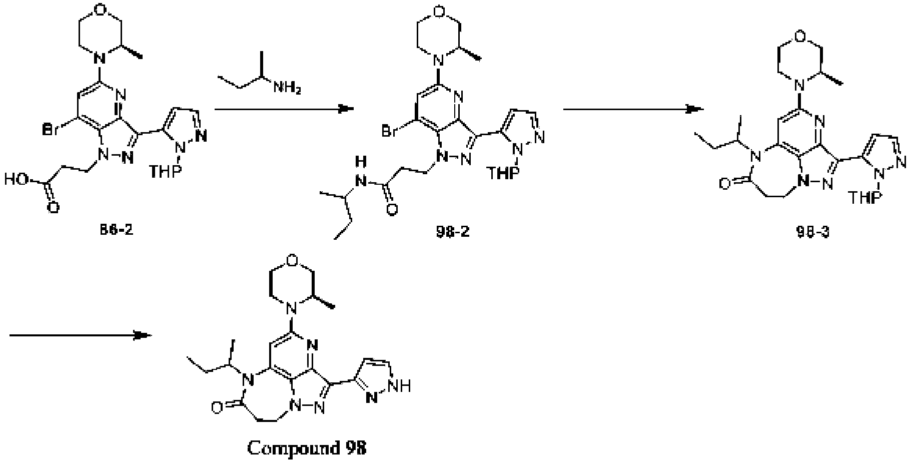

Similarly to the preparation method of Example 86, the title Compound **98** was prepared using sec-butylamine instead of methylamine hydrochloride. 6-(Sec-butyl)-4-((*R*)-3-methylmorpholinyl)-2-(1*H*-pyrazol-3-yl)-8,9-dihydro-1,3,6,9a-tetraazabenzo[*cd*]azulene-7(6*H*)-one (Compound **98)** (16.0 mg, 42.9%) was obtained. LCMS (ESI) [M+H]⁺: 410.0. ¹H NMR (400 MHz, DMSO-d6) δ 13.39 - 12.78 (m, 1H), 7.90 - 7.46 (m, 1H), 7.00 (s, 1H), 6.64 (s, 1H), 4.64 - 4.51 (m, 2H), 4.39 (s, 2H), 3.97 (dd, *J =* 11.2, 3.0 Hz, 2H), 3.75 (d, *J =* 11.2 Hz, 1H), 3.67 (dd, *J* = 11.2, 2.8 Hz, 1H), 3.52 (td, *J =* 11.8, 2.8 Hz, 1H), 3.14 (td, *J =* 12.8, 3.6 Hz, 1H), 2.99 - 2.83 (m, 2H), 2.10 - 1.94 (m, 1H), 1.80 - 1.64 (m, 1H), 1.45 (t, *J =* 7.2 Hz, 3H), 1.14 (d, *J =* 6.4 Hz, 3H), 0.73 (dt, *J =* 15.2, 7.4 Hz, 3H).

### Example 99: Preparation of (R)-6-((1-methyl-1H-pyrazol-4-yl)methyl)-4-(3-methylmorpholinyl)-2-(1H-pyrazol-3-yl)-8,9-dihydro-1,3,6,9a-tetraazabenzo[cd]azulene-7(6H)-one (Compound 99)

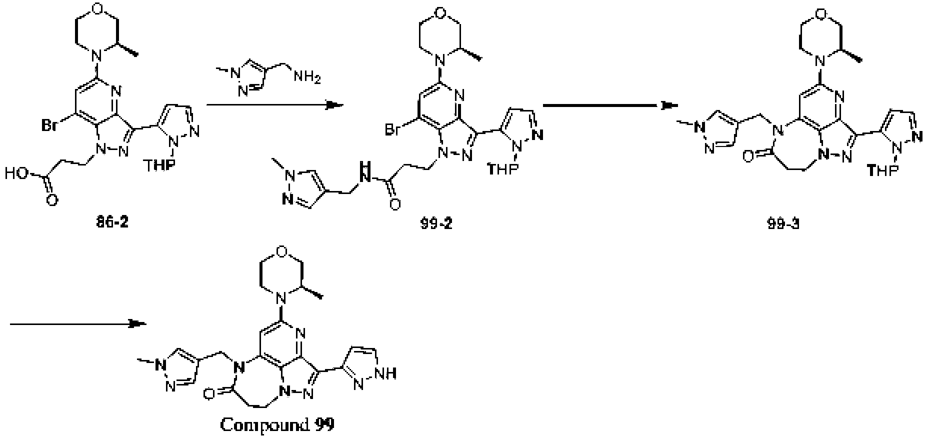

Similar to the preparation method of Example 86, the title Compound **99** was prepared using 4-(aminomethyl)-1-methylpyrazole instead of methylamine hydrochloride. (*R*)-6-((1-Methyl-1*H*-pyrazol-4-yl)methyl)-4-(3-methylmorpholinyl)-2-(1*H*-pyrazol-3-yl)-8,9-di hydro-1,3,6,9a-tetraazabenzo[*cd*]azulene-7(6*H*)-one (Compound **99)** (46.8 mg, 56.8%) was obtained. LCMS (ESI) [M+H]⁺: 448.2. ¹H NMR (400 MHz, CD₃OD) δ 7.70 (s, 1H), 7.62 (s, 1H), 7.51 (s, 1H), 7.13 (s, 1H), 6.82 (s, 1H), 5.28 (d, *J =* 15.6 Hz, 1H), 5.14 (d, *J =* 15.6 Hz, 1H), 4.74 - 4.69 (m, 2H), 4.40 - 4.33 (m, 1H), 4.09 - 4.07 (m, 1H), 4.00 (d, *J* = 12.4 Hz, 1H), 3.88 - 3.86 (m, 2H), 3.85 (s, 3H), 3.74 - 3.66 (m, 1H), 3.31 - 3.29 (m, 1H), 3.23 - 3.18 (m, 2H), 1.25 (d, *J* = 6.8 Hz, 3H).

### Example 100: Preparation of 6-(1-cyclopropylethyl)-4-((R)-3-methylmorpholinyl)-2-(1H-pyrazol-3-yl)-8,9-dihydro-1,3,6,9a-tetraazabenzo[cd]azulene-7(6H)-one (Compound 100)

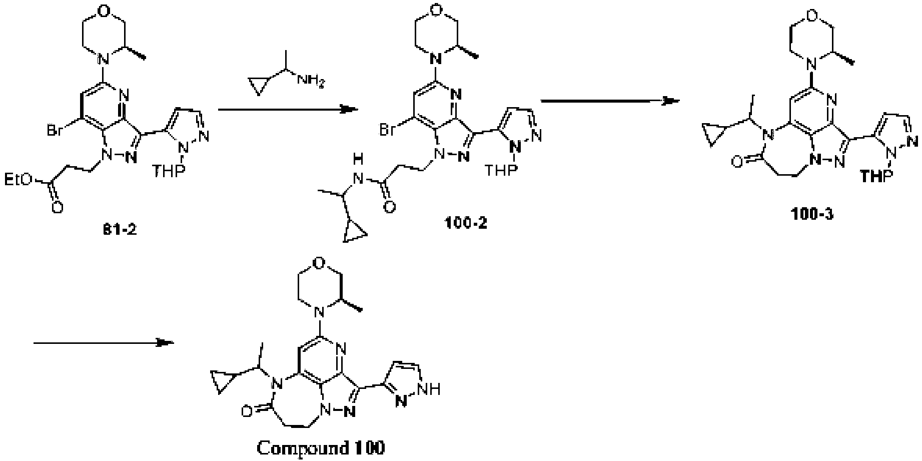

Similarly to the preparation method of Example 81, the title Compound **100** was prepared using (1-cyclopropylethyl)amine instead of isopropylamine. 6-(1-Cyclopropylethyl)-4-((R)-3-methylmorpholinyl)-2-(1*H*-pyrazol-3-yl)-8,9-dihydro-1,3,6,9a-tetraazabenzo[*cd*]azulene-7(6*H*)-one (Compound **100)** (8.8 mg, 17.6%) was obtained. LCMS (ESI) [M+H]⁺: 422.4. ¹H NMR (400 MHz, CD₃OD) δ 7.67 (s, 1H), 7.11 (s, 1H), 6.81 (s, 1H), 4.68 - 4.64 (m, 2H), 4.41 (d, *J =* 6.4 Hz, 1H), 4.07 - 4.02 (m, 1H), 3.95 (s, 1H), 3.84 (d, *J =* 2.0 Hz, 2H), 3.71 - 3.65 (m, 1H), 3.36 (d, *J =* 3.6 Hz, 1H), 3.05 - 2.99 (m, 2H), 1.65 - 1.60 (m, 3H), 1.30 - δ 1.26 (m, 3H), 1.23 (d, *J* = 7.2 Hz, 2H), 0.68 (m, 1H), 0.43 - 0.35 (m, 2H), 0.16 - 0.09 (m, 1H).

### Example 101: Preparation of (R)-4-(3-methylmorpholinyl)-6-(3-methyloxetan-3-yl)-2-(1H-pyrazol-3-yl)-8,9-dihydro-1,3,6,9a-tetraazabenzo[cd]azulene-7(6H)-one (Compound 101)

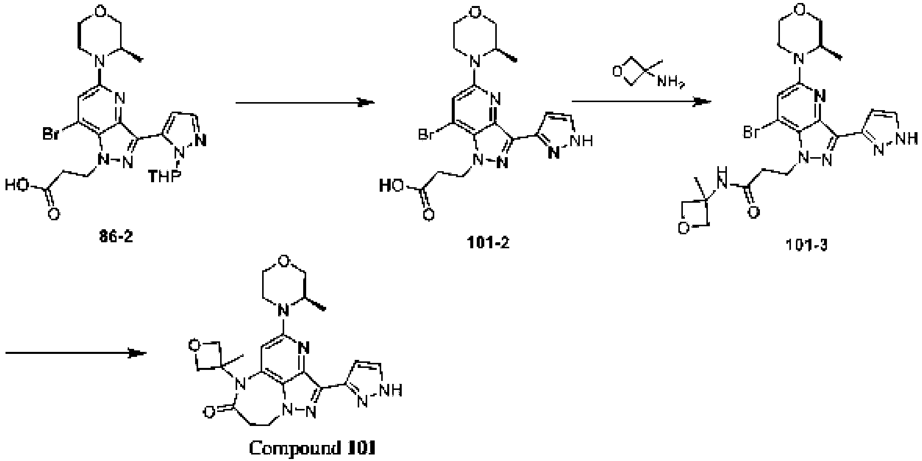

Step 1: Intermediate **86-2** (150 mg, 0.29 mmol) was dissolved in tetrahydrofuran (2 mL), and hydrochloric acid solution (1 mL, 1 M) was added at room temperature. The mixture was stirred at room temperature for half an hour. The reaction mixture was concentrated to yield a crude product, Intermediate **101-2** (110 mg, 87.5%). LCMS (ESI) [M+H]⁺: 435.0, 437.0.

Step 2: Intermediate **101-2** (100 mg, 0.23 mmol) was dissolved in *N,N*-dimethylformamide (5 mL), and diisopropylethylamine (0.19 mL, 1.15 mmol) and *O*-(7-azabenzotriazol-1-yl)-*N,N,N',N*'-tetramethyluronium hexafluorophosphate (174.7 mg, 0.46 mmol) were added in sequence. The reaction mixture was stirred for 10 minutes, and then 3-methyl-3-aminooxetane (40.0 mg, 0.46 mmol) was added. The reaction mixture was stirred for an additional 20 minutes. The reaction was quenched by the addition of ice water, and extracted with ethyl acetate (10 mL × 3). The organic phase was washed sequentially with water and saturated brine, dried over anhydrous sodium sulfate, filtered, and concentrated to yield a crude product. The crude product was separated and purified by silica gel chromatography to yield Intermediate **101-3** (25 mg, 21.6%). LCMS (ESI) [M+H]⁺: 504.3.

Step 3: Intermediate **101-3** (25 mg, 0.05 mmol) was dissolved in 1,4-dioxane (1 mL). Cesium carbonate (48.4 mg, 0.15 mmol), palladium acetate (5.6 mg, 0.025 mmol), and 4,5-bis(diphenylphosphino-9,9-dimethylxanthene) (14.3 mg, 0.025 mmol) were added. The reaction mixture was stirred at 80°C under a nitrogen atmosphere for 1 hour. The mixture was filtered and concentrated under reduced pressure to remove the solvent to yield a crude product. The crude product was separated and purified by preparative reverse-phase high performance liquid chromatography to give (*R*)-4-(3-methylmorpholinyl)-6-(3-methyloxetan-3-yl)-2-(1*H*-pyrazol-3-yl)-8,9-dihydro-1,3,6,9a-tetraazabenzo[*cd*]azulene-7(6*H*)-one (Compound **101)** (0.8 mg, 3.8%). LCMS (ESI) [M+H]⁺: 424.2. ¹H NMR (400 MHz, CD₃OD) δ 7.72 (s, 1H), 7.15 (s, 1H), 6.08 (s, 1H), 4.85 - 4.71 (m, 3H), 4.68 - 4.26 (m, 5H), 4.13 - 4.07 (m, 1H), 3.98 (d, *J* = 12.8 Hz, 1H), 3.87 (s, 2H), 3.77 - 3.66 (m, 1H), 3.18 - 3.05 (m, 2H), 2.11 (s, 3H), 1.30 (d, *J =* 6.4 Hz, 3H).

### Example 102: Preparation of (R)-6-(1-(difluoromethyl)cyclopropyl)-4-(3-methylmorpholinyl)-2-(1H-pyrazol-3-yl)-8,9-dihydro-1,3,6,9a-tetraazabenzo[cd]azulene-7(6H)-one (Compound 102)

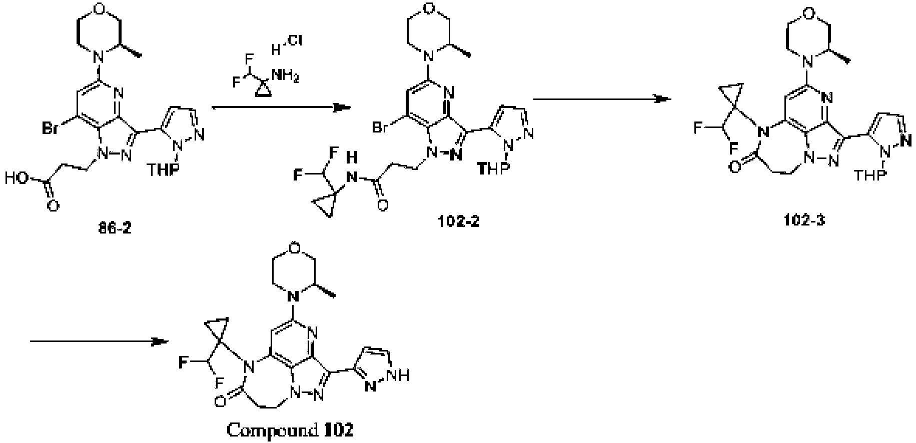

Similar to the preparation method of Example 86, the title Compound **102** was prepared using 1-(difluoromethyl)cyclopropane-1-amine hydrochloride instead of methylamine hydrochloride. (*R*)-6-(1-(Difluoromethyl)cyclopropyl)-4-(3-methylmorpholinyl)-2-(1*H*-pyrazol-3-yl)-8,9-dihydro-1,3,6,9a-tetraazabenzo[*cd*]azulene-7(6*H*)-one (Compound **102)** (12.2 mg, 14.5%) was obtained. LCMS (ESI) [M+H]⁺: 444.4. ¹H NMR (400 MHz, CD₃OD) δ 7.74 - 7.63 (m, 1H), 7.15 - 7.05 (m, 1H), 6.99 (d, *J* = 6.4 Hz, 1H), 6.56 - 6.21 (m, 1H), 4.72 - 4.65 (m, 1H), 4.64 - 4.56 (m, 1H), 4.36 - 4.23 (m, 1H), 4.08 - 3.97 (m, 2H), 3.86 - 3.88 (m, 2H), 3.72 - 3.64 (m, 1H), 3.37 - 3.31 (m, 1H), 3.23 - 3.15 (m, 1H), 3.12 - 3.04 (m, 1H), 1.60 (s, 1H), 1.54 - 1.47 (m, 1H), 1.31 (d, *J =* 6.8 Hz, 2H), 1.26 (d, *J =* 6.8 Hz, 3H).

### Example 103: Preparation of (R)-6-(4-hydroxy-4-methylpent-2-yn-1-yl)-4-(3-methylmorpholinyl)-2-(1H-pyrazol-3-yl)-8,9-dihydro-1,3,6,9a-tetraazabenzo[cd]azulene-7(6H)-one (Compound 103)

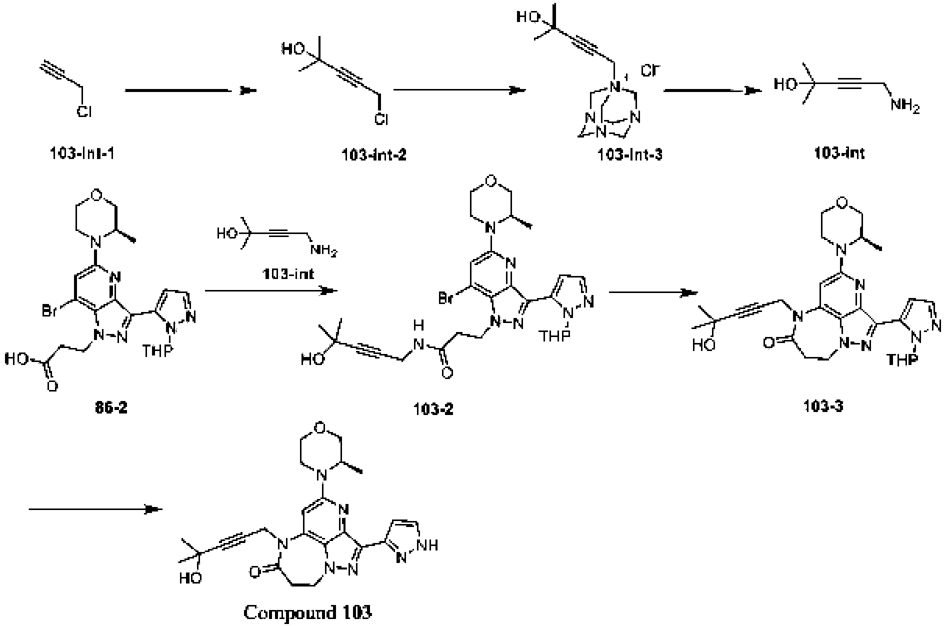

Step 1: 3-Chloropropyne (12 g, 161.1 mmol) was dissolved in tetrahydrofuran (160 mL), and slowly added dropwise with n-butyllithium (67.1 mL, 2.4 M) in n-hexane at -78°C. After the addition was complete, the mixture was stirred for half an hour, and then slowly added dropwise with acetone (11.9 mL, 161.1 mmol). The reaction mixture was stirred at -78°C for 2 hours. The reaction was quenched with saturated ammonium chloride solution (150 mL) in an ice-water bath. The aqueous phase was extracted with ethyl acetate (140 mL × 3). The organic phase was washed sequentially with water and saturated brine, dried over anhydrous sodium sulfate, filtered, and spin-dried to obtain a crude product. The crude product was purified by silica gel column chromatography to yield Intermediate **103-2** (15 g, 70.2%). ¹H NMR (400 MHz, CDCl₃) δ 4.18 (s, 2H), 1.55 (s, 6H).

Step 2: Intermediate **103-2** (15 g, 113.1 mmol) was dissolved in chloroform (500 mL), and hexamethylenetetramine (17.4 g, 124.4 mmol) was added at room temperature. The reaction mixture was heated to reflux and stirred for 18 hours, and filtered to yield Intermediate **103-3** (20 g, 74.5%).

Step 3: Intermediate **103-3** (15 g, 63.2 mmol) was dissolved in ethyl acetate (80 mL), and concentrated hydrochloric acid (8 mL) was added at room temperature. The reaction mixture was heated to reflux, stirred for 18 hours, and filtered to yield a crude product, which was purified by reverse-phase column chromatography (20% methanol in water) to yield Intermediate **103-int** (3.2 g, 44.7%). LCMS (ESI) [M+H]⁺: 114.2.

Step 4: Similar to the preparation method in Example 86, the title Compound **103** was prepared using Intermediate **103-int** instead of methylamine hydrochloride. (*R*)-6-(4-Hydroxy-4-methylpent-2-yn-1-yl)-4-(3-methylmorpholinyl)-2-(1*H*-pyrazol-3-yl)-8,9-dihydro-1,3,6,9a-tetraa zabenzo[cd]azulene-7(6H)-one (Compound **103)** (7.7 mg, 18%) was obtained. LCMS (ESI) [M+H]⁺: 450.4. ¹H NMR (400 MHz, CD₃OD) δ 7.67 (s, 1H), 7.10 (s, 1H), 7.00 (s, 1H), 4.95 - 4.85 (m, 2H), 4.70 - 4.63 (m, 2H), 4.47 (d, *J =* 6.8 Hz, 1H), 4.09 - 4.01 (m, 2H), 3.85 (d, *J* = 2.0 Hz, 2H), 3.69 - 3.67 (m, 1H), 3.40 - 3.33 (m, 1H), 3.19 - 3.12 (m, 2H), 1.42 (s, 6H), 1.31 (d, *J* = 6.8 Hz, 3H).

### Example 104: Preparation of 2-(4-((R)-3-methylmorpholino)-7-oxo-2-(1H-pyrazol-3-yl)-8,9-dihydro-1,3,6,9a-tetraazabenzo[cd]azulene-6(7H)-yl)propanamide (Compound 104)

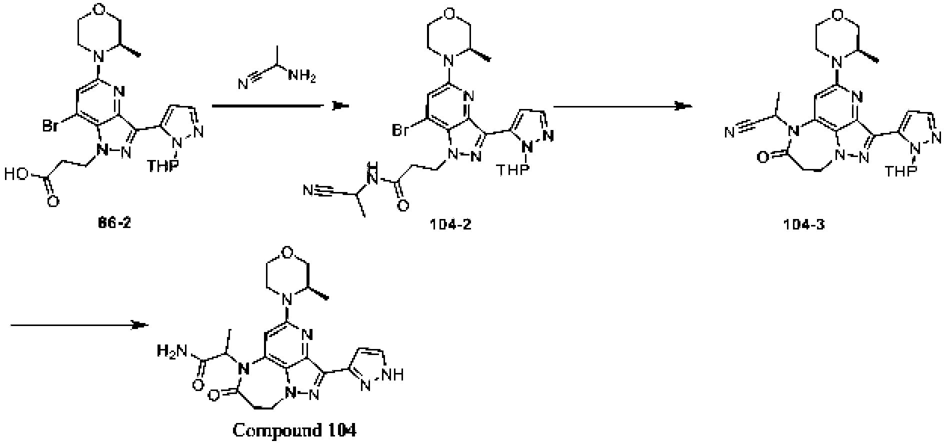

Similar to the preparation method of Example 86, the title Compound **104** was prepared using 2-aminopropionitrile instead of methylamine hydrochloride. 2-(4-((*R*)-3-Methylmorpholino)-7-oxo-2-(1*H*-pyrazol-3-yl)-8,9-dihydro-1,3,6,9a-tetraazabenzo[c d]azulene-6(7H)-yl)propanamide (Compound 104) (4.0 mg, 10%) was obtained. LCMS (ESI) [M+H]⁺: 425.4. ¹H NMR (400 MHz, CD₃OD) δ 7.67 (s, 1H), 7.10 (s, 1H), 6.69 (d, *J* = 2.4 Hz, 1H), 5.53 - 5.45 (m, 1H), 4.75 - 4.66 (m, 2H), 4.40 - 4.30 (m, 1H), 4.05 - 4.03 (m, 1H), 3.97 (t, *J* = 13.6 Hz, 1H), 3.83 (d, *J =* 2.0 Hz, 2H), 3.69 - 3.62 (m, 1H), 3.36 - 3.34 (m, 1H), 3.16 - 3.11 (m, 2H), 1.57 (t, *J* = 7.2 Hz, 3H), 1.28 - 1.26 (m, 3H).

### Example 105: Preparation of (R)-1-(4-(3-methylmorpholinyl)-7-oxo-2-(1H-pyrazol-3-yl)-8,9-dihydro-1,3,6,9a-tetraazabenzo[cd]azulene-6(7H)-yl)cyclopropane-1-carbonitrile (Compound 105)

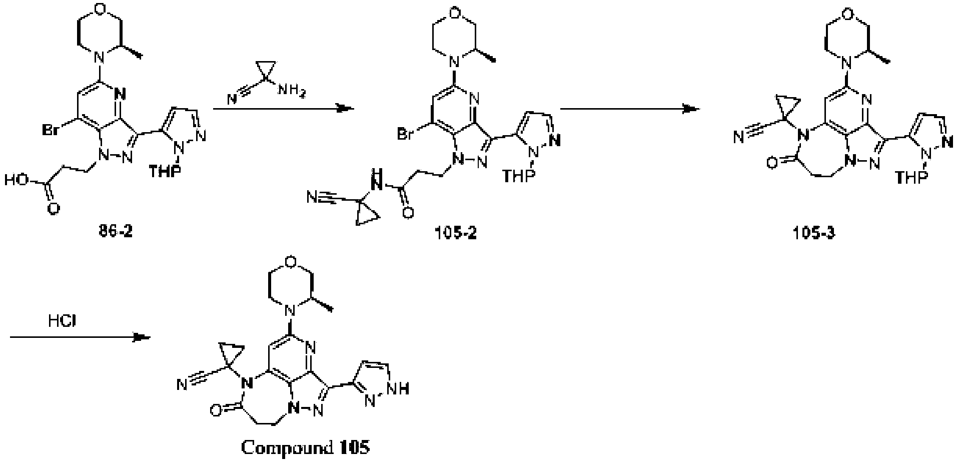

Similarly to the preparation method of Example 86, the Compound 105 was prepared using 1-amino-1-cyclopropylcarbonitrile hydrochloride instead of methylamine hydrochloride. (*R*)-1-(4-(3-methylmorpholinyl)-7-oxo-2-(1*H*-pyrazol-3-yl)-8,9-dihydro-1,3,6,9a-tetraazabenzo| *cd*]azulene-6(7*H*)-yl)cyclopropane-1-carbonitrile (Compound **105)** (14.0 mg, 34%) is obtained. LCMS (ESI) [M+H]⁺: 419.4. ¹H NMR (400 MHz, CD₃OD) δ 7.67 (s, 1H), 7.10 (s, 1H), 6.83 (s, 1H), 4.69 - 4.61 (m, 2H), 4.41 (d, *J =* 4.4 Hz, 1H), 4.12 - 4.02 (m, 2H), 3.87 (d, *J =* 2.4 Hz, 2H), 3.72 - 3.70 (m, 1H), 3.40 - 3.33 (m, 1H), 3.23 (s, 2H), 1.97 (d, *J =* 30.8 Hz, 2H), 1.67 (s, 1H), 1.32 (d, *J =* 6.8 Hz, 3H), 1.24 (s, 1H).

### Example 106: Preparation of 6-(but-3-en-2-yl)-4-((R)-3-methylmorpholinyl)-2-(1H-pyrazol-3-yl)-8,9-dihydro-1,3,6,9a-tetraazabenzo[cd]azulene-7(6H)-one (Compound 106)

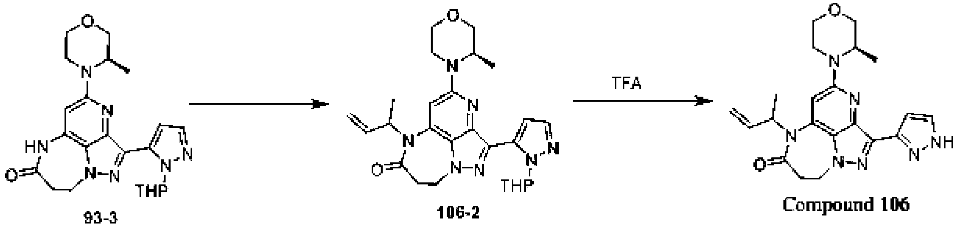

Step 1: Intermediate **93-3** (100 mg, 0.23 mmol) and cesium carbonate (148.9 mg, 0.46 mmol) were dissolved in *N*,*N*-dimethylformamide (1 mL), and 3-chloro-1-butene (41.4 mg, 0.46 mmol) was added at room temperature. The reaction mixture was warmed to 60°C and stirred for half an hour. The reaction mixture was cooled to room temperature and quenched with ice water (10 mL). The aqueous phase was extracted with ethyl acetate (20 mL × 3). The combined organic phases were washed sequentially with water and saturated brine, dried over anhydrous sodium sulfate, filtered, and spin-dried to obtain the crude product. The crude product was purified by silica gel column chromatography to yield Intermediate **106-2** (100 mg, 89%). LCMS (ESI) [M+H]⁺: 492.4.

Step 2: Intermediate **106-2** (100 mg, 0.20 mmol) was dissolved in dichloromethane (1 mL), and trifluoroacetic acid (0.2 mL) was added. The reaction mixture was stirred at room temperature for 1 hour. The reaction mixture was concentrated to obtain a crude product. The crude product was separated and purified by preparative reverse-phase high performance liquid chromatography to give 6-(but-3-en-2-yl)-4-((*R*)-3-methylmorpholinyl)-2-(1*H*-pyrazol-3-yl)-8,9-dihydro-1,3,6,9a-tetraazabenzo[*cd*]azulene-7(6*H*)-one (Compound **106)** (50 mg, 60.4%). LCMS (ESI) [M+H]⁺: 408.2. ¹H NMR (400 MHz, DMSO-d6) δ 7.68 (d, *J =* 1.8 Hz, 1H), 7.03 (s, 1H), 6.70 - 6.64 (m, 1H), 6.20 - 6.03 (m, 1H), 5.55 (s, 1H), 5.31 - 5.20 (m, 2H), 4.65 - 4.62 (m, 2H), 4.21 (s, 2H), 4.00 (d, *J =* 11.2 Hz, 1H), 3.93 (d, *J =* 11.4 Hz, 1H), 3.77 (d, *J =* 13.2 Hz, 1H), 3.73 - 3.66 (m, 1H), 3.53 (t, *J =* 11.2 Hz, 1H), 3.21 - 3.09 (m, 1H), 3.06 (s, 2H), 1.50 - 1.45 (m, 3H), 1.19 - 1.14 (m, 3H).

### Example 107: Preparation of (R)-6-(3-(methoxymethyl)bicyclo[1.1.1]pentan-1-yl)-4-(3-methylmorpholinyl)-2-(1H-pyrazol-3-yl)-8,9-dihydro-1,3,6,9a-tetraazabenzo[cd]azulene-7(6H)-one (Compound 107)

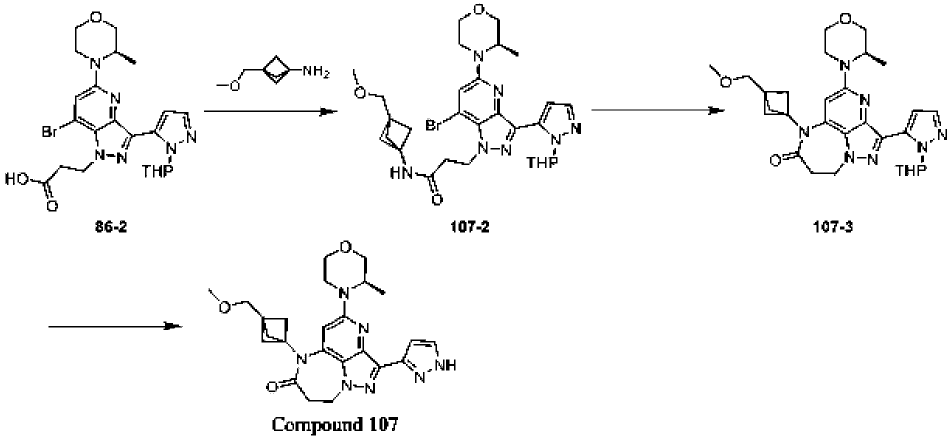

Similar to the preparation method of Example 86, the title Compound **107** was prepared using 3-(methoxymethyl)bicyclo[1.1.1]pentan-1-amine hydrochloride instead of methylamine hydrochloride. (*R*)-6-(3-(Methoxymethyl)bicyclo[1.1.1]pentan-1-yl)-4-(3-methylmorpholinyl)-2-(1*H*-pyrazol-3-yl)-8,9-dihydro-1,3,6,9a-tetraazabenzo[*cd*]azulene-7(6*H*)-one (Compound **107)** (6.7 mg, 16%) was obtained. LCMS (ESI) [M+H]⁺: 464.4. ¹H NMR (400 MHz, CD₃OD) δ 7.68 (s, 1H), 7.12 (s, 1H), 6.85 (s, 1H), 4.72 - 4.69 (m, 2H), 4.49 (s, 1H), 4.12 (d, *J* = 8.8 Hz, 1H), 4.02 (s, 1H), 3.91 (s, 2H), 3.73 (d, *J =* 11.6 Hz, 1H), 3.60 (s, 2H), 3.42 (s, 4H), 3.04 (s, 2H), 2.38 (s, 6H), 1.35 (d, *J =* 6.6 Hz, 3H).

### Example 108: Preparation of (R)-6-([1,1'-bis(cyclopropane)]-1-yl)-4-(3-methylmorpholinyl)-2-(1H-pyrazol-3-yl)-8,9-dihydro-1,3,6,9a-tetraazabenzo[cd]azulene-7(6H)-one (Compound 108)

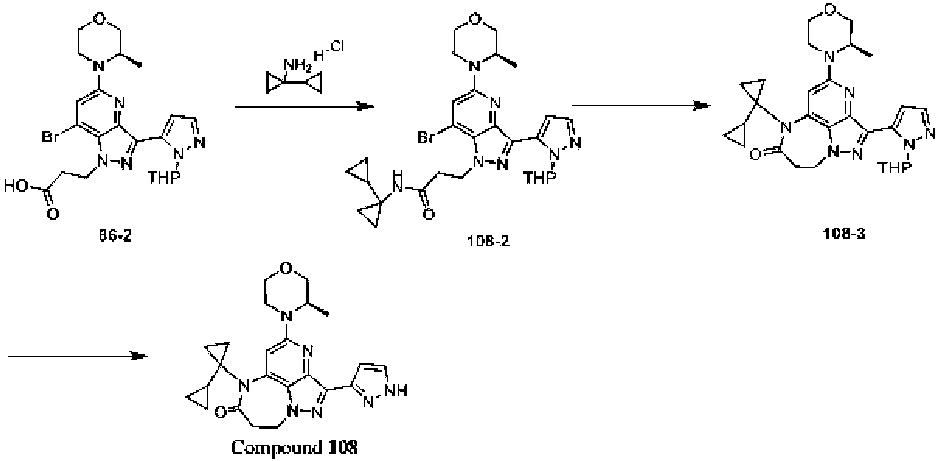

Similar to the preparation method of Example 86, the title Compound **108** was prepared using [1,1'-bis(cyclopropane)]-1-amine hydrochloride instead of methylamine hydrochloride. (*R*)-6-([1,1'-bis(cyclopropane)]-1-yl)-4-(3-methylmorpholinyl)-2-(1*H*-pyrazol-3-yl)-8,9-dihydro-1,3,6,9a-tetraazabenzo[*cd*]azulene-7(6*H*)-one (Compound **108)** (3.0 mg, 17.9%) was obtained. LCMS (ESI) [M+H]⁺: 434.2. ¹H NMR (400 MHz, CD₃OD) δ 7.73 - 7.60 (m, 1H), 7.20 - 7.00 (m, 1H), 7.03 (s, 1H), 4.61 - 4.59 (m, 2H), 4.34 (s, 1H), 4.06 - 4.04 (m, 2H), 3.86 (s, 2H), 3.74 - 3.65 (m, 1H), 3.37 - 3.30 (m, 1H), 3.10 - 3.08 (m, 2H), 1.68 - 1.54 (m, 1H), 1.35 - 1.26 (m, 3H), 1.22 - 1.03 (m, 2H), 0.89 - 0.80 (m, 1H), 0.61 - 0.39 (m, 5H).

### Example 109: Preparation of (R)-6-(3,3-difluorocyclobutyl)-4-(3-methylmorpholinyl)-2-(1H-pyrazol-3-yl)-8,9-dihydro-1,3,6,9a-tetraazabenzo[cd]azulene-7(6H)-one (Compound 109)

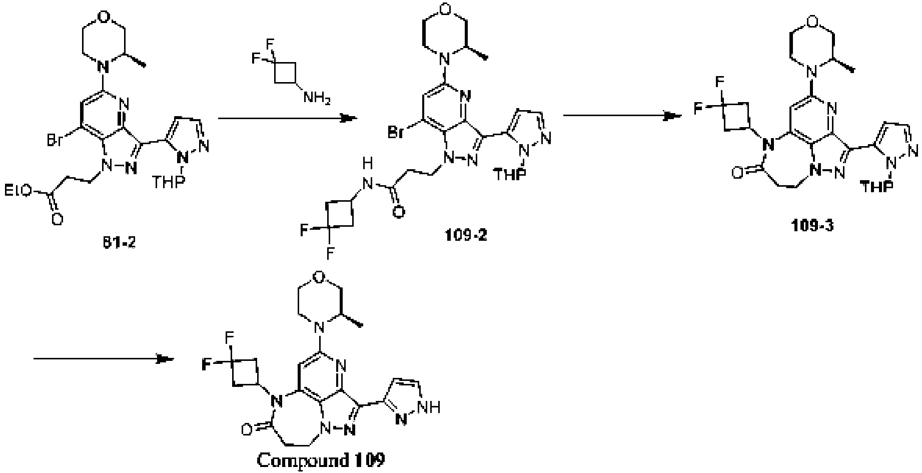

Similarly to the preparation method of Example 81, the title Compound 109 was prepared using 3,3-difluorocyclobutylamine instead of isopropylamine. (*R*)-6-(3,3-Difluorocyclobutyl)-4-(3-methylmorpholinyl)-2-(1*H*-pyrazol-3-yl)-8,9-dihydro-1,3,6,9a-tetraazabenzo[cd]azulene-7(6 *H*)-one (Compound **109)** (11 mg, 19%) was obtained. LCMS (ESI) [M+H]⁺: 444.4. ¹H NMR (400 MHz, DMSO-d6) δ 13.14 (s, 1H), 7.67 (s, 1H), 7.03 (s, 1H), 6.48 (s, 1H), 4.76 - 4.60 (m, 2H), 4.57 - 4.43 (m, 2H), 4.07 - 3.96 (m, 2H), 3.77 (d, *J* = 11.2 Hz, 1H), 3.71 - 3.69 (m, 1H), 3.55 - 3.53 (m, 1H), 3.17- 3.15 (m, 3H), 3.02 (t, *J =* 5.2 Hz, 2H), 2.63 (d, *J =* 12.8 Hz, 2H), 1.17 (d, *J* = 6.8 Hz, 3H).

### Example 110: Preparation of (R)-6-(bicyclo[1.1.1]pentan-1-yl)-4-(3-methylmorpholinyl)-2-(1H-pyrazol-3-yl)-8,9-dihydro-1,3,6,9a-tetraazabenzo[cd]azulene-7(6H)-one (Compound 110)

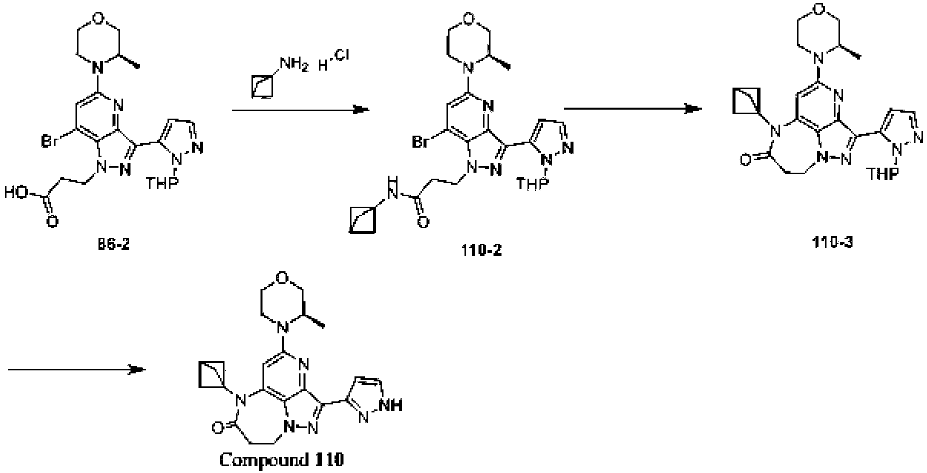

Similarly to the preparation method of Example 86, the title Compound **110** was prepared using 1-bicyclo[1.1.1]pentanamine hydrochloride instead of methylamine hydrochloride. (*R*)-6-(Bicyclo[1.1.1]pentan-1-yl)-4-(3-methylmorpholinyl)-2-(1*H*-pyrazol-3-yl)-8,9-dihydro-1,3 ,6,9a-tetraazabenzo[*cd*]azulene-7(6*H*)-one (Compound **110)** (4.8 mg, 5.8%) was obtained. LCMS (ESI) [M+H]⁺: 420.4. ¹H NMR (400 MHz, CD₃OD) δ 7.67 (s, 1H), 7.10 (s, 1H), 6.81 (s, 1H), 4.68 - 4.59 (m, 2H), 4.46 - 4.37 (m, 1H), 4.09 - 4.02 (m, 1H), 3.98 (d, *J* = 13.2 Hz, 1H), 3.84 (d, *J* = 2.0 Hz, 2H), 3.73 - 3.63 (m, 1H), 3.36 (d, *J* = 9.2 Hz, 1H), 3.00 - 2.93 (m, 2H), 2.57 (s, 1H), 2.40 (s, 6H), 1.29 (d, *J =* 6.8 Hz, 3H).

### Example 111A: Preparation of 4-((R)-3-methylmorpholino)-2-(1H-pyrazol-3-yl)-6-((S)-4,4,4-trifluorobutan-2-yl)-8,9-dihydro-1,3,6,9a-tetraazabenzo[cd]azulene-7(6H)-one (Compound 111A)

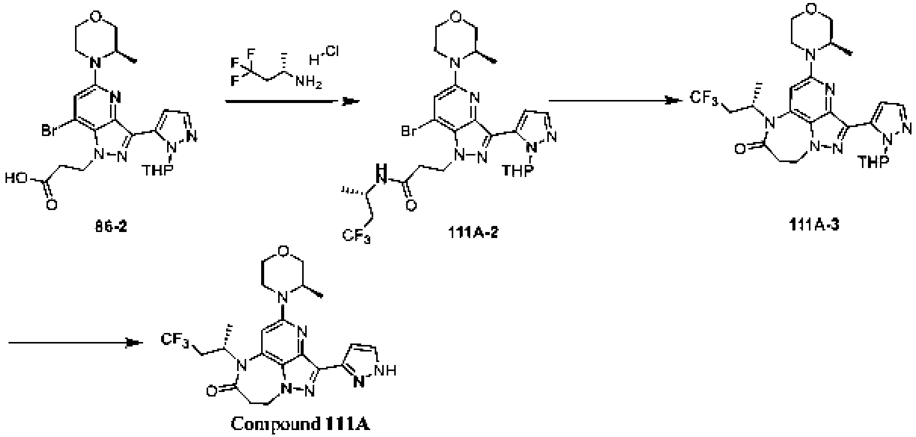

Similarly to the preparation method of Example 86, the title Compound **111A** was prepared using (*S*)-4,4,4-trifluorobutan-2-amine hydrochloride instead of methylamine hydrochloride. 4-((*R*)-3-methylmorpholino)-2-(1*H-*pyrazol-3-yl)-6-((*S*)-4,4,4-trifluorobutan-2-yl)-8,9-dihydro-1 ,3,6,9a-tetraazabenzo[*cd*]azulene-7(6*H*)-one (Compound **111A)** (8.4 mg, 33.1%) was obtained. LCMS (ESI) [M+H]⁺: 464.2. ¹H NMR (400 MHz, DMSO-d6) δ 13.11 - 13.09 (m, 1H), 7.71 - 7.69 (m, 1H), 7.06 - 7.04 (m, 1H), 6.73 - 6.71 (m, 1H), 4.72 - 4.39 (m, 4H), 4.02 - 4.00 (m, 2H), 3.76 - 3.74 (m, 2H), 3.57 - 3.55 (m, 1H), 3.19 - 3.17 (m, 1H), 2.96 - 2.94 (m, 2H), 2.75 - 2.73 (m, 1H), 1.64 (d, *J =* 5.2 Hz, 3H), 1.17 (d, *J =* 5.2 Hz, 3H).

### Example 111B: Preparation of 4-((R)-3-methylmorpholino)-2-(1H-pyrazol-3-yl)-6-((R)-4,4,4-trifluorobutan-2-yl)-8,9-dihydro-1,3,6,9a-tetraazabenzo[cd]azulene-7(6H)-one (Compound 111B)

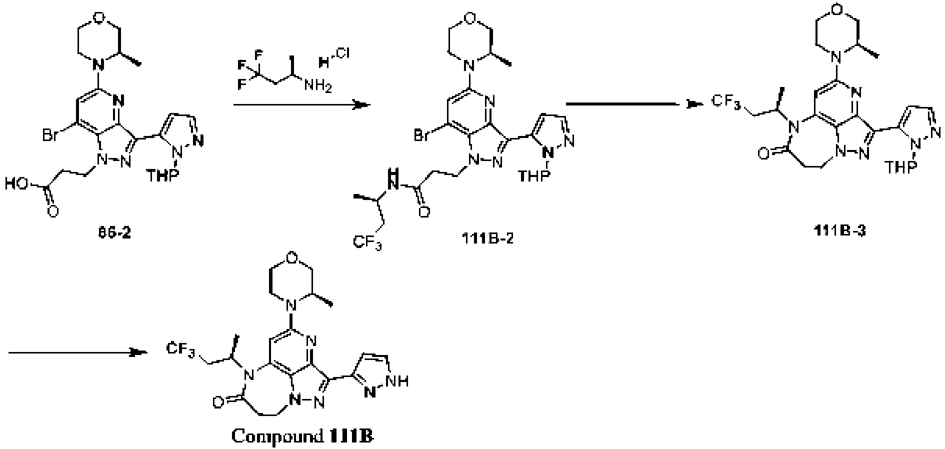

Similarly to the preparation method of Example 86, the title Compound **111B** was prepared using (*R*)-4,4,4-trifluorobutan-2-amine hydrochloride instead of methylamine hydrochloride. 4-((*R*)-3-methylmorpholino)-2-(1*H*-pyrazol-3-yl)-6-((*R*)-4,4,4-trifluorobutan-2-yl)-8,9-dihydro-1 ,3,6,9a-tetraazabenzo[*cd*]azulene-7(6*H*)-one (Compound **111B)** (10.9 mg, 21.8%) was obtained. LCMS (ESI) [M+H]⁺: 464.2. ¹H NMR (400 MHz, DMSO-d6) δ 13.35 - 12.87 (m, 1H), 7.89 - 7.47 (m, 1H), 7.16 - 6.94 (m, 1H), 6.81 - 6.68 (m, 1H), 4.75 - 4.38 (m, 4H), 4.10 - 3.92 (m, 2H), 3.84 - 3.67 (m, 2H), 3.61 - 3.51 (m, 1H), 3.42 - 3.34 (m, 1H),3.23 - 3.13 (m, 1H), 3.03 - 2.89 (m, 2H), 2.78 - 2.66 (m, 1H), 1.65 (d, *J =* 6.6 Hz, 3H), 1.17 (d, *J =* 6.4 Hz, 3H).

### Example 112: Preparation of (R)-6-(1-(fluoromethyl)cyclopropyl)-4-(3-methylmorpholinyl)-2-(1H-pyrazol-3-yl)-8,9-dihydro-1,3,6,9a-tetraazabenzo[cd]azulene-7(6H)-one (Compound 112)

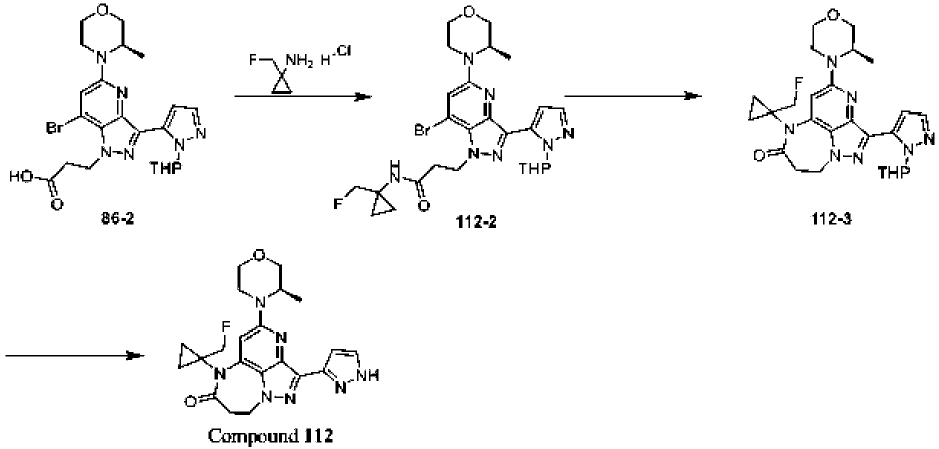

Similarly to the preparation method of Example 86, the title Compound **112** was prepared using 1-(fluoromethyl)cyclopropylamine hydrochloride instead of methylamine hydrochloride. (*R*)-6-(1-(Fluoromethyl)cyclopropyl)-4-(3-methylmorpholinyl)-2-(1*H*-pyrazol-3-yl)-8,9-dihydro-1,3,6,9a-tetraazabenzo[cd]azulene-7(6H)-one (Compound **112)** (52 mg, 62.3%) was obtained. LCMS (ESI) [M+H]⁺: 426.4. ¹H NMR (400 MHz, DMSO-d6) δ 13.23 - 12.92 (m, 1H), 7.81 - 7.51(s, 1H), 7.10 - 6.95 (m, 2H), 5.32 - 5.06 (m, 1H), 4.71 - 4.49 (m, 2H), 4.37 - 4.17 (m, 2H), 4.04 - 4.00 (m, 2H), 3.85 - 3.69 (m, 2H), 3.62 - 3.52 (m, 1H), 3.22 - 3.03 (m, 3H), 1.43 - 1.24 (m, 3H), 1.23 - 1.15 (m, 3H), 0.68 - 0.65 (m, 1H).

### Example 113: Preparation of (R)-6-(1-(methoxymethyl)cyclopropyl)-4-(3-methylmorpholinyl)-2-(1H-pyrazol-3-yl)-8,9-dihydro-1,3,6,9a-tetraazabenzo[cd]azulene-7(6H)-one (Compound 113)

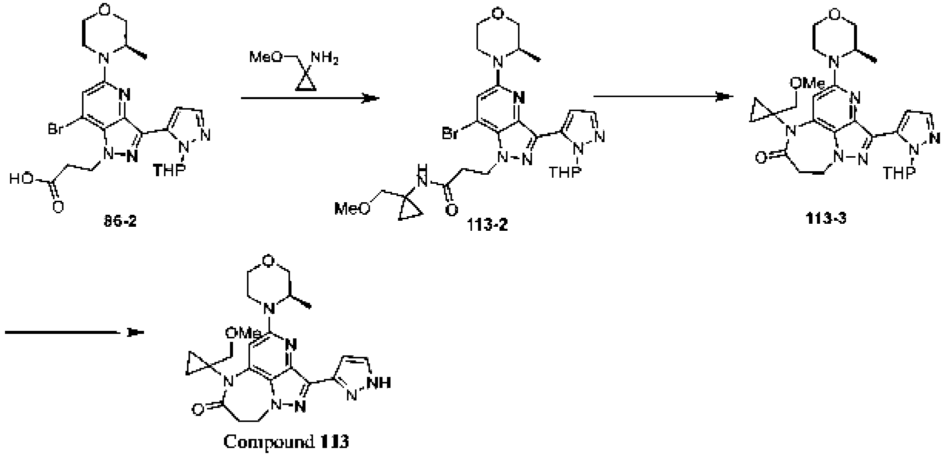

Similarly to the preparation method of Example 86, the title Compound **113** was prepared using 1-(methoxymethyl)cyclopropane-1-amine instead of methylamine hydrochloride. (*R*)-6-(1-(Methoxymethyl)cyclopropyl)-4-(3-methylmorpholinyl)-2-(1*H*-pyrazol-3-yl)-8,9-dihyd ro-1,3,6,9a-tetraazabenzo[*cd*]azulene-7(6*H*)-one (Compound **113)** (5.7 mg, 27.2%) was obtained. LCMS (ESI) [M+H]⁺: 438.0. ¹H NMR (400 MHz, CD₃OD) δ 7.74 - 7.61 (m, 1H), 7.18 - 7.02 (m, 1H), 4.69 - 4.63 (m, 1H), 4.58 - 4.56 (m, 1H), 4.30 - 4.25 (m, 2H), 4.10 - 4.08 (m, 2H), 3.85 (s, 2H), 3.70 - 3.68 (m, 1H), 3.41 (d, *J =* 2.4 Hz, 3H), 3.39 - 3.30 (m, 1H), 3.13 - 3.11 (m, 2H), 3.04 - 3.02 (m, 1H), 1.30 - 1.29 (m, 4H), 1.14 - 1.12 (m, 2H), 0.65 - 0.56 (m, 1H).

### Example 114: Preparation of (R)-6-(1-(hydroxymethyl)cyclopropyl)-4-(3-methylmorpholinyl)-2-(1H-pyrazol-3-yl)-8,9-dihydro-1,3,6,9a-tetraazabenzo[cd]azulene-7(6H)-one (Compound 114)

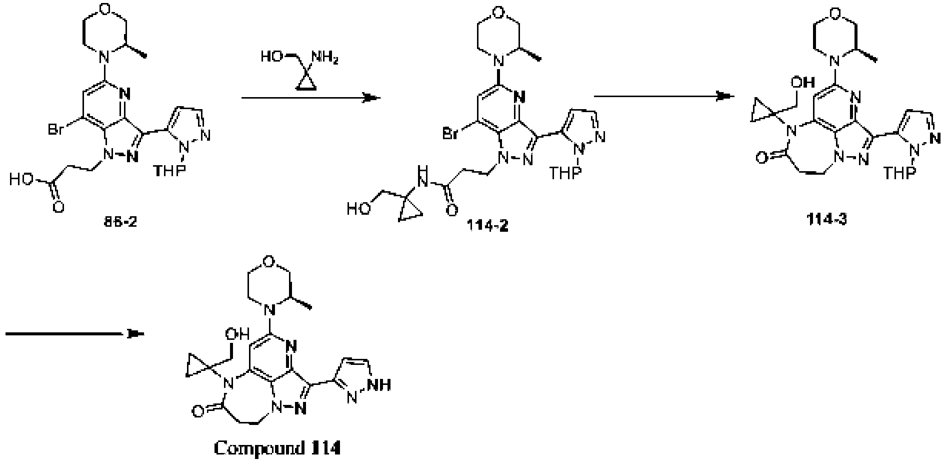

Similarly to the preparation method of Example 86, the title Compound **114** was prepared using 1-aminocyclopropylmethanol instead of methylamine hydrochloride. (*R*)-6-(1-(Hydroxymethyl)cyclopropyl)-4-(3-methylmorpholinyl)-2-(1*H*-pyrazol-3-yl)-8,9-dihydr o-1,3,6,9a-tetraazabenzo[*cd*]azulene-7(6*H*)-one (Compound **114)** (5.6 mg, 19.2%) was obtained. LCMS (ESI) [M+H]⁺: 423.9. ¹H NMR (400 MHz, DMSO-d6) δ 13.20 (s, 1H), 7.60 (s, 1H), 7.50 (s, 1H), 7.03 (s, 1H), 5.33 (t, *J =* 5.8 Hz, 1H), 4.70 - 4.48 (m, 2H), 4.33 - 4.29 (m, 2H), 4.02 (d, *J* = 10.8 Hz, 2H), 3.82 - 3.78 (m, 1H), 3.77 - 3.69 (m, 1H), 3.60 - 3.54 (m, 1H), 3.21 - 3.10 (m, 2H), 3.08 - 2.92 (m, 2H), 1.20 (dd, *J* = 19.6, 6.6 Hz, 3H), 1.20 - 1.14 (m, 1H), 1.10 - 0.99 (m, 2H), 0.48 - 0.34 (m, 1H).

### Example 115: Preparation of (R)-6-((3-fluorobicyclo[1.1.1]pentan-1-yl)methyl)-4-(3-methylmorpholinyl)-2-(1H-pyrazol-3-yl)-8,9-dihydro-1,3,6,9a-tetraazabenzo[cd]azulene-7(6H)-one (Compound 115)

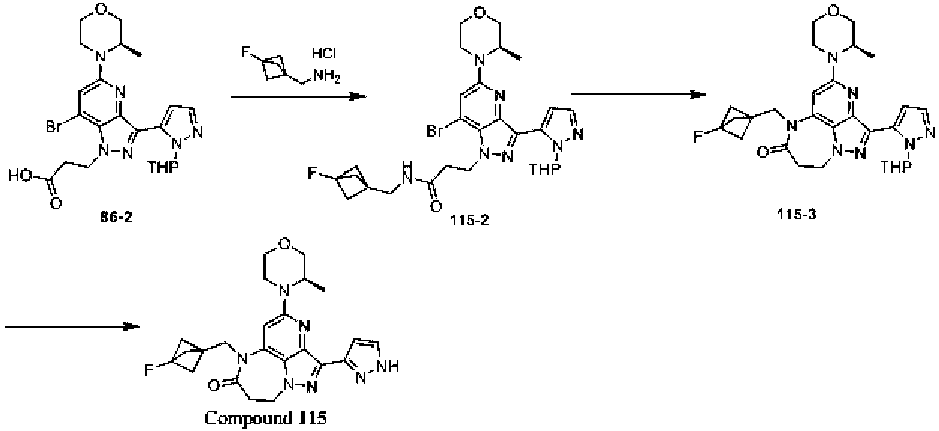

Similar to the preparation method of Example 86, the title Compound **115** was prepared using (3-fluorobicyclo[1.1.1]pentan-1-yl)methanamine hydrochloride instead of methylamine hydrochloride. (*R*)-6-((3-Fluorobicyclo[1.1.1]pentan-1-yl)methyl)-4-(3-methylmorpholinyl)-2-(1*H*-pyrazol-3-yl)-8,9-dihydro-1,3,6,9a-tetraazabenzo[*cd*]azulene-7(6*H*)-one (Compound **115)** (43.1 mg, 73.0%) was obtained. LCMS (ESI) [M+H]⁺: 452.2. ¹H NMR (400 MHz, DMSO-d6) δ 13.09 (m, 1H), 7.69 (m, 1H), 7.04 (s, 1H), 6.68 (s, 1H), 4.72 - 4.58 (m, 2H), 4.52 - 4.35 (m, 3H), 4.07 - 3.93 (m, 2H), 3.83 - 3.68 (m, 2H), 3.60 - 3.51 (m, 1H), 3.20 - 3.12 (m, 1H), 3.11 - 2.97 (m, 2H), 1.97 (d, *J =* 2.0 Hz, 6H), 1.17 (d, *J =* 6.4 Hz, 3H).

### Example 116: Preparation of (R)-1-((4-(3-methylmorpholinyl)-7-oxo-2-(1H-pyrazol-3-yl)-8,9-dihydro-1,3,6,9a-tetraazabenzo[cd]azulene-6(7H)-yl)methyl)cyclopropane-1-carbonitrile (Compound 116)

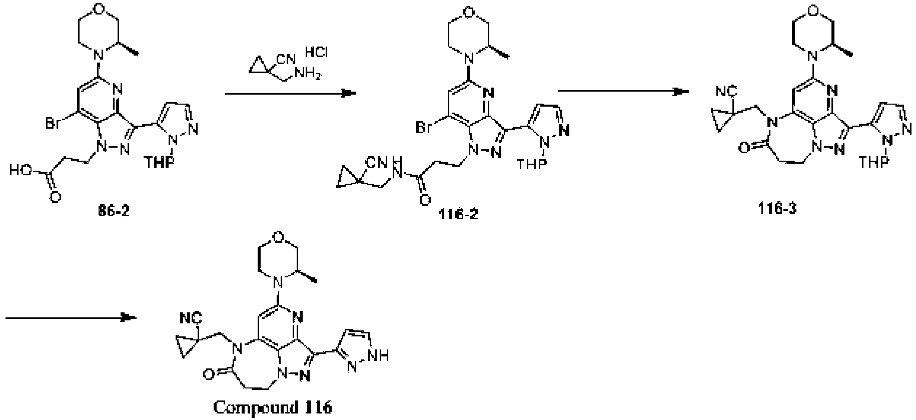

Similarly to the preparation method of Example 86, the title Compound **116** was prepared using 1-(aminomethyl)cyclopropylcarbonitrile hydrochloride instead of methylamine hydrochloride. (*R*)-1-((4-(3-Methylmorpholinyl)-7-oxo-2-(1*H*-pyrazol-3-yl)-8,9-dihydro-1,3,6,9a-tetraazabenzo[*cd*]azulen-6(7H)-yl)methyl)cyclopropane-1-carbonitrile (Compound **116)** (14.3 mg, 24.4%) was obtained. LCMS (ESI) [M+H]⁺: 433.2. ¹H NMR (400 MHz, DMSO-d6) δ 13.38 - 12.82 (m, 1H), 7.90 - 7.47 (m, 1H), 7.25 - 6.88 (m, 2H), 4.63 (d, *J =* 7.6 Hz, 2H), 4.51 (brs, 1H), 4.39 (s, 2H), 4.13 - 3.97 (m, 2H), 3.78 (d, *J =* 11.2 Hz, 1H), 3.74 - 3.65 (m, 1H), 3.62 - 3.48 (m, 1H), 3.24 - 3.14 (m, 1H), 3.10 (brs, 2H), 1.31 - 1.22 (m, 2H), 1.19 (d, *J =* 6.8 Hz, 3H), 1.17 - 1.13 (m, 2H).

### Example 119: Preparation of 6-isopropyl-8-methyl-4-((R)-3-methylmorpholino)-2-(1H-pyrazol-3-yl)-8,9-dihydro-1,3,6,9a-tetraazabenzo[cd]azulene-7(6H)-one (Compound 119)

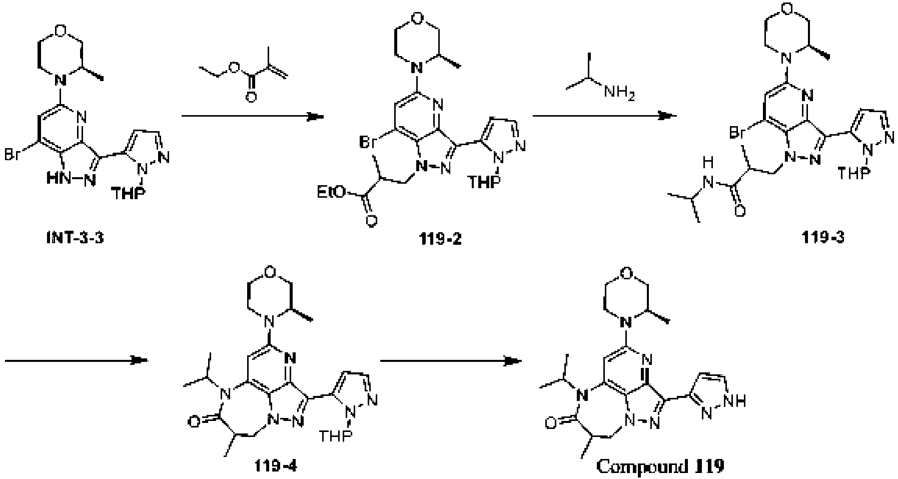

Step 1: Under a nitrogen atmosphere, **INT-3-3** (60 mg, 0.134 mmol) was dissolved in acetonitrile (1 mL), and ethyl methacrylate (46.0 mg, 0.402 mmol) and 1,8-diazabicyclo[5.4.0]undec-7-ene (20.4 mg, 0.134 mmol) were added in sequence. The reaction mixture was stirred at 80°C for 12 hours. The reaction mixture was cooled to room temperature and quenched with ice-cold water (20 mL). The mixture was extracted with ethyl acetate (10 mL × 3). The combined organic phases were washed sequentially with water and saturated brine, dried over anhydrous sodium sulfate, filtered, and concentrated to yield a crude product, which was purified by silica gel column chromatography to yield Intermediate **119-2** (30 mg, 0.053 mmol). LCMS (ESI) [M+H]⁺: 561.0.

Step 2: Similarly to the preparation method of Example 81, the title Compound **119** was prepared using Intermediate **119-2** instead of Intermediate **81-3.** 6-Isopropyl-8-methyl-4-((*R*)-3-methylmorpholino)-2-(1*H*-pyrazol-3-yl)-8,9-dihydro-1,3,6,9a-tetraazabenzo[cd]azulene -7(6H)-one (Compound **119)** (71.0 mg, 62.7%) was obtained. LCMS (ESI) [M+H]⁺: 410.4. ¹H NMR (400 MHz, CDCl₃) δ 7.74 (s, 1H), 6.94 (s, 1H), 6.64 (s, 1H), 4.92 - 4.81 (m, 1H), 4.50 (d, *J* = 8.8 Hz, 2H), 4.33 (d, *J =* 6.4 Hz, 1H), 4.16 (d, *J =* 8.8 Hz, 1H), 3.92 (d, *J =* 8.0 Hz, 2H), 3.79 - 3.72 (m, 2H), 3.46 - 3.37 (m, 1H), 2.99 (d, *J =* 4.0 Hz, 1H), 1.63 - 1.60 (m, 3H), 1.56 - 1.51 (m, 3H), 1.38 - 1.32 (m, 6H).

### Example 120: Preparation of (R)-4-(6-isopropyl-2-(1H-pyrazol-3-yl)-6,7,8,9-tetrahydro-1,3,6,9a-tetraazabenzo[cd]azulen-4-yl)-3-methylmorpholine (Compound 120)

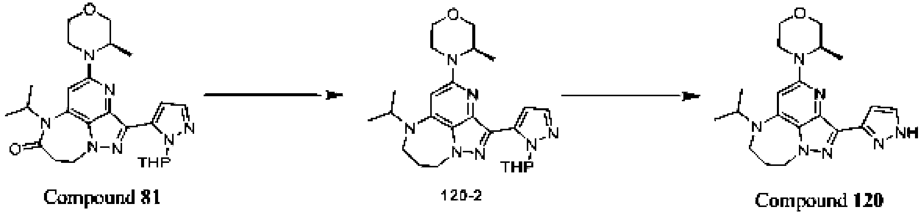

Step 1: Under a nitrogen atmosphere, Intermediate **81-4** (160.0 mg, 0.33 mmol) was dissolved in tetrahydrofuran (5 mL), and then a solution of borane in tetrahydrofuran (3.3 mL, 1 M) was added under ice-cooling. The reaction mixture was stirred at 50°C for 12 hours. The mixture was cooled to room temperature. Saturated brine (15 mL) was added to the mixture, and the aqueous phase was extracted with dichloromethane (15 mL × 3). The organic phase was dried over anhydrous sodium sulfate, filtered, and concentrated to yield a crude product, which was purified by silica gel column chromatography to yield Intermediate **120-2** (100.0 mg, 64.4%). LCMS (ESI) [M+H]⁺: 466.2.

Step 2: Intermediate **120-2** (100.0 mg, 0.21 mmol) was dissolved in tetrahydrofuran (2 mL), and then dilute hydrochloric acid (1 mL, 2 M) was added. The reaction mixture was stirred at room temperature for half an hour. After the reaction was completed, saturated sodium bicarbonate solution (10 mL) was added to the reaction mixture, and the aqueous phase was extracted with dichloromethane (15 mL × 3). The organic phase was dried over anhydrous sodium sulfate, filtered, and concentrated to yield a crude product, which was purified by reverse-phase high performance liquid chromatography to give (*R*)-4-(6-isopropyl-2-(1*H-*pyrazol-3-yl)-6,7,8,9-tetrahydro-1,3,6,9a-tetraazabenzo[cd]azulen-4-yl)-3-methylmorpholine (Compound **120)** (35.0 mg, 42.7%). LCMS (ESI) [M+H]⁺: 382.3. ¹H NMR (400 MHz, DMSO-d6) δ 12.95 (s, 1H), 7.61 (s, 1H), 7.01 (s, 1H), 6.05 (s, 1H), 4.43 - 4.33 (m, 3H), 4.29 (dt, *J =* 12.7, 6.4 Hz, 1H), 3.99 - 3.85 (m, 2H), 3.76 (m, 2H), 3.52 (td, *J =* 11.6, 2.5 Hz, 1H), 3.40 - 3.35 (m, 2H), 3.11 (td, *J =* 12.3, 2.7 Hz, 1H), 2.22 - 2.14 (m, 2H), 1.23 (d, *J =* 6.4 Hz, 6H), 1.13 (d, *J* = 6.6 Hz, 3H).

### Example 121: Preparation of (R)-3-methyl-4-(6-(methylsulfonyl)-2-(1H-pyrazol-3-yl)-6,7,8,9-tetrahydro-1,3,6,9a-tetraazabenzo[cd]azulen-4-yl)morpholine (Compound 121)

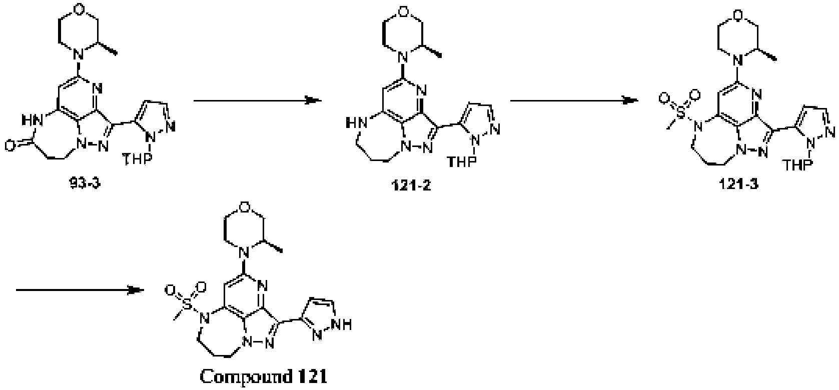

Step 1: Intermediate **93-3** (200.0 mg, 0.46 mmol) was dissolved in tetrahydrofuran (2 mL), and borane in tetrahydrofuran (0.5 mL, 1 M) was added. The reaction mixture was stirred at room temperature for 1 hour. Methanol (5 mL) was added, the mixture was stirred continuously for 30 minutes, and then the reaction was quenched. The reaction mixture was concentrated under reduced pressure to remove the solvent to obtain a crude product. The crude product was separated and purified by silica gel chromatography to yield Intermediate **121-2** (150.0 mg, 77.5%). LCMS (ESI) [M+H]⁺: 424.0.

Step 2: Under a nitrogen atmosphere, Intermediate **121-2** (100.0 mg, 0.24 mmol) was dissolved in tetrahydrofuran (2 mL), cooled to -78°C, and slowly added dropwise with lithium diisopropylamide (0.47 mL, 2.5 M). After the addition was completed, the reaction mixture was stirred at -78°C for another half hour, then added dropwise with methanesulfonyl chloride (81.1 mg, 0.708 mmol). The mixture was continuously stirred for 1 hour, and then gradually warmed to room temperature. The reaction was quenched by the addition of saturated ammonium chloride, and the aqueous phase was extracted with dichloromethane (10 mL × 3). The combined organic phases were washed sequentially with water and saturated brine, dried over anhydrous sodium sulfate, filtered, and concentrated to yield a crude product. The crude product was separated and purified by silica gel chromatography to yield Intermediate **121-3** (70.0 mg, 59.1%). LCMS (ESI) [M+H]⁺: 502.4.

Step 3: Intermediate **121-3** (50.0 mg, 0.10 mmol) was dissolved in tetrahydrofuran (3 mL), and then dilute hydrochloric acid (1 mL, 2 M) was added. The reaction mixture was stirred at room temperature for half an hour. The reaction was quenched by the addition of saturated sodium bicarbonate solution, and the aqueous phase was extracted with dichloromethane (10 mL × 3). The combined organic phases were washed sequentially with water and saturated brine, dried over anhydrous sodium sulfate, filtered, and concentrated to yield a crude product. The crude product was separated and purified by preparative reverse-phase high performance liquid chromatography to give (*R*)-3-methyl-4-(6-(methylsulfonyl)-2-(1*H*-pyrazol-3-yl)-6,7,8,9-tetrahydro-1,3,6,9a-tetraazabenzo[*cd*]azulen-4-yl)morpholine (Compound 121) (5.0 mg, 12.0%). LCMS (ESI) [M+H]⁺: 418.2. ¹H NMR (400 MHz, DMSO-d6) δ 7.67 (s, 1H), 7.07 (s, 1H), 6.96 (s, 1H), 4.46 - 4.39 (m, 1H), 4.38 - 4.29 (m, 2H), 4.13 - 4.04 (m, 1H), 4.03 - 3.88 (m, 3H), 3.77 (d, *J* = 11.2 Hz, 1H), 3.70 (dd, *J =* 11.2, 2.8 Hz, 1H), 3.54 (td, *J =* 11.6, 2.8 Hz, 1H), 3.34 (s, 3H), 3.15 (td, *J =* 12.5, 3.6 Hz, 1H), 2.41 - 2.30 (m, 1H), 2.07 (s, 1H), 1.16 (d, *J =* 6.6 Hz, 3H).

### Example 122: Preparation of (R)-4-(6-cyclopropyl-2-(1H-pyrazol-3-yl)-6,7,8,9-tetrahydro-1,3,6,9a-tetraazabenzo[cd]azulen-4-yl)-3-methylmorpholine (Compound 122)

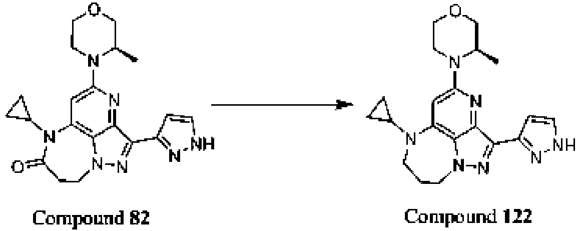

Step 1: Compound **82** (50.0 mg, 0.127 mmol) was dissolved in tetrahydrofuran (2 mL), and then a solution of borane in tetrahydrofuran (1.3 mL, 1 M) was added. The reaction mixture was stirred at room temperature for 1 hour. Methanol (10 mL) was added to the reaction mixture, and the mixture was stirred at reflux for 2 hours. The reaction mixture was concentrated to obtain a crude product, which was purified by preparative reverse-phase high performance liquid chromatography to give (*R*)-4-(6-cyclopropyl-2-(1*H*-pyrazol-3-yl)-6,7,8,9-tetrahydro-1,3,6,9a-tetraazabenzo[*cd*]azulen-4-yl)-3-methylmorpholine (Compound **122)** (15.0 mg, 31.1%). LCMS (ESI) [M+H]⁺: 380.4. ¹H NMR (400 MHz, DMSO-d6) δ 12.97 (s, 1H), 7.61 (d, *J =* 1.6 Hz, 1H), 7.02 (d, *J =* 1.7 Hz, 1H), 6.45 (s, 1H), 4.39 - 4.31 (m, 2H), 4.31 - 4.23 (m, 1H), 4.02 - 3.89 (m, 2H), 3.76 (d, *J =* 11.1 Hz, 1H), 3.70 (dd, *J =* 11.2, 2.7 Hz, 1H), 3.67 - 3.49 (m, 3H), 3.12 (td, *J =* 12.5, 3.5 Hz, 1H), 2.73 - 2.64 (m, 1H), 2.27 - 2.10 (m, 2H), 1.16 (d, *J =* 6.6 Hz, 3H), 1.04 - 0.94 (m, 2H), 0.70 - 0.58 (m, 2H).

### Example 123: Preparation of (R)-2-methyl-1-(4-(3-methylmorpholinyl)-2-(1H-pyrazol-3-yl)-8,9-dihydro-1,3,6,9a-tetraazabenzo[cd]azulene-6(7H)-yl)propan-2-ol (Compound 123)

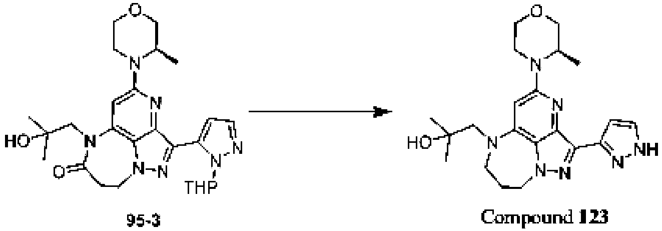

Step 1: Under a nitrogen atmosphere, Intermediate **95-3** (50 mg, 0.098 mmol) was dissolved in tetrahydrofuran (5.0 mL), and added dropwise with a solution of borane in tetrahydrofuran (1.0 mL, 1 M). The reaction mixture was stirred at 70°C for 12 hours. The reaction was quenched by the addition of methanol (10.0 mL) and hydrochloric acid (0.5 mL, 1 M). The pH was adjusted to approximately 8 with saturated sodium bicarbonate solution, and the aqueous phase was extracted with dichloromethane (30 mL × 3). The combined organic phases were washed sequentially with water and saturated brine, dried over anhydrous sodium sulfate, filtered, and concentrated to yield a crude product. The crude product was then separated and purified by preparative reverse-phase high-performance liquid chromatography to yield (R)-2-methyl-1-(4-(3-methylmorpholinyl)-2-(1*H*-pyrazol-3-yl)-8,9-dihydro-1,3,6,9a-tetraazabenzo[*cd*]azulene-6(7H)-yl)propan-2-ol (Compound **123)** (5.1 mg, 13%). LCMS (ESI) [M+H]⁺: 412.4. ¹H NMR (400 MHz, CD₃OD) δ 8.47 (s, 0.6H), 7.74 (s, 1H), 6.95 (s, 1H), 6.38 (s, 1H), 4.55 - 4.54 (m, 2H), 4.31 - 4.29 (m, 1H), 4.09 (d, *J =* 11.2 Hz, 1H), 3.89 (d, *J =* 11.2 Hz, 4H), 3.81 (d, *J =* 13.6 Hz, 1H), 3.71 (d, *J* = 15.2 Hz, 2H), 3.59 (d, *J =* 15.2 Hz, 1H), 3.42 (s, 1H), 2.44 (s, 2H), 1.37 (s, 6H), 1.32 (d, *J* = 6.8 Hz, 3H).

### Example 132: Preparation of (R)-7-cyclopropyl-4-(3-methylmorpholino)-2-(1H-pyrazol-3-yl)-2,7,8,9-tetrahydro-6H-1,2,3,7-tetraazabenzo[cd]azulene-6-one (Compound 132)

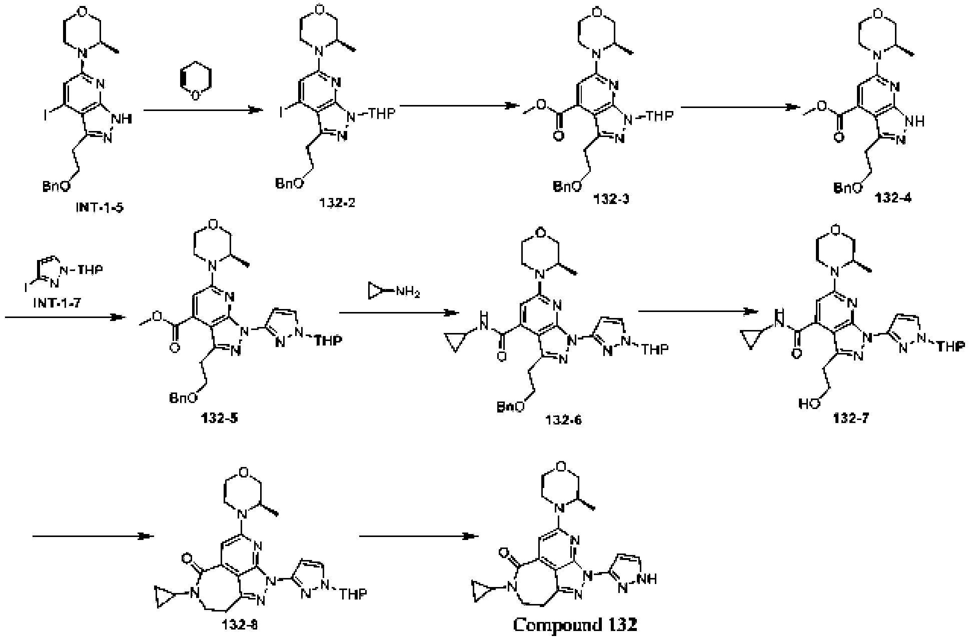

Step 1: Intermediate **INT-1-5** (6.0 g, 12.55 mmol) was dissolved in tetrahydrofuran (20 mL), and 3,4-dihydro-2*H*-pyran (2.1 g, 25.09 mmol) and p-toluenesulfonic acid (0.43 g, 2.51 mmol) were added in sequence. The reaction mixture was stirred at 80°C under a nitrogen atmosphere for 3 hours. After the reaction was completed, the mixture was cooled to room temperature, and tetrahydrofuran was removed under reduced pressure to obtain a crude product, which was then purified by silica gel column chromatography to yield Intermediate **132-2** (6.8 g, 96.4%). LCMS (ESI) [M+H]⁺: 563.2.

Step 2: Intermediate **132-2** (1.75 g, 3.11 mmol) was dissolved in methanol (10 mL), and 4-dimethylaminopyridine (0.80 g, 6.22 mmol), palladium acetate (0.10 g, 0.31 mmol), 4,5-bis(diphenylphosphino)-9,9-dimethylxanthene (0.40 g, 0.62 mmol), and dicobalt octacarbonyl (0.40 g, 1.24 mmol) were added in sequence. The reaction mixture was stirred at 80°C under a nitrogen atmosphere for half an hour. The mixture was then cooled to 25°C. The reaction mixture was spin-dried, and the residual solid was purified by silica gel column chromatography to yield Intermediate **132-3** (1.20 g, 78.0%). LCMS (ESI) [M+H]⁺: 495.2.

Step 3: Intermediate **132-3** (3.20 g, 6.47 mmol) was dissolved in tetrahydrofuran (60 mL), then dilute hydrochloric acid (64.7 mL, 1 M) was added, and the reaction mixture was stirred at room temperature for 1 hour. The reaction mixture was directly spin-dried to yield crude Intermediate **132-4** (2.60 g, 89.9%). LCMS (ESI) [M+H]⁺: 411.2.

Step 4: Intermediate 132-4 (1.00 g, 2.44 mmol) was dissolved in toluene (20 mL), and Intermediate **INT-1-7** (2.00 g, 7.31 mmol), cuprous iodide (0.50 g, 2.44 mmol), N,N-dimethylethylenediamine (0.30 g, 3.65 mmol), and cesium carbonate (2.40 g, 7.31 mmol) were added in sequence. The reaction mixture was stirred at 100°C under a nitrogen atmosphere for 16 hours. The reaction mixture was cooled to room temperature, and ice water was added to quench the reaction. The aqueous phase was extracted with ethyl acetate (30 mL × 3). The organic phase was washed with water and saturated brine. The organic phase was dried over anhydrous sodium sulfate, filtered and concentrated to obtain a crude product, which was separated and purified by silica gel column chromatography to yield Intermediate 132-5 (950 mg, 69.6%). LCMS (ESI) [M+H]⁺: 561.4.

Step 5: Compound 132-5 (650.0 mg, 1.16 mmol) was dissolved in tetrahydrofuran (4 mL), and cyclopropylamine (661.9 mg, 11.59 mmol) and dimethylaluminum chloride (5.80 mL, 1 M) were added in sequence. The reaction mixture was stirred at 80°C under a nitrogen atmosphere for 2 hours. The reaction mixture was cooled to room temperature and ice-cold water was added to quench the reaction. The mixture was extracted with ethyl acetate (30 mL × 3). The organic phase was washed sequentially with water and saturated brine. The organic phase was dried over anhydrous sodium sulfate, filtered, and concentrated to obtain a crude product, which was separated and purified by silica gel column chromatography to yield Intermediate 132-6 (610.0 mg, 89.80%). LCMS (ESI) [M+H]⁺: 586.3.

Step 6: Intermediate 132-6 (600.0 mg, 1.02 mmol) was dissolved in ethanol (2 mL), and 10% palladium-on-carbon (containing approximately 50% water) (545.0 mg, 5.12 mmol) and palladium hydroxide (719.1 mg, 5.12 mmol) were added in sequence. The reaction mixture was stirred at 80°C under a hydrogen atmosphere for 3 hours. The reaction mixture was cooled to room temperature and filtered through diatomite. The filtrate was concentrated to yield Intermediate 132-7 (330.0 mg, 65.0%). LCMS (ESI) [M+H]⁺: 496.2.

Step 7: 132-7 (300 mg, 0.61 mmol) was dissolved in toluene (2 mL), and cyanomethylenetributylphosphorane (730.6 mg, 3.03 mmol) was added. The reaction mixture was stirred at 130°C under a nitrogen atmosphere for 2 hours. The reaction mixture was cooled to room temperature and ice-cold water was added to quench the reaction. The resulting mixture was extracted with ethyl acetate (10 mL × 3). The organic phase was washed sequentially with water and saturated brine. The organic phase was dried over anhydrous sodium sulfate, filtered, and concentrated to obtain a crude product, which was separated and purified by silica gel column chromatography to yield Intermediate 132-8 (210.0 mg, 72.6%). LCMS (ESI) [M+H]⁺: 478.2.

Step 8: Intermediate **132-8** (150.0 mg, 0.314 mmol) was dissolved in tetrahydrofuran (2 mL), followed by the addition of dilute hydrochloric acid (3.14 mL, 1 M). The reaction mixture was stirred at room temperature for 1 hour. The reaction solution was separated and purified by reverse-phase high performance liquid chromatography to give (*R*)-7-cyclopropyl-4-(3-methylmorpholino)-2-(1*H*-pyrazol-3-yl)-2,7,8,9-tetrahydro-6*H*-1,2,3,7-te traazabenzo[*cd*]azulene-6-one (Compound 132) (15.6 mg, 12.6%). LCMS (ESI) [M+H]⁺ : 394.4. ¹H NMR (400 MHz, DMSO-d6) δ 12.83 (s, 1H), 7.84 (s, 1H), 7.24 (s, 1H), 6.77 (d, *J =* 2.4 Hz, 1H), 4.48 (d, *J* = 5.2 Hz, 1H), 4.08 (d, *J =* 12.4 Hz, 1H), 4.01 (dd, *J =* 11.2, 3.2 Hz, 1H), 3.87 (d, *J =* 5.4 Hz, 2H), 3.80 (d, *J* = 11.2 Hz, 1H), 3.68 (dd, *J* = 11.6, 2.8 Hz, 1H), 3.53 (td, *J* = 11.6, 2.8 Hz, 1H), 3.23 (td, *J* = 12.8, 3.6 Hz, 1H), 3.04 (dd, *J =* 10.0, 4.2 Hz, 2H), 3.01 - 2.96 (m, 1H), 1.23 (d, *J =* 6.8 Hz, 3H), 0.92 - 0.71 (m, 4H).

### Example 135: Preparation of (3R)-4-(6-cyclopropyl-7-(oxetan-3-yl)-2-(1H-pyrazol-3-yl)-6,7,8,9-tetrahydro-2H-1,2,3,7-tetraazabenzo[cd]azulen-4-yl)-3-methylmorpholine (Compound 135)

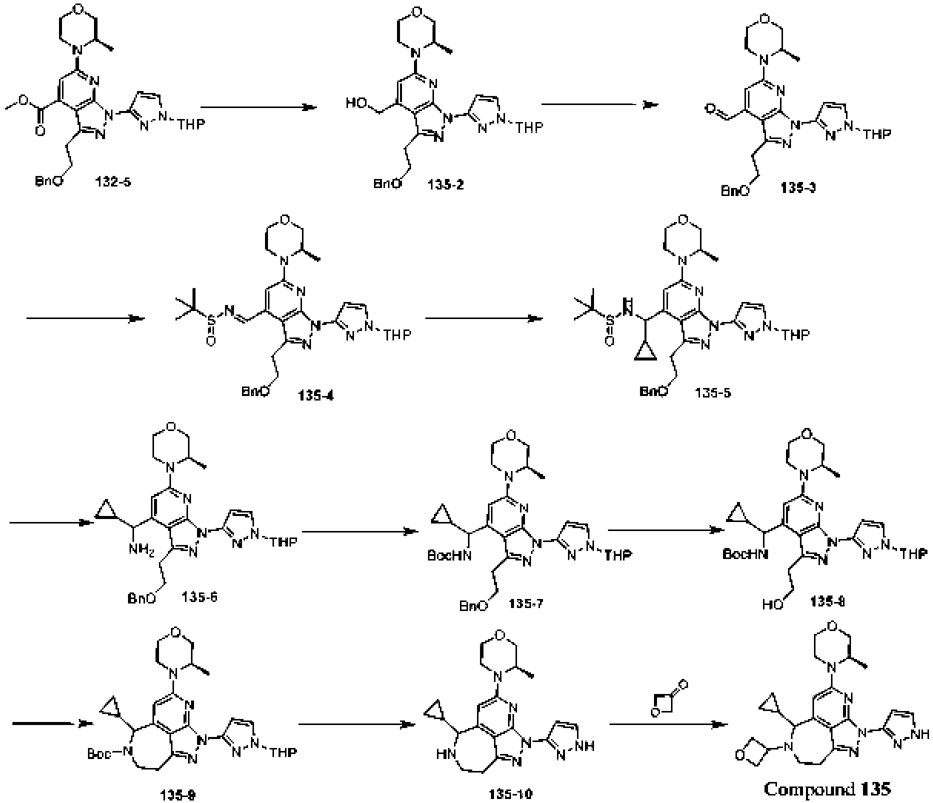

Step 1: Intermediate **132-5** (7.0 g, 12.5 mmol) was dissolved in ethanol/tetrahydrofuran (2/1) (150 mL), lithium chloride (2.1 g, 49.9 mmol) was added, and then sodium borohydride (1.9 g, 49.9 mmol) was added in batches. The reaction mixture was stirred at room temperature for 16 hours. The reaction mixture was quenched with water at 25°C. The resulting mixture was separated, and the aqueous phase was extracted with ethyl acetate (100 mL × 3). The organic phase was washed sequentially with water and saturated brine, dried over anhydrous sodium sulfate, and filtered. The filtrate was concentrated under reduced pressure to obtain the crude product, which was then separated and purified by silica gel column chromatography to yield Intermediate **135-2** (5.9 g, 89%). LCMS (ESI) [M+H]⁺: 533.0.

Step 2: Intermediate **135-2** (5.9 g, 11.1 mmol) was dissolved in dichloromethane (100 mL), then Dess-Martin periodinane (9.40 g, 22.2 mmol) was added. The reaction mixture was stirred and reacted at room temperature for 1 hour. The reaction mixture was quenched with water at 25°C. The resulting mixture was separated, and the aqueous phase was extracted with dichloromethane (100 mL × 3). The organic phase was washed sequentially with water and saturated brine, dried over anhydrous sodium sulfate, and filtered. The filtrate was concentrated under reduced pressure to yield a crude product, which was then purified by silica gel column chromatography to yield Intermediate **135-3** (5.5 g, 94%). LCMS (ESI) [M+H]⁺: 531.1.

Step 3: Intermediate **135-3** (1.2 g, 2.26 mmol) was dissolved in tetrahydrofuran (20 mL), and tetraethyl titanate (1.00 g, 4.52 mmol) and tert-butylsulfenamide (0.50 g, 4.53 mmol) were added in sequence. The mixture was stirred at 70°C for 3 hours. The reaction mixture was quenched with water at 25°C, filtered, and the filtrate was extracted with ethyl acetate (30 mL × 3). The organic phase was washed sequentially with water and saturated brine, dried over anhydrous sodium sulfate, and filtered. The filtrate was concentrated under reduced pressure to obtain a crude product, which was purified by silica gel column chromatography to yield Intermediate **135-4** (0.96 g, 67%). LCMS (ESI) [M+H]⁺: 634.2.

Step 4: Intermediate **135-4** (2.01 g, 3.13 mmol) was dissolved in dichloromethane (40 mL). The reaction mixture was cooled to -60°C, and cyclopropylmagnesium bromide (3.31 mL, 1 M) was added dropwise. The reaction mixture was stirred at -60°C for 1 hour, then warmed to 25°C and stirred for 1 hour. The reaction mixture was quenched with ice-cold water at 25°C. The resulting mixture was separated, and the aqueous phase was extracted with dichloromethane (50 mL × 3). The organic phase was washed sequentially with water and saturated brine, dried over anhydrous sodium sulfate, and filtered. The filtrate was concentrated under reduced pressure to obtain a crude product, which was separated and purified by silica gel column chromatography to yield Intermediate **135-5** (1.40 g, 62.5%). LCMS (ESI) [M+H]⁺: 676.2.

Step 5: Intermediate **135-5** (1.40 g, 2.07 mmol) was dissolved in tetrahydrofuran (30 mL), and hydrochloric acid (2.07 mL, 3 M) was added. The reaction mixture was stirred at 25°C for 1 hour. After monitoring the reaction for completion, the reaction mixture was quenched with aqueous sodium hydroxide (1 M) at 25°C, and the pH was adjusted to 8. The resulting mixture was separated, and the aqueous phase was extracted with ethyl acetate (50 mL × 3). The organic phase was washed sequentially with water and saturated brine, dried over anhydrous sodium sulfate, and filtered. The filtrate was concentrated under reduced pressure to yield crude Intermediate **135-6** (0.92 g, 78%). LCMS (ESI) [M+H]⁺: 572.4.

Step 6: Similarly to the preparation method of Example 40, the title Compound **135** was prepared using Intermediate **135-6** instead of Intermediate **INT-5.** (3*R*)-4-(6-cyclopropyl-7-(oxetan-3-yl)-2-(1*H*-pyrazol-3-yl)-6,7,8,9-tetrahydro-2*H*-1,2,3,7-tetraazabenzo[*cd*]azulen-4-yl)-3 -methylmorpholine (Compound **135**) (18.0 mg, 44.8%) was obtained. LCMS (ESI) [M+H]⁺: 436.2. ¹H NMR (400 MHz, CD₃OD) δ 7.75 (s, 1H), 6.94 (s, 1H), 6.60 (s, 1H), 4.77 (d, *J* = 6.4 Hz, 2H), 4.65 (d, *J =* 6.4 Hz, 2H), 4.51 (dd, *J =* 18.4, 7.6 Hz, 1H), 4.39 (dd, *J =* 13.6, 6.8 Hz, 1H), 4.16 - 4.05 (m, 2H), 3.98 - 3.89 (m, 1H), 3.88 - 3.78 (m, 2H), 3.66 (t, *J* = 11.2 Hz, 1H), 3.52 - 3.46 (m, 1H), 3.40 (dd, *J =* 10.4, 5.6 Hz, 1H), 3.30 (dd, *J =* 11.6, 4.4 Hz, 1H), 3.17 - 3.06 (m, 2H), 1.33 (d, *J =* 6.8 Hz, 4H), 0.73 - 0.64 (m, 2H), 0.58 - 0.48 (m, 2H).

### Example 136: Preparation of (3R)-4-(6-isopropyl-7-(oxetan-3-yl)-2-(1H-pyrazol-3-yl)-6,7, 8,9-tetrahydro-2H-1,2,3,7-tetraazabenzo[cd]azulen-4-yl)-3-methylmorpholine (Compound 136)

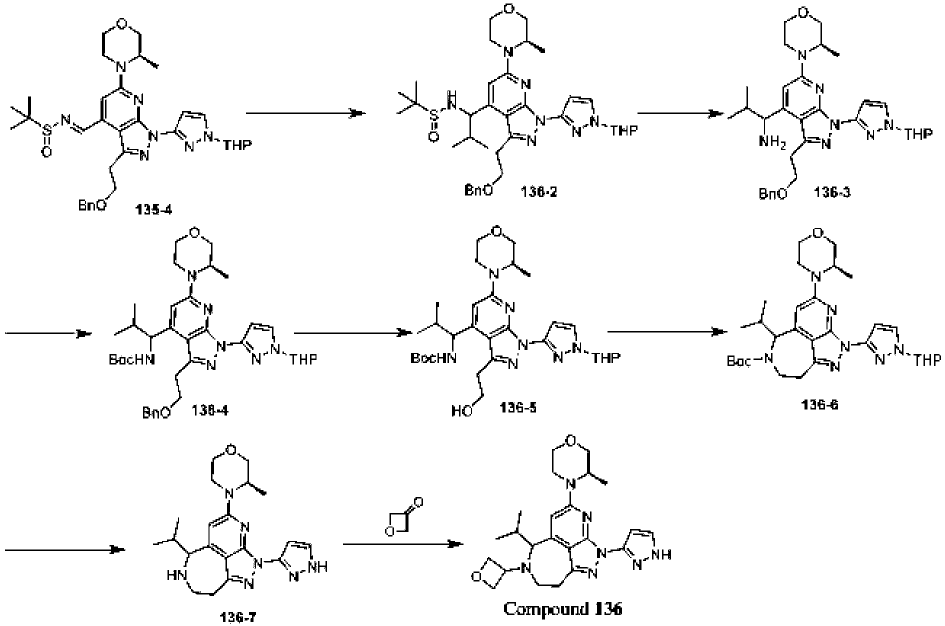

Similar to the preparation method of Example 135, the title Compound **136** was prepared using isopropylmagnesium bromide instead of cyclopropylmagnesium bromide. (3*R*)-4-(6-Isopropyl-7-(oxetan-3-yl)-2-(1*H*-pyrazol-3-yl)-6,7,8,9-tetrahydro-2*H*-1,2,3,7-tetraazab enzo[*cd*]azulen-4-yl)-3-methylmorpholine (Compound **136**) (80 mg, 69.8%) was obtained. LCMS (ESI) [M+H]⁺: 438.5. ¹H NMR (400 MHz, CD₃OD) δ 7.71 (s, 1H), 6.90 (s, 1H), 6.50 (s, 1H), 4.78 (t, *J =* 6.4 Hz, 1H), 4.67 (t, *J =* 6.6 Hz, 1H), 4.56 - 4.51 (m, 2H), 4.46 - 4.40 (m, 1H), 4.32 - 4.28 (m, 1H), 4.12 (dd, *J =* 13.6, 2.4 Hz, 1H), 4.02 (dd, *J =* 11.4, 3.6 Hz, 1H), 3.80 - 3.77 (m, 2H), 3.66 - 3.56 (m, 3H), 3.30 - 3.24 (m, 2H), 3.07 - 2.91 (m, 2H), 2.12 - 2.03 (m, 1H), 1.29 (d, *J =* 6.7 Hz, 3H), 1.17 (d, *J =* 6.6 Hz, 3H), 0.99 (d, *J =* 6.6 Hz, 3H).

### Example 137: Preparation of (R)-7-methyl-4-(3-methylmorpholinyl)-2-(1H-pyrazol-3-yl)-2,7,8,9-tetrahydro-6H-1,2,3,7-tetraazabenzo[cd]azulene-6-one (Compound 137)

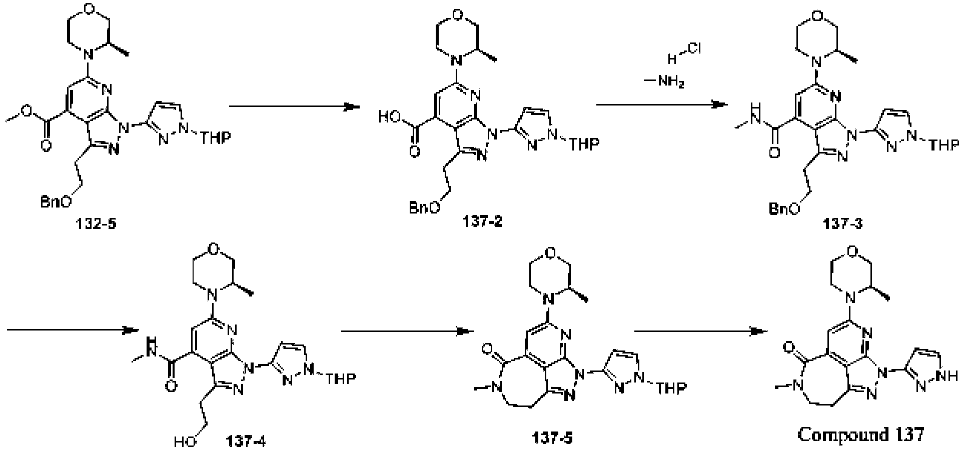

Step 1: Intermediate **132-5** (1.50 g, 2.68 mmol) was dissolved in tetrahydrofuran (30 mL), and lithium hydroxide (0.20 g, 5.35 mmol) was added. The reaction mixture was stirred at room temperature for 2 hours. The reaction mixture was quenched with ice-cold water at 25°C. The resulting mixture was separated, and the aqueous phase was extracted with ethyl acetate (30 mL × 3). The organic phase was washed sequentially with water and saturated brine, dried over anhydrous sodium sulfate, and filtered. The filtrate was concentrated under reduced pressure to yield crude Intermediate **137-2** (1.41 g, 96.4%). LCMS (ESI) [M+H]⁺: 547.2.

Step 2: Intermediate **137-2** (1.41 g, 2.58 mmol) was dissolved in *N,N*-dimethylformamide (15 mL), and O-(7-Azabenzotriazol-1-yl)-*N*,*N*,*N'*,*N'*-tetramethyluronium hexafluorophosphate (1.2 g, 3.10 mmol), *N,N*-diisopropylethylamine (1.00 g, 7.74 mmol), and methylamine hydrochloride (1.70 g, 25.80 mmol) were added in sequence. The reaction mixture was stirred at room temperature for 16 hours. The reaction mixture was quenched with ice-cold water at 25°C. The resulting mixture was separated, and the aqueous phase was extracted with ethyl acetate (40 mL × 3). The organic phase was washed sequentially with water and saturated brine, dried over anhydrous sodium sulfate, filtered, and concentrated to obtain a crude product. The crude product was separated and purified by silica gel column chromatography to yield Intermediate **137-3** (1.32 g, 91.4%). LCMS (ESI) [M+H]⁺: 560.2.

Step 3: Similar to the preparation method of Example 132, the title Compound **137** was prepared using Intermediate **137-3** instead of Intermediate **132-6.** (*R*)-7-Methyl-4-(3-methylmorpholinyl)-2-(1*H*-pyrazol-3-yl)-2,7,8,9-tetrahydro-6H-1,2,3,7-tetraa zabenzo[*cd*]azulene-6-one (Compound **137**) (10.0 mg, 12.3%) was obtained. LCMS (ESI) [M+H]⁺: 368.2. ¹H NMR (400 MHz, CD₃OD) δ 7.69 (d, *J =* 2.4 Hz, 1H), 7.26 (s, 1H), 6.86 (d, *J* = 2.4 Hz, 1H), 4.56 - 4.45 (m, 1H), 4.15 - 4.08 (m, 1H), 4.00 (dd, *J =* 11.4, 3.8 Hz, 1H), 3.86 (d, *J* = 19.8 Hz, 2H), 3.80 (d, *J* = 11.4 Hz, 1H), 3.74 (dd, *J* = 11.4, 3.2 Hz, 1H), 3.59 (td, *J* = 12.0, 3.2 Hz, 1H), 3.34 (dd, *J* = 13.0, 4.0 Hz, 1H), 3.30 (d, *J* = 4.0 Hz, 1H), 3.26 (s, 3H), 3.18 - 3.06 (m, 2H), 1.29 (d, *J* = 6.8 Hz, 3H).

### Example 138A and Example 138B: Preparation of (3R)-4-(6-cyclopropyl-7-(methylsulfonyl)-2-(1H-pyrazol-3-yl)-6,7,8,9-tetrahydro-2H-1,2,3,7-tetraazabenzo[cd]azulen-4 -yl)-3-methylmorpholine (Compound 138A, Isomer 1) and preparation of (3R)-4-(6-cyclopropyl-7-(methylsulfonyl)-2-(1H-pyrazol-3-yl)-6,7,8,9-tetrahydro-2H-1,2,3,7-tetraazabenzo[cd]azulen-4 -yl)-3-methylmorpholine (Compound 138B, Isomer 2)

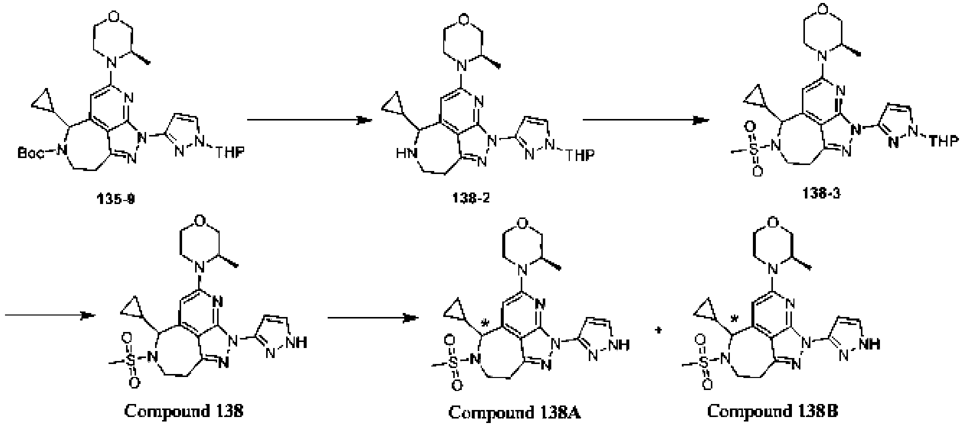

Step 1: Intermediate **135-9** (170.0 mg, 0.30 mmol) was dissolved in dichloromethane (3 mL), and 2,6-dimethylpyridine (323.3 mg, 3.0 mmol) and trimethylsilyl trifluoromethanesulfonate (268.2 mg, 1.2 mmol) were added in sequence. The reaction mixture was stirred at room temperature for 2 hours. The reaction mixture was quenched with ice water at 25°C. The resulting mixture was separated, and the aqueous phase was extracted with ethyl acetate (20 mL × 3). The organic phase was washed sequentially with water and saturated brine, dried over anhydrous sodium sulfate, filtered, and concentrated to obtain a crude product. The crude product was separated and purified by silica gel column chromatography to yield Intermediate **138-2** (135.0 mg, 96.6%). LCMS (ESI) [M+H]⁺: 464.2.

Step 2: Similar to the preparation method of Example 29, Compound **138** was prepared using Intermediate **138-2** instead of Intermediate **5-1.** A diastereomeric mixture of (3*R*)-4-(6-cyclopropyl-7-(methylsulfonyl)-2-(1*H*-pyrazol-3-yl)-6,7,8,9-tetrahydro-2*H*-1,2,3,7-tetr aazabenzo[*cd*]azulen-4-yl)-3-methylmorpholine (Compound **138**) (25.0 mg, 26.9%) was obtained. LCMS (ESI) [M+H]⁺: 458.2.

Step 3: The diastereomeric mixture of (3*R*)-4-(6-cyclopropyl-7-(methylsulfonyl)-2-(1*H-*pyrazol-3-yl)-6,7,8,9-tetrahydro-2*H*-1,2,3,7-tetraazabenzo[*cd*]azulen-4-yl)-3-methylmorpholine (Compound **138**) (25.0 mg) was further purified by chiral SFC. SFC analysis method: Daicel ChiralPAK^{®}AS column (100 × 3.0 mm, 3 µm), mobile phase: 30% methanol (containing 0.1% diethylamine) in CO₂, temperature: 35°C, flow rate: 1.5 mL/min, detection wavelength: 214 nm.

The first peak was (3*R*)-4-(6-cyclopropyl-7-(methylsulfonyl)-2-(1*H*-pyrazol-3-yl)-6,7,8,9-tetrahydro-2*H*-1,2,3,7-tetraazabenzo[*cd*]azulen-4-yl)-3-methylmorpholine (Compound **138A,** Isomer 1) (5.4 mg). LCMS (ESI) [M+H]⁺: 458.2. HPLC-SFC: Rt = 0.772 min. ¹H NMR (400 MHz, DMSO-d6) δ 12.78 (s, 1H), 7.84 (s, 1H), 6.77 (d, *J* = 21.2 Hz, 2H), 4.48 (d, *J* = 8.8 Hz, 2H), 4.09 - 3.99 (m, 4H), 3.78 (d, *J =* 11.2 Hz, 1H), 3.68 (d, *J =* 9.2 Hz, 1H), 3.52 (t, *J =* 11.6 Hz, IH), 3.22 - 3.13 (m, 2H), 2.91 (s, 3H), 1.42 (s, 1H), 1.27 - 1.19 (m, 4H), 0.94 (s, 1H), 0.65 (d, *J* = 6.8 Hz, 2H), 0.51 (s, 1H).

The second peak was (3*R*)-4-(6-cyclopropyl-7-(methylsulfonyl)-2-(1*H*-pyrazol-3-yl)-6,7,8,9-tetrahydro-2*H*-1,2,3,7-tetraazabenzo[*cd*]azulen-4-yl)-3-methylmorpholine (Compound **138B,** Isomer 2) (4.6 mg). LCMS (ESI) [M+H]⁺: 458.2. HPLC-SFC: Rt = 1.115 min. ¹H NMR (400 MHz, DMSO-d6) δ 12.78 (s, 1H), 7.84 (s, 1H), 6.77 (d, *J* = 21.2 Hz, 2H), 4.48 (d, *J* = 8.8 Hz, 2H), 4.09 - 3.99 (m, 4H), 3.78 (d, *J =* 11.2 Hz, 1H), 3.68 (d, *J =* 9.2 Hz, 1H), 3.52 (t, *J* = 11.6 Hz, IH), 3.22 - 3.13 (m, 2H), 2.91 (s, 3H), 1.42 (s, 1H), 1.27 - 1.19 (m, 4H), 0.94 (s, 1H), 0.65 (d, *J* = 6.8 Hz, 2H), 0.51 (s, 1H).

### Example 139: Preparation of (R)-7-(bicyclo[1.1.1]pentan-1-yl)-4-(3-methylmorpholinyl)-2-(1H-pyrazol-3-yl)-2,7,8,9-tetrahydro-6H-1,2,3,7-tetraazabenzo[cd]azulene-6-one (Compound 139)

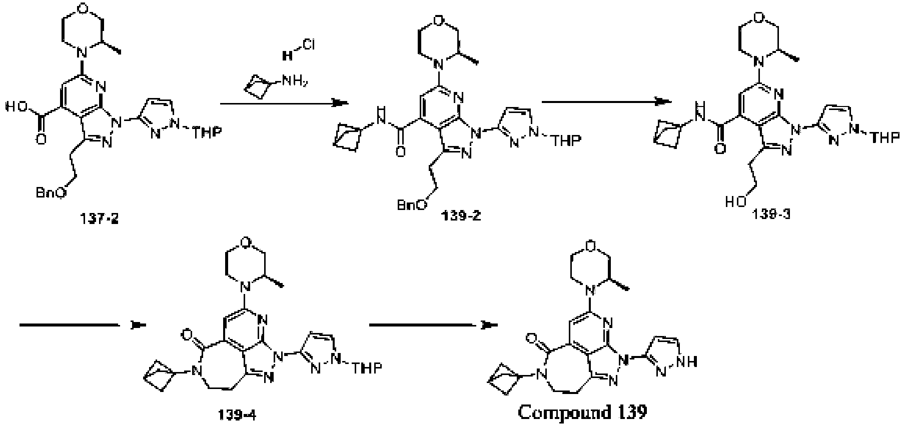

Similarly to the preparation method of Example 137, the title Compound **139** was prepared using bicyclo[1,1,1]pentan-1-amine hydrochloride instead of methylamine hydrochloride. (*R*)-7-(Bicyclo[1.1.1]pentan-1-yl)-4-(3-methylmorpholinyl)-2-(1*H*-pyrazol-3-yl)-2,7,8,9-tetrahyd ro-6*H*-1,2,3,7-tetraazabenzo[*cd*]azulen-6-one (Compound 139) (8.0 mg, 16.0%) was obtained. LCMS (ESI) [M+H]⁺: 420.4. ¹H NMR (400 MHz, CD₃OD) δ 7.74 (s, 1H), 7.26 (s, 1H), 6.90 (s, 1H), 4.54 (s, 1H), 4.15 (d, *J =* 13.2 Hz, 1H), 4.06 - 4.01 (m, 1H), 3.84 (d, *J =* 11.2 Hz, 3H), 3.80 - 3.76 (m, 1H), 3.66 - 3.60 (m, 1H), 3.37 (d, *J =* 9.2 Hz, 1H), 3.11 (d, *J =* 4.4 Hz, 2H), 2.56 (s, 1H), 2.31 (s, 6H), 1.32 (d, *J =* 6.8 Hz, 3H).

### Example 140A and Example 140B: Preparation of (3R)-4-(6-isopropyl-7-(methylsulfonyl)-2-(1H-pyrazol-3-yl)-6,7,8,9-tetrahydro-2H-1,2,3,7-tetraazabenzo[cd]azulen-4-yl)-3-methylmorp holine (Compound 140A, Isomer 1) and preparation of (3R)-4-(6-isopropyl-7-(methylsulfonyl)-2-(1H-pyrazol-3-yl)-6,7,8,9-tetrahydro-2H-1,2,3,7-tetraazabenzo[cd]azulen-4-yl)-3-methylmorp holine (Compound 140B, Isomer 2)

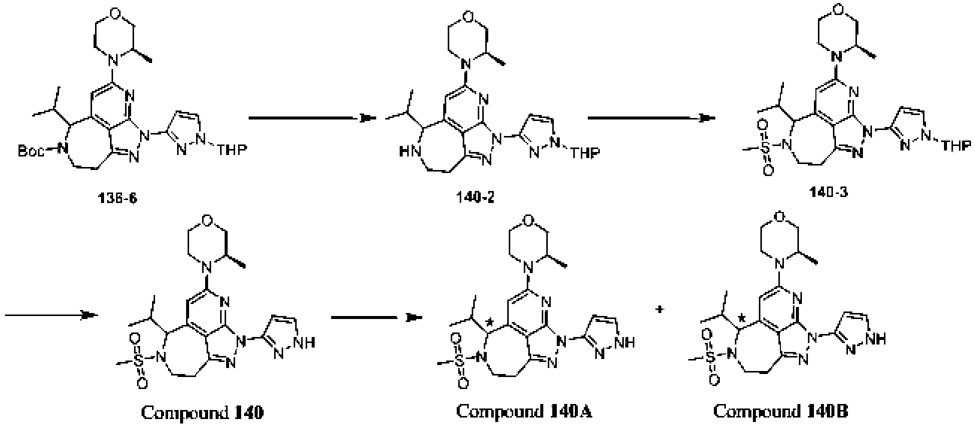

Step 1: Intermediate **136-6** (400 mg, 0.71 mmol) was dissolved in acetonitrile (10 mL) at 0°C, and added dropwise with trimethylsilyl trifluoromethanesulfonate (314 mg, 1.41 mmol). The reaction mixture was stirred at 0°C for 1 hour. The reaction was quenched by adding aqueous sodium bicarbonate, and the aqueous phase was extracted with ethyl acetate (30 mL × 3). The organic phase was washed sequentially with water and saturated brine, dried over anhydrous sodium sulfate, filtered, and concentrated to obtain a crude product. The crude product was separated and purified by silica gel column chromatography to yield Intermediate **140-2** (240 mg, 72.9%).

Step 2: Similar to the preparation method of Example 22, Compound **140** was prepared using Intermediate **140-2** instead of Intermediate **5-1.** A diastereomeric mixture of (3*R*)-4-(6-isopropyl-7-(methylsulfonyl)-2-(1*H*-pyrazol-3-yl)-6,7,8,9-tetrahydro-2*H*-1,2,3,7-tetraa zabenzo[*cd*]azulen-4-yl)-3-methylmorpholine (Compound **140**) (50.0 mg, 73.9%) was obtained. LCMS (ESI) [M+H]⁺: 460.2.

Step 3: The diastereomeric mixture of (3*R*)-4-(6-isopropyl-7-(methylsulfonyl)-2-(1*H-*pyrazol-3-yl)-6,7,8,9-tetrahydro-2*H*-1,2,3,7-tetraazabenzo[*cd*]azulen-4-yl)-3-methylmorpholine (Compound **140**) (50.0 mg) was further purified by chiral SFC. SFC analysis method: Daicel ChiralPAK^{®} OJ column (100 × 3.0 mm, 3 µm), mobile phase: 10% methanol (containing 0.1% ammonia) in CO₂, temperature: 35°C, flow rate: 1.5 mL/min, detection wavelength: 210 nm.

The first peak was (3*R*)-4-(6-isopropyl-7-(methylsulfonyl)-2-(1*H*-pyrazol-3-yl)-6,7,8,9-tetrahydro-2*H*-1,2,3,7-tetraazabenzo[*cd*]azulen-4-yl)-3-methylmorpholine (Compound **140A,** Isomer 1) (15 mg). LCMS (ESI) [M+H]⁺: 460.2. HPLC-SFC: Rt = 1.982 min. ¹H NMR (400 MHz, DMSO-d6) δ 12.76 (s, 1H), 7.79 (s, 1H), 6.76 (d, *J =* 2.1 Hz, 1H), 6.65 (s, 1H), 4.65 (d, *J* = 10.0 Hz, 1H), 4.43 - 4.39 (m, 1H), 4.14 - 3.91 (m, 3H), 3.79 - 3.64 (m, 3H), 3.55 - 3.45 (m, 1H), 3.29 - 3.24 (m, 1H), 3.22 - 3.08 (m, 2H), 2.85 (s, 3H), 2.20 - 2.05 (m, 1H), 1.19 (d, *J =* 6.6 Hz, 3H), 1.02 (t, *J =* 5.9 Hz, 6H).

The second peak was (3*R*)-4-(6-isopropyl-7-(methylsulfonyl)-2-(1*H*-pyrazol-3-yl)-6,7,8,9-tetrahydro-2*H*-1,2,3,7-tetraazabenzo[*cd*]azulen-4-yl)-3-methylmorpholine (Compound **140B,** Isomer 2) (16 mg). LCMS (ESI) [M+H]⁺: 460.2. HPLC-SFC: Rt = 2.482 min. ¹H NMR (400 MHz, DMSO-d6) δ 12.77 (s, 1H), 7.80 (s, 1H), 6.76 (d, *J =* 1.9 Hz, 1H), 6.63 (s, 1H), 4.67 (d, *J* = 9.9 Hz, 1H), 4.41 (d, *J =* 6.7 Hz, 1H), 4.17 - 3.90 (m, 3H), 3.79 - 3.60 (m, 3H), 3.55 - 3.46 (m, 1H), 3.29 - 3.24 (m, 1H), 3.22 - 3.15 (m, 1H), 3.14 - 3.09 (m, 1H), 2.84 (s, 3H), 2.19 - 2.05 (m, 1H), 1.20 (d, *J =* 6.6 Hz, 3H), 1.05 - 10.99 (m, 6H).

### Example 141A and Example 141B: Preparation of (3R)-4-(6-isopropyl-7-methyl-2-(1H-pyrazol-3-yl)-6,7,8,9-tetrahydro-2H-1,2,3,7-tetraazabenzo[cd]azulen-4-yl)-3-methylmorp holine (Compound 141A, Isomer 1) and preparation of (3R)-4-(6-isopropyl-7-methyl-2-(1H-pyrazol-3-yl)-6,7,8,9-tetrahydro-2H-1,2,3,7-tetraazabenzo[cd]azulen-4-yl)-3-methylmorp holine (Compound 141B, Isomer 2)

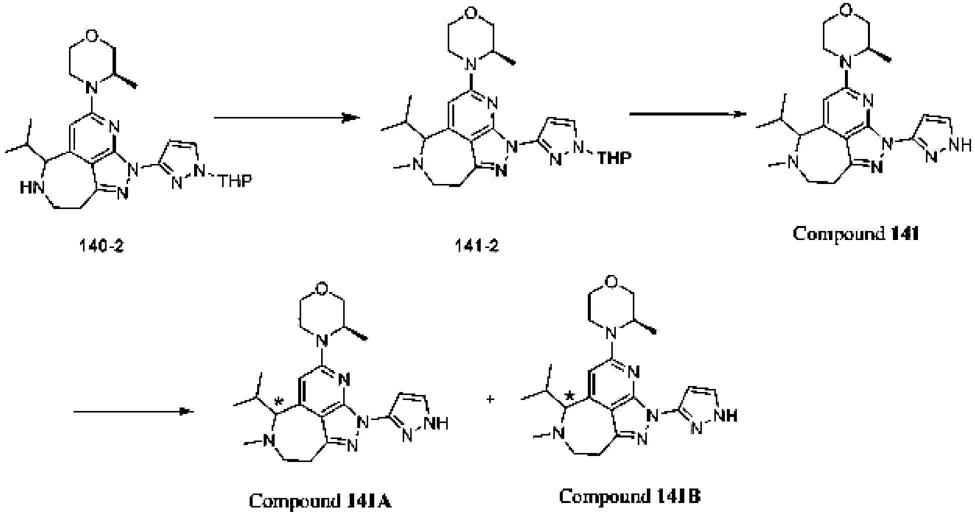

Step 1: Intermediate **140-2** (100 mg, 0.22 mmol) was dissolved in isopropyl acetate (5.0 mL) at 0°C, and paraformaldehyde (96.7 mg, 1.07 mmol) and sodium cyanoborohydride (40.5 mg, 0.64 mmol) were added. The reaction mixture was stirred at room temperature for 16 hours. The reaction was quenched by the addition of saturated aqueous ammonium chloride, and the aqueous phase was extracted with ethyl acetate (20 mL × 3). The organic phase was washed sequentially with water and saturated brine. The organic phase was dried over anhydrous sodium sulfate, filtered, and concentrated to obtain a crude product. The crude product was purified by silica gel chromatography to yield Intermediate **141-2** (80 mg, 77.7%). LCMS (ESI) [M+H]⁺: 480.5.

Step 2: Intermediate **141-2** (80 mg, 0.17 mmol) was dissolved in hydrochloric acid/ethyl acetate solution (5.0 mL, 4 M) at 0°C. The reaction mixture was stirred at room temperature for 1 hour. The reaction mixture was concentrated to obtain a crude product. The crude product was separated and purified by reverse-phase high-performance liquid chromatography to obtain a diastereomeric mixture of (3*R*)-4-(6-isopropyl-7-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **141**) (50 mg, 75.7%). LCMS (ESI) [M+H]⁺: 396.4.

Step 3: The diastereomeric mixture of (3*R*)-4-(6-isopropyl-7-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **141**) (50 mg) was further purified by chiral SFC. SFC analysis method: Daicel CHIRALPAK^{®} OJ column (100 × 3.0 mm, 3 µm), mobile phase: 10% methanol (containing 0.1% ammonia) in CO₂, temperature: 35°C, flow rate: 1.5 mL/min, detection wavelength: 210 nm.

The first peak was (3*R*)-4-(6-isopropyl-7-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **141A,** Isomer 1) (13 mg). LCMS (ESI) [M+H]⁺: 396.4. HPLC-SFC: Rt = 1.153 min. ¹H NMR (400 MHz, DMSO-d6) δ 12.73 (s, 1H), 7.78 (s, 1H), 6.77 (d, *J =* 2.2 Hz, 1H), 6.51 (s, 1H), 4.39 - 4.35 (m, 1H), 4.06 (d, *J* = 12.7 Hz, 1H), 4.02 - 3.91 (m, 1H), 3.76 (d, *J* = 11.4 Hz, 1H), 3.66 (dd, *J* = 11.3, 2.5 Hz, 1H), 3.61 - 3.41 (m, 3H), 3.26 - 3.15 (m, 2H), 3.12 - 3.02 (m, 1H), 2.99 - 2.91 (m, 1H), 2.44 (s, 3H), 1.97 - 1.93 (m, 1H), 1.19 (d, *J =* 6.6 Hz, 3H), 1.08 (d, *J =* 6.6 Hz, 3H), 0.90 (d, *J =* 6.6 Hz, 3H).

The second peak was (3*R*)-4-(6-isopropyl-7-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **141B,** Isomer 2) (15 mg). LCMS (ESI) [M+H]⁺: 396.4. HPLC-SFC: Rt = 1.832 min. ¹H NMR (400 MHz, DMSO-d6) δ 12.73 (s, 1H), 7.79 (s, 1H), 6.77 (s, 1H), 6.49 (s, 1H), 4.42 (d, *J =* 4.8 Hz, 1H), 4.09 - 3.93 (m, 2H), 3.75 (d, *J* = 11.3 Hz, 1H), 3.69 - 3.62 (m, 1H), 3.60 - 3.43 (m, 3H), 3.23 - 3.13 (m, 2H), 3.09 - 3.04 (m, 1H), 2.94 (d, *J =* 16.0 Hz, 1H), 2.45 (s, 3H), 1.98 - 1.90 (m, 1H), 1.20 (d, *J =* 6.6 Hz, 3H), 1.07 (d, *J =* 6.6 Hz, 3H), 0.89 (d, *J =* 6.6 Hz, 3H).

### Example 142A and Example 142B: Preparation of (3R)-4-(6-cyclopropyl-7-methyl-2-(1H-pyrazol-3-yl)-6,7,8,9-tetrahydro-2H-1,2,3,7-tetraazabenzo[cd]]azulen-4-yl)-3-methylmorp holine (Compound 142A, Isomer 1) and preparation of (3R)-4-(6-cyclopropyl-7-methyl-2-(1H-pyrazol-3-yl)-6,7,8,9-tetrahydro-2H-1,2,3,7-tetraazabenzo[cd]]azulen-4-yl)-3-methylmorp holine (Compound 142B, Isomer 2)

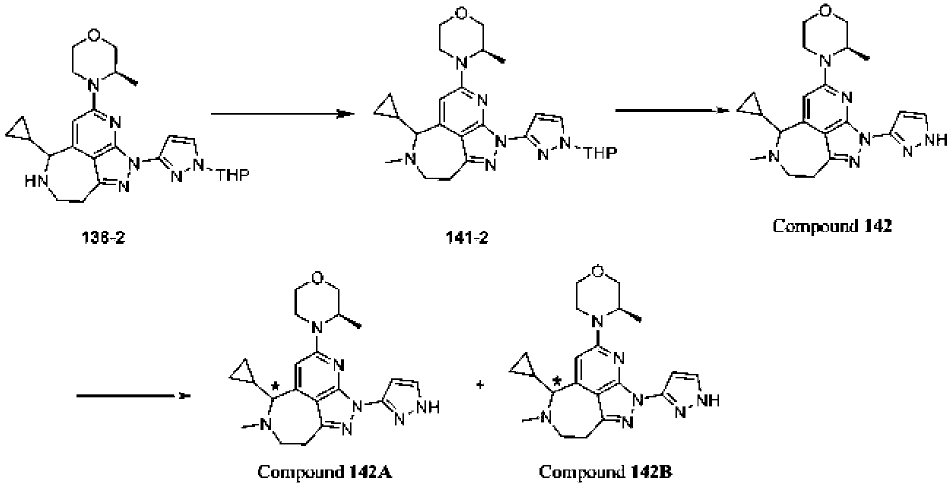

Step 1: Similar to the preparation method of Example 141, the title Compound **142** was prepared by using Intermediate **138-2** instead of Intermediate **140-2.** (3*R*)-4-(6-Cyclopropyl-7-methyl-2-(1*H*-pyrazol-3-yl)-6,7,8,9-tetrahydro-2*H*-1,2,3,7-tetraazabenzo[*cd*]]azulen-4-yl)-3-m ethylmorpholine (Compound **142**) (80.0 mg, 74.7%) was obtained. LCMS (ESI) [M+H]⁺: 394.2.

Step 2: The diastereomeric mixture of (3*R*)-4-(6-cyclopropyl-7-methyl-2-(1*H*-pyrazol-3-yl)-6,7,8,9-tetrahydro-2*H*-1,2,3,7-tetraazabenzo[*cd*]]azulen-4-yl)-3-methylmorpholine (Compound **142**) (80 mg) was further purified by chiral SFC. SFC analysis method: Daicel ChiralCel OD column (150 × 4.6 mm, 5 µm), mobile phase: 20% methanol (containing 0.1% ammonia) in n-hexane, flow rate: 1.0 mL/min, detection: 210/254 nm.

The first peak was (3*R*)-4-(6-cyclopropyl-7-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **142A,** Isomer 1) (15 mg). LCMS (ESI) [M+H]⁺: 393.9. HPLC-SFC: Rt = 2.673 min. ¹H NMR (400 MHz, DMSO-d6) δ 12.72 (s, 1H), 7.80 (abs, 1H), 6.77 (abs, 1H), 6.62 (s, 1H), 4.45 - 4.35 (m, 1H), 4.10 - 3.91 (m, 2H), 3.86 - 3.73 (m, 2H), 3.65 (dd, *J* = 11.3, 2.8 Hz, 1H), 3.54 - 3.46 (m, 1H), 3.38 - 3.32 (m, 1H), 3.25 - 3.09 (m, 3H), 3.06 - 2.97 (m, 1H), 2.43 (s, 3H), 1.25 - 1.15 (m, 4H), 0.62 - 0.53 (m, 2H), 0.48 - 0.35 (m, 2H).

The second peak was (3*R*)-4-(6-cyclopropyl-7-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **142B,** Isomer 2) (13 mg). LCMS (ESI) [M+H]⁺: 393.9. HPLC-SFC: Rt = 3.536 min. ¹H NMR (400 MHz, DMSO-d6) δ 12.72 (s, 1H), 7.80 (abs, 1H), 6.77 (abs, 1H), 6.62 (s, 1H), 4.45 - 4.35 (m, 1H), 4.10 - 3.91 (m, 2H), 3.86 - 3.73 (m, 2H), 3.65 (dd, *J* = 11.3, 2.8 Hz, 1H), 3.54 - 3.46 (m, 1H), 3.38 - 3.32 (m, 1H), 3.25 - 3.09 (m, 3H), 3.06 - 2.97 (m, 1H), 2.43 (s, 3H), 1.25 - δ 1.15 (m, 4H), 0.62 - 0.53 (m, 2H), 0.48 - 0.35 (m, 2H).

### Example 143: Preparation of diastereomeric mixture of (3R)-4-(6-cyclopropyl-7-(cyclopropylsulfonyl)-2-(1H-pyrazol-3-yl)-6,7,8,9-tetrahydro-2H-1,2,3,7-tetraazabenzo[cd]-azul en-4-yl)-3-methylmorpholine (Compound 143)

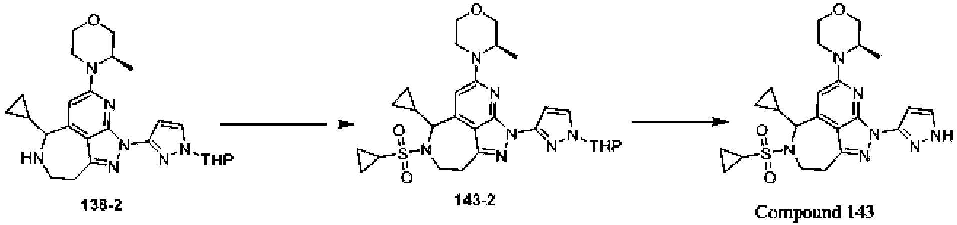

Step 1: Similar to the preparation method of Example 138, the title Compound **143** was prepared by using cyclopropanesulfonyl chloride instead of methanesulfonyl chloride. A diastereomeric mixture of (3*R*)-4-(6-cyclopropyl-7-(cyclopropylsulfonyl)-2-(1*H*-pyrazol-3-yl)-6,7,8,9-tetrahydro-2*H*-1,2,3,7-tetraazabenzo[*cd*]-azulen-4-yl)-3-methylmorpholine (Compound **143**) (1.0 mg, 5.9%) was obtained. LCMS(ESI) [M+H]⁺: 484.2. ¹H NMR (400 MHz, CD₃OD) δ 7.72 (s, 1H), 6.91 (s, 1H), 6.67 - 6.63 (m, 1H), 4.54 - 4.48 (m, 2H), 4.19 - 4.17 (m, 1H), 4.15 - 4.06 (m, 2H), 4.05 - 4.01 (m, 1H), 3.85 - 3.81 (m, 1H), 3.80 - 3.75 (m, 1H), 3.65 - 3.60 (m, 1H), 3.48 - 3.43 (m, 1H), 3.27 - 3.21 (m, 2H), 2.29 - 2.23 (m, 1H), 1.41 - 1.37 (m, 1H), 1.29 (d, *J =* 2.0 Hz, 3H), 1.05 - 1.01 (m, 1H), 0.98 - 0.93 (m, 1H), 0.8 - 0.82 (m, 2H), 0.77 - 0.75 (m, 1H), 0.72 - 0.68 (m, 1H), 0.66 - 0.58 (m, 2H).

### Example 144A and Example 144B: Preparation of (3R)-4-(6-ethyl-7-(methylsulfonyl)-2-(1H-pyrazol-3-yl)-6,7,8,9-tetrahydro-2H-1,2,3,7-tetraazabenzo[cd]-azulen-4-yl)-3-methylmorp holine (Compound 144A, Isomer 1) and preparation of (3R)-4-(6-ethyl-7-(methylsulfonyl)-2-(1H-pyrazol-3-yl)-6,7,8,9-tetrahydro-2H-1,2,3,7-tetraazabenzo[cd]-azulen-4-yl)-3-methylmorp holine (Compound 144B, Isomer 2)

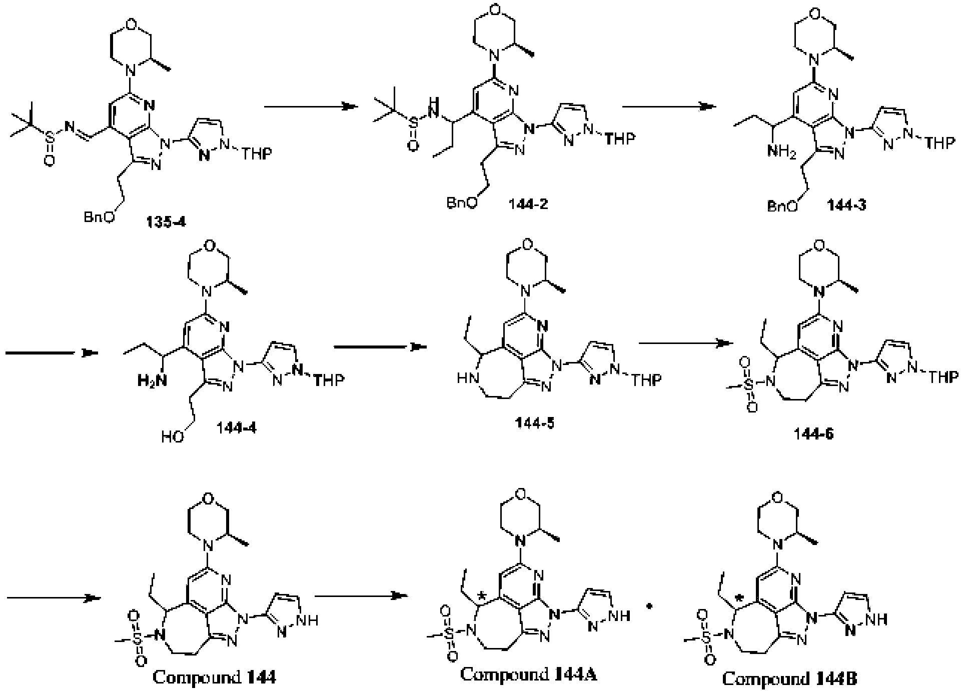

Step 1: Under a nitrogen atmosphere, Intermediate **135-4** (2.0 g, 3.15 mmol) was dissolved in tetrahydrofuran (30 mL). The reaction mixture was cooled to -78°C, and then ethylmagnesium bromide (15.7 mL, 15.7 mmol) was added dropwise. The reaction mixture was stirred at -78°C for half an hour. After the reaction was completed, the reaction mixture was quenched with saturated aqueous ammonium chloride (50 mL) at 0°C. The mixture was extracted with ethyl acetate (50 mL × 3). The combined organic phases were washed sequentially with water and saturated brine, dried over anhydrous sodium sulfate, and filtered. The filtrate was concentrated under reduced pressure to obtain a crude product, which was separated and purified by silica gel column chromatography to yield Intermediate **144-2** (2.0 g, 95.5%). LCMS (ESI) [M+H]⁺: 664.4.

Step 2: Intermediate **144-2** (2.0 g, 3.01 mmol) was dissolved in tetrahydrofuran (10 mL), and hydrochloric acid (1.0 mL, 6 M) was added. The reaction mixture was stirred at 25°C for half an hour. After the reaction was completed, the reaction mixture was quenched with saturated aqueous sodium bicarbonate. The mixture was extracted with dichloromethane (50 mL × 3). The combined organic phases were washed sequentially with water and saturated brine, dried over anhydrous sodium sulfate, and filtered. The filtrate was concentrated under reduced pressure to obtain a crude product, which was separated and purified by silica gel column chromatography to yield Intermediate **144-3** (1.2 g, 71.2%). LCMS (ESI) [M+H]⁺: 559.9.

Step 3: Intermediate **144-3** (1.2 g, 2.14 mmol) was dissolved in methanol (20 mL), and 10% palladium-on-carbon (containing approximately 50% water) (600 mg) and palladium hydroxide (600 mg) were added in sequence. The reaction mixture was stirred at 60°C under a hydrogen atmosphere for 18 hours. The reaction mixture was cooled to room temperature and filtered through diatomite. The filtrate was concentrated to yield Intermediate **144-4** (1.0 g, 98.8%). LCMS (ESI) [M+H]⁺: 470.4.

Step 4: Intermediate **144-4** (1.0 g, 1.78 mmol) was dissolved in toluene (20 mL), and cyanomethylenetributylphosphorane (1.03 g, 4.25 mmol) was added. The reaction mixture was stirred at 130°C under a nitrogen atmosphere for 18 hours. The reaction mixture was cooled to room temperature and quenched with ice water. The resulting mixture was extracted with ethyl acetate (20 mL × 3). The organic phase was washed sequentially with water and saturated brine. The organic phase was dried over anhydrous sodium sulfate, filtered, and concentrated to obtain a crude product, which was separated and purified by silica gel column chromatography to yield Intermediate **144-5** (900 mg, 93.6%). LCMS (ESI) [M+H]⁺: 452.2.

Step 5: Intermediate **144-5** (200 mg, 0.44 mmol) was dissolved in dichloromethane (5 mL), and triethylamine (152 mg, 1.32 mmol) and methanesulfonyl chloride (101 mg, 0.88 mmol) were added in sequence. The reaction mixture was stirred at room temperature for half an hour. The reaction was quenched with ice water (20 mL), and the resulting mixture was extracted with dichloromethane (20 mL × 3). The organic phase was washed sequentially with water and saturated brine. The organic phase was dried over anhydrous sodium sulfate, filtered, and concentrated to obtain a crude product. The crude product was purified by silica gel column chromatography to yield Intermediate **144-6** (106 mg, 45.0%). LCMS (ESI) [M+H]⁺: 529.8.

Step 6: Intermediate **144-6** (106 mg, 0.20 mmol) was dissolved in tetrahydrofuran (10 mL), and hydrochloric acid solution (5.0 mL, 6 M) was added. The reaction mixture was stirred at room temperature for 3 hours. After the reaction was completed, the reaction mixture was quenched with saturated aqueous sodium bicarbonate solution. The mixture was extracted with dichloromethane (30 mL × 3). The combined organic phases were washed sequentially with water and saturated brine, dried over anhydrous sodium sulfate, and filtered. The filtrate was concentrated under reduced pressure to obtain a crude product, which was then separated and purified by silica gel column chromatography to obtain a diastereomeric mixture of (3*R*)-4-(6-ethyl-7-(methylsulfonyl)-2-(1*H*-pyrazol-3-yl)-6,7,8,9-tetrahydro-2*H*-1,2,3,7-tetraazabenzo[*cd*]-azulen-4-yl)-3-methylmorpholine (Compound **144**) (35 mg, 39.3%). LCMS (ESI) [M+H]⁺: 446.2.

Step 7: The diastereomeric mixture of (3*R*)-4-(6-ethyl-7-(methylsulfonyl)-2-(1*H*-pyrazol-3-yl)-6,7,8,9-tetrahydro-2*H*-1,2,3,7-tetraazabenzo[*cd*]-azulen-4-yl)-3-methylmorpholine (Compound **144**) (35 mg) was further purified by chiral SFC. SFC analysis method: Column: Daicel ChiralPAK IH (150 × 4.6 mm, 5 µm), mobile phase: 15% methanol in CO₂, temperature: 35°C, flow rate: 2.0 mL/min, detection wavelength: 210 nm.

The first peak was (3*R*)-4-(6-ethyl-7-(methylsulfonyl)-2-(1*H*-pyrazol-3-yl)-6,7,8,9-tetrahydro-2*H*-1,2,3,7-tetraazabenzo[*cd*]-azulen-4-yl)-3-methylmorpholine (Compound **144A,** Isomer 1) (10.2 mg). LCMS (ESI) [M+H]⁺: 446.2. HPLC-SFC: Rt = 1.637 min. ¹H NMR (400 MHz, DMSO-d6) δ 12.76 (s, 1H), 7.81 (s, 1H), 6.76 (s, 2H), 5.10 - 5.04 (m, 1H), 4.45 - 4.40 (m, 1H), 4.08 - 4.03 (m, 1H), 4.00 - 3.95 (m, 2H), 3.78 - 3.74 (m, 1H), 3.66 - 3.60 (m, 2H), 3.52 - 3.45 (m, 1H), 3.32 (s, 1H), 3.20 - 3.15 (m, 1H), 3.11 - 3.06 (m, 1H), 2.89 (s, 3H), 1.93 - 1.83 (m, 2H), 1.20 (d, *J =* 6.4 Hz, 3H), 0.97 (t, *J =* 7.2 Hz, 3H).

The second peak was (3*R*)-4-(6-ethyl-7-(methylsulfonyl)-2-(1*H*-pyrazol-3-yl)-6,7,8,9-tetrahydro-2*H*-1,2,3,7-tetraazabenzo[*cd*]-azulen-4-yl)-3-methylmorpholine (Compound **144B,** Isomer 2) (9.2 mg). LCMS (ESI) [M+H]⁺: 446.2. HPLC-SFC: Rt = 3.161 min. ¹H NMR (400 MHz, DMSO-d6) δ 12.76 (s, 1H), 7.81 (s, 1H), 6.77 - 6.74 (m, 2H), 5.08 - 5.04 (m, 1H), 4.46 - 4.40 (m, 1H), 4.06 - 4.02 (m, 1H), 3.99 - 3.94 (m, 2H), 3.78 - 3.74 (m, 1H), 3.67 - 3.60 (m, 2H), 3.53 - 3.45 (m, 1H), 3.32 (s, 1H), 3.19 - 3.15 (m, 1H), 3.11 (s, 1H), 2.90 (s, 3H), 1.92 - 1.83 (m, 2H), 1.18 (d, *J =* 6.4 Hz, 3H), 0.97 (t, *J =* 7.2 Hz, 3H).

### Example 145A and Example 145B: Preparation of (3R)-4-(7-(cyclopropylsulfonyl)-6-ethyl-2-(1H-pyrazol-3-yl)-6,7,8,9-tetrahydro-2H-1,2,3,7-tetraazabenzo[cd]-azulen-4-yl)-3-methylmorp holine (Compound 145A, Isomer 1) and preparation of (3R)-4-(7-(cyclopropylsulfonyl)-6-ethyl-2-(1H-pyrazol-3-yl)-6,7,8,9-tetrahydro-2H-1,2,3,7-tetraazabenzo[cd]-azulen-4-yl)-3-methylmorp holine (Compound 145B, Isomer 2)

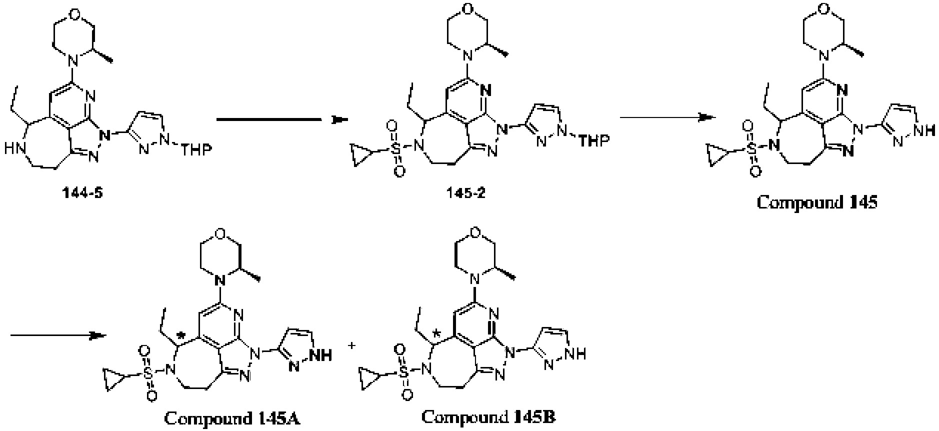

Step 1: Similar to the preparation method of Example 144, the title Compound **145** was prepared using cyclopropanesulfonyl chloride instead of methanesulfonyl chloride. A diastereomeric mixture of (3R)-4-(7-(cyclopropylsulfonyl)-6-ethyl-2-(1H-pyrazol-3-yl)-6,7,8,9-tetrahydro-2*H*-1,2,3,7-tetraazabenzo[*cd*]-azulen-4-yl)-3-methylmorpholine (Compound **145**) (20 mg, 50.0%) was obtained. LCMS (ESI) [M+H]⁺: 472.4.

Step 2: The diastereomeric mixture of (3*R*)-4-(7-(cyclopropylsulfonyl)-6-ethyl-2-(1*H-*pyrazol-3-yl)-6,7,8,9-tetrahydro-2*H*-1,2,3,7-tetraazabenzo[*cd*]-azulen-4-yl)-3-methylmorpholine (Compound **145**) (30 mg) was further purified by chiral SFC. SFC analysis method: Daicel ChiralCEL IH column (150 × 4.6 mm, 5 µm), mobile phase: 15% methanol in n-hexane, flow rate: 1.0 mL/min, detection wavelength: 210/254 nm.

The first peak was (3*R*)-4-(7-(cyclopropylsulfonyl)-6-ethyl-2-(1*H*-pyrazol-3-yl)-6,7,8,9-tetrahydro-2*H*-1,2,3,7-tetraazabenzo[*cd*]-azulen-4-yl)-3-methylmorpholine (Compound **145A,** Isomer 1) (4.5 mg). LCMS (ESI) [M+H]⁺: 472.4. HPLC-SFC: Rt = 1.689 min. ¹H NMR (400 MHz, DMSO-d6) δ 12.74 (s, 1H), 7.81 (s, 1H), 6.76 (s, 1H), 6.78 (s, 1H) 5.04 (dd, *J =* 10.6, 5.0 Hz, 1H), 4.41 (s, 1H), 4.05 (d, *J =* 12.8 Hz, 1H), 3.97 (d, *J =* 12.6 Hz, 2H), 3.76 (d, *J =* 11.3 Hz, 1H), 3.65 (dd, *J* = 16.8, 8.7 Hz, 2H), 3.50 (dd, *J =* 11.5, 9.2 Hz, 1H), 3.13 (t, *J* = 16.6 Hz, 2H), 2.48 - 2.42 (m, 1H), 1.99 - 1.80 (m, 2H), 1.23 (s, 1H), 1.19 (d, *J =* 6.6 Hz, 3H), 0.99 (t, *J =* 7.2 Hz, 3H), 0.97 - 0.93 (m, 1H), 0.89 - 0.78 (m, 2H), 0.66 - 0.55 (m, 1H).

The second peak was (3*R*)-4-(7-(cyclopropylsulfonyl)-6-ethyl-2-(1*H*-pyrazol-3-yl)-6,7,8,9-tetrahydro-2*H*-1,2,3,7-tetraazabenzo[*cd*]-azulen-4-yl)-3-methylmorpholine (Compound **145B,** Isomer 2) (3.5 mg). LCMS (ESI) [M+H]⁺: 472.4. HPLC-SFC: Rt = 2.821 min. ¹H NMR (400 MHz, DMSO-d6) δ 12.74 (s, 1H), 7.81 (s, 1H), 6.76 (s, 1H), 6.77 (s, 1H), 5.03 (dd, *J =* 10.6, 5.3 Hz, 1H), 4.41 (s, 1H), 4.03 (d, *J =* 13.3 Hz, 1H), 4.00 - 3.93 (m, 2H), 3.76 (d, *J =* 11.3 Hz, 1H), 3.72 - 3.59 (m, 2H), 3.55 - 3.44 (m, 1H), 3.13 (t, *J =* 17.2 Hz, 2H), 2.48 - 2.42 (m, 1H), 1.99 - 1.80 (m, 2H), 1.23 (s, 1H), 1.18 (d, *J =* 6.6 Hz, 3H), 1.00 (t, *J =* 7.2 Hz, 3H), 0.97 - 0.93 (m, 1H), 0.92 - 0.82 (m, 2H), 0.72 - 0.64 (m, 1H).

### Example 146: Preparation of (R)-6,6-dimethyl-4-(3-methylmorpholino)-2-(1H-pyrazol-3-yl)-2,6,8,9-tetrahydro-7-oxa-1,2,3-triazabenzo[cd]azulene (Compound 146)

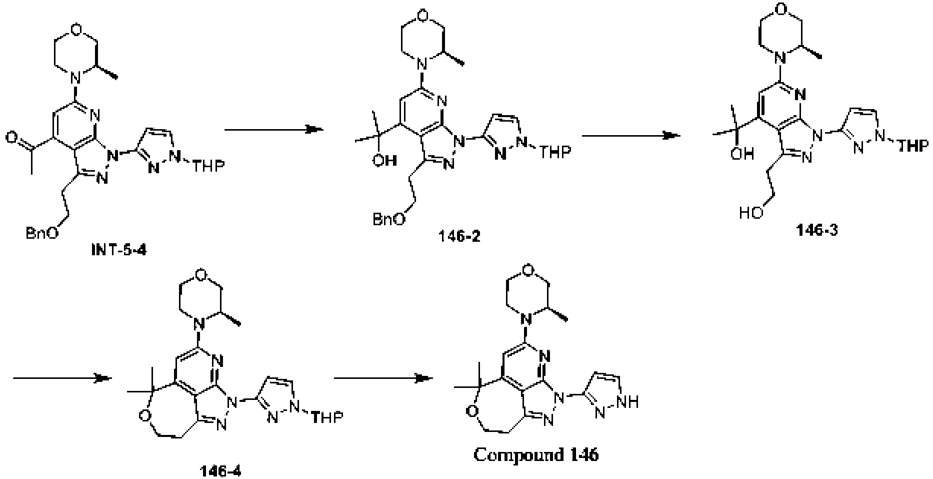

Step 1: Similarly to the preparation method of Intermediate **135-10,** the title Compound **146** was prepared using Intermediate **INT-5-4** instead of Intermediate **135-4,** and methylmagnesium bromide instead of cyclopropylmagnesium bromide. (*R*)-6,6-Dimethyl-4-(3-methylmorpholino)-2-(1*H*-pyrazol-3-yl)-2,6,8,9-tetrahydro-7-oxa-1,2,3-triazabenzo[cd]azulene (Compound **146**) (3.4 mg, 20.9%) was obtained. LCMS(ESI) [M+H]⁺: 369.4. ¹H NMR (400 MHz, CD₃OD) δ 7.72 (s, 1H), 6.91 (s, 1H), 6.48 (s, 1H), 4.56 - 4.48 (m, 1H), 4.17 (t, *J* = 4.8 Hz, 2H), 4.07 (dd, *J =* 13.2, 2.4 Hz, 1H), 4.02 (dd, *J =* 11.2, 3.6 Hz, 1H), 3.82 (d, *J =* 11.2 Hz, 1H), 3.77 (dd, *J =* 11.2, 3.2 Hz, 1H), 3.62 (td, *J =* 12.0, 3.2 Hz, 1H), 3.35 - 3.32 (m, 1H), 3.29 - 3.25 (m, 1H), 3.18 - 3.09 (m, 2H).

### Example 147: Preparation of (R)-4-(3-methylmorpholino)-2-(1H-pyrazol-3-yl)-8,9-dihydro-2H-7-oxa-1,2,3-triazaspiro[benzo[cd]azulene-6,1'-cyclobutane]-1(9a),2a,2al(5a),4-tetraene (Compound 147)

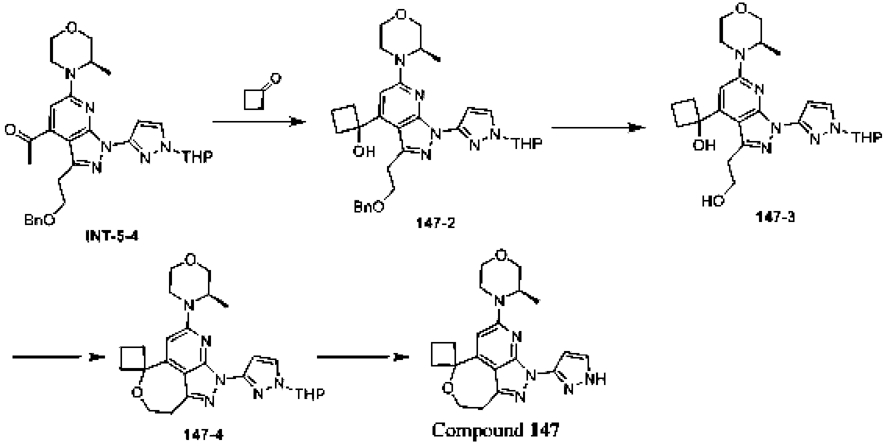

Step 1: Similar to the preparation method of Intermediate **135-10,** the title Compound **147** was prepared using Intermediate **INT-5-4** instead of Intermediate **135-4,** and a solution of cyclobutanone, isopropylmagnesium chloride and lithium chloride in tetrahydrofuran instead of cyclopropylmagnesium bromide. (*R*)-4-(3-Methylmorpholino)-2-(1*H*-pyrazol-3-yl)-8,9-dihydro-2*H*-7-oxa-1,2,3-triazaspiro[benzo[*cd*]azulene-6,1'-cyclobutane]-1(9a),2a,2a1(5a),4-tetraene (Compound **147**) (13.3 mg, 10.8%) was obtained. LCMS(ESI) [M+H]⁺: 381.2. ¹H NMR (400 MHz, DMSO-d6) δ 12.78 (s, 1H), 7.79 (s, 1H), 6.75 (s, 2H), 4.55 - 4.53 (m, 1H), 4.10 - 4.08 (m, 1H), 4.00 - 3.98 (m, 3H), 3.79 - 3.77 (m, 1H), 3.69 - 3.67 (m, 1H), 3.57 - 3.48 (m, 2H), 3.19 - 3.17 (m, 2H), 3.09 - 3.07 (m, 2H), 2.47 - 2.45 (m, 2H), 2.10 - 2.08 (m, 2H), 1.20 (d, *J =* 6.8 Hz, 3H).

### Example 148: Preparation of diastereomeric mixture of 6-(difluoromethyl)-4-((R)-3-methylmorpholino)-2-(1H-pyrazol-3-yl)-2,6,8,9-tetrahydro-7-oxa-1,2,3-triazabenzo[cd]azulene (Compound 148)

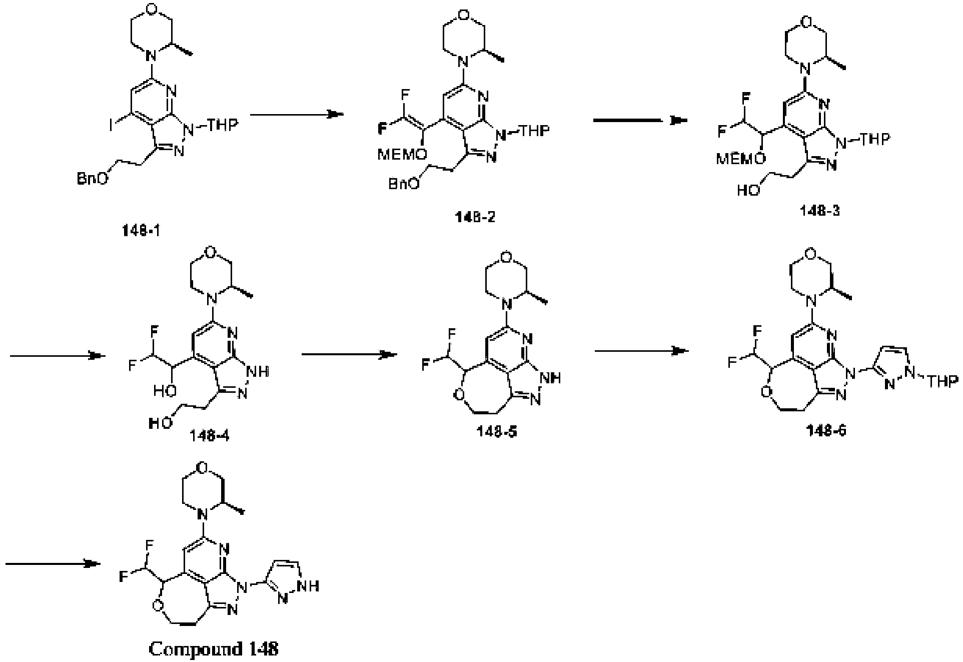

Step 1: Intermediate **148-1** (4.8 g, 8.53 mmol) and trifluoroborate (Cas: 1131736-79-2, 5.1 g, 18.7 mmol) were dissolved in a mixture of dioxane (50 mL) and water (5 mL), and n-butyl-bis(1-adamantyl)phosphine (0.6 g, 1.70 mmol), palladium acetate (0.4 g, 1.70 mmol), and cesium carbonate (8.3 g, 25.6 mmol) were added in sequence. The reaction mixture was stirred at 100°C under a nitrogen atmosphere for 18 hours. After the reaction was completed, the reaction mixture was cooled to room temperature. The mixture was extracted with dichloromethane (50 mL × 3). The combined organic phases were washed sequentially with water and saturated brine, dried over anhydrous sodium sulfate, and filtered. The filtrate was concentrated under reduced pressure to obtain a crude product, which was separated and purified by silica gel column chromatography to yield Intermediate **148-2** (4.0 g, 77.8%). LCMS (ESI) [M+H]⁺: 603.4.

Step 2: Intermediate **148-2** (2.0 g, 3.31 mmol) was dissolved in methanol (40 mL), and wet palladium-on-carbon (1.0 g) and palladium hydroxide-on-carbon (1.0 g) were added. The mixture was stirred at 60°C under a hydrogen atmosphere for 18 hours. After the reaction was completed, the reaction mixture was cooled to room temperature, filtered and concentrated to obtain a crude product. The crude product was separated and purified by silica gel chromatography to yield Intermediate **148-3** (1.7 g, 99.6 %). LCMS (ESI) [M+H]⁺: 515.2.

Step 3: At room temperature, Intermediate **148-3** (1.0 g, 1.94 mmol) was dissolved in tetrahydrofuran (10 mL), added dropwise with aqueous hydrochloric acid (10 mL, 12 M). The reaction mixture was stirred at 25°C for 0.5 h. The reaction was quenched by adding ice water, the pH was adjusted to 8 with saturated sodium bicarbonate solution, and the aqueous phase was extracted with dichloromethane (50 mL × 3). The combined organic phases were washed sequentially with water and saturated brine, dried over anhydrous sodium sulfate, filtered, and concentrated to obtain a crude product. The crude product was purified by silica gel chromatography to yield Intermediate **148-4** (0.4 g, 60.1%). LCMS (ESI) [M+H]⁺: 343.2.

Step 4: Intermediate **148-4** (0.4 g, 1.16 mmol) was dissolved in toluene (10 mL), and cyanomethylenetributylphosphorane (0.84 g, 3.48 mmol) was added. The reaction mixture was stirred at 100°C under a nitrogen atmosphere for 18 hours. The reaction mixture was cooled to room temperature and quenched with ice-cold water. The resulting mixture was extracted with ethyl acetate (20 mL × 3). The organic phase was washed sequentially with water and saturated brine. The organic phase was dried over anhydrous sodium sulfate, filtered, and concentrated to obtain a crude product, which was then separated and purified by silica gel column chromatography to yield Intermediate **148-5** (150 mg, 39.6%). LCMS (ESI) [M+H]⁺: 324.8.

Step 5: Intermediate **148-5** (150 mg, 0.46 mmol) was dissolved in toluene (10 mL), and copper iodide (88 mg, 0.46 mmol), potassium phosphate (295 mg, 1.33 mmol), *N,N*-dimethylethylenediamine (41 mg, 0.46 mmol), and Intermediate **INT-1-7** (386 mg, 1.32 mmol) were added. Under a nitrogen atmosphere, the reaction mixture was stirred at 100°C for 18 hours. The reaction mixture was cooled to room temperature and quenched by the addition of ice-cold water. The organic phase was separated, and the aqueous phase was extracted with dichloromethane (50 mL × 3). The organic phase was washed sequentially with water and saturated brine, dried over anhydrous sodium sulfate, filtered, and concentrated to yield a crude product. The crude product was separated and purified by silica gel chromatography to yield Intermediate **148-6** (60 mg, 27.3%). LCMS (ESI) [M+H]⁺: 475.3.

Step 6: Intermediate **148-6** (60 mg, 0.13 mmol) was dissolved in tetrahydrofuran (2 mL), and aqueous hydrochloric acid (1 mL, 6 M) was added. The reaction mixture was stirred at room temperature for 0.5 hours. The reaction was quenched by the addition of saturated sodium bicarbonate solution, and the aqueous phase was extracted with ethyl acetate (20 mL × 3). The organic phase was washed sequentially with water and saturated brine, dried over anhydrous sodium sulfate, and filtered. The filtrate was concentrated under reduced pressure to yield a crude product. The crude product was separated and purified by reverse-phase high performance liquid chromatography to give a diastereomeric mixture of 6-(difluoromethyl)-4-((*R*)-3-methylmorpholino)-2-(1*H*-pyrazol-3-yl)-2,6,8,9-tetrahydro-7-oxa-1,2,3-triazabenzo[*cd*]azulene (Compound **148**) (1.0 mg, 2.0%). LCMS(ESI) [M+H]⁺: 391.0. ¹H NMR (400 MHz, CD₃OD) δ 7.73 (s, 1H), 6.92 (s, 1H), 6.71 (s, 1H), 6.49 - 6.17 (m, 1H), 5.25 - 5.15 (m, 1H), 4.61 - 4.44 (m, 3H), 4.10 - 4.00 (m, 3H), 3.85 - 3.81 (m, 1H), 3.79 - 3.74 (m, 1H), 3.66 - 3.59 (m, 1H), 3.28 - 3.23 (m, 1H), 3.16 - 3.10 (m, 1H), 1.31 - 1.28 (m, 3H).

### Example 149: Preparation of (R)-3-methyl-4-(6,6,7-trimethyl-2-(1H-pyrazol-3-yl)-6,7,8,9-tetrahydro-2H-1,2,3,7-tetraazabenzo[cd]azulen-4-yl)morpholine (Compound 149)

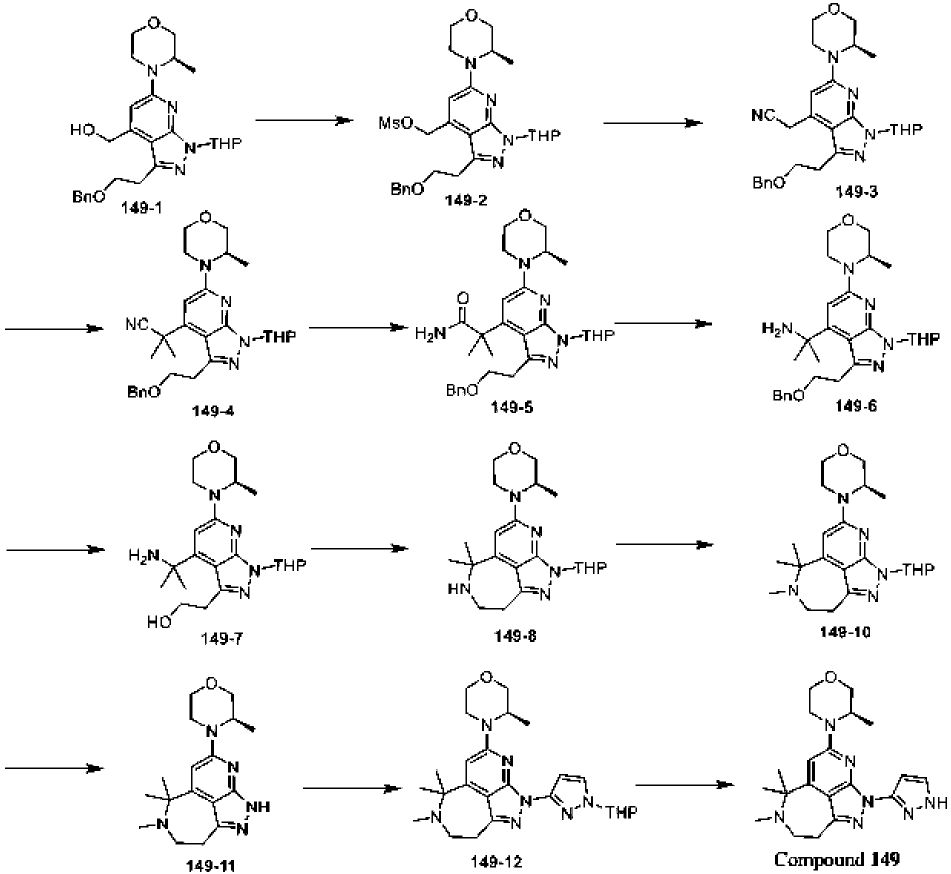

Step 1: Intermediate **149-1** (6.2 g, 13.29 mmol) was dissolved in dichloromethane (60 mL) at room temperature, and triethylamine (3.68 mL, 26.58 mmol) and methanesulfonyl chloride (2.3 g, 19.93 mmol) were added. The reaction mixture was stirred at room temperature for 1 hour. The reaction mixture was directly concentrated under reduced pressure to obtain a crude product. The crude product was separated and purified by silica gel column chromatography to yield Intermediate **149-2** (6.3 g, 87.0%). LCMS (ESI) [M+H]⁺: 545.0.

Step 2: Intermediate **149-2** (6.2 g, 11.38 mmol) was dissolved in acetonitrile (100 mL) at room temperature, and 18-crown-6 (6.0 g, 22.76 mmol) and potassium cyanide (1.1 g, 17.07 mmol) was added. The reaction mixture was reacted at room temperature for 3 hours. When the reaction was completed, the reaction was quenched by adding ice water. The resulting mixture was extracted with ethyl acetate (50 mL × 3). The combined organic phases were washed sequentially with water and saturated brine, dried over anhydrous sodium sulfate, and filtered. The filtrate was concentrated under reduced pressure to obtain a crude product, which was then purified by silica gel column chromatography to yield Intermediate **149-3** (3.1 g, 57.3%). LCMS (ESI) [M+H]⁺: 476.2.

Step 3: Intermediate **149-3** (2.9 g, 6.10 mmol) was dissolved in anhydrous tetrahydrofuran (50 mL), and sodium hydride (0.7 g, 18.29 mmol) was added under an ice-water bath. The reaction mixture was stirred at 0°C for 0.5 hour, and iodomethane (4.3 g, 30.49 mmol) was added. The reaction mixture was continuously stirred for 1 hour, and then ice water (50 mL) was added to quench the reaction. The resulting mixture was extracted with ethyl acetate (50 mL × 3). The combined organic phases were washed sequentially with water and saturated brine, dried over anhydrous sodium sulfate, and filtered. The filtrate was concentrated under reduced pressure to obtain a crude product, which was separated and purified by silica gel column chromatography to yield Intermediate **149-4** (2.7 g, 87.9%). LCMS (ESI) [M+H]⁺: 504.2.

Step 4: Intermediate **149-4** (2.9 g, 6.10 mmol) was dissolved in ethanol/water (1:1, v/v, 100 mL), and sodium hydroxide (1.9 g, 48.65 mmol) was added. The reaction mixture was stirred at 100°C for 16 hours. The reaction mixture was cooled to room temperature, and the ethanol was partially removed under reduced pressure. The remaining mixture was extracted with ethyl acetate (100 mL × 3). The combined organic phases were washed sequentially with water and saturated brine, dried over anhydrous sodium sulfate, and filtered. The filtrate was concentrated under reduced pressure to obtain a crude product, which was separated and purified by silica gel column chromatography to yield Intermediate **149-5** (3.6 g, 70.9%). LCMS (ESI) [M+H]⁺: 522.4.

Step 5: Intermediate **149-5** (900 mg, 1.73 mmol) and sodium hydroxide (380 mg, 9.49 mmol) were dissolved in acetonitrile/water (5:3, V/V, 12 mL). The reaction mixture was cooled to 0°C, and trichloroisocyanuric acid (136 mg, 0.59 mmol) was added. The reaction mixture was stirred at 0°C for 0.5 hours, then heated to 80°C and stirred for 0.5 hours. The reaction mixture was cooled to room temperature and extracted with dichloromethane (20 mL × 3). The combined organic phases were washed sequentially with water and saturated brine, dried over anhydrous sodium sulfate, and filtered. The filtrate was concentrated under reduced pressure to obtain a crude product, which was purified by silica gel column chromatography to yield Intermediate **149-6** (550 mg, 64.6%). LCMS (ESI) [M+H]⁺: 494.2.

Step 6: Intermediate **149-6** (550 mg, 1.11 mmol) was dissolved in ethanol (10 mL), and 10% palladium-on-carbon (containing approximately 50% water) (30 mg) and palladium hydroxide (30 mg) were added in sequence. The reaction mixture was stirred at 80°C under a hydrogen atmosphere for 18 hours. The reaction mixture was cooled to room temperature and filtered through diatomite. The filtrate was concentrated to yield Intermediate **149-7** (435 mg, 96.8%). LCMS (ESI) [M+H]⁺: 404.0.

Step 7: Similar to the preparation of Example 148, the title Compound **149** was prepared using Intermediate **149-6** instead of Intermediate **148-4.** (*R*)-3-Methyl-4-(6,6,7-trimethyl-2-(1*H*-pyrazol-3-yl)-6,7, 8,9-tetrahydro-2*H*-1,2,3,7-tetraazabenzo[*cd*]azulen-4-yl)morpholine (Compound **149**) (110 mg, 64.0%) was obtained. LCMS(ESI) [M+H]⁺: 382.0. ¹H NMR (400 MHz, DMSO-d6) δ 10.59 (s, 1H), 7.83 (d, *J =* 2.4 Hz, 1H), 6.76 (d, *J =* 2.4 Hz, 1H), 6.73 (s, 1H), 4.53 (d, *J =* 5.6 Hz, 1H), 4.10 (d, *J =* 12.0 Hz, 2H), 3.99 (dd, *J =* 11.2, 3.2 Hz, 1H), 3.78 (d, *J =* 11.2 Hz, 1H), 3.65 (d, *J =* 8.8 Hz, 2H), 3.48 (dt, *J =* 10.4, 8.4 Hz, 2H), 3.33 (s, 1H), 3.19 (s, 1H), 2.89 (s, 3H), 1.98 (s, 3H), 1.77 (s, 3H), 1.20 (d, *J =* 6.7 Hz, 3H).

### Example 150: Preparation of diastereomeric mixture of 6-methyl-4-((R)-3-methylmorpholino)-2-(1H-pyrazol-3-yl)-2,6,8,9-tetrahydro-7H-1,2,3,7-tetraazabenzene[cd]azu lene-7-sulfonamide (Compound 150)

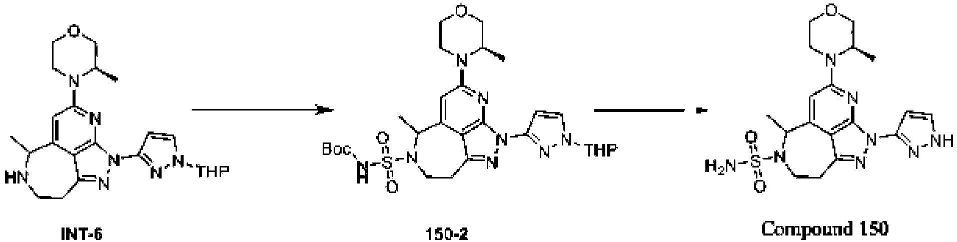

Step 1: Under a nitrogen atmosphere, chlorosulfonyl isocyanate (32.4 mg, 0.23 mmol) and tert-butanol (2.5 mL) were dissolved in dichloromethane (4.0 mL). The reaction mixture was stirred at -10°C for 0.5 hour, and then a solution of Intermediate **INT-6** (100 mg, 0.23 mmol) and *N,N*-diisopropylethylamine (29.6 mg, 0.23 mmol) in dichloromethane (1.0 mL) was added. The reaction mixture was stirred at -10°C for 2 hours. After the reaction was completed, saturated ammonium chloride (20 mL) was added to quench the reaction, and the aqueous phase was extracted with dichloromethane (20 mL × 3). The combined organic phases were washed sequentially with water and saturated brine, dried over anhydrous sodium sulfate, filtered, and concentrated to obtain a crude product, which was purified by silica gel column chromatography to yield Intermediate **150-2** (60 mg, 42.6%). LCMS (ESI) [M+H]⁺: 617.2.

Step 2: Intermediate **150-2** (55 mg, 0.09 mmol) was dissolved in tetrahydrofuran (1 mL), and hydrochloric acid (1 mL, 2 M) was added. The reaction mixture was stirred at room temperature for 0.5 hour. The reaction mixture was quenched with saturated aqueous sodium bicarbonate. The mixture was extracted with dichloromethane (30 mL × 3). The combined organic phases were washed sequentially with water and saturated brine, dried over anhydrous sodium sulfate, and filtered. The filtrate was concentrated under reduced pressure to obtain a crude product, which was separated and purified by silica gel column chromatography to give a diastereomeric mixture of 6-methyl-4-((*R*)-3-methylmorpholino)-2-(1*H*-pyrazol-3-yl)-2,6,8,9-tetrahydro-7*H*-1,2,3,7-tetraazabenzene[*cd*]azulene-7-sulfonamide (Compound **150**) (5.5 mg, 14.3%). LCMS (ESI) [M+H]⁺: 433.2. ¹H NMR (400 MHz, DMSO-d6) δ 12.74 (s, 1H), 7.81 (s, 1H), 6.90 (s, 2H), 6.77 (s, 1H), 6.69 - 6.67 (m, 1H), 5.39 - 5.37 (m, 1H), 4.48 - 4.46 (m, 1H), 4.06 - 4.04 (m, 1H), 3.97 - 3.95 (m, 1H), 3.90 - 3.88 (m, 1H), 3.80 - 3.73 (m, 1H), 3.66 - 3.64 (m, 1H), 3.61 - 3.59 (m, 1H), 3.51 - 3.49 (m, 1H), 3.32 - 3.20 (m, 1H), 3.20 - 3.11 (m, 1H), 3.03 - 3.01 (m, 1H), 1.57 (d, *J =* 7.2 Hz, 3H), 1.20 - 1.18 (m, 3H).

### Example 151: Preparation of isomeric mixture of (3R)-4-(7-((2-fluorocyclopropyl) sulfonyl)-6-methyl-2-(1H-pyrazol-3-yl)-6,7,8,9-tetrahydro-2H-1,2,3,7-tetraazabenzo[cd]azulen-4 -yl)-3-methylmorpholine (Compound 151)

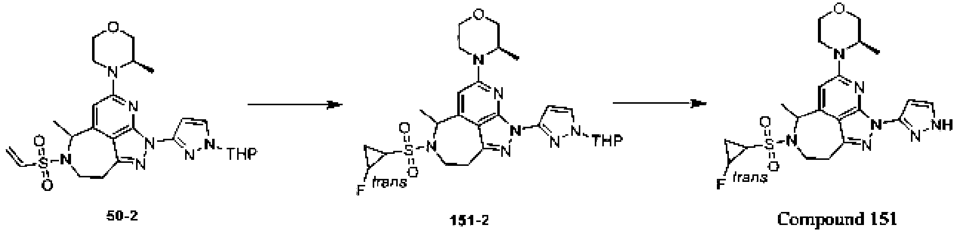

Step 1: Intermediate **50-2** (500 mg, 0.95 mmol) was dissolved in anhydrous tetrahydrofuran (10 mL), and fluoromethyl-phenyl-(2,3,4,5-tetramethylphenyl)sulfonium tetrafluoroborate (1.37 g, 3.79 mmol) was added at 0°C. The reaction mixture was stirred at 0°C for 10 minutes, and then sodium hydride (152 mg, 3.79 mmol) was added. After the mixture was reacted at room temperature for 24 hours, fluoromethyl-phenyl-(2,3,4,5-tetramethylphenyl)sulfonium tetrafluoroborate (687 mg, 1.90 mmol) and sodium hydride (152 mg, 3.79 mmol) were further added. The mixture was reacted at room temperature for 12 hours, filtered, and the filter cake was washed with tetrahydrofuran (10 mL × 3). The filtrate was spin-dried and concentrated to obtain a crude product, which was purified by silica gel column chromatography to give Intermediate **151-2** (80mg, 15.1%). LCMS (ESI) [M+H]⁺: 560.2.

Step 2: Intermediate **151-2** (80 mg, 0.14 mmol) was dissolved in methanol (1 mL), and hydrochloric acid (1 mL, 1 M) was added. The reaction mixture was stirred at room temperature for 2 hours. The reaction mixture was spin-dried and concentrated to obtain a crude product, which was then separated and purified by silica gel column chromatography to obtain an isomeric mixture of (3*R*)-4-(7-((2-fluorocyclopropyl)sulfonyl)-6-methyl-2-(1*H*-pyrazol-3-yl)-6,7,8,9-tetrahydro-2*H*-1 ,2,3,7-tetraazabenzo[*cd*]azulen-4-yl)-3-methylmorpholine (Compound **151**) (60 mg, 88.3%). LCMS (ESI) [M+H]⁺: 476.4.

Step 3: The isomeric mixture of (3*R*)-4-(7-((2-fluorocyclopropyl)sulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorp holine (Compound **151**) (60 mg) was further purified by chiral SFC. SFC analysis method: Daicel ChiralCel ICS column (150 × 4.6 mm, 5 µm), mobile phase: 15% methanol in n-hexane, flow rate: 1.0 mL/min, detection wavelength: 210/254 nm.

The first peak was (3*R*)-4-(7-((2-fluorocyclopropyl)sulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **151A,** Isomer 1) (7 mg). LCMS (ESI) [M+H]⁺: 476.4. HPLC-SFC: Rt = 6.774 min. ¹H NMR (400 MHz, CD₃OD) δ 7.71 (s, 1H), 6.91 (s, 1H), 6.65 (s, 1H), 5.49 - 5.42 (m, 1H), 5.05 - 4.89 (m, 1H), 4.54 - 4.45 (m, 1H), 4.15 - 3.99 (m, 3H), 3.92 - 3.74 (m, 3H), 3.67 - 3.56 (m, 1H), 3.51 - 3.38 (m, 1H), 3.26 - 3.17 (m, 1H), 3.01 - 2.88 (m, 1H), 1.69 (d, *J =* 7.1 Hz, 3H), 1.64 - δ 1.42 (m, 3H), 1.29 (d, *J =* 7.5 Hz, 3H), 0.94 - 0.84 (m, 1H).

The second peak was (3*R*)-4-(7-((2-fluorocyclopropyl)sulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **151B,** Isomer 2) (7 mg). LCMS (ESI) [M+H]⁺: 476.4. HPLC-SFC: Rt = 7.559 min. ¹H NMR (400 MHz, CD₃OD) δ 7.72 (s, 1H), 6.91 (s, 1H), 6.64 (s, 1H), 5.46 (q, *J =* 7.3 Hz, 1H), 5.07 - 4.89 (m, 1H), 4.54 - 4.47 (m, 1H), 4.16 - 3.98 (m, 3H), 3.92 - 3.73 (m, 3H), 3.67 - 3.56 (m, 1H), 3.45 - 3.37 (m, 1H), 3.21 (d, *J =* 17.1 Hz, 1H), 3.05 - 2.91 (m, 1H), 1.69 (d, *J =* 7.1 Hz, 3H), 1.66 - 1.41 (m, 3H), 1.28 (d, *J =* 6.7 Hz, 3H), 0.93 - 0.84 (m, 1H).

The third peak was (3*R*)-4-(7-((2-fluorocyclopropyl)sulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **151C,** Isomer 3) (6 mg). LCMS (ESI) [M+H]⁺: 476.4. HPLC-SFC: Rt = 8.420 min. ¹H NMR (400 MHz, CD₃OD) δ 7.72 (s, 1H), 6.91 (s, 1H), 6.64 (s, 1H), 5.50 (q, *J =* 7.1 Hz, 1H), 5.15 - 4.92 (m, 1H), 4.55 - 4.46 (m, 1H), 4.14 - 3.99 (m, 3H), 3.91 - 3.73 (m, 3H), 3.67 - 3.58 (m, 1H), 3.46 - 3.38 (m, 1H), 3.20 (d, *J =* 17.0 Hz, 1H), 3.12 - 3.01 (m, 1H), 1.70 (d, *J =* 7.1 Hz, 3H), 1.60 - 1.40 (m, 3H), 1.30 (d, *J =* 6.7 Hz, 3H), 0.93 - 0.84 (m, 1H).

The fourth peak was (3*R*)-4-(7-((2-fluorocyclopropyl)sulfonyl)-6-methyl-2-(1*H*-pyrazol-3-yl)-6,7,8,9-tetrahydro-2*H*-1,2,3,7-tetraazabenzo[*cd*]azulen-4-yl)-3-methylmorpholine (Compound **151D,** Isomer 4) (6 mg). LCMS (ESI) [M+H]⁺: 476.4. HPLC-SFC: Rt = 10.205 min. ¹H NMR (400 MHz, CD₃OD) δ 7.72 (s, 1H), 6.91 (s, 1H), 6.63 (s, 1H), 5.50 (q, *J =* 7.2 Hz, 1H), 5.13 - 4.93 (m, 1H), 4.55 - 4.46 (m, 1H), 4.14 - 3.97 (m, 3H), 3.91 - 3.74 (m, 3H), 3.67 - 3.58 (m, 1H), 3.45 - 3.34 (m, 1H), 3.19 (d, *J =* 17.4 Hz, 1H), 3.14 - 3.02 (m, 1H), 1.70 (d, *J =* 7.1 Hz, 3H), 1.63 - 1.41 (m, 3H), 1.28 (d, *J =* 6.6 Hz, 3H), 0.93 - 0.83 (m, 1H).

### Example 152A and Example 152B: Preparation of (3R)-4-(7-(N,S-dimethylsulfonylimino)-6-methyl-2-(1H-pyrazol-3-yl)-6,7,8,9-tetrahydro-2H-1,2,3,7-tetraazabenzo[cd]azulen-4-yl)-3-me thylmorpholine (Compound 152A, Isomer Mixture 1) and preparation of (3R)-4-(7-(N,S-dimethylsulfonylimino)-6-methyl-2-(1H-pyrazol-3-yl)-6,7,8,9-tetrahydro-2H-1,2, 3,7-tetraazabenzo[cd]azulen-4-yl)-3-methylmorpholine (Compound 152B, Isomer Mixture 2)

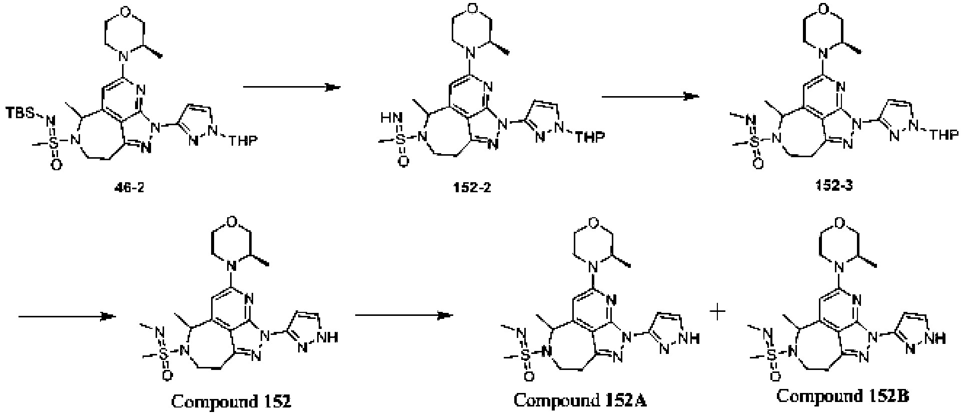

Step 1: Intermediate **46-2** (220 mg, 0.35 mmol) was dissolved in tetrahydrofuran (5 mL), and tetrabutylammonium fluoride (137 mg, 0.53 mmol) was added in sequence. The reaction mixture was stirred at room temperature for 1 hour. After the reaction was completed, saturated aqueous ammonium chloride (20 mL) was added to quench the reaction, and the aqueous phase was extracted with ethyl acetate (30 mL × 3). The combined organic phases were washed sequentially with water and saturated brine, dried over anhydrous sodium sulfate, filtered, and concentrated to obtain a crude product, which was purified by silica gel column chromatography to yield Intermediate **152-2** (140 mg, 77.8%). LCMS (ESI) [M+H]⁺: 515.4.

Step 2: Intermediate **152-2** (120 mg, 0.23 mmol) was dissolved in anhydrous tetrahydrofuran (5 mL), and sodium hydride (28 mg, 0.7 mmol) was added under ice-water bath. The reaction mixture was stirred at 0°C for 0.5 hour, and then iodomethane (67 mg, 0.47 mmol) was added. The reaction mixture was stirred for another hour and then quenched with saturated aqueous ammonium chloride (20 mL). The resulting mixture was extracted with ethyl acetate (30 mL × 3). The combined organic phases were washed sequentially with water and saturated brine, dried over anhydrous sodium sulfate, and filtered. The filtrate was concentrated under reduced pressure to obtain a crude product, which was then separated and purified by silica gel column chromatography to yield Intermediate **152-3** (80 mg, 66.7%). LCMS (ESI) [M+H]⁺: 529.5.

Step 3: Intermediate **152-3** (80 mg, 0.15 mmol) was dissolved in a solution of hydrochloric acid in ethyl acetate (5.0 mL, 4 M) at 0°C. The reaction mixture was stirred at room temperature for 1 hour. The reaction mixture was concentrated to yield a crude product. The crude product was separated and purified by preparative reverse-phase high-performance liquid chromatography to yield isomeric mixtures.

The first peak was an isomeric mixture of (3*R*)-4-(7-(*N*,*S*-dimethylsulfonylimino)-6-methyl-2-(1*H*-pyrazol-3-yl)-6,7,8,9-tetrahydro-2*H*-1,2,3,7-tetraazabenzo[*cd*]azulen-4-yl)-3-methylmorp holine (Compound **152A,** isomeric mixture 1) (14 mg). LCMS (ESI) [M+H]⁺: 445.4. ¹H NMR (400 MHz, CD₃OD) δ 7.73 (s, 1H), 6.92 (s, 1H), 6.67 (s, 1H), 5.32 (q, *J =* 7.2 Hz, 1H), 4.51 (d, *J* = 6.6 Hz, 1H), 4.16 - 3.97 (m, 3H), 3.92 - 3.79 (m, 2H), 3.76 (d, *J =* 11.4 Hz, 1H), 3.65 - 3.57 (m, 1H), 3.48 - 3.39 (m, 1H), 3.36 - 3.31 (m, 1H), 3.18 (d, *J* = 17.8 Hz, 1H), 2.71 (s, 3H), 2.64 (s, 3H), 1.69 (d, *J* = 7.1 Hz, 3H), 1.29 (dd, *J =* 6.6, 4.0 Hz, 3H).

The second peak was an isomeric mixture of (3*R*)-4-(7-(*N,S-*dimethylsulfonylimino)-6-methyl-2-(1*H*-pyrazol-3-yl)-6,7,8,9-tetrahydro-2*H*-1,2,3,7-tetraazabenzo[*cd*]azulen-4-yl)-3-meth ylmorpholine (Compound **152B,** isomeric mixture 2) (5 mg). LCMS (ESI) [M+H]⁺: 445.4. ¹H NMR (400 MHz, CD₃OD) δ 7.71 (s, 1H), 6.90 (s, 1H), 6.64 (s, 1H), 5.38 (q, *J* = 7.0 Hz, 1H), 4.55 - 4.45 (m, 1H), 4.16 - 3.91 (m, 3H), 3.87 - 3.69 (m, 3H), 3.61 (dd, *J* = 16.6, 7.2 Hz, 1H), 3.35 - 3.30 (m, 1H), 3.28 - 3.25 (m, 1H), 3.18 (d, *J =* 17.2 Hz, 1H), 3.01 (s, 3H), 2.29 (d, *J =* 2.6 Hz, 3H), 1.68 - 1.57 (m, 3H), 1.29 (d, *J =* 6.6 Hz, 3H).

### Example 153: Preparation of isomeric mixture of (3R)-4-(7-(N-cyclopropyl-S-methylsulfonylimino)-6-methyl-2-(1H-pyrazol-3-yl)-6,7,8,9-tetrahy dro-2H-1,2,3,7-tetraazabenzo[cd]azulen-4-yl)-3-methylmorpholine (Compound 153)

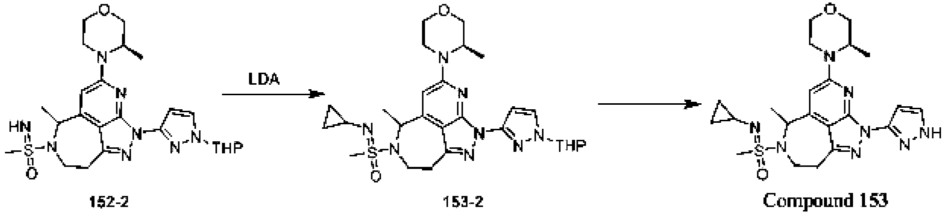

Step 1: Similar to the preparation method of Example 152, the title Compound 153 was prepared using cyclopropane bromide instead of iodomethane. An isomeric mixture of (3*R*)-4-(7-(*N*-cyclopropyl-*S*-methylsulfonylimino)-6-methyl-2-(1*H*-pyrazol-3-yl)-6,7,8,9-tetrahy dro-2*H*-1,2,3,7-tetraazabenzo[*cd*]azulen-4-yl)-3-methylmorpholine (Compound **153**) (4.0 mg, 23.6%) was obtained. LCMS (ESI) [M+H]⁺: 471.3. ¹H NMR (400 MHz, DMSO-d6) δ 12.45 (s, 1H), 7.47 - 7.29 (m, 1H), 6.69 (s, 1H), 6.40 (s, 1H), 5.60 - 5.47 (m, 1H), 4.43 (s, 1H), 4.09 - 3.89 (m, 3H), 3.88 - 3.73 (m, 2H), 3.69 - 3.55(m, 2H), 3.50 (t, *J =* 11.8 Hz, 1H), 3.17 (d, *J =* 11.6 Hz, 1H), 3.07 - 2.97 (m, 1H), 2.85 - 2.78 (m, 3H), 2.02 - 1.93 (m, 1H), 1.58 - 1.51 (m, 3H), 1.21 - 1.16 (m, 3H), 0.96 (d, *J =* 6.4 Hz, 2H), 0.77 - 0.71 (m, 2H).

### Example 154: Preparation of (R)-6-(1-methylcyclopropyl)-4-(3-methylmorpholino)-2-(1H-pyrazol-3-yl)-8,9-dihydro-1,2a,3,6-tetraazabenzo[cd]azulene-7(6H)-one (Compound 154)

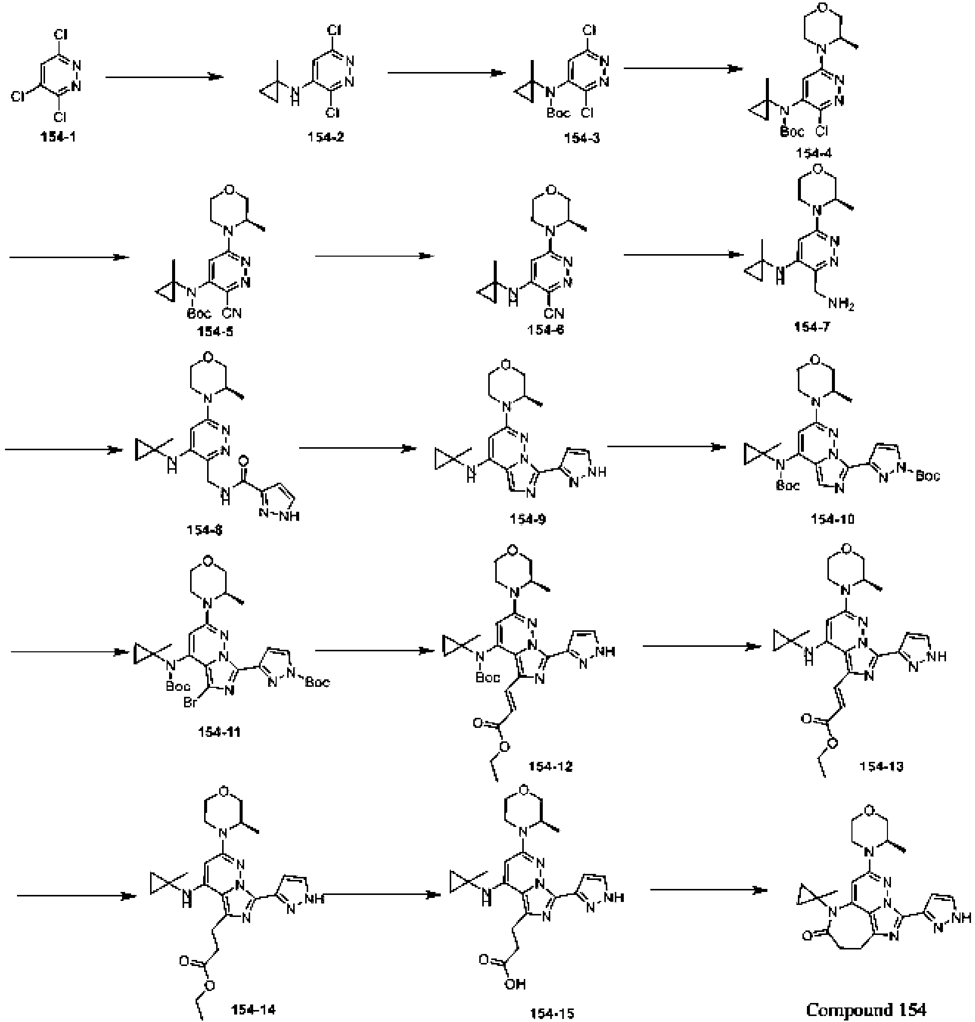

Step 1: 3,4,6-Trichloropyridazine (1.83 g, 9.98 mmol) was dissolved in acetonitrile (40 mL), and 1-methylcyclopropylamine hydrochloride (2.10 g, 192.2 mmol) and N,N-diisopropylethylamine (2.60 g, 19.96 mmol) were added. The reaction mixture was heated to 50°C under a nitrogen atmosphere and stirred for 3 hours. The reaction mixture was directly concentrated to obtain the crude product, which was then separated and purified by silica gel column chromatography to yield Intermediate **154-2** (1.10 g, 66.7%). LCMS (ESI) [M+H]⁺: 218.0.

Step 2: Intermediate **154-2** (90 mg, 0.41 mmol) was dissolved in tetrahydrofuran (5 mL), and 4-dimethylaminopyridine (101 mg, 0.83 mmol) and di-tert-butyl dicarbonate (180 mg, 0.83 mmol) were added in sequence. The reaction mixture was stirred at 70°C under a nitrogen atmosphere for 18 hours. The reaction mixture was cooled to room temperature. The reaction mixture was concentrated under reduced pressure to obtain the crude product. The crude product was separated and purified by silica gel chromatography to yield Intermediate 154-3 (110 mg, 83.8%). LCMS (ESI) [M+H]⁺: 318.2.

Step 3: Intermediate 154-3 (50 mg, 0.16 mmol) was dissolved in toluene (2 mL). N,N-diisopropylethylamine (40.6 mg, 0.32 mmol), cuprous bromide (11.3 mg, 0.08), (R)-3-methylmorpholine (28.2 mg, 0.08 mmol), and palladium acetate (3.6 mg, 0.016 mmol) were added sequentially. The reaction mixture was stirred at 130°C under a nitrogen atmosphere for 16 hours. The reaction mixture was cooled to room temperature and concentrated under reduced pressure to obtain a crude product. The crude product was separated and purified by silica gel chromatography to yield Intermediate **154-4** (20 mg, 33.2%). LCMS (ESI) [M+H]⁺: 383.2.

Step 4: Intermediate **154-4** (80 mg, 0.21 mmol) was dissolved in dioxane/water (4:1, V/V, 3 mL), and 1,1'-binaphthyl-2,2'-bis(diphenylphosphine) (26 mg, 0.042 mmol), sodium carbonate (44.3 mg, 0.42 mmol), potassium ferrocyanide (38.6 mg, 0.105 mmol), and allylpalladium chloride (7.6 mg, 0.021 mmol) were added sequentially. The reaction mixture was stirred at 90°C under a nitrogen atmosphere for 16 hours. The reaction mixture was cooled to room temperature and quenched with ice-cold water. The resulting mixture was extracted with ethyl acetate (20 mL × 3). The organic phase was washed sequentially with water and saturated brine. The organic phase was dried over anhydrous sodium sulfate, filtered, and concentrated to obtain a crude product, which was separated and purified by silica gel column chromatography to yield Intermediate **154-5** (51 mg, 65.4%). LCMS (ESI) [M+H]⁺: 374.0.

Step 5: Intermediate **154-5** (6.7 g, 17.94 mmol) was dissolved in dichloromethane (70 mL), and 2,6-lutidine (7.7 g, 71.75 mmol) and trimethylsilyl iodide (5.0 g, 35.88 mmol) were added sequentially. The reaction mixture was stirred at room temperature for 1 hour. The reaction mixture was directly concentrated to obtain a crude product. The crude product was separated and purified by silica gel column chromatography to yield Intermediate **154-6** (3.0 g, 61.2%). LCMS (ESI) [M+H]⁺: 274.4.

Step 6: Intermediate **154-6** (3.0 g, 10.98 mmol) was dissolved in methanolic ammonia (40 mL, 7 M), and Raney nickel (0.6 g) was added. The mixture was stirred at room temperature under a hydrogen atmosphere for 3 days. After the reaction was completed, the reaction mixture was filtered through diatomite. The filtrate was concentrated to yield Intermediate **154-7** (2.8 g, 92.0%). LCMS (ESI) [M+H]⁺: 278.2.

Step 7: Intermediate **154-7** (10 mg, 0.036 mmol) was dissolved in dichloromethane (1 mL), and freshly prepared pyrazole-3-carboxylic acid N-hydroxysuccinimide ester (7.5 mg, 0.036 mmol) and triethylamine (0.1 mL) were added sequentially. The reaction mixture was stirred at room temperature for 0.5 hours. The reaction mixture was directly concentrated to yield a crude product. The crude product was separated and purified by silica gel column chromatography to yield Intermediate **154-8** (2.0 mg, 14.9%). LCMS (ESI) [M+H]⁺: 372.3.

Step 8: Intermediate **154-8** (300 mg, 0.81 mmol) was dissolved in dichloromethane (6 mL), and phosphorus oxychloride (0.75 mL, 8.04 mmol) was added. The reaction mixture was stirred at 80°C under a nitrogen atmosphere for 12 hours. The reaction mixture was directly concentrated to obtain a crude product. The crude product was separated and purified by silica gel column chromatography and preparative high-performance liquid chromatography to yield Intermediate **154-9** (150 mg, 52.5%). LCMS (ESI) [M+H]⁺: 354.2.

Step 9: Intermediate **154-9** (310 mg, 0.88 mmol) was dissolved in tetrahydrofuran (10 mL), and 4-dimethylaminopyridine (322 mg, 2.63 mmol) and di-tert-butyl dicarbonate (957 mg, 4.39 mmol) were added sequentially. The reaction mixture was stirred at 75°C under a nitrogen atmosphere for 3 hours. The reaction mixture was cooled to room temperature. The reaction mixture was concentrated under reduced pressure to obtain a crude product. The crude product was separated and purified by silica gel chromatography to yield Intermediate **154-10** (280 mg, 44.7%). LCMS (ESI) [M+H]⁺: 554.2.

Step 10: Intermediate **154-10** (38 mg, 0.069 mmol) was dissolved in dimethyl sulfoxide (1 mL), and dibromohydantoin (29.4 mg, 0.103 mmol) was added. The reaction mixture was stirred at 35°C for 1 hour under a nitrogen atmosphere. After the reaction was completed, ice water was added to quench the reaction. The resulting mixture was extracted with ethyl acetate (10 mL × 3). The combined organic phases were washed sequentially with water and saturated brine, dried over anhydrous sodium sulfate, and filtered. The filtrate was concentrated under reduced pressure to obtain a crude product, which was separated and purified by silica gel chromatography to yield Intermediate **154-11** (25 g, 57.6%). LCMS (ESI) [M+H]⁺: 632.4.

Step 11: Intermediate **154-11** (50 mg, 0.079 mmol) and (E)-2-(ethoxycarbonyl)vinylboronic acid pinacol ester (35.7 mg, 0.158 mmol) were dissolved in a mixture of tetrahydrofuran and water (4:1, v/v, 5 mL). [n-Butyldi(1-adamantyl)phosphine](2-amino-1,1'-biphenyl-2-yl) palladium(II) methanesulfonate (5.8 mg, 0.008 mmol) and potassium phosphate (50.3 mg, 0.237 mmol) were added sequentially. The reaction mixture was stirred at 100°C under a nitrogen atmosphere for 16 hours. After the reaction was completed, the reaction mixture was cooled to room temperature, and ice water was added to quench the reaction. The resulting mixture was extracted with ethyl acetate (20 mL × 3). The combined organic phases were washed sequentially with water and saturated brine, dried over anhydrous sodium sulfate, and filtered. The filtrate was concentrated under reduced pressure to yield a crude product, which was separated and purified by silica gel chromatography to yield Intermediate **154-12** (8 mg, 18.3%). LCMS (ESI) [M+H]⁺: 552.2.

Step 12: Intermediate **154-12** (315 mg, 0.57 mmol) was dissolved in methanol (2 mL). Hydrochloric acid (2.9 mL, 2 M) was added, and the reaction mixture was stirred at room temperature for 16 hours. Ammonia was added to quench the reaction, and the resulting mixture was extracted with ethyl acetate (20 mL × 3). The combined organic phases were washed sequentially with water and saturated brine, dried over anhydrous sodium sulfate, and filtered. The filtrate was concentrated under reduced pressure to obtain a crude product, which was separated and purified by silica gel chromatography to yield Intermediate **154-13** (120 mg, 43.1%). LCMS (ESI) [M+H]⁺: 452.2.

Step 13: Intermediate **154-13** (120 mg, 0.266 mmol) was dissolved in ethyl acetate (3 mL), and palladium-on-carbon (12 mg) was added. The mixture was stirred at room temperature under a hydrogen atmosphere for 3 days. After the reaction was completed, the reaction mixture was filtered and concentrated to yield crude product Intermediate **154-14** (92 mg, 76.3%). LCMS (ESI) [M+H]⁺: 454.2.

Step 14: Intermediate **154-14** (92 mg, 0.203 mmol) was dissolved in methanol (2 mL), and sodium hydroxide (0.81 mL, 0.5 M) was added. The reaction mixture was stirred at room temperature for 0.5 hours. The reaction mixture was directly concentrated under reduced pressure to yield crude Intermediate **154-15** (61 mg, 70.7%). LCMS (ESI) [M+H]⁺: 426.2.

Step 15: Intermediate **154-15** (31 mg, 0.073 mmol) was dissolved in phosphorus oxychloride (1 mL), and pyridine (11.5 mg, 0.146 mmol) was added. The reaction mixture was stirred at room temperature under a nitrogen atmosphere for 3 hours. Aqueous ammonia was added to quench the reaction, and the resulting mixture was extracted with ethyl acetate (10 mL × 3). The combined organic phases were washed sequentially with water and saturated brine, dried over anhydrous sodium sulfate, and filtered. The filtrate was concentrated under reduced pressure to obtain a crude product, which was then separated and purified by preparative reverse-phase high performance liquid chromatography to yield (*R*)-6-(1-methylcyclopropyl)-4-(3-methylmorpholino)-2-(1*H*-pyrazol-3-yl)-8,9-dihydro-1,2*a*,3,6-tetraazabenzo[*cd*]azulene-7(6*H* )-one (Compound **154**) (1.9 mg, 6.4%). LCMS(ESI) [M+H]⁺: 408.4. ¹H NMR (400 MHz, CD₃OD) δ 7.82 (s, 1H), 7.25 (s, 1H), 6.64 (d, *J =* 4.0 Hz, 1H), 4.33 - 4.25 (m, 1H), 4.07 (dd, *J =* 11.6, 3.6 Hz, 1H), 3.96 - 3.83 (m, 3H), 3.70 (t, *J =* 11.6 Hz, 1H), 3.48 - 3.43 (m, 1H), 3.14 - 3.05 (m, 2H), 3.03 - 2.93 (m, 2H), 1.56 (d, *J =* 8.4 Hz, 3H), 1.40 - 1.37 (m, 3H), 1.17 - 1.00 (m, 3H), 0.88 - 0.79 (m, 1H).

### Biological Evaluation

### Test Example 1: Inhibitory effect of the compounds of the present disclosure on ATR kinase activity

Test compounds to be tested were serially diluted with DMSO (maximum concentration 1 µM, 10 concentrations with 3-fold serial dilution) and added to the wells of a 384-well reaction plate (Corning, #4512). 1 nM ATR kinase (Eurofins, #14-953), 5 mM substrate (FAM-labeled RAD17 peptide, FAM-ASELPASQPQPFSAKKK), 5 mM ATP and the compounds with corresponding concentrations reacted in a kinase reaction solution (50 mM HEPES, pH 7.5; 0.0015% Brij-35; 10 mM MnCl₂, total volume of 20 µL) at 28°C for 3 hours. The reaction was terminated by adding 30 µL of stop solution (100 mM HEPES, pH 7.5; 0.015% Brij-35; 0.2% Coating Reagent #3, PerkinElmer; 50 mM EDTA). The reaction plate was placed on a Caliper instrument (PerkinElmer) for testing. The relative inhibitory activity of each compound concentration group was calculated using the following formula: % Inhibition = (Conversion rate of the blank control group without compound added - Conversion rate of the test compound group) / (Conversion rate of the blank control group - Conversion rate of the test compound group). The IC₅₀ values were calculated using GraphPad analysis and fitted. The results are shown in Table 1, wherein the IC₅₀ ranges are defined as follows: A: ≤ 10 nM; 100 nM ≥ B > 10 nM; 1000 nM ≥ C > 100 nM; 2500 nM ≥ D > 1000 nM.

**Table 1. Inhibitory effect (IC₅₀) of compounds on ATR kinase activity.**

| Compound No. | ATR kinase IC₅₀ (nM) | Compound No. | ATR kinase IC₅₀ (nM) |
|---|---|---|---|
| Compound 1 | C | Compound 75 | A |
| Compound 2 | B | Compound 77 | A |
| Compound 3 | B | Compound 78 | A |
| Compound 4 | B | Compound 79 | A |
| Compound 5 | B | Compound 81 | A |
| Compound 6 | C | Compound 82 | A |
| Compound 7 | B | Compound 83 | B |
| Compound 8 | B | Compound 84 | A |
| Compound 9 | B | Compound 85 | B |
| Compound 10 | B | Compound 86 | C |
| Compound 11 | B | Compound 87 | B |
| Compound 12 | B | Compound 88 | A |
| Compound 13A | B | Compound 89 | A |
| Compound 13B | C | Compound 90 | B |
| Compound 14 | B | Compound 91 | B |
| Compound 15 | B | Compound 92 | B |
| Compound 16 | A | Compound 93 | B |
| Compound 17 | B | Compound 94 | B |
| Compound 18 | A | Compound 95 | B |
| Compound 19 | A | Compound 96 | B |
| Compound 20A | B | Compound 97 | A |
| Compound 20B | B | Compound 98 | A |
| Compound 21 | B | Compound 99 | B |
| Compound 22 | A | Compound 100 | B |
| Compound 23 | A | Compound 101 | B |
| Compound 24 | A | Compound 102 | A |
| Compound 27 | A | Compound 103 | B |
| Compound 28 | B | Compound 104 | B |
| Compound 31A | A | Compound 105 | A |
| Compound 31B | A | Compound 106 | A |
| Compound 32A | B | Compound 107 | A |
| Compound 32B | B | Compound 108 | A |
| Compound 33A | A | Compound 109 | B |
| Compound 33B | C | Compound 110 | A |
| Compound 34 | A | Compound 112 | A |
| Compound 35A | A | Compound 113 | B |
| Compound 35B | A | Compound 114 | A |
| Compound 36A | A | Compound 115 | A |
| Compound 36B | A | Compound 116 | B |
| Compound 37A | B | Compound 119 | B |
| Compound 37B | A | Compound 120 | B |
| Compound 38 | A | Compound 121 | B |
| Compound 39A | A | Compound 122 | B |
| Compound 39B | A | Compound 123 | B |
| Compound 40A | A | Compound 132 | B |
| Compound 40B | B | Compound 135 | B |
| Compound 41A | A | Compound 136 | B |
| Compound 41B | A | Compound 137 | B |
| Compound 42A | A | Compound 138A | A |
| Compound 42B | B | Compound 138B | A |
| Compound 44A | B | Compound 139 | A |
| Compound 46C | A | Compound 141A | A |
| Compound 47A | A | Compound 141B | A |
| Compound 47B | A | Compound 142A | A |
| Compound 48A | B | Compound 142B | A |
| Compound 48B | A | Compound 144A | A |
| Compound 49A | A | Compound 144B | A |
| Compound 49B | B | Compound 145A | B |
| Compound 50 | A | Compound 145B | A |
| Compound 52 | B | Compound 146 | A |
| Compound 53 | B | Compound 147 | A |
| Compound 54 | B | Compound 149 | A |
| Compound 55 | B | Compound 151A | A |
| Compound 56 | B | Compound 151B | A |
| Compound 57 | B | Compound 151C | B |
| Compound 58 | B | Compound 151D | C |
| Compound 61 | D | Compound 152A | A |
| Compound 62 | D | Compound 152B | B |
| Compound 63 | C | Compound 153 | B |
| Compound 72A | C | Compound 154 | A |
| Compound 72B | C | | |

### Test Example 2: LoVo cell proliferation assay

LoVo (ATCC, #CRL-229) cells in logarithmic growth phase were digested, counted, and plated into 96-well cell culture plates at 2,000 cells per well. The plates containing cells were incubated overnight at 37°C in a 5% CO₂ incubator, and then test compounds with 3-fold gradient dilutions (maximum concentration: 10 µM, 9 concentrations) were added. DMSO was used as a blank control. After the LoVo cells containing the compounds or DMSO were incubated for an additional 5 days, an equal volume of CellTiter-Glo reagent (Promega, #7573) was added. After incubation at room temperature for 30 minutes, chemiluminescence was read using an Envision 2105 (PerkinElmer). The cell proliferation inhibition rate (%) for wells treated with each concentration of the compounds was calculated using the following formula. Then, with compound concentration as the horizontal axis and cell proliferation inhibition rate (%) as the vertical axis, GraphPad software was used to plot the data and determine the IC₅₀ values of the test compounds. The results are shown in Table 2, wherein the IC₅₀ ranges are defined as follows: A: ≤ 100 nM; 1000 nM ≥ B > 100 nM; C > 1000 nM.

Cell proliferation inhibition (%) = (1 - luminescence value of compound-treated group / average luminescence value of DMSO blank control group) × 100.

**Table 2. Inhibitory effect of compounds on LoVo growth**

| Compound No. | LoVo IC₅₀ (nM) | Compound No. | LoVo IC₅₀ (nM) |
|---|---|---|---|
| Compound 1 | B | Compound 71 | B |
| Compound 2 | B | Compound 72A | C |
| Compound 3 | B | Compound 72B | B |
| Compound 4 | B | Compound 73A | C |
| Compound 5 | B | Compound 73B | C |
| Compound 6 | B | Compound 74A | C |
| Compound 7 | B | Compound 74B | C |
| Compound 8 | B | Compound 75 | A |
| Compound 9 | C | Compound 76 | A |
| Compound 10 | C | Compound 77 | A |
| Compound 11 | B | Compound 78 | A |
| Compound 12 | B | Compound 79 | B |
| Compound 13A | B | Compound 80 | A |
| Compound 13B | C | Compound 81 | A |
| Compound 14 | B | Compound 82 | A |
| Compound 15 | B | Compound 83 | A |
| Compound 16 | B | Compound 84 | A |
| Compound 17 | C | Compound 85 | A |
| Compound 18 | A | Compound 86 | B |
| Compound 19 | B | Compound 87 | A |
| Compound 20A | C | Compound 88 | A |
| Compound 20B | C | Compound 89 | B |
| Compound 21 | B | Compound 90 | B |
| Compound 22 | A | Compound 91 | B |
| Compound 23 | B | Compound 92 | B |
| Compound 24 | B | Compound 93 | B |
| Compound 25 | A | Compound 95 | B |
| Compound 26 | B | Compound 96 | B |
| Compound 27 | B | Compound 97 | B |
| Compound 28 | C | Compound 98 | A |
| Compound 31A | A | Compound 99 | B |
| Compound 31B | A | Compound 100 | B |
| Compound 32A | B | Compound 101 | B |
| Compound 32B | A | Compound 102 | A |
| Compound 33A | A | Compound 103 | B |
| Compound 33B | B | Compound 105 | A |
| Compound 34 | B | Compound 106 | A |
| Compound 35A | A | Compound 107 | B |
| Compound 35B | A | Compound 108 | B |
| Compound 36A | B | Compound 109 | B |
| Compound 36B | A | Compound 110 | A |
| Compound 37A | A | Compound 111A | B |
| Compound 37B | A | Compound 111B | B |
| Compound 38 | B | Compound 112 | A |
| Compound 39A | A | Compound 113 | B |
| Compound 39B | C | Compound 114 | B |
| Compound 40A | B | Compound 115 | A |
| Compound 40B | B | Compound 116 | B |
| Compound 41A | A | Compound 119 | B |
| Compound 41B | A | Compound 120 | B |
| Compound 42A | A | Compound 121 | A |
| Compound 42B | A | Compound 122 | B |
| Compound 43 | B | Compound 123 | B |
| Compound 44A | B | Compound 132 | A |
| Compound 44B | B | Compound 135 | A |
| Compound 45A | B | Compound 136 | A |
| Compound 45B | B | Compound 138A | B |
| Compound 46A | B | Compound 138B | A |
| Compound 46B | B | Compound 139 | B |
| Compound 46C | A | Compound 140A | A |
| Compound 46D | B | Compound 140B | B |
| Compound 47A | A | Compound 141A | A |
| Compound 47B | A | Compound 141B | A |
| Compound 48A | B | Compound 142A | A |
| Compound 48B | A | Compound 142B | A |
| Compound 49A | A | Compound 143 | B |
| Compound 49B | B | Compound 144A | B |
| Compound 50 | A | Compound 144B | A |
| Compound 51 | B | Compound 145A | B |
| Compound 52 | C | Compound 145B | B |
| Compound 53 | C | Compound 146 | A |
| Compound 54 | B | Compound 147 | A |
| Compound 55 | B | Compound 148 | A |
| Compound 56 | C | Compound 149 | A |
| Compound 57 | B | Compound 150 | B |
| Compound 58 | B | Compound 151A | A |
| Compound 59A | B | Compound 151B | A |
| Compound 59B | B | Compound 151C | B |
| Compound 60 | B | Compound 151D | B |
| Compound 62 | C | Compound 152A | A |
| Compound 63 | B | Compound 152B | B |
| Compound 64 | B | Compound 153 | B |
| Compound 65 | B | Compound 154 | A |
| Compound 66 | B | | |

## Claims

1. A compound represented by Formula I, its isomer, isotope-labeled compound, or pharmaceutically acceptable salt thereof: 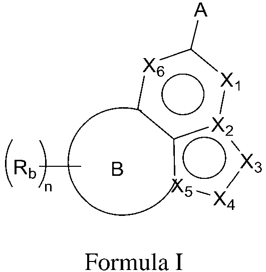 wherein:
○ indicates that the ring to which it belongs is an aromatic ring system;
X₁ is selected from the group consisting of N or C(R_{X1});
X₂ is selected from the group consisting of C or N;
X₃ is selected from the group consisting of N, C(Rx₂), or N(R_{X3});
X₄ is selected from the group consisting of N or C(R_{X4});
X₅ is selected from the group consisting of C or N;
X₆ is selected from the group consisting of N, N(R_{X5}), or C(R_{X6});
R_{X1}, R_{X2}, R_{X3}, R_{X4}, R_{X5}, and R_{X6} are each independently selected from the group consisting of H, halogen, C₆₋₁₀ aryl, C₃₋₁₀ cycloalkyl, C₃₋₁₀ cycloalkenyl, 3- to 10-membered heterocyclyl, or 5- to 10-membered heteroaryl, wherein the C₆₋₁₀ aryl, C₃₋₁₀ cycloalkyl, C₃₋₁₀ cycloalkenyl, 3- to 10-membered heterocyclyl, and 5- to 10-membered heteroaryl are each optionally substituted with 1, 2, or 3 R_{X} groups;
each R_{X} is independently selected from the group consisting of halogen, cyano, hydroxyl, C₁₋₆ alkyl, C₁₋₆ heteroalkyl, C₃₋₁₀ cycloalkyl, 3- to 10-membered heterocyclyl, or -NR_{c}R_{d}, wherein the C₁₋₆ alkyl, C₁₋₆ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
A is selected from the group consisting of 4- to 10-membered heterocyclyl, wherein the 4-to 10-membered heterocyclyl is optionally substituted with 1, 2, or 3 Rₐ groups;
each Rₐ is independently selected from the group consisting of C₁₋₁₀ alkyl, C₁₋₁₀ heteroalkyl, amino, or -S(=O)₂-C₁₋₁₀ alkyl;
Ring B is selected from the group consisting of 5- to 7-membered heterocyclic ring, 5- to 7-membered carbocyclic ring, benzene ring, or 5- to 7-membered heteroaromatic ring;
each R_{b} is independently selected from the group consisting of halogen, hydroxyl, cyano, amino, oxo, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, or 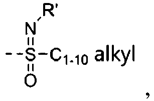 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₃₋₁₀ cycloalkyl, -CO-C₁₋₁₀ alkyl, -CONRₑR_{f}, -C(O)OC₁₋₁₀ alkyl, -SO₂NR_{g}Rₕ, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl and 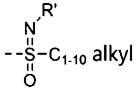 are each optionally substituted with 1, 2, 3 or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, or -CONRᵢRⱼ;
R' is selected from the group consisting of H, C₁₋₁₀ alkyl, C₃₋₁₀ cycloalkyl, or CN;
R_{c}, R_{d}, Rₑ, R_{f}, R_{g}, Rₕ, Rᵢ, and Rⱼ are each independently selected from the group consisting of H or C₁₋₁₀ alkyl;
alternatively, two R_{b} groups are connected together to form a C₃₋₁₀ cycloalkyl or a 3- to 10-membered heterocyclyl, wherein the C₃₋₁₀ cycloalkyl and 3- to 10-membered heterocyclyl are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, C₁₋₁₀ heteroalkyl, C₃₋₁₀ cycloalkyl, or 3- to 10-membered heterocyclyl;
n is selected from the group consisting of 0, 1, 2, 3, or 4.

2. The compound, its isomer, isotope-labeled compound, or pharmaceutically acceptable salt thereof according to claim 1, wherein the compound has structural Formula II: 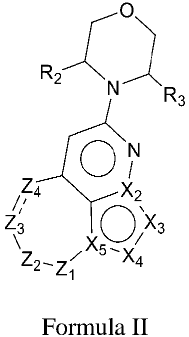 wherein:
○ indicates that the ring to which it belongs is an aromatic ring system;
-̅ -̅ -̅ represents either a single bond or a double bond;
R₁ and R₂ are each independently selected from the group consisting of hydrogen or C₁₋₆ alkyl; or
R₁ and R₂, together with the carbon atom to which they are attached and the adjacent nitrogen atom, form a 4- to 8-membered azacyclic ring;
X₂ is selected from the group consisting of C or N;
X₃ is selected from the group consisting of C(R₃) or N(R₃);
X₄ is selected from the group consisting of N or CH;
X₅ is selected from the group consisting of C or N, and when X₅ is N, X₃ is C(R₃);
R₃ is selected from the group consisting of C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heterocyclyl are each optionally substituted with 1, 2, or 3 R₃ₐ groups,
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, and 3- to 10-membered heterocyclyl are each optionally substituted with 1, 2, or 3 substituents independently selected from the group consisting of halogen, cyano, hydroxyl, or C₁₋₆ alkyl;
R₄ and R₅ are each independently selected from the group consisting of hydrogen or C₁₋₁₀ alkyl;
Z₁ is CR₆R₇;
Z₂ is selected from the group consisting of CR₈R₉, N, NR₁₀, O, S, -C(O)-, or -SO₂-;
Z₃ is selected from the group consisting of CR₁₁, CR₁₂R₁₃, N, NR₁₄, O, -C(O)-, -SO₂-, or -S(O)(NR₁₅)-;
Z₄ is selected from the group consisting of CR₁₆, CR₁₇R₁₈, N, NR₁₉, O, S, -C(O)-, -SO₂-, or -S(O)(NR₂₀)-;
one or two of Z₁, Z₂, Z₃, and Z₄ may be absent; and the ring containing X₅, Z₁, Z₂, Z₃, and Z₄ is a stable ring structure;
R₆, R₇, R₈, R₉, R₁₀, R₁₁, R₁₂, R₁₃, R₁₄, R₁₅, R₁₆, R₁₇, R₁₈, R₁₉, R₂₀ are each independently selected from the group consisting of hydrogen, halogen, hydroxyl, cyano, amino, C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl or 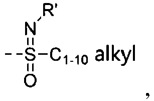 wherein the C₁₋₁₀ alkyl, C₁₋₁₀ heteroalkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, -CO-C₃₋₁₀ cycloalkyl, -CO-C₁₋₁₀ alkyl, -CONR₂₁R₂₂, -C(O)OC₁₋₁₀ alkyl, -SO₂NR₂₃R₂₄, -SO₂C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl and 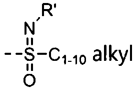 are each optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, hydroxy-C₁₋₁₀ alkyl, C₁₋₁₀ heteroalkyl, C₃₋₁₀ cycloalkyl, 3- to 10-membered heterocyclyl, -CONR₂₅R₂₆;
R' is selected from the group consisting of H, C₁₋₁₀ alkyl, C₃₋₁₀ cycloalkyl, or CN;
R₂₁, R₂₂, R₂₃, R₂₄, R₂₅, and R₂₆ are each independently selected from the group consisting of H or C₁₋₁₀ alkyl;
alternatively, R₆ and R₇, R₈ and R₉, R₁₂ and R₁₃, or R₁₇ and R₁₈ are connected together to form a C₃₋₁₀ cycloalkyl or a 3- to 10-membered heterocyclyl, wherein the C₃₋₁₀ cycloalkyl or 3-to 10-membered heterocyclyl is optionally substituted with 1, 2, 3, or 4 substituents independently selected from the group consisting of deuterium, halogen, hydroxyl, cyano, amino, C₁₋₁₀ alkyl, halogenated C₁₋₁₀ alkyl, C₁₋₁₀ heteroalkyl, C₃₋₁₀ cycloalkyl, 3- to 10-membered heterocyclyl.

3. The compound, its isomer, isotope-labeled compound, or pharmaceutically acceptable salt thereof according to claim 2, wherein:
R₆, R₇, R₈, R₉, R₁₁, R₁₂, R₁₃, R₁₆, R₁₇, and R₁₈ are each independently hydrogen, halogen, hydroxyl, cyano, C₁₋₁₀ alkyl, C₃₋₁₀ cycloalkyl, 3- to 10-membered heterocyclyl, C₁₋₁₀ alkoxy, C₂₋₅ alkenyl, C₂₋₅ alkynyl, C₆₋₁₀ aryl, 5- to 10-membered heteroaryl, -COC₁₋₁₀ alkyl, -CONR₂₁R₂₂, -C(O)OC₁₋₁₀ alkyl, -SO₂NR₂₃R₂₄ or -SO₂C₁₋₁₀ alkyl, wherein the C₁₋₁₀ alkyl, C₃₋₁₀ cycloalkyl, 3-to 10-membered heterocyclyl, C₁₋₁₀ alkoxy, C₂₋₅ alkenyl, C₂₋₅ alkynyl, C₆₋₁₀ aryl, 5- to 10-membered heteroaryl, -CO-C₁₋₁₀ alkyl, -CONR₂₁R₂₂, -C(O)OC₁₋₁₀ alkyl, -SO₂NR₂₃R₂₄, and -SO₂C₁₋₁₀ alkyl are each optionally substituted with 1, 2, 3, or 4 substituents independently selected from the group consisting of halogen, hydroxyl, cyano, amino, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, and -CONR₂₅R₂₆;
alternatively, R₆ and R₇, R₈ and R₉, R₁₂ and R₁₃, or R₁₇ and R₁₈ are connected together to form a C₃₋₁₀ cycloalkyl, wherein the C₃₋₁₀ cycloalkyl is optionally substituted with 1 or 2 substituents independently selected from the group consisting of halogen, hydroxyl, cyano, amino, C₁₋₆ alkyl, halogenated C₁₋₆ alkyl, C₁₋₆ heteroalkyl, C₃₋₆ cycloalkyl, or 3- to 6-membered heterocyclyl;
R₁₀, R₁₄, R₁₅, R₁₉, and R₂₀ are each independently hydrogen, C₁₋₁₀ alkyl, C₃₋₁₀ cycloalkyl, 3-to 10-membered heterocyclyl, C₂₋₆ alkenyl, C₂₋₆ alkynyl, C₆₋₁₀ aryl, 5- to 10-membered heteroaryl, C₁₋₁₀ alkoxy, -CO-C₁₋₁₀ alkyl, -CO-C₃₋₁₀ cycloalkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, or 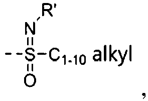 wherein the C₁₋₁₀ alkyl, C₃₋₁₀ cycloalkyl, 3- to 10-membered heterocyclyl, C₂₋₅ alkenyl, C₂₋₅ alkynyl, C₆₋₁₀ aryl, 5- to 10-membered heteroaryl, C₁₋₁₀ alkoxy, -CO-C₁₋₁₀ alkyl, -CO-C₃₋₁₀ cycloalkyl, -CONR₂₁R₂₂, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₆ alkyl-C₃₋₁₀ cycloalkyl, -C₁₋₆ alkyl-3- to 10-membered heterocyclyl, -C₁₋₆ alkyl-C₆₋₁₀ aryl, -C₁₋₆ alkyl-5- to 10-membered heteroaryl, and 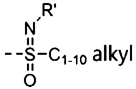 are each optionally substituted with 1, 2, 3, or 4 substituents independently selected from the group consisting of deuterium, halogen, hydroxyl, cyano, amino, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, or -CONR₂₅R₂₆;
R' is selected from the group consisting of H, C₁₋₁₀ alkyl, C₃₋₁₀ cycloalkyl, or CN;
R₂₁, R₂₂, R₂₃, R₂₄, R₂₅, and R₂₆ are each independently selected from the group consisting of H or C₁₋₆ alkyl.

4. The compound, its isomer, isotope-labeled compound, or pharmaceutically acceptable salt thereof according to claim 2 or 3, wherein the compound has structural Formula IIA, IIB, or IIC: 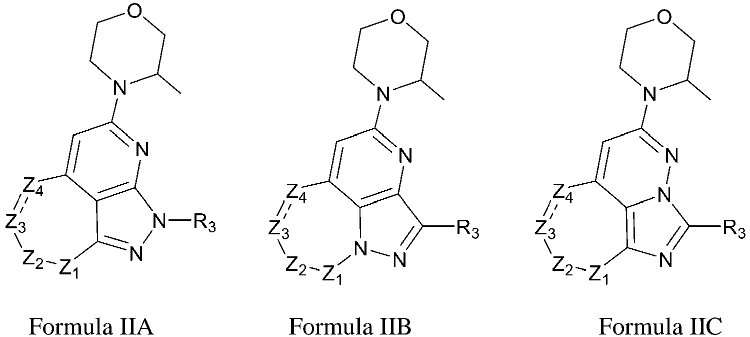 wherein:
R₃ is selected from the group consisting of C₆₋₁₀ aryl, C₃₋₁₀ cycloalkyl, or 5- 10-membered heteroaryl, wherein the C₆₋₁₀ aryl, C₃₋₁₀ cycloalkyl, and 5- 10-membered heteroaryl are each optionally substituted with 1, 2, or 3 R₃ₐ groups;
each R₃ₐ is independently selected from the group consisting of halogen, cyano, hydroxyl, C₁₋₁₀ alkyl, C₁₋₁₀ heteroalkyl, C₃₋₁₀ cycloalkyl, 3- to 10-membered heterocyclyl, or -NR₄R₅, wherein the C₁₋₁₀ alkyl, C₁₋₁₀ heteroalkyl, C₃₋₁₀ cycloalkyl, 3- to 10-membered heterocyclyl and -NR₄R₅ are each optionally substituted with 1, 2, or 3 substituents independently selected from the group consisting of halogen, cyano, hydroxyl, or C₁₋₆ alkyl;
R₄ and R₅ are each independently hydrogen or C₁₋₁₀ alkyl.

5. The compound, its isomer, or pharmaceutically acceptable salt thereof according to claim 4, wherein R₃ is a 5- to 6-membered heteroaryl, and the 5- to 6-membered heteroaryl is optionally substituted with 1 or 2 substituents independently selected from the group consisting of C₁₋₆ alkyl.

6. The compound, its isomer, isotope-labeled compound, or pharmaceutically acceptable salt thereof according to any one of claims 2 to 5, wherein:
Z₁ is CR₆R₇;
Z₂ is selected from the group consisting of CR₈R₉ or -C(O)-;
Z₃ is selected from the group consisting of CR₁₁, CR₁₂R₁₃, N, NR₁₄, O, or -C(O)-;
Z₄ is selected from the group consisting of absence, CR₁₆, CR₁₇R₁₈, N, NR₁₉, or -C(O)-;
R₆, R₇, R₈, R₉, R₁₁, R₁₂, R₁₃, R₁₆, R₁₇, and R₁₈ are each independently selected from the group consisting of hydrogen, hydroxyl, C₁₋₁₀ alkyl, C₃₋₁₀ cycloalkyl, 3- to 10-membered heterocyclyl, C₆₋₁₀ aryl, or 5- to 10-membered heteroaryl, wherein the C₁₋₁₀ alkyl, C₃₋₁₀ cycloalkyl, 3- to 10-membered heterocyclyl, C₆₋₁₀ aryl, and 5- to 10-membered heteroaryl are each optionally substituted with 1 or 2 substituents independently selected from the group consisting of halogen, C₁₋₆ alkyl, or halogenated C₁₋₆ alkyl;
alternatively, R₆ and R₇, R₈ and R₉, R₁₂ and R₁₃, or R₁₇ and R₁₈ are connected together to form a C₃₋₁₀ cycloalkyl, wherein the C₃₋₁₀ cycloalkyl is optionally substituted with 1 or 2 substituents independently selected from the group consisting of halogen, hydroxyl, cyano, amino, C₁₋₆ alkyl, halogenated C₁₋₆ alkyl, C₁₋₆ heteroalkyl, C₃₋₆ cycloalkyl, or 3- to 6-membered heterocyclyl;
R₁₄ and R₁₉ are each independently selected from the group consisting of hydrogen, C₁₋₁₀ alkyl, C₃₋₁₀ cycloalkyl, a 3- to 10-membered heterocyclyl, C₂₋₆ alkenyl, C₂₋₆ alkynyl, C₆₋₁₀ aryl, 5-to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl or 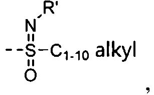 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5-to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, -C₁₋₄ alkyl-5- to 10-membered heteroaryl, and 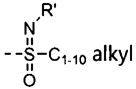 are each optionally substituted with 1, 2 or 3 substituents independently selected from the group consisting of deuterium, halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, 3- to 6-membered heterocyclyl, -CONR₂₅R₂₆;
R' is selected from the group consisting of H, C₁₋₁₀ alkyl, C₃₋₁₀ cycloalkyl, or CN;
R₂₅ and R₂₆ are each independently selected from the group consisting of hydrogen or C₁₋₆ alkyl.

7. The compound, its isomer, isotope-labeled compound, or pharmaceutically acceptable salt thereof according to any one of claims 2 to 6, wherein:
Z₁ is CR₆R₇;
R₆ and R₇ are each independently selected from the group consisting of hydrogen or C₁₋₁₀ alkyl.

8. The compound, its isomer, isotope-labeled compound, or pharmaceutically acceptable salt thereof according to any one of claims 2 to 7, wherein:
Z₂ is selected from the group consisting of CR₈R₉ or -C(O)-;
R₈ and R₉ are each independently selected from the group consisting of hydrogen or C₁₋₁₀ alkyl.

9. The compound, its isomer, isotope-labeled compound, or pharmaceutically acceptable salt thereof according to any one of claims 2 to 8, wherein:
Z₃ is selected from the group consisting of CR₁₁, CR₁₂R₁₃, N, NR₁₄, O, or -C(O)-;
R₁₁, R₁₂, and R₁₃ are each independently selected from the group consisting of hydrogen, hydroxyl, C₁₋₁₀ alkyl, C₃₋₁₀ cycloalkyl, 3- to 10-membered heterocyclyl, or 5- 10-membered heteroaryl, wherein the C₁₋₁₀ alkyl, C₃₋₁₀ cycloalkyl, 3- to 10-membered heterocyclyl, and 5-10-membered heteroaryl are each optionally substituted with 1 or 2 substituents independently selected from the group consisting of C₁₋₆ alkyl or halogenated C₁₋₆ alkyl;
R₁₄ is selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂NR₂₃R₂₄, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl or 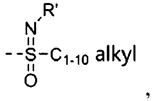 wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -CO-C₁₋₁₀ alkyl, -CO-C₃₋₁₀ cycloalkyl, -C(O)OC₁₋₁₀ alkyl, -SO₂-C₁₋₁₀ alkyl, -SO₂-C₂₋₆ alkenyl, -SO₂-C₂₋₆ alkynyl, -SO₂-C₃₋₁₀ cycloalkyl, -SO₂-3- to 10-membered heterocyclyl, -C₁₋₄ alkyl-C₃₋₁₀ cycloalkyl, -C₁₋₄ alkyl-3- to 10-membered heterocyclyl, and 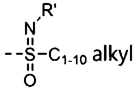 are each optionally substituted with 1, 2, or 3 substituents independently selected from the group consisting of halogen, hydroxyl, cyano, C₁₋₆ alkyl, or halogenated C₁₋₆ alkyl;
R' is selected from the group consisting of H, C₁₋₁₀ alkyl, C₃₋₁₀ cycloalkyl, or CN;
R₂₃ and R₂₄ are each independently selected from the group consisting of hydrogen or C₁₋₁₀ alkyl.

10. The compound, its isomer, isotope-labeled compound, or pharmaceutically acceptable salt thereof according to any one of claims 2 to 9, wherein:
Z₄ is selected from the group consisting of absence, CR₁₆, CR₁₇R₁₈, N, NR₁₉, or -C(O)-;
R₁₆, R₁₇, and R₁₈ are each independently selected from the group consisting of hydrogen, C₁₋₁₀ alkyl, or C₃₋₁₀ cycloalkyl, wherein the C₁₋₁₀ alkyl and C₃₋₁₀ cycloalkyl are each optionally substituted with 1 or 2 substituents independently selected from the group consisting of halogen, C₁₋₆ alkyl, or halogenated C₁₋₆ alkyl;
alternatively, R₁₇ and R₁₈ are connected together to form a C₃₋₁₀ cycloalkyl, wherein the C₃₋₁₀ cycloalkyl is optionally substituted with 1 or 2 substituents independently selected from the group consisting of halogen, hydroxyl, cyano, amino, C₁₋₆ alkyl, halogenated C₁₋₆ alkyl, C₁₋₆ heteroalkyl, C₃₋₆ cycloalkyl, or 3- to 6-membered heterocyclyl;
R₁₉ is selected from the group consisting of hydrogen, C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -SO₂-C₁₋₁₀ alkyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl or -C₁₋₄ alkyl-5- to 10-membered heteroaryl, wherein the C₁₋₁₀ alkyl, C₃₋₁₀ cycloalkyl, 3- to 10-membered heterocyclyl, C₂₋₆ alkenyl, C₂₋₆ alkynyl, C₆₋₁₀ aryl, 5- to 10-membered heteroaryl, -SO₂C₁₋₁₀ alkyl, -C₁₋₄ alkyl-C₃₋₁₀ cycloalkyl, -C₁₋₄ alkyl-3- to 10-membered heterocyclyl, -C₁₋₄ alkyl-C₆₋₁₀ aryl, and -C₁₋₄ alkyl-5- to 10-membered heteroaryl are each optionally substituted with 1, 2, or 3 substituents independently selected from the group consisting of halogen, hydroxyl, cyano, C₁₋₆ alkyl, halogenated C₁₋₆ alkyl, hydroxy-C₁₋₆ alkyl, C₁₋₆ heteroalkyl, C₃₋₆ cycloalkyl, and -CONR₂₅R₂₆; and
R₂₅ and R₂₆ are each independently selected from the group consisting of hydrogen or C₁₋₆ alkyl.

11. The compound, its isomer, isotope-labeled compound, or pharmaceutically acceptable salt thereof according to any one of claims 1 to 10, wherein the compound is selected from the group consisting of:

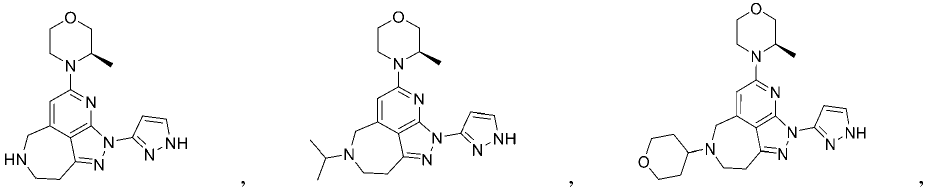

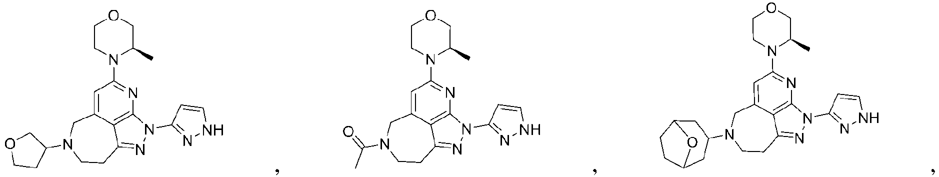

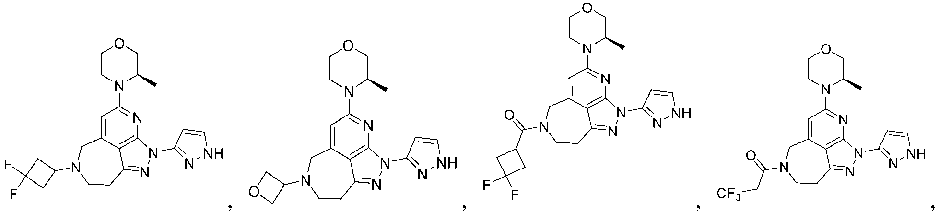

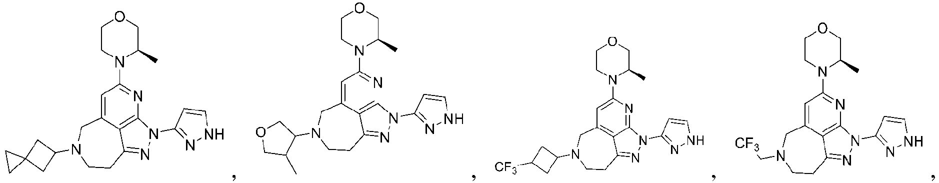

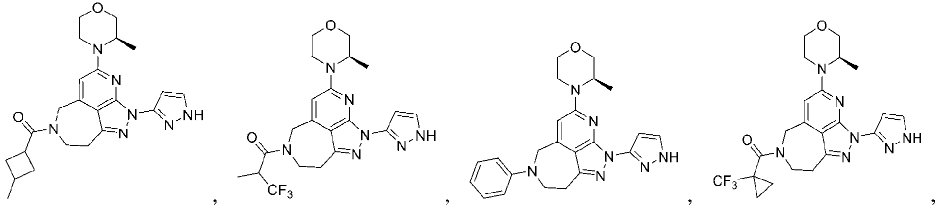

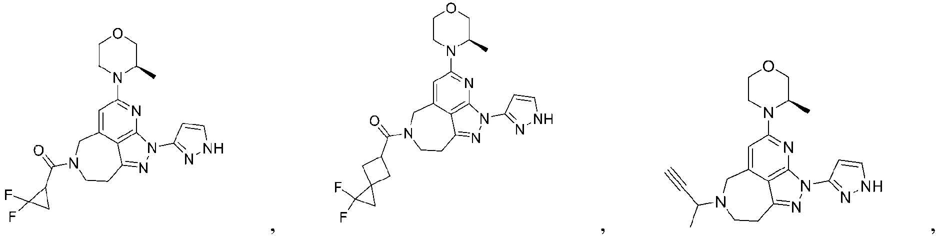

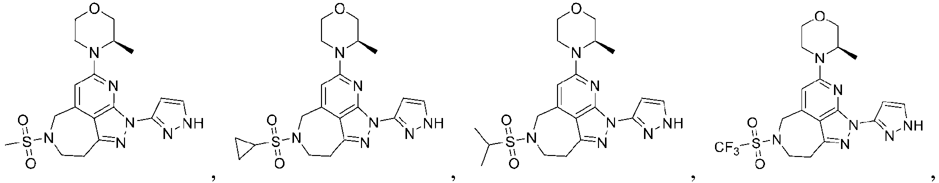

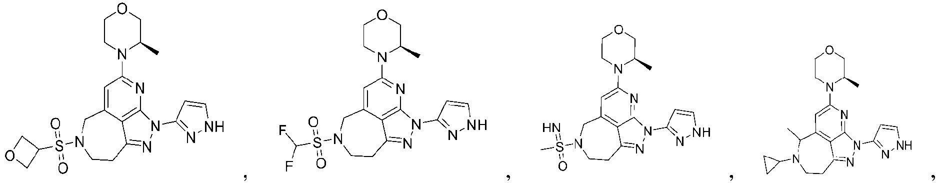

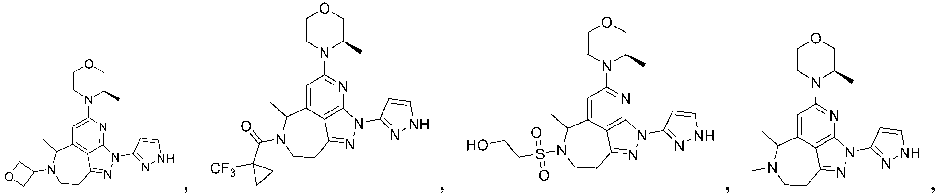

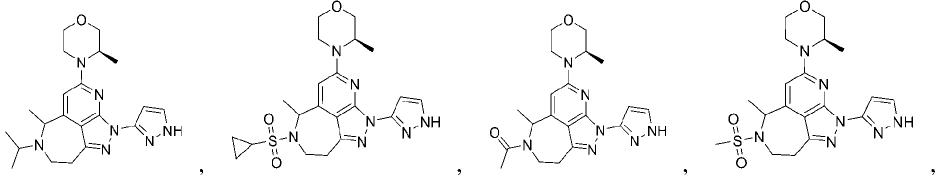

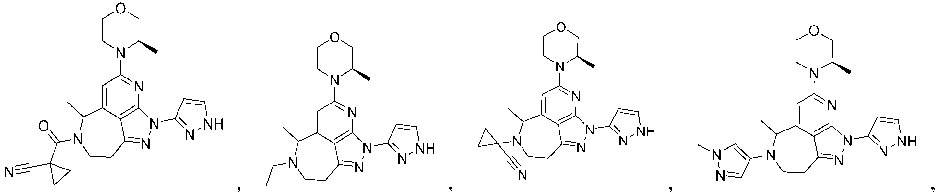

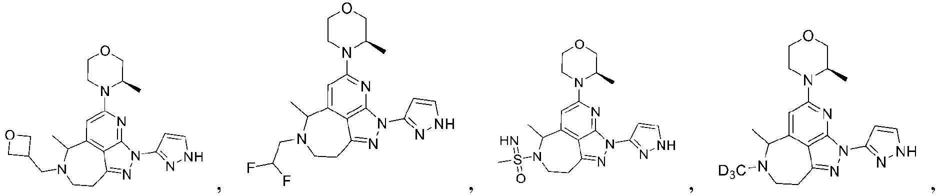

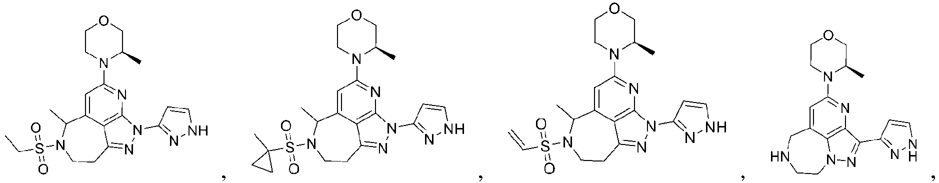

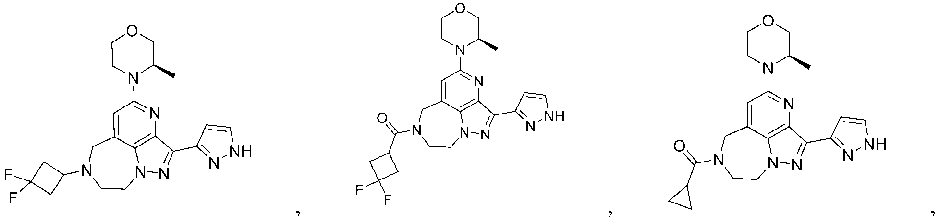

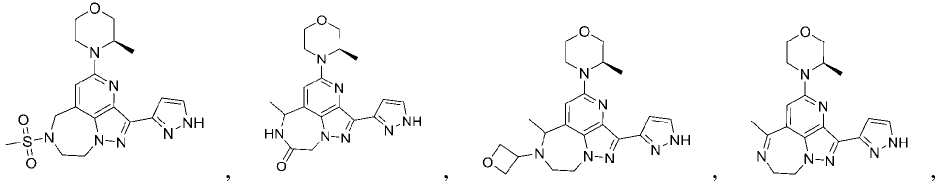

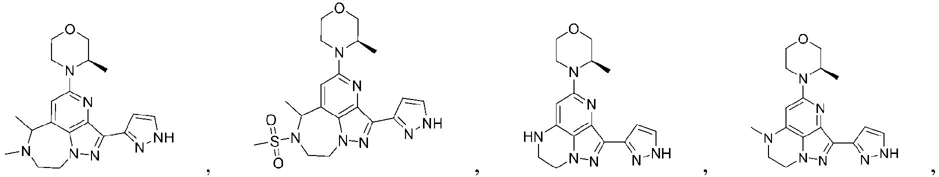

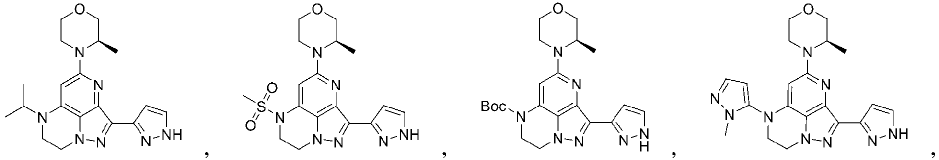

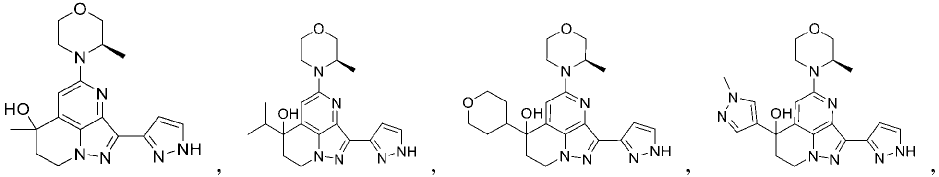

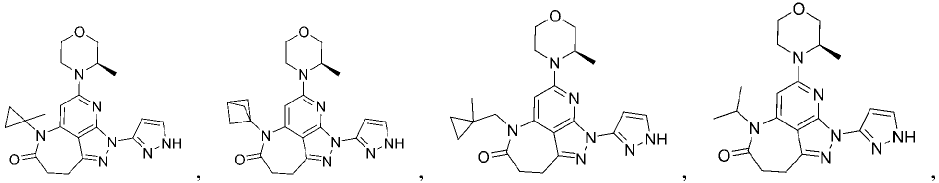

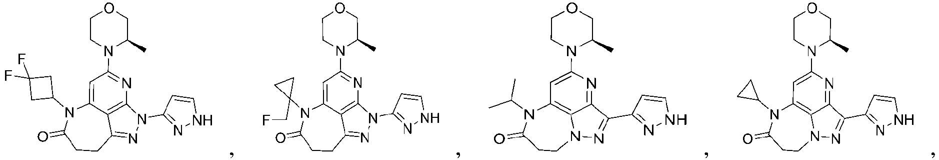

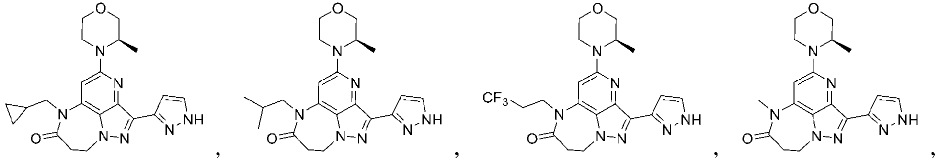

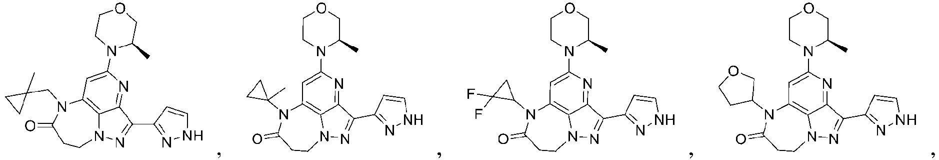

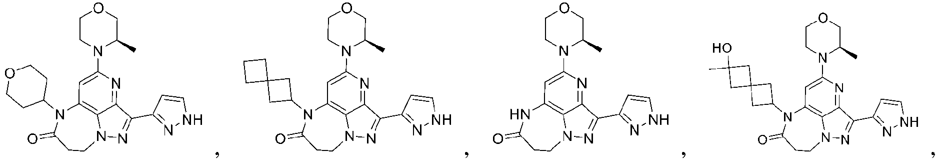

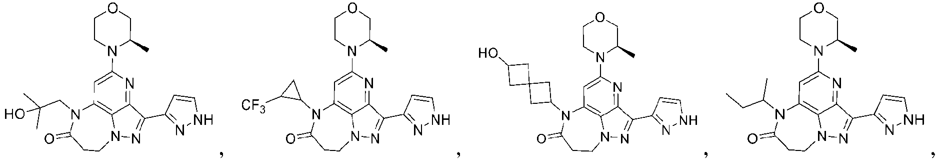

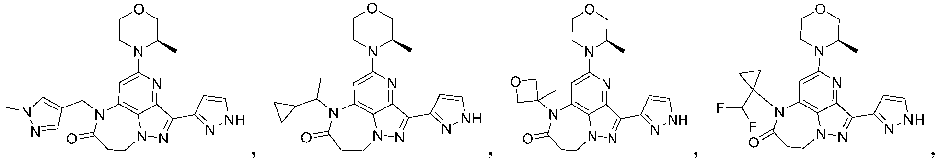

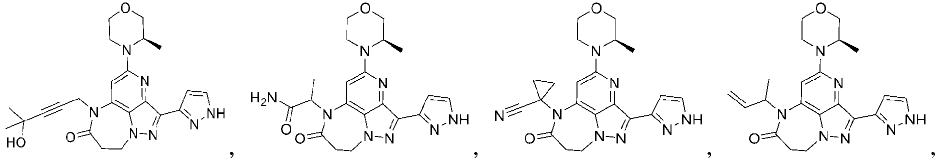

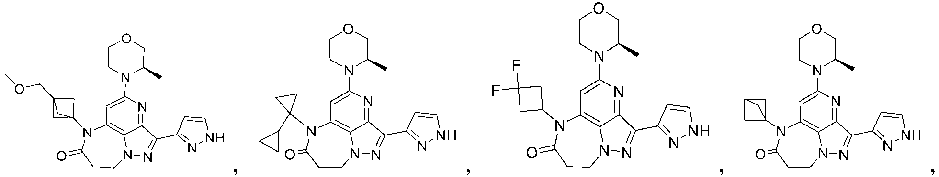

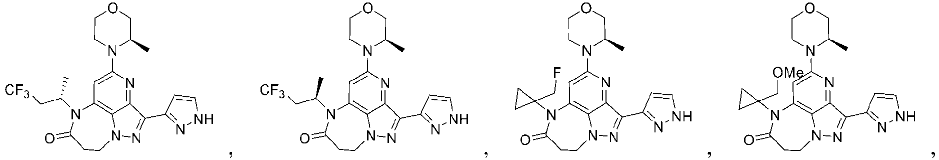

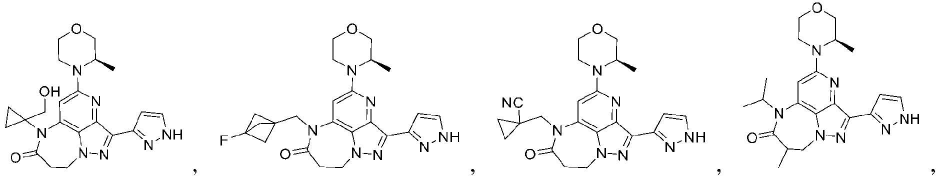

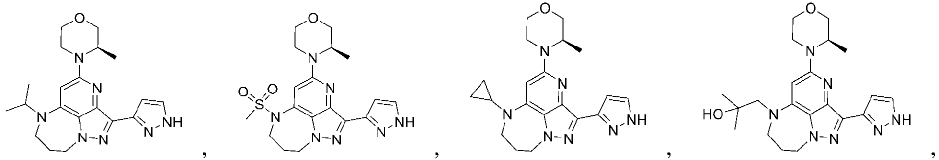

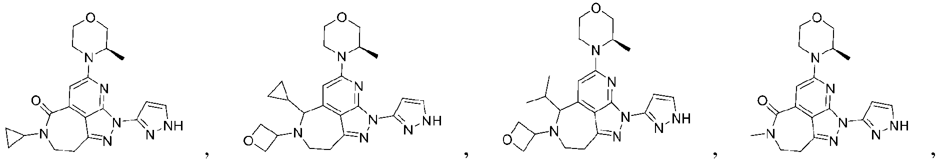

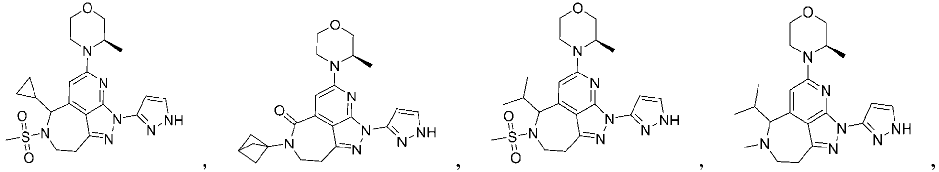

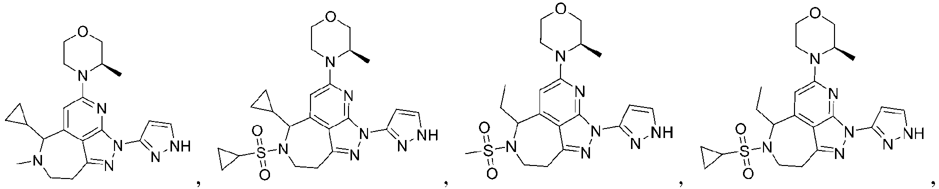

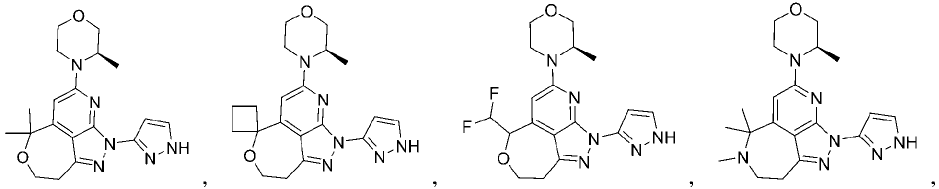

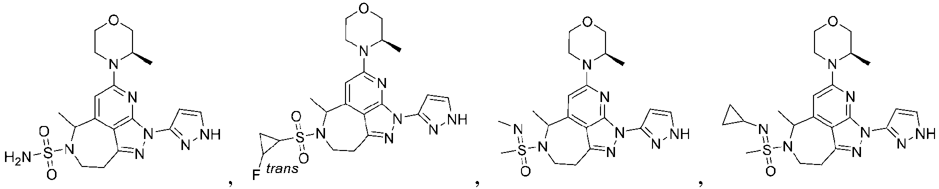

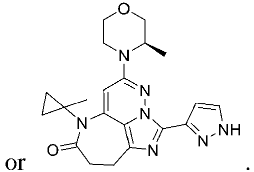

12. A pharmaceutical composition, which comprises the compound, its isomer, isotope-labeled compound, or pharmaceutically acceptable salt thereof according to any one of claims 1 to 11, and a pharmaceutically acceptable excipient.

13. A compound, its isomer, isotope-labeled compound, or pharmaceutically acceptable salt thereof, according to any one of claims 1 to 11, for use as a medicament.

14. A compound, its isomer, isotope-labeled compound, or pharmaceutically acceptable salt thereof according to any one of claims 1 to 11 for use in the treatment of cancer.

15. The compound for use according to claim 14, wherein the cancer is a colon cancer.
